(19) Europäisches Patentamt
European Patent Office
Office européen des brevets

(11) EP 3 718 545 A1

(12) **EUROPEAN PATENT APPLICATION**
published in accordance with Art. 153(4) EPC

(43) Date of publication:
**07.10.2020 Bulletin 2020/41**

(21) Application number: **18883950.0**

(22) Date of filing: **28.11.2018**

(51) Int Cl.:
***A61K 31/4245*** *(2006.01)* ***A61K 45/00*** *(2006.01)*
***A61P 35/00*** *(2006.01)* ***A61P 43/00*** *(2006.01)*
***C07D 249/04*** *(2006.01)*

(86) International application number:
**PCT/JP2018/043697**

(87) International publication number:
**WO 2019/107386 (06.06.2019 Gazette 2019/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.11.2017 JP 2017229681**

(71) Applicant: **Taiho Pharmaceutical Co., Ltd.**
**Chiyoda-ku, Tokyo 101-8444 (JP)**

(72) Inventors:
- **UENO, Hiroyuki**
**Tsukuba-shi**
**Ibaraki 300-2611 (JP)**
- **HOSHINO, Takuya**
**Tsukuba-shi**
**Ibaraki 300-2611 (JP)**

(74) Representative: **Schiener, Jens**
**Wächtershäuser & Hartz**
**Patentanwaltspartnerschaft mbB**
**Weinstraße 8**
**80333 München (DE)**

(54) **ANTITUMOR AGENT**

(57) The present invention provides a method of enhancing an antitumor effect by a compound strongly inhibiting ribonucleotide reductase (RNR) or a salt thereof.

A combination formulation involving combined administration of a sulfonamide compound represented by Formula (I) [In the formula, $X^1$ represents an oxygen atom or the like; $X^2$ represents an oxygen atom; $X^3$ represents -NH-; $X^4$ represents a hydrogen atom or the like; $R^1$ represents $-C(R^{11})(R^{12})-$ or the like; $R^{11}$ and $R^{12}$ are the same or different and each represents a hydrogen atom or the like; $R^2$ represents an optionally substituted C6-C14 aromatic hydrocarbon group or the like; $R^3$ represents an optionally substituted C6-C14 aromatic hydrocarbon group or the like; $R^4$ represents a hydrogen atom or the like] or a salt thereof, having RNR inhibitory activity, and other antitumor agent(s).

[Figure 1]

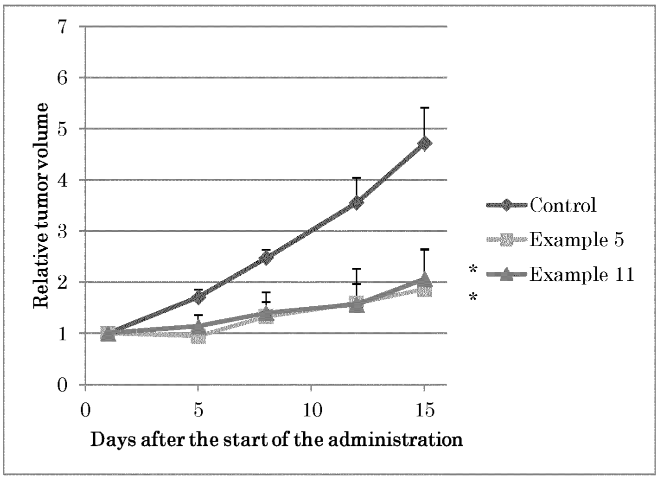




Printed by Jouve, 75001 PARIS (FR)

## Description

Technical Field

The present invention relates to an antitumor agent and more particularly relates to a combination formulation comprising a sulfonamide compound or a salt thereof and other antitumor agent(s) in combination, and an agent for enhancing an antitumor effect of other antitumor agent(s).

Background Art

Ribonucleotide reductase (hereinafter also referred to as RNR) is composed of a hetero-oligomer of a large subunit M1 and a small subunit M2, and expression of both is required for enzyme activity. RNR recognizes ribonucleoside 5'-diphosphate (hereinafter also referred to as NDP) as a substrate and catalyzes a reduction reaction to 2'-deoxyribonucleoside 5'-diphosphate (hereinafter also referred to as dNDP). Since RNR is a rate-limiting enzyme in the de novo dNTP synthesis pathway, RNR plays an essential role in DNA synthesis and repair (Non-Patent Document 1).

The enzymatic activity of RNR is closely related to cell proliferation, and there is a report that the enzymatic activity is particularly high in cancer (Non-Patent Document 2). Indeed, in various types of solid tumors and blood cancers, numerous correlations have been reported with overexpression of M2, a subunit of RNR, and their prognosis (Non-Patent Documents 3 and 4). In addition, cell growth inhibition by inhibiting RNR and antitumor effect in vivo have been reported in cell lines derived from several cancer types and in nonclinical models (Non-Patent Documents 5 and 6), thus it is strongly suggested that RNR is one of important target molecules for cancer treatment.

Conventionally, hydroxyurea (hereinafter also referred to as HU) and 3-aminopyridine-2-carboxaldehyde thiosemicarbazone (hereinafter also referred to as 3-AP) are known as compounds having an RNR inhibitory activity. These compounds differ in structure from the sulfonamide compounds of the present invention. Although HU has been used clinically for over 30 years, its RNR inhibitory activity is very weak and its effect is limited (Non-Patent Document 7). In addition, tolerance to the use of HU is also considered a problem (Non-Patent Document 8). Meanwhile, 3-AP has a structure having the capability to chelate to metal ions, and it has been known that 3-AP chelates mainly to iron ions, thereby inhibiting RNR (Non-Patent Document 9). However, 3-AP has been suggested as having an off-target effect to various other iron-ion-requiring proteins, and it has been known that side effects such as hypoxia, dyspnea, methemoglobinemia and the like are caused in clinical cases (Non-Patent Document 10).

Therefore, it has been strongly desired to develop an RNR inhibitor which has a better RNR inhibitory activity and a structure which does not chelate with metal ions and is useful for diseases associated with RNR, such as tumors.

Some combination effects brought about by combinations of RNR inhibitors and additional antitumor agents have been reported. For example, enhancement in cell proliferation inhibitory effect on a non-small cell lung cancer cell line by the combination of an RNR inhibitor 3-AP and an antimetabolite cytarabine or gemcitabine has been reported (Non-Patent Document 11). Also, in vivo enhancement in life extending effect by the combination of 3-AP and a topoisomerase inhibiting drug etoposide or a platinum drug cisplatin, etc. has been reported using mouse leukemia models (Non-Patent Document 12).

Citation List

Non Patent Literature

Non-Patent Document 1: Annu. Rev. Biochem. 67, 71-98. (1998)
Non-Patent Document 2: J. Biol. Chem. 245, 5228-5233. (1970)
Non-Patent Document 3: Nat. Commun. 5, 3128 doi: 10.1038 / ncomms 4128 (2014)
Non-Patent Document 4: Clin. Sci. 124, 567-578. (2013)
Non-Patent Document 5: Expert. Opin. Ther. Targets 17, 1423 - 1437 (2013)
Non-Patent Document 6: Biochem. Pharmacol. 59, 983-991 (2000)
Non-Patent Document 7: Biochem. Pharmacol. 78, 1178- 11 85 (2009)
Non-Patent Document 8: Cancer Res. 54, 3686-3691 (1994)
Non-Patent Document 9: Pharmacol. Rev. 57, 547-583 (2005)
Non-Patent Document 10: Future Oncol. 8, 145-150 (2012)
Non-Patent Document 11: Biochem. Pharmacol. 73, 1548-1557 (2007)
Non-Patent Document 12: Biochem. Pharmacol. 59, 983-991 (2000)

Summary of Invention

Technical Problem

An object of the present invention is to provide a method of enhancing an antitumor effect by a compound strongly inhibiting RNR

Solution to Problem

As a result of extensive studies to solve the above-mentioned problems, the inventors of the present invention have found that a group of compounds having a sulfonamide structure represented by the following formula (I) has excellent antitumor effect enhancing activity by combined use with other compound(s) having an antitumor effect (other antitumor agent(s)), and completed the present invention.

The present invention provides the following: [1] to [15].

[1] A combination formulation for treating and/or preventing tumor, comprising a sulfonamide compound represented by the following formula (I):

[Formula 1]

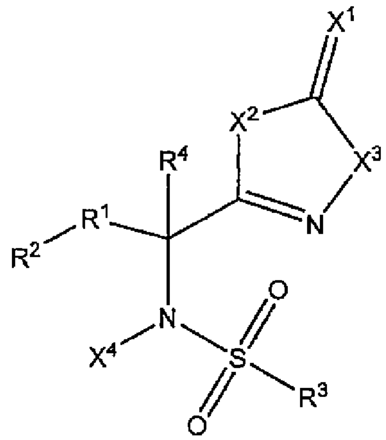

(I)

[In the formula,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;

(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)]
or a salt thereof, and other antitumor agent(s).

[2] The combination formulation according to [1], wherein in formula (I):

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
$R^{11}$ and $R^{12}$ are the same or different and represent a hydrogen atom or a C1-C6 alkyl group;
$R^2$ represents a C6-C14 aromatic hydrocarbon group, wherein $R^2$ may have $R^{21}$ as a substituent;
$R^{21}$ represents a halogen atom or a C1-C6 alkyl group (when two or more of $R^{21}$ are present, $R^{21}$ are the same as or different from each other);
$R^3$ represents a C6-C14 aromatic hydrocarbon group which may have $R^{31}$ as a substituent or may be fused with a 4-8 membered saturated heterocyclic ring (wherein the saturated heterocyclic ring may have Rc as a substituent);
$R^{31}$ represents a halogen atom or an aminocarbonyl group (when two or more of $R^{31}$ are present, $R^{31}$ are the same as or different from each other);
Rc represents a halogen atom, a hydroxy group, or a C1-C6 alkyl group (when two or more of Rc are present, Rc are the same as or different from each other); and
$R^4$ represents a hydrogen atom.

[3] The combination formulation according to [1] or [2], wherein in formula (I),

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
one of $R^{11}$ and $R^{12}$ represents a hydrogen atom, and the other represents a C1-C6 alkyl group;
$R^2$ represents a phenyl group, wherein $R^2$ may have $R^{21}$ as a substituent;
$R^{21}$ represents a halogen atom or a C1-C6 alkyl group (when two or more of $R^{21}$ are present, $R^{21}$ are the same as or different from each other);
$R^3$ represents a phenyl group which may have $R^{31}$ as a substituent or may be fused with a monocyclic 6 membered saturated heterocyclic ring having one oxygen atom (wherein the saturated heterocyclic ring may have Rc as a substituent);
$R^{31}$ represents a halogen atom or an aminocarbonyl group (when two or more of $R^{31}$ are present, $R^{31}$ are the same as or different from each other);
Rc represents a halogen atom, a hydroxy group, or a C1-C6 alkyl group (when two or more of Rc are present, Rc are the same as or different from each other); and
$R^4$ represents a hydrogen atom.

[4] The combination formulation according to any one of [1]-[3], wherein in formula (I),

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
one of $R^{11}$ and $R^{12}$ represents a hydrogen atom, and the other represents a methyl group;
$R^2$ represents a phenyl group having $R^{21}$ as a substituent;
$R^{21}$ represents a halogen atom or a C1-C6 alkyl group (when two or more of $R^{21}$ are present, $R^{21}$ are the same as or different from each other);
$R^3$ represents a phenyl group having $R^{31}$ as a substituent or a chromanyl group having Rc as a substituent;
$R^{31}$ represents a halogen atom or an aminocarbonyl group (when two or more of $R^{31}$ are present, $R^{31}$ are the

same as or different from each other);
Rc represents a halogen atom, a hydroxy group, or a C1-C6 alkyl group (when two or more of Rc are present, Rc are the same as or different from each other); and
$R^4$ represents a hydrogen atom.

[5] The combination formulation according to any one of [1]-[4], wherein the sulfonamide compound is selected from the following compounds (1)-(5):

(1) 5-bromo-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide;
(2) 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide;
(3) 5-chloro-2-(N-((1S,2R)-2-(3-chloro-6-fluoro-2-methylphenyl)-1-(5-oxo-4,5-dihydro-1,3,4-oxadiazol-2-yl)propyl)sulfamoyl)benzamide;
(4) 5-chloro-N-((1S,2R)-2-(3-chloro-6-fluoro-2-methylphenyl)-1-(5-oxo-4,5-dihydro-1,3,4-oxadiazol-2-yl)propyl)-4-hydroxy-4-methylchromane-8-sulfonamide; and
(5) 5-chloro-N-((1S,2R)-2-(6-fluoro-2,3-dimethylphenyl)-1-(5-oxo-4,5-dihydro-1,3,4-oxadiazol-2-yl)propyl)-4-hydroxychromane-8-sulfonamide.

[6] The combination formulation according to any one of [1]-[5], wherein the sulfonamide compound represented by formula (I) or a salt thereof and the other antitumor agent(s) are administered concurrently, sequentially, or in a staggered manner.
[7] The combination formulation according to any one of [1]-[6], wherein the other antitumor agent(s) is at least one or more selected from an antimetabolite, a platinum drug, a plant alkaloid drug, and a molecular targeting drug.
[8] The combination formulation according to any one of [1]-[7], wherein the other antitumor agent(s) is at least one or more selected from 5-fluorouracil (5-FU), trifluridine, fludarabine (or an active metabolite fludarabine nucleoside), cytarabine, gemcitabine, decitabine, guadecitabine, azacitidine, cisplatin, oxaliplatin, carboplatin, etoposide, AZD6738, prexasertib, SCH900776, luminespib, olaparib, talazoparib, lapatinib, sunitinib, cabozantinib, and midostaurin.
[9] An agent for enhancing an antitumor effect of other antitumor agent(s), comprising a sulfonamide compound represented by the following formula (I):

[Formula 2]

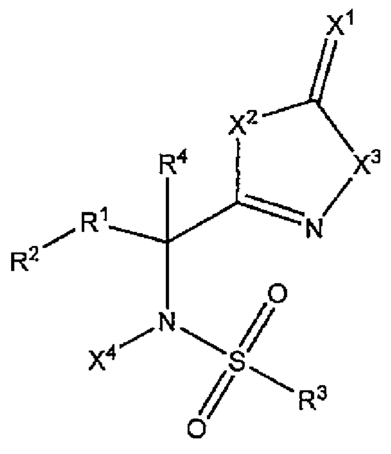

(I)

[In the formula,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;

$R^1$ represents $-C(R^{11})(R^{12})-$ or $-C(=CH_2)-$;

$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;

$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;

$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and

$R^4$ represents a hydrogen atom or a C1-C6 alkyl group;

(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)]

or a salt thereof as an active ingredient.

[10] An antitumor agent for treating a cancer patient dosed with other antitumor agent(s), comprising a sulfonamide compound or a salt thereof, wherein the sulfonamide compound is a compound represented by the following formula (I):

[Formula 3]

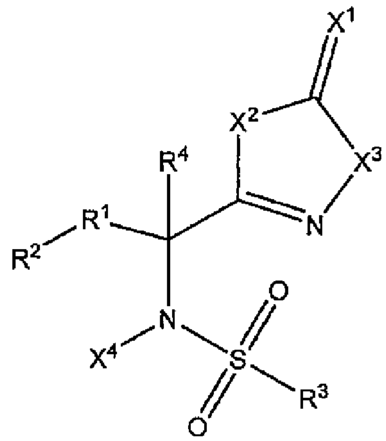

(I)

[In the formula,

$X^1$ represents an oxygen atom or a sulfur atom;

$X^2$ represents an oxygen atom or -NH-;

$X^3$ represents -NH- or an oxygen atom;

$X^4$ represents a hydrogen atom or a C1-C6 alkyl group;

$R^1$ represents $-C(R^{11})(R^{12})-$ or $-C(=CH_2)-$;

$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;

$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;

$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)].

[11] A pharmaceutical composition comprising a sulfonamide compound or a salt thereof and other antitumor agent(s), wherein the sulfonamide compound is a compound represented by the following formula (I):

[Formula 4]

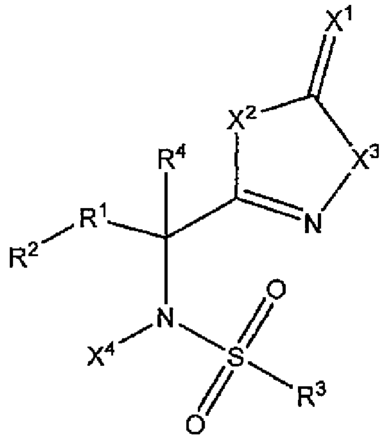

(I)

[In the formula,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)].

[12] A sulfonamide compound represented by the following formula (I):

[Formula 5]

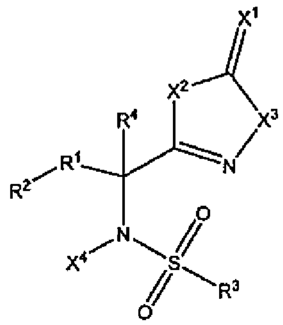

(I)

[In the formula,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)]
or a salt thereof for use in treating and/or preventing tumor by administering the sulfonamide compound or a salt thereof and other antitumor agent(s) concurrently, sequentially, or in a staggered manner.

[13] A product comprising a sulfonamide compound represented by the following formula (I):

[Formula 6]

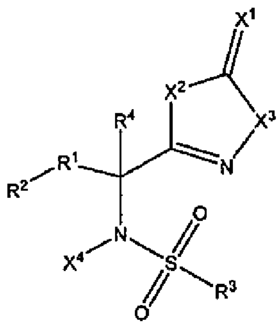

(I)

[In the formula,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)]
or a salt thereof for use in treating and/or preventing tumor by administering the sulfonamide compound or a salt thereof and other antitumor agent(s) concurrently, sequentially, or in a staggered manner,
and the other antitumor agent(s), as a combination formulation.

[14] A method of treating and/or preventing tumor comprising administering a sulfonamide compound represented by the following formula (I):

[Formula 7]

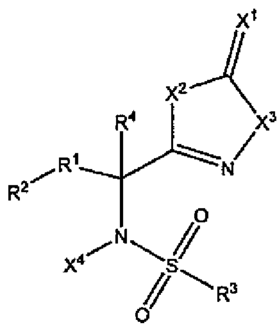

(I)

[In the formula,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)]
or a salt thereof and other antitumor agent(s) concurrently, sequentially, or in a staggered manner.

[15] A method of treating and/or preventing tumor comprising administering a sulfonamide compound represented by the following formula (I):

[Formula 8]

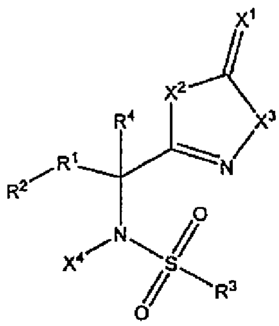

(I)

[In the formula,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms

to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)]
or a salt thereof to a cancer patient dosed with other antitumor agent(s).

The present invention also relates to the following aspects:
A pharmaceutical composition for preventing and/or treating tumor, comprising the above-mentioned sulfonamide compound represented by formula (I) or a salt thereof and other antitumor agent(s).
The sulfonamide compound represented by the above formula (I) or a salt thereof for enhancing an antitumor effect of other antitumor agent(s).
Use of the sulfonamide compound represented by the above formula (I) or a salt thereof for enhancing an antitumor effect of other antitumor agent(s).
Use of the sulfonamide compound represented by the above formula (I) or a salt thereof for manufacturing an agent for enhancing an antitumor effect of other antitumor agent(s).
A method of preventing and/or treating tumor comprising the step of administering prophylactically and/or therapeutically effective amounts of the sulfonamide compound represented by the above formula (I) or a salt thereof and other antitumor agent(s) in combination to a patient.
A method of preventing and/or treating tumor comprising the step of administering a prophylactically and/or therapeutically effective amount of the sulfonamide compound represented by the above formula (I) or a salt thereof to a cancer patient dosed with other antitumor agent(s).
A method of enhancing an antitumor effect comprising the step of administering a therapeutically and/or prophylactically effective amount of the sulfonamide compound represented by the above formula (I) or a salt thereof to a cancer patient dosed with other antitumor agent(s).
A product comprising the sulfonamide compound represented by the above formula (I) or a salt thereof and other antitumor agent(s) as a combination formulation used concurrently, sequentially, or in a staggered manner in preventing and/or treating tumor.
The present specification encompasses the contents disclosed in Japanese Patent Application No. 2017-229681 on which the priority of the present application is based.

Advantageous Effects of Invention

According to the present invention, cancer treatment that exerts an excellent antitumor effect can be performed while side effects are prevented.

Brief Description of Drawings

[Figure 1] It is a diagram illustrating an antitumor effect by the oral administration of Example Compounds 5 and 11 to human-derived blood cancer cell line subcutaneous transplantation mice in terms of daily variation of relative tumor volume (hereinafter also referred to as "RTV").
[Figure 2] It is a diagram illustrating an antitumor effect by the oral administration of Example Compounds 1 and 14 to human-derived blood cancer cell line subcutaneous transplantation mice in terms of daily variation of RTV.
[Figure 3] It is a diagram illustrating an antitumor effect by the oral administration of Example Compounds 209A, 222A and 235A to human-derived blood cancer cell line subcutaneous transplantation mice in terms of daily variation of RTV.
[Figure 4] It is a diagram illustrating an antitumor effect by the oral administration of Example Compounds 200A and 228A to human-derived blood cancer cell line subcutaneous transplantation mice in terms of daily variation of RTV.

Detailed Description of the Invention

In the present invention, RNR inhibitors that bring about an excellent synergistic effect with other antitumor agent(s) are sulfonamide compounds represented by the above formula (I) or salts thereof.

"CA-CB" as used herein for description of groups refers to a group having a carbon number of A-B. For example, "C1-C6 alkyl group" represents an alkyl group having 1 to 6 carbon atoms. The term "A-B membered" indicates that the number of atoms constituting the ring (ring members) is A-B. For example, "5-10 membered unsaturated heterocyclic group" means an unsaturated heterocyclic group whose ring member is 5-10.

"Substituent" as used herein refers to a halogen atom, a hydroxy group, an amino group, an oxo group, a cyano group, a nitro group, a carboxyl group, an aminocarbonyl group, a thioamide group, a C1-C6 alkyl group, a C2-C6 alkynyl group, a C3-C6 cycloalkyl group, a C1-C6 alkoxy group, a C1-C6 alkoxy C1-C6 alkoxy group, a halogeno C1-C6 alkyl group, a halogeno C1-C6 alkoxy group, a C6-C14 aromatic hydrocarbon group, an unsaturated heterocyclic group, a saturated heterocyclic group, a nitrogen-containing saturated heterocyclic group, a nitrogen-containing saturated heterocyclic carbonyl group, a C1-C14 acyl group, a C1-C14 acylamino group, a C2-C7 alkoxycarbonyl group, a C1-C14 acyloxy group, C7-C13 aralkyloxy group and the like.

"Halogen atom" as used herein refers to a fluorine atom, a chlorine atom, a bromine atom, and an iodine atom.

"C1-C6 alkyl group" as used herein refers to a straight or branched saturated hydrocarbon group having 1 to 6 carbon atoms, such as a methyl group, an ethyl group, an n-propyl group, an isopropyl group, an n-butyl group, an isobutyl group, a tert-butyl group, an n-pentyl group, an isopentyl group, a hexyl group and the like.

"C2-C6 alkynyl group" as used herein refers to an unsaturated straight-chain or branched hydrocarbon group having 2 to 6 carbon atoms and at least one triple bond, e.g., ethynyl, 1- or 2-propynyl group, 1-, 2- or 3-butynyl group, 1-methyl-2-propynyl group and the like.

"C3-C6 cycloalkyl group" as used herein refers to a saturated cyclic hydrocarbon group having 3 to 6 carbon atoms, for example, a cyclopropyl group, a cyclobutyl group, a cyclopentyl group, a cyclohexyl group and the like.

"C1-C6 alkoxy group" as used herein refers to an oxy group to which a straight-chain or branched saturated hydrocarbon group having 1 to 6 carbon atoms is bonded, for example, a methoxy group, an ethoxy group, a propoxy group, an isopropoxy group, an n-butoxy group, an isobutoxy group, a tert-butoxy group, a pentyloxy group, an isopentyloxy group, a hexyloxy group and the like.

"C1-C6 alkoxy C1-C6 alkoxy group" as used herein refers to a C1-C6 alkoxy group in which one of the hydrogen atom of the C1-C6 alkoxy group is substituted with a C1-C6 alkoxy group, for example, a methoxymethoxy group, a methoxyethoxy group, a methoxy propoxy group, an ethoxymethoxy group, an ethoxyethoxy group, a propoxy methoxy group and the like.

"halogeno C1-C6 alkyl group" as used herein refers to a C1-C6 alkyl group in which one or more hydrogen atoms are substituted with a halogen atom, for example, a fluoromethyl group, a difluoromethyl group, a trifluoromethyl group, a trichloromethyl group, a fluoroethyl group, 1,1,1-trifluoroethyl group, a mono fluoro-n-propyl group, a perfluoro-n-propyl group, a perfluoro isopropyl group and the like.

"C6-C14 aromatic hydrocarbon group" as used herein refers to a monocyclic or polycyclic aromatic hydrocarbon group having 6 to 14 carbon atoms, for example, a phenyl group, a naphthyl group, an anthracenyl group, a phenanthryl group, a fluorenyl group and the like.

"Unsaturated heterocyclic group" as used herein refers to a monocyclic or polycyclic unsaturated heterocyclic group having at least one hetero atom selected from a nitrogen atom, a sulfur atom and an oxygen atom (preferably 1 to 4, more preferably 1 to 3). The unsaturated heterocyclic group includes a fully unsaturated heterocyclic group (a fully unsaturated heterocyclic group) and a partially unsaturated heterocyclic group (a partially unsaturated heterocyclic group).

A fully unsaturated heterocyclic group includes, for example, a pyrrolyl group, an imidazolyl group, a pyrazolyl group, a triazolyl group, a tetrazolyl, a furanyl (a furyl group), an oxazolyl group, an isoxazolyl group, an oxadiazolyl group, a thiophenyl group (a thienyl group), a thiazolyl group, an isothiazolyl group, a thiadiazolyl group, a pyridinyl group (a pyridyl group), a pyrimidinyl group (pyrimidyl group), a pyrazinyl group (a pyrazyl group), a pyridazinyl group, an indolyl group, an isoindolyl group, an indazolyl group (a benzpyrazol group), a benzimidazolyl group, a benzotriazolyl group, an azaindolyl group, a pyrrolopyridinyl group, an imidazopyridinyl group, a pyrazolopyridinyl group, a triazolopyridinyl group, a pyrrolopyrimidinyl group, an imidazopyrimidinyl group, a pyrazolopyrimidinyl group, a benzofuranyl group, a benzoxazolyl group, a benzothiophenyl group (a benzothienyl group), a benzothiazolyl group, a benzothiadiazolyl group, a benzofuranyl group (a benzofuryl group), a quinolyl group, an isoquinolyl group, a quinazolinyl group, a quinoxalyl group and the like.

A partially unsaturated heterocyclic group includes, for example, a dihydropyranyl group, a dihydro triazolyl group, a dihydrofuranyl group, a dihydrooxadiazolyl group, a dihydroquinolyl group, a dihydroquinazolinyl group, an indolinyl group, a tetrahydroisoquinolyl group, a methylenedioxyphenyl group, an ethylenedioxy phenyl group, a dihydrobenzofuranyl group, a dihydro-benzoxazolyl group, a dihydropyridooxazinyl group and the like.

"Saturated heterocyclic group" as used herein refers to a single or polycyclic fully saturated heterocyclic group having at least one hetero atom selected from a nitrogen atom, a sulfur atom and an oxygen atom (preferably 1 to 4, more preferably 1 to 3), and includes, for example, an azetidinyl group, a pyrrolidinyl group, a piperidinyl group, a piperazinyl group, a hexamethyleneimino group, a morpholino group, a thiomorpholino group, a homopiperazinyl group, a tetrahydrofuranyl group, a tetrahydropyranyl group, a tetrahydrothiophenyl group, a thiazolidinyl group, an oxazolidinyl group and the like.

"Nitrogen-containing saturated heterocyclic group" as used herein refers to a saturated heterocyclic group having one or more nitrogen atoms, which optionally includes a hetero atom other than nitrogen atom, and includes, for example, a morpholino group.

"Nitrogen-containing saturated heterocyclic carbonyl group" as used herein refers to a carbonyl group to which a nitrogen-containing saturated heterocyclic group is bonded, and includes, for example, a morpholinocarbonyl group.

"C1-C14 acyl group", as used herein refers to a carbonyl group to which a hydrogen atom, a C1-C6 alkyl group, a C6-C14 aromatic hydrocarbon group or an unsaturated heterocyclic group is bonded, and includes, for example: a formyl group; a (C1-C6 alkyl) carbonyl group such as an acetyl group, a propanoyl group, a butanoyl group; a (C3-C6 cycloalkyl) carbonyl group such as a cyclopropanoyl group, a cyclobutanoyl group; or a (C6-C13) arylcarbonyl group such as a benzoyl group, a naphthyl carbonyl group, a fluorenylcarbonyl group.

"C1-C14 acylamino group" as used herein refers to an amino group in which one or two hydrogen atoms are substituted with a C1-C14 acyl group, and includes, for example, an acetylamino group, a propanoylamino group, a butanoylamino group, a cyclopropanoyl amino group.

"C2-C7 alkoxycarbonyl group", as used herein refers to a carbonyl group to which a C1-C6 alkoxy group is bonded, and includes, for example, a methoxycarbonyl group, an ethoxycarbonyl group, an n-propoxycarbonyl group, an isopropoxycarbonyl group, an n-butoxycarbonyl group, a tert-butoxycarbonyl group and the like.

"C1-C14 acyloxy group" as used herein refers to, for example, a formyloxy group; a (C1-C6 alkyl)carbonyloxy group such as a methyl carbonyloxy group, an ethyl carbonyloxy group, an n-propyl carbonyloxy group, an isopropylcarbonyloxy group, an n-butylcarbonyloxy group, an iso-butylcarbonyloxy group, a tert-butylcarbonyloxy group, an n-pentylcarbonyloxy group, an iso-pentylcarbonyloxy group, a hexylcarbonyloxy group and the like; a (C3-C6 cycloalkyl)carbonyloxy group such as a cyclopropanoyloxy group, a cyclobutanoyloxy group and the like; a (C6-C13 aryl)carbonyloxy group such as a phenylcarbonyloxy group, naphthylcarbonyloxy group, a fluorenylcarbonyloxy group and the like.

"C7-C13 aralkyloxy group" as used herein refers to an alkylloxy group in which one hydrogen atom is substituted with an aryl group, and includes, for example, a benzyloxy group, a phenethyloxy group, a naphthylmethyloxy group, a fluorenylmethyloxy group and the like.

"Saturated or partially unsaturated hydrocarbon ring" as used herein refers to a monocyclic or polycyclic saturated or partially unsaturated hydrocarbon ring, and includes, for example, a cyclopropane ring, a cyclobutane ring, a cyclopentane ring, a cyclohexane ring, a cycloheptane ring, a cyclooctane ring, a cyclobutene ring, a cyclopentene ring, a cyclohexene ring, a cycloheptene ring, a cyclooctadiene ring and the like.

"Saturated or partially unsaturated heterocyclic ring" as used herein refers to a monocyclic or polycyclic saturated or partially unsaturated heterocyclic a ring having a hetero atom selected from a nitrogen atoms a sulfur atom and an oxygen atom, and includes, for example, an oxirane ring, an azetidine ring, a pyrrolidine ring, an imidazolidine ring, a piperidine ring, a piperazine ring, a morpholine ring, a tetrahydrofuran ring, a tetrahydropyran ring, a dioxane ring, a tetrahydrothiophene ring, a dihydropyran ring, a dihydrofuran ring and the like.

"Spiro heterocyclic group" as used herein refers to a saturated or unsaturated spiro heterocyclic group having a spiro carbon atom and a hetero atom selected from a nitrogen atom, a sulfur atom and an oxygen atom, and includes, for example, a 2-oxa-6-azaspiro[3.4]octanyl group, a 2-oxa-7-azaspiro[3.5]nonanyl group and the like.

"Bridged heterocyclic group" as used herein refers to a bridged heterocyclic group having more than one ring, which have two bridgehead carbons and a hetero atom selected from a nitrogen atom, a sulfur atom and an oxygen atom, and includes, for example, a 3-oxa-8-azabicyclo[3.2.1]octanyl group, an 8-oxa-3-azabicyclo[3.2.1]octanyl group and the like.

In the compounds represented by the formula (I) of the present specification, $X^1$ is an oxygen atom or a sulfur atom. $X^1$ is preferably an oxygen atom.

In the compounds represented by the formula (I) of the present specification, $X^2$ is an oxygen atom or -NH-. $X^2$ is preferably an oxygen atom.

In the compounds represented by the formula (I) of the present specification, $X^3$ is - NH- or an oxygen atom. $X^3$ is preferably -NH-.

In the compounds of the formula (I), $X^4$ is a hydrogen atom or a C1-C6 alkyl group. "C1-C6 alkyl group" represented by $X^4$ is preferably a C1-C3 alkyl group, more preferably a methyl group.

$X^4$ is preferably a hydrogen atom or a methyl group, more preferably a hydrogen atom.

In the compounds of the formula (I), $R^1$ is, $-C(R^{11})(R^{12})-$ or $-C(=CH_2)-$.

In $-C(R^{11})(R^{12})-$, $R^{11}$ and $R^{12}$ are the same or different, and are a hydrogen atom, a halogen atom, a hydroxy

group, or a C1-C6 alkyl group, alternatively taken together with the carbon atoms to which they attach to form a saturated hydrocarbon ring having 3 to 8 carbon atoms.

"Halogen atom" represented by $R^{11}$ and $R^{12}$ is preferably a fluorine atom, a chlorine atom, a bromine atom, more preferably a fluorine atom.

"C1-C6 alkyl group" indicated in $R^{11}$ and $R^{12}$ is preferably a C1-C3 alkyl group, more preferably a methyl group or an ethyl group, more preferably a methyl group.

"Saturated hydrocarbon ring having 3 to 8 carbon atoms", which is formed by combining $R^{11}$ and $R^{12}$ together with the carbon atoms to which they attached, is preferably a monocyclic saturated hydrocarbon ring of 3 to 6 carbon atoms, and more preferably a cyclopropane ring.

Preferably, $R^{11}$ is a halogen atom, a hydroxy group, or a C1-C6 alkyl group, and $R^{12}$ is a hydrogen atom, a halogen atom, a hydroxy group, or a C1-C6 alkyl group, alternatively $R^{11}$ and $R^{12}$ are taken together with the carbon atoms to which they are attached to form a saturated hydrocarbon ring having 3 to 8 carbon atoms. More preferably, $R^{11}$ is a C1-C6 alkyl group, and $R^{12}$ is a hydrogen atom, and more preferably $R^{11}$ is a methyl group, and $R^{12}$ is a hydrogen atom.

$R^1$ is preferably $-C(R^{11}) (R^{12})-$, $R^{11}$ is a halogen atom, a hydroxy group, or a C1-C6 alkyl group, and $R^{12}$ is a hydrogen atom, a halogen atom, hydroxy group, or a C1-C6 alkyl group, alternatively $R^{11}$ and $R^{12}$ are taken together with the carbon atoms to which they are attached to form a saturated hydrocarbon ring having 3 to 8 carbon atoms. More preferably, $- C(R^{11}) (R^{12})-$, and, $R^{11}$ is a C1-C6 alkyl group, $R^{12}$ is a hydrogen atom. Even more preferably, it is $-CH(CH_3)-$.

In the compounds of the formula (I), $R^2$ is a C6-C14 aromatic hydrocarbon group or a 9-10 membered fully unsaturated heterocyclic group.

"C6-C14 aromatic hydrocarbon group" represented by $R^2$ is preferably a C6-C10 aromatic hydrocarbon group, more preferably a phenyl group or a naphthyl group, even more preferably a phenyl group.

Furthermore, "fully unsaturated heterocyclic group having 9-10 membered" represented by $R^2$ is preferably a bicyclic 9-10 membered fully unsaturated heterocyclic group having 1-3 heteroatoms selected from a nitrogen atom, a sulfur atom and an oxygen atom, more preferably a bicyclic 9-10 membered fully unsaturated heterocyclic group having 1-2 hetero atoms selected from a nitrogen atom and a sulfur atom, even more preferably a benzothiophenyl group, a benzothiazolyl group, a quinolyl group.

In the compounds of the formula (I), $R^2$ may be unsubstituted or may have a substituent. Further, when $R^2$ has two substituents on the carbon atoms adjacent each other on the aromatic hydrocarbon ring, $R^2$ may form a 4 to 8-membered saturated or partially unsaturated hydrocarbon ring or a heterocyclic ring having substituent(s), wherein the substitutes are fused to form a ring together with the carbon atom to which they are attached.

When $R^2$ has a substituent, the substituted position of the substituent is not particularly limited, but, for example, preferably 2, 3, 5, or 6-position when $R^2$ is a phenyl group. Furthermore, the number of substituent is not particularly limited, but preferably zero, *i.e.* it is unsubstituted or 1-4, and more preferably 1-4 or 1-3. When the number of substituents is two or more, the types of the substituent may be the same or different.

In the compounds of formula (I), preferably, $R^2$ may be substituted with the "substituent", more preferably, $R^2$ may be substituted with $R^{21}$. Also, preferably, when $R^2$ has two substituents on the carbon atoms adjacent each other on the aromatic hydrocarbon ring, the substituents may be fused together with the carbon atom to which they are attached to form a saturated or partially unsaturated 4-8 membered hydrocarbon ring or heterocyclic ring optionally substituted with Rz.

$R^{21}$, which can be substituted at $R^2$, is a halogen atom, an aminocarbonyl group, a cyano group, a C1-C6 alkyl group which may be substituted with Rx, a C3-C6 cycloalkyl group which may be substituted with Rx, a C2-C6 alkynyl group which may be substituted with Rx, a C6-C14 aromatic hydrocarbon group which may be substituted with Ry, or an unsaturated 5-10 membered heterocyclic ring which may be substituted with Rz.

The position at which $R^{21}$ is a substituted is not particularly limited, but, for example, preferably 2, 3, 5, or 6-position when $R^2$ is a phenyl group. Furthermore, the number of the substituent $R^{21}$ is not particularly limited, but preferably zero, *i.e.* it is unsubstituted, or 1-4, more preferably 1-4 or 1-3. When the number of the substituent $R^{21}$ is two or more, the types of the substituent may be the same or different.

"Halogen atom" indicated in $R^{21}$ is preferably a fluorine atom, a chlorine atom, or a bromine atom.

"C1-C6 alkyl group" in the "C1-C6 alkyl group which may be substituted with Rx" indicated in $R^{21}$ is preferably a C1-C3 alkyl group, more preferably a methyl group or an ethyl group.

The substituent Rx in the "C1-C6 alkyl group which may be substituted with Rx" indicated in $R^{21}$ is a halogen atom or a C6-C14 aromatic hydrocarbon group. The substituent Rx is preferably a halogen atom, more preferably a fluorine atom. The number of Rx which is substituted at C1-C6 alkyl group is not particularly limited, but preferably zero, *i.e.,* unsubstituted, or 1-3. When the number of substituent Rx is 2 or more, the types of the substituent may be the same or different.

"C3-C6 cycloalkyl group" in the "C3-C6 cycloalkyl group which may be substituted with Rx" indicated in $R^{21}$ is

preferably a cyclopropyl group.

Rx in the "C3-C6 cycloalkyl group which may be substituted with Rx" indicated in $R^{21}$ is a halogen atom as mentioned above, or a C6-C14 aromatic hydrocarbon group, preferably a halogen atom, more preferably a fluorine atom. The number of Rx substituted at the C3-C6 cycloalkyl group is not particularly limited, but preferably zero, *i.e.* it is unsubstituted, or 1, more preferably 0. When the number of substituents Rx is 2 or more, the types of the substituent may be the same or different.

"C2-C6 alkynyl group" in the "C2-C6 alkynyl group which may be substituted with Rx" indicated in $R^{21}$ is preferably a C2-C4 alkynyl group, more preferably an ethynyl group.

The substituent Rx in the "C2-C6 alkynyl group may be substituted with Rx" indicated in $R^{21}$ is a halogen atom as mentioned above, or a C6-C14 aromatic hydrocarbon group, preferably a C6-C14 aromatic hydrocarbon group, more preferably a C6-C10 aromatic hydrocarbon group, more preferably a phenyl group.

The number of Rx substituted at the C2-C6 alkynyl group is not particularly limited, but preferably zero, *i.e.* it is unsubstituted, or 1, more preferably 1. When the number of the substituents Rx is 2 or more, the types of the substituent may be the same or different.

"C6-C14 aromatic hydrocarbon group" in the "C6-C14 aromatic hydrocarbon group which may be substituted with Ry" indicated in $R^{21}$ is preferably a C6-C10 aromatic hydrocarbon group, more preferably a phenyl group.

The substituent Ry in the "C6-C14 aromatic hydrocarbon group which may be substituted with Ry" indicated in $R^{21}$ is a halogen atom or a C1-C6 alkoxy group.

A halogen atom indicated in Ry is preferably a fluorine atom or chlorine atom. Also, a C1-C6 alkoxy group indicated in Ry is preferably a C1-C3 alkoxy group, more preferably a methoxy group. The substituent Ry in the "C6-C14 aromatic hydrocarbon group which may be substituted with Ry" indicated in $R^{21}$ is preferably a fluorine atom, a chlorine atom, or a C1-C3 alkoxy group, more preferably a fluorine atom, a chlorine atom or a methoxy group. The number of Ry substituted in the C6-C14 aromatic hydrocarbon group is not particularly limited, but preferably zero, *i.e.* unsubstituted, or it is 1 or 2. When the number of the substituents Ry is 2 or more, the types of substituent may be the same or different.

"5 to 10-membered unsaturated heterocyclic group" in the "5 to 10-membered unsaturated heterocyclic group optionally substituted with Rz" indicated in $R^{21}$ is preferably a fully or partially unsaturated monocyclic or bicyclic 5-10 membered heterocyclic group having 1-3 hetero atoms selected from a nitrogen atom, a sulfur atom and an oxygen atom, more preferably a monocyclic or bicyclic 5 to 10-membered unsaturated heterocyclic group having 1 to 3 hetero atoms selected from a nitrogen atom, a sulfur atom or an oxygen atom, more preferably a monocyclic 5-6 membered unsaturated heterocyclic group having 1-3 nitrogen atoms or an oxygen atom. Preferably, it is a pyrrolyl group, an imidazolyl group, a pyrazolyl group, a pyridyl group, a pyrimidyl group, an oxazolyl group, a dihydropyridooxazinyl group, more preferably, a pyrazolyl group, a pyridyl group, a pyrimidyl group, an oxazolyl group, a dihydropyridooxazinyl group, more preferably a pyrazolyl group.

The substituent Rz in the "5 to 10-membered unsaturated heterocyclic group optionally substituted with Rz" indicated in $R^{21}$ is a halogen atom, a C1-C6 alkyl group, a halogeno C1-C6 alkyl group, a C3-C6 cycloalkyl group, a C1-C6 alkoxy group, a C6-C14 aromatic hydrocarbon group, a nitrogen-containing saturated heterocyclic group, or a nitrogen-containing saturated heterocyclic carbonyl group.

"Halogen atom" indicated in Rz is preferably a fluorine atom or a chlorine atom.

"C1-C6 alkyl group" indicated in Rz is preferably a C1-C3 alkyl group, more preferably a methyl group, or an ethyl group.

"Halogeno C1-C6 alkyl group" indicated in Rz is preferably a halogeno C1-C3 alkyl group, more preferably a difluoromethyl group or a trifluoromethyl group. "C3-C6 cycloalkyl group" indicated in Rz is preferably a cyclopropyl group or a cyclobutyl group.

"C1-C6 alkoxy group" indicated in Rz is preferably a C1-C3 alkoxy group, more preferably a methoxy group.

"C6-C14 aromatic hydrocarbon group" indicated in Rz is preferably a phenyl group. "Nitrogen-containing saturated heterocyclic group" represented by Rz is preferably a morpholino group or a piperidinyl group.

"Nitrogen-containing saturated heterocyclic carbonyl group" indicated in Rz is preferably a morpholinocarbonyl group.

The substituent Rz in the "5 to 10-membered unsaturated heterocyclic group optionally substituted with Rz" is preferably a halogen atom, a C1-C6 alkyl group, a halogeno C1-C6 alkyl group, a C3-C6 cycloalkyl group, a C1-C6 alkoxy group, a phenyl group, a morpholino group, a piperidinyl group, or a morpholinocarbonyl group, more preferably a C1-C6 alkyl group, more preferably a methyl group. The number of Rz which is substituted at the 5 to 10-membered unsaturated heterocyclic group is not particularly limited, but preferably zero, *i.e.* unsubstituted, or preferably 1 or 2. When the number of the substituent Rz is 2 or more, the type of the substituent may be the same or different.

$R^{21}$, which can be substituted at $R^2$, is preferably, a halogen atom, an aminocarbonyl group, a cyano group, a C1-C6 alkyl group (optionally substituted with a halogen atom), a C3-C6 cycloalkyl group, a C2-C6 alkynyl group (optionally substituted with a C6-C14 aromatic hydrocarbon group) a C6-C14 aromatic hydrocarbon group (optionally substituted with a group selected from a halogen atom and a C1-C6 alkoxy group), or a monocyclic or bicyclic 5 to 10-membered unsaturated heterocyclic group having 1 to 3 hetero atoms selected from a nitrogen atom, a sulfur atom and an oxygen atom (optionally substituted with a group selected from a halogen atom, a C1-C6 alkyl group, a halogeno C1-C6 alkyl group, a C3-C6 cycloalkyl group, a C1-C6 alkoxy group, a C6-C14 aromatic hydrocarbon group, a nitrogen-containing saturated heterocyclic group, and a nitrogen-containing saturated heterocyclic carbonyl group).

More preferably, a halogen atom, a cyano group, a C1-C6 alkyl group (optionally substituted with a halogen atom), a C3-C6 cycloalkyl group, a phenyl group (optionally substituted with a group selected from the group consisting of a halogen atom or a C1-C6 alkoxy group), or monocyclic or bicyclic 5 to 10-membered unsaturated heterocyclic group having 1 to 3 hetero atom(s) selected from a nitrogen atom, a sulfur atom and an oxygen atom (optionally substituted with a group selected from a halogen atom, a C1-C6 alkyl group, a halogeno C1-C6 alkyl group, a C3-C6 cycloalkyl group, a C1-C6 alkoxy group, a morpholino group, a piperidinyl group and a morpholinocarbonyl group).

More preferably, a halogen atom, a C1-C6 alkyl group, or a monocyclic 5 or 6-membered unsaturated heterocyclic group having 1 to 3 of a nitrogen atom(s) (optionally substituted with a C1-C6 alkyl group).

More preferably, a halogen atom or a C1-C6 alkyl group.

In the compounds of the formula (I), when the number of the substituents at $R^2$ is 2 or more, and there are two substituents at the carbons which are adjacent each other on the aromatic hydrocarbon ring, "4 to 8-membered saturated or partially unsaturated hydrocarbon ring or heterocyclic ring which may have substituent(s)", which is formed by combining the substituents and the carbon atom to which they are attached, is a ring, for example a ring fused to a benzene ring." Saturated or partially unsaturated 4 to 8-membered hydrocarbon ring or heterocyclic ring" in the "4-8 membered saturated or partially unsaturated hydrocarbon ring or heterocyclic ring, which may have substituent(s)" is preferably a monocyclic saturated or partially unsaturated hydrocarbon ring, or a monocyclic 4 to 8-membered saturated or partially unsaturated heterocyclic ring having 1 to 3 hetero atoms selected from a nitrogen atom, a sulfur atom and an oxygen atoms, more preferably, a saturated or partially unsaturated hydrocarbon ring having 4 to 8 carbon atoms, more preferably, a monocyclic saturated or partially unsaturated hydrocarbon ring having 4 to 6 carbon atoms, or a monocyclic 4-6 membered saturated or partially unsaturated heterocyclic ring having 1 to 3 heteroatoms selected from nitrogen atom, a sulfur atom, and an oxygen atom, and even more preferably, a monocyclic saturated or partially unsaturated hydrocarbon ring having 5 or 6 carbon atoms, more preferably a saturated hydrocarbon ring having 5 carbon atoms.

The substituent Rz in the "4 to 8-membered saturated or partially unsaturated hydrocarbon ring or heterocyclic ring optionally substituted with Rz" is, as mentioned above, a halogen atom, a C1-C6 alkyl group, a halogeno C1-C6 alkyl group, a C3-C6 cycloalkyl group, a C1-C6 alkoxy group, a C6-C14 aromatic hydrocarbon group, a nitrogen-containing saturated heterocyclic group, or a nitrogen-containing saturated heterocyclic carbonyl group, preferably a C1-C6 alkyl group, and more preferably, a C1-C3 alkyl group, and even more preferably, a methyl group. The number of Rz which substitutes at a saturated or partially unsaturated hydrocarbon ring or heterocyclic ring is not particularly limited, but preferably zero, *i.e.,* unsubstituted, or it is one, more preferably it is zero, *i.e.,* unsubstituted. When the number of the substituents Rz is 2 or more, the type of substituent may be the same or different.

"Saturated or partially unsaturated 4-8 membered hydrocarbon ring or heterocyclic ring optionally substituted with Rz" is preferably a monocyclic saturated or partially unsaturated hydrocarbon ring having 4 to 8 carbon atoms, which is optionally substituted with Rz, or a monocyclic 4-8 membered saturated or partially unsaturated heterocyclic ring having 1 to 3 hetero atoms selected from a nitrogen atom, a sulfur atom, and an oxygen atom, more preferably a monocyclic saturated or partially unsaturated hydrocarbon ring having 4 to 8 carbon atoms (which may be substituted with a C1-C6 alkyl group) or a monocyclic saturated or partially unsaturated 4-8 membered heterocyclic ring having 1-3 heteroatoms selected from a nitrogen atom, a sulfur atom and an oxygen atom (optionally substituted with a C1-C6 alkyl group), more preferably a saturated or partially unsaturated monocyclic hydrocarbon ring having 4 to 8 carbon atoms (optionally substituted with a C1-C6 alkyl group), more preferably a monocyclic saturated or partially unsaturated hydrocarbon ring having 5 or 6 carbon atoms (optionally substituted with a C1-C6 alkyl group).

In the compounds represented by formula (I), a fused ring, which is formed when the compound has two substituents on the carbon atoms adjacent each other on the aromatic hydrocarbon ring of $R^2$, is for example, a dihydroindene ring, a tetrahydronaphthalene ring, a dihydrobenzofuran ring.

In the compounds represented by formula (I), $R^2$ is preferably a C6-C14 aromatic hydrocarbon group or a bicyclic fully unsaturated 9-10 membered heterocyclic group having 1 to 3 heteroatoms selected from a nitrogen atom, a sulfur atom and an oxygen atoms, and $R^2$ may be substituted with $R^{21}$, and when $R^2$ has two substituents on the carbon atom adjacent each other on the aromatic hydrocarbon ring, $R^2$ may be a monocyclic saturated or partially unsaturated hydrocarbon ring having 4 to 8 carbon atoms (optionally substituted with a C1-C6 alkyl group) wherein the substituents are fused together with the carbon atom to which each of the substituent is bonded, or a monocyclic 4-8 membered saturated or partially unsaturated heterocyclic ring having 1 to 3 hetero atoms selected from a nitrogen atom, a sulfur atom and an oxygen atoms (optionally substituted with a C1-C6 alkyl group); and
$R^{21}$ is a halogen atom, an aminocarbonyl group, a cyano group, a C1-C6 alkyl group (optionally substituted with a halogen atom), a C3-C6 cycloalkyl group, a C2-C6 alkynyl group (optionally substituted with a C6-C14 aromatic hydrocarbon group), a C6-C14 aromatic hydrocarbon group (optionally substituted with a group selected from the group consisting of a halogen atom and a C1-C6 alkoxy group), or a monocyclic or bicyclic 5-10 membered unsaturated heterocyclic ring having 1 to 3 hetero atoms selected from a nitrogen atom, a sulfur atom, and an oxygen atom (optionally substituted with a group selected from the group consisting of a halogen atom, a C1-C6 alkyl group, a halogeno C1-C6 alkyl group, a C3-C6 cycloalkyl group, a C1-C6 alkoxy group, a C6-C14 aromatic hydrocarbon group, a nitrogen-containing saturated heterocyclic group, and a nitrogen-containing saturated heterocyclic carbonyl group).

In the compounds represented by formula (I), $R^2$ is more preferably a C6-C14 aromatic hydrocarbon group, wherein $R^2$ may be substituted with $R^{21}$, and when $R^2$ has two substituents on the carbon atom adjacent each other on the aromatic hydrocarbon ring, $R^2$ may form a monocyclic saturated or partially unsaturated hydrocarbon ring having 4 to 8 carbon atoms (optionally substituted with a C1-C6 alkyl group) wherein the substituents are fused together with the carbon atom to which each of the substituent is bonded;

$R^{21}$ is a halogen atom, a cyano group, a C1-C6 alkyl group (optionally substituted with a halogen atom), a C3-C6 cycloalkyl group, a phenyl group (optionally substituted with a group selected from the group consisting of a halogen atom a C1-C6 alkoxy group), or a monocyclic or bicyclic 5-10 membered unsaturated heterocyclic ring having 1 to 3 hetero atoms selected from a nitrogen atom, a sulfur atom and an oxygen atom (optionally substituted with a group selected from the group consisting of a halogen atom, a C1-C6 alkyl group, a halogeno C1-C6 alkyl group, a C3-C6 cycloalkyl group, a C1-C6 alkoxy group, a morpholino group, a piperidinyl group and a morpholinocarbonyl group).

Also, in the compounds represented by formula (I), $R^2$ is more preferably a C6-C10 aromatic hydrocarbon group, wherein $R^2$ may be substituted with $R^{21}$, and when $R^2$ has two substituents on the carbon atom adjacent each other on the aromatic hydrocarbon ring, $R^2$ may form a monocyclic saturated or partially unsaturated hydrocarbon ring having 5 or 6 carbon atoms (optionally substituted with a C1-C6 alkyl group) wherein the substituents are fused together with the carbon atom to which each of the substituents is bonded; and

$R^{21}$ is a halogen atom, a C1-C6 alkyl group, or a monocyclic 5 or 6-membered unsaturated heterocyclic ring having 1-3 nitrogen atom(s) (optionally substituted with a C1-C6 alkyl group).

Also, in the compounds represented by formula (I), $R^2$ is especially preferably a phenyl group or a naphthyl group (optionally substituted with a group selected from the group consisting of a halogen atom and a C1-C6 alkyl group); an indanyl group (2,3-dihydro-1H-indenyl group); or a tetrahydronaphthyl group.

In the compounds represented by formula (I), $R^3$ is a C6-C14 aromatic hydrocarbon group or a 5 to 10-membered fully unsaturated heterocyclic group.

"C6-C14 aromatic hydrocarbon group" indicated in $R^3$ is preferably a C6-C10 aromatic hydrocarbon group, more preferably a phenyl group, or a naphthyl group, particularly preferably a phenyl group.

"5 to 10-membered fully unsaturated heterocyclic group" indicated in $R^3$ is a monocyclic or bicyclic 5 to 10-membered fully unsaturated heterocyclic group having 1-3 hetero atoms selected from a nitrogen atom, a sulfur atom and an oxygen atom, more preferably, a monocyclic or bicyclic 5 to 7-membered fully unsaturated heterocyclic group having 1-3 heteroatoms selected from a nitrogen atom, a sulfur atom and an oxygen atom, particularly preferably a monocyclic 5 to 6-membered fully unsaturated heterocyclic ring having 1-3 heteroatoms selected from a nitrogen atom, a sulfur atom and an oxygen atoms. Preferably, an imidazolyl group, a pyridyl group, a thiophenyl group, an indolyl group, an indazolyl group, a benzopyranyl group, a benzotriazolyl group, a benzothiadiazolyl group, an isoxazolyl group, a quinolyl group, more preferably an imidazolyl group, a pyridyl group, a thiophenyl group, an indolyl group, an indazolyl group, a benzopyranyl group, a benzotriazolyl group, a benzothiadiazolyl group, a quinolyl group, more preferably a pyridyl group, a thiophenyl group, an indolyl group, an indazolyl group, a benzopyranyl group, a benzotriazolyl group, a quinolyl group, more preferably a pyridyl group.

In the compounds represented by formula (I), $R^3$ may be unsubstituted or may have a substituent. Also, when $R^3$ has two substituents on the carbon atoms adjacent each other on the aromatic hydrocarbon ring, $R^3$ may form a 4 to 8-membered saturated or partially unsaturated hydrocarbon ring or heterocyclic ring, which may be substituted, wherein the substituents are fused together with the carbon atom to which each of the substituents is bonded; and

When $R^3$ has a substituent, the position of the substituent is not particularly limited. Although the number of the substituent is not limited, it is particularly preferably 0, i.e. unsubstituted. Alternatively, the number of the substituent is 1 to 4, more preferably 1 to 3. When the number of substituent is two or more, the types of the substituent may be the same or different.

In the compounds represented by formula (I), preferably $R^3$ may be substituted with the "substituent", more preferably $R^3$ may be substituted with $R^{31}$. Also, preferably, when $R^3$ has two substituents on the carbon atoms adjacent each other on the aromatic hydrocarbon ring, $R^3$ may form a 4 to 8-membered saturated or partially unsaturated hydrocarbon ring or heterocyclic ring, which may be substituted with Rc, wherein the substituents are fused together with the carbon atom to which each of the substituents is bonded.

$R^{31}$, which can be substituted at $R^3$, is a halogen atom, a cyano group, a nitro group, a carboxyl group, a thioamide group, a C1-C6 alkyl group which may be substituted with Ra, an amino group which may be substituted with Ra, a C3-C6 cycloalkyl group which may be substituted with Rb, a C1-C6 alkoxy group which may be substituted with Rb, a C2-C7 alkoxycarbonyl group, a C1-C14 acyl group which may be substituted with Rb, a C6-C14 aromatic hydrocarbon ring which may be substituted with Rb, an 5 to 10-membered unsaturated heterocyclic ring which may be substituted with Rc, an aminocarbonyl group which may be substituted with Rd and Re, or $-S(=O)_2Rf$.

Although the number of the substituent is not limited, it is particularly preferably 0, i.e. unsubstituted. Alternatively, the number of the substituent is 1 to 4, more preferably 1 to 3. When the number of substituent is two or more, the types of the substituent may be the same or different.

"Halogen atom" indicated in $R^{31}$ is preferably a fluorine atom, a chlorine atom, or a bromine atom, more preferably a chlorine atom, or a bromine atom.

"C1-C6 alkyl group" of "a C1-C6 alkyl group which may be substituted with Ra" indicated in $R^{31}$ is preferably a C1-C3 alkyl group, more preferably a methyl group.

The substituent Ra of "a C1-C6 alkyl group which may be substituted with Ra" indicated in $R^{31}$ is a halogen atom, a hydroxy group, a C1-C14 acyl group, a C1-C14 acyloxy group, a C2-C6 alkynyl group, or a C1-C6 alkoxy C1-C6 alkoxy group.

"Halogen atom" indicated in Ra is preferably a fluorine atom.

"C1-C14 acyl group" indicated in Ra is preferably an acetyl group.

"C1-C14 acyloxy group" indicated in Ra is preferably an acetyloxy group.

"C2-C6 alkynyl group" indicated in Ra is preferably an ethynyl group, 1-propynyl group.

"C1-C6 alkoxy C1-C6 alkoxy group" indicated in Ra is preferably a methoxymethoxy group.

The substituent Ra of "a C1-C6 alkyl group may be substituted with Ra" indicated in $R^{31}$ is preferably a halogen atom, a hydroxy group, a C1-C6 acyloxy group, a C2-C6 alkynyl group, or a C1-C6 alkoxy C1-C6 alkoxy group, more preferably a halogen atom, or a hydroxy group. Although the number of Ra which is substituted at the C1-C6 alkyl is not particularly limited, preferably zero, i.e. unsubstituted, or one or more. When the number of the substituents Ra is 2 or more, the types of the substituent may be the same or different.

Ra of "an amino group optionally substituted with Ra" indicated in $R^{31}$ is a halogen atom, a hydroxy group, a C1-C14 acyl group, a C1-C14 acyloxy group, a C2-C6 alkynyl group, or a C1-C6 alkoxy C1-C6 alkoxy group, preferably a C1-C14 acyl group, more preferably an acetyl group.

The number of Ra substituted at the amino group is not particularly limited, preferably zero, i.e. unsubstituted, or is 1, more preferably 0.

"C3-C6 cycloalkyl group" in the "C3-C6 cycloalkyl group optionally substituted with Rb" indicated in $R^{31}$ is preferably a cyclopropyl group.

Rb in the "C3-C6 cycloalkyl group optionally substituted with Rb" indicated in $R^{31}$ is a halogen atom, an amino group, or a C1-C6 alkoxy group.

"Halogen atom" indicated in Rb is preferably a fluorine atom.

"C1-C6 alkoxy group" indicated in Rb is preferably a C1-C3 alkoxy group, more preferably a methoxy group.

Rb in the "C3-C6 cycloalkyl group optionally substituted with Rb" indicated in $R^{31}$ is preferably an amino group. The number of Rb substituting at the C3-C6 cycloalkyl group is not particularly limited, preferably zero, i.e. unsubstituted, or is 1, more preferably 0. When the number of substituents Rb is two or more, the types of the substituent may be the same or different.

"C1-C6 alkoxy group" in the "C1-C6 alkoxy group optionally substituted with Rb" indicated in $R^{31}$ is preferably a C1-C3 alkoxy group, more preferably a methoxy group.

Rb in the "C1-C6 alkoxy group optionally substituted with Rb" indicated in $R^{31}$ is, as mentioned above, a halogen atom, an amino group, or a C1-C6 alkoxy group, preferably a halogen atom, more preferably a fluorine atom. Although number of Rb substituent to a C1-C6 alkoxy group is not limited, it is zero, i.e. unsubstituted, or one or two. When the number of substituent Rb is two or more, the types of the substituent may be the same or different.

"C2-C7 alkoxycarbonyl group" indicated in $R^{31}$ is preferably a C2-C4 alkoxycarbonyl group, more preferably a methoxycarbonyl group.

"C1-C14 acyl group" in the "C1-C14 acyl group optionally substituted with Rb" indicated in $R^{31}$ is preferably an acetyl group.

Rb in the "C1-C14 acyl group optionally substituted with Rb" indicated in $R^{31}$ is, as mentioned above, a halogen atom, an amino group, or a C1-C6 alkoxy group, preferably a halogen atom, more preferably a fluorine atom. Although number of Rb substituent at a C1-C14 acyl group is not limited, it may be zero, i.e. unsubstituted, or one to three. When the number of substituents Rb is two or more, the types of the substituent may be the same or different.

"Thioamide group" indicated in $R^{31}$ is preferably $-C(=S)-NH_2$.

"C6-C14 aromatic hydrocarbon group" in the "C6-C14 aromatic hydrocarbon group optionally substituted with Rb" indicated in $R^{31}$ is preferably a C6-C10 aromatic hydrocarbon group, and more preferably a phenyl group.

The substituent Rb in the "C6-C14 aromatic hydrocarbon group optionally substituted with Rb" indicated in $R^{31}$ is, as mentioned above, a halogen atom, an amino group, or a C1-C6 alkoxy group, and preferably a halogen atom or a C1-C3 alkoxy group, and more preferably a halogen atom, and more preferably a fluorine atom. Although the number of Rb substituting at a C6-C14 aromatic hydrocarbon group is not particularly limited, it is preferably zero, i.e. unsubstituted,

or it is one. When the number of the substituents Rb is 2 or more, the type of groups may be the same or different.

"5 to 10-membered unsaturated heterocyclic group" in the "5 to 10-membered unsaturated heterocyclic group optionally substituted with Rc" indicated in $R^{31}$ is preferably a monocyclic or bicyclic 5-10 membered fully or partially unsaturated heterocyclic group having 1 to 4 hetero atoms selected from a nitrogen atom, a sulfur atom and an oxygen atom, more preferably a monocyclic 5 to 6-membered unsaturated heterocyclic group having 1 to 4 hetero atoms selected from a nitrogen atom, a sulfur atom and an oxygen atom. Preferably it is a pyrrolyl group, an imidazolyl group, a pyrazolyl group, a tetrazolyl group, an isoxazolyl group, an oxadiazolyl group, a dihydro oxadiazolyl group, preferably a pyrazolyl group, a 1,3,4-oxadiazolyl group, a 2,3-dihydro-1,3,4-oxazolyl group.

The substituent Rc in the "5-10 membered unsaturated heterocyclic group optionally substituted with one or more of Rc" indicated in $R^{31}$ is a halogen atom, a hydroxy group, an amino group, an oxo group, a C1-C6 alkyl group optionally substituted with a hydroxy group, a halogeno C1-C6 alkyl group, a C1-C14 acyl, or a C1-C14 acylamino group, a C1-C14 acyloxy group, or a C7-C13 aralkyloxy group.

"Halogen atom" indicated in Rc is preferably a fluorine atom.

"C1-C6 alkyl groups optionally substituted with a hydroxy group" indicated in Rc is preferably a C1-C3 alkyl group optionally substituted with a hydroxy group, and more preferably a methyl group or a hydroxyethyl group.

"Halogeno C1-C6 alkyl group" represented by Rc is preferably a halogeno C1-C3 alkyl group, more preferably a trifluoromethyl group, a difluoroethyl group.

"C1-C14 acyl group" indicated in Rc is preferably an acetyl group or a cyclopropanoyl group.

"C1-C14 acylamino group" indicated in Rc is preferably an acetylamino group.

"C1-C14 acyloxy group" indicated in Rc is preferably an acetyloxy group.

"C7-C13 aralkyloxy group" indicated in Rc is preferably a benzyloxy group.

Rc in the "5 to 10-membered unsaturated heterocyclic group optionally substituted with Rc" indicated in $R^{31}$ is preferably a halogen atom, a C1-C6 alkyl group, or an oxo group, more preferably a C1-C6 alkyl group or an oxo group, more preferably a C1-C6 alkyl group. Although the number of Rc substituting at 5 to 10-membered unsaturated heterocyclic group is not particularly limited, it is preferably zero, i.e. unsubstituted, or preferably it is one or more than 2, more preferably it is zero. When the number of the substituents Rc is 2 or more, the type of groups may be the same or different.

"An amino carbonyl group optionally substituted with Rd and Re" indicated in $R^{31}$ is specifically represented by the following group (II).

[Formula 9]

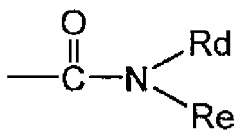

(II)

Rd and Re are the same or different and represent: a hydrogen atom; a hydroxy group; a C7-C13 aralkyloxy group; or C1-C6 alkyl group optionally substituted with hydroxyl groups; alternatively taken together with a nitrogen atom which is adjacent to Rd and Re to form a saturated or unsaturated 4 to 10-membered heterocyclic ring group optionally substituted with an amino group, a spiro heterocyclic ring group, or a bridged heterocyclic ring group.

"C7-C13 aralkyloxy group" indicated in Rd or Re is preferably a benzyloxy group.

"C1-C6 alkyl group optionally substituted with hydroxy groups" indicated in Rd or Re is preferably a C1-C3 alkyl group optionally substituted with a hydroxy group, more preferably a methyl group, or a hydroxyethyl group.

"A saturated heterocyclic group" in the "4 to 10-membered saturated heterocyclic group optionally substituted with an amino group" in Rd or Re is preferably a monocyclic or bicyclic 4 to 10-membered saturated heterocyclic group having 1 to 3 heteroatoms selected from a nitrogen atom, a sulfur atom, and an oxygen atom, preferably a 5 to 6-membered monocyclic saturated heterocyclic group having 1 to 3 heteroatoms selected from a nitrogen atom, a sulfur atom, and an oxygen atom, more preferably an azetidinyl group, a pyrrolidinyl group, a piperidino group, a piperazinyl group, a morpholino group.

"An unsaturated heterocyclic group" in the "4 to 10-membered saturated or unsaturated heterocyclic group optionally substituted with an amino group", which is formed together with Rd or Re and the adjacent nitrogen atoms, is preferably a monocyclic or bicyclic or 5 to 10-membered unsaturated heterocyclic group having 1 to 3 hetero atoms selected from a nitrogen atom, a sulfur atom, an oxygen atom, more preferably a monocyclic 5 to 6-membered unsaturated heterocyclic group having 1 to 3 hetero atoms selected from a nitrogen atom, a sulfur atom, an oxygen atom, particularly preferably a pyrrolyl group.

"Spiroheterocyclic group" formed together with Rd or Re and the adjacent nitrogen atom is preferably a monosupiro heterocyclic group, more preferably an oxoazaspirononanylcarbamoyl group, or an azasupirooctanylcarbamoyl group.

"Bridged heterocyclic group" formed together with Rd or Re and the adjacent nitrogen atom indicated is preferably a bicyclic bridged heterocyclic group, more preferably an oxoazabicyclooctanylcarbamoyl group.

The substituents Rd and Re in the "aminocarbonyl group optionally substituted with Rd and Re" indicated in $R^{31}$ are preferably the same or different, and present a hydroxy group or a C1-C6 alkyl group, alternatively taken together with the adjacent nitrogen atom to form a monocyclic 5 to 6-membered saturated heterocyclic group, which may be substituted with an amino group, having 1 to 3 heteroatoms selected from a nitrogen atom, a sulfur atom and an oxygen atom, a monosupiro heterocyclic group or a bicyclic bridged heterocyclic group.

"An amino carbonyl group optionally substituted with Rd and Re" indicated in $R^{31}$ is preferably a $-CONH_2$ group, (a mono or di-C1-C6 alkyl)aminocarbonyl group, a hydroxyamino group, a (C7-C13 aralkyl)oxyaminocarbonyl group, or a cyclicaminocarbonyl group, more preferably a $-CONH_2$ group, (a mono or di-C1-C3 alkyl)aminocarbonyl group, a hydroxyaminocarbonyl group, a benzyloxycarbonylgroup, a pyrrolidin-1-ylcarbonyl group, a piperidin-1-ylcarbonyl group, a piperazin-1-ylcarbonyl group, a morpholin-4-ylcarbonyl group, an azetidin-1-ylcarbonyl group, an oxo azabicyclooctanylcarbonyl group, an oxo azaspiro nonanylcarbonyl group, an azaspirooctanylcarbonyl group, more preferably a $-CONH_2$ group, a dimethylaminocarbonyl group, or a pyrrolidin-1-ylcarbonyl group.

Rf of "$-S(=O)_2Rf$" indicated in $R^{31}$ is an amino group, a C1-C6 alkyl group, or a 4 to 10-membered saturated heterocyclic group.

C1-C6 alkyl group indicated in Rf is preferably a C1-C3 alkyl group, more preferably a methyl group.

A 4 to 10-membered saturated heterocyclic group indicated in Rf is preferably a monocyclic or bicyclic 4 to 10-membered saturated heterocyclic group having 1 to 3 heteroatoms selected from a nitrogen atom, a sulfur atom and an oxygen atom, more preferably a monocyclic 5 to 6-membered saturated heterocyclic group having 1 to 3 heteroatoms selected from a nitrogen atom, a sulfur atom, and an oxygen atom, more preferably a pyrrolidinyl group, a piperidino group, or a piperazinyl group.

"$-S(=O)_2Rf$" indicated in $R^{31}$ is preferably an aminosulfonyl group, a methylsulfonyl group, or a piperidinosulfonyl group.

$R^{31}$ which may be substituted with $R^3$ is preferably a halogen atom, a cyano group, a nitro group, a carboxyl group, a thioamide group, a C1-C6 alkyl group (which may be substituted with a group selected from the group consisting of a halogen atom, a hydroxy group, a C1-C14 acyl group, a C1-C14 acyloxy group, a C2-C6 alkynyl and a C1-C6 alkoxy C1-C6 alkoxy group), an amino group (which may be substituted with a C1-C14 acyl group), a C3-C6 cycloalkyl group (which may be substituted with an amino group), a C1-C6 alkoxy group (which may be substituted with halogen atoms), a C2-C7 alkoxycarbonyl group, a C1-C14 acyl group (which may be substituted with halogen atoms), a C6-C14 aromatic hydrocarbon group (which may be substituted with a group selected from the group consisting of a halogen atom, an amino group and a C1-C6 alkoxy group), monocyclic or bicyclic 5 to 10 membered unsaturated heterocyclic ring having 1 to 4 hetero atoms selected from a nitrogen atom, a sulfur atom and an oxygen atom (which may be substituted with a group consisting of a halogen atom, an oxo group, and a C1-C6 alkyl group), an aminocarbonyl group optionally substituted with Rd and Re (wherein, Rd and Re are the same or different, and present a hydrogen atom, a hydroxy group, a C7-C13 aralkyloxy group, or a C1-C6 alkyl group which may be substituted with a hydroxyl group, alternatively they are taken together with the adjacent nitrogen atom to form a monocyclic or bicyclic 4-10 membered saturated or unsaturated heterocyclic group having 1 to 3 hetero atoms selected from a nitrogen atom, a sulfur atom and an oxygen atom, a spiro heterocyclic group, or a bridged heterocyclic group), or $-S(=O)_2Rf$ (wherein Rf is an amino group, a C1-C6 alkyl group, or a 4-10 membered saturated heterocyclic group).

More preferably, it is a halogen atom, a cyano group, a nitro group, a carboxyl group, a thioamide group, a C1-C6 alkyl group (which may be substituted with a group selected from the group consisting of a halogen atom, a hydroxy group, a C1-C14 acyloxy group, a C2-C6 alkynyl group and a C1-C6 alkoxy C1-C6 alkoxy group), an amino group, a C3-C6 cycloalkyl group (which may be substituted with an amino group), a C1-C6 alkoxy group (which may be substituted with a halogen atom), a C2-C7 alkoxycarbonyl group, a C1-C14 acyl group (which may be substituted with a halogen atom), C6-C10 aromatic hydrocarbon group (which may be substituted with a halogen atom), a monocyclic or bicyclic 5 to 10-membered unsaturated heterocyclic group having 1 to 4 hetero atoms selected from a nitrogen atom, a sulfur atom and an oxygen atom (which may be substituted with a group selected from the group consisting of a C1-C6 alkyl group and an oxo group), a $-CONH_2$ group, a (mono- or di-C1-C6 alkyl)aminocarbonyl group, a hydroxyaminocarbonyl group, a (C7-C13 aralkyl)oxyaminocarbonyl group, a cyclic aminocarbonyl group, an aminosulfonyl group, a C1-C6 alkylsulfonyl group, or a piperidinosulfonyl a group.

More preferably, it is a halogen atom, an amino group, a C1-C6 alkyl group (which may be substituted with a group selected from the group consisting of a halogen atom and a hydroxy group) a C1-C6 alkoxy group (which may be substituted with halogen atoms), a monocyclic 5 or 6-membered unsaturated heterocyclic group having 1 to 4 hetero atoms selected from a nitrogen atom, a sulfur atom and an oxygen atom, a $-CONH_2$ group, a (mono or di C1-C6 alkyl) aminocarbonyl group, or a hydroxyamino group.

More preferably, it is a halogen atom, an amino group, a C1-C6 alkoxy group, or a - $CONH_2$ group.

When the compound of the formula (I) has two or more substituents on $R^3$ and two substituents on the carbon atoms adjacent each other on the aromatic hydrocarbon ring of $R^3$, the "4 to 8-membered saturated or partially unsaturated hydrocarbon ring or heterocyclic ring which may be substituted", which is formed with the carbon atoms to which they are attached, is the ring, such as a ring fused to a benzene ring. "4 to 8-membered saturated or partially unsaturated hydrocarbon ring or heterocyclic ring" in the "4 to 8-membered saturated or partially unsaturated hydrocarbon ring or heterocyclic ring which may be substituted" is preferably a monocyclic saturated or partially unsaturated hydrocarbon ring having 4 to 8 carbon atoms, or 4 to 8-membered saturated or partially unsaturated hetero ring having 1 to 4 hetero atoms selected from the group consisting of a nitrogen atom, a sulfur atom and an oxygen atom, more preferably, a monocyclic 4 to 6-membered saturated or partially unsaturated heterocyclic ring having 1 to 3 hetero atoms selected from the group consisting of a nitrogen atom, a sulfur atom and an oxygen atom, more preferably a monocyclic 6-membered saturated or partially unsaturated heterocyclic ring having one or two oxygen atom(s).

Substituent Rc in the "4 to 8-membered saturated or partially unsaturated hydrocarbon ring or heterocyclic ring which is optionally substituted with Rc" is a halogen atom, a hydroxy group, an amino group, an oxo group, a C1-C6 alkyl group which is optionally substituted with a hydroxy group, a halogeno C1-C6 alkyl group, a C1-C14 acyl group, a C1-C14 acylamino group, a C1-C14 acyloxy group, or a C7-C13 aralkyloxy group, preferably a hydroxy group, an amino group, an oxo group, or a C1-C6 alkyl group which is optionally substituted with a hydroxy group, a halogeno C1-C6 alkyl group, a C1-C14 acyl group, a C1-C14 acyloxy group, more preferably a hydroxy group, or a C1-C6 alkyl group. The number of Rc which substitutes at a saturated or partially unsaturated hydrocarbon ring or heterocyclic ring is not particularly limited, but is preferably 1 to 3. When the number of substituent Rc is 2 or more, the type of groups may be the same or different.

"4 to 8-membered saturated or partially unsaturated hydrocarbon ring or heterocyclic ring which is optionally substituted with Rc" is preferably a monocyclic saturated or partially unsaturated hydrocarbon ring (which is optionally substituted with a group selected from the group consisting of a halogen atom, a hydroxy group, an amino group, an oxo group, a C1-C6 alkyl group optionally substituted with a hydroxy group, a halogeno C1-C6 alkyl group, a C1-C14 acyl group, a C1-C14 acylamino group, a C1-C14 acyloxy group and a C7-C13 aralkyloxy group), a monocyclic 4 to 8-membered saturated or partially unsaturated heterocyclic ring having 1 to 4 hetero atoms selected from a nitrogen atom, a sulfur atom, and an oxygen atom from sulfur atom and an oxygen atom (which is optionally substituted with a group selected from the group consisting of a halogen atom, a hydroxy group, an amino group, an oxo group, a C1-C6 alkyl group optionally substituted with a hydroxy group, a halogeno C1-C6 alkyl group, a C1-C14 acyl group, a C1-C14 acylamino group, a C1-C14 acyloxy group and a C7-C13 aralkyloxy group).

More preferably, a monocyclic saturated or partially unsaturated hydrocarbon ring having 4 to 8 carbon atoms (which is optionally substituted with a group selected from the group consisting of a halogen atom, a hydroxy group, an amino group, an oxo group, and a C1-C6 alkyl group optionally substituted with a hydroxy group, a halogeno C1-C6 alkyl group a C1-C14 acyl group, a C1-C14 acylamino group, and a C1-C14 acyloxy group), or a monocyclic 4 to 8-membered saturated or partially unsaturated heterocyclic ring having 1 to 3 hetero atoms selected from a nitrogen atom, a sulfur atom and an oxygen atom (which is optionally substituted with a group selected from the group consisting of a halogen atom, a hydroxy group, an amino group, an oxo group, a C1-C6 alkyl group optionally substituted with a hydroxy group, a halogeno C1-C6 alkyl group, a C1-C14 acyl group, a C1-C14 acylamino group, and a C1-C14 acyloxy group).

More preferably, a monocyclic 4 to 6-membered heterocyclic ring having 1 to 3 heteroatoms selected from a nitrogen atom, a sulfur atom and an oxygen atom, (which is optionally substituted with a group selected from the group consisting of a hydroxy group, an amino group, an oxo group, a C1-C6 alkyl group, a halogeno C1-C6 alkyl group, a C1-C14 acylamino group and a C1-C14 acyloxy group).

More preferably, a monocyclic 6-membered saturated or partially unsaturated heterocyclic ring having 1 or two oxygen atom(s) (which is optionally substituted with a group selected from the group consisting of a hydroxyl group and a C1-C6 alkyl group).

In the compounds represented by the formula (I), a fused ring which is formed when there are two substituents on the carbon atoms adjacent each other on the aromatic hydrocarbon ring of $R^3$, is for example, a chroman ring, a dihydrobenzoxazine ring, a dihydroindene ring, an indoline ring, a tetrahydroquinoxaline ring, a dihydrobenzodioxane ring, a tetrahydronaphthalene ring, a tetrahydroquinoline ring, a tetrahydroisoquinoline ring, a dihydrobenzothiophene ring, an isoindoline ring, a dihydroisobenzofuran ring, a dihydrobenzoimidazole ring, and the like.

In the compounds represented by the formula (I), $R^3$ is preferably a C6-C14 aromatic hydrocarbon group, or a monocyclic or bicyclic 5 to 10-membered fully unsaturated heterocyclic group having 1 to 3 heteroatom(s) selected from a nitrogen atom, a sulfur atom and an oxygen atom, wherein $R^3$ may be substituted with $R^{31}$, or when $R^3$ has two substituents on the carbon atoms which are adjacent each other on the aromatic hydrocarbon ring, the substituents may be fused together with carbon atoms to which the substituents are attached to form a monocyclic saturated or partially unsaturated hydrocarbon ring having 4 to 8 carbon atoms (which is optionally substituted with a group consisting of the group selected from a halogen atom, a hydroxy group, an amino group, an oxo group, a C1-C6 alkyl group optionally

substituted with a hydroxy group, a halogeno C1-C6 alkyl group, a C1-C14 acyl group, a C1-C14 acylamino group, a C1-C14 acyloxy group, and a C7-C13 aralkyloxy group), or, a monocyclic 4 to 8-membered saturated or partially unsaturated heterocyclic ring having 1 to 4 hetero atoms selected from a nitrogen atom, a sulfur atom or an oxygen atom (optionally substituted with a group selected from the group consisting of a halogen atom, a hydroxy group, an amino group, an oxo group, a C1-C6 alkyl group optionally substituted with a hydroxy group, a halogeno C1-C6 alkyl groups, a C1-C14 acyl group, a C1-C14 acylamino group, a C1-C14 acyloxy group, a C7-C13 aralkyloxy group);

$R^{31}$ is a halogen atom, a cyano group, a nitro group, a carboxyl group, a thioamide group, a C1-C6 alkyl group (optionally substituted with a group selected from the group consisting of a halogen atom, a hydroxy group, a C1-C14 acyl group, a C1-C14 acyloxy group, a C2-C6 alkynyl group and a C1-C6 alkoxy C1-C6 alkoxy group), an amino group (optionally substituted with a C1-C14 acyl group), a C3-C6 cycloalkyl group (optionally substituted with an amino group), a C1-C6 alkoxy group (optionally substituted with halogen atoms), a C2-C7 alkoxycarbonyl group, a C1-C14 acyl group (optionally substituted with a halogen atom), a C6-C14 aromatic hydrocarbon group (optionally substituted with a group selected from the group consisting of a halogen atom, an amino group and a C1-C6 alkoxy group), a monocyclic or bicyclic 5 to 10-membered unsaturated heterocyclic group having 1 to 4 heteroatoms selected from a nitrogen atom, a sulfur atom and an oxygen atom (optionally substituted with a group selected from the group consisting of a halogen atom, an oxo group, and a C1-C6 alkyl group), an amino carbonyl group optionally substituted with Rd and Re (wherein Rd and Re are the same or different, and are a hydrogen atom, hydroxy group, a C7-C13 aralkyloxy group, a C1-C6 alkyl group which is optionally substituted with a hydroxyl group, alternatively taken together with the adjacent nitrogen atom to form a monocyclic or bicyclic 4 to 10-membered saturated or unsaturated heterocyclic group having 1 to 3 heteroatoms selected from a nitrogen, a sulfur and an oxygen atom, which may be substituted with an amino group, a spiro heterocyclic group, or a bridged heterocyclic group), or $-S(=O)_2Rf$ (wherein Rf is an amino group, a C1-C6 alkyl group, or a 4 to 10-membered saturated heterocyclic group).

In the compounds represented by the formula (I), $R^3$ is more preferably a C6-C10 aromatic hydrocarbon group, or a monocyclic or bicyclic 5 to 10-membered fully unsaturated heterocyclic group having 1 to 3 hetero atoms selected from a nitrogen atom, a sulfur atom and an oxygen atom, wherein $R^3$ is optionally substituted with $R^{31}$, and when it has two substituents on the carbon atoms which are adjacent each other on the aromatic hydrocarbon ring, the substituents may be fused together with carbon atoms to which the substituents are attached to form a monocyclic saturated or partially unsaturated hydrocarbon ring having 4 to 8 carbon atoms (which is optionally substituted with a group consisting of the group selected from a halogen atom, a hydroxy group, an amino group, an oxo group, a C1-C6 alkyl group optionally substituted with a hydroxy group, a halogeno C1-C6 alkyl group, a C1-C14 acyl group, a C1-C14 acylamino group, and a C1-C14 acyloxy group), or a monocyclic 4 to 8-membered saturated or partially unsaturated heterocyclic ring having 1 to 3 hetero atoms selected from a nitrogen atom, a sulfur atom and an oxygen atom (optionally substituted with a group selected from the group consisting of a halogen atom, a hydroxy group, an amino group, an oxo group, a C1-C6 alkyl group optionally substituted with a hydroxy group; a halogeno C1-C6 alkyl groups; a C1 -C14 acyl group; a C1-C14 acylamino group; a C1-C14 acyloxy group);

$R^{31}$ is a halogen atom, a cyano group, a nitro group, a carboxyl group, thioamide group, a C1-C6 alkyl group (optionally substituted with a group selected from the group consisting of a halogen atom, a hydroxy group, a C1-C14 acyloxy group, a C2-C6 alkynyl group and a C1-C6 alkoxy C1-C6 alkoxy group), an amino group, a C3-C6 cycloalkyl group (optionally substituted with an amino group), a C1-C6 alkoxy group (optionally substituted with a halogen atom), a C2-C7 alkoxycarbonyl group, a C1-C14 acyl group (optionally substituted with a halogen atom), C6-C10 aromatic hydrocarbon group (which may be substituted with a halogen atom), a monocyclic or bicyclic 5 to 10-membered unsaturated heterocyclic group having 1 to 4 hetero atoms selected from a nitrogen atom, a sulfur atom, and an oxygen atom (optionally substituted with a group selected from the group consisting of a C1-C6 alkyl group or an oxo group), $-CONH_2$ group, (mono- or di-C1-C6 alkyl) aminocarbonyl group, a hydroxyamino group, (C7-C13 aralkyl) oxy aminocarbonyl group, a cyclic amino carbonyl group, an aminosulfonyl group, a C1-C6 alkylsulfonyl group, or a piperidinosulfonyl group.

In the compounds represented by the formula (I), $R^3$ is more preferably a C6-C10 aromatic hydrocarbon group (wherein the C6-C10 aromatic hydrocarbon group is optionally substituted with $R^{31}$, and when a C6-C10 aromatic hydrocarbon group has two substituents on the carbon atoms which are adjacent each other on the aromatic hydrocarbon ring, the substituents may be fused together with carbon atoms to which the substituents are attached to form a monocyclic 4 to 6-membered saturated or partially unsaturated hetero ring having 1 to 3 hetero atoms (which is optionally substituted with a group consisting of a hydroxy group, an amino group, an oxo group, a C1-C6 alkyl group, a halogeno C1-C6 alkyl group, a C1-C14 acylamino group, and a C1-C14 acyloxy group), or a monocyclic 5 to 6-membered fully unsaturated heterocyclic group having 1 to 3 hetero atoms selected from a nitrogen atom, a sulfur atom and an oxygen atom (which is optionally substituted with a group selected from the group consisting of a halogen atom, a C1-C6 alkyl group optionally substituted with a hydroxyl group, a C1-C6 alkoxy group, a C2-C7 alkoxycarbonyl group, a $-CONH_2$ group (mono- or di-C1-C6 alkyl) aminocarbonyl group, a pyrrolidin-1-ylcarbonyl group, a morpholin-4-ylcarbonyl group, a 2-oxa-7-azaspiro[3.5]nonanyl group, a 3-oxa-8-azabicyclo[3.2.1]octanyl group, and an 8-oxa-3-azabicyclo[3.2.1]octanyl group);

$R^{31}$ is a halogen atom, an amino group, a C1-C6 alkyl group (which is optionally substituted with a group

selected from the group consisting of a halogen atom and a hydroxy group), a C1-C6 alkoxy group (which is optionally substituted with a halogen atom), a 5 or 6-membered unsaturated heterocyclic group having 1 to 4 hetero atoms selected from a nitrogen atom, a sulfur atom and an oxygen atom, a $-CONH_2$ group, a (mono or di-C1-C6 alkyl) aminocarbonyl group, or a hydroxyamino group.

Also, in the compounds represented by formula (I), $R^3$ is particularly preferably a phenyl group (wherein the phenyl group may be substituted with $R^{31}$, and when a phenyl group has two substituents on the carbon atoms which are adjacent each other on a benzene ring, the substituents may be fused together with carbon atoms to which the substituents are attached to form a monocyclic 6-membered saturated or partially unsaturated hetero ring having one or two oxygen atoms (which is optionally substituted with a group selected from the group consisting of a hydroxy group and a C1-C6 alkyl group)), or a pyridyl group (optionally substituted with a $-CONH_2$ group, a (mono or di C1-C6 alkyl) aminocarbonyl group, or a pyrrolidin-1-yl carbonyl group);

$R^{31}$ is a halogen atom, an amino group, a C1-C6 alkoxy group, or a $-CONH_2$ group.

In the compounds represented by the formula (I), $R^4$ is a hydrogen atom, or a C1-C6 alkyl group.

"C1-C6 alkyl group" indicated in $R^4$ is preferably a C1-C3 alkyl group, more preferably a methyl group.

$R^4$ is preferably a hydrogen atom, or a methyl group, more preferably a hydrogen atom.

In the sulfonamide compounds of formula (I), preferred compounds include the following.

In formula (I),

$X^1$ represents an oxygen atom or a sulfur atom;

$X^2$ represents an oxygen atom;

$X^3$ represents -NH-;

$X^4$ represents a hydrogen atom or a methyl group;

$R^1$ represents $-C(R^{11})(R^{12})$ (wherein $R^{11}$ and $R^{12}$ are the same or different, and a hydrogen atom or C1-C6 alkyl group);

$R^2$ represents a C6-C14 aromatic hydrocarbon group, wherein $R^2$ may be substituted with $R^{21}$, and when $R^2$ has two substituents on the carbon atoms which are adjacent each other on the aromatic hydrocarbon ring, the substituents may be fused together with carbon atoms to which the substituents are attached to form a monocyclic saturated or partially unsaturated hydrocarbon ring having 4 to 8 carbons (which is optionally substituted with a C1-C6 alkyl group);

$R^{21}$ is a halogen atom, a cyano group, C1-C6 alkyl group (which is optionally substituted with a halogen atom), a C3-C6 cycloalkyl group, a phenyl group (which is optionally substituted with a group selected from the group consisting of a halogen atom and a C1-C6 alkoxy group), or a monocyclic or bicyclic 5 to 10-membered unsaturated heterocyclic group having 1 to 3 hetero atoms selected from a nitrogen atom, a sulfur atom and an oxygen atom (which is optionally substituted with a group selected from the group consisting of a halogen atom, a C1-C6 alkyl group, a halogeno C1-C6 alkyl group, a C3-C6 cycloalkyl group, a C1-C6 alkoxy group, a morpholino group, a piperidinyl group and a morpholino-carbonyl group);

$R^3$ is a C6-C10 aromatic hydrocarbon group, or a monocyclic or bicyclic 5 to 10-membered fully unsaturated heterocyclic group having 1 to 3 hetero atoms selected from a nitrogen atom, a sulfur atom and an oxygen atom, wherein $R^3$ is optionally substituted with $R^{31}$, and when $R^3$ has two substituents on the carbon atoms which are adjacent each other on the aromatic hydrocarbon ring, the substituents may be fused together with carbon atoms to which the substituents are attached to form a monocyclic saturated or partially unsaturated hydrocarbon ring having 4 to 8 carbon atoms (which is optionally substituted with a group consisting of the group selected from a halogen atom, a hydroxy group, an amino group, an oxo group, a C1-C6 alkyl group optionally substituted with a hydroxy group, a halogeno C1-C6 alkyl group, a C1-C14 acyl group, a C1-C14 acylamino group, and a C1-C14 acyloxy group), or a monocyclic 4 to 8-membered saturated or partially unsaturated heterocyclic ring having 1 to 3 hetero atoms selected from a nitrogen atom, a sulfur atom and an oxygen atom (optionally substituted with a group selected from the group consisting of a halogen atom, a hydroxy group, an amino group, an oxo group, a C1-C6 alkyl group optionally substituted with a hydroxy group; a halogeno C1-C6 alkyl group; a C1-C14 acyl group; a C1-C14 acylamino group; and C1-C14 acyloxy group);

$R^{31}$ is a halogen atom, a cyano group, a nitro group, a carboxyl group, a thioamide group, a C1-C6 alkyl group (optionally substituted with a group selected from the group consisting of a halogen atom, a hydroxy group, a C1-C14 acyloxy group, a C2-C6 alkynyl group and a C1-C6 alkoxy C1-C6 alkoxy group), an amino group, a C3-C6 cycloalkyl group (optionally substituted with an amino group), a C1-C6 alkoxy group (optionally substituted with a halogen atom), a C2-C7 alkoxycarbonyl group, a C1-C14 acyl group (optionally substituted with a halogen atom), a C6-C10 aromatic hydrocarbon ring (optionally substituted with a halogen atom), a monocyclic or bicyclic 5 to 10-membered unsaturated heterocyclic group having 1 to 4 hetero atoms selected from a nitrogen atom, a sulfur atom, and an oxygen atom (optionally substituted with a group selected from the group consisting of a C1-C6 alkyl group and an oxo group),$-CONH_2$ group, a (mono- or di-C1-C6 alkyl)aminocarbonyl group, a hydroxyaminocarbonyl group, a (C7-C13 aralkyloxy)oxyaminocarbonyl group, a cyclic aminocarbonyl group, an aminosulfonyl group, a C1-C6 alkylsulfonyl group, or a piperidinosulfonyl group; and

$R^4$ represents a hydrogen atom;

(with the proviso that $X^1$ is an oxygen atom when $X^2$ represents an oxygen atom, $X^3$ represents -NH-, $X^4$ represents a

hydrogen atom, $R^1$ represents $-CH_2-$, $R^2$ represents a phenyl group, $R^3$ represents 4-methylphenyl group, and $R^4$ represents a hydrogen atom)
or a salt thereof.

Furthermore, in the sulfonamide compounds of formula (I) of the present invention, more preferable compounds include the following.

In formula (I),
$X^1$ represents an oxygen atom;
$X^2$ represents an oxygen atom;
$X^3$ represents -NH-;
$X^4$ represents a hydrogen atom;
$R^1$ represents $-C(R^{11})(R^{12})$ (wherein $R^{11}$ represents a C1-C6 alkyl group, and $R^{12}$ represents a hydrogen atom);
$R^2$ represents a C6-C10 aromatic hydrocarbon group, wherein $R^2$ may be substituted with $R^{21}$, and when $R^2$ has two substituents on the carbon atoms which are adjacent each other on the aromatic hydrocarbon ring, the substituents may be fused together with carbon atoms to which the substituents are attached to form a monocyclic saturated or partially unsaturated hydrocarbon ring having 5 or 6 carbons (which is optionally substituted with a C1-C6 alkyl group);
$R^{21}$ is a halogen atom, a C1-C6 alkyl group or a monocyclic 5 to 6-menbered unsaturated heterocyclic group having 1 to 3 nitrogen atom(s) (which is optionally substituted with a C1-C6 alkyl group);
$R^3$ is a C6-C10 aromatic hydrocarbon group (wherein the C6-C10 aromatic hydrocarbon group is optionally substituted with $R^{31}$, and when a C6-C10 aromatic hydrocarbon group has two substituents on the carbon atoms which are adjacent each other on the aromatic hydrocarbon ring, the substituents may be fused together with carbon atoms to which the substituents are attached to form a monocyclic 4 to 6-membered saturated or partially unsaturated heterocyclic ring having 1 to 3 hetero atoms selected from a nitrogen atom, a sulfur atom and an oxygen atom (optionally substituted with a group selected from the group consisting of a hydroxy group, an amino group, an oxo group, a C1-C6 alkyl group, a halogeno C1-C6 alkyl group, a C1-C14 acyl group, a C1-C14 acylamino group, and C1-C14 acyloxy group) or a monocyclic 5 to 6-membered fully unsaturated heterocyclic group having 1 to 3 hetero atoms selected from a nitrogen atom, a sulfur atom and an oxygen atom (which is optionally substituted with a group selected from the group consisting of a halogen atom, a C1-C6 alkyl group optionally substituted with a hydroxyl group, a C1-C6 alkoxy group, a C2-C7 alkoxycarbonyl group, a $-CONH_2$ group, (mono- or di-C1-C6 alkyl) aminocarbonyl group, a pyrrolidin-1-ylcarbonyl group, a morpholin-4-ylcarbonyl group, a 2-oxa-7-azaspiro[3.5]nonanyl group, a 3-oxa-8-azabicyclo[3.2.1]octanyl group, and an 8-oxa-3-azabicyclo[3.2.1]octanyl group);
$R^{31}$ is a halogen atom, an amino group, a C1-C6 alkyl group (optionally substituted with a group selected from the group consisting of a halogen atom and a hydroxy group), a C1-C6 alkoxy group (optionally substituted with a halogen atom), a monocyclic 5 to 6-membered unsaturated heterocyclic group having 1 to 4 hetero atoms selected from a nitrogen atom, a sulfur atom, and an oxygen atom, a $-CONH_2$ group, (mono- or di-C1-C6 alkyl)aminocarbonyl group, a hydroxyaminocarbonyl group; and
$R^4$ represents a hydrogen atom;
or a salt thereof.

In the sulfonamide compounds of formula (I), more preferable compounds include the following.

In formula (I),
$X^1$ represents an oxygen atom;
$X^2$ represents an oxygen atom;
$X^3$ represents -NH-;
$X^4$ represents a hydrogen atom;
$R^1$ represents $-C(R^{11})(R^{12})$ (wherein $R^{11}$ represents a methyl group, and $R^{12}$ represents a hydrogen atom);
$R^2$ represents a phenyl group or a naphthyl group, wherein $R^2$ may be substituted with $R^{21}$, and when $R^2$ has two substituents on the carbon atoms which are adjacent each other on the aromatic hydrocarbon ring, the substituents may be fused together with carbon atoms to which the substituents are attached to form a monocyclic saturated or partially unsaturated hydrocarbon ring having 5 or 6 carbons (which is optionally substituted with a C1-C6 alkyl group);
$R^{21}$ is a halogen atom or a C1-C6 alkyl group;
$R^3$ is a phenyl group (wherein the phenyl group is optionally substituted with $R^{31}$, and when a phenyl group has two substituents on the carbon atoms which are adjacent each other on a benzene ring, the substituents may be fused together with carbon atoms to which the substituents are attached to form a monocyclic 6-membered saturated or partially unsaturated heterocyclic ring having 1 or 2 oxygen atom(s) (optionally substituted with a group selected from the group consisting of a hydroxyl group and a C1-C6 alkyl group), or a pyridyl group (optionally substituted with a $-CONH_2$ group, a (mono- or di-C1-C6 alkyl) aminocarbonyl group, a pyrrolidin-1-ylcarbonyl group)
$R^{31}$ is a halogen atom, an amino group, a C1-C6 alkoxy group, a $-CONH_2$ group; and
$R^4$ represents a hydrogen atom;
or a salt thereof.

Particularly preferable sulfonamide compounds include the following.

(1) 5-bromo-2-(N-((1S,2R)-2-(6-fluoro-2,3 -dimethylphenyl)-1-(5-oxo-4,5-dihydro-1,3,4-oxadiazol-2-yl)propyl)sulfamoyl)benzamide;
(2) 5-chloro-2-(N-((1S,2R)-2-(2,3-dimethylphenyl)-1-(5-oxo-4,5-dihydro-1,3,4-oxadiazol-2-yl)propyl)sulfamoyl)benzamide;
(3) 5-bromo-2-(N-((1S,2R)-2-(2,3-dimethylphenyl)-1-(5-oxo-4,5-dihydro-1,3,4-oxadiazol-2-yl)propyl)sulfamoyl)benzamide;
(4) 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide;
(5) 5-chloro-2-(N-((1S,2R)-2-(2-fluoronaphtalen-1-yl)-1-(5-oxo-4,5-dihydro-1,3,4-oxadiazol-2-yl)propyl)sulfamoyl)benzamide;
(6) 5-chloro-2-(N-((1S,2R)-2-(3-ethyl-6-fluoro-2-methylphenyl)-1-(5-oxo-4,5-dihydro-1,3,4-oxadiazol-2-yl)propyl)sulfamoyl)benzamide;
(7) 5-chloro-2-(N-((1S,2R)-2-(3-chloro-6-fluoro-2-methylphenyl)-1-(5-oxo-4,5-dihydro-1,3,4-oxadiazol-2-yl)propyl)sulfamoyl)benzamide;
(8) 5-bromo-2-(N-((1S,2R)-2-(3-chloro-6-fluoro-2-methylphenyl)-1-(5-oxo-4,5-dihydro-1,3,4-oxadiazol-2-yl)propyl)sulfamoyl)benzamide;
(9) 2-(N-((1S,2R)-2-(3-bromo-6-fluoro-2-methylphenyl)-1-(5-oxo-4,5-dihydro-1,3,4-oxadiazol-2-yl)propyl)sulfamoyl)-5-chloro-benzamide;
(10) 5-chloro-N-((1S,2R)-2-(6-fluoro-2,3-dimethylphenyl)-1-(5-oxo-4,5-dihydro-1,3,4-oxadiazol-2-yl)propyl)-6-(pyrrolidine-1-carbonyl)pyridine-2-sulfonamide;
(11) 5-chloro-N-((1S,2R)-2-(6-fluoro-2,3-dimethylphenyl)-1-(5-oxo-4,5-dihydro-1,3,4-oxadiazol-2-yl)propyl)-4-hydroxy-4-methyl-d3-chroman-8-sulfonamide;
(12) 5-chloro-N-((1S,2R)-2-(3-chloro-6-fluoro-2-methylphenyl)-1-(5-oxo-4,5-dihydro-1,3,4-oxadiazol-2-yl)propyl)-4-hydroxy-4-methyl-chroman-8-sulfonamide;
(13) N-((1S,2R)-2-(3-bromo-6-fluoro-2-methylphenyl)-1-(5-oxo-4,5-dihydro-1,3,4-oxadiazol-2-yl)propyl)-5-chloro-4-hydroxy-4-methyl-chroman-8-sulfonamide;
(14) 5-chloro-N-((1S,2R)-2-(3-chloro-6-fluoro-2-methylphenyl)-1-(5-oxo-4,5-dihydro-1,3,4-oxadiazol-2-yl)propyl)-4-hydroxy-4-methyl-d3-chroman-8-sulfonamide;
(15) 5-chloro-N-((1S,2R)-2-(6-fluoro-2,3-dimethylphenyl)-1-(5-oxo-4,5-dihydro-1,3,4-oxadiazol-2-yl)propyl)-4-hydroxychroman-8-sulfonamide;
(16) 3-chloro-6-(N-((1S,2R)-2-(6-fluoro-2,3-dimethylphenyl)-1-(5-oxo-4,5-dihydro-1,3,4-oxadiazol-2-yl)propyl)sulfamoyl)-N,N-dimethylpicolinamide;
(17) 4-amino-2-methoxy-N-((1S,2R)-2-(8-methylnaphthalen-1-yl)-1-(5-oxo-4,5-dihydro-1,3,4-oxadiazol-2-yl)propyl)benzenesulfonamide;
(18) 4-amino-N-((1S,2R)-2-(2,3-dihydro-1H-inden-4-yl)-1-(5-oxo-4,5-dihydro-1,3,4-oxadiazol-2-yl)propyl)-2-methoxybenzenesulfonamide; and
(19) 5-chloro-2-[[(1S,2R)-3,3,3-trideuterio-2-(6-fluoro-2,3-dimethylphenyl)-1-(2-oxo-3H-1,3,4-oxadiazol-5-yl)propyl]sulfamoyl]benzamide.

A method of preparing the sulfonamide compound according to the present invention is described by giving examples. The compounds of the formula (I) of the present invention, for example, can be prepared by the following production method. However, the present invention is not limited to this method.

[Formula 10]

[Step A]

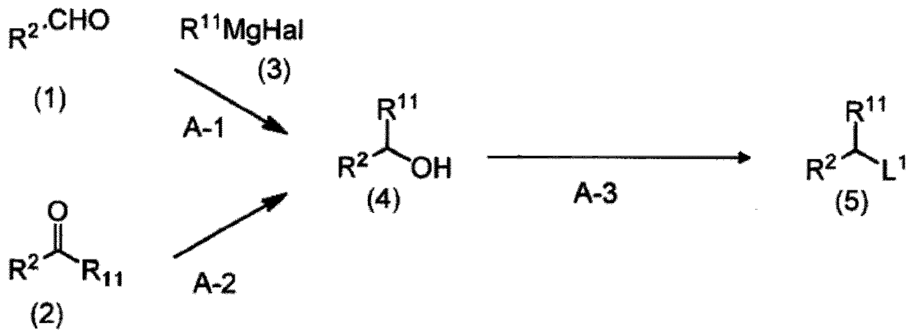


[wherein, $L^1$ represents a leaving group. The symbols have the same meanings as defined above.]

[A-1]

In this process, a compound represented by general formula (4) can be prepared by reacting a compound represented by general formula (1) with an organometallic reagent (3) such as Grignard reagent represented by $R^{11}$MgHal.

Hal represents a halogen atom.

The amount of Grignard reagent (3) 0.8 to 20 equivalents relative to compound (1), preferably 1.0 to 10 equivalents. The reaction temperature is -80°C to 100°C, preferably -78°C to 50°C. The reaction time is 0.1 to 24 hours, preferably 0. 1 to 3 hours.

In this step, a compound represented by general formula (4), wherein $R^{11}$ is H, can be prepared by reacting the compound represented by formula (1) with a well-known reducing agent instead of Grignard reagent (3).

The reducing agents to be used include, for example, sodium borohydride, lithium borohydride, lithium aluminum hydride, diethoxy aluminum lithium hydride, triethoxy lithium aluminum hydride, tri-t-butoxy aluminum lithium hydride, aluminum magnesium hydride, aluminum hydride magnesium chloride, sodium aluminum hydride, sodium triethoxyaluminum hydride, bis(2-methoxyethoxy) aluminum sodium hydride, diisobutylaluminum hydride (hereinafter DIBAL-H) and the like, and preferably sodium borohydride.

The reaction solvent to be used is not particularly limited as long as it does not affect the reaction, for example, ethers (diethyl ether, tetrahydrofuran, 1,4-dioxane, 1,2-dimethoxyethane, and the like), alcohols (methanol, ethanol, and the like), and water, and preferably methanol.

The amount of reducing agent used is 0.8 to 10 equivalents relative to the compound (1), preferably 1 to 5 equivalents.

The reaction temperature is from 0°C to the boiling point temperature of the solvent, preferably 0 to 40°C. The reaction time is from 0.05 to 24 hours, preferably 0.2 to 2 hours. Thus, the compound represented by general formula (4) obtained in the above manner can be subjected to the next step with or without isolation and purification by a well-known separation and purification means described below

[A-2]

In this step, a compound represented by general formula (4) can be prepared by reacting a compound represented by general formula (2) with well-known reducing agents,

The reducing agents to be used include sodium borohydride.

The reaction solvents to be used are not particularly limited as long as they do not affect the reaction, and, for example, ethers (diethyl ether, tetrahydrofuran, 1,4-dioxane, 1,2-dimethoxyethane, etc.), alcohols (methanol, ethanol, 2-propanol, tert-butanol, ethylene glycol, etc.), water and the like, preferably methanol or ethanol.

The amount of reducing agent is 0.8 to 10 equivalents relative to the compound (2), preferably 1 to 5 equivalents.

The reaction temperature is between 0°C and the boiling point temperature of the solvent, preferably 0 to 40°C. The reaction time is from 0.05 to 24 hours, preferably 0.2 to 2 hours. Thus, the obtained compound represented by general formula (4) can be subjected to the next step with or without isolation and purification by well-known separation and purification means described below.

[A-3]

In this process, a compound represented by general formula (5) can be prepared by reacting a compound represented by general formula (4) with a halogenating agent or sulfonyl halide hydrocarbons.
Leaving groups represented by $L^1$ are, for example, a halogen atom such as a chlorine atom, a bromine atom or an iodine atom, a methylsulfonyloxy group, a trifluoromethylsulfonyloxy group, an organic sulfonyloxy group such as a p-tolylsulfonyloxy group.
The reaction solvents to be used are not particularly limited as long as they do not affect the reaction, for example, ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride, etc.), aromatic hydrocarbons (benzene, toluene, xylene, pyridine, etc.), and preferably ethers.
The halogenating agents to be used are, for example, thionyl chloride, oxalyl chloride, phosphorus pentachloride, phosphorus trichloride, thionyl bromide, phosphorus tribromide and the like. Preferably, it is thionyl chloride or phosphorus tribromide. The sulfonyl halide hydrocarbons are, for example, methanesulfonyl chloride, ethanesulfonyl chloride, p-toluenesulfonyl chloride or phenylsulfonyl chloride and the like.
The reaction solvents to be used are not particularly limited as long as they do not affect the reaction, and, for example, ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride), aromatic hydrocarbons (benzene, toluene, xylene, etc.), and preferably dichloromethane.
The amount of the halogenating agent or sulfonyl halide hydrocarbons is 0.3 equivalents to 20 equivalents relative to the compound (4), preferably 0.3 to 4 equivalents.
The reaction temperature is -20°C to 100°C, preferably from 0°C to 100°C. The reaction time is generally 0.01 to 200 hours, preferably 0.5 hour to 24 hours. Thus, the obtained compound represented by general formula (5) can be subjected to the next step with or without isolation and purification by well-known separation and purification means described below.

[Formula 11]

[Step B]

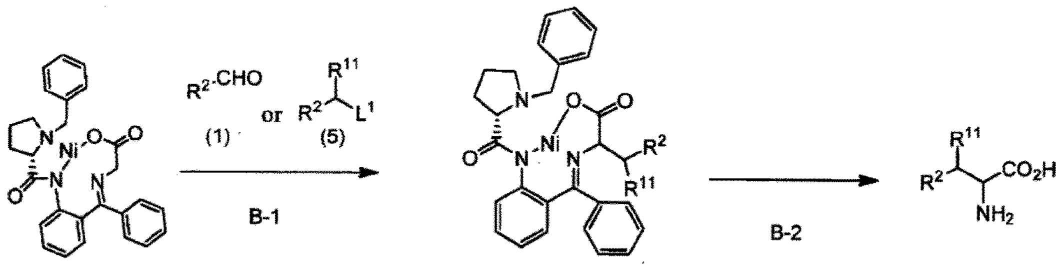


(6) (7) (8)

[Symbols in the formula are as defined above.]

[B-1]

In this process, a nickel complex represented by general formula (7) is prepared by reacting a compound represented by general formula (1) or (5) with a readily available compound represented by formula (6).
The reaction solvents to be used are not particularly limited as long as they do not affect the reaction, and for example, organic solvents or mixtures thereof such as ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride), aromatic hydrocarbons (benzene, toluene, xylene, etc.), aliphatic hydrocarbons (hexane, pentane, cyclohexane, etc.), nitriles (acetonitrile, propionitrile etc.), amides (N,N-dimethylformamide (hereinafter, also referred to as DMF), N,N-dimethylacetamide, N-methylpyrrolidinone, and preferably DMF.
The bases to be used are, for example: organic amines such as triethylamine, tripropylamine, diisopropylethylamine, N-methylmorpholine, pyridine, lutidine, or collidine; alkali metal salts such as sodium carbonate, potassium carbonate, cesium carbonate, sodium phosphate, potassium phosphate, sodium hydroxide, potassium hydroxide; alkali metal alkoxides such as sodium methoxide, sodium ethoxide, sodium tert-butoxide, potassium tert-butoxide; a strong base lithium amide such as lithium diisopropylamide; a strong base hexamethyldisilazane such as lithium hexamethyl

disilazane, sodium hexamethyldisilazane, potassium hexamethyldisilazane; and preferably sodium hydroxide, potassium hydroxide, potassium tert- butoxide and the like.

The amount of the base to be used is usually 0.1 to 100 equivalents relative to compound (6), preferably 1 to 20 equivalents.

The amount of compound (1) or (5) is 0.5 to 10 equivalents relative to compound (6), preferably 1 to 5 equivalents.

The reaction temperature is -80 to 50°C, preferably -60 to 40°C. The reaction time is 0.2 to 24 hours, preferably 0.5 to 6 hours. The pressure used in the above preparing method may not be particularly limited, and examples thereof include, about 0.1 to 10 atm. A nickel complex represented by general formula (7) which is obtained in this method can be subjected to the next step with or without isolation and purification by well-known separation and purification means described below.

[B-2]

In this step, an amino acid represented by general formula (8) can be prepared by reacting the nickel complex or a salt thereof with an acid represented by general formula (7).

The acids to be used are not particularly limited but include publicly known acids. The acids may be an inorganic acid or an organic acid. The inorganic acids include such as hydrochloric acid, nitric acid, sulfuric acid, and perchloric acid. The organic acids include such as acetic acid, trifluoroacetic acid, methanesulfonic acid, trifluoromethanesulfonic acid, oxalic acid, propionic acid, butyric acid, valeric acid, and the like. Preferably, the acid is hydrochloric acid, sulfuric acid, trifluoroacetic acid, or methanesulfonic acid, more preferably, it is hydrochloric acid, or methanesulfonic acid.

The amount of the acid is not particularly limited, and usually 0.1 to 50 equivalents relative to the nickel complex represented by general formula (7), and preferably 0.3 to 10 equivalents.

The solvent to be used is preferably alcohol, more preferable to methanol or ethanol.

The reaction temperature is usually 0°C to 100°C, and preferably 40 to 80°C. The reaction time is usually 0.1 to 72 hours, and preferably 0.1 to 10 hours. The pressure used in the above preparing method is not particularly limited, and examples thereof include, 0.1 to 10 atm. The amino acid represented by general formula (8) obtained in the present method can be subjected to the next process with or without a separation and purification means by well-known separation and purification means described below or transformation between protection and deprotection.

[Formula 12]

[Step C]

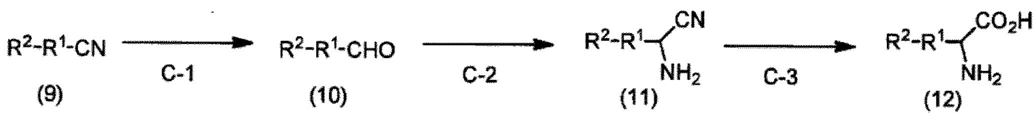


[Symbols in the formula are as defined above.]

[C-1]

In this step, a compound represented by general formula (10) can be prepared by reacting a compound represented by general formula (9) with a well-known reducing agent.

The reducing agent is tri(ethoxy) aluminum lithium hydride, tri(sec-butyl)boron lithium hydride, or DIBAL-H, and the like, and preferably DIBAL-H. The amount of the reducing agent to be used is usually 1 to 10 equivalents relative to the compound represented by general formula (9), preferably 2.0 to 10 equivalents.

The solvent to be used is ether type solvents (tetrahydrofuran, 1,4-dioxane, etc.), aprotic polar solvents (N,N-dimethylformamide, dimethyl sulfoxide, acetonitrile, etc.), halogen solvents (dichloromethane, chloroform, etc.), aromatic hydrocarbon solvents (toluene, xylene, etc.) or a mixed solvent thereof and the like, and preferably dichloromethane.

The reaction temperature is -100°C to 50°C, preferably -100 to 10°C. The reaction time is 0.1 to 24 hours, preferably 0.2 to 5 hours.

The pressure used in the above preparing method may not be particularly limited, and examples thereof include, from about 0.1 to 10 atm.

The compound represented by general formula (10) which is the obtained in this method can be subjected to the next step with or without isolation and purification by well-known separation and purification means described below.

The compound represented by general formula (9) can be prepared by the methods described in the reference

(international publication No. WO2011/071,565), or, if necessary, combining the methods described in the reference examples and examples.

[C-2]

In this step, a compound represented by general formula (11) is prepared by reacting with a compound represented by general formula (10) with a cyanide agent and ammonia.

The cyanide agent to be used is, for example, hydrogen cyanide, metal cyanides, cyanohydrin compounds, acyl cyanides, halogenated cyanides and the like. The metal cyanides are, for example, alkali metal cyanides such as sodium cyanides, potassium cyanides; alkaline earth metal cyanides such as calcium cyanide; transition metal cyanides such as copper cyanide. Preferably, it is potassium cyanide.

The ammonia used in in the present step can be ammonia gas, liquid ammonia or an aqueous ammonia solution, and an aqueous ammonia solution is desirable in terms of that it does not require complicated reaction apparatus.

The solvent to be used is not particularly limited as long as it does not affect the reaction, and it includes ethers (tetrahydrofuran, 1,4-dioxane, etc.), aprotic polar solvents (N,N-dimethylformamide, dimethyl sulfoxides, acetonitrile, etc.), halogen solvents (dichloromethane, chloroform, etc.), aromatic hydrocarbon solvents such as toluene, alcohol solvents (methanol, ethanol, etc.), water, and a mixed solvent thereof, and preferably water and a mixed solvent of methanol.

The amount of cyanide agent to be used is generally 1 to 10 equivalents relative to compound (10), preferably 2.0 to 5.0 equivalents. The amount of ammonia used in the reaction is preferably 1.5 to 10 equivalents relative to the compound (10), and more preferably 1.8 to 2.5 equivalents. Ammonium chloride is added as needed. Its amount is usually 0.2 to 2.0 equivalents relative to the compound of (10), preferably 0.1 to 0.5 equivalent.

The reaction temperature is -100°C to 100°C, preferably 0 to 60°C. The reaction time is 0.1 to 24 hours, preferably 0.2 to 5 hours. The pressure used in the above preparing method may not be particularly limited, and examples thereof include, from about 0.1 to 10 atm. The compound represented by general formula (11) can be subjected to the next step with or without isolation and purification by well-known separation and purification means as described below.

[C-3]

In this process, the compound represented by general formula (12) is prepared in the same manner as [B-2] described above using the compound represented by general formula (11). The compound represented by general formula (12) can be subjected to the next step with or without isolation and purification by well-known separation and purification means as described below. Hereinafter, post process for the compounds represented by general formulae (8) and (12) are described as an example.

Furthermore, in the present process, $R^1$ and $R^2$ can be converted to the structures corresponding to protection/deprotection groups or the present invention.

[Formula 13]

[Step D]

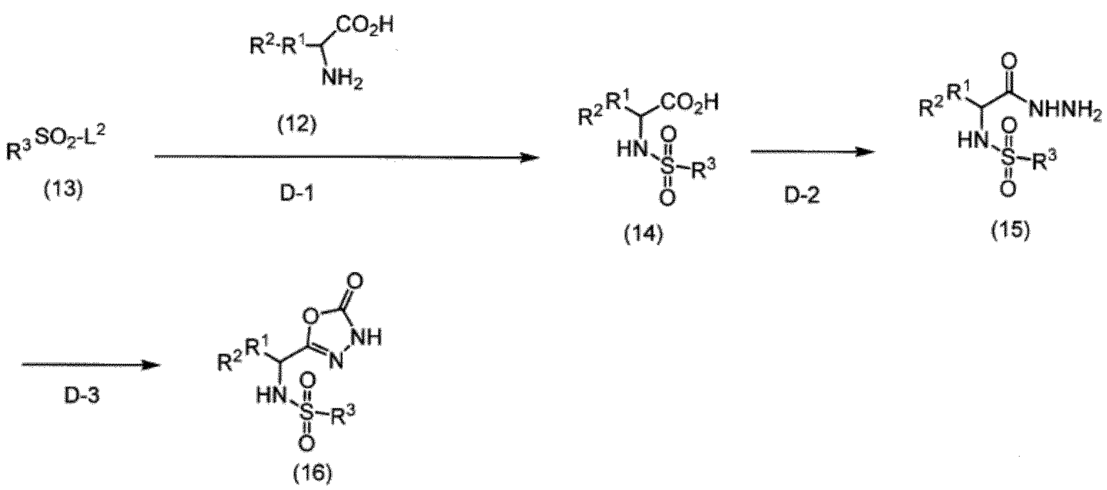

[In the formula, $L^2$ represents a leaving group. The symbols have the same meanings as defined above.]

[D-1]

In this step, a carboxylic acid represented by general formula (14) can be prepared by reacting an amino acid represented by general formula (12) with a sulfonic acid halide represented by general formula (13) in the presence of a base.

The base to be used is alkali metal salts such as sodium carbonate, potassium carbonate, cesium carbonate, sodium phosphate, potassium phosphate, sodium hydroxide, or organic amines such as trimethylamine or potassium hydroxide, triethylamine, tripropylamine, diisopropylethylamine, N-methylmorpholine, pyridine, lutidine, and collidine are exemplified, and preferably triethylamine.

The reaction solvent to be used is not particularly limited as long as it does not affect the reaction, and it is organic solvents or water, etc. such as ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride), aromatic hydrocarbons (benzene, toluene, xylene, etc.), aliphatic hydrocarbons (hexane, pentane, cyclohexane, etc.), nitriles (acetonitrile, propionitrile etc.), amides (DMF, N,N-dimethylacetamide, N-methylpyrrolidinone and the like. These solvents may be used in a mixture at an appropriate ratio.

The number of equivalents of base and an amine is from 0.5 to 10 equivalents, respectively, preferably 1.0 to 5.0 equivalents.

The amount of the sulfonic acid halide is appropriately set by the compounds represented by general formula (12), but is not limited to, and usually, is 1.0 to 5.0 equivalents relative to the compound represented by general formula (12), more preferably 1.0 to 2.5 equivalents.

The reaction temperature is appropriately by the compounds represented by general formula (12), but is not limited to, and, for example, a -20 to 70°C, preferably 0 to 40°C. The reaction time is generally 0.1 to 24 hours, preferably 0.2 to 6.0 hours. The compound represented by general formula (14) can be subjected to the next step with or without isolation and purification by well-known separation and purification means as described below.

The compound represented by general formula (13) can be prepared by the methods described in the reference (Tetrahedoron Lett. 51,418-421 (2010)), or, if necessary, combining the methods described in the reference examples and examples.

[D-2]

In this step, a compound represented by general formula (15) can be prepared by reacting a carboxylic acid represented by general formula (14) with a condensing agent and hydrazine. Alternatively, it can be prepared by reacting hydrazine derivative having an appropriate protecting group with the carboxylic acid represented by general formula (14) in the same manner, and then carrying out the reaction for eliminating the protecting group.

The condensing agent is, for example 1,1'-carbonyldiimidazole (hereinafter, CDI), dicyclohexylcarbodiimide, 1-ethyl-3-(3-dimethylaminopropyl)carbodiimide hydrochloride and the like, preferably CDI.

The solvent to be used is not particularly limited as long as it does not affect the reaction, for example, organic solvents such as ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride), aromatic hydrocarbons (benzene, toluene, xylene, etc.), aliphatic hydrocarbons (hexane, pentane, cyclohexane, etc.), nitriles (acetonitrile, propionitrile etc.), and amides (DMF, N,N-dimethylacetamide, N-methylpyrrolidinone, and they can be used alone or in combination.

The amount of the condensing agent with respect to the compound represented by Formula (14) is generally 1 to 50 equivalents, preferably about 1 to 5. The amount of hydrazine relative to the compound represented by general formula (14) is generally 1 to 100 equivalents, preferably 1-5 equivalents. The base is organic bases such as triethylamine, pyridine, 4-dimethylaminopyridine, diazabicycloundecene and the like.

The reaction temperature is -20 to 80°C, preferably 0 to 40°C. The reaction time is usually from 0.05 to 24 hours, more preferably 0.05 to 6 hours. The compound represented by general formula (15) can be subjected to the next step with or without isolation and purification by well-known separation and purification means as described below.

[D-3]

In this step, a compound represented by general formula (16) of the present invention can be prepared by cyclization of the compound represented by general formula (15) with the acylating agent.

The acylating agent is, for example isobutyl chloroformate, CDI, phosgene, triphosgene and the like, preferably CDI. The base is, organic bases such as triethylamine, N,N-diisopropylethylamine, pyridine, 4-dimethylaminopyridine, and like diazabicycloundecene and the like.

The amount of the acylating agent with respect to the compound represented by Formula (15) is typically preferably 1 to 50 equivalents, and more preferably 1 to 5 equivalents.

The solvent to be used is not particularly limited as long as it does not affect the reaction, for example, organic solvents such as ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride), aromatic hydrocarbons (benzene, toluene, xylene, etc.), aliphatic hydrocarbons (hexane, pentane, cyclohexane, etc.), nitriles (acetonitrile, propionitrile etc.), amides (DMF, N,N-dimethylacetamide, N-methylpyrrolidinone, etc.) and the like, and they may be used singly or as a mixture.

The reaction temperature is -20 to 80°C, preferably 0 to 50°C. The reaction time is generally 0.5 to 24 hours, preferably 0.5 to 8 hours. The compound represented by general formula (16) can be subjected to the next step with or without isolation and purification by well-known separation and purification means as described below.

The compound represented by general formula (16) of the present invention can be synthesized by 1) protecting the amino group of the amino acid of the compound represented by above general formula (12) with a well-known suitable protecting group, 2) converting the carboxylic acid moiety to the oxadiazolone ring in the same method as [D-2], 3) deprotecting the protective group in a well-known method, 4) sulfonamidation in the same manner as [D-1].

[Formula 14]

[Step E]

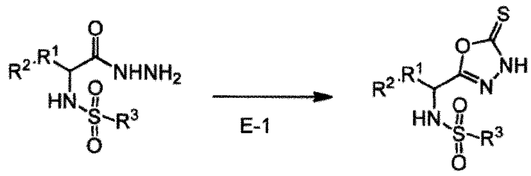


(15) (17)

[In the formula, the symbols have the same meanings as defined above.]

[E-1]

In this step, a compound represented by general formula (17) of the present invention can be prepared by reacting the compound represented by general formula (15) with carbon disulfide.

The base used in this reaction is, for example, alkali metal salts such as sodium hydroxide, potassium hydroxide, organic amines such as triethylamine, alkali metal alkoxides, such as sodium methoxide, sodium ethoxide, sodium tert-butoxide, potassium tert-butoxide, metal amides such as lithium diisopropylamide, and preferably potassium hydroxide.

The amount of the base to be used is, with respect to the compound represented by Formula (15), generally 1 to 20 equivalents, preferably 1 to 5 equivalents. The amount of carbon disulfide is, with respect to the compound represented by Formula (15), generally 1 to 20 equivalents, preferably 1 to 5 equivalents.

The solvent to be used is not particularly limited as long as it does not affect the reaction, for example, organic solvents, water such as alcohols (methanol, ethanol, propanol), ethers (diethyl ether, tetrahydrofuran, 1,4-dioxane, 1,2-dimethoxyethane), halogenated hydrocarbons (dichloromethane, chloroform, 1,2-dichloroethane, carbon tetrachloride), aromatic hydrocarbons (benzene, toluene, xylene, etc.), aliphatic hydrocarbons (hexane, pentane, cyclohexane), amides (DMF, N,N-dimethylacetamide, N-methylpyrrolidinone, and the like, and they can be used singly or as a mixture.

The reaction temperature is 0 to 150°C, preferably between 20 to 100°C. The reaction time is generally from 0.5 to 24 hours, preferably 1.0 to 12 hours. The compound represented by general formula (17) of the present invention can be isolated and purified by well-known separation and purification means.

[Formula 15]

[Step F]

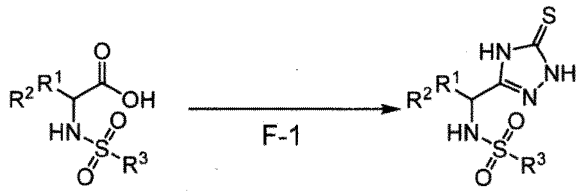


(14) (18)

[In the formula, the symbols have the same meanings as defined above.]

[F-1]

In this step, a compound of general formula (18) can be prepared by condensation and simultaneously cyclization of the compound represented by general formula (1 4) and thiosemicarbazide.

The condensing agent is, for example CDI, dicyclohexylcarbodiimide, 1-ethyl-3-(3-dimethylaminopropyl)carbodiimide hydrochloride and the like, preferably 1-ethyl-3-(3-dimethylaminopropyl)carbodiimide hydrochloride.

The solvent to be used is not particularly limited as long as it does not affect the reaction, for example, organic solvents such as ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride), aromatic hydrocarbons (benzene, toluene, xylene, etc.), aliphatic hydrocarbons (hexane, pentane, cyclohexane, etc.), nitriles (acetonitrile, propionitrile etc.), amides (DMF, N,N-dimethylacetamide, N-methylpyrrolidinone and the like. They may be used singly or as a mixture.

The amount of the condensing agent is, with respect to the compound represented by general formula (14), 1.0 to 50 equivalents, preferably 1 to 5 equivalents. The amount of thiosemicarbazide is, with respect to the compound represented by general formula (14), generally 1 to 100 equivalents, preferably 1.0 to 5.0 equivalents. The base is organic bases such as triethylamine, N,N-diisopropylethylamine, pyridine, 4-dimethylaminopyridine, diazabicycloundecene and the like.

The reaction temperature is -20 to 180°C, preferably 0 to 100°C. The reaction time is usually 0.05 to 24 hours, preferably 0.05 to 6 hours. The compound represented by formula (18) of the present invention can be isolated and purified by well-known separation and purification means as described below.

[Formula 16]

[Step G]

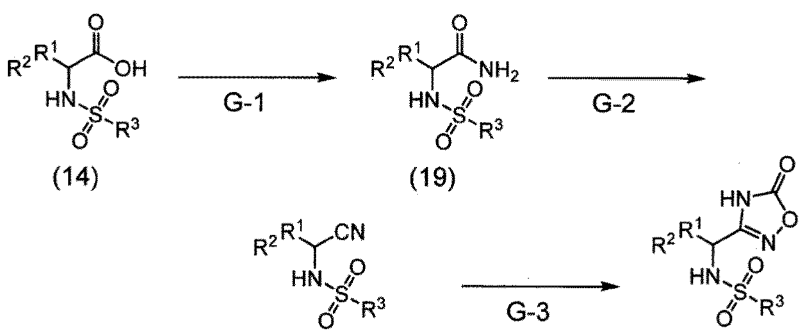


(14) (19) (20) (21)

[In the formula, the symbols have the same meanings as defined above.]

[G-1]

In this step, a compound represented by general formula (19) can be prepared by reacting the carboxylic acid

represented by general formula (14) with a condensation agent and ammonia.

The condensing agent is, for example, 1,1'-carbonyldiimidazole (hereinafter, CDI), dicyclohexylcarbodiimide, 1-ethyl-3-(3-dimethylaminopropyl)carbodiimide hydrochloride and the like, preferably CDI.

The solvent to be used is not particularly limited as long as it does not affect the reaction, for example, organic solvents such as ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride), aromatic hydrocarbons (benzene, toluene, xylene, etc.), aliphatic hydrocarbons (hexane, pentane, cyclohexane, etc.), nitriles (acetonitrile, propionitrile etc.), amides (DMF, N,N-dimethylacetamide, N-methylpyrrolidinone and the like. They may be used singly or as a mixture.

The amount of the condensing agent with respect to the compound represented by general formula (14) is generally 1 to 50 equivalents, preferably 1 to 5 equivalents. Ammonia is used as an aqueous solution or hydrochloric acid salt, and its amount relative to the compound represented by general formula (14) is generally 1 to 100 equivalents, preferably 1.0 to 5.0 equivalents. The bases include, for example, organic bases such as triethylamine, pyridine, 4-dimethylaminopyridine, diazabicycloundecene and the like.

The reaction temperature is -20 to 80°C, preferably 0 to 40°C. The reaction time is usually from 0.05 to 24 hours, preferably 0.05 to 6 hours. The compound represented by general formula (19) can be subjected to the next step with or without isolation and purification by well-known separation and purification means as described below.

[G-2]

In this step, a nitrile represented by general formula (20) can be prepared from the amide compound represented by general formula (19).

Dehydrating agents include, for example, oxalyl chloride, thionyl chloride, cyanuric chloride and the like, preferably cyanuric chloride.

The solvent to be used is not particularly limited as long as it does not affect the reaction, for example, organic solvents such as ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride), aromatic hydrocarbons (benzene, toluene, xylene, etc.), aliphatic hydrocarbons (hexane, pentane, cyclohexane, etc.), nitriles (acetonitrile, propionitrile etc.), amides (DMF, N,N-dimethylacetamide, N-methylpyrrolidinone and the like. They may be used singly or as a mixture.

The amount of dehydrating agent with respect to the compound represented by general formula (19) is usually 1 to 50 equivalents.

The reaction temperature is -20 to 80°C, preferably between 0 to 40°C. The reaction time is usually from 0.05 to 24 hours, preferably from 0.05 to 3 hours. The compound represented by general formula (20) can be subjected to the next step with or without isolation and purification by well-known separation and purification means as described below.

[G-3]

In this step, an amidoxime compound is obtained from the nitrile compound represented by general formula (20) by adding hydroxylamine, and then it reacts with an acylating agent followed by cyclization reaction with application of heat to produce a compound represented by general formula (21).

The amount of the hydroxylamine to be used for preparing amidoxime is generally 1 to 50 equivalents in reaction to the compound represented by general formula (20).

The solvent to be used is not particularly limited as long as it does not affect the reaction, for example, organic solvent such as ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride), aromatic hydrocarbons (benzene, toluene, xylene, etc.), aliphatic hydrocarbons (hexane, pentane, cyclohexane, etc.), nitriles (acetonitrile, propionitrile etc.), amides (DMF, N,N-dimethylacetamide, N-methylpyrrolidinone and the like). They may be used singly or as a mixture.

The reaction temperature is -20 to 100°C, preferably 0 to 60°C. The reaction time is generally from 0.05 to 3 days, preferably 0.05 to 12 hours. The obtained amidoxime compound represented by general formula (20) can be subjected to the next step with or without isolation and purification by well-known separation and purification means as described below.

The acylating agent used for amide oxime is, for example, chloroformate, 2-ethylhexyl, CDI, phosgene, triphosgene and the like, preferably chloroformate 2-ethylhexyl. The base to be used includes organic bases such triethylamine, N,N-diisopropylethylamine, pyridine, 4-dimethylaminopyridine, diazabicycloundecene and the like.

The amount of the acylating agent is usually 1 to 50 equivalents relative to the amide oxime compound, and more preferably about 1 to 3 equivalents.

The solvent to be used is not particularly limited as long as it does not affect the reaction, for example, organic solvents such as ethers (diethyl ether, tetrahydrofuran, 1,4-dioxane, 1,2-dimethoxyethane, etc.), halogenated hydrocarbons (dichloromethane, chloroform, 1,2-dichloroethane, carbon tetrachloride), aromatic hydrocarbons (benzene, toluene, xylene, etc.), aliphatic hydrocarbons (hexane, pentane, cyclohexane, etc.), nitriles (acetonitrile, propionitrile etc.), amides (DMF, N,N-dimethylacetamide, N-methylpyrrolidinone, etc.) and the like), and they can be used singly or as a mixture, and they can be switched to other solvents during the reaction.

The reaction temperature with the acylating agent is to -20, 80°C, preferably 0 to 40°C. The reaction time is generally 0.5 to 24 hours, preferably 0.5 to 3 hours. The reaction temperature used for cyclization reaction of the obtained acylated compound is 0 to 150°C, preferably 0 to 120°C. The reaction time is generally from 0.5 to 24 hours, preferably 0.5 to 12 hours. The obtained compound represented by general formula (21) can be subjected to the next step with or without isolation and purification by well-known separation and purification means as described below.

The compound represented by formula (I) of the present invention and intermediates thereof can be isolated and purified by well-known separation and purification means such as recrystallization, crystallization, distillation, or column chromatography. The sulfonamide compound of formula (I) and synthetic intermediates are usually possible to form a pharmacologically acceptable salt thereof in a well-known manner, and also can be converted to each other.

When optical isomers, stereoisomers, tautomers, or rotary isomers are present in the sulfonamide compound represented by formula (I), the sulfonamide compound represented by formula (I) encompasses these isomers or the mixture thereof. For example, when an optical isomer in the sulfonamide compound represented by formula (I) is present, unless otherwise stated, racemate and an optical isomer resolved from a racemate are also encompassed in the sulfonamide compound represented by formula (I). These isomers can be obtained by a well-known synthetic method, separation means (concentration, solvent extraction, column chromatography, recrystallization and the like) with a single compound, respectively. In the sulfonamide compound represented by formula (I), for example, when $X^1$ = oxygen atom, $X^2$= oxygen atom, $X^3$= NH, there are tautomers as shown below, any of the isomers are also included in the present invention.

[Formula 17]

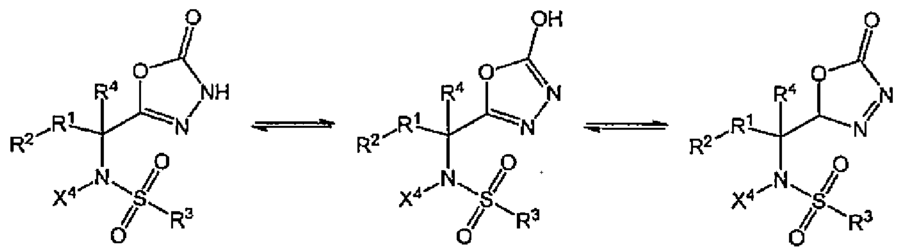

The sulfonamide compound represented by formula (I) or a salt thereof may be amorphous (amorphous) or a crystalline form, and the crystalline form may be a single crystalline form or polymorphic mixture, which are encompassed in the sulfonamide compound represented by formula (I) or a salt thereof. The crystals can be prepared by applying a well-known crystallization method.

Furthermore, the sulfonamide compound represented by formula (I) or a salt thereof can be a solvate (e.g., hydrate etc.) or a non-solvate, both of which are encompassed in the sulfonamide compound represented by formula (I) or a salt thereof. The compounds labeled with isotopes (e.g., deuterium, $^3H$, $^{14}C$, $^{35}S$, $^{125}I$, etc.) and the like are also encompassed in the sulfonamide compound represented by formula (I) or a salt thereof.

Although the prodrugs of the sulfonamide compound represented by formula (I) or a salt thereof are also included in the present invention, the prodrugs refer to the compounds which convert into the sulfonamide compound represented by formula (I) or a salt thereof by a reaction with an enzyme or gastric acid under the physiological condition in the living body, i.e., the compounds which convert into the sulfonamide compound represented by formula (I) or a salt thereof by enzymatic oxidation, reduced, or hydrolysis and the like or the compounds which convert into the sulfonamide compound represented by formula (I) or a salt thereof by gastric acid. Furthermore, a prodrug of the sulfonamide compound represented by formula (I) or a salt thereof may be the compounds which convert into the sulfonamide compound represented by formula (I) or a salt thereof under physiological conditions as described in Hirokawa Shoten 1990 annual "Development of Pharmaceuticals" Volume 7 Molecular Design pages 163-198.

A salt of the sulfonamide compound represented by formula (I) means a salt that is pharmaceutically acceptable.

The sulfonamide compound represented by formula (I) or a salt thereof has an inhibitory activity against RNR The sulfonamide compound represented by formula (I) or a salt thereof is useful as a medicament for prevention or treatment of RNR-related diseases without causing side effects based on the off-target effects of the iron ions requiring protein due to its excellent RNR inhibitory activity and its structure that does not chelate to metal ions.

Use of the sulfonamide compound represented by formula (I) or a salt thereof and other antitumor agent(s) in

combination enhances the antitumor effect. A combination formulation of the sulfonamide compound represented by formula (I) or a salt thereof and other antitumor agent(s) may be one formulation form (i.e., a blending agent) or may be combined administrations in two or more separate formulation forms.

In the present invention, the antitumor effect can be evaluated on the basis of, for example, decrease in tumor volume, stagnant tumor growth, or prolongation of survival periods.

In one embodiment, an antitumor agent comprising the sulfonamide compound represented by formula (I) or a salt thereof and other antitumor agent(s) is provided. Furthermore, in another embodiment, an agent for enhancing an antitumor effect of other antitumor agent(s), comprising the sulfonamide compound represented by formula (I) or a salt thereof as an active ingredient is provided.

The other antitumor agent(s) is not particularly limited but includes antimetabolites, platinum drugs, plant alkaloid drugs, and molecular targeting drugs.

The antimetabolites include 5-fluorouracil (5-FU), 5-fluoro-2'-deoxyuridine (FdUrd), tegafur, tegafur-uracil (e.g., UFT), tegafur-gimeracil-oteracil (e.g., TS-1), pemetrexed, trifluridine, trifluridine-tipiracil hydrochloride (e.g., Lonsurf), fludarabine (or an active metabolite fludarabine nucleoside), cytarabine, gemcitabine, capecitabine, nelarabine, clofarabine, and DNA methylation inhibitors (decitabine, guadecitabine, azacitidine, etc.). 5-Fluorouracil (5-FU), trifluridine, fludarabine (or an active metabolite fludarabine nucleoside), cytarabine, gemcitabine, or a DNA methylation inhibitors (decitabine, guadecitabine, azacitidine, etc.) is preferred, and 5-fluorouracil (5-FU), trifluridine, fludarabine (or an active metabolite fludarabine nucleoside), cytarabine, gemcitabine, decitabine, guadecitabine, or azacitidine is preferred.

The platinum drugs include cisplatin, oxaliplatin, carboplatin, and nedaplatin and are preferably cisplatin, oxaliplatin, or carboplatin.

The plant alkaloid drugs include microtube inhibiting drugs such as paclitaxel, docetaxel, vinblastine, vincristine, vindesine, vinorelbine, and eribulin, and topoisomerase inhibiting drugs such as irinotecan (or an active metabolite SN-38), nogitecan, and etoposide. A topoisomerase inhibiting drug such as irinotecan (or an active metabolite SN-38), nogitecan, and etoposide is preferred, a topoisomerase II inhibiting drug such as etoposide is more preferred, and etoposide is even more preferred.

The molecular targeting drugs include ATR (ataxia telangiectasia and Rad3 related protein) inhibitors, Chkl (checkpoint kinase 1) inhibitors, HSP (heat shock protein) 90 inhibitors, PARP (poly ADP ribose polymerase) inhibitors, EGFR (epidermal growth factor receptor) inhibitors, Her2 inhibitors, VEGFR (vascular endothelial growth factor receptor) inhibitors, PDGFR (platelet-derived growth factor receptor) inhibitors, MET inhibitors, AXL inhibitors, RET inhibitors, FLT3 (fms-related tyrosine kinase 3) inhibitors, KIT inhibitors, CSF1R (colony-stimulating factor 1 receptor) inhibitors, TIE2 (tunica interna endothelial cell kinase 2) inhibitors, and TRKB inhibitors.

The ATR inhibitors include AZD6738, berzosertib, BAY1895344, and VX-803. AZD6738 is preferred.

The Chkl inhibitors include prexasertib, SCH900776, GDC-0575, and CCT245737. Prexasertib or SCH900776 is preferred.

The HSP90 inhibitors include luminespib, ganetespib, onalespib, and 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide. Luminespib is preferred.

The PARP inhibitors include olaparib, rucaparib, niraparib, veliparib, and talazoparib. Olaparib or talazoparib is preferred.

The EGFR inhibitors include small molecule inhibitors such as lapatinib, gefitinib, erlotinib, afatinib, and vandetanib, and anti-EGFR antibodies such as cetuximab and panitumumab. A small molecule inhibitor such as lapatinib and vandetanib is preferred, and lapatinib is more preferred. Furthermore, multikinase inhibitors are also accepted.

The Her2 inhibitors include small molecule inhibitors such as lapatinib, and anti-Her2 antibodies such as trastuzumab, pertuzumab, and trastuzumab emtansine. A small molecule inhibitor such as lapatinib is preferred, and lapatinib is more preferred. Furthermore, multikinase inhibitors are also accepted.

The VEGFR inhibitors are inhibitors of at least one of VEGFR1, VEGFR2, and VEGFR3 and include small molecule inhibitors such as sunitinib, cabozantinib, midostaurin, sorafenib, vandetanib, pazopanib, lenvatinib, and axitinib, and anti-VEGFR antibodies such as ramucirumab. Sunitinib, cabozantinib, or midostaurin is preferred. Furthermore, multikinase inhibitors are also accepted.

The PDGFR inhibitors are PDGFR$\alpha$ and/or PDGFR$\beta$ inhibitors and include sunitinib, midostaurin, pazopanib, lenvatinib, and sorafenib. Sunitinib or midostaurin is preferred. Furthermore, multikinase inhibitors are also accepted.

The MET inhibitors include cabozantinib, crizotinib, and tepotinib. Cabozantinib is preferred. Furthermore, multikinase inhibitors are also accepted.

The AXL inhibitors include cabozantinib and gilteritinib. Cabozantinib is preferred. Furthermore, multikinase inhibitors are also accepted.

The RET inhibitors include sunitinib, cabozantinib, sorafenib, lenvatinib, and vandetanib. Sunitinib or cabozantinib is preferred. Furthermore, multikinase inhibitors are also accepted.

The FLT3 inhibitors include sunitinib, cabozantinib, midostaurin, gilteritinib, and sorafenib. Sunitinib, cabozantinib, or midostaurin is preferred. Furthermore, multikinase inhibitors are also accepted.

The KIT inhibitors include sunitinib, midostaurin, pazopanib, lenvatinib, and sorafenib. Sunitinib or midostaurin is preferred. Furthermore, multikinase inhibitors are also accepted.

The CSF1R inhibitors include sunitinib, BLZ-945, and ARRY-382. Sunitinib is preferred. Furthermore, multikinase inhibitors are also accepted.

The TIE2 inhibitors include cabozantinib. Cabozantinib is preferred. Furthermore, multikinase inhibitors are also accepted.

The TRKB inhibitors include cabozantinib and entrectinib. Cabozantinib is preferred. Furthermore, multikinase inhibitors are also accepted.

"Multikinase inhibitor" herein is a compound having inhibitory activity against two or more kinases, for example, lapatinib having EGFR inhibitory activity and HER2 inhibitory activity.

The other antitumor agent(s) is preferably an antimetabolite, a platinum drug, a plant alkaloid drug, or a molecular targeting drug, more preferably an antimetabolite, a platinum drug, a topoisomerase II inhibiting drug, or a molecular targeting drug, even more preferably an antimetabolite, a platinum drug, a topoisomerase II inhibiting drug, an ATR inhibitor, a Chkl inhibitor, a HSP90 inhibitor, a PARP inhibitor, an EGFR inhibitor, a Her2 inhibitor, a VEGFR inhibitor, a PDGFR inhibitor, a MET inhibitor, an AXL inhibitor, a RET inhibitor, a FLT3 inhibitor, or a KIT inhibitors, even more preferably an antimetabolite, a platinum drug, etoposide, an ATR inhibitor, a Chkl inhibitor, luminespib, olaparib, talazoparib, lapatinib, sunitinib, cabozantinib, or midostaurin, even more preferably an antimetabolite, a platinum drug, etoposide, AZD6738, prexasertib, SCH900776, luminespib, olaparib, talazoparib, lapatinib, sunitinib, cabozantinib, or midostaurin, even more preferably an antimetabolite, cisplatin, oxaliplatin, carboplatin, etoposide, AZD6738, prexasertib, SCH900776, luminespib, olaparib, talazoparib, lapatinib, sunitinib, cabozantinib, or midostaurin, even more preferably 5-fluorouracil (5-FU), trifluridine, fludarabine, cytarabine, gemcitabine, a DNA methylation inhibitor, cisplatin, oxaliplatin, carboplatin, etoposide, AZD6738, prexasertib, SCH900776, luminespib, olaparib, talazoparib, lapatinib, sunitinib, cabozantinib, or midostaurin, even more preferably 5-fluorouracil (5-FU), trifluridine, fludarabine, cytarabine, gemcitabine, decitabine, guadecitabine, azacitidine, cisplatin, oxaliplatin, carboplatin, etoposide, AZD6738, prexasertib, SCH900776, luminespib, olaparib, talazoparib, lapatinib, sunitinib, cabozantinib, or midostaurin.

Tumors of interest in the present invention are not particularly limited as long as an effect of enhancing an antitumor effect is exerted. A tumor on which the sulfonamide compound represented by formula (I) or a salt thereof exerts an antitumor effect is preferred, and an RNR-related malignant tumor is more preferred.

The "RNR-related malignant tumor" includes malignant tumors whose incidence can be decreased or whose symptom is in remission or alleviated and/or completely cured by deleting or suppressing and/or inhibiting functions of RNR Malignant tumors of interest is not particularly limited, head and neck cancer, gastrointestinal cancer (esophageal cancer, gastric cancer, duodenal cancer, liver cancer, biliary tract cancer (gallbladder · bile duct cancer, etc.), pancreatic cancer, colorectal cancer (colon cancer, rectal cancer etc.), etc.), lung cancer (non-small cell lung cancer, small cell lung cancer, mesothelioma, etc.), breast cancer, genital cancer (ovarian cancer, uterine cancer (cervical cancer, endometrial cancer, etc.), etc.), urinary cancer (kidney cancer, bladder cancer, prostate cancer, testicular tumor, etc.), hematopoietic tumors (leukemia, malignant lymphoma, multiple myeloma, etc.), bone and soft tissue tumors, skin cancer, brain tumor and the like. Gastrointestinal cancer (esophageal cancer, gastric cancer, duodenal cancer, liver cancer, biliary tract cancer (gallbladder · bile duct cancer, etc.), pancreatic cancer, colorectal cancer (colon cancer, rectal cancer etc.), etc.), lung cancer (non-small cell lung cancer, small cell lung cancer, mesothelioma, etc.), or hematopoietic tumors (leukemia, malignant lymphoma, multiple myeloma, etc.), are preferred, large intestine cancer, pancreatic cancer, lung cancer (non-small cell lung cancer, small cell lung cancer, mesothelioma, etc.), or hematopoietic tumors (leukemia, malignant lymphoma, multiple myeloma, etc.) are more preferred, and large intestine cancer, pancreatic cancer, lung cancer (non-small cell lung cancer, small cell lung cancer, mesothelioma, etc.), or leukemia is even more preferred.

"RNR" herein includes a human or non-human RNR, preferably a human RNR

When using the sulfonamide compound represented by formula (I) or a salt thereof as a medicine, it is optionally formulated with a pharmaceutically acceptable carrier, and various dosage forms can be adopted depending on the prevention or therapeutic purposes, and the dosage forms may be, for example, any of oral agents, injections, suppositories, ointments, and patches. Since the sulfonamide compound represented by formula (I) or a salt thereof has an excellent oral absorbability, oral agents are preferable. These dosage forms can be prepared by preparation methods commonly known by a person with ordinary skill in the art.

With respect to pharmaceutically acceptable carriers, conventional various organic or inorganic carrier substances are used as pharmaceutical materials, and it is formulated as: excipients, binders, disintegrating agents, lubricants, coloring agents for solid formulations; and solvents, solubilizing agents, suspending agents, isotonizing agents, buffers, soothing agent for liquid formulations and the like. Further, if necessary, pharmaceutical additives can also be used, which include such as preservative agents, antioxidants, coloring agents, sweetening agents, flavoring agents, stabilizing agents.

With respect to the pharmaceutically acceptable carriers and the pharmaceutical additives, in general, they include, for example, as the excipient, lactose, sucrose, sodium chloride, glucose, starch, calcium carbonate, kaolin,

microcrystalline cellulose, silicic acid and the like; as arebinders, water, ethanol, propanol, simple syrup, a glucose solution, a starch solution, a gelatin solution, carboxymethyl cellulose, hydroxypropyl cellulose, hydroxypropyl starch, methyl cellulose, ethyl cellulose, shellac, calcium phosphate, polyvinylpyrrolidone, and the like; as disintegrants, dry starch, sodium alginate, agar powder, sodium hydrogen carbonate, calcium carbonate, sodium lauryl sulfate, stearic acid monoglyceride, lactose and the like; as lubricants, purified talc stearate, borax, polyethylene glycol and the like; as colorant, titanium oxide, iron oxide and the like; as flavoring agents, sucrose, orange peel, citric acid, tartaric acid and the like.

When preparing an oral solid formulation, it can be prepared by adding an excipient to the sulfonamide compound represented by formula (I) or the other antitumor agent(s), and if necessary, further adding binders, disintegrants, lubricants, colorants, or flavoring agents and the like, followed by formulating into tablets, coated tablets, granules, powders, capsules and the like.

When preparing injectable forms, it can be prepared by adding pH control agents, buffers, stabilizers, isotonic agents, local anesthetic agents and the like to the sulfonamide compound represented by formula (I) or the other antitumor agent(s), followed by formulating into subcutaneous, intramuscular and intravenous injections with a conventional manner.

A preparation of the other antitumor agent(s) also includes DDS (drug delivery system) formulations thereof. For example, "paclitaxel" includes albumin-bound paclitaxel (e.g., Abraxane) and paclitaxel micelle (e.g., NK105), and the like, and "cisplatin" includes cisplatin micelle (e.g., NC-6004) and the like.

The amount of the sulfonamide compound represented by formula (I) to be formulated in each dosage unit forms described above can be, in general, per dosage unit form, 0.05 to 1000 mg for the oral dosage, about 0.01 to 500 mg for injection, 1 to 1000 mg for suppositories with the proviso that they may be altered depending on the symptoms of the patients to be applied or its dosage forms.

Further, the daily dose of a drug with the dosage form is, with respect to the sulfonamide compound represented by formula (I), 0.05 to 5000 mg, preferably 0.1 to 2000 mg per day for adult (body weight 50 kg), and preferably the aforementioned amount is administered once or 2 to 3 times a day with the proviso that they may be altered depending on symptoms of the patients, weight, age, or gender and the like.

The schedule of administration of the sulfonamide compound represented by general formula (I) or a salt thereof and the other antitumor agent(s) is appropriately selected within a range in which each active ingredient exerts an antitumor effect, and the active ingredients are administered concurrently or separately in a staggered manner. For separate administration, either of the active ingredients may be administered first.

The sulfonamide compound represented by general formula (I) or a salt thereof and the other antitumor agent(s) may be formulated into two or more of dosage forms of each active ingredient or may be formulated collectively into one dosage form, on the basis of the dosage form or the schedule of administration of each active ingredient. Further, the preparations may be manufactured and distributed in one package suitable for combined use, or may be manufactured and sold in separate packages.

## Examples

The present invention is described below in more detail with examples and test examples, but the present invention is not intended to be limited to these examples.

Various reagents used in the examples were commercially available products, unless otherwise stated. Biotage Ltd. SNAP-ULTRA (registered trademark) Silica prepacked column was used for a silica gel column chromatography, or Biotage made SNAP KP-C18-HS (registered trademark) Silica prepacked column was used for a reverse phase silica gel column chromatography. HPLC purified by preparative reverse phase column chromatography was performed under the following conditions. Injection volume and gradient was carried out appropriately.

Column: YMC-Actus Triart C18, 30 × 50 mm, 5 μm
UV detection: 254 nm
Column flow rate: 40 mL / min
Mobile phase: water / acetonitrile (0.1% formic acid)
Injection amount: 1.0 mL
Gradient: water / acetonitrile (10 % to 90 %)

AL400 (400MHz; JEOL (JEOL)) and Mercury400 (400MHz; Agilent Technologies) were used for NMR spectra, and tetramethylsilane was used as an internal standard when tetramethylsilane was included in the heavy solvent, otherwise it was measured using NMR solvent as an internal standard, showing all δ value in ppm. Furthermore, LCMS spectra were measured under the following conditions using a Waters made ACQUITY SQD (quadrupole). Column: Waters made ACQUITY UPLC (registered trademark) BEH C18, 2.1 × 50 mm, 1.7 μm

MS detection: ESI negative
UV detection: 254 and 280 nm
Column flow rate: 0.5 mL / min
Mobile phase: water / acetonitrile (0.1 % formic acid)
Injection amount: 1 μL

Gradient (table 1)

| Time (min) | Water | Acetonitrile |
|---|---|---|
| 0 | 95 | 5 |
| 0.1 | 95 | 5 |
| 2.1 | 5 | 95 |
| 3.0 | STOP | |

The meanings of the abbreviations are shown below.

s: singlet
d: doublet
t: triplet
q: quartet
dd: double doublet
dt: double triplet
td: triple doublet
tt: triple triplet
ddd: double double doublet
ddt: double double triplet
dtd: double triple doublet
tdd: triple-double doublet
m: multiplet
br: broad
brs: broad singlet
DMSO-$d_6$: deuterated dimethyl sulfoxide
$CDCl_3$: heavy chloroform
$CD_3OD$: heavy methanol
CDI: 1,1'-carboxymethyl sulfonyl diimidazole
DAST: N,N-diethylaminosulfur trifluoride
DIBAL-H: diisobutylaluminum hydride
DMF: dimethylformamide
DMSO: dimethylsulfoxide
THF: Tetrahydrofuran
WSC = EDCI = 1-ethyl-3-(3-dimethylaminopropyl)carbodiimide
HOBt= 1-hydroxybenzotriazole

[Reference Example A1 Synthesis of 2-(1-bromoethyl)-1-fluoro-3,4-dimethylbenzene]

[Formula 18]

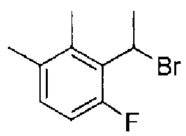

(Step 1) 1-(6-fluoro-2,3-dimethylphenyl)ethanol

After dropping a diethyl ether solution of methylmagnesium bromide (3.0 M, 70 mL) to a THF solution of 6-fluoro-2,3-dimethyl-benzaldehyde (22.0g) (300mL) at 0°C, the reaction mixture was stirred at room temperature for 1 hour. Under ice-bath condition, a saturated aqueous ammonium chloride solution (150 mL) was added dropwise, and ethyl acetate (200 mL) was added, and the resultant was separated into different layers. The organic layer was successively washed with HCl (1M, 200 mL), water (200 mL) and saline (200 mL), and then dried over anhydrous magnesium sulfate, and concentrated under reduced pressure to obtain 1-(6-fluoro-2,3 dimethylphenyl)ethanol (23.7 g).

(Step 2)

Phosphorus tribromide (26.5 mL) was added dropwise at 0°C to a chloroform solution (120 mL) of 1-(6-fluoro-2,3-dimethylphenyl)ethanol (23.7 g) obtained in the above Step 1, and the reaction solution was stirred for 30 minutes at 0°C. The reaction mixture was added to an ice-cold saturated aqueous sodium bicarbonate (1L). After chloroform (500 mL) was added to the mixture, the resultant was separated into different layers, and the organic layer was successively washed with water (200 mL) and saline (200 mL). The organic layer was dried over anhydrous magnesium sulfate to give the title compound (29.5 g) by concentrating under reduced pressure.

[Reference Example A2 to A41]

Aldehyde and methylmagnesium bromide were reacted together as starting materials in the same manner as in Reference Example A1, Step 1 and Step 2, and then the resultant was reacted with phosphorus tribromide to obtain the compounds of Reference Examples A2 to A41. However, the compounds of Reference Examples A40 and A41 were obtained in the same procedure using ethylmagnesium bromide and methyl iodide-d3-magnesium respectively instead of methylmagnesium bromide.

[Table 1]

| Reference Example | Starting Material | Synthesized Compound | Reference Example | Starting Material | Synthesized Compound |
|---|---|---|---|---|---|
| A2 | 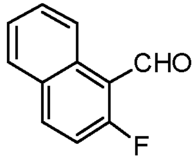 | 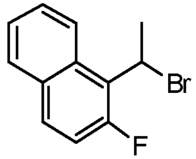 | A9 | 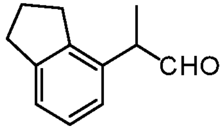 | 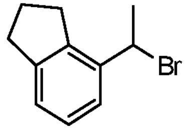 |
| A3 | 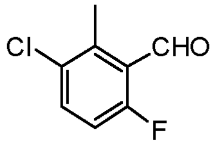 | 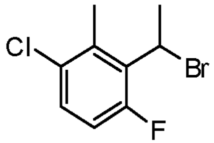 | A10 | 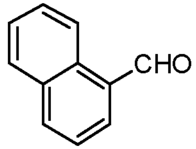 | 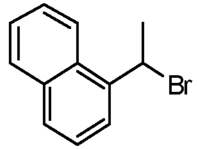 |
| A4 | 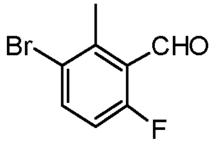 | 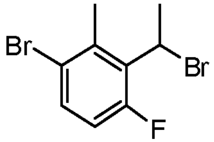 | A11 | 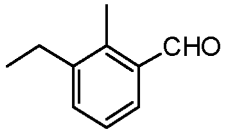 | 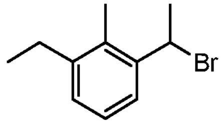 |
| A5 | 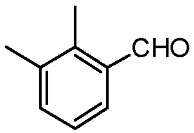 | 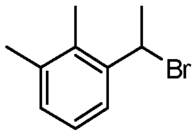 | A12 | 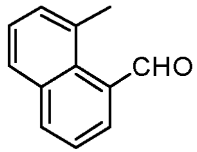 | 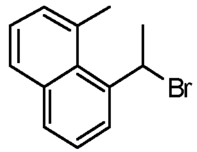 |
| A6 | 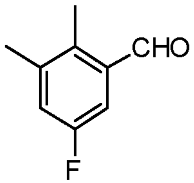 | 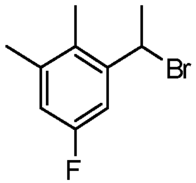 | A13 | 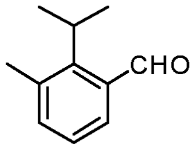 | 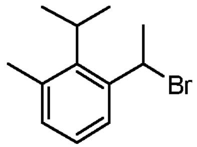 |
| A7 | 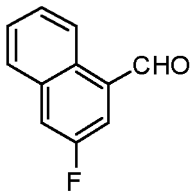 | 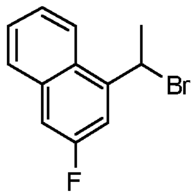 | A14 | 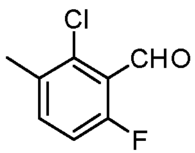 | 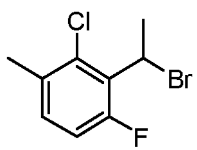 |

(continued)

| Reference Example | Starting Material | Synthesized Compound | Reference Example | Starting Material | Synthesized Compound |
|---|---|---|---|---|---|
| A8 | 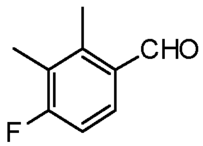 | 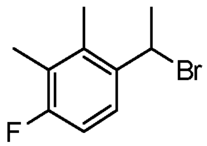 | A15 | 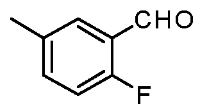 | 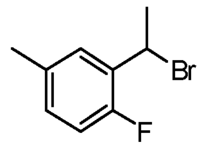 |
| A16 | 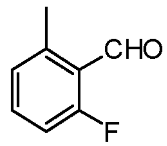 | 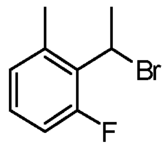 | A23 | 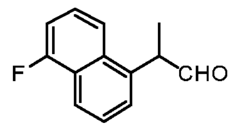 | 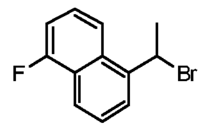 |
| A17 | 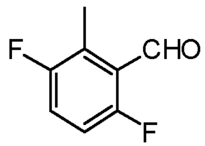 | 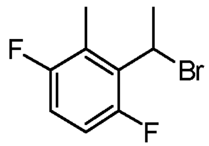 | A24 | 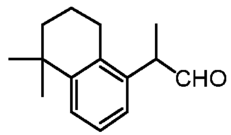 | 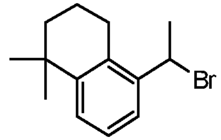 |
| A18 | 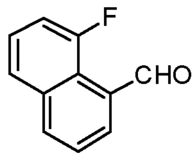 | 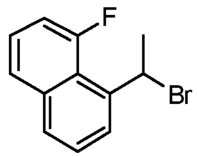 | A25 | 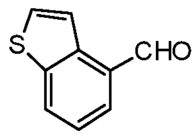 | 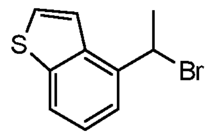 |
| A19 | 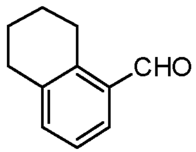 | 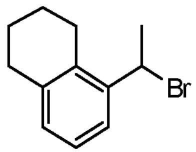 | A26 | 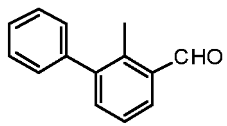 | 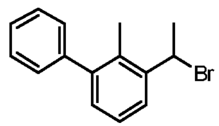 |
| A20 | 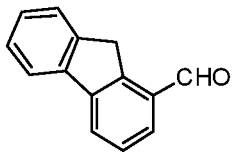 | 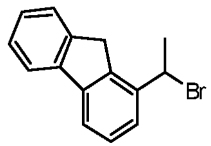 | A27 | 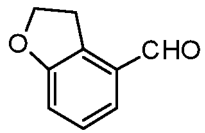 | 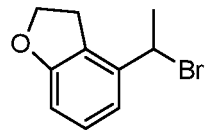 |

(continued)

| Reference Example | Starting Material | Synthesized Compound | Reference Example | Starting Material | Synthesized Compound |
|---|---|---|---|---|---|
| A21 | 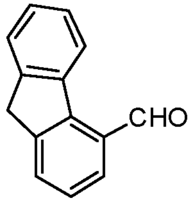 | 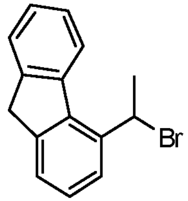 | A28 | 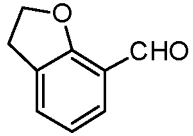 | 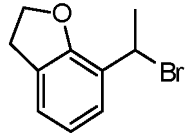 |
| A22 | 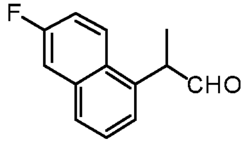 | 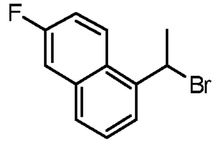 | A29 | 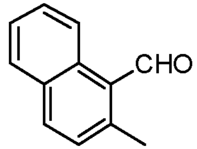 | 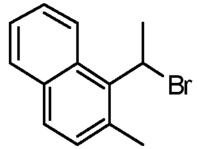 |
| A30 | 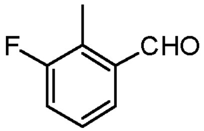 | 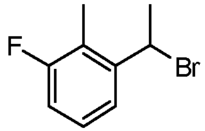 | A37 | 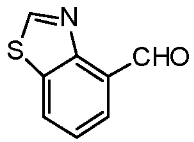 | 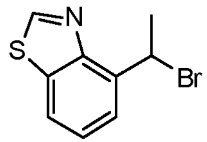 |
| A31 | 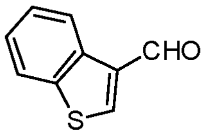 | 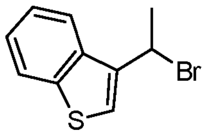 | A38 | 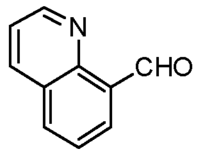 | 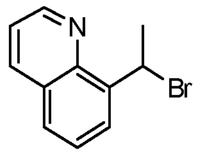 |
| A32 | 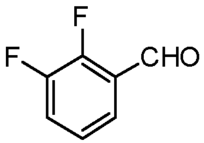 | 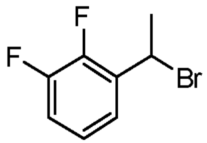 | A39 | 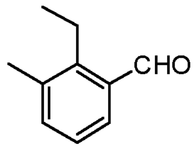 | 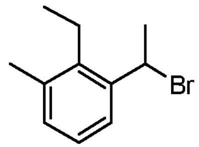 |
| A33 | 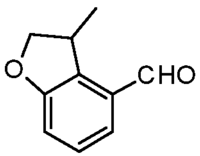 | 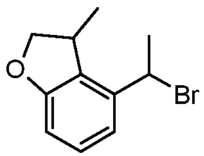 | A40 | 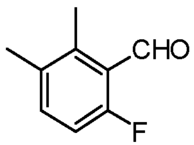 | 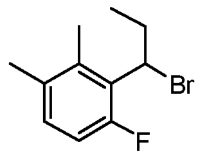 |

(continued)

| Reference Example | Starting Material | Synthesized Compound | Reference Example | Starting Material | Synthesized Compound |
|---|---|---|---|---|---|
| A34 | 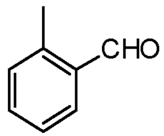 | 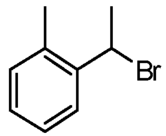 | A41 | 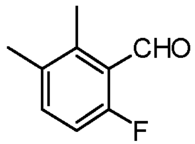 | 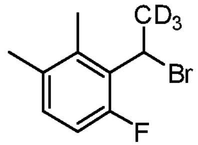 |
| A35 | 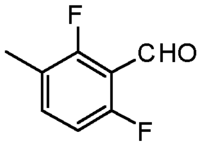 | 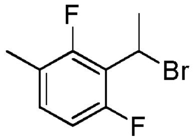 | | | |
| A36 | 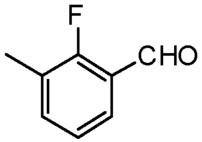 | 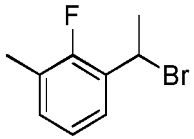 | | | |

[Reference Example B1 Synthesis of 2-(1-bromoethyl)-4-ethyl-1-fluoro-3-methylbenzene]

[Formula 19]

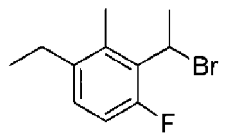

(Step 1) 2-bromo-3-ethyl-6-fluorobenzaldehyde

To a THF solution (150 mL) of 2-bromo-1-ethyl-4-fluorobenzene (14.4 g), a THF solution of lithium diisopropylamide (1.5 M, 54 mL) was added dropwise at -78°C. After stirring the reaction solution for 30 minutes, DMF (6.5 mL) was added and the mixture was further stirred for 20 minutes. Water (50 mL) and hydrochloric acid (6 M, 50 mL) were successively added dropwise to the reaction solution, and the mixture was extracted twice with hexane (100 mL). The combined organic layer was washed with saturated saline (50 mL) twice, dried over anhydrous sodium sulfate, and concentrated under reduced pressure, and 2-bromo-3-ethyl-6-fluorobenzaldehyde (14.5 g) was obtained.

(Step 2) 3-ethyl-6-fluoro-2-methylbenzaldehyde

To a 1,4-dioxane solution (200 mL) of 2-bromo-3-ethyl-6-fluorobenzaldehyde obtained from Step 1 above (14.5 g), water (90 mL), tripotassium phosphate (32.0 g), methylboronic acid (6.4 g) and [bis (diphenylphosphino) ferrocene] palladium (II) dichloride dichloromethane additive (1.75 g) were added, and the reaction solution was heated under reflux at 110°C for 2 hours. The reaction solution was allowed to cool to room temperature, and the mixture was further stirred for 2 hours after hexane (90 mL) was added. The reaction solution was filtered through CELITE, and the filtrate was separated after the residue was washed with hexane. The organic layer was washed twice with saturated saline (100 mL), and after being dried over anhydrous sodium sulfate, it was concentrated under reduced pressure. The residue was purified by silica gel column chromatography (eluent: hexane / ethyl acetate), and 3-ethyl-6-fluoro-2-methylbenzaldehyde (8.4 g) was obtained.

(Step 3)

According to the methods of Reference Example A1 Steps 1 and 2, using 3-ethyl-6-fluoro-2-methylbenzaldehyde (8.4 g) obtained in the above Step 2, the same operation was carried out to obtain the title compound.

[Reference Examples B2 to B6]

According to the methods of Reference Example B1 Steps 1 and 2 and Reference Example A1 Steps 1 and 2, the following compounds of Reference Examples B2 to B5 were synthesized. Also, according to the methods of Reference Example B1 Step 1, and Reference Example A1 Steps 1 and 2, the compound of Reference Example B6 was synthesized.

[Table 2]

| Reference Example | Starting Material | Synthesized Compound | Reference Example | Starting Material | Synthesized Compound |
|---|---|---|---|---|---|
| B2 | 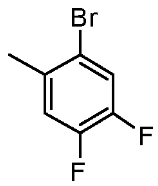 | 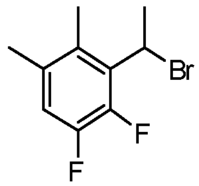 | B5 | 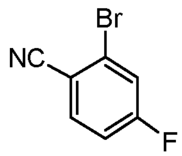 | 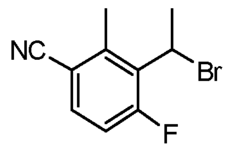 |
| B3 | 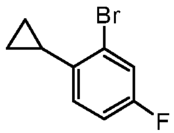 | 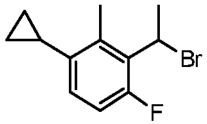 | B6 | 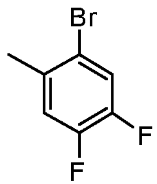 | 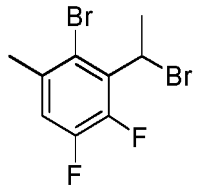 |
| B4 | 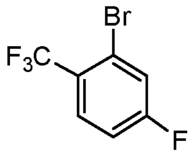 | 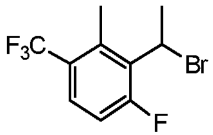 | | | |

[Reference Example C1 Synthesis of 7-(1-chloroethyl)-1-methyl-2,3-dihydro-1H-indene]

[Formula 20]

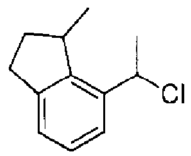

Sodium borohydride (261 mg) was added to a methanol solution (5.0 mL) of 1-(3-methyl-2,3-dihydro-1H-inden-4-yl)ethanone (1.0 g), and the mixture was stirred at room temperature for 30 minutes. The reaction solution was added to water (10 mL) and then extracted twice with ethyl acetate (20 mL). The combined organic layer was washed with saturated saline (20 mL), dried over anhydrous sodium sulfate, and concentrated under reduced pressure. The obtained residue was dissolved in dichloromethane (5.0 mL), thionyl chloride (2.0 mL) was added at room temperature, and the reaction solution was stirred at 50°C for 30 minutes. Water was added to the reaction solution, and the mixture was extracted twice with ethyl acetate (20 mL). The combined organic layer was washed with saturated saline (20 mL), dried over anhydrous sodium sulfate, and concentrated under reduced pressure to give the title compound (1.1 g).

[Reference examples C2 to C4]

According to the method of Reference Example C1, the following compounds of Reference Examples C2 to C4 were synthesized.

[Table 3]

| Reference Example | Starting Material | Synthesized Compound |
|---|---|---|
| C2 | 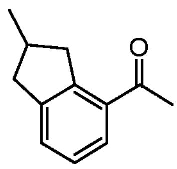 | 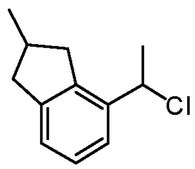 |
| C3 | 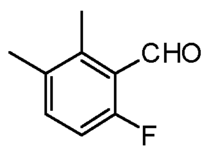 | 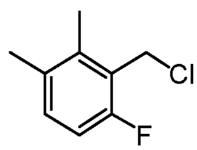 |
| C4 | 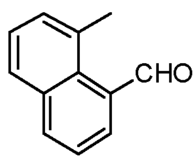 | 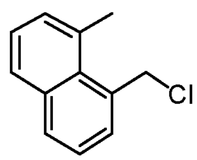 |

[Reference Example D1 Synthesis of (2S, 3R)-2-amino-3-(6-fluoro-2,3-dimethylphenyl)butanoic acid]

[Formula 21]

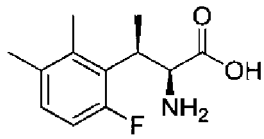

A DMF solution (50 mL) of 2-(1-bromoethyl)-1-fluoro-3,4-dimethylbenzene (14.0 g) obtained in Reference Example A1 was added dropwise to a DMF solution (50 mL) of (S)-2-[o-[(N-benzylprolyl)amino]phenyl]-benzylideneamino-acetate (2-)-N,N,N-nickel (II) (14.5g), and potassium hydroxide (16.3 g), and the mixture was stirred at the same temperature for 1 hour. A saturated ammonium chloride solution (50 mL) and ethyl acetate (50 mL) were added to the reaction solution, the layers were separated, and the aqueous layer was extracted twice with ethyl acetate (50 mL). The combined organic layers were washed successively with water (50 mL) and saturated saline (50 mL), dried over anhydrous sodium sulfate, and concentrated under reduced pressure. The residue was purified by silica gel column chromatography (eluent: ethyl acetate / hexane). The obtained compound was dissolved in methanol (120 mL), hydrochloric acid (3 M, 90 mL) was added, and the mixture was stirred at 80°C for 45 minutes. Methanol was distilled off under reduced pressure, and chloroform (50 mL) and water (50 mL) were added to the residue. The aqueous layer was washed with chloroform (50 m L) and concentrated under reduced pressure. The residue was purified by reverse phase silica gel column chromatography (methanol / water) to give the title compound (2.0 g). $^1$H NMR ($CD_3OD$) $\delta$: 7.03 (dd, J=8.2, 5.7 Hz, 1H), 6.79 (dd, J=11.7, 8.4 Hz, 1H), 3.74-3.87 (m, 2H), 2.29 (s, 3H), 2.25 (s, 3H), 1.40 (dd, J=6.8, 2.4 Hz, 3H)

[Reference examples D2 to D58]

After the alkylating agent obtained in Reference Examples A2 to A41, Reference Examples B1 to B6, and Reference Examples C1 to C4 and (S)-2-[o-[(N-benzylprolyl)amino]phenyl]-benzylideneamino-acetate (2-)-N,N,N-nickel (II) were reacted, the compounds of Reference Examples D2 to D58 shown below were prepared by acid hydrolysis. However, for the compound of Reference Example D56, 6-fluoro-2,3-dimethylbenzaldehyde was used as a starting material, and the compounds of Reference Examples D57 and 58 were prepared by the same method by using (R)-2-[o-[(N-benzylprolyl)amino] phenyl]-benzylideneamino-acetate (2-)-N,N,N-nickel (II) instead of (S)-2-[o-[(N-benzylprolyl)amino]phenyl]-benzylideneamino-acetate (2-)-N,N,N-nickel (II).

[Table 4]

| Reference Example | Starting Material (Reference example number or structural formula) | Synthesized Compound | Reference Example | Starting Material (Reference example number or structural formula) | Synthesized Compound |
|---|---|---|---|---|---|
| D2 | A1 | 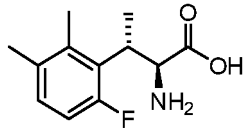 | D8 | A7 | 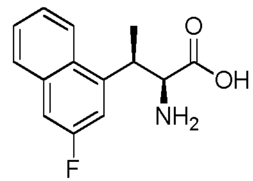 |
| D3 | A2 | 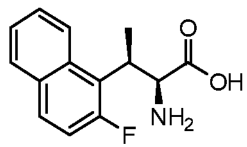 | D9 | A8 | 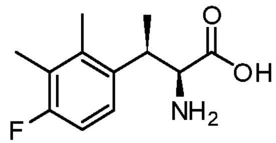 |
| D4 | A3 | 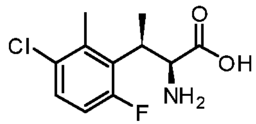 | D10 | A9 | 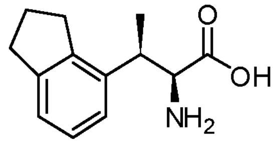 |
| D5 | A4 | 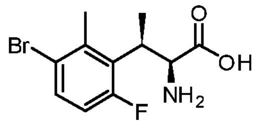 | D11 | A10 | 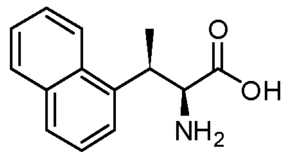 |
| D6 | A5 | 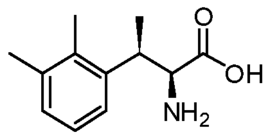 | D12 | A11 | 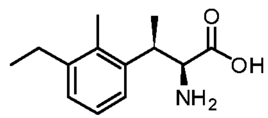 |
| D7 | A6 | 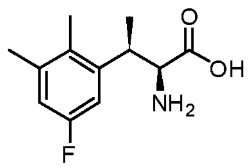 | D13 | A12 | 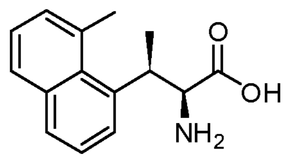 |

(continued)

| Reference Example | Starting Material (Reference example number or structural formula) | Synthesized Compound | Reference Example | Starting Material (Reference example number or structural formula) | Synthesized Compound |
|---|---|---|---|---|---|
| Reference Example | Starting Material (Reference example number or structural formula) | Synthesized Compound | Reference Example | Starting Material (Reference example number or structural formula) | Synthesized Compound |
| D14 | A13 | 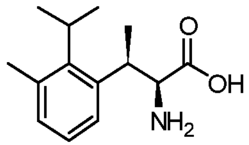 | D20 | A18 | 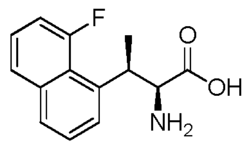 |
| D15 | A14 | 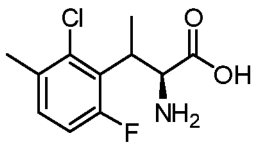 | D21 | A19 | 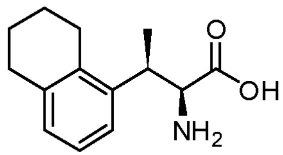 |
| D16 | A15 | 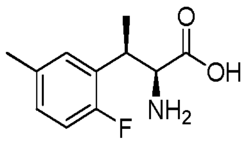 | D22 | A20 | 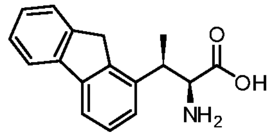 |
| D17 | A16 | 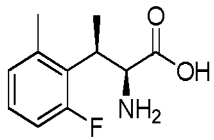 | D23 | A21 | 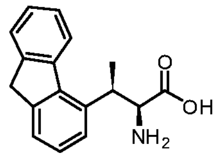 |
| D18 | A16 | 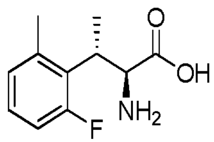 | D24 | A22 | 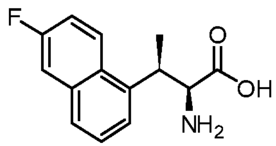 |

(continued)

| Reference Example | Starting Material (Reference example number or structural formula) | Synthesized Compound | Reference Example | Starting Material (Reference example number or structural formula) | Synthesized Compound |
|---|---|---|---|---|---|
| D19 | A17 | 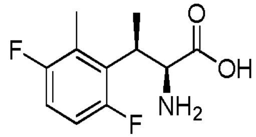 | D25 | A23 | 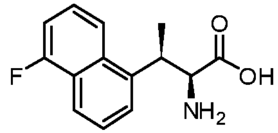 |

| Reference Example | Starting Material (Reference example number or structural formula) | Synthesized Compound | Reference Example | Starting Material (Reference example number or structural formula) | Synthesized Compound |
|---|---|---|---|---|---|
| D26 | A24 | 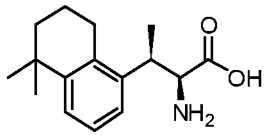 | D32 | A30 | 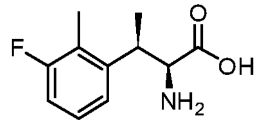 |
| D27 | A25 | 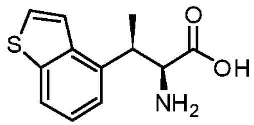 | D33 | A31 | 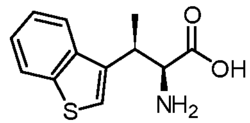 |
| D28 | A26 | 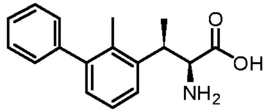 | D34 | A32 | 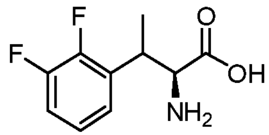 |
| D29 | A27 | 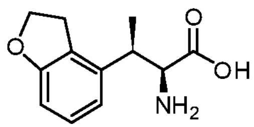 | D35 | A33 | 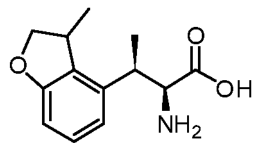 |

(continued)

| Reference Example | Starting Material (Reference example number or structural formula) | Synthesized Compound | Reference Example | Starting Material (Reference example number or structural formula) | Synthesized Compound |
|---|---|---|---|---|---|
| D30 | A28 | 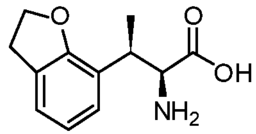 | D36 | A33 | 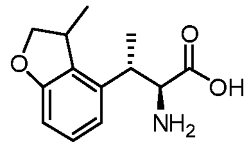 |
| D31 | A29 | 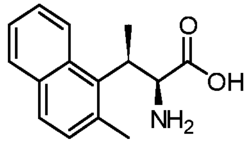 | D37 | A34 | 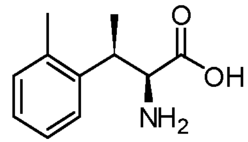 |

| Reference Example | Starting Material (Reference example number or structural formula) | Synthesized Compound | Reference Example | Starting Material (Reference example number or structural formula) | Synthesized Compound |
|---|---|---|---|---|---|
| D38 | A35 | 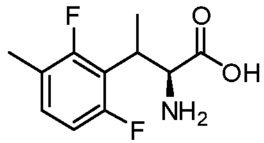 | D45 | B1 | 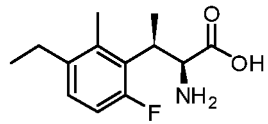 |
| D39 | A36 | 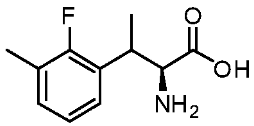 | D46 | B2 | 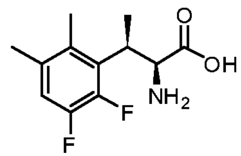 |
| D40 | A37 | 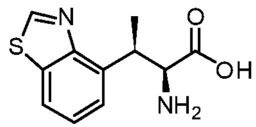 | D47 | B3 | 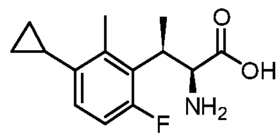 |

(continued)

| Reference Example | Starting Material (Reference example number or structural formula) | Synthesized Compound | Reference Example | Starting Material (Reference example number or structural formula) | Synthesized Compound |
|---|---|---|---|---|---|
| D41 | A38 | 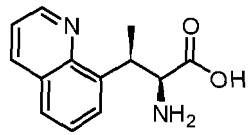 | D48 | B4 | 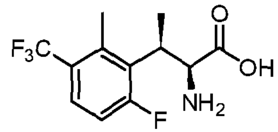 |
| D42 | A39 | 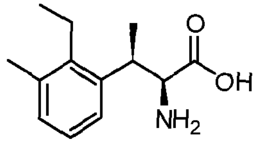 | D49 | B5 | 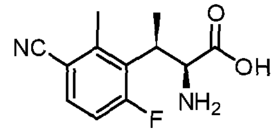 |
| D43 | A40 | 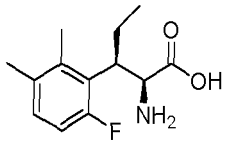 | D50 | B6 | 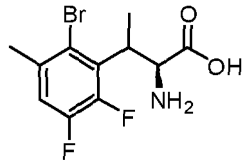 |
| D44 | A41 | 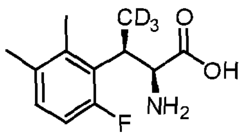 | D51 | C1 | 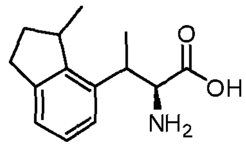 |

| Reference Example | Starting Material (Reference example number or structural formula) | Synthesized Compound | Reference Example | Starting Material (Reference example number or structural formula) | Synthesized Compound |
|---|---|---|---|---|---|
| D52 | C1 | 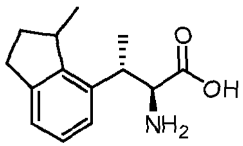 | D56 | 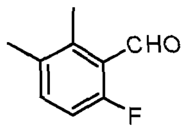 | 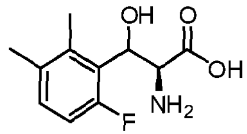 |

(continued)

| Reference Example | Starting Material (Reference example number or structural formula) | Synthesized Compound | Reference Example | Starting Material (Reference example number or structural formula) | Synthesized Compound |
|---|---|---|---|---|---|
| D53 | C2 | 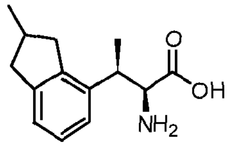 | D57 | A1 | 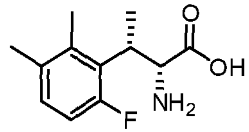 |
| D54 | C3 | 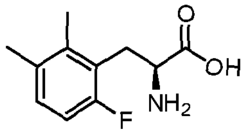 | D58 | A1 | 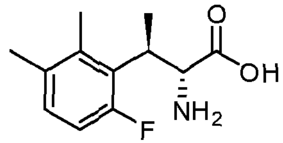 |
| D55 | C4 | 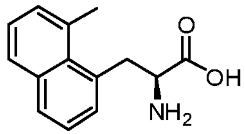 | | | |

[Reference Example D59 Synthesis of 2-Amino-3-(6-fluoro-2,3-dimethylphenyl)-3-methylbutanoic acid monohydrochloride]

[Formula 22]

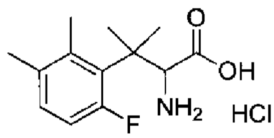

(Step 1) 2-(6-fluoro-2,3-dimethylphenyl)-2-methylpropanal

2-(6-fluoro-2,3-dimethylphenyl)-2-methylpropanenitrile (700 mg) was dissolved in dichloromethane (35 mL) and cooled to -78°C. A toluene solution (1.0 M, 10 mL) of diisobutylaluminum hydride was added, and the reaction solution was stirred for 1 hour at the same temperature. Methanol (5.0 mL) and CELITE (20 g) were sequentially added, and the mixture was stirred at room temperature for 1 hour. The reaction solution was filtered through CELITE, washed with hexane / ethyl acetate = 1/1 (30 mL), and the filtrate was concentrated under reduced pressure. The residue was purified by silica gel column chromatography (eluent: Hexane / ethyl acetate) to obtain 2-(6-fluoro-2,3-dimethylphenyl)-2-methylpropanal (400 mg).

(Step 2) 2-amino-3-(6-fluoro-2,3-dimethylphenyl)-3-methylbutanonitrile

2-(6-fluoro-2,3-dimethylphenyl)-2-methylpropanal (400 mg) obtained in the above Step 1 was dissolved in methanol (7.0 mL) and water (10 ml), 28% aqueous ammonia (280 μL), potassium cyanide (130 mg), and ammonium chloride (110 mg) were added, and the reaction solution was stirred for 12 hours at 70°C. A saturated aqueous sodium hydrogen carbonate solution (5.0 mL) was added to the reaction solution, and the mixture was extracted with ethyl acetate (20 mL). The organic layer was washed with saturated saline (20 mL), dried over anhydrous sodium sulfate, and concentrated under reduced pressure. The residue was purified by silica gel column chromatography (eluent: hexane / ethyl acetate) to obtain 2-amino-3-(6-fluoro-2,3-dimethylphenyl)-3-methylbutanonitrile (380 mg).

(Step 3)

2-amino-3-(6-fluoro-2,3-dimethylphenyl)-3-methylbutanonitrile (380 mg) obtained from the above Step 2 was dissolved in hydrochloric acid (12M, 5.0 mL), and the reaction solution was stirred for 12 hours at 100°C. The reaction solution was cooled to room temperature and was concentrated under reduced pressure to obtain the title compound (300 mg).

[Reference Example D60 Synthesis of 2-Amino-2-(1-(6-fluoro-2,3-dimethylphenyl)cyclopropyl)acetic acid monohydrochloride]

[Formula 23]

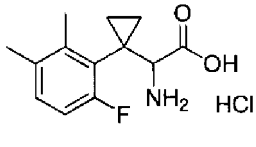

The title compound was synthesized according to the method of Reference Example D59, using 1-(6-fluoro-2,3-dimethylphenyl)cyclopropanecarbonitrile instead of 2-(6-fluoro-2,3-dimethylphenyl)-2-methylpropanenitrile.

[Reference Example D61 Synthesis of 2-Amino-3-(6-fluoro-2,3-dimethylphenyl)-3-butenoic acid monohydrochloride]

[Formula 24]

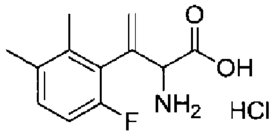

(Step 1) 2-(6-fluoro-2,3-dimethyl)-2-hydroxy-propanenitrile

In dichloromethane (20 mL) solution of 1-(6-fluoro-2,3-dimethylphenyl)ethanone (1.3 g), zinc iodide (480 mg) and trimethylsilyl cyanide (2.0 mL) were added, and the reaction mixture was stirred for 12 hours at room temperature. An aqueous solution of sodium hydroxide (2 M, 10 mL) was added to the reaction solution, and the mixture was extracted with ethyl acetate / hexane = 1/1 (20 mL). The organic layer was washed with hydrochloric acid (2 M, 20 mL) and saturated saline (20 mL) in this order, dried over anhydrous sodium sulfate, and concentrated under reduced pressure. The residue was purified by silica gel column chromatography (eluent: hexane / ethyl acetate) to obtain 2-(6-fluoro-2,3-dimethyl)-2-hydroxy-propanenitrile (1.4 g).

(Step 2) 2-fluoro-2-(6-fluoro-2,3-dimethylphenyl)propanenitrile

To dichloromethane solution (5.0 mL) of 2-(6-fluoro-2,3-dimethyl)-2-hydroxy-propanenitrile (170 mg) obtained from the above Step 1, DAST (150 μL) was added, and the reaction solution was stirred at room temperature for 12 hours. A saturated aqueous sodium hydrogen carbonate solution (10 mL) was added to the reaction solution, and the mixture was extracted with ethyl acetate / hexane = 1/1 (20 mL). The organic layer was washed with saturated saline (10 mL), dried over anhydrous sodium sulfate, and concentrated under reduced pressure. The residue was purified by silica gel column chromatography (eluent: hexane / ethyl acetate) to obtain 2-fluoro-2-(6-fluoro-2,3-dimethylphenyl)propanenitrile (100 mg).

(Step 3) 2-amino-3-fluoro-3-(6-fluoro-2,3-dimethylphenyl)-butanenitrile

From 2-fluoro-2-(6-fluoro-2,3-dimethylphenyl)propanenitrile obtained in the above Step 2, according to the method of Reference Example D59 Steps 1-2, 2-amino-3-fluoro-3-(6-fluoro-2,3-dimethylphenyl)-butanenitrile was obtained.

(Step 4) 2-amino-3-(6-fluoro-2,3-dimethylphenyl)-3-butenoic acid monohydrochloride

2-Amino-3-fluoro-3-(6-fluoro-2,3-dimethylphenyl)-butanenitrile (460 mg) obtained in the above Step 3 was dissolved in hydrochloric acid (12 M, 3.0 mL), and the mixture was stirred for 12 hours at 100°C. The mixture was cooled to room temperature and concentrated under reduced pressure to obtain the title compound.

[Reference Example E1 Synthesis of 5-chloro-8-(chlorosulfonyl)-4-methyl-d 3-chroman-4-yl acetate]

[Formula 25]

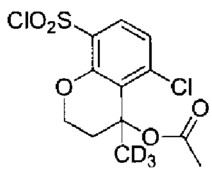

(Step 1) 8-bromo-5-chloro-4-methylchroman-4-ol

THF (50 mL) was added to a diethyl ethyl ether solution (1.0 M, 63 mL) of methyl iodide-d3-magnesium, and a THF solution (50 mL) of 8-bromo-5-chlorochroman-4-one (7.5 g) was added dropwise at room temperature. The reaction solution was stirred for 10 minutes at the same temperature, in an ice bath, hydrochloric acid (1M, 50 mL) was slowly added dropwise, and ethyl acetate (50 mL) was added to separate layers. The aqueous layer was extracted with ethyl acetate (50 mL), and the combined organic layer was washed with saturated saline (50 mL), dried over anhydrous sodium sulfate, and concentrated under reduced pressure. The residue was purified by silica gel column chromatography (eluent: hexane / ethyl acetate) to obtain 8-bromo-5-chloro-4-methylchroman-4-ol (7.7 g).

(Step 2) 8-bromo-5-chloro-4-methyl-d3-chroman-4-yl acetate

To an anhydrous acetic acid solution (100 mL) of 8-bromo-5-chloro-4-methylchroman-4-ol (7.7 g) obtained in the above Step 1, an acetonitrile solution (12 mL) of scandium trifluoromethanesulfonate (III) (340 mg) was added dropwise at -40°C, and the reaction solution was stirred for 30 minutes at the same temperature. A saturated aqueous sodium hydrogen carbonate solution (100 mL) and ethyl acetate (100 mL) were sequentially added to the reaction solution, and the layers were separated. The aqueous layer was extracted with ethyl acetate (100 mL), and the combined organic layers were washed twice with a saturated aqueous sodium hydrogen carbonate solution (100 mL) and once with saturated saline (100 mL). The organic layer was dried over anhydrous sodium sulfate and concentrated under reduced pressure. The residue was purified by silica gel column chromatography (eluent: hexane / ethyl acetate) to obtain 8-bromo-5-chloro-4-methyl-d3-chroman-4-yl acetate (8.9 g).

(Step 3) 8-(benzylthio)-5-chloro-4-methyl-d3-chroman-4-yl acetate

To a 1,4-dioxane solution (70 mL) of 8-bromo-5-chloro-4-methyl-d3 -chroman-4-yl acetate (6.7 g) obtained in the above Step 2, 4,5-bis(diphenylphosphino)-9,9-dimethylxanthene (600 mg), tris(dibenzylideneacetone) dipalladium (0) (480 mg), N,N-diisopropylethylamine (7.2 mL) and benzylmercaptan (2.8ml) were added, and the reaction solution was stirred for 2 hours at 90°C. The reaction solution was allowed to cool to room temperature and filtered through CELITE. After washing the residue with hexane (50 mL), water (50 mL) was added to the filtrate for layering. The organic layer was washed with saturated saline (50 mL), dried over anhydrous sodium sulfate, and concentrated under reduced pressure. The reaction solution was concentrated under reduced pressure, and the residue was purified by silica gel column chromatography (eluent: hexane / ethyl acetate) to obtain 8-(Benzylthio)-5-chloro-4-methyl-d3-chroman-4-yl acetate (6.3 g).

(Step 4)

To an acetonitrile solution (100 mL) of 8-(benzylthio)-5-chloro-4-methyl-d3-chroman-4-yl acetate (6.3 g) obtained in the above Step 3, water (3 mL), acetic acid (4.3 mL) and 1,3-dichloro-5,5-dimethylhydantoin (7.2 g) were each added, and the reaction solution was stirred for 30 minutes at the same temperature. A saturated aqueous sodium hydrogen carbonate solution (70 mL) and ethyl acetate (70 mL) were added to the reaction solution, and the layers were separated. The aqueous layer was extracted with ethyl acetate (70 mL). The combined organic layer was washed with saturated saline (70 mL), dried over anhydrous sodium sulfate, and concentrated under reduced pressure. The residue was purified by silica gel column chromatography (eluent: hexane / ethyl acetate) to obtain the title compound (5.3 g).

[Reference Example E5 Synthesis of 5-chloro-8-(chlorosulfonyl)-4-(trifluoromethyl)chroman-4-yl acetate]

[Formula 26]

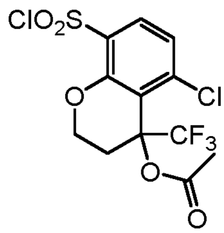

(Step 1) 8-bromo-5-chloro-4-(trifluoromethyl)chroman-4-ol

To a THF solution (4 mL) of 8-bromo-5-chloro-chromanon-4-one (398.2 mg), cesium fluoride (340.2 mg) and trifluoromethyltrimethylsilane (0.68 mL) were added at room temperature, and the reaction solution was stirred for 4 hours. An ammonium chloride aqueous solution (5 mL) was added to the reaction solution, and the mixture was extracted with ethyl acetate / hexane = 1/1 (15 mL). The organic layer was washed with saturated saline (10 mL), dried over anhydrous sodium sulfate, and concentrated under reduced pressure. The residue was purified by column chromatography (eluent: hexane / ethyl acetate) to obtain 8-bromo-5-chloro-4-(trifluoromethyl)chromanone-4-ol (139.2 mg).

(Step 2)

From 8-bromo-5-chloro-4-(trifluoromethyl)chroman-4-ol obtained from the above Step 1, the title compound was obtained according to the method of Reference Example E1 Step 2-4.

[Reference Example E6 Synthesis of 8-(chlorosulfonyl)-4-(trifluoromethyl)chroman-4-yl acetate]

The compound of Reference Example E6 was synthesized according to the method of Reference Example E5 steps 1 and 2 using 8-bromo-chromanon-4-one as a starting material.

[Reference examples E2 to E4 and E7 to E34]

According to the method of Reference Example E1 Steps 1-4, the compounds of Reference Examples E2 to E4 were synthesized. According to the method of Reference Examples E1 Step 3 and 4, the compounds of Reference Examples E7 to E32 were synthesized. According to the method of Reference Example E1 Step 2-4, the compounds of Reference Example E33 and E34 were synthesized. The compounds of Reference Examples E2 to E4 and E7 to E34, and the starting materials are listed in the following table.

[Table 5]

| Reference Example | Starting Material | Synthesized Compound | Reference Example | Starting Material | Synthesized Compound |
|---|---|---|---|---|---|
| E2 | 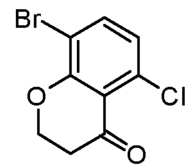 | 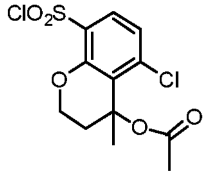 | E9 | 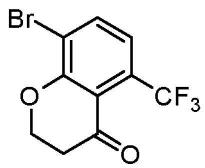 | 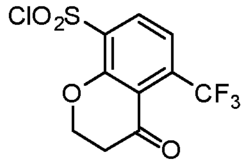 |
| E3 | 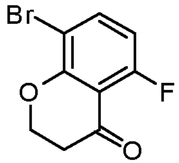 | 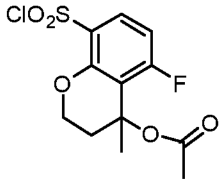 | E10 | 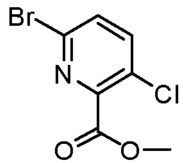 | 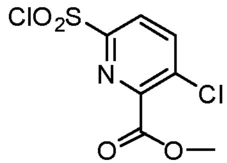 |
| E4 | 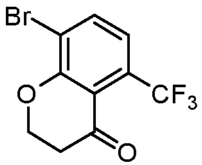 | 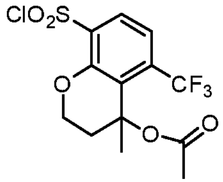 | E11 | 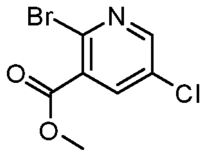 | 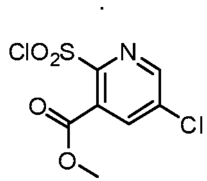 |
| E7 | 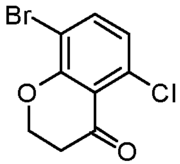 | 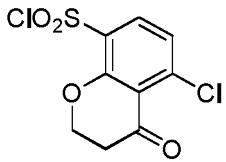 | E12 | 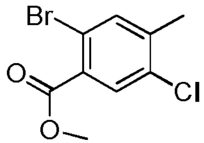 | 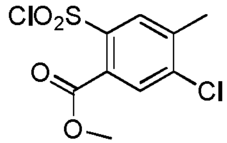 |
| E8 | 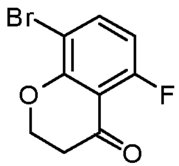 | 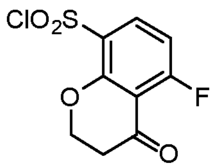 | E13 | 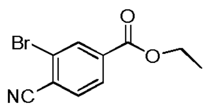 | 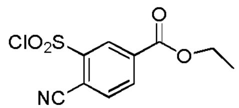 |

(continued)

| Reference Example | Starting Material | Synthesized Compound | Reference Example | Starting Material | Synthesized Compound |
|---|---|---|---|---|---|
| | | | E14 | 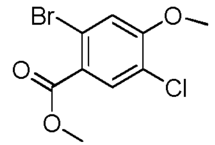 | 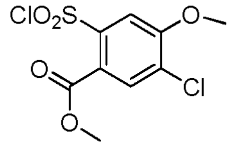 |
| | | | E15 | 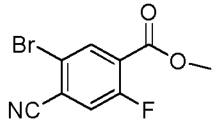 | 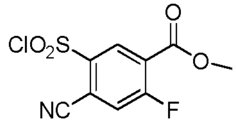 |
| | | | | | |
| Reference Example | Starting Material | Synthesized Compound | Reference Example | Starting Material | Synthesized Compound |
| E16 | 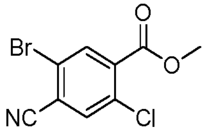 | 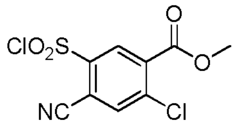 | E23 | 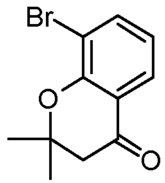 | 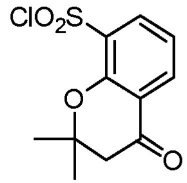 |
| E17 | 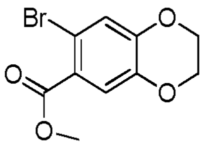 | 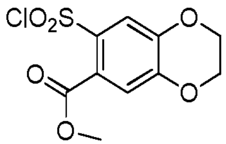 | E24 | 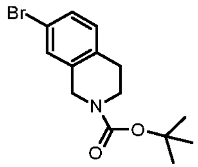 | 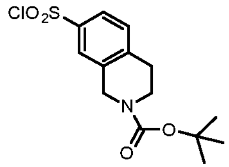 |
| E18 | 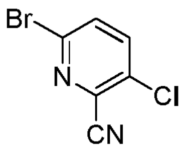 | 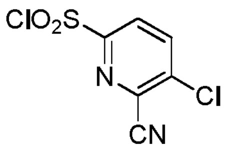 | E25 | 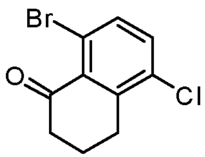 | 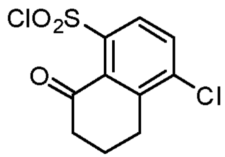 |

(continued)

| Reference Example | Starting Material | Synthesized Compound | Reference Example | Starting Material | Synthesized Compound |
|---|---|---|---|---|---|
| E19 | 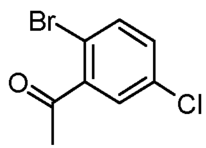 | 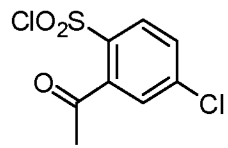 | E26 | 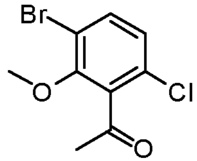 | 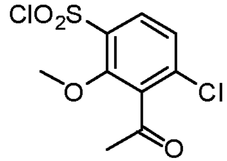 |
| E20 | 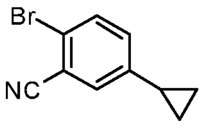 | 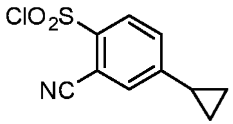 | E27 | 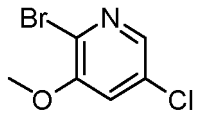 | 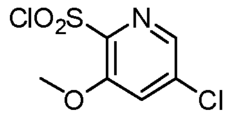 |
| E21 | 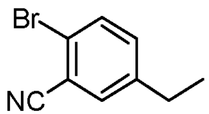 | 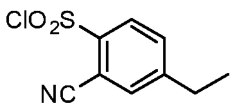 | E28 | 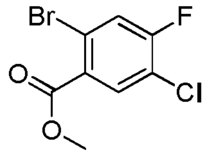 | 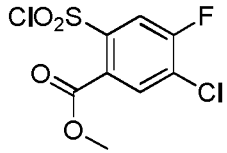 |
| E22 | 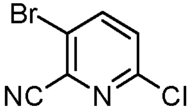 | 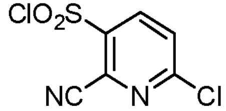 | E29 | 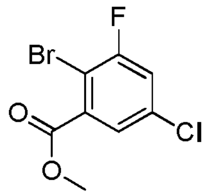 | 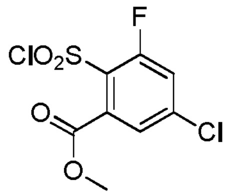 |
| | | | | | |
| Reference Example | Starting Material | Synthesized Compound | Reference Example | Starting Material | Synthesized Compound |
| E30 | 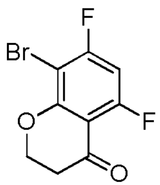 | 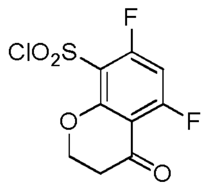 | E33 | 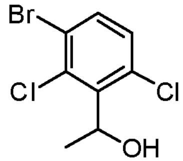 | 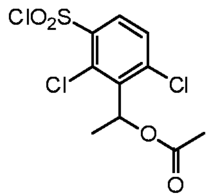 |

(continued)

| Reference Example | Starting Material | Synthesized Compound | Reference Example | Starting Material | Synthesized Compound |
|---|---|---|---|---|---|
| E31 | 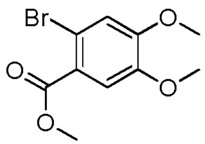 | 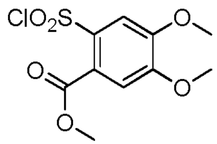 | E34 | 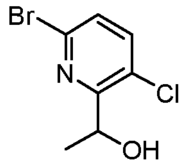 | 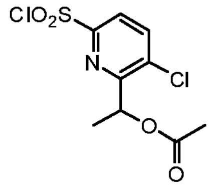 |
| E32 | 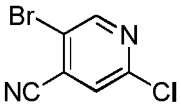 | 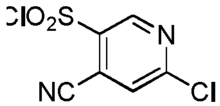 | | | |

[Reference Example E35 Synthesis of 5-Chloro-6-(pyrrolidine-1-carbonyl)pyridine-2-sulfonyl chloride]

[Formula 27]

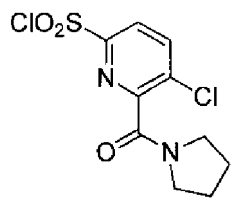

(Step 1) methyl 6-(benzylthio)-3-chloropicolinate

According to the method of Reference Example E1 Step 3, methyl 6-(benzylthio)-3-chloropicolinate was obtained from methyl 6-bromo-3-chloropicolinate.

(Step 2) 6-(benzylthio)-3-chloropicolinic acid

Methyl 6-(benzylthio)-3-chloropicolinate (1.0 g) obtained in the above Step 1 was dissolved in THF (5.0 mL) and water (1.0 ml), lithium hydroxide (165 mg) was added, and the reaction solution was stirred at room temperature for 16 hours. The reaction solution was added to hydrochloric acid (1 M, 10 mL) and extracted twice with ethyl acetate (20 mL). The organic layer was washed with saturated saline (10 mL), dried over anhydrous sodium sulfate, and concentrated under reduced pressure to obtain 6-(benzylthio)-3-chloropicolinic acid (920 mg).

(Step 3) (6-(benzylthio)-3-chloropyridin-2-yl)(pyrrolidin-1-yl)methanone

6-(benzylthio)-3-chloro-picolinic acid (100 mg) obtained in the above Step 2 was dissolved in DMF (2.5 mL), CDI (the 116 mg) was added, the reaction solution was stirred at room temperature for 10 minutes, and then triethylamine (150 μ) and pyrrolidine (60 μ) were added, and the reaction solution was stirred for 12 hours at 50°C. The reaction solution was added to water (20 mL) and extracted with ethyl acetate (20 mL). The organic layer was washed with saturated saline (20 mL), dried over anhydrous sodium sulfate, and concentrated under reduced pressure. The residue was purified by silica gel column chromatography (eluent: hexane / ethyl acetate) to obtain (6-(benzylthio)-3-chloropyridin-2-yl)(pyrrolidin-1-yl)methanone (105 mg).

(Step 4)

The title compound was obtained from (6-(benzylthio)-3-chloropyridin-2-yl)(pyrrolidin-1-yl)methanone obtained in the above Step 3 according to the method of Reference Example E1 Step 4.

[Reference examples E36 to E43]

According to the method of Reference Examples E35 Step 3 and E1 Step 4, the compounds of Reference Examples E36 to E43 shown below were synthesized from 6-(benzylthio)-3-chloropicolinic acid obtained from Reference Example E35 Step 2

[Table 6]

| Reference Example | Synthesized Compound | Reference Example | Synthesized Compound |
|---|---|---|---|
| E36 | 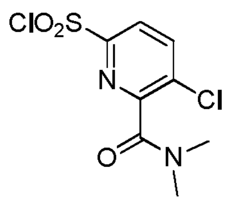 | E40 | 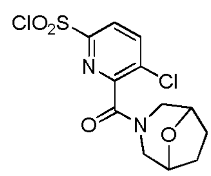 |

(continued)

| Reference Example | Synthesized Compound | Reference Example | Synthesized Compound |
|---|---|---|---|
| E37 | 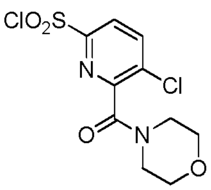 | E41 | 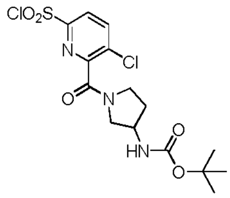 |
| E38 | 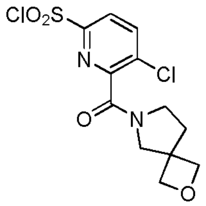 | E42 | 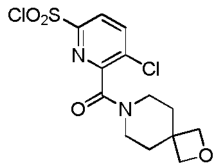 |
| E3 9 | 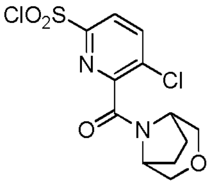 | E43 | 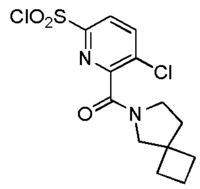 |

[Reference Example E44 Synthesis of 1-(6-chloro-3-(chlorosulfonyl)-2-methoxyphenyl)ethyl acetate]

[Formula 28]

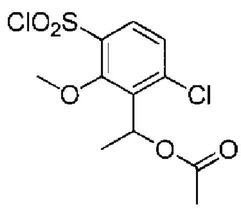

(Step 1) 3-bromo-6-chloro-2-methoxybenzaldehyde

According to the method of Reference Example B1 Step 1, 3-bromo-6-chloro-2-methoxybenzaldehyde was obtained from 1-bromo-4-chloro-2-methoxybenzene.

(Step 2) 1-(3-bromo-6-chloro-2-methoxyphenyl)ethanol

From 3-bromo-6-chloro-2-methoxybenzaldehyde obtained in the above Step 1, 1-(3-bromo-6-chloro-2-methoxyphenyl)ethanol was obtained according to the method of Reference Example A1 Step 1.

(Step 3) 1-(3-bromo-6-chloro-2-methoxyphenyl)ethyl acetate

1-(3-bromo-6-chloro-2-methoxyphenyl)ethanol (1.9 g) obtained in the above Step 2 was dissolved in dichloromethane (20 mL), triethylamine (2.0 mL), N,N-dimethyl-4-aminopyridine (100 mg), and acetic acid anhydride (1.2 mL) were successively added, and the reaction solution was stirred for 30 minutes at room temperature. The reaction solution was concentrated under reduced pressure, and the residue was purified by silica gel column chromatography (eluent: hexane / ethyl acetate) to obtain 1-(3-bromo-6-chloro-2-methoxyphenyl)ethyl acetate)(2.2g).

(Step 4)

From the 1-(3-bromo-6-chloro-2-methoxyphenyl)ethyl acetate obtained in the above Step 3, the title compound was obtained according to the method of Reference Examples E1 Steps 3 and 4.

[Reference Example E45 Synthesis of 1-(5-Chloro-2-(chlorosulfonyl)-3-methoxypyridin-4-yl)ethyl acetate]

[Formula 29]

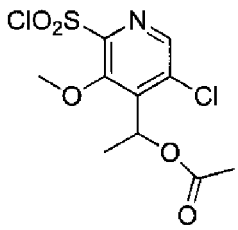

According to each of the methods of Reference Example B1 Step 1, Reference Example A1 Step 1, Reference Example E44 Step 3 and Reference Example E1 Steps 3 and 4, the title compound was obtained using 2-bromo -5-chloro-3-methoxypyridine instead of 1-bromo-4-chloro-2-methoxybenzene.

[Reference Example E46 Synthesis of 2-(6-chloro-3-(chlorosulfonyl)-2-methoxyphenyl)propan-2-yl acetate]

[Formula 30]

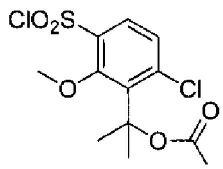

(Step 1) 1-(3-bromo-6-chloro-2-methoxyphenyl)ethanone

To a dichloromethane solution (30 mL) of 1-(3-bromo-6-chloro-2-methoxyphenyl)ethanol (2.8 g) obtained by Reference Example E44 Step 2, 1,1,1-triacetoxy-1,1-dihydro-1,2-benziodoxol-3-(1H)-one (5.4 g) was added, and the reaction solution was stirred for 20 minutes at room temperature. The reaction solution was added dropwise to a mixed solution of a saturated sodium hydrogen carbonate aqueous solution / a sodium hydrogen sulfite solution = 1/1 (50 mL) in an ice bath, and the layers were separated. The organic layer was washed with saturated saline (20 mL), dried over anhydrous sodium sulfate, and concentrated under reduced pressure. The residue is purified by silica gel column chromatography (eluent: hexane / ethyl acetate) to obtain 1-(3-bromo-6-chloro-2-methoxyphenyl)ethanone (2.7 g).

(Step 2) 1-(3-bromo-6-chloro-2-methoxyphenyl)ethyl acetate

From 1-(3-bromo-6-chloro-2-methoxyphenyl)ethanone obtained in the above Step 1, 1-(3-bromo-6-chloro-2-methoxyphenyl)ethyl acetate was obtained according to the method of Reference Example E1 Steps 1 and 2.

(Step 3)

From the 1-(3-bromo-6-chloro-2-methoxyphenyl)ethyl acetate (500 mg) obtained in the above Step 2, the title compound was obtained according to the method of Reference Examples E1 steps 3 and 4.

[Reference Example E47 Synthesis of 4-Chloro-2-(2,2-difluoroethoxy)benzene-1-sulfonyl chloride]

[Formula 31]

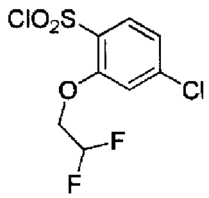

(Step 1) 1-bromo-4-chloro-2-(2,2-difluoroethoxy)benzene

To a DMF solution (5 mL) of 2-bromo-5-chlorophenol (244mg), potassium carbonate (325 mg) and 2,2-difluoroethyl 4-methylbenzenesulfonate (320 mg) were added, and the reaction solution was stirred for 3 hours at 95°C. The reaction solution was added to an aqueous sodium hydroxide solution (1 M, 20 mL) and extracted with toluene / ethyl acetate = 1/1 (20 mL). The organic layer was washed with saturated saline (20 mL), dried over anhydrous sodium sulfate, and concentrated under reduced pressure. The residue was purified by silica gel column chromatography (eluent: hexane / ethyl acetate) to obtain 1-bromo-4-chloro-2-(2,2-difluoroethoxy)benzene (315 mg).

(Step 2)

The title compound was synthesized from 1-bromo-4-chloro-2-(2,2-difluoroethoxy)benzene obtained in the above Step 1 according to the method of Reference Examples E1 Steps 3 and 4.

[Reference examples E48 and E49]

According to the methods of Reference Example E47 Step 1 and Reference Example E1 Steps 3 and 4, the compounds of Reference Examples E48 and 49 shown below were synthesized. However, regarding Reference Example 48, sodium chlorodifluoroacetate was used instead of 2,2-difluoroethyl 4-methylbenzenesulfonate.

[Table 7]

| Reference Example | Starting Material | Synthesized Compound |
|---|---|---|
| E48 | 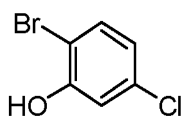 | 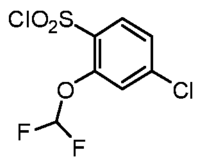 |
| E49 | 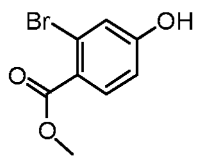 | 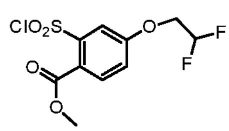 |

[Reference Example E50 Synthesis of 4-chloro-2-(isoxazol-5-yl)benzene-1-sulfonyl chloride]

[Formula 32]

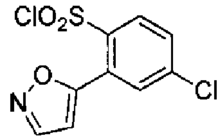

(Step 1) 5-(2-bromo-5-chlorophenyl)isoxazole

An N,N-dimethylformamide dimethyl acetal solution (6.0 mL) of 1-(2-bromo-5-chlorophenyl)ethanone (400 mg) was stirred for 16 hours at 140°C. After cooling to room temperature, the reaction mixture was concentrated under reduced pressure, and the resulting residue was purified by silica gel column chromatography (eluent: hexane / ethyl acetate). The obtained compound was dissolved in methanol (4.0 mL), hydroxylamine hydrochloride (175 mg) was added, and the reaction solution was stirred at room temperature for 16 hours. The reaction solution was added to an aqueous sodium bicarbonate solution (20 mL) and extracted with ethyl acetate (20mL). The organic layer was washed with saturated saline (20 mL), dried over anhydrous sodium sulfate, and concentrated under reduced pressure. The residue was purified by silica gel column chromatography to purify (eluent: hexane / ethyl acetate) to obtain 5-(2-bromo-5-chlorophenyl)isoxazole (430 mg).

(Step 2)

From 5-(2-bromo-5-chlorophenyl)isoxazole obtained in the above Step 1, the title compound was obtained according to the method of Reference Example E1 Steps 3,4.

[Reference Example E51 Synthesis of tert-butyl benzyloxy(5-chloro-2-(chlorosulfonyl)benzoyl)carbamate]

[Formula 33]

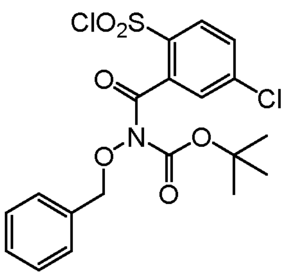

(Step 1) N-(benzyloxy)-2-(benzylthio)-5-chlorobenzamide

According to the method of Reference Example E1 Step 3, N-(benzyloxy)-2-(benzylthio)-5-chlorobenzamide was synthesized from N-(benzyloxy)-2-bromo-5-chlorobenzamide.

(Step 2) tert-butyl benzyloxy (2-(benzylthio)-5-chloro-benzoyl)carbamate

To a dichloromethane (10 mL) solution of N-(benzyloxy)-2-(benzylthio)-5-chlorobenzamide (433 mg) obtained from Reference Example 1, N,N-dimethyl-4-aminopyridine (280 mg) and di-tert-butyl dicarbonate (740 mg) were added, and the reaction solution was stirred for 16 hours at 55°C. The reaction solution was concentrated under reduced pressure, and the resulting residue was purified by silica gel column chromatography (eluent: hexane / ethyl acetate) to obtain tert-butylbenzyloxy (2-(benzylthio)-5-chlorobenzoyl)carbamate (549 mg).

(Step 3)

From the tert-butylbenzyloxy (2-(benzylthio)-5-chlorobenzoyl)carbamate obtained in the above Step 2, the title compound was obtained according to the method of Reference Example E1 Step 4.

[Reference Example E52 Synthesis of tert-butyl (5-chloro-2-(chlorosulfonyl)benzoyl)(methyl)carbamate]

[Formula 34]

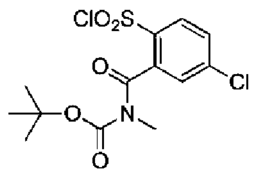

(Step 1) 2-bromo-5-chloro-N-methylbenzamide

From 2-bromo-5-chlorobenzoic acid and methylamine, 2-bromo-5-chloro-N-methylbenzamide was obtained according to the method of Reference Example E35 Step 3.

(Step 2) tert-butyl (2-bromo-5-chlorobenzoyl)(methyl)carbamate

(2-bromo-5-chlorobenzoyl)(methyl)carbamate was obtained from 2-bromo-5-chloro-N-methylbenzamide obtained in the above step 1 according to the method of Reference Example E51 Step 2.

(Step 3)

From the tert-butyl (2-bromo-5-chlorobenzoyl)(methyl)carbamate obtained in the above Step 2, the title compound was obtained according to the method of Reference Examples E1 steps 3 and 4.

[Reference Example E53 Synthesis of methyl 5-chloro-2-(chlorosulfonyl)-4-nitrobenzoate]

[Formula 35]

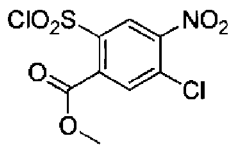

(Step 1) methyl 2-bromo-5-chloro-4-nitrobenzoate

To a 2-methyl-2-propanol solution (5 mL) of 1-bromo-4-chloro-2-methyl-5-nitrobenzene (1.0 g), water (5 mL), anisole (2.5 mL), and potassium permanganate (1.6 g) were added, and the reaction solution was stirred at 100°C for 20 hours. The reaction solution was cooled to room temperature, filtered through CELITE, and washed with water (10 mL) and ethyl acetate (10 mL). The combined filtrates were added to hydrochloric acid (1 M, 20 mL), and the layers were separated. The aqueous layer was extracted three times with ethyl acetate (20 mL). The combined organic layer was washed with saturated saline (20 mL), dried over anhydrous sodium sulfate, and concentrated under reduced pressure. The obtained residue was dissolved in methanol (5.0 mL), dichloromethane (10 mL) and a hexane solution of trimethylsilyldiazomethane (0.6 M, 6.0 mL) were added, and the reaction solution was stirred at room temperature for 20 minutes. The reaction solution was concentrated under reduced pressure, and the residue was purified by silica gel column chromatography (eluent: hexane / ethyl acetate) to obtain methyl 2-bromo-5-chloro-4-nitrobenzoate (529 mg).

(Step 2)

From the methyl 2-bromo-5-chloro-4-nitrobenzoate obtained in the above Step 1, the title compound was

obtained according to the method of Reference Example E1 Steps 3 and 4.

[Reference Example E54 Synthesis of 4-chloro-2-(5-oxo-4,5-dihydro-1,3,4-oxadiazol-2-yl)benzene-1-sulfonyl chloride]

[Formula 36]

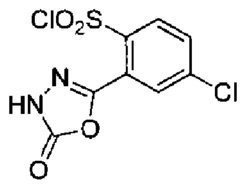

(Step 1) 5-(2-bromo-5-chlorophenyl)-1,3,4-oxadiazol-2(3H)-one

CDI (310 mg) was added to a THF (6.0 mL) suspension of 2-bromo-5-chlorobenzoic acid (300 mg), and the reaction solution was stirred at room temperature for 20 minutes. The reaction solution was ice-cooled, hydrazine · monohydrate (160 μL) was added, and the reaction solution was stirred at the same temperature for 20 minutes. The reaction solution was added to water (15 mL) and extracted with ethyl acetate (15 mL). The organic layer was washed with saturated saline (20 mL), dried over anhydrous sodium sulfate, and concentrated under reduced pressure. The obtained residue was dissolved in 1,4-dioxane (6.0 mL), CDI (310 mg) was added, and the reaction solution was stirred at 45°C for 2 hours. The reaction solution was added to water (15 mL) and extracted with ethyl acetate (15 mL). The organic layer was washed with saturated saline (20 mL), dried over anhydrous sodium sulfate, and concentrated under reduced pressure. The resulting residue was purified by silica gel column chromatography (eluent: hexane / ethyl acetate) to obtain 5-(2-bromo-5-chlorophenyl)-1,3,4-oxadiazol-2(3H)-one (300 mg).

(Step 2)

From the 5-(2-bromo-5-chlorophenyl)-1,3,4-oxadiazol-2(3H)-one obtained in the above Step 1, the title compound is obtained in accordance with the method of Steps 3 and 4 of Reference Example E1.

[Reference Example E55 Synthesis of tert-butyl N-tert-butoxycarbonyl-N-(1-(5-chloro-2-chlorosulfonyl-phenyl)cyclopropyl]carbamate]

[Formula 37]

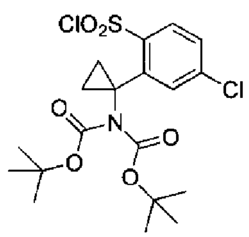

(Step 1) 1-(2-benzylsulfanyl-5-chlorophenyl)cyclopropanamine

To a THF (10 mL) suspension of 2-(benzylthio)-5-chlorobenzonitrile (1.0 g) and titanium tetraisopropoxide (1.3 mL), a diethyl ether solution (3.0 M, 3.0 mL) of methylmagnesium bromide was added dropwise at -78°C, and the reaction solution was stirred at the same temperature for 10 minutes. To the reaction solution, boron trifluoride diethyl ether complex (1.1 mL) was added, and the mixture was further stirred at room temperature for 1 hour, and then water (5 mL) and an aqueous sodium hydroxide solution (1 M, 5 mL) were added to separate layers, the aqueous layer was extracted with diethyl ether (20 mL). The combined organic layers were washed with saturated saline (20 mL), dried over anhydrous

magnesium sulfate, and concentrated under reduced pressure. The residue was purified by silica gel column chromatography (eluent: hexane / ethyl acetate) to obtain 1-(2-benzylsulfanyl-5-chloro-phenyl)cyclopropanamine (490 mg).

(Step 2) tert-butyl N-[1-(2-benzylsulfanyl-5-chloro-phenyl)-cyclopropyl]-N-tert-butoxycarbonyl-carbamate

To a 1,2-dichloroethane solution (10 mL) of 1-(2-benzylsulfanyl-5-chlorophenyl)cyclopropanamine (490 mg) obtained in the above Step 1, N,N-dimethyl-4-aminopyridine (210 mg) and di-tert-butyl dicarbonate (1.8 g) were added, and the reaction solution was stirred at 50°C for 16 hours. The reaction solution was added to hydrochloric acid (1 M, 10 mL) and extracted with ethyl acetate (15 mL). The organic layer was washed with saturated saline (20 mL), dried over anhydrous magnesium sulfate, and concentrated under reduced pressure. The residue was purified by silica gel column chromatography (eluent: hexane) to obtain tert-butyl N-[1-(2-benzylsulfanyl-5-chloro-phenyl)cyclopropyl]-N-tert-butoxycarbonyl-carbamate (502 mg).

(Step 3)

From tert-butyl N-[1-(2-benzylsulfanyl-5-chloro-phenyl)cyclopropyl]-N-tert-butoxycarbonyl-carbamate obtained in the above Step 2, the title compound is obtained in accordance with the method of Reference Example E1 step 4.

[Reference Example E56 Synthesis of methyl 6-(bis(tert-butoxycarbonyl)amino)-3-chlorosulfonyl-2-methoxy-benzoate]

[Formula 38]

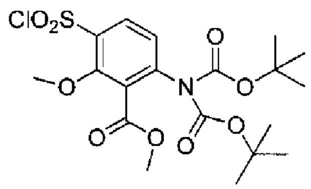

(Step 1) methyl 6-(bis(tert-butoxycarbonyl)amino)-3-bromo-2-methoxybenzoate

From methyl 6-amino-3-bromo-2-methoxybenzoate, methyl 6-(bis(tert-butoxycarbonyl)amino)-3-bromo-2-methoxybenzoate was obtained according to the method of Reference Example E55 Step 2.

(Step 2)

The title compound was obtained from methyl 6-(bis(tert-butoxycarbonyl)amino)-3-bromo-2-methoxybenzoate obtained in the above step 1 according to the method of Reference Examples E1 Steps 3 and 4.

[Reference Example E57 Synthesis of 5-Chloro-4,4-difluorochroman-8-sulfonyl chloride]

[Formula 39]

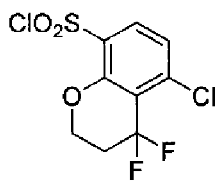

(Step 1) 8-(benzylthio)-5-chlorochroman-4-one

From 8-bromo-5-chlorochroman-4-one, 8-(benzylthio)-5-chlorochroman-4-one was obtained according to the method of Reference Example E1 Step 3.

(Step 2) 8-(benzylthio)-5-chloro-4,4-difluorochroman

From 8-(benzylthio)-5-chlorochroman-4-one (125 mg) obtained in the above Step 1, 8-(benzylthio)-5-chloro-4,4-difluorochroman was obtained according to the method of Reference Example D61 Step 2.

(Step 3)

From the 8-(benzylthio)-5-chloro-4,4-difluorochroman obtained in the above Step 2, the title compound was obtained according to the method of Reference Example E1 Step 4.

[Reference Example E58 Synthesis of tert-Butyl 5-chloro-8-(chlorosulfonyl)-2H-benzo[b][1,4]oxazin-4(3H)-carboxylate]

[Formula 40]

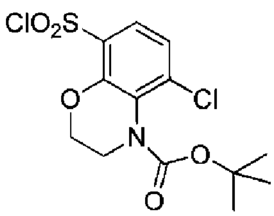

(Step 1) 8-bromo-5-chloro-3,4-dihydro-2H-benzoxazine

1,2-dibromoethane (500 μL) and potassium carbonate (3.0 g) were added to a DMF solution (6 mL) of 2-amino-6-bromo-3-chlorophenol (1.3 g), and the reaction solution was stirred for 12 hours at 100°C. The reaction solution was allowed to cool to room temperature, a saturated aqueous ammonium chloride solution (10 mL) and ethyl acetate (10 mL) were added to the reaction solution, the layers were separated, and the aqueous layer was extracted with ethyl acetate (20 mL). The combined organic layer was washed with saturated saline (10 mL), dried over anhydrous sodium sulfate, and concentrated under reduced pressure. The residue was purified by silica gel column chromatography (eluent: hexane / ethyl acetate) to obtain 8-bromo-5-chloro-3, 4-dihydro-2H-benzoxazine (400 mg).

(Step 2) tert-butyl 8-bromo-5-chloro-2H-benzo[b][1,4]oxazin-4(3H)-carboxylate

To dioxane solution (5 mL) of 8-bromo-5-chloro-3,4-dihydro-2H-1,4-benzoxazine (223 mg), 4-dimethylaminopyridine (44 mg), triethylamine (0.25 mL) and di-tert-butyl dicarbamate (458 mg) were added at room temperature, and the reaction solution was stirred for 2 hours. The reaction solution was concentrated under reduced pressure, and the residue was purified by column chromatography (eluent: hexane / ethyl acetate) to obtain tert-butyl 8-bromo-5-chloro-2H-benzo [b][1,4]oxazin-4(3H)-carboxylate (140 mg).

(Step 3)

From tert-butyl 8-bromo-5-chloro-2H-benzo [b][1,4]oxazin-4(3H)-carboxylate obtained in the above Step 2, the title compound is obtained according to the method of Steps 3 and 4 of Reference Example E1.

[Reference Example E59 Synthesis of tert-butyl 8-(chlorosulfonyl)-2H-benzo [b][1,4]oxazin-4(3H)-carboxylate]

[Formula 41]

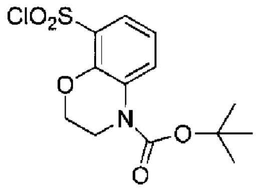

(Step 1) tert-butyl 8-bromo-2H-benzo[b][1,4]oxazin-4(3H)-carboxylate

From 8-bromo-3,4-dihydro-2H-benzo[b][1,4]oxazine, tert-butyl 8-bromo-2H-benzo[b][1,4]oxazin-4(3H)-carboxylate was obtained according to the method of Reference Example E58 Step 2.

(Step 2)

From tert-butyl 8-bromo-2H-benzo[b][1,4]oxazin-4(3H)-carboxylate obtained in the above Step 1, the title compound was obtained, in accordance with Reference Example E1 Steps 3 and 4.

[Reference Example E60 Synthesis of tert-butyl 4-(chlorosulfonyl)-1H-indole-1-carboxylate]

[Formula 42]

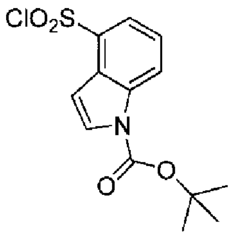

From the commercially available tert-butyl 4-bromo-1H-indole-1-carboxylate (Ark Pharm, Inc.), the title compound was obtained according to the method of Reference Example E1 steps 3 and 4.

[Reference Example E61 Synthesis of 5-chloro-4-ethyl-3,4-dihydro-2H-benzo[b][1,4]oxazin-8-sulfonyl chloride]

[Formula 43]

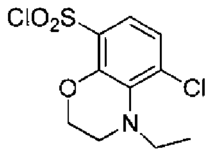

(Step 1) 8-bromo-5-chloro-4-ethyl-2,3-dihydro-1,4-benzoxazine

To a DMSO solution (2.0 mL) of 8-bromo-5-chloro-3,4-dihydro-2H-benzoxazine (380 mg) obtained in Reference Example E58 Step 1, potassium hydroxide (120 mg) and ethyl iodide (100 μ) were added, and the reaction solution was stirred at 100°C for 2 hours. The reaction solution was allowed to cool to room temperature, a saturated aqueous solution of ammonium chloride (10 mL) and ethyl acetate (10 mL) were added to separate layers, and the aqueous layer was

extracted with ethyl acetate (10 mL). The combined organic layer was washed with saturated saline (10 mL), dried over anhydrous sodium sulfate, and concentrated under reduced pressure. The reaction solution was concentrated under reduced pressure, and the residue was purified by silica gel column chromatography (eluent: hexane / ethyl acetate) to obtain 8-bromo-5-chloro-4-ethyl-2,3-dihydro-1,4-benzoxazine (105 mg).

(Step 2)

From the 8-bromo-5-chloro-4-ethyl-2,3-dihydro-1,4-benzoxazine obtained in the above Step 1, the title compound was obtained according to the method of Reference Examples E1 Steps 3 and 4.

[Reference Example E62 Synthesis of 4-(cyclopropanecarbonyl)-3,4-dihydro-2H-benzo[b][1,4]oxazin-8-sulfonyl chloride]

[Formula 44]

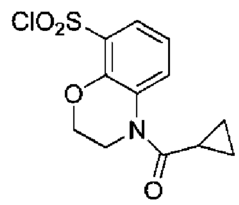

(Step 1) (8-bromo-2H-benzo[b][1,4]oxazin-4(3H)-yl)(cyclopropyl)methanone

Sodium hydride (18 mg) was added to a THF solution (2.0 mL) of 8-bromo-3,4-dihydro-2H-benzo[b][1,4]oxazine (62 mg) at 0°C, and the reaction solution was stirred for 30 minutes. Cyclopropanecarbonyl chloride (170 μL) was added to the reaction solution, and the mixture was further stirred at room temperature for 2 hours. A saturated ammonium chloride aqueous solution (10 mL) and ethyl acetate (10 mL) were sequentially added to the reaction solution to separate layers, and the aqueous layer was extracted with ethyl acetate (10 mL). The combined organic layer was washed with saturated saline (10 mL), dried over anhydrous sodium sulfate, and concentrated under reduced pressure. The resulting residue was purified by silica gel column chromatography (eluent: hexane / ethyl acetate) to give (8-bromo-2H-benzo[b][1,4]oxazin-4(3H)-yl)(cyclopropyl)methanone (87 mg).

(Step 2)

From (8-bromo-2H-benzo[b][1,4]oxazin-4(3H)-yl)(cyclopropyl)methanone obtained in the above Step 1 according to the method of Steps 3 and 4 of Reference Example E1, the title compound was obtained.

[Reference Example E63 Synthesis of 5-chloro-4-(2,2-difluoroethyl)-3-oxo-3,4-dihydro-2H-benzo[b][1,4]oxazin-8-sulfonyl chloride]

[Formula 45]

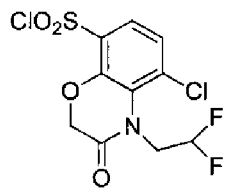

(Step 1) 8-bromo-5-chloro-2H-benzo[b][1,4]oxazin-3(4H)-one

2-amino-6-bromo-3-chlorophenol (140 mg) was dissolved in THF (2.0 mL), chloroacetyl chloride (100 μL) and sodium hydrogencarbonate (240 mg) were added and the reaction solution was stirred at room temperature for 3 hours. Potassium carbonate (440 mg) was added to the reaction solution, and the mixture was further stirred at 80°C for 5 hours. The reaction solution was allowed to cool to room temperature, and a saturated aqueous solution of ammonium chloride (10 mL) and ethyl acetate (10 mL) were added to separate layers, and the aqueous layer was extracted with ethyl acetate (20 mL). The combined organic layer was washed with saturated saline (10 mL), dried over anhydrous sodium sulfate, and concentrated under reduced pressure to give 8-bromo-5-chloro-2H-benzo[b][1,4]oxazin-3(4H)-one (160 mg).

(Step 2) 8-bromo-5-chloro-4-(2,2-difluoroethyl)-2H-benzo[b][1,4]oxazin-3(4H)-one

To a DMF (2.5 mL) solution of 8-bromo-5-chloro-2H-benzo[b][1,4]oxazin-3(4H)-one (69 mg) obtained in the above Step 1, potassium carbonate (420 mg) and 2,2-difluoroethyl paratoluene sulfonate (500 mg) were sequentially added, and the reaction solution was stirred at 100°C for 3 hours. The reaction solution was allowed to cool to room temperature, and a saturated aqueous solution of ammonium chloride (10 mL) and ethyl acetate (10 mL) were added to separate layers, and the aqueous layer was extracted with ethyl acetate (10 mL). The combined organic layer was washed with saturated saline (10 mL), dried over anhydrous sodium sulfate, and concentrated under reduced pressure. The obtained residue was purified by silica gel column chromatography (eluent: hexane / ethyl acetate) to give 8-bromo-5-chloro-4-(2,2-difluoroethyl)-2H-benzo[b][1,4]oxazin-3(4H)-one (85 mg).

(Step 3) 8-(benzylthio)-5-chloro-4-(2,2-difluoroethyl)-2H-benzo[b][1,4]oxazin-3(4H)-one

From 8-bromo-5-chloro-4-(2,2-difluoroethyl)-2H-benzo[b][1,4]oxazin-3(4H)-one obtained in the above Step 2, 8-(benzylthio)-5-chloro-4-(2,2-difluoroethyl)-2H-benzo[b][1,4]oxazin-3 (4H)-one was obtained according to the method of Reference Example E1 Step 3.

(Step 4)

From the 8-(benzylthio)-5-chloro-4-(2,2-difluoroethyl)-2H-benzo[b][1,4]oxazin-3(4H)-one, the title compound was obtained in the above Step 3, according to the method of Reference Example E1 Step 4.

[Reference Example E64 and E65]

From 8-bromo-5-chloro-2H-benzo[b][1,4]oxazin-3(4H)-one obtained from Reference Example E63 Step 1, the following compounds of reference examples E64 and E65 are synthesized according to method of Reference Example E63 Step 2, and Reference Example E1 Steps 3 and 4.

[Table 8]

| Reference Example | Alkylating agent | Synthesized Compound |
|---|---|---|
| E64 | MeI | 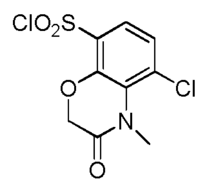 |
| E65 | 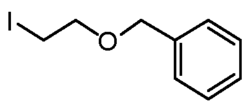 | 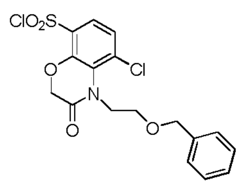 |

[Reference Example E66 Synthesis of 5-chloro-4-(2,2-difluoroethyl)-3,4-dihydro-2H-benzo[b][1,4]oxazine-8-sulfonyl chloride]

[Formula 46]

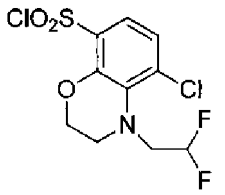

(Step 1) 8-(benzylthio)-5-chloro-4-(2,2-difluoroethyl)-3,4-dihydro-2H-benzo[b][1,4]oxazine

To a THF solution (5 mL) of 8-(benzylthio)-5-chloro-4-(2,2-difluoroethyl)-2H-benzo[b][1,4]oxazin-3(4H)-one (270 mg) obtained from Reference Example 63 Step 3, dimethylsulfide borane (1.0 mL) was added, and the reaction solution was stirred at 70°C for 4 hours. The reaction solution was allowed to cool to room temperature, methanol (5 mL), ethyl acetate (10 mL), and water (10 mL) were added in order to separate layers, and the aqueous layer was extracted with ethyl acetate (10 mL). The combined organic layer was washed with saturated saline (10 mL), dried over anhydrous sodium sulfate, and concentrated under reduced pressure. The residue was purified by silica gel column chromatography (eluent: hexane / ethyl acetate) to obtain 8-(benzylthio)-5-chloro-4-(2,2-difluoroethyl)-3,4-dihydro-2H-benzo[b][1,4]oxazine (154 mg).

(Step 2)

From the 8-(benzylthio)-5-chloro-4-(2,2-difluoroethyl)-3,4-dihydro-2H-benzo[b][1,4]oxazine obtained in the above Step 1, the title compound was obtained according to the method of Reference Example E1 Step 4.

[Reference Example E67 Synthesis of 2-cyano-5-(morpholine-4-carbonyl)benzene-1-sulfonyl chloride]

[Formula 47]

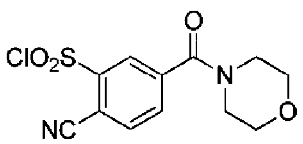

(Step 1) ethyl 3-(benzylthio)-4-cyanobenzoate

Ethyl 3-(benzylthio)-4-cyanobenzoate was obtained from ethyl 3-bromo-4-cyanobenzoate according to the method of Reference Example E1 Step 3.

(Step 2) 6-(benzylthio)-4-cyano-benzoic acid

An aqueous sodium hydroxide solution (3 M, 4.0 mL) was added to a THF (4.0 mL) solution of ethyl 3-(benzylthio)-4-cyanobenzoate (344 mg) obtained in the above Step 1, and the reaction solution was stirred at room temperature for 16 hours. The reaction solution was added to hydrochloric acid (1 M, 15 mL) and extracted twice with ethyl acetate (20 mL). The combined organic layer was washed with saturated saline (10 mL), dried over anhydrous sodium sulfate, and concentrated under reduced pressure to obtain 6-(benzylthio)-4-cyanobenzoic acid (210 mg).

(Step 3) 2-(benzylthio)-4-(morpholine-4-carbonyl)benzonitrile

Using 6-(benzylthio)-4-cyano-benzoic acid obtained from the above Step 2 and morpholine, 2-(benzylthio)-4-(morpholine-4-carbonyl)benzonitrile was obtained according to Reference Example E35 Step 3.

(Step 4)

From the 2-(benzylthio)-4-(morpholine-4-carbonyl)benzonitrile obtained in the above Step 3, the title compound was obtained according to the method of Reference Example E1 Step 4.

[Reference Example E68 Synthesis of 2-cyano-5-(dimethylcarbamoyl)benzene-1-sulfonyl chloride]

[Formula 48]

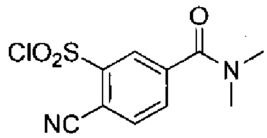

(Step 1) 2-(benzylthio)-4-cyano-N,N-dimethylbenzamide

According to the method of Reference Example E35 Step 3, from 6-(benzylthio)-4-cyanobenzoic acid obtained in Reference Example E67 Step 2 and dimethylamine, 2-(benzylthio)-4-cyano-N,N-dimethylbenzamide was obtained.

(Step 2)

From the 2-(benzylthio)-4-cyano-N,N-dimethylbenzamide obtained in the above Step 1, the title compound was obtained according to the method of Reference Example E1 Step 4.

[Reference Example E69 Synthesis of 4-chloro-2-cyano-5-(dimethylcarbamoyl)benzene-1-sulfonyl chloride]

[Formula 49]

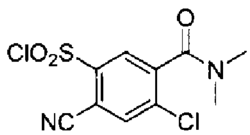

The title compound was synthesized from methyl 5-bromo-2-chloro-4-cyanobenzoate according to each of the methods of Reference Example E1 Step 3, Reference Example E67 Step 2, Reference Example E35 Step 3 and Reference Example E1 Step 4.

[Reference Example E70 Synthesis of tert-butyl (5-chloro-8-(chlorosulfonyl)chroman-4-yl)carbamate]

[Formula 50]

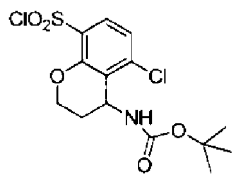

(Step 1) 8-benzyl-sulfanyl-5-chlorochroman-4-one

From 8-bromo-5-chlorochroman-4-one, 8-benzylsulfanyl-5-chlorochroman-4-one was obtained according to the method of Reference Example E1 Step 3.

(Step 2) 8-benzyl-sulfanyl-5-chlorochroman-4-amine

8-benzylsulfanyl-5-chlorochroman-4-one (460 mg) obtained in the above Step 1 was dissolved in methanol (3.0 mL), ammonium chloride (1.2 g) was added, and the reaction solution was stirred at room temperature for 2 hours. Sodium cyanoborohydride (670 mg) was added to the reaction solution, and the mixture was further stirred at 80°C for 14 hours. A saturated aqueous sodium hydrogen carbonate solution (10 mL), an aqueous sodium hydroxide solution (5 M, 10 mL) and chloroform (20 mL) were added successively to the reaction solution to separate layers, and the aqueous layer was extracted twice with chloroform (20 mL). The combined organic layer was washed with saturated saline (10 mL), dried over anhydrous sodium sulfate, and concentrated under reduced pressure. The residue was purified by silica gel column chromatography (ethyl acetate) to obtain 8-benzylsulfanyl-5-chlorochroman-4-amine (216 mg).

(Step 3) tert-butyl (8-(benzylthio)-5-chlorochroman-4-yl)carbamate

From 8-benzylsulfanyl-5-chloro-chroman-4-amine (216 mg) obtained in the above Step 2, tert-butyl (8-(benzylthio)-5-chlorochroman-4-yl)carbamate was obtained according to Reference Example E58 Step 2.

(Step 4)

From the tert-butyl (8-(benzylthio)-5-chlorochroman-4-yl)carbamate obtained in the above Step 3, the title compound was obtained according to the method of Reference Example E1 Step 4.

[Reference Example E71 Synthesis of 4-acetamido-5-chlorochroman-8-sulfonyl chloride]

[Formula 51]

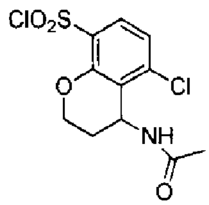

(Step 1) N-(8-bromo-5-chlorochromanon-4-yl)acetamide

8-Bromo-5-chlorochromanon-4-amine (250 mg) was dissolved in DMF (2.0 mL) and THF (7.0 mL), N,N-dimethyl-4-aminopyridine (45 mg), triethylamine (400 μL) and acetic anhydride (200 μL) were sequentially added, and the mixture was stirred at room temperature for 2 hours. Water (10 mL) was added to the reaction solution, and the mixture was extracted with ethyl acetate (10 mL). The organic layer was washed with saturated saline (20 mL), dried over anhydrous sodium sulfate, and concentrated under reduced pressure. The residue was purified by silica gel column chromatography

(eluent: hexane / ethyl acetate) to obtain N-(8-bromo-5-chlorochromanon-4-yl)acetamide (260 mg).

(Step 2)

From the N-(8-bromo-5-chlorochromanon-4-yl)acetamide obtained in the above Step 1, the title compound was obtained according to the method of Reference Examples E1 steps 3 and 4.

[Reference Example E72 Synthesis of 1-(3- chloro-6-(chlorosulfonyl)pyridin-2-yl)-2,2,2-trifluoroethylacetate]

[Formula 52]

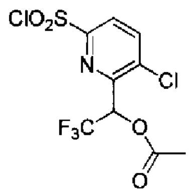

(Step 1) 1-(6-bromo-3-chloropyridin-2-yl)-2,2,2-trifluoroethanol

Cesium fluoride (700 mg) and (trifluoromethyl)trimethylsilane (700 μL) were added to a THF (10 mL) solution of 6-bromo-3-chloropicolinaldehyde (770 mg), and the reaction solution was stirred at room temperature for 4 hours. A saturated aqueous sodium hydrogen carbonate solution (10 mL) was added to the reaction solution, and the mixture was extracted with ethyl acetate (20 mL). The organic layer was washed with saturated saline (10 mL), dried over anhydrous sodium sulfate, and concentrated under reduced pressure. The residue was purified by silica gel column chromatography (eluent: hexane / ethyl acetate) to obtain 1-(6-bromo-3 -chloropyridin-2-yl)-2,2,2-trifluoroethanol (600mg).

(Step 2)

From 1-(6-bromo-3-chloropyridin-2-yl)-2,2,2-trifluoroethanol obtained in the above Step 1, according to the method of Reference Examples E44 Step 3 and E1 Steps 3 to 4, the title compound was obtained.

[Reference Example E73 Synthesis of methyl 5-bromo-2-(chlorosulfonyl)nicotinate]

[Formula 53]

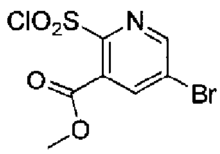

(Step 1) methyl 2-(benzylthio)-5-bromo-nicotinate

Sodium hydride (285 mg) was added to a THF (5.0 mL) solution of benzyl mercaptan (700 μL) at 0°C, and the reaction solution was stirred at room temperature for 15 min. A THF (3.0 mL) solution of methyl 2,5-dibromonicotinate (1.59 g) was added dropwise to the reaction solution, and the mixture was stirred at 0°C for 20 minutes. The reaction solution was added to water (10 mL) and extracted with ethyl acetate (20 mL). The organic layer was washed with saturated saline (10 mL), dried over anhydrous sodium sulfate, and concentrated under reduced pressure. The residue was purified by silica gel column chromatography (eluent: hexane / ethyl acetate) to obtain methyl 2-(benzylthio)-5-bromonicotinate (1.5 g).

(Step 2)

From the methyl 2-(benzylthio)-5-bromonicotinate obtained in the above Step 1, the title compound was obtained according to the method of Reference Example E1 Step 4.

[Reference Example F1 Synthesis of 5-((1S,2R)-1-Amino-2-(6-fluoro-2,3-dimethylphenyl)propyl)-1,3,4-oxadiazol-2 (3H)-one monohydrochloride]

[Formula 54]

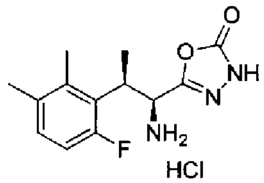

(Step 1) (2S, 3R)-2-((tert- butoxycarbonyl)amino)-3-(6-fluoro-2,3-dimethylphenyl)butanoic acid

Water (9 mL), 1,4-dioxane (9 mL) and triethylamine (955 μL) were sequentially added to (2S, 3R)-2-amino-3-(6-fluoro-2,3-dimethylphenyl)butanoic acid (515 mg) obtained in Reference Example D1, and the mixture was cooled to 0°C. Di-tert-butyl dicarbonate (650 mg) was added to the reaction solution at the same temperature, and the mixture was stirred for 45 minutes. The reaction solution was added to hydrochloric acid (1 M, 20 mL) and extracted with ethyl acetate (20 mL). The organic layer was washed with saturated saline (20 mL), dried over anhydrous magnesium sulfate, and concentrated under reduced pressure. The residue is purified by silica gel column chromatography (eluent: hexane / ethyl acetate / 2% acetic acid) to obtain (2S, 3R)-2 -((tert-butoxycarbonyl)amino)-3-(6-fluoro-2,3-dimethylphenyl)butanoic acid (745 mg).

(Step 2) tert-butyl ((1 S,2R)-2-(6-fluoro-2,3-dimethylphenyl)-1-(5-oxo-4,5-dihydro-1,3,4-oxadiazol-2-yl)propyl)carbamate

To a THF solution (14.0 mL) of (2S, 3R)-2-(tert-butoxycarbonylamino)-3-(6-fluoro-2,3-dimethylphenyl)butanoic acid (440 mg) obtained in the above Step 1, CDI (302 mg) was added, and the reaction solution was stirred at room temperature for 20 minutes. The reaction solution was cooled to 0°C, hydrazine · monohydrate (200 μL) was added, and the mixture was stirred at the same temperature for 30 minutes. The reaction solution was added to water (20 mL) and extracted with ethyl acetate (20 mL). The organic layer was washed with saturated saline (20 mL), dried over anhydrous sodium sulfate, and concentrated under reduced pressure. CDI (560 mg) was added to a 1,4-dioxane (14 mL) solution of the obtained residue, and the reaction solution was stirred at room temperature for 30 minutes. The reaction solution was added to water (20 mL) and extracted with ethyl acetate (20 mL). The organic layer was washed with saturated saline (20 mL), dried over anhydrous sodium sulfate, and concentrated under reduced pressure. The obtained residue was purified by silica gel column chromatography by purification (eluent: hexane / ethyl acetate) to obtain tert-butyl ((1S,2R)-2-(6-fluoro-2,3-dimethylphenyl)-1-(5-oxo-4,5-dihydro-1,3,4-oxadiazol-2-yl)propyl)carbamate

(356 mg).

(Step 3)

tert-butyl ((1S ,2R)-2-(6-fluoro-2,3-dimethylphenyl)-1-(5 -oxo-4,5 -dihydro-1,3,4-oxadiazol-2-yl)propyl)carbamate (550 mg) obtained in the above Step 2 was dissolved in hydrochloric acid-1,4-dioxane (4 M, 5.0 mL), and the reaction solution was stirred at room temperature for 1.5 hours. The reaction solution was concentrated under reduced pressure to obtain the title compound.

[Reference examples F2 to F10]

According to the method of Reference Example F1 Steps 1 to 3, the following compounds of Reference Examples F2 to F10 were synthesized.

[Table 9]

| Reference Example | Starting Material (Reference example number or structural formula) | Synthesized Compound | Reference Example | Starting Material (Reference example number or structural formula) | Synthesized Compound |
|---|---|---|---|---|---|
| F2 | Reference Example D6 | 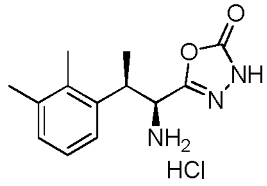 | F7 | Reference Example D4 | 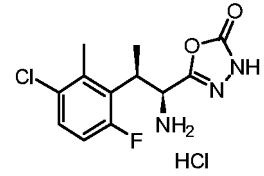 |
| F3 | Reference Example D3 | 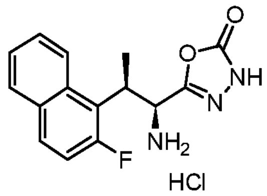 | F8 | Reference Example D5 | 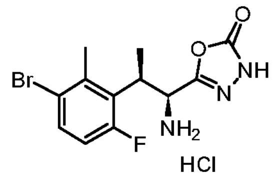 |
| F4 | Reference Example D13 | 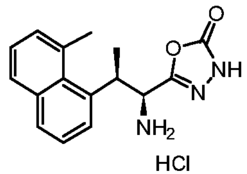 | F9 | Reference Example D45 | 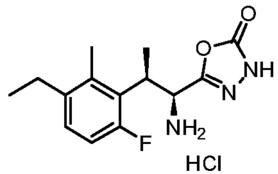 |
| F5 | Reference Example D10 | 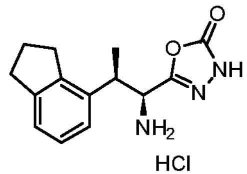 | F10 | Reference Example D61 | 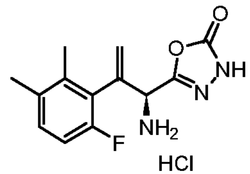 |
| F6 | Reference Example D41 | 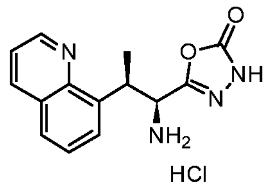 | | | |

[Example 1]

Synthesis of 5-bromo-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide

(Step 1)

To a 1,4-dioxane (5.0 mL) solution and water (5.0 mL) of (2S, 3R)-2-amino-3-(6-fluoro-2,3-dimethylphenyl)butanoic acid (300 mg) obtained in Reference Example D1, triethylamine (570 μL) was added and then cooled to 0°C. 4-Bromo-2-cyanobenzene-1-sulfonyl chloride (362 mg) was added to the reaction solution, and the mixture was stirred at the same temperature for 45 minutes. The reaction solution was added to hydrochloric acid (1 M, 15 mL) and extracted with ethyl acetate (15 mL). The organic layer was washed with saturated saline (20 mL), dried over anhydrous magnesium sulfate, and concentrated under reduced pressure. The residue was purified by silica gel column chromatography (eluent: hexane / ethyl acetate / 2% acetic acid) to obtain (2S, 3R)-2-(4-bromo-2-cyanophenylsulfonamido)-3-(6-fluoro-2,3-dimethylphenyl)butanoic acid (465 mg).

(Step 2)

To a THF (5.0 mL) solution of (2S, 3R)-2-(4-bromo-2-cyanophenylsulfonamido)-3-(6-fluoro-2,3-dimethylphenyl)butanoic acid (465 mg) obtained in the above Step 1, CDI (210 mg) was added, and the reaction solution was stirred at room temperature for 20 minutes. The reaction solution was cooled to 0°C, hydrazine · monohydrate (200 μL) was added, and the mixture was stirred at the same temperature for 20 minutes. The reaction solution was added to water (20 mL) and extracted with ethyl acetate (20 mL). The organic layer was washed with saturated saline (20 mL), dried over anhydrous sodium sulfate, and concentrated under reduced pressure.

CDI (211 mg) was added to a 1,4-dioxane (4.0 mL) solution of the obtained residue, and the reaction solution was stirred at 45°C for 1 hour. The reaction solution was added to water (20 mL) and extracted with ethyl acetate (20 mL). The organic layer was washed with saturated saline (20 mL), dried over anhydrous sodium sulfate, and concentrated under reduced pressure. The obtained residue was purified by silica gel column chromatography (eluent: hexane / ethyl acetate) to obtain 4-bromo-2-cyano-N-((1S,2R)-2-(6-fluoro-2,3-dimethylphenyl)-1-(5-oxo-4,5-dihydro-1,3,4-oxadiazol-2-yl)propyl)benzenesulfonamide (386 mg).

(Step 3)

To a DMSO (5.0 mL) solution of 4-bromo-2-cyano-N-((1S,2R)-2-(6-fluoro-2,3-dimethylphenyl)-1-(5-oxo-4,5-dihydro-1,3,4-oxadiazol-2-yl)propyl)benzenesulfonamide (386 mg) obtained in the above Step 2, hydrogen peroxide water (1.0 mL) and potassium carbonate (420 mg) were added sequentially in an ice bath, and the reaction solution was stirred at 60°C for 2.5 hours. The reaction solution was slowly added to hydrochloric acid (1 M, 15 mL) in an ice bath and then extracted with ethyl acetate (15 mL). The organic layer was washed with saturated saline (20 mL), dried over anhydrous sodium sulfate, and concentrated under reduced pressure. The residue was purified by silica gel column chromatography (eluent: hexane / ethyl acetate) to give the title compound.

[Examples 2 to 128]

Compounds of Examples 2 to 43 were synthesized according to the method of Example 1 Steps 1 to 3. Compounds of Examples 44 to 128 were synthesized according to the method of Example 1 Step 1 and 2. The necessary raw materials are listed in the following table. "ArSO2Cl" in the subsequent tables refers to an arylsulfonyl chloride compound used in a reaction with starting materials in each Example.

[Table 10]

| Example | Starting Material | ArSO2Cl | Name of the Synthesized Compound |
|---|---|---|---|
| 2 | Reference Example D10 | E22 | 6-Chloro-3-(N-((1S,2R)-2-(2,3-dihydro-1H-inden-4-yl)-1-(5-oxo-4, 5-dihydro-1,3,4-oxadiazol-2-yl)propyl)sulfamoyl) picolinamide |

(continued)

| Example | Starting Material | ArSO2Cl | Name of the Synthesized Compound |
|---|---|---|---|
| 3 | Reference Example D6 | 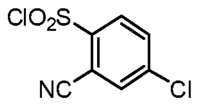 | 5-chloro-2-(N-((1S,2R)-2-(2,3-dimethylphenyl)-1-(5-oxo-4,5-dihydro-1,3,4-oxadiazol-2-yl)propyl)sulfamoyl)benzamide |
| 4 | Reference Example D6 | 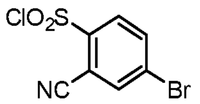 | 5-bromo-2-(N-((1S,2R)-2-(2,3-dimethylphenyl)-1-(5-oxo-4,5-dihydro-1,3,4-oxadiazol-2-yl)propyl)sulfamoyl)benzamide |
| 5 | Reference Example D1 | 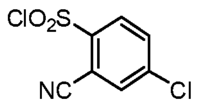 | 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl) benzamide |
| 6 | Reference Example D3 | 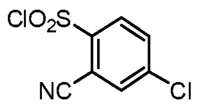 | 5-chloro-2-(N-((1S,2R)-2-(2-fluoronaphthalen-1-yl)-1-(5-oxo-4,5-dihydro-1,3, 4-oxadiazol-2-yl)propyl)sulfamoyl)benzamide |
| 7 | Reference Example D7 | 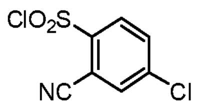 | 5-chloro-2-(N-((1S,2R)-2-(5-fluoro-2,3-dimethylphenyl)-1-(5-oxo-4, 5-dihydro-1,3, 4- oxadiazol-2-yl)propyl)sulfamoyl) benzamide |
| 8 | Reference Example D20 | 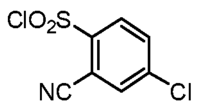 | 5-chloro-2-(N-((1S,2R)-2-(8-fluoronaphthalen-1 -yl)-1-(5-oxo-4, 5-dihydro-1,3,4-oxadiazol-2-yl)propyl)sulfamoyl)benzamide |
| 9 | Reference Example D8 | 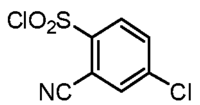 | 5-chloro-2-(N-((1S,2R)-2-(3-fluoronaphthalen-1-yl)-1-(5-oxo-4,5-dihydro-1,3,4-oxadiazol-2-yl)propyl)sulfamoyl)benzamide |
| 10 | Reference Example D1 | 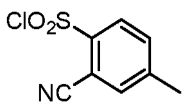 | 2-(N-((1S,2R)-2-(3-6-fluoro-2,3-dimethylphenyl)-1-(5-oxo-4,5-dihydro- 1,3,4-oxadiazol-2-yl)propyl)sulfamoyl)-5-methylbenzamide |
| 11 | Reference Example D45 | 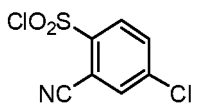 | 5-chloro-2-(N-((1S,2R)-2-(3-ethyl-6-fluoro-2-methylphenyl)-1-(5-oxo-4,5-dihydro-1,3,4-oxadiazol-2-yl)propyl)sulfamoyl) benzamide |
| 12 | Reference Example D3 | 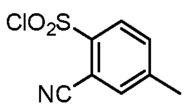 | 2-(N-((1S,2R)-2-(2-Fluoronaphthalen-1-yl)-1-(5-oxo-4,5-dihydro-1,3,4-oxadiazol-2-yl)propyl)sulfamoyl)-5-methylbenzamide |
| 13 | Reference Example D46 | 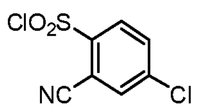 | 5-chloro-2-(N-((1S,2R)-2-(2,3-difluoro-5,6-dimethylphenyl)-1-(5-oxo-4,5-dihydro-1,3,4-oxadiazol-2-yl)propyl)sulfamoyl) benzamide |
| 14 | Reference Example D4 | 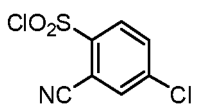 | 5-chloro-2-(N-((1S,2R)-2-(3-chloro-6-fluoro-2-methylphenyl)-1-(5-oxo-4,5-dihydro-1,3,4-oxadiazol-2-yl)propyl)sulfamoyl) benzamide |
| 15 | Reference Example D3 | 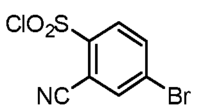 | 5-bromo-2-(N-((1S,2R)-2-(2-fluoronaphthalen-1-yl)-1-(5-oxo-4,5-dihydro-1,3,4-oxadiazol-2-yl)propyl)sulfamoyl)benzamide |

(continued)

| Example | Starting Material | ArSO2Cl | Name of the Synthesized Compound |
|---|---|---|---|
| 16 | Reference Example D1 | Reference Example E20 | 5-Cyclopropyl-2-(N-((1S,2R)-2-(6-Fluoro-2,3-dimethylphenyl)-1-(5-oxo-4,5-dihydro-1,3,4-oxadiazol-2-yl)propyl)sulfamoyl)benzamide |
| 17 | Reference Example D15 | 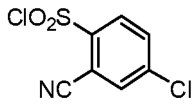 | 5-chloro-2-(N-((1S)-2-(2-chloro-6-fluoro-3-methylphenyl)-1-(5-oxo-4,5-dihydro-1,3,4-oxadiazol-2-yl)propyl)sulfamoyl)benzamide |
| 18 | Reference Example D1 | Reference Example E21 | 5-Ethyl-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide |
| 19 | Reference Example D1 | Reference Example E22 | 6-Chloro-3-(N-((1S,2R)-2-(6-fluoro-2,3-dimethylphenyl)-1-(5-oxo-4,5-dihydro-1,3,4-oxadiazol-2-yl)propyl)sulfamoyl)picolinamide |
| 20 | Reference Example D1 | 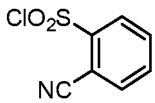 | 2-(N-((1S,2R)-2-(6-fluoro-2,3-dimethylphenyl)-1-(5-oxo-4,5-dihydro-1,3,4-oxadiazol-2-yl)propyl)sulfamoyl)benzamide |
| 21 | Reference Example D16 | 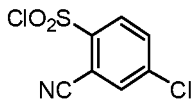 | 5-chloro-2-(N-((1S,2R)-2-(2-fluoro-5-methylphenyl)-1-(5-oxo-4,5-dihydro-1,3,4-oxadiazol-2-yl)propyl)sulfamoyl)benzamide |
| 22 | Reference Example D17 | 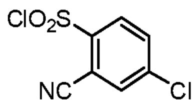 | 5-chloro-2-(N-((1S,2R)-2-(2-fluoro-6-methylphenyl)-1-(5-oxo-4,5-dihydro-1,3,4-oxadiazol-2-yl)propyl)sulfamoyl)benzamide |
| 23 | Reference Example D18 | 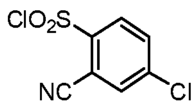 | 5-chloro-2-(N-((1S,2S)-2-(2-fluoro-6-methylphenyl)-1-(5-oxo-4,5-dihydro-1,3,4-oxadiazol-2-yl)propyl)sulfamoyl) benzamide |
| 24 | Reference Example D37 | 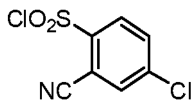 | 5-chloro-2-(N-((1S,2R)-1-(5-oxo-4,5-dihydro-1,3,4-oxadiazol-2-yl)-2-(o-tolyl)propyl)sulfamoyl)benzamide |
| 25 | Reference Example D4 | 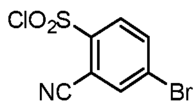 | 5-bromo-2-(N-((1S,2R)-2-(3-chloro-6-fluoro-2-methylphenyl)-1-(5-oxo-4,5-dihydro-1,3,4-oxadiazol-2-yl)propyl)sulfamoyl)benzamide |
| 26 | Reference Example D47 | 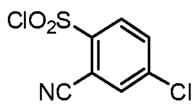 | 5-chloro-2-(N-((1S,2R)-2-(3-cyclopropyl-6-fluoro-2-methylphenyl)-1-(5-oxo-4,5-dihydro-1,3,4-oxadiazol-2-yl)propyl)sulfamoyl)benzamide |
| 27 | Reference Example D48 | 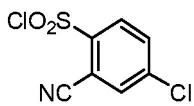 | 5-chloro-2-(N-((1S,2R)-2-(6-fluoro-2-methyl-3-(trifluoromethyl)phenyl)-1-(5-oxo-4,5-dihydro-1,3,4-oxadiazol-2-yl)propyl)sulfamoyl)benzamide |
| 28 | Reference Example D19 | 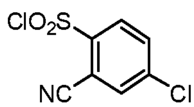 | 5-chloro-2-(N-((1S,2R)-2-(3,6-difluoro-2-methylphenyl)-1-(5-oxo-4,5-dihydro-1,3,4-oxadiazol-2-yl)propyl)sulfamoyl)benzamide |

(continued)

| Example | Starting Material | ArSO2Cl | Name of the Synthesized Compound |
|---|---|---|---|
| 29 | Reference Example D49 | 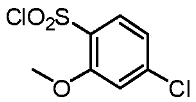 | 3-((1S,2R)-1-(4-chloro-2-methoxyphenylsulfonamido)-1-(5-oxo-4,5-dihydro-1,3,4-oxadiazol-2-yl)propan-2-yl)-4-fluoro-2-methylbenzamide |
| 30 | Reference Example D5 | 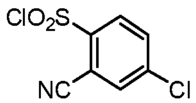 | 2-(N-((1S,2R)-2-(3-bromo-6-fluoro-2-methylphenyl)-1-(5-oxo-4,5-dihydro-1,3,4-oxadiazol-2-yl)propyl)sulfamoyl)-5-chlorobenzamide |
| 31 | Reference Example D1 | Reference Example E18 | 3-chloro-6-(N-((1S,2R)-2-(6-fluoro-2,3-dimethylphenyl)-1-(5-oxo-4,5-dihydro-1,3,4-oxadiazol-2-yl)propyl)sulfamoyl) picolinamide |
| 32 | Reference Example D2 | 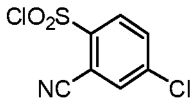 | 5-chloro-2-(N-((1S,2S)-2-(6-fluoro-2,3-dimethylphenyl)-1-(5-oxo-4,5-dihydro-1,3,4-oxadiazol-2-yl)propyl)sulfamoyl) benzamide |
| 33 | Reference Example D57 | 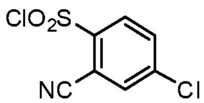 | 5-chloro-2-(N-((1R,2S)-2-(6-fluoro-2,3-dimethylphenyl)-1-(5-oxo-4,5-dihydro-1,3,4-oxadiazol-2-yl)propyl)sulfamoyl) benzamide |
| 34 | Reference Example D58 | 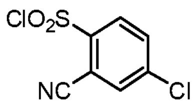 | 5-chloro-2-(N-((1R,2R)-2-(6-fluoro-2,3-dimethylphenyl)-1-(5-oxo-4,5-dihydro-1,3,4-oxadiazol-2-yl)propyl)sulfamoyl) benzamide |
| 35 | Reference Example D5 | 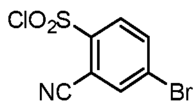 | 5-bromo-2-(N-((1S,2R)-2-(3-bromo-6-fluoro-2-methylphenyl)-1-(5-oxo-4,5-dihydro-1,3,4-oxadiazol-2-yl)propyl)sulfamoyl) benzamide |
| 36 | Reference Example D1 | Reference Example E67 | 2-(N-((1S,2R)-2-(6-fluoro-2,3-dimethylphenyl)-1-(5-oxo-4,5-dihydro-1,3,4-oxadiazol-2-yl)propyl)sulfamoyl)-4-(morpholine-4-carbonyl)benzamide |
| 37 | Reference Example D1 | Reference Example E68 | 3-(N-((1S,2R)-2-(6-fluoro-2,3-dimethylphenyl)-1-(5-oxo-4,5-dihydro-1,3,4-oxadiazol-2-yl)propyl)sulfamoyl)-N1,N1-dimethylterephthalamide |
| 38 | Reference Example D1 | Reference Example E16 | 4-carbamoyl-2-chloro-5-(N-((1S,2R)-2-(6-fluoro-2,3-dimethylphenyl)-1-(5-oxo-4,5-dihydro-1,3,4-oxadiazol-2-yl) propyl)sulfamoyl)benzoic acid |
| 39 | Reference Example D1 | Reference Example E69 | 2-chloro-5-(N-((1S,2R)-2-(6-fluoro-2,3-dimethylphenyl)-1-(5-oxo-4,5-dihydro-1,3,4-oxadiazol-2-yl)propyl) sulfamoyl)-N1,N1-dimethyl terephthalamide |
| 40 | Reference Example D1 | Reference Example E32 | 2-chloro-5-(N-((1S,2R)-2-(6-fluoro-2,3-dimethylphenyl)-1-(5-oxo-4,5-dihydro-1,3,4-oxadiazol-2-yl)propyl)sulfamoyl) isonicotinamide |
| 41 | Reference Example D1 | 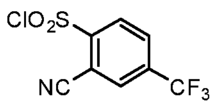 | 2-(N-((1S,2R)-2-(6-fluoro-2,3-dimethylphenyl)-1-(5-oxo-4,5-dihydro-1,3,4-oxadiazol-2-yl)propyl)sulfamoyl)-5-(trifluoromethyl)benzamide |
| 42 | Reference Example D5 | 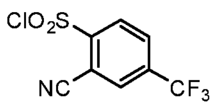 | 2-(N-((1S,2R)-2-(3-bromo-6-fluoro-2-methylphenyl)-1-(5-oxo-4,5-dihydro-1,3,4-oxadiazol-2-yl)propyl)sulfamoyl)-5-(trifluoromethyl)benzamide |

(continued)

| Example | Starting Material | ArSO2Cl | Name of the Synthesized Compound |
|---|---|---|---|
| 43 | Reference Example D44 | 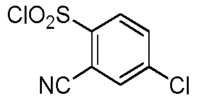 | 5-chloro-2-[[(1S,2R)-3,3,3-trideuterio-2-(6-fluoro-2,3-dimethylphenyl)-1-(2-oxo-3H-1,3,4-oxadiazol-5-yl)propyl] sulfamoyl]benzamide |
| 44 | Reference Example D11 | 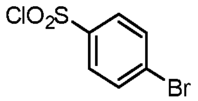 | 4-bromo-N-((1S,2R)-2-(naphthalen-1-yl)-1-(5-oxo-4,5-dihydro-1,3,4-oxadiazol-2-yl)propyl)benzenesulfonamide |
| 45 | Reference Example D27 | 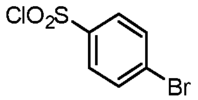 | N-((1S,2R)-2-(benzo[b]thiophen-4-yl)-1-(5-oxo-4,5-dihydro-1,3,4-oxadiazol-2-yl)propyl)-4-bromobenzenesulfonamide |
| 46 | Reference Example D11 | 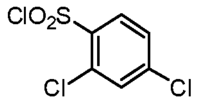 | 2,4-dichloro-N-((1S,2R)-2-(naphthalen-1-yl)-1-(5-oxo-4,5-dihydro-1,3,4-oxadiazol-2-yl)propyl)benzenesulfonamide |
| 47 | Reference Example D21 | 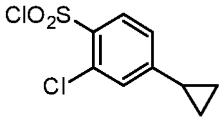 | 2-chloro-4-cyclopropyl-N-((1S,2R)-1-(5-oxo-4,5-dihydro-1,3,4-oxadiazol-2-yl)-2-(5,6,7,8-tetrahydronaphthalen-1-yl) propyl)benzenesulfonamide |
| 48 | Reference Example D51 | 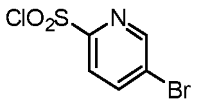 | 5-bromo-N-((1S)-2-(3-methyl-2,3-dihydro-1 H-inden-4-yl)-1-(5-oxo-4,5-dihydro-1,3,4-oxadiazol-2-yl)propyl)pyridine-2-sulfonamide |
| 49 | Reference Example D22 | 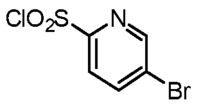 | N-((1S,2R)-2-(9H-fluoren-1-yl)-1-(5-oxo-4,5-dihydro-1,3,4-oxadiazol-2-yl)propyl)-5-bromopyridine-2-sulfonamide |
| 50 | Reference Example D23 | 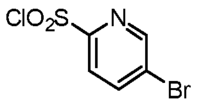 | N-((1S,2R)-2-(9H-fluoren-4-yl)-1-(5-oxo-4,5-dihydro-1,3,4-oxadiazol-2-yl)propyl)-5-bromopyridine-2-sulfonamide |
| 51 | Reference Example D11 | 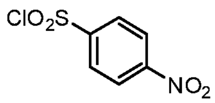 | N-((1S,2R)-2-(naphthalen-1-yl)-1-(5-oxo-4,5-dihydro-1,3,4-oxadiazol-2-yl)propyl)-4-nitrobenzene sulfonamide |
| 52 | Reference Example D21 | 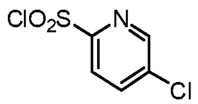 | 5-chloro-N-((1S,2R)-1-(5-oxo-4,5-dihydro-1,3,4-oxadiazol-2-yl)-2-(5,6,7,8-tetrahydronaphthalen-1-yl)propyl)pyridine-2-sulfonamide |
| 53 | Reference Example D21 | 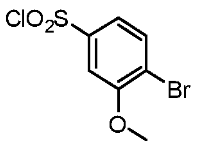 | 4-bromo-3-methoxy-N-((1S,2R)-1-(5-oxo-4,5-dihydro-1,3,4-oxadiazol-2-yl)-2-(5,6,7,8-tetrahydronaphthalen-1-yl)propyl) benzene sulfonamide |
| 54 | Reference Example D21 | 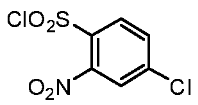 | 4-chloro-2-nitro-N-((1S,2R)-1-(5-oxo-4,5-dihydro-1,3,4-oxadiazol-2-yl)-2-(5,6,7,8-tetrahydronaphthalen-1-yl)propyl) benzene sulfonamide |
| 55 | Reference Example D21 | 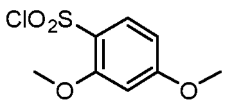 | 2,4-dimethoxy-N-((1S,2R)-1-(5-oxo-4,5-dihydro-1,3,4-oxadiazol-2-yl)-2-(5,6,7,8-tetrahydronaphthalen-1-yl)propyl) benzene sulfonamide |

(continued)

| Example | Starting Material | ArSO2Cl | Name of the Synthesized Compound |
|---|---|---|---|
| 56 | Reference Example D24 | 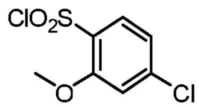 | 4-chloro-N-((1S,2R)-2-(6-fluoro-naphthalen-1-yl)-1-(5-oxo-4,5-dihydro-1,3,4-oxadiazol-2-yl)propyl)-2-methoxybenzenesulfonamide |
| 57 | Reference Example D21 | 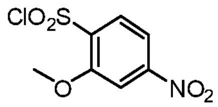 | 2-methoxy-4-nitro-N-((1S,2R)-1-(5-oxo-4,5-dihydro-1,3,4-oxadiazol-2-yl)-2-(5,6,7,8-tetrahydronaphthalen-1-yl)propyl) benzenesulfonamide |
| 58 | Reference Example D21 | 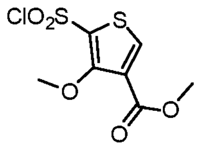 | methyl 4-methoxy-5-(N-((1S,2R)-1-(5-oxo-4,5-dihydro-1,3,4-oxadiazol-2-yl)-2-(5,6,7,8-tetrahydronaphthalen-1-yl)propyl) sulfamoyl)thiophene-3-carboxylate |
| 59 | Reference Example D10 | 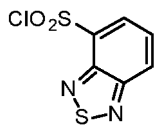 | N-((1S,2R)-2-(2,3-dihydro-1H-inden-4-yl)-1-(5-oxo-4,5-dihydro-1,3,4-oxadiazol-2yl)propyl)benzo[c][1,2,5] thiadiazole-4-sulfonamide |
| 60 | Reference Example D10 | 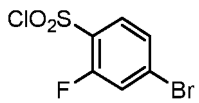 | 4-bromo-N-((1S,2R)-2-(2,3-dihydro-1H-inden-4-yl)-1-(5-oxo-4,5-dihydro-1,3,4-oxadiazol-2-yl)propyl)-2-fluorobenzenesulfonamide |
| 61 | Reference Example D10 | 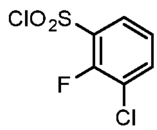 | 3-chloro-N-((1S,2R)-2-(2,3-dihydro-1H-inden-4-yl)-1-(5-oxo-4,5-dihydro-1,3,4-oxadiazol-2-yl)propyl)-2-fluorobenzenesulfonamide |
| 62 | Reference Example D33 | 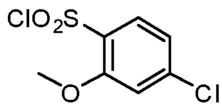 | N-((1S,2R)-2-(benzo[b]thiophen-3-yl)-1-(5-oxo-4,5-dihydro-1,3,4-oxadiazol-2-yl)propyl)-4-chloro-2-methoxybenzenesulfonamide |
| 63 | Reference Example D40 | 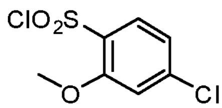 | N-((1S,2R)-2-(benzo[d]thiazol-4-yl)-1-(5-oxo-4,5-dihydro-1,3,4-oxadiazol-2-yl)propyl)-4-chloro-2-methoxybenzenesulfonamide |
| 64 | Reference Example D30 | 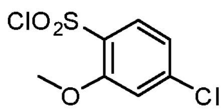 | 4-chloro-N-((1S,2R)-2-(2,3-dihydrobenzofuran-7-yl)-1-(5-oxo-4,5-dihydro-1,3,4-oxadiazol-2-yl)propyl)-2-methoxybenzenesulfonamide |
| 65 | Reference Example D31 | 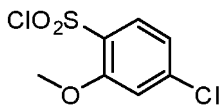 | 4-chloro-2-methoxy-N-((1S,2R)-2-(2-methylnaphthalen-1-yl)-1-(5-oxo-4,5-dihydro-1,3,4-oxadiazol-2-yl)propyl) benzenesulfonamide |
| 66 | Reference Example D29 | 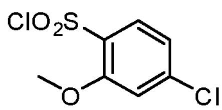 | 4-chloro-N-((1S,2R)-2-(2,3-dihydrobenzofuran-4-yl)-1-(5-oxo-4,5-dihydro-1,3,4-oxadiazol-2-yl)propyl)-2-methoxybenzenesulfonamide |
| 67 | Reference Example D53 | 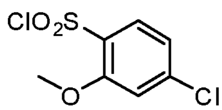 | 4-chloro-2-methoxy-N-((1S,2R)-2-(2-methyl-2,3-dihydro-1H-inden-4-yl)-1-(5-oxo-4,5-dihydro-1,3,4-oxadiazol-2-yl)propyl) benzenesulfonamide |

(continued)

| Example | Starting Material | ArSO2Cl | Name of the Synthesized Compound |
|---|---|---|---|
| 68 | Reference Example D10 | 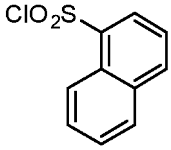 | N-((1S,2R)-2-(2,3-dihydro-1H-inden-4-yl)-1-(5-oxo-4,5-dihydro-1,3,4-oxadiazol-2-yl)propyl)naphthalene-1-sulfonamide |
| 69 | Reference Example D52 | 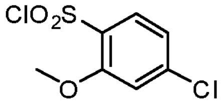 | 4-chloro-2-methoxy-N-((1S,2S)-2-(3-methyl-2,3-dihydro-1H-inden-4-yl)-1-(5-oxo-4,5-dihydro-1,3,4-oxadiazol-2-yl)propyl) benzenesulfonamide |
| 70 | Reference Example D28 | 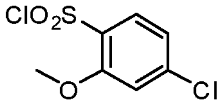 | 4-chloro-2-methoxy-N-((1S,2R)-2-(2-methyl-[1,1'-biphenyl]-3-yl)-1-(5-oxo-4,5-dihydro-1,3,4-oxadiazol-2-yl)propyl) benzenesulfonamide |
| 71 | Reference Example D13 | 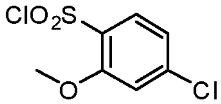 | 4-chloro-2-methoxy-N-((1S,2R)-2-(8-methylnaphthalen-1-yl)-1-(5-oxo-4,5-dihydro-1,3,4-oxadiazol-2-yl)propyl) benzenesulfonamide |
| 72 | Reference Example D35 | 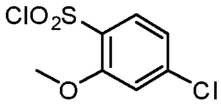 | 4-chloro-2-methoxy-N-((1S,2R)-2-(3-methyl-2,3-dihydrobenzofuran-4-yl)-1-(5-oxo-4,5-dihydro-1,3,4-oxadiazol-2-yl)propyl)benzenesulfonamide |
| 73 | Reference Example D36 | 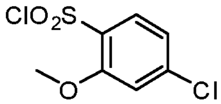 | 4-chloro-2-methoxy-N-((1S,2S)-2-(3 -methyl-2,3 -dihydrobenzofuran-4-yl)-1-(5-oxo-4,5-dihydro-1,3,4-oxadiazol-2-yl)propyl)benzenesulfonamide |
| 74 | Reference Example D34 | 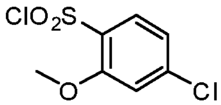 | 4-chloro-N-((1S)-2-(2,3-difluoropheiryl)-1-(5-oxo-4,5-dihydro-1,3,4-oxadiazol-2-yl)propyl)-2-methoxybenzenesulfonamide |
| 75 | Reference Example D32 | 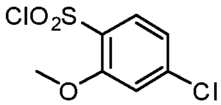 | 4-chloro-N-((1S,2R)-2-(3-fluoro-2-methylphenyl)-1-(5-oxo-4,5-dihydro-1,3,4-oxadiazol-2-yl)propyl)-2-methoxybenzenesulfonamide |
| 76 | Reference Example D3 | 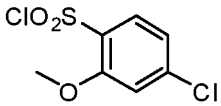 | 4-chloro-N-((1S,2R)-2-(2-fluoronaphthalen-1-yl)-1-(5-oxo-4,5-dihydro-1,3,4-oxadiazol-2-yl)propyl)-2 -methoxybenzenesulfonamide |
| 77 | Reference Example D9 | 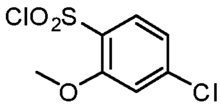 | 4-chloro-N-((1S,2R)-2-(4-fluoro-2,3-dimethylphenyl)-1-(5-oxo-4,5-dihydro-1,3,4-oxadiazol-2-yl)propyl)-2-methoxybenzenesulfonamide |
| 78 | Reference Example D55 | 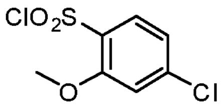 | (S)-4-chloro-2-methoxy-N-(2-(8-methylnaphthalen-1-yl)-1-(5-oxo-4,5-dihydro-1,3,4-oxadiazol-2-yl)ethyl) benzenesulfonamide |
| 79 | Reference Example D38 | 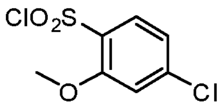 | 4-chloro-N-((1S)-2-(2,6-difluoro-3-methylphenyl)-1-(5-oxo-4,5-dihydro-1,3,4-oxadiazol-2-yl)propyl)-2-methoxybenzenesulfonamide |

(continued)

| Example | Starting Material | ArSO2Cl | Name of the Synthesized Compound |
|---|---|---|---|
| 80 | Reference Example D39 | 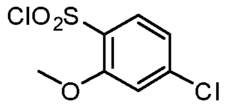 | 4-chloro-N-((1S)-2-(2-fluoro-3-methylphenyl)-1-(5-oxo-4,5-dihydro-1,3,4-oxadiazol-2-yl)propyl)-2-methoxybenzenesulfonamide |
| 81 | Reference Example D13 | Reference Example E57 | 5-chloro-4,4-difluoro-N-((1S,2R)-2-(8-methylnaphthalen-1-yl)-1-(5-oxo-4,5-dihydro-1,3,4-oxadiazol-2-yl)propyl) chroman-8-sulfonamide |
| 82 | Reference Example D25 | 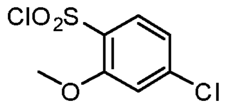 | 4-chloro-N-((1S,2R)-2-(5-fluoronaphthalen-1-yl)-1-(5-oxo-4,5-dihydro-1,3,4-oxadiazol-2-yl)propyl)-2-methoxybenzenesulfonamide |
| 83 | Reference Example D1 | 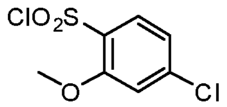 | 4-chloro-N-((1S,2R)-2-(6-fluoro-2,3-dimethylphenyl)-1-(5-oxo-4,5-dihydro-1,3,4-oxadiazol-2-yl)propyl)-2-methoxybenzenesulfonamide |
| 84 | Reference Example D1 | 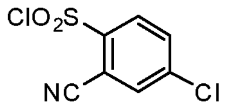 | 4-chloro-2-cyano-N-((1S,2R)-2-(6-fluoro-2,3-dimethylphenyl)-1-(5-oxo-4,5-dihydro-1,3,4-oxadiazol-2-yl)propyl) benzenesulfonamide |
| 85 | Reference Example D14 | 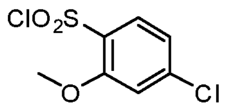 | 4-chloro-N-((1S,2R)-2-(2-isopropyl-3-methylphenyl)-1-(5-oxo-4,5-dihydro-1,3,4-oxadiazol-2-yl)propyl)-2-methoxybenzenesulfonamide |
| 86 | Reference Example D12 | 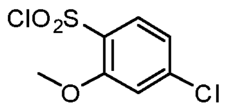 | 4-chloro-N-((1S,2R)-2-(3-ethyl-2-methylphenyl)-1-(5-oxo-4,5-dihydro-1,3,4-oxadiazol-2-yl)propyl)-2-methoxybenzenesulfonamide |
| 87 | Reference Example D42 | 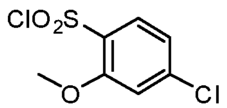 | 4-chloro-N-((1S,2R)-2-(2-ethyl-3-methylphenyl)-1-(5-oxo-4,5-dihydro-1,3,4-oxadiazol-2-yl)propyl)-2-methoxybenzenesulfonamide |
| 88 | Reference Example D1 | Reference Example E7 | 5-chloro-N-((1S,2R)-2-(6-fluoro-2,3-dimethylphenyl)-1-(5-oxo-4,5-dihydro-1,3,4-oxadiazol-2-yl)propyl)-4-oxochroman-8-sulfonamide |
| 89 | Reference Example D50 | 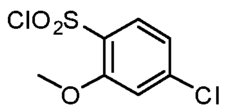 | N-((1S)-2-(2-bromo-5,6-difluoro-3-methylphenyl)-1-(5-oxo-4,5-dihydro-1,3,4-oxadiazol-2-yl)propyl)-4-chloro-2-methoxybenzenesulfonamide |
| 90 | Reference Example D46 | 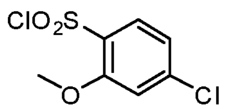 | 4-chloro-N-((1S,2R)-2-(2,3-difluoro-5,6-dimethylphenyl)-1-(5-oxo-4,5-dihydro-1,3,4-oxadiazol-2-yl)propyl)-2-methoxybenzenesulfonamide |
| 91 | Reference Example D1 | Reference Example E50 | 4-chloro-N-((1S,2R)-2-(6-fluoro-2,3-dimethylphenyl)-1-(5-oxo-4,5-dihydro-1,3,4-oxadiazol-2-yl)propyl)-2-(isoxazol-5-yl) benzenesulfonamide |
| 92 | Reference Example D1 | 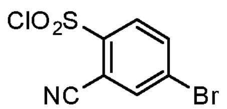 | 4-bromo-2-cyano-N-((1S,2R)-2-(6-fluoro-2,3-dimethylphenyl)-1-(5-oxo-4,5-dihydro-1,3,4-oxadiazol-2-yl) propyl)benzenesulfonamide |

(continued)

| Example | Starting Material | ArSO2Cl | Name of the Synthesized Compound |
|---|---|---|---|
| 93 | Reference Example D1 | Reference Example E64 | 5-chloro-N-((1S,2R)-2-(6-fluoro-2,3-dimethylphenyl)-1-(5-oxo-4,5-dihydro-1,3,4-oxadiazol-2-yl)propyl)-4-methyl-3-oxo-3,4-dihydro-2H-benzo[b][1,4]oxazine-8-sulfonamide |
| 94 | Reference Example D1 | Reference Example E61 | 5-chloro-4-ethyl-N-((1S,2R)-2-(6-fluoro-2,3-dimethylphenyl)-1-(5-oxo-4,5-dihydro-1,3,4-oxadiazol-2-yl)propyl)-3,4-dihydro-2H-benzo[b][1,4]oxazine-8-sulfonamide |
| 95 | Reference Example D1 | Reference Example E2 | 5-chloro-8-(N-((1S,2R)-2-(6-fluoro-2,3-dimethylphenyl)-1-(5-oxo-4,5-dihydro-1,3,4-oxadiazol-2-yl)propyl)sulfamoyl)-4-methylchroman-4-yl acetate |
| 96 | Reference Example D45 | 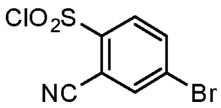 | 5-bromo-2-(N-((1S,2R)-2-(3-ethyl-6-fluoro-2-methylphenyl)-1-(5-oxo-4,5-dihydro-1,3,4-oxadiazol-2-yl)propyl)sulfamoyl) benzamide |
| 97 | Reference Example D1 | Reference Example E23 | N-((1S,2R)-2-(6-fluoro-2,3-dimethylphenyl)-1-(5-oxo-4,5-dihydro-1,3,4-oxadiazol-2-yl)propyl)-2,2-dimethyl-4-oxochroman-8-sulfonamide |
| 98 | Reference Example D1 | 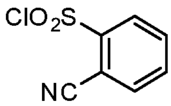 | 2-cyano-N-((1S,2R)-2-(6-fluoro-2,3-dimethylphenyl)-1-(5-oxo-4,5-dihydro-1,3,4-oxadiazol-2-yl)propyl) benzenesulfonamide |
| 99 | Reference Example D1 | Reference Example E62 | 4-(cyclopropanecarbonyl)-N-((1S,2R)-2-(6-fluoro-2,3-dimethylphenyl)-1-(5-oxo-4,5-dihydro-1,3,4-oxadiazol-2-yl) propyl)-3,4-dihydro-2H-benzo[b][1,4]oxazine-8-sulfonamide |
| 100 | Reference Example D4 | Reference Example E2 | 5-chloro-8-(N-((1S,2R)-2-(3-chloro-6-fluoro-2-methylphenyl)-1-(5-oxo-4,5-dihydro-1,3,4-oxadiazol-2-yl)propyl)sulfamoyl)-4-methylchroman-4-yl acetate |
| 101 | Reference Example D1 | Reference Example E63 | 5-chloro-4-(2,2-difluoroethyl)-N-((1S,2R)-2-(6-fluoro-2,3-dimethylphenyl)-1-(5-oxo-4,5-dihydro-1,3,4-oxadiazol-2-yl) propyl)-3-oxo-3,4-dihydro-2H-benzo[b][1,4]oxazine-8-sulfonamide |
| 102 | Reference Example D1 | Reference Example E66 | 5-chloro-4-(2,2-difluoroethyl)-N-((1S,2R)-2-(6-fluoro-2,3-dimethylphenyl)-1-(5-oxo-4,5-dihydro-1,3,4-oxadiazol-2-yl) propyl)-3,4-dihydro-2H-benzo[b][1,4]oxazine-8-sulfonamide |
| 103 | Reference Example D1 | 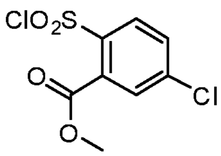 | methyl 5-chloro-2-(N-((1S,2R)-2-(6-fluoro-2,3-dimethylphenyl)-1-(5-oxo-4,5-dihydro-1,3,4-oxadiazol-2-yl) propyl)sulfamoyl)benzoate |
| 104 | Reference Example D49 | 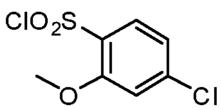 | 4-chloro-N-((1S,2R)-2-(3-cyano-6-fluoro-2-methylphenyl)-1-(5-oxo-4,5-dihydro-1,3,4-oxadiazol-2-yl)propyl)-2-methoxybenzenesulfonamide |
| 105 | Reference Example D1 | Reference Example E18 | 5-chloro-6-cyano-N-((1S,2R)-2-(6-fluoro-2,3-dimethylphenyl)-1-(5-oxo-4,5-dihydro-1,3,4-oxadiazol-2-yl)propyl)pyridine-2-sulfonamide |
| 106 | Reference Example D1 | Reference Example E6 | 8-(N-((1S,2R)-2-(6-fluoro-2,3-dimethylphenyl)-1-(5-oxo-4,5-dihydro-1,3,4-oxadiazol-2-yl)propyl)sulfamoyl)-4-(tifluoromethyl)chroman-4-yl acetate |

(continued)

| Example | Starting Material | ArSO2Cl | Name of the Synthesized Compound |
|---|---|---|---|
| 107 | Reference Example D1 | Reference Example E25 | 4-chloro-N-((1S,2R)-2-(6-fluoro-2,3-dimethylphenyl)-1-(5-oxo-4,5-dihydro-1,3,4-oxadiazol-2-yl)propyl)-8-oxo-5,6,7,8-tetrahydronaphthalene-1-sulfonamide |
| 108 | Reference Example D1 | Reference Example E46 | 2-(6-chloro-3-(N-((1S,2R)-2-(6-fluoro-2,3-dimethylphenyl)-1-(5-oxo-4,5-dihydro-1,3,4-oxadiazol-2-yl)propyl)sulfamoyl)-2-methoxyphenyl)propan-2-yl acetate |
| 109 | Reference Example D1 | Reference Example E10 | methyl 3-chloro-6-(N-((1S,2R)-2-(6-fluoro-2,3-dimethylphenyl)-1-(5-oxo-4,5-dihydro-1,3,4-oxadiazol-2-yl) propyl)sulfamoyl)picolinate |
| 110 | Reference Example D1 | 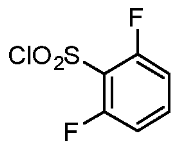 | 2,6-difluoro-N-((1S,2R)-2-(6-fluoro-2,3-dimethylphenyl)-1-(5-oxo-4,5-dihydro-1,3,4-oxadiazol-2-yl)propyl) benzenesulfonamide |
| 111 | Reference Example D1 | 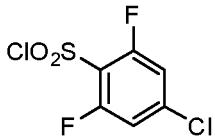 | 4-chloro-2,6-difluoro-N-((1S,2R)-2-(6-fluoro-2,3-dimethylphenyl)-1-(5-oxo-4,5-dihydro-1,3,4-oxadiazol-2-yl) propyl)benzenesulfonamide |
| 112 | Reference Example D1 | Reference Example E27 | 5-chloro-N-((1S,2R)-2-(6-fluoro-2,3-dimethylphenyl)-1-(5-oxo-4,5-dihydro-1,3,4-oxadiazol-2-yl)propyl)-3-methoxypyridine-2-sulfonamide |
| 113 | Reference Example D1 | Reference Example E37 | 5-chloro-N-((1S,2R)-2-(6-fluoro-2,3-dimethylphenyl)-1-(5-oxo-4,5-dihydro-1,3,4-oxadiazol-2-yl)propyl)-6-(morpholine-4-carbonyl)pyridine-2-sulfonamide |
| 114 | Reference Example D1 | Reference Example E35 | 5-chloro-N-((1S,2R)-2-(6-fluoro-2,3-dimethylphenyl)-1-(5-oxo-4,5-dihydro-1,3,4-oxadiazol-2-yl)propyl)-6-(pyrrolidine-1-carbonyl)pyridine-2-sulfonamide |
| 115 | Reference Example D1 | Reference Example E43 | 5-chloro-N-((1S,2R)-2-(6-fluoro-2,3-dimethylphenyl)-1-(5-oxo-4,5-dihydro-1,3,4-oxadiazol-2-yl)propyl)-6-(6-azaspiro [3.4]octane-6-carbonyl)pyridine-2-sulfonamide |
| 116 | Reference Example D1 | Reference Example E39 | 6-(3-oxa-8-azabicyclo[3.2.1]octane-8-carbonyl)-5-chloro-N-((1S,2R)-2-(6-fluoro-2,3-dimethylphenyl)-1-(5-oxo-4,5-dihydro-1,3,4-oxadiazol-2-yl)propyl)pyridine-2-sulfonamide |
| 117 | Reference Example D1 | Reference Example E40 | 6-(8-oxa-3-azabicyclo[3.2.1]octane-3-carbonyl)-5-chloro-N-((1S,2R)-2-(6-fluoro-2,3-dimethylphenyl)-1-(5-oxo-4,5-dihydro-1,3,4-oxadiazol-2-yl)propyl)pyridine-2-sulfonamide |
| 118 | Reference Example D1 | Reference Example E11 | methyl 5-chloro-2-(N-((1S,2R)-2-(6-fluoro-2,3-dimethylphenyl)-1-(5-oxo-4,5-dihydro-1,3,4-oxadiazol-2-yl) propyl)sulfamoyl)nicotinate |
| 119 | Reference Example D1 | Reference Example E73 | methyl 5-bromo-2-(N-((1S,2R)-2-(6-fluoro-2,3-dimethylphenyl)-1-(5-oxo-4,5-dihydro-1,3,4-oxadiazol-2-yl) propyl)sulfamoyl)nicotinate |
| 120 | Reference Example D1 | Reference Example E45 | 1-(5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)-3-methoxypyridin-4-yl)ethyl acetate |

(continued)

| Example | Starting Material | ArSO2Cl | Name of the Synthesized Compound |
|---|---|---|---|
| 121 | Reference Example D1 | Reference Example E28 | methyl 5-chloro-4-fluoro-2-(N-((1S,2R)-2-(6-fluoro-2,3-dimethylphenyl)-1-(5-oxo-4,5-dihydro-1,3,4-oxadiazol-2-yl) propyl)sulfamoyl)benzoate |
| 122 | Reference Example D1 | Reference Example E14 | methyl 5-chloro-2-(N-((1S,2R)-2-(6-fluoro-2,3-dimethylphenyl)-1-(5-oxo-4,5-dihydro-1,3,4-oxadiazol-2-yl) propyl)sulfamoyl)-4-methoxybenzoate |
| 123 | Reference Example D1 | Reference Example E5 | 5-chloro-8-(N-((1S,2R)-2-(6-fluoro-2,3-dimethylphenyl)-1-(5-oxo-4,5-dihydro-1,3,4-oxadiazol-2-yl)propyl)sulfamoyl)-4-(trifluoromethyl)chroman-4-yl acetate |
| 124 | Reference Example D5 | Reference Example E7 | N-((1S,2R)-2-(3-bromo-6-fluoro-2-methylphenyl)-1-(5-oxo-4,5-dihydro-1,3,4-oxadiazol-2-yl)propyl)-5-chloro-4-oxochroman-8-sulfonamide |
| 125 | Reference Example D59 | 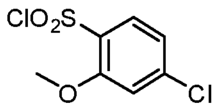 | (S)-4-chloro-N-(2-(6-fluoro-2,3-dimethylphenyl)-2-methyl-1-(5-oxo-4,5-dihydro-1,3,4-oxadiazol-2-yl)propyl)-2-methoxybenzenesulfonamide |
| 126 | Reference Example D54 | 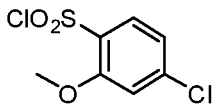 | (S)-4-chloro-N-(2-(6-fluoro-2,3-dimethylphenyl)-1-(5-oxo-4,5-dihydro-1,3,4-oxadiazol-2-yl)ethyl)-2-methoxybenzenesulfonamide |
| 127 | Reference Example D60 | 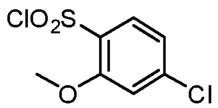 | (S)-4-chloro-N-((1-(6-fluoro-2,3-dimethylphenyl)cyclopropyl)(5 -oxo-4,5-dihydro-1,3,4-oxadiazol-2-yl)methyl)-2-methoxybenzenesulfonamide |
| 128 | Reference Example D43 | 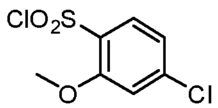 | 4-chloro-N-((1S,2R)-2-(6-fluoro-2,3-dimethylphenyl)-1-(5-oxo-4,5-dihydro-1,3,4-oxadiazol-2-yl)butyl)-2-methoxybenzenesulfonamide |

[Example 129]

Synthesis of 5-Chloro-8-(N-((1S,2R)-2-(6-fluoro-2,3-dimethylphenyl)-1-(5-oxo-4,5-dihydro-1,3,4-oxadiazol-2-yl)propyl)sulfamoyl)-4-methyl-d3-chroman-4-yl acetate

To a pyridine (1.5 mL) solution of 5-((1S,2R)-1-amino-2-(6-fluoro-2,3-dimethylphenyl)propyl)-1,3,4-oxadiazol-2(3H)-one monohydrochloride (45 mg) obtained from Reference Example F1, 5-chloro-8-(chlorosulfonyl)-4-methyl-d3-chroman-4-ylacetate (80 mg) obtained in Reference Example E1 was added, and the reaction solution was stirred at room temperature for 12 hours. The reaction solution was concentrated under reduced pressure, and the obtained residue was purified by silica gel column chromatography (eluent: hexane / ethyl acetate) to obtain the title compound (59 mg) as a 1: 1 diastereomer mixture.

[Examples 130 to 185]

According to the method of Example 129, the following compounds of Examples 130 to 185 and Examples 338 to 343 were synthesized. The necessary raw materials are listed in the following table.

[Table 11]

| Example | Starting Material | ArSO2Cl | Name of the Synthesized Compound |
|---|---|---|---|
| 130 | Reference Example F6 | 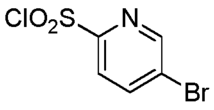 | 5-bromo-N-((1S,2R)-1-(5-oxo-4,5-dihydro-1,3,4-oxadiazol-2-yl)-2-(quinoline-8-yl)propyl)pyridine-2-sulfonamide |
| 131 | Reference Example F5 | 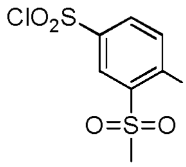 | N-((1S,2R)-2-(2,3-dihydro-1H-inden-4-yl)-1-(5-oxo-4,5-dihydro-1,3,4-oxadiazol-2-yl)propyl)-4-fluoro-3-(methylsulfonyl)benzenesulfonamide |
| 132 | Reference Example F5 | 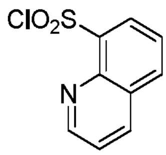 | N-((1S,2R)-2-(2,3-dihydro-1H-inden-4-yl)-1-(5-oxo-4,5-dihydro-1,3,4-oxadiazol-2-yl)propyl)quinoline-8-sulfonamide |
| 133 | Reference Example F5 | 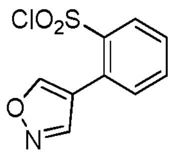 | N-((1S,2R)-2-(2,3-dihydro-1H-inden-4-yl)-1-(5-oxo-4,5-dihydro-1,3,4-oxadiazol-2-yl)propyl)-2-(isoxazol-4-yl) benzenesulfonamide |
| 134 | Reference Example F 5 | 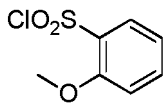 | N-((1S,2R)-2-(2,3-dihydro-1H-inden-4-yl)-1-(5-oxo-4,5-dihydro-1,3,4-oxadiazol-2-yl)propyl)-2-methoxybenzenesulfonamide |
| 135 | Reference Example F 5 | 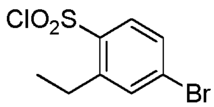 | 4-bromo-N-((1S,2R)-2-(2,3-dihydro-1H-inden-4-yl)-1-(5-oxo-4,5-dihydro-1,3,4-oxadiazol-2-yl)propyl)-2-ethylbenzenesulfonamide |
| 136 | Reference Example F5 | 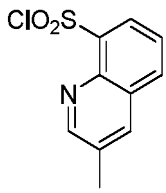 | N-((1S,2R)-2-(2,3-dihydro-1H-inden-4-yl)-1-(5-oxo-4,5-dihydro-1,3,4-oxadiazol-2-yl)propyl)-3-methylquinoline-8-sulfonamide |
| 137 | Reference Example F 5 | 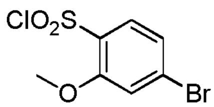 | 4-bromo-N-((1S,2R)-2-(2,3-dihydro-1H-inden-4-yl)-1-(5-oxo-4,5-dihydro-1,3,4-oxadiazol-2-yl)propyl)-2 -methoxybenzenesulfonamide |
| 138 | Reference Example F5 | 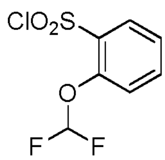 | 2-(difluoromethoxy)-N-((1S,2R)-2-(2,3-dihydro-1H-inden-4-yl)-1-(5-oxo-4,5-dihydro-1,3,4-oxadiazol-2-yl)propyl) benzenesulfonamide |
| 139 | Reference Example F5 | 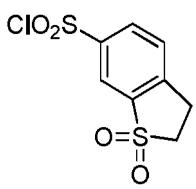 | N-((1S,2R)-2-(2,3-dihydro-1H-inden-4-yl)-1-(5-oxo-4,5-dihydro-1,3,4-oxadiazol-2-yl)propyl)-2,3-dihydrobenzo[b] thiophene-6-sulfonamide 1,1-dioxide |

(continued)

| Example | Starting Material | ArSO2Cl | Name of the Synthesized Compound |
|---|---|---|---|
| 140 | Reference Example F5 | 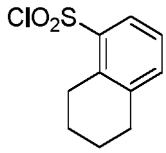 | N-((1S,2R)-2-(2,3-dihydro-1H-inden-4-yl)-1-(5-oxo-4,5-dihydro-1,3,4-oxadiazol-2-yl)propyl)-5,6,7,8-tetrahydronaphthalene-1-sulfonamide |
| 141 | Reference Example F 5 | 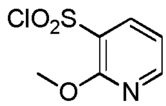 | N-((1S,2R)-2-(2,3-dihydro-1H-inden-4-yl)-1-(5-oxo-4,5-dihydro-1,3,4-oxadiazol-2-yl)propyl)-2-methoxypyridine-3-sulfonamide |
| 142 | Reference Example F5 | E34 | 1-(3-chloro-6-(N-((1S,2R)-2-(2,3-dihydro-1H-inden-4-yl)-1-(5-oxo-4,5-dihydro-1,3,4-oxadiazol-2-yl)propyl) sulfamoyl)pyridin-2-yl)ethylacetate |
| 143 | Reference Example F5 | 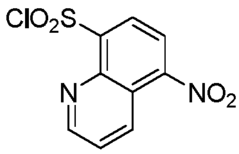 | N-((1S,2R)-2-(2,3-dihydro-1H-1-(5-oxo-4,5-dihydro-1,3,4-oxadiazol-2-yl)propyl)-5-nitroquinoline-8-sulfonamide |
| 144 | Reference Example F1 | Reference Example E44 | 1-(6-chloro-3-(N-((1S,2R)-2-(6-fluoro-2,3-dimethylphenyl)-1-(5-oxo-4,5-dihydro-1,3,4-oxadiazol-2-yl)propyl) sulfamoyl)-2-methoxyphenyl)ethyl acetate |
| 145 | Reference Example F1 | Reference Example E47 | 4-chloro-2-(2,2-difluoroethoxy)-N-((1S,2R)-2-(6-fluoro-2,3-dimethylphenyl)-1-(5-oxo-4,5-dihydro-1,3,4-oxadiazol-2-yl) propyl)benzenesulfonamide |
| 146 | Reference Example F1 | Reference Example E48 | 4-chloro-2-(difluoromethoxy)-N-((1S,2R)-2-(6-fluoro-2,3-dimethylphenyl)-1-(5-oxo-4,5-dihydro-1,3,4-oxadiazol-2-yl) propyl)benzenesulfonamide |
| 147 | Reference Example F1 | Reference Example E19 | 2-acetyl-4-chloro-N-((1S,2R)-2-(6-fluoro-2,3-dimethylphenyl)-1-(5-oxo-4,5-dihydro-1,3,4-oxadiazol-2-yl) propyl)benzenesulfonamide |
| 148 | Reference Example F1 | 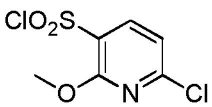 | 6-chloro-N-((1S,2R)-2-(6-fluoro-2,3-dimethylphenyl)-1-(5-oxo-4,5-dihydro-1,3,4-oxadiazol-2-yl)propyl)-2-methoxypyridine-3-sulfonamide |
| 149 | Reference Example F1 | E54 | 4-chloro-N-((1S,2R)-2-(6-fluoro-2,3-dimethylphenyl)-1-(5-oxo-4,5-dihydro-1,3,4-oxadiazol-2-yl)propyl)-2-(5-oxo-4,5-dihydro-1,3,4-oxadiazol-2-yl)benzenesulfonamide |
| 150 | Reference Example F2 | 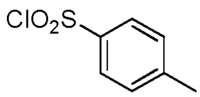 | N-((1S,2R)-2-(2,3-dimethylphenyl)-1-(5-oxo-4,5-dihydro-1,3,4-oxadiazol-2-yl)propyl)-4-methylbenzenesulfonamide |
| 151 | Reference Example F1 | 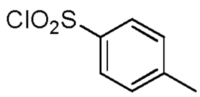 | N-((1S,2R)-2-(6-fluoro-2,3-dimethylphenyl)-1-(5-oxo-4,5-dihydro-1,3,4-oxadiazol-2-yl)propyl)-4-methylbenzenesulfonamide |
| 152 | Reference Example F1 | E60 | N-((1S,2R)-2-(6-fluoro-2,3-dimethylphenyl)-1-(5-oxo-4,5-dihydro-1,3,4-oxadiazol-2-yl)propyl)-1H-indole-4-sulfonamide |

(continued)

| Example | Starting Material | ArSO2Cl | Name of the Synthesized Compound |
|---|---|---|---|
| 153 | Reference Example F 1 | 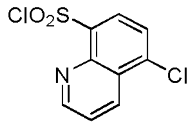 | 5-chloro-N-((1S,2R)-2-(6-fluoro-2,3-dimethylphenyl)-1-(5-oxo-4,5-dihydro-1,3,4-oxadiazol-2-yl)propyl)quinoline-8-sulfonamide |
| 154 | Reference Example F1 | 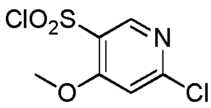 | 6-chloro-N-((1S,2R)-2-(6-fluoro-2,3-dimethylphenyl)-1-(5-oxo-4,5-dihydro-1,3,4-oxadiazol-2-yl)propyl)-4-methoxypyridine-3-sulfonamide |
| 155 | Reference Example F1 | Reference Example E38 | 5-chloro-N-((1S,2R)-2-(6-fluoro-2,3-dimethylphenyl)-1-(5-oxo-4,5-dihydro-1,3,4-oxadiazol-2-yl)propyl)-6-(2-oxa-6-azaspiro[3.4]octane-6-carbonyl)pyridine-2-sulfonamide |
| 156 | Reference Example F1 | Reference Example E42 | 5-chloro-N-((1S,2R)-2-(6-fluoro-2,3-dimethylphenyl)-1-(5-oxo-4,5-dihydro-1,3,4-oxadiazol-2-yl)propyl)-6-(2-oxa-7-azaspiro[3.5]nonane-7-carbonyl)pyridine-2-sulfonamide |
| 157 | Reference Example F1 | Reference Example E71 | N-(5-chloro-8-(N-((1S,2R)-2-(6-fluoro-2,3-dimethylphenyl)-1-(5-oxo-4,5-dihydro-1,3,4-oxadiazol-2-yl)propyl) sulfamoyl)chroman-4-yl)acetamide |
| 158 | Reference Example F1 | 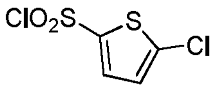 | 5-chloro-N-((1S,2R)-2-(6-fluoro-2,3-dimethylphenyl)-1-(5-oxo-4,5-dihydro-1,3,4-oxadiazol-2-yl)propyl)thiophene-2-sulfonamide |
| 159 | Reference Example F1 | Reference Example E8 | 5-fluoro-N-((1S,2R)-2-(6-fluoro-2,3-dimethylphenyl)-1-(5-oxo-4,5-dihydro-1,3,4-oxadiazol-2-yl)propyl)-4-oxochroman-8-sulfonamide |
| 160 | Reference Example F1 | 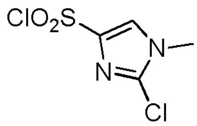 | 2-chloro-N-((1S,2R)-2-(6-fluoro-2,3-dimethylphenyl)-1-(5-oxo-4,5-dihydro-1,3,4-oxadiazol-2-yl)propyl)-1-methyl-1H-imidazole-4-sulfonamide |
| 161 | Reference Example F1 | 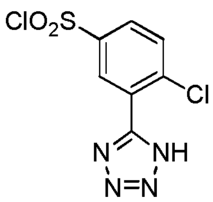 | 4-chloro-N-((1S,2R)-2-(6-fluoro-2,3-dimethylphenyl)-1-(5-oxo-4,5-dihydro-1,3,4-oxadiazol-2-yl)propyl)-3-(1H-tetrazol-5-yl)benzenesulfonamide |
| 162 | Reference Example F1 | 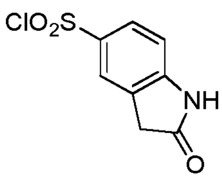 | N-((1S,2R)-2-(6-fluoro-2,3-dimethylphenyl)-1-(5-oxo-4,5-dihydro-1,3,4-oxadiazol-2-yl)propyl)-2-oxoindoline-5-sulfonamide |
| 163 | Reference Example F1 | 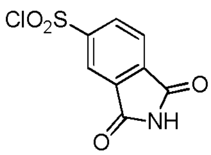 | N-((1S,2R)-2-(6-fluoro-2,3-dimethylphenyl)-1-(5-oxo-4,5-dihydro-1,3,4-oxadiazol-2-yl)propyl)-1,3-dioxoisoindoline-5-sulfonamide |

(continued)

| Example | Starting Material | ArSO2Cl | Name of the Synthesized Compound |
|---|---|---|---|
| 164 | Reference Example F1 | 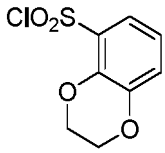 | N-((1S,2R)-2-(6-fluoro-2,3-dimethylphenyl)-1-(5-oxo-4,5-dihydro-1,3,4-oxadiazol-2-yl)propyl)-2,3-dihydrobenzo[b][1,4]dioxin-5-sulfonamide |
| 165 | Reference Example F1 | 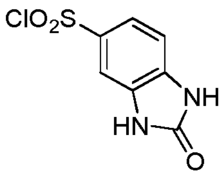 | N-((1S,2R)-2-(6-fluoro-2,3-dimethylphenyl)-1-(5-oxo-4,5-dihydro-1,3,4-oxadiazol-2-yl)propyl)-2-oxo-2,3-dihydro-1H-benzo[d]imidazole-5-sulfonamide |
| 166 | Reference Example F1 | 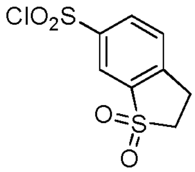 | N-((1S,2R)-2-(6-fluoro-2,3-dimethylphenyl)-1-(5-oxo-4,5-dihydro-1,3,4-oxadiazol-2-yl)propyl)-2,3-dihydrobenzo[b]thiophene-6-sulfonamide 1,1-dioxide |
| 167 | Reference Example F8 | Reference Example E2 | 8-(N-((1S,2R)-2-(3-bromo-6-fluoro-2-methylphenyl)-1-(5-oxo-4,5-dihydro-1,3,4-oxadiazol-2-yl)propyl)sulfamoyl)-5-chloro-4-methylchroman-4-yl acetate |
| 168 | Reference Example F1 | 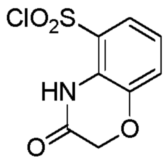 | N-((1S,2R)-2-(6-fluoro-2,3-dimethylphenyl)-1-(5-oxo-4,5-dihydro-1,3,4-oxadiazol-2-yl)propyl)-3-oxo-3,4-dihydro-2H-benzo[b][1,4]oxazine-5-sulfonamide |
| 169 | Reference Example F1 | 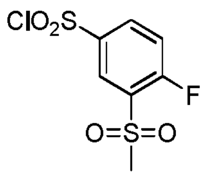 | 4-fluoro-N-((1S,2R)-2-(6-fluoro-2,3-dimethylphenyl)-1-(5-oxo-4,5-dihydro-1,3,4-oxadiazol-2-yl)propyl)-3-(methylsulfonyl)benzenesulfonamide |
| 170 | Reference Example F1 | 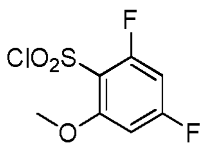 | 2,4-difluoro-N-((1S,2R)-2-(6-fluoro-2,3-dimethylphenyl)-1-(5-oxo-4,5-dihydro-1,3,4-oxadiazol-2-yl)propyl)-6-methoxybenzenesulfonamide |
| 171 | Reference Example F1 | 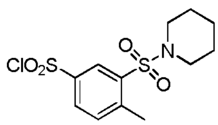 | N-((1S,2R)-2-(6-fluoro-2,3-dimethylphenyl)-1-(5-oxo-4,5-dihydro-1,3,4-oxadiazol-2-yl)propyl)-4-methyl-3-(piperidin-1-ylsulfonyl)benzene sulfonamide |
| 172 | Reference Example F1 | 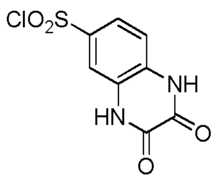 | N-((1S,2R)-2-(6-fluoro-2,3 -dimethylphenyl)-1-(5-oxo-4,5-dihydro-1,3,4-oxadiazol-2-yl)propyl)-2,3-dioxo-1,2,3,4-tetrahydroquinoxaline-6-sulfonamide |

(continued)

| Example | Starting Material | ArSO2Cl | Name of the Synthesized Compound |
|---|---|---|---|
| 173 | Reference Example F1 | 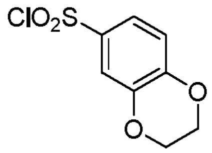 | N-((1S,2R)-2-(6-fluoro-2,3-dimethylphenyl)-1-(5-oxo-4,5-dihydro-1,3,4-oxadiazol-2-yl)propyl)-2,3-dihydrobenzo[b][1,4]dioxin-6-sulfonamide |
| 174 | Reference Example F1 | 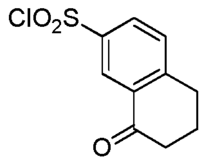 | N-((1S,2R)-2-(6-fluoro-2,3-dimethylphenyl)-1-(5-oxo-4,5-dihydro-1,3,4-oxadiazol-2-yl)propyl)-8-oxo-5,6,7,8-tetrahydronaphthalene-2-sulfonamide |
| 175 | Reference Example F1 | 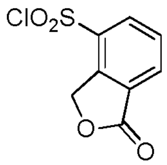 | N-((1S,2R)-2-(6-fluoro-2,3 -dimethylphenyl)-1-(5-oxo-4,5-dihydro-1,3,4-oxadiazol-2-yl)propyl)-1-oxo-1,3-dihydroisobenzofuran-4-sulfonamide |
| 176 | Reference Example F 1 | 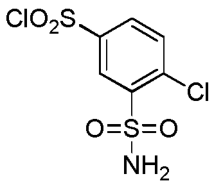 | 4-chloro-N1-((1S,2R)-2-(6-fluoro-2,3-dimethylphenyl)-1-(5-oxo-4,5-dihydro-1,3,4-oxadiazol-2-yl)propyl)benzene-1,3-disulfonamide |
| 177 | Reference Example F1 | 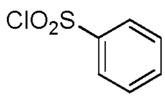 | N-((1S,2R)-2-(6-fluoro-2,3-dimethylphenyl)-1-(5-oxo-4,5-dihydro-1,3,4-oxadiazol-2-yl)propyl)benzenesulfonamide |
| 178 | Reference Example F1 | 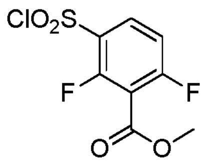 | methyl 2,6-difluoro-3-(N-((1S,2R)-2-(6-fluoro-2,3-dimethylphenyl)-1-(5-oxo-4,5-dihydro-1,3,4-oxadiazol-2-yl)propyl)sulfamoyl)benzoate |
| 179 | Reference Example F1 | 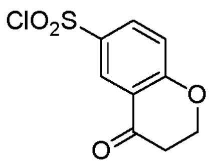 | N-((1S,2R)-2-(6-fluoro-2,3-dimethylphenyl)-1-(5-oxo-4,5-dihydro-1,3,4-oxadiazol-2-yl)propyl)-4-oxochroman-6-sulfonamide |
| 180 | Reference Example F1 | Reference Example E53 | methyl 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)-4-nitrobenzoate |
| 181 | Reference Example F1 | 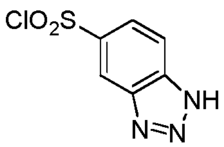 | N-((1S,2R)-2-(6-fluoro-2,3-dimethylphenyl)-1-(5-oxo-4,5-dihydro-1,3,4-oxadiazol-2-yl)propyl)-1H-benzo[d][1,2,3]triazole-5-sulfonamide |
| 182 | Reference Example F1 | 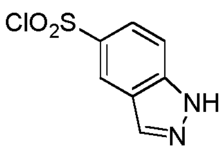 | N-((1S,2R)-2-(6-fluoro-2,3-dimethylphenyl)-1-(5-oxo-4,5-dihydro-1,3,4-oxadiazol-2-yl)propyl)-1H-indazole-5-sulfonamide |

(continued)

| Example | Starting Material | ArSO2Cl | Name of the Synthesized Compound |
|---|---|---|---|
| 183 | Reference Example F1 | 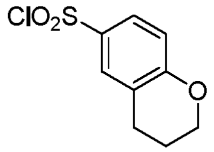 | N-((1S,2R)-2-(6-fluoro-2,3-dimethylphenyl)-1-(5-oxo-4,5-dihydro-1,3,4-oxadiazol-2-yl)propyl)chroman-6-sulfonamide |
| 184 | Reference Example F1 | 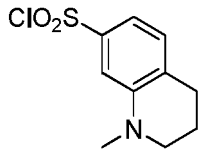 | N-((1S,2R)-2-(6-fluoro-2,3-dimethylphenyl)-1-(5-oxo-4,5-dihydro-1,3,4-oxadiazol-2-yl)propyl)-1-methyl-1,2,3,4-tetrahydroquinoline-7-sulfonamide |
| 185 | Reference Example F10 | 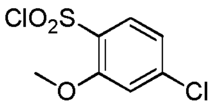 | (S)-4-chloro-N-(2-(6-fluoro-2,3-dimethylphenyl)-1-(5-oxo-4,5-dihydro-1,3,4-oxadiazol-2-yl)allyl)-2-methoxybenzenesulfonamide |
| 338 | Reference Example F1 | 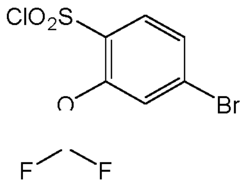 | 4-bromo-N-((1S,2R)-(6-fluoro-2,3-dimethylphenyl)-1-(5-oxo-4,5-dihydro-1,3,4-oxadiazol-2-yl)propyl)-2-(trifluoromethoxy)benzenesulfonamide |
| 339 | Reference Example F1 | 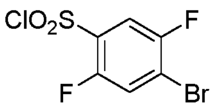 | 4-bromo-2,5-difluoro-N-((1S,2R)-(6-fluoro-2,3-dimethylphenyl)-1-(5-oxo-4,5-dihydro-1,3,4-oxadiazol-2-yl) propyl)benzenesulfonamide |
| 340 | Reference Example F1 | 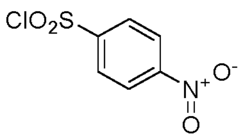 | N-((1S,2R)-(6-fluoro-2,3-dimethylphenyl)-1-(5-oxo-4,5-dihydro-1,3,4-oxadiazol-2-yl)propyl)-4-nitrobenzenesulfonamide |
| 341 | Reference Example F1 | 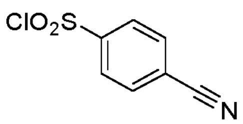 | 4-cyano-N-((1S,2R)-(6-fluoro-2,3-dimethylphenyl)-1-(5-oxo-4,5-dihydro-1,3,4-oxadiazol-2-yl)propyl) benzenesulfonamide |
| 342 | Reference Example F1 | 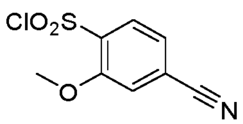 | 4-cyano-N-((1S,2R)-(6-fluoro-2,3-dimethylphenyl)-1-(5-oxo-4,5-dihydro-1,3,4-oxadiazol-2-yl)propyl)-2-methoxybenzenesulfonamide |
| 343 | Reference Example F1 | 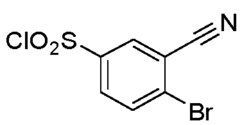 | 4-bromo-3-cyano-N-((1S,2R)-(6-fluoro-2,3-dimethylphenyl)-1-(5-oxo-4,5-dihydro-1,3,4-oxadiazol-2-yl) propyl)benzenesulfonamide |

[Example 186]

Synthesis of 2,4-difluoro-N-((1S,2R)-2-(6-fluoro-2,3-dimethylphenyl)-1-(5-oxo-4,5-dihydro-1,3,4-oxadiazol-2-yl)propyl)-3-(2-hydroxypropan-2-yl)benzenesulfonamide

(Step 1)

Methyl 3-(chlorosulfonyl)-2,6-difluorobenzoate (33 mg) was added to a pyridine (1.0 mL) solution of 5-((1S,2R)-1-amino-2-(6-fluoro-2,3-dimethylphenyl)propyl)-1,3,4-oxadiazol-2(3H)-one monohydrochloride (20 mg) obtained from Reference Example F1, and the reaction solution was stirred for 12 hours at room temperature. The reaction solution was concentrated under reduced pressure, and the obtained residue was purified by silica gel column chromatography (eluent: hexane / ethyl acetate) to obtain methyl 2,6-difluoro-3-(N-((1S,2R)-2-(6-fluoro-2,3-dimethylphenyl)-1-(5-oxo-4,5-dihydro-1,3,4-oxadiazol-2-yl)propyl)sulfamoyl)benzoate (12.5 mg).

(Step 2)

To a THF (2.0 mL) solution of methyl 2,6-difluoro-3-(N-((1S,2R)-2-(6-fluoro-2,3-dimethylphenyl)-1-(5-oxo-4,5-dihydro-1,3,4-oxadiazol-2-yl)propyl)sulfamoyl)benzoate (12.5 mg) obtained from the above Step 1, a diethyl ether (3.0 M, 84 μL) solution of methylmagnesium bromide was added dropwise at 0°C, and the reaction solution was stirred for 1 hour at room temperature. A saturated ammonium chloride aqueous solution (10 mL) was added dropwise in an ice bath, ethyl acetate (10 mL) was added, and the layers were separated. The organic layer was washed successively with hydrochloric acid (1 M, 10 mL), water (10 mL) and saturated saline (10 mL), dried over anhydrous magnesium sulfate, and concentrated under reduced pressure. The residue was purified by silica gel column chromatography (eluent: hexane / ethyl acetate) to obtain the title compound.

[Examples 187 to 195]

According to the method of Example 186, the following compounds of Examples 187 to 195 were synthesized. The necessary raw materials are listed in the following table.

[Table 12]

| Example | Starting Material | ArSO2Cl | Name of the Synthesized Compound |
|---|---|---|---|
| 187 | Reference Example F5 | 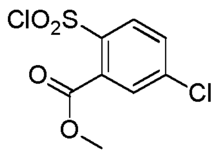 | 4-chloro-N-((1S,2R)-2-(2,3-dihydro-1H-inden-4-yl)-1-(5-oxo-4,5-dihydro-1,3,4-oxadiazol-2-yl)propyl)-2-(2-hydroxypropan-2-yl)benzenesulfonamide |
| 188 | Reference Example F5 | 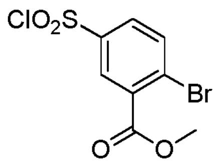 | 4-bromo-N-((1S,2R)-2-(2,3-dihydro-1H-inden-4-yl)-1-(5-oxo-4,5-dihydro-1,3,4-oxadiazol-2-yl)propyl)-3-(2-hydroxypropan-2-yl)benzenesulfonamide |
| 189 | Reference Example F5 | 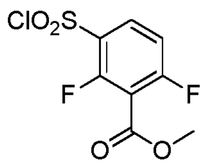 | N-((1S,2R)-2-(2,3-dihydro-1H-inden-4-yl)-1-(5-oxo-4,5-dihydro-1,3,4-oxadiazol-2-yl)propyl)-2,4-difluoro-3-(2-hydroxypropan-2-yl)benzenesulfonamide |
| 190 | Reference Example F5 | 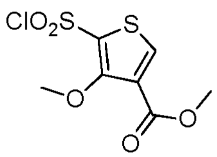 | N-((1S,2R)-2-(2,3-dihydro-1H-inden-4-yl)-1-(5-oxo-4,5-dihydro-1,3,4-oxadiazol-2-yl)propyl)-4-(2-hydroxypropan-2-yl)-3 -methoxythiophene-2-sulfonamide |
| 191 | Reference Example F5 | 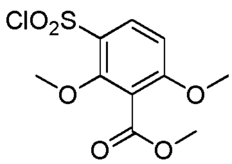 | N-((1S,2R)-2-(2,3-dihydro-1H-inden-4-yl)-1-(5-oxo-4,5-dihydro-1,3,4-oxadiazol-2-yl)propyl)-3-(2-hydroxypropan-2-yl)-2,4-dimethoxybenzenesulfonamide |
| 192 | Reference Example F5 | Reference Example E10 | 5-chloro-N-((1S,2R)-2-(2,3-dihydro-1H-inden-4-yl)-1-(5-oxo-4,5-dihydro-1,3,4-oxadiazol-2-yl)propyl)-6-(2-hydroxypropan-2-yl)pyridine-2-sulfonamide |

(continued)

| Example | Starting Material | ArSO2Cl | Name of the Synthesized Compound |
|---|---|---|---|
| 193 | Reference Example F1 | Reference Example E10 | 5-chloro-N-((1S,2R)-2-(6-fluoro-2,3-dimethylphenyl)-1-(5-oxo-4,5-dihydro-1,3,4-oxadiazol-2-yl)propyl)-6-(2-hydroxypropan-2-yl)pyridine-2-sulfonamide |
| 194 | Reference Example F1 | 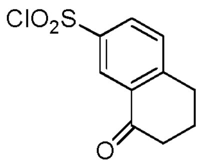 | N-((1S,2R)-2-(6-fluoro-2,3-dimethylphenyl)-1-(5-oxo-4,5-dihydro-1,3,4-oxadiazol-2-yl)propyl)-8-hydroxy-8-methyl-5,6,7,8-tetrahydronaphthalene-2-sulfonamide |
| 195 | Reference Example F1 | 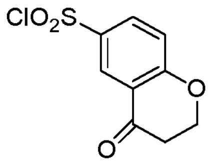 | N-((1S,2R)-2-(6-fluoro-2,3-dimethylphenyl)-1-(5-oxo-4,5-dihydro-1,3,4-oxadiazol-2-yl)propyl)-4-hydroxy-4-methylchroman-6-sulfonamide |

[Example 196]

Synthesis of 5-fluoro-2-(N-((1S,2R)-2-(6-fluoro-2,3-dimethylphenyl)-1-(5-oxo-4,5-dihydro-1,3,4-oxadiazol-2-yl)propyl)sulfamoyl)-4-(2-hydroxypropan-2-yl)benzamide

(Step 1)

From 5-((1S,2R)-1-amino-2-(6-fluoro-2,3-dimethylphenyl)propyl)-1,3,4-oxadiazol-2(3H)-one monohydrochloride (60 mg) obtained from Reference Example F1 and methyl 2-fluoro-5-(chlorosulfonyl)-4-cyanobenzoate (94 mg) obtained in Reference Example E15, in accordance with the method of Example 129, methyl 4-(cyano-2-fluoro-5 (N-((1S,2R)-2-(6-fluoro-2,3-dimethylphenyl)-1-(5-oxo-4,5-dihydro-1,3,4-oxadiazol-2-yl)propyl)sulfamoyl)benzoate (49 mg) was obtained.

(Step 2)

From methyl 4-cyano-2-fluoro-5-(N-((1S,2R)-2-(6-fluoro-2,3-dimethylphenyl)-1-(5-oxo-4,5-dihydro-1,3,4-oxadiazol-2-yl)propyl)sulfamoyl)benzoate (49 mg) obtained from the above Step 1, according to the method of Example 186 Step 2, 2-cyano-4-fluoro-N-((1S,2R)-2-(6-fluoro-2,3-dimethylphenyl)-1-(5-oxo-4,5-dihydro-1,3,4-oxadiazol-2-yl)propyl)-5-(2-hydroxypropan-2-yl)benzenesulfonamide (27.5 mg) was obtained.

(Step 3)

From 2-cyano-4-fluoro-N-((1S,2R)-2-(6-fluoro-2,3-dimethylpheny1)-1-(5-oxo-4,5-dihydro-1,3,4-oxadiazol-2-yl)propyl)-5-(2-hydroxypropan-2-yl)benzenesulfonamide (27.5 mg) obtained from the above Step 2, according to the method of Example 1 Step 3, the title compound was obtained.

[Examples 197 to 199]

According to the method of Example 129, Example 186 Step 2, Example 1 Step 3, the following compounds of Examples 197 to 199 were synthesized. The necessary raw materials are listed in the following table. However, for Example 199, the synthesis was carried out using 1-propynyl magnesium bromide instead of methyl magnesium bromide.

[Table 13]

| Example | Starting Material | ArSO2Cl | Name of the Synthesized Compound |
|---|---|---|---|
| 197 | Reference Example F1 | Reference Example E13 | 2-(N-((1S,2R)-2-(6-fluoro-2,3-dimethylphenyl)-1-(5-oxo-4,5-dihydro-1,3,4-oxadiazol-2-yl)propyl)sulfamoyl)-4-(2-hydroxypropan-2-yl)benzamide |
| 198 | Reference Example F1 | Reference Example E16 | 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)-4-(2-hydroxypropan-2-yl) benzamide |
| 199 | Reference Example F1 | Reference Example E13 | 2-(N-((1S,2R)-2-(6-fluoro-2,3-dimethylphenyl)-1-(5-oxo-4,5-dihydro-1,3,4-oxadiazol-2-yl)propyl)sulfamoyl)-4-(hydroxyhepta-2,5-diyn-4-yl) benzamide |

[Example 200]

Synthesis of 5-chloro-N-((1S,2R)-2-(6-fluoro-2,3-dimethylphenyl)-1-(5-oxo-4,5-dihydro-1,3,4-oxadiazol-2-yl)propyl)-4-hydroxy-4-methyl-d3-chroman-8-sulfonamide Isomer A and Isomer B

1 : 1 diastereomer mixture of 5-chloro-8-(N-((1S,2R)-2-(6-fluoro-2,3-dimethylphenyl)-1-(5-oxo-4,5-dihydro-1,3,4-oxadiazol-2-yl)propyl)sulfamoyl)-4-methyl-d3-chroman-4-yl acetate (59 mg) obtained from Example 129 was dissolved in methanol (2.0 mL) and water (1.0 mL), lithium hydroxide (5 mg) was added, and the reaction solution was stirred at 55°C for 1 hour. After concentrating the reaction solution, hydrochloric acid (1 M, 10 mL) and ethyl acetate (10 mL) were added to the residue, and the layers were separated. The aqueous layer was extracted with ethyl acetate (10 mL), and the combined organic layers were washed with saturated saline (10 mL). The organic layer was dried over anhydrous sodium sulfate and concentrated under reduced pressure. The residue was purified by reverse phase HPLC (water / acetonitrile), and the fractions were concentrated to give each of two diastereomeric products. The substance eluted first was designated Compound A, and the substance eluted later was designated as Compound B.

[Examples 201 to 229]

According to the method of Example 200, the following compounds of Examples 201 to 229 were synthesized. In the case of separating the diastereomers, the previously eluted compound was designated as A and the later eluted compound as B. The ratio of diastereomers is 1: 1 mixture unless otherwise specified. The necessary raw materials are listed in the following table.

[Table 14]

| Example | Starting Material | ArSO2Cl | Name of the Synthesized Compound |
|---|---|---|---|
| 201 | Reference Example F5 | Reference Example E33 | 2,4-dichloro-N-((1S,2R)-2-(2,3-dihydro-1H-inden-4-yl)-1-(5-oxo-4,5-dihydro-1,3,4-oxadiazol-2-yl)propyl)-3-(1-hydroxyethyl)benzenesulfonamide (diastereomer mixture) |
| 202 | Reference Example F5 | Reference Example E34 | 5-chloro-N-((1S,2R)-2-(2,3-dihydro-1H-inden-4-yl)-1-(5-oxo-4,5-dihydro-1,3,4-oxadiazol-2-yl)propyl)-6-(1-hydroxyethyl) pyridine-2-sulfonamide (diastereomer mixture) |
| 203 | Reference Example F2 | Reference Example E44 | 4-chloro-N-((1S,2R)-2-(2,3-dimethylphenyl)-1-(5-oxo-4,5-dihydro-1,3,4-oxadiazol-2-yl)propyl)-3-(1-hydroxyethyl)-2-methoxybenzenesulfonamide (diastereomer mixture) |
| 204 | Reference Example F1 | Reference Example E44 | 4-chloro-N-((1S,2R)-2-(6-fluoro-2,3-dimethylphenyl)-1-(5-oxo-4,5-dihydro-1,3,4-oxadiazol-2-yl)propyl)-3-(1-hydroxyethyl)-2-methoxybenzenesulfonamide (diastereomer mixture) |

(continued)

| Example | Starting Material | ArSO2Cl | Name of the Synthesized Compound |
|---|---|---|---|
| 205 | Reference Example F3 | Reference Example E44 | 4-chloro-N-((1S,2R)-2-(2-fluoronaphthalene-1-yl)-1-(5-oxo-4,5-dihydro-1,3,4-oxadiazol-2-yl)propyl)-3-(1-hydroxyethyl)-2-methoxybenzenesulfonamide (diastereomer mixture) |
| 206A | Reference Example F9 | Reference Example E44 | 4-chloro-N-((1S,2R)-2-(3-ethyl-6-fluoro-2-methylphenyl)-1-(5-oxo-4,5-dihydro-1,3,4-oxadiazol-2-yl)propyl)-3-(1-hydroxyethyl)-2-methoxybenzenesulfonamide |
| 207A 207B | Reference Example F1 | Reference Example E2 | 5-chloro-N-((1S,2R)-2-(6-fluoro-2,3-dimethylphenyl)-1-(5-oxo-4,5-dihydro-1,3,4-oxadiazol-2-yl)propyl)-4-hydroxy-4-methylchroman-8-sulfonamide |
| 208A 208B | Reference Example | Reference Example E44 | 4-chloro-N-((1S,2R)-2-(6-fluoro-2,3-dimethylphenyl)-1-(5-oxo-4,5-dihydro-1,3,4-oxadiazol-2-yl)propyl)-3-(1-hydroxyethyl)-2-methoxybenzenesulfonamide |
| 209A 209B | Reference Example F7 | Reference Example E2 | 5-chloro-N-((1S,2R)-2-(3-chloro-6-fluoro-2-methylphenyl)-1-(5-oxo-4,5-dihydro-1,3,4-oxadiazol-2-yl)propyl)-4-hydroxy-4-methylchroman-8-sulfonamide |
| 210 | Reference Example F1 | 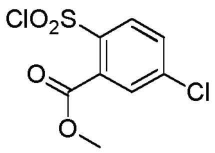 | 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl) benzoic acid |
| 211A | Reference Example F1 | Reference Example E6 | N-((1S,2R)-2-(6-fluoro-2,3-dimethylphenyl)-1-(5-oxo-4,5-dihydro-1,3,4-oxadiazol-2-yl)propyl)-4-hydroxy-4-(trifluoromethyl)chroman-8-sulfonamide |
| 212 | Reference Example F1 | Reference Example E46 | 4-chloro-N-((1S,2R)-2-(6-fluoro-2,3-dimethylphenyl)-1-(5-oxo-4,5-dihydro-1,3,4-oxadiazol-2-yl)propyl)-3-(2-hydroxypropan-2-yl)-2-methoxybenzenesulfonamide |
| 213 | Reference Example F1 | Reference Example E11 | 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl) nicotinic acid |
| 214 | Reference Example F1 | Reference Example E73 | 5-bromo-2-(N-((1S,2R)-2-(6-fluoro-2,3-dimethylphenyl)-1-(5-oxo-4,5-dihydro-1,3,4-oxadiazol-2-yl)propyl)sulfamoyl) nicotinic acid |
| 215 | Reference Example F1 | Reference Example E10 | 3-chloro-6-(N-((1S,2R)-2-(6-fluoro-2,3-dimethylphenyl)-1-(5-oxo-4,5-dihydro-1,3,4-oxadiazol-2-yl)propyl)sulfamoyl) picolinic acid |
| 216 | Reference Example F1 | Reference Example E45 | 5-chloro-N-((1S,2R)-2-(6-fluoro-2,3-dimethylphenyl)-1-(5-oxo-4,5-dihydro-1,3,4-oxadiazol-2-yl)propyl)-4-(1-hydroxyethyl)-3-methoxypyridine-2-sulfonamide (diastereomer mixture) |
| 217 | Reference Example F1 | Reference Example E28 | 5-chloro-4-fluoro-2-(N-((1S,2R)-2-(6-fluoro-2,3-dimethylphenyl)-1-(5-oxo-4,5-dihydro-1,3,4-oxadiazol-2-yl) propyl)sulfamoyl)benzoic acid |
| 218 | Reference Example F1 | Reference Example E12 | 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)-4-methylbenzoic acid |

(continued)

| Example | Starting Material | ArSO2Cl | Name of the Synthesized Compound |
|---|---|---|---|
| 219 | Reference Example F1 | Reference Example E29 | 5-chloro-3-fluoro-2-(N-((1S,2R)-2-(6-fluoro-2,3-dimethylphenyl)-1-(5-oxo-4,5-dihydro-1,3,4-oxadiazol-2-yl) propyl)sulfamoyl)benzoic acid |
| 220A 220B | Reference Example | Reference Example E5 | 5-chloro-N-((1S,2R)-2-(6-fluoro-2,3-dimethylphenyl)-1-(5-oxo-4,5-dihydro-1,3,4-oxadiazol-2-yl)propyl)-4-hydroxy-4-(trifluoromethyl)chroman-8-sulfonamide |
| 221 | Reference Example F1 | Reference Example E72 | 5-chloro-N-((1S,2R)-2-(6-fluoro-2,3-dimethylphenyl)-1-(5-oxo-4,5-dihydro-1,3,4-oxadiazol-2-yl)propyl)-6-(2,2,2-trifluoro-1-hydroxyethyl)pyridine-2-sulfonamide (diastereomer mixture) |
| 222A 222B | Reference Example F8 | Reference Example E2 | N-((1S,2R)-2-(3-bromo-6-fluoro-2-methylphenyl)-1-(5-oxo-4,5-dihydro-1,3,4-oxadiazol-2-yl)propyl)-5-chloro-4-hydroxy-4-methylchroman-8-sulfonamide |
| 223 | Reference Example F1 | Reference Example E17 | 7-(N-((1S,2R)-2-(6-fluoro-2,3-dimethylphenyl)-1-(5-oxo-4,5-dihydro-1,3,4-oxadiazol-2-yl)propyl)sulfamoyl)-2,3-dihydrobenzo[b][1,4]dioxin-6-Carboxylic Acid |
| 224A 224B | Reference Example F1 | Reference Example E3 | 5-fluoro-N-((1S,2R)-2-(6-fluoro-2,3-dimethylphenyl)-1-(5-oxo-4,5-dihydro -1,3,4-oxadiazol-2-yl)propyl)-4-hydroxy -4-methylchroman-8-sulfonamide |
| 225A | Reference Example F1 | Reference Example E4 | N-((1S,2R)-2-(6-fluoro-2,3-dimethylphenyl)-1-(5-oxo-4,5-dihydro-1,3,4-oxadiazol-2-yl)propyl)-4-hydroxy-4-methyl-5-(tifluoromethyl)chroman-8-sulfonamide |
| 226A 226B | Reference Example F8 | Reference Example E1 | N-((1S,2R)-2-(3-bromo-6-fluoro-2-methylphenyl)-1-(5-oxo-4,5-dihydro-1,3,4-oxadiazol-2-yl)propyl)-5-chloro-4-hydroxy-4-methyl-d3-chroman-8-sulfonamide |
| 227A 227B | Reference Example F8 | Reference Example E3 | N-((1S,2R)-2-(3-bromo-6-fluoro-2-methylphenyl)-1-(5-oxo-4,5-dihydro-1,3,4-oxadiazol-2-yl)propyl)-5-fluoro-4-hydroxy-4-methyl-d3 -chroman-8-sulfonamide |
| 228A 228B | Reference Example F7 | Reference Example E1 | 5-chloro-N-((1S,2R)-2-(3-chloro-6-fluoro-2-methylphenyl)-1-(5-oxo-4,5-dihydro-1,3,4-oxadiazol-2-yl)propyl)-4-hydroxy-4-methyl-d3-chroman-8-sulfonamide |
| 229A 229B | Reference Example F7 | Reference Example E3 | N-((1S,2R)-2-(3-chloro-6-fluoro-2-methylphenyl)-1-(5-oxo-4,5-dihydro-1,3,4-oxadiazol-2-yl)propyl)-5-fluoro-4-hydroxy-4-methylchroman-8-sulfonamide |

[Example 230]

Synthesis of 5-fluoro-N-((1S,2R)-2-(6-fluoro-2,3-dimethylphenyl)-1-(5-oxo-4,5-dihydro-1,3,4-oxadiazol-2-yl)propyl)-4-hydroxychroman-8-sulfonamide Isomer A and Isomer B

(Step 1)

Using 5-((1S,2R)-1-amino-2-(6-fluoro-2,3-dimethylphenyl)propyl)-1,3,4-oxadiazol-2(3 H)-one monohydrochloride (40 mg) obtained from Reference Example F1 and 5-fluoro-4-oxochroman-8-sulfonyl chloride (60 mg) obtained from Reference Example E8, 5-fluoro-N-((1S,2R)-2-(6-fluoro-2,3-dimethylphenyl)-1-(5-oxo-4,5-dihydro-1,3,4-oxadiazol-2-yl)propyl)-4-oxochroman-8-sulfonamide (44 mg) was obtained in accordance with the method of Example 129.

(Step 2)

Sodium borohydride (13.5 mg) was added to an ethanol (2.0 mL) solution of 5-fluoro-N-((1S,2R)-2-(6-fluoro-2,3-dimethylphenyl)-1-(5-oxo-4,5-dihydro-1,3,4-oxadiazol-2-yl)propyl)-4-oxochroman-8-sulfonamide (44 mg) obtained from the above Step 1 and the reaction solution was stirred at room temperature for 30 minutes. After concentrating the reaction solution under reduced pressure, water (10 mL) and ethyl acetate (10 mL) were added to the residue, separated, and the aqueous layer was extracted with ethyl acetate (10 mL). The combined organic layer was washed with saturated saline (10 mL), dried over anhydrous sodium sulfate, and concentrated under reduced pressure. The residue was purified by reverse phase HPLC (water / acetonitrile), and the fractions were concentrated to obtain each of two diastereomeric products. The substance eluted first was designated as Compound A, and the substance eluted later was designated as Compound B.

[Examples 231 to 244]

According to the method of Example 129 and Example 230 Step 2, the compounds of Examples 231 to 244 shown below were synthesized. In the case of separating the diastereomers, the first eluted compound was designated as A and the later eluted compound as B. The ratio of diastereomers is 1: 1 mixture unless otherwise specified. The necessary raw materials are listed in the following table.

[Table 15]

| Example | Starting Material | ArSO2Cl | Name of the Synthesized Compound |
|---|---|---|---|
| 231 | Reference Example F5 | 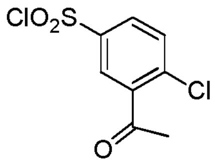 | 4-chloro-N-((1S,2R)-2-(2,3-dihydro-1H-inden-4-yl)-1-(5-oxo-4,5-dihydro-1,3,4-oxadiazol-2-yl)propyl)-3-(1-hydroxyethyl) benzenesulfonamide (diastereomer mixture) |
| 232 | Reference Example F4 | Reference Example E7 | 5-chloro-4-hydroxy-N-((1S,2R)-2-(8-methylnaphthalene-1-yl)-1-(5-oxo-4,5-dihydro-1,3,4-oxadiazol-2-yl)propyl) chroman-8-sulfonamide (diastereomer mixture) |
| 233 | Reference Example F3 | Reference Example E7 | 5-chloro-N-((1S,2R)-2-(2-fluoronaphthalene-1-yl)-1-(5-oxo-4,5-dihydro-1,3,4-oxadiazol-2-yl)propyl)-4-hydroxychroman-8-sulfonamide (diastereomer mixture) |
| 234A | Reference Example F3 | Reference Example E7 | 5-chloro-N-((1S,2R)-2-(2-fluoronaphthalene-1-yl)-1-(5-oxo-4,5-dihydro-1,3,4-oxadiazol-2-yl)propyl)-4-hydroxychroman-8-sulfonamide |
| 235A 23 5B | Reference Example F1 | Reference Example E7 | 5-chloro-N-((1S,2R)-2-(6-fluoro-2,3-dimethylphenyl)-1-(5-oxo-4,5-dihydro-1,3,4-oxadiazol-2-yl)propyl)-4-hydroxychroman-8-sulfonamide |
| 236 | Reference Example F1 | Reference Example E19 | 4-chloro-N-((1S,2R)-2-(6-fluoro-2,3-dimethylphenyl)-1-(5-oxo-4,5-dihydro-1,3,4-oxadiazol-2-yl)propyl)-2-(1-hydroxyethyl)benzenesulfonamide (diastereomer mixture) |
| 237A | Reference Example F1 | Reference Example E23 | N-((1S,2R)-2-(6-fluoro-2,3-dimethylphenyl)-1-(5-oxo-4,5-dihydro-1,3,4-oxadiazol-2-yl)propyl)-4-hydroxy-2,2-dimethylchroman-8-sulfonamide |
| 238A | Reference Example F7 | Reference Example E7 | 5-chloro-N-((1S,2R)-2-(3-chloro-6-fluoro-2-methylphenyl)-1-(5-oxo-4,5-dihydro-1,3,4-oxadiazol-2-yl)propyl)-4-hydroxychroman-8-sulfonamide |
| 239A | Reference Example F9 | Reference Example E7 | 5-chloro-N-((1S,2R)-2-(3-ethyl-6-fluoro-2-methylphenyl)-1-(5-oxo-4,5-dihydro-1,3,4-oxadiazol-2-yl)propyl)-4-hydroxychroman-8-sulfonamide |

(continued)

| Example | Starting Material | ArSO2Cl | Name of the Synthesized Compound |
|---|---|---|---|
| 240 | Reference Example F1 | Reference Example E25 | 4-chloro-N-((1S,2R)-2-(6-fluoro-2,3-dimethylphenyl)-1-(5-oxo-4,5-dihydro-1,34-oxadiazol-2-yl)propyl)-8-hydroxy-5,6,7,8-tetrahydronaphthalene-1-sulfonamide (diastereomer mixture) |
| 241 | Reference Example F1 | Reference Example E8 | 5-fluoro-N-((1S,2R)-2-(6-fluoro-2,3-dimethylphenyl)-1-(5-oxo-4,5-dihydro-1,3,4-oxadiazol-2-yl)propyl)-4-hydroxychroman-8-sulfonamide (diastereomer mixture) |
| 242 | Reference Example F1 | Reference Example E30 | 5,7-difluoro-N-((1S,2R)-2-(6-fluoro-2,3-dimethylphenyl)-1-(5-oxo-4,5-dihydro-1,3,4-oxadiazol-2-yl)propyl)-4-hydroxychroman-8-sulfonamide (diastereomer mixture) |
| 243A 243B | Reference Example F8 | Reference Example E7 | N-((1S,2R)-2-(3-bromo-6-fluoro-2-methylphenyl)-1-(5-oxo-4,5-dihydro-1,3,4-oxadiazol-2-yl)propyl)-5-chloro-4-hydroxychroman-8-sulfonamide |
| 244A 244B | Reference Example F1 | Reference Example E9 | N-((1S,2R)-2-(6-fluoro-2,3-dimethylphenyl)-1-(5-oxo-4,5-dihydro-1,3,4-oxadiazol-2-yl)propyl)-4-hydroxy-5-(tifluoromethyl)chroman-8-sulfonamide |

[Example 245]

Synthesis of 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)nicotinamide

(Step 1)

Using (2S,3R)-2-amino-3-(6-fluoro-2,3-dimethylphenyl)butanoic acid (100 mg) obtained from Reference Example D1 and methyl 5-chloro-2-(chlorosulfonyl)nicotinate (140 mg) obtained from Reference Example E11, according to the method of Example 1 Steps 1, 2, methyl 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)nicotinate (174 mg) was obtained.

(Step 2)

Methyl 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)nicotinate (174 mg) obtained from the above Step 1 was dissolved in THF (2.5 mL) and water (2.5 mL), lithium hydroxide (30 mg) was added, and the reaction solution was stirred at 50°C for 16 hours. The reaction solution was added to hydrochloric acid (1 M, 15 mL) and extracted with ethyl acetate (15 mL). The organic layer was washed with saturated saline (20 mL), dried over anhydrous magnesium sulfate, and concentrated under reduced pressure. The residue was purified by silica gel column chromatography (eluent: hexane / ethyl acetate / 2% acetic acid) to obtain 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)nicotinic acid (145 mg).

(Step 3)

To a toluene (1.2 mL) solution of 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)nicotinic acid (10 mg) obtained from the above Step 2, DMF (30 μL) and thionyl chloride (60 μL) were sequentially added, and the reaction solution was stirred at 95°C for 40 minutes. The reaction solution was allowed to cool to room temperature and then concentrated under reduced pressure. The THF (2.0 mL) solution of the residue was slowly added dropwise to 28% aqueous ammonia solution (1.0 mL) at -10°C, and the reaction solution was stirred at room temperature for 30 min. The reaction solution was added to hydrochloric acid (1 M, 10 mL) and extracted with ethyl acetate (10 mL). The organic layer was washed with saturated saline (10 mL), dried over anhydrous sodium sulfate, and concentrated under reduced pressure. The residue was purified by silica gel column chromatography (eluent: hexane / ethyl acetate) to give the title compound.

[Examples 246 to 264]

Compounds of Examples 246 to 264 shown below were synthesized according to the procedures of Example 1 Steps 1 and 2, and Example 245 Step 2 and 3. The necessary raw materials are listed in the following table.

[Table 16]

| Example | Starting Material | ArSO2Cl | Amine | Name of the Synthesized Compound |
|---|---|---|---|---|
| 246 | Reference Example D1 | 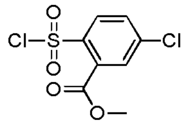 | 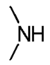 | 5-chloro-2-(N-((1S,2R)-2-(6-fluoro-2,3-dimethylphenyl)-1-(5-oxo-4,5-dihydro-1,3,4-oxadiazol-2-yl)propyl) sulfamoyl)-N,N-dimethylbenzamide |
| 247 | Reference Example D1 | 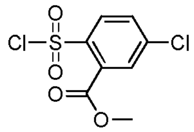 | 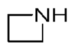 | 2-(azetidine-1-carbonyl)-4-chloro-N-((1S,2R)-2-(6-fluoro-2,3-dimethylphenyl)-1-(5-oxo-4,5-dihydro-1,3,4-oxadiazol-2 -yl)propyl) benzenesulfonamide |
| 248 | Reference Example D1 | 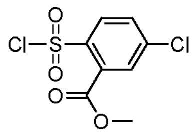 | 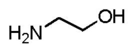 | 5-chloro-2-(N-((1S,2R) -2-(6 -fluoro-2,3-dimethylphenyl)-1-(5-oxo-4,5-dihydro-1,3,4-oxadiazol-2-yl)propyl)sulfamoyl)-N-(2-hydroxyethyl)benzamide |
| 249 | Reference Example D4 | Reference Example E11 | $NH_3$ | 5-chloro-2-(N-((1S,2R)-2-(3-chloro-6-fluoro-2-methylphenyl)-1-(5-oxo-4,5-dihydro-1,3,4-oxadiazol-2-yl)propyl)sulfamoyl)nicotinamide |
| 250 | Reference Example D1 | Reference Example E73 | $NH_3$ | 5-bromo-2-(N-((1S,2R)-2-(6-fluoro-2,3-dimethylphenyl)-1-(5-oxo-4,5-dihydro-1,3,4-oxadiazol-2-yl)propyl)sulfamoyl)nicotinamide |
| 251 | Reference Example D1 | Reference Example E73 | MeNH2 | 5-bromo-2-(N-((1S,2R)-2-(6-fluoro-2,3-dimethylphenyl)-1-(5-oxo-4,5-dihydro-1,3,4-oxadiazol-2-yl)propyl)sulfamoyl)-N-methylnicotinamide |
| 252 | Reference Example D1 | Reference Example E10 | MeNH2 | 3-chloro-6-(N-((1S,2R)-2-(6-fluoro-2,3-dimethylphenyl)-1-(5-oxo-4,5-dihydro-1,3,4-oxadiazol-2-yl)propyl)sulfamoyl)-N-methylpicolinamide |
| 253 | Reference Example D1 | Reference Example E10 | 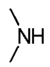 | 3-chloro-6-(N-((1S,2R)-2-(6-fluoro-2,3-dimethylphenyl)-1-(5-oxo-4,5-dihydro-1,3,4-oxadiazol-2-yl)propyl)sulfamoyl)-N,N-dimethylpicolinamide |
| 254 | Reference Example D4 | Reference Example E73 | $NH_3$ | 5-bromo-2-(N-((1S,2R)-2-(3-chloro-6-fluoro-2-methylphenyl)-1-(5-oxo-4,5-dihydro-1,3,4-oxadiazol-2-yl)propyl)sulfamoyl)nicotinamide |
| 255 | Reference Example D1 | Reference Example E28 | $NH_3$ | 5-chloro-4-fluoro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide |
| 256 | Reference Example D1 | 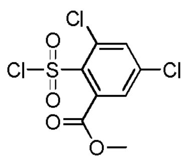 | $NH_3$ | 3,5-dichloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide |

(continued)

| Example | Starting Material | ArSO2Cl | Amine | Name of the Synthesized Compound |
|---|---|---|---|---|
| 257 | Reference Example D1 | Reference Example E12 | $NH_3$ | 5-chloro-2-(N-((1S,2R)-2-(6 -fluoro-2,3-dimethylphenyl)-1-(5-oxo-4,5-dihydro-1,3,4-oxadiazol-2-yl)propyl)sulfamoyl)-4-methylbenzamide |
| 258 | Reference Example D1 | Reference Example E29 | $NH_3$ | 5-chloro-3-fluoro-2-(N-((1S,2R)-2-(6-fluoro-2,3-dimethylphenyl)-1-(5-oxo-4,5-dihydro-1,3,4-oxadiazol-2-yl)propyl) sulfamoyl)benzamide |
| 259 | Reference Example D1 | Reference Example E14 | $NH_3$ | 5-chloro-2-(N-((1S,2R)-2-(6-fluoro-2,3-dimethylphenyl)-1-(5-oxo-4,5-dihydro-1,3,4-oxadiazol-2-yl)propyl) sulfamoyl)-4-methoxybenzamide |
| 260 | Reference Example D1 | Reference Example E31 | $NH_3$ | 2-(N-((1S,2R)-2-(6-fluoro-2,3-dimethylphenyl)-1-(5-oxo-4,5-dihydro-1,3,4-oxadiazol-2-yl) propyl)sulfamoyl)-4,5-dimethoxybenzamide |
| 261 | Reference Example D1 | Reference Example E17 | $NH_3$ | 7-(N-((1S,2R)-2-(6-fluoro-2,3-dimethylphenyl)-1-(5-oxo-4,5-dihydro-1,3,4-oxadiazol-2-yl) propyl)sulfamoyl)-2,3-dihydrobenzo[b][1,4] dioxin-6-carboxamide |
| 262 | Reference Example D1 | Reference Example E53 | $NH_3$ | 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)-4-nitrobenzamide |
| 263 | Reference Example D1 | Reference Example E47 | $NH_3$ | 4-(2,2-difluoroethoxy)-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide |
| 264 | Reference Example D5 | Reference Example E11 | $NH_3$ | 2-(N-((1S,2R)-2-(3-bromo-6-fluoro-2-methylphenyl)-1-(5-oxo-4,5-dihydro-1,3,4-oxadiazol-2-yl)propyl)sulfamoyl)-5-chloronicotinamide |

[Example 265]

Synthesis of 4-amino-N-((1S,2R)-2-(2-fluoronaphthalen-1-yl)-1-(5-oxo-4,5-dihydro-1,3,4-oxadiazol-2-yl)propyl)-2-methoxybenzenesulfonamide

(Step 1)

Using (2S,3R)-2-amino-3-(2-fluoronaphthalen-1-yl)butanoic acid (45 mg) obtained in Reference Example D3 and 2-methoxy-4-nitrobenzene-1-sulfonyl chloride (60 mg), according to the method of Example 1, Steps 1 and 2, N-((1S,2R)-2-(2-fluoronaphthalen-1-yl)-1-(5-oxo-4,5-dihydro-1,3,4-oxadiazol-2-yl)propyl)-2-methoxy-4-nitrobenzenesulfonamide (32 mg) was obtained.

(Step 2)

N-((1S,2R)-2-(2-fluoronaphthalen-1-yl)-1-(5-oxo-4,5-dihydro-1,3,4-oxadiazol-2-yl)propyl)-2-methoxy-4-nitrobenzenesulfonamide (32 mg) obtained from the above Step 1 was dissolved in ethanol (2.0 mL) and water (1.0 mL), iron (30 mg) and ammonium chloride (20mg) were sequentially added, and the reaction solution was stirred at 80°C for 1 hour. The reaction solution was filtered through CELITE, and the residue was washed with ethyl acetate (10 mL). The combined filtrates were concentrated and the residue was purified by silica gel column chromatography (eluent: hexane / ethyl acetate) to give the title compound.

[Examples 266 to 272]

Compounds of Examples 266 to 272 shown below were synthesized according to the method of Example 1 Steps 1 and 2 and Example 265 Step 2. The necessary raw materials are listed in the following table.

[Table 17]

| Example | Starting Material | ArSO2Cl | Name of the Synthesized Compound |
|---|---|---|---|
| 266 | Reference Example D21 | 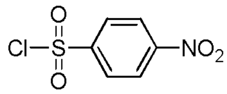 | 4-amino-N-((1S,2R)-1-(5-oxo-4,5-dihydro-1,3,4-oxadiazol-2-yl)-2-(5,6,7,8-tetrahydronaphthalen-1-yl) propyl)benzenesulfonamide |
| 267 | Reference Example D10 | 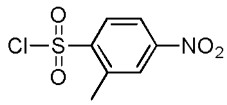 | 4-amino-N-((1S,2R)-2-(2,3-dihydro-1H-inden-4-yl)-1-(5-oxo-4,5-dihydro-1,3,4-oxadiazol-2-yl)propyl)-2-methylbenzenesulfonamide |
| 268 | Reference Example D10 | 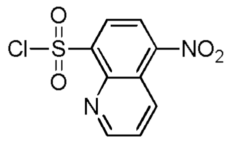 | 5-amino-N-((1S,2R)-2-(2,3-dihydro-1H-inden-4-yl)-1-(5-oxo-4,5-dihydro-1,3,4-oxadiazol-2-yl)propyl)quinoline-8-sulfonamide |
| 269 | Reference Example D13 | 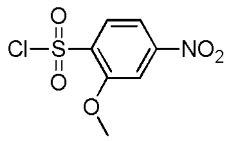 | 4-amino-2-methoxy-N-((1S,2R)-2-(8-methylnaphthalen-1-yl)-1-(5-oxo-4,5-dihydro-1,3,4-oxadiazol-2-yl)propyl) benzenesulfonamide |
| 270 | Reference Example D10 | 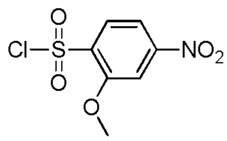 | 4-amino-N-((1S,2R)-2-(2,3-dihydro-1H-inden-4-yl)-1-(5-oxo-4,5-dihydro-1,3,4-oxadiazol-2-yl)propyl)-2-methoxybenzenesulfonamide |
| 271 | Reference Example D1 | 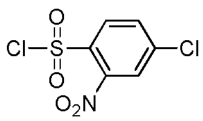 | 2-amino-4-chloro-N-((1S,2R)-2-(6-fluoro-2,3-dimethylphenyl)-1-(5-oxo-4,5-dihydro-1,3,4-oxadiazol-2-yl)propyl)benzenesulfonamide |
| 272 | Reference Example D1 | Reference Example E53 | methyl 4-amino-5-chloro-2-(N-((1S,2R)-2-(6-fluoro-2,3-dimethylphenyl)-1-(5-oxo-4,5-dihydro-1,3,4-oxadiazol-2-yl)propyl) sulfamoyl)benzoate |

[Example 273]

Synthesis of 5-chloro-N-((1S,2R)-2-(6-fluoro-2,3-dimethylphenyl)-1-(5-oxo-4,5-dihydro-1,3,4-oxadiazol-2-yl)propyl)-4-(2-hydroxyethyl)-3-oxo-3,4-dihydro-2H-benzo[b][1,4]oxazine-8-sulfonamide

(Step 1)

Using (2S,3R)-2-amino-3-(6-fluoro-2,3-dimethylphenyl)butanoic acid (50 mg) obtained in Reference Example D1 and 4-(2-(benzyloxy)ethyl)-5-chloro-3,4-dihydro-2H-benzo[b][1,4]oxazine-8-sulfonyl chloride (142 mg) obtained in Reference Example E65, according to the method of Example 1 Steps 1 and 2, 4-(2-(benzyloxy)ethyl)-5-chloro-N-((1S,2R)-2-(6-fluoro-2,3-dimethylphenyl)-1-(5-oxo-4,5-dihydro-1,3,4-oxadiazol-2-yl)propyl)-3-oxo-3,4-dihydro-2H-benzo[b][1,4]oxazine-8-sulfonamide (22 mg) was obtained.

(Step 2)

To a THF (1.5 mL) solution of 4-(2-(benzyloxy)ethyl)-5-chloro-N-((1S,2R)-2-(6-fluoro-2,3-dimethylphenyl)-1-(5-

oxo-4,5-dihydro-1,3,4-oxadiazol-2-yl)propyl)-3-oxo-3,4-dihydro-2H-benzo[b][1,4]oxazine-8-sulfonamide (20 mg) obtained from the above Step 1, 20 wt% palladium hydroxide (30 mg) was added, and the reaction mixture was stirred at room temperature for 30 minutes under hydrogen atmosphere. The reaction solution was filtered through CELITE, and the residue was washed with hexane / ethyl acetate = 1/1 (10 mL), and the combined filtrate was concentrated under reduced pressure. The residue was purified by silica gel column chromatography (eluent: hexane / ethyl acetate) to obtain the title compound.

[Example 274]

Synthesis of N-(5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)phenyl)acetamide

To a dichloromethane (1.0 mL) solution of 2-Amino-4-chloro-N-((1S,2R)-2-(6-fluoro-2,3-dimethylphenyl)-1-(5-oxo-4,5-dihydro-1,3,4-oxadiazol-2-yl)propyl)benzenesulfonamide (5.0 mg) obtained from Example 271, Pyridine (5.0 μL) and acetic anhydride (4.0 μL) were sequentially added, and the reaction solution was stirred at room temperature for 3 hours. The reaction solution was added to hydrochloric acid (1 M, 5.0 mL) and extracted with ethyl acetate (10 mL). The organic layer was washed with saturated saline (10 mL), dried over anhydrous sodium sulfate, and concentrated under reduced pressure. The residue was purified by silica gel column chromatography (eluent: hexane / ethyl acetate) to give the title compound.

[Example 275]

Synthesis of 5-chloro-N-((1S,2R)-2-(6-fluoro-2,3-dimethylphenyl)-1-(5-oxo-4,5-dihydro-1,3,4-oxadiazol-2-yl)propyl)-3,4-dihydro-2H-benzo[b][1,4]oxazine-8-sulfonamide

(Step 1)

From 5-((1S,2R)-1-amino-2-(6-fluoro-2,3 -dimethylphenyl)propyl)-1,3,4-oxadiazol-2(3H)-one monohydrochloride (14.3 mg) obtained in Reference Example F1 and tert-butyl 5-chloro-8-(chlorosulfonyl)-2H-benzo[b][1,4]oxazin-4(3H)-carboxylate (25.3 mg) obtained in Reference Example E58, according to the method of Example 129, tert-butyl 5-chloro-8-(N-((1S,2R)-2-(6-fluoro-2,3-dimethylphenyl)-1-(5-oxo-4,5-dihydro-1,3,4-oxadiazol-2-yl)propyl)sulfamoyl)-2H-benzo[b][1,4]oxazine-4(3H)-carboxylate (30. 4 mg) was obtained.

(Step 2)

To tert-butyl 5-chloro-8-(N-((1S,2R)-2-(6-fluoro-2,3-dimethylphenyl)-1-(5-oxo-4,5-dihydro-1,3,4-oxadiazol-2-yl)propyl)sulfamoyl)-2H-benzo[b][1,4]oxazin- 4(3H)-carboxylate (30.4 mg) obtained from the above Step 1, hydrochloric acid -1, 4-dioxane (4 M, 5.0 mL) was added, and the reaction solution was stirred at room temperature for 1 hour. The reaction solution was concentrated under reduced pressure, and the obtained residue was purified by reversed phase HPLC (water / acetonitrile) to obtain the title compound.

[Examples 276 to 283]

Compounds of Examples 276 to 283 shown below were synthesized according to the method of Example 129 and Example 275 Step 2. The necessary raw materials are listed in the following table.

[Table 18]

| Example | Starting Material | ArSO2Cl | Name of the Synthesized Compound |
|---|---|---|---|
| 276 | Reference Example D10 | Reference Example E56 | methyl 6-amino-3-(N-((1S,2R)-2-(2,3-dihydro-1H-inden-4-yl)-1-(5-oxo-4,5-dihydro-1,3,4-oxadiazol-2-yl)propyl)sulfamoyl)-2-methoxybenzoate |
| 277 | Reference Example D1 | Reference Example E52 | 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)-N-methylbenzamide |

(continued)

| Example | Starting Material | ArSO2Cl | Name of the Synthesized Compound |
|---|---|---|---|
| 278 | Reference Example D1 | Reference Example E70 | 4-amino-5-chloro-N-((1S,2R)-2-(6-fluoro-2,3-dimethylphenyl)-1-(5-oxo-4,5-dihydro-1,3,4-oxadiazol-2-yl)propyl)chroman-8-sulfonamide |
| 279 | Reference Example D1 | Reference Example E55 | 2-(1-aminocyclopropyl)-4-chloro-N-((1S,2R)-2-(6-fluoro-2,3-dimethylphenyl)-1-(5-oxo-4,5-dihydro-1,3,4-oxadiazol-2-yl)propyl) benzenesulfonamide |
| 280 | Reference Example D1 | Reference Example E59 | N-((1S,2R)-2-(6-fluoro-2,3-dimethylphenyl)-1-(5-oxo-4,5-dihydro-1,3,4-oxadiazol-2-yl)propyl)-3,4-dihydro-2H-benzo[b][1,4]oxazine-8-sulfonamide |
| 281 | Reference Example D1 | Reference Example E24 | N-((1S,2R)-2-(6-fluoro-2,3-dimethylphenyl)-1-(5-oxo-4,5-dihydro-1,3,4-oxadiazol-2-yl)propyl)-1,2,3,4-tetrahydroisoquinoline-5-sulfonamide |
| 282 | Reference Example D1 | Reference Example E51 | N-(benzyloxy)-5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide |
| 283 | Reference Example D1 | Reference Example E41 | 6-(3-aminopyrrolidine-1-carbonyl)-5-chloro-N-((1S,2R)-2-(6-fluoro-2,3-dimethylphenyl)-1-(5-oxo-4,5-dihydro-1,3,4-oxadiazol-2-yl)propyl) pyridine-2-sulfonamide |

[Example 284]

Synthesis of 4-acetyl-5-chloro-N-((1S,2R)-2-(2,3-dimethylphenyl)-1-(5-oxo-4,5-dihydro-1,3,4-oxadiazol-2-yl)propyl)-3,4-dihydro-2H-benzo[b][1,4]oxazine-8-sulfonamide

To a dichloromethane (1.0 mL) solution of 5-chloro-N-((1S,2R)-2-(6-fluoro-2,3-dimethylphenyl)-1-(5-oxo-4,5-dihydro-1,3,4-oxadiazol-2-yl)propyl)-3,4-dihydro-2H-benzo[b][1,4]oxazine-8-sulfonamide (31.5 mg) obtained in Example 275, triethylamine (40 μL) and acetic anhydride (20 μL) were sequentially added, and the reaction solution was stirred at room temperature for 2 hours. The reaction solution was concentrated under reduced pressure, and the obtained residue was purified by reversed phase HPLC (water / acetonitrile) to obtain the title compound.

[Example 285]

Synthesis of 2-(N-((1S,2R)-2-(6-fluoro-2,3-dimethylphenyl)-1-(5-oxo-4,5-dihydro-1,3,4-oxadiazol-2-yl)propyl)sulfamoyl)-N-hydroxybenzamide

(Step 1)

Using (2S,3R)-2-amino-3-(6-fluoro-2,3-dimethylphenyl)butanoic acid (125 mg) obtained in Reference Example D1 and tert-butylbenzyloxy (5-chloro-2-(chlorosulfonyl)benzoyl)carbamate (280 mg) obtained in Reference Example E51 as a starting material, according to the method of Example 1 Step 1, (2S,3R)-2-(2-((benzyloxy)(tert-butoxycarbonyl)carbamoyl)-4-chlorophenylsulfonamido)-3-(6-fluoro-2,3-dimethylphenyl)butanoic acid (250 mg) was synthesized.

(Step 2)

(2S,3R)-2-(2-((benzyloxy)(tert-butoxycarbonyl)carbamoyl)-4-chlorophenylsulfonamido)-3-(6-fluoro-2,3-dimethylphenyl)butanoic acid (250 mg) obtained in the above Step 1 was dissolved in hydrochloric acid-1,4-dioxane (4 M, 4 mL), and the reaction solution was stirred at 45°C for 2.5 hours. The reaction solution was concentrated under reduced pressure and the residue was purified by silica gel column chromatography (eluent: hexane / ethyl acetate / 2% acetic acid) to give (2S,3R)-2-(2-((benzyloxy)carbamoyl)-4-chlorophenylsulfonamido)-3-(6-fluoro-2,3-dimethylphenyl)butanoic acid (215 mg).

(Step 3)

From (2S,3R)-2-(2-((benzyloxy)carbamoyl)-4-chlorophenylsulfonamido)-3-(6-fluoro-2,3-dimethylphenyl)butanoic acid (215 mg) obtained from the above Step 2, according to the method of Example 1 Step 2, N-(benzyloxy)-5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide (75 mg) was given.

(Step 4)

To a methanol (4.0 mL) solution of N-(benzyloxy)-5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide (75 mg) obtained from the above Step 3, 10% palladium-carbon (55 mg) was added, and the reaction solution was stirred under a hydrogen atmosphere for 1.5 hours. Insoluble matter was removed by CELITE filtration, and the residue was washed with methanol (10 mL). The combined filtrate was concentrated under reduced pressure, and the residue was purified by silica gel column chromatography (eluent: hexane / ethyl acetate) to give the title compound.

[Example 286]

Synthesis of 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)-N-hydroxybenzamide

To dichloromethane (3.0 mL) solution of N-(benzyloxy)-5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide (66 mg) obtained from Example 285 Step 3, boron tribromide (1.0 M, 170 μL) was added at -60°C, and the reaction solution was stirred at 0°C for 1 hour. Methanol (1.0 mL) was added to the reaction solution, and the mixture was concentrated under reduced pressure. The residue was purified by silica gel column chromatography (eluent: hexane / ethyl acetate / 2% acetic acid) to obtain the title compound.

[Example 287]

Synthesis of 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzothioamide

To a toluene (500 μL) solution of 5-chloro-2-((1S,2R)-2-(6-fluoro-2,3-dimethylphenyl)-1-(2-oxo-3H-1,3,4-oxadiazol-5-yl)propyl)sulfamoyl)benzamide (15 mg) obtained in Example 5, Lawesson's reagent (20 mg) was added at room temperature, and the reaction solution was stirred at 100°C for 12 hours. After allowing to cool to room temperature and concentrating under reduced pressure, the obtained residue was purified by silica gel column chromatography (eluent: hexane / ethyl acetate) to obtain the title compound.

[Example 288]

Synthesis of 5-chloro-N-((1S,2R)-2-(6-fluoro-2,3-dimethylphenyl)-1-(5-oxo-4,5-dihydro-1,3,4-oxadiazol-2-yl)propyl)-4-methyl-3,4-dihydro-2H-benzo[b][1,4]oxazine-8-sulfonamide

To a methanol (1.0 mL) solution of 5-chloro-N-((1S,2R)-2-(6-fluoro-2,3-dimethylphenyl)-1-(5-oxo-4,5-dihydro-1,3,4-oxadiazol-2-yl)propyl)-3,4-dihydro-2H-benzo[b][1,4]oxazine-8-sulfonamide (48 mg) obtained in Example 275, acetic acid (20 μL) and aqueous 37% formaldehyde solution (30 μL) were added successively, and the reaction solution was stirred at room temperature for 30 minutes. Sodium borohydride (12 mg) was added to the reaction solution, and the mixture was further stirred for 20 minutes. Water (15 mL) was added to the reaction solution, and the mixture was extracted with ethyl acetate / hexane = 1/1 (15 mL). The organic layer was washed with saturated saline (10 mL), dried over anhydrous sodium sulfate, and concentrated under reduced pressure. The residue was purified by reverse phase HPLC (water / acetonitrile) to give the title compound.

[Example 289]

Synthesis of 5-chloro-N-((1S,2R)-2-(3-chloro-6-fluoro-2-methylphenyl)-1-(5-oxo-4,5-dihydro-1,3,4-oxadiazol-2-yl)propyl)-4-methyl-3,4-dihydro-2H-benzo[b][1,4]oxazine-8-sulfonamide

(Step 1)

From 5-((1S,2R)-1-amino-2-(3 -chloro-6-fluoro-2-methylphenyl)propyl)-1,3,4-oxadiazol-2 (3H)-one monohydrochloride (10.3 mg) obtained in Reference Example F7 and tert-butyl 5-chloro-8-(chlorosulfonyl)-2H-benzo[b][1,4]oxazin-4 (3H)-carboxylate (25.3 mg) obtained from Reference Example E58, according to the method of Example 129, tert-butyl 5-chloro-8-(N-((1S,2R)-2-(3-chloro-6-fluoro-2-methylphenyl)-1-(5-oxo-4,5-dihydro-1,3,4-oxadiazol-2-yl)propyl)sulfamoyl)-2H-benzo[b][1,4]oxazin-4(3H)-carboxylate (25.4 mg) was obtained.

(Step 2)

To tert-butyl 5-chloro-8-(N-((1S,2R)-2-(3-chloro-6-fluoro-2-methylphenyl)-1-(5-oxo-4,5-dihydro-1,3,4-oxadiazol-2-yl)propyl)sulfamoyl)-2H-benzo[b][1,4]oxazin-4 (3H)-carboxylate (25.4 mg) obtained from the above Step 1, hydrochloric acid-1,4-dioxane (4 M, 5.0 mL) was added, and the reaction solution was stirred at room temperature for 1 hour. The reaction solution was concentrated under reduced pressure, and the obtained residue was purified by reversed phase HPLC (water / acetonitrile) to obtain 5-chloro-N-((1S,2R)-2-(3-chloro-6-fluoro-2-methylphenyl)-1 -(5 -oxo-4,5-dihydro-1,3,4-oxadiazol-2-yl)propyl)-3,4-dihydro-2H-benzo[b][1,4]oxazine-8-sulfonamide (15.2 mg).

(Step 3)

From 5-chloro-N-((1S,2R)-2-(3-chloro-6-fluoro-2-methylphenyl)-1-(5-oxo-4,5-dihydro-1,3,4-oxadiazol-2-yl)propyl)-3,4-dihydro-2H-benzo[b][1,4]oxazine-8-sulfonamide (15.2 mg) obtained from the above Step 2, according to the method of Example 288, the title compound was synthesized.

[Example 290]

Synthesis of 4-chloro-N-((1S)-2-(6-fluoro-2,3-dimethylphenyl)-2-hydroxy-1-(5-oxo-4,5-dihydro-1,3,4-oxadiazol-2-yl)ethyl)-2-methoxybenzenesulfonamide

(Step 1)

Using (2S)-2-amino-3-(6-fluoro-2,3-dimethylphenyl)-3-hydroxypropionic acid (139 mg) obtained from Reference Example D56 and 4-chloro-2-methoxybenzenesulfonyl chloride (175 mg), according to the method of Example 1 Step 1, (2S)-2-(4-chloro-2-methoxyphenylsulfonamido)-3-(6-fluoro-2,3-dimethylphenyl)-3 -hydroxypropionic acid (163 mg) was synthesized.

(Step 2)

To a DMF (10 mL) solution of (2S)-2-(4-chloro-2-methoxyphenylsulfonamido)-3-(6-fluoro-2,3-dimethylphenyl)-3-hydroxypropionic acid (163 mg) obtained from the above Step 1, Imidazole (753 mg) and tert-butyldimethylchlorosilane (563 mg) were sequentially added, and the reaction solution was stirred at 60°C for 12 hours. The reaction solution was added to water (20 mL) and extracted with ethyl acetate / hexane = 1/1 (30 mL). The organic layer was washed with saturated saline (20 mL), dried over anhydrous sodium sulfate, and concentrated under reduced pressure. The residue was dissolved in methanol (10 mL) and THF (2.0 mL), potassium carbonate (1.0 g) and water (2.0 mL) were added, and the reaction solution was stirred at room temperature for 2 hours. The reaction solution was added to hydrochloric acid (1 M, 20 mL) and extracted with ethyl acetate / hexane = 1/1 (30 mL). The organic layer was washed with saturated saline (20 mL), dried over anhydrous sodium sulfate, and concentrated under reduced pressure. The residue was purified by silica gel column chromatography (eluent: hexane / ethyl acetate) to obtain (2S)-3-((tert-butyldimethylsilyl)oxy)-2-(4-chloro-2-methoxyphenylsulfonamido)-3-(6-fluoro-2,3-dimethylphenyl)propionic acid (175 mg).

(Step 3)

From (2S)-3-((tert-butyldimethylsilyl)oxy)-2-(4-chloro-2-methoxyphenylsulfonamido)-3-(6-fluoro-2,3-dimethylphenyl)propionic acid (175 mg) obtained from the above Step 2, according to the method of Example 1 Step 2, N-((1S)-2-((tert-butyldimethylsilyl)oxy)-2-(6-fluoro-2,3-dimethylphenyl)-1-(5-oxo-4,5-dihydro-1,3,4-oxadiazol-2-yl)ethyl)-4-chloro-2-methoxybenzenesulfonamide (126 mg) was obtained.

(Step 4)

To a THF (6.0 mL) solution of N-((1S)-2-((tert-butyldimethylsilyl)oxy)-2-(6-fluoro-2,3-dimethylphenyl)-1-(5-oxo-4,5-dihydro-1,3,4-oxadiazol-2-yl)ethyl)-4-chloro-2-methoxybenzenesulfonamide (126 mg) obtained in the above Step 3, acetic acid (600 μL) and tetra-n-butylammonium fluoride (6.0 mL) were sequentially added, and the mixture was stirred at room temperature for 2 hours. The reaction solution was added to water (20 mL) and extracted with ethyl acetate / hexane = 1/1 (30 mL). The organic layer was washed with saturated saline (20 mL), dried over anhydrous sodium sulfate, and concentrated under reduced pressure. The residue was purified by silica gel column chromatography (eluent: hexane / ethyl acetate) to give the title compound as a diastereomeric mixture.

[Example 291]

Synthesis of 4-chloro-N-((1R)-2-fluoro-2-(6-fluoro-2,3 -dimethylphenyl)-1-(5-oxo-4,5-dihydro-1,3,4-oxadiazol-2-yl)ethyl)-2-methoxybenzenesulfonamide

To a dichloromethane (200 μL) solution of 4-chloro-N-((1S)-2-(6-fluoro-2,3-dimethylphenyl)-2-hydroxy-1-(5-oxo-4,5-dihydro-1,3,4-oxadiazol-2-yl)ethyl)-2-methoxybenzenesulfonamide (5.6 mg) obtained from Example 290, DAST (10 μL) was added, and the mixture was stirred at room temperature for 2 hours. Saturated aqueous sodium hydrogen carbonate solution (5.0 mL) was added to the reaction solution, and the mixture was extracted with ethyl acetate / hexane = 1/1 (10 mL). The organic layer was washed with saturated saline (5.0 mL), dried over anhydrous sodium sulfate, and concentrated under reduced pressure. The residue was purified by reverse phase HPLC (water / acetonitrile) to obtain the title compound as a diastereomeric mixture.

[Example 292]

Synthesis of 5-chloro-4-fluoro-N-((1S,2R)-2-(2-fluoronaphthalen-1-yl)-1-(5-oxo-4,5-dihydro-1,3,4-oxadiazol-2-yl)propyl)chroman-8-sulfonamide

From 5-chloro-N-((1S,2R)-2-(2-fluoronaphthalen-1-yl)-1-(5-oxo-4,5-dihydro-1,3,4-oxadiazol-2-yl)propyl)-4-hydroxychroman-8-sulfonamide (13 mg) obtained in Example 233, according to the method of Example 291, the title compound was obtained as a 1: 1 diastereomeric mixture.

[Example 293]

Synthesis of 5-chloro-N-((1S,2R)-2-(6-fluoro-2,3-dimethylphenyl)-1-(5-oxo-4,5-dihydro-1,3,4-oxadiazol-2-yl)propyl)-6-(2,2,2-trifluoroacetyl)pyridine-2-sulfonamide

From 5-chloro-N-((1S,2R)-2-(6-fluoro-2,3-dimethylphenyl)-1-(5-oxo-4,5-dihydro-1,3,4-oxadiazol-2-yl)propyl)-6-(2,2,2-trifluoro-1-hydroxyethyl)pyridine-2-sulfonamide (15.6 mg) obtained in Example 221, according to the method of Reference Example E46 Step 1, the title compound was obtained.

[Example 294]

Synthesis of 3-acetyl-4-chloro-N-((1S,2R)-2-(6-fluoro-2,3-dimethylphenyl)-1-(5-oxo-4,5-dihydro-1,3,4-oxadiazol-2-yl)propyl)-2-methoxybenzenesulfonamide

From 4-chloro-N-((1S,2R)-2-(6-fluoro-2,3-dimethylphenyl)-1-(5-oxo-4,5-dihydro-1,3,4-oxadiazol-2-yl)propyl)-3-(1-hydroxyethyl)-2-methoxybenzenesulfonamide obtained in Example 204, the title compound was obtained according to the method of Reference Example E46 Step 1.

[Example 295]

Synthesis of 5-chloro-N-((1S,2R)-2-(2,3-dimethylphenyl)-1-(5-oxo-4,5-dihydro-1,3,4-oxadiazol-2-yl)propyl)-2H-chromene-8-sulfonamide

(Step 1)

From (2S,3R)-2-amino-3-(2,3-dimethylphenyl)butanoic acid (58 mg) obtained in Reference Example D6 and 5-chloro-4-oxochroman-8-sulfonyl chloride (88 mg) obtained in Reference Example E7, in accordance with the procedures of Example Steps 1 and 2, 5-chloro-N-((1S,2R)-2-(2,3-dimethylphenyl)-1-(5-oxo-4,5-dihydro-1,3,4-oxadiazol-2-yl)propyl)-4-oxochroman-8-sulfonamide (63.4 mg) was obtained.

(Step 2)

From 5-chloro-N-((1S,2R)-2-(2,3-dimethylphenyl)-1-(5-oxo-4,5-dihydro-1,3,4-oxadiazol-2-yl)propyl)-4-oxochroman-8-sulfonamide (63.4 mg) obtained from the Step 1 above, according to the method of Example 230 Step 2, 5-chloro-N-((1S,2R)-2-(2,3-dimethylphenyl)-1-(5-oxo-4,5-dihydro-1,3,4-oxadiazol-2-yl)propyl)-4-hydroxychroman-8-sulfonamide (48 mg) was obtained as a diastereomeric mixture.

(Step 3)

To a toluene (2.0 mL) solution of 5-chloro-N-((1S,2R)-2-(2,3-dimethylphenyl)-1-(5-oxo-4,5-dihydro-1,3,4-oxadiazol-2-yl)propyl)-4-hydroxychroman-8-sulfonamide (10 mg) obtained from the above Step 2, p-toluenesulfonic acid monohydrate (2.0 mg) was added, and the reaction solution was stirred at 110°C for 30 minutes. The reaction solution was added to water (5 mL) and extracted with ethyl acetate (10 mL). The organic layer was washed with saturated saline (10 mL), dried over anhydrous sodium sulfate, and concentrated under reduced pressure. The residue was purified by silica gel column chromatography (eluent: hexane / ethyl acetate) to give the title compound.

[Example 296]

Synthesis of 4-chloro-N-((1S,2R)-2-(2,3-dihydro-1H-inden-4-yl)-1-(5-oxo-4,5-dihydro-1,3,4 oxadiazol-2-yl)propyl)-2-(hydroxymethyl)benzenesulfonamide

(Step 1)

Using (2S,3R)-2-amino-3-(2,3-dihydro-1H-inden-4-yl)butanoic acid (50 mg) obtained from Reference Example D10 and methyl 5-chloro-2-(chlorosulfonyl)benzoate (71 mg), according to the method of steps 1 and 2 of Example 1, methyl 5-chloro-2-(N-((1S,2R)-2-(2,3-dihydro-1H-inden-4-yl)-1-(5-oxo-4,5-dihydro-1,3,4-oxadiazol-2-yl)propyl)sulfamoyl)benzoate (43 mg) was obtained.

(Step 2)

To a THF (2.0 mL) solution of methyl 5-chloro-2-(N-((1S,2R)-2-(2,3-dihydro-1H-inden-4-yl)-1-(5-oxo-4,5-dihydro-1,3,4-oxadiazol-2-yl)propyl)sulfamoyl)benzoate (15 mg) obtained in the above Step 1, a THF solution of lithium borohydride (2 M, 100 μL) was added, and the reaction solution was stirred at 60°C for 1 hour. The reaction solution was added to water (10 mL) and extracted with ethyl acetate (15 mL). The organic layer was washed with saturated saline (10 mL), dried over anhydrous sodium sulfate, and concentrated under reduced pressure. The residue was purified by silica gel column chromatography (eluent: hexane / ethyl acetate) to give the title compound.

[Example 297]

Synthesis of 4-chloro-N-((1S,2R)-2-(6-fluoro-2,3-dimethylphenyl)-1-(5-oxo-4,5-dihydro-1,3,4-oxadiazol-2-yl)propyl)-2-methoxy-3-(1-(methoxymethoxy)ethyl)benzenesulfonamide

To a toluene (1.5 mL) solution of 4-chloro-N-((1S,2R)-2-(6-fluoro-2,3-dimethylphenyl)-1-(5-oxo-4,5-dihydro-1,3,4-oxadiazol-2-yl)propyl)-3-(1-hydroxyethyl)-2-methoxybenzenesulfonamide (10 mg) obtained from Example 204, N, N-diisopropylethylamine (25 μL) and chloromethyl methyl ether (10 μL) were sequentially added, and the reaction solution was stirred at room temperature for 3 hours. The reaction solution was concentrated under reduced pressure,

and the residue was purified by silica gel column chromatography (eluent: hexane / ethyl acetate) to obtain the title compound.

[Example 298]

Synthesis of 4-chloro-N-((1S,2R)-2-(4-fluoro-4'-methoxy-2-methyl-[1,1'-biphenyl]-3-yl)-1-(5-oxo-4,5-dihydro-1,3,4-oxadiazol-2-yl)propyl)-2-methoxybenzenesulfonamide

(Step 1)

Using (2S,3R)-2-amino-3-(3-bromo-6-fluoro-2-methylphenyl)butanoic acid (200 mg) obtained in Reference Example D5 and 4-chloro-2-methoxybenzenesulfonyl chloride (280 mg), according to the method of steps 1 and 2 of Example 1,N-((1S,2R)-2-(3-bromo-6-fluoro-2-methylphenyl)-1-(5-oxo-4,5-dihydro-1,3,4-oxadiazol-2-yl)propyl)-4-chloro-2-methoxybenzenesulfonamide (262 mg) was synthesized.

(Step 2)

To a 1,4-dioxane (1.0 mL) solution of N-((1S,2R)-2-(3-bromo-6-fluoro-2-methylphenyl)-1-(5-oxo-4,5-dihydro-1,3,4-oxadiazol-2-yl)propyl)-4-chloro-2-methoxybenzenesulfonamide (11 mg), 4-methoxyphenylboronic acid (5.0 mg), [1,1'-bis (diphenylphosphino) ferrocene] palladium (II) dichloride dichloromethane adduct (4.0 mg), and a sodium carbonate aqueous solution (2 M, 100 μL) was added sequentially at room temperature, and the reaction solution was stirred at 100°C for 1 hour. The reaction solution was allowed to cool to room temperature, insoluble matter was removed by CELITE filtration, and the residue was washed with hexane / ethyl acetate = 1/1 (10 mL). The combined filtrate was concentrated under reduced pressure, and the obtained residue was purified by reverse phase HPLC (water / acetonitrile) to give the title compound.

[Examples 299-324]

Using N-((1S,2R)-2-(3-bromo-6-fluoro-2-methylphenyl)-1-(5-oxo-4,5-dihydro-1,3,4-oxadiazol-2-yl)propyl)-4-chloro-2-methoxybenzenesulfonamide obtained from Example 298 Step 1, according to the method of Example 298, Step 2, compounds of Examples 299 to 324 shown below were synthesized. The boronic acids or boronic acid esters used are listed in the following table.

[Table 19]

| Example | Reagent | Name of the Synthesized Compound |
|---|---|---|
| 299 | 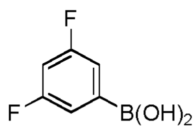 | 4-chloro-2-methoxy-N-((1S,2R)-1-(5-oxo-4,5-dihydro-1,3,4-oxadiazol-2-yl)-2-(3',4,5'-trifluoro-2-methyl-[1,1'-biphenyl]-3-yl)propyl)benzenesulfonamide |
| 300 | 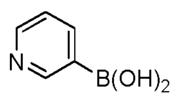 | 4-chloro-N-((1S,2R)-2-(6-fluoro-2-methyl-3-(pyridin-3-yl)phenyl)-1-(5-oxo-4,5-dihydro-1,3,4-oxadiazol-2-yl)propyl)-2-methoxybenzenesulfonamide |
| 301 | 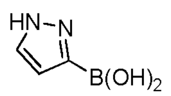 | 4-chloro-N-((1S,2R)-2-(6-fluoro-2-methyl-3-(1H-pyrazol-3-yl)phenyl)-1-(5-oxo-4,5-dihydro-1,3,4-oxadiazol-2-yl)propyl)-2-methoxybenzenesulfonamide |
| 302 | 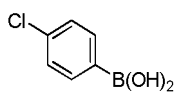 | 4-chloro-N-((1S,2R)-2-(4'-chloro-4-fluoro-2-methyl-[1,1'-biphenyl]-3-yl)-1-(5-oxo-4,5-dihydro-1,3,4-oxadiazol-2-yl)propyl)-2-methoxybenzenesulfonamide |
| 303 | 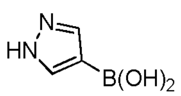 | 4-chloro-N-((1S,2R)-2-(6-fluoro-2-methyl-3-(1H-pyrazol-4-yl)phenyl)-1-(5-oxo-4,5-dihydro-1,3,4-oxadiazol-2-yl)propyl)-2-methoxybenzenesulfonamide |

(continued)

| Example | Reagent | Name of the Synthesized Compound |
|---|---|---|
| 304 | 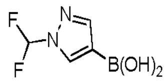 | 4-chloro-N-((1S,2R)-2-(3-(1-(difluoromethyl)-1H-pyrazol-4-yl)-6-fluoro-2-methylphenyl)-1-(5-oxo-4,5-dihydro-1,3,4-oxadiazol-2-yl)propyl)-2-methoxybenzenesulfonamide |
| 305 | 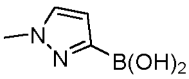 | 4-chloro-N-((1S,2R)-2-(6-fluoro-2-methyl-3-(1-methyl-1H-pyrazol-3-yl)phenyl)-1-(5-oxo-4,5-dihydro-1,3,4-oxadiazol-2-yl)propyl)-2-methoxybenzenesulfonamide |
| 306 | 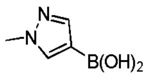 | 4-chloro-N-((1S,2R)-2-(6-fluoro-2-methyl-3-(1-methyl-1H-pyrazol-4-yl)phenyl)-1-(5-oxo-4,5-dihydro-1,3,4-oxadiazol-2-yl)propyl)-2-methoxybenzenesulfonamide |
| 307 | 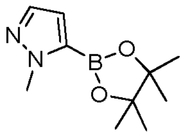 | 4-chloro-N-((1S,2R)-2-(6-fluoro-2-methyl-3-(1-methyl-1H-pyrazol-5-yl)phenyl)-1-(5-oxo-4,5-dihydro-1,3,4-oxadiazol-2-yl)propyl)-2-methoxybenzenesulfonamide |
| 308 | 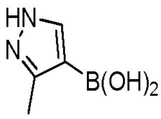 | 4-chloro-N-((1S,2R)-2-(6-fluoro-2-methyl-3-(3-methyl-1H-pyrazol-4-yl)phenyl)-1-(5-oxo-4,5-dihydro-1,3,4-oxadiazol-2-yl)propyl)-2-methoxybenzenesulfonamide |
| 309 | 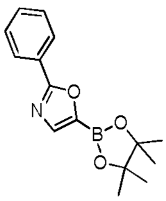 | 4-chloro-N-((1S,2R)-2-(6-fluoro-2-methyl-3-(2-phenyloxazol-5 -yl)phenyl)-1-(5-oxo-4,5-dihydro-1,3,4-oxadiazol-2-yl)propyl)-2-methoxybenzenesulfonamide |
| 310 | 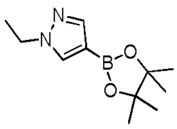 | 4-chloro-N-((1S,2R)-2-(3-(1-ethyl-1H-pyrazol-4-yl)-6-fluoro-2-methylphenyl)-1-(5-oxo-4,5-dihydro-1,3,4-oxadiazol-2-yl)propyl)-2-methoxybenzenesulfonamide |
| 311 | 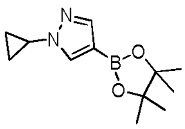 | 4-chloro-N-((1S,2R)-2-(3-(1-cyclopropyl-1H-pyrazol-4-yl)-6-fluoro-2-methylphenyl)-1-(5-oxo-4,5-dihydro-1,3,4-oxadiazol-2-yl)propyl)-2-methoxybenzenesulfonamide |
| 312 | 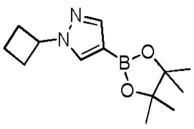 | 4-chloro-N-((1S,2R)-2-(3-(1-cyclobutyl-1H-pyrazol-4-yl)-6-fluoro-2-methylphenyl)-1-(5-oxo-4,5-dihydro-1,3,4-oxadiazol-2-yl)propyl)-2-methoxybenzenesulfonamide |
| 313 | 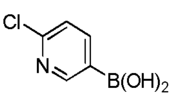 | 4-chloro-N-((1S,2R)-2-(3-(6-chloropyridin-3-yl)-6-fluoro-2-methylphenyl)-1-(5-oxo-4,5-dihydro-1,3,4-oxadiazol-2-yl)propyl)-2-methoxybenzenesulfonamide |
| 314 | 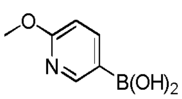 | 4-chloro-N-((1S,2R)-2-(6-fluoro-3-(6-methoxypyridin-3-yl)-2-methylphenyl)-1-(5-oxo-4,5-dihydro-1,3,4-oxadiazol-2-yl)propyl)-2-methoxybenzenesulfonamide |
| 315 | 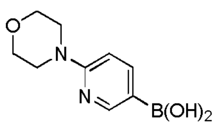 | 4-chloro-N-((1S,2R)-2-(6-fluoro-2-methyl-3-(6-morpholinopyridin-3-yl)phenyl)-1-(5-oxo-4,5-dihydro-1,3,4-oxadiazol-2-yl)propyl)-2-methoxybenzenesulfonamide |

(continued)

| Example | Reagent | Name of the Synthesized Compound |
|---|---|---|
| 316 | 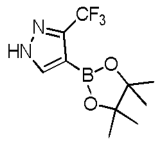 | 4-chloro-N-((1S,2R)-2-(6-fluoro-2-methyl-3-(3-(tifluoromethyl)-1H-pyrazol-4-yl)phenyl)-1-(5-oxo-4,5-dihydro-1,3,4-oxadiazol-2-yl)propyl)-2-methoxybenzenesulfonamide |
| 317 | 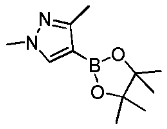 | 4-chloro-N-((1S,2R)-2-(3-(1,3-dimethyl-1H-pyrazol-4-yl)-6-fluoro-2-methylphenyl)-1-(5-oxo-4,5-dihydro-1,3,4-oxadiazol-2-yl)propyl)-2-methoxybenzenesulfonamide |
| 318 | 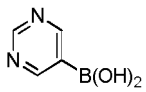 | 4-chloro-N-((1S,2R)-2-(6-fluoro-2-methyl-3-(pyrimidin-5-yl)phenyl)-1-(5-oxo-4,5-dihydro-1,3,4-oxadiazol-2-yl)propyl)-2-methoxybenzenesulfonamide |
| 319 | 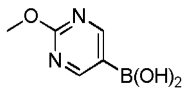 | 4-chloro-N-((1S,2R)-2-(6-fluoro-3-(2-methoxypyrimidin-5-yl)-2-methylphenyl)-1-(5-oxo-4,5-dihydro-1,3,4-oxadiazol-2-yl)propyl)-2-methoxybenzenesulfonamide |
| 320 | 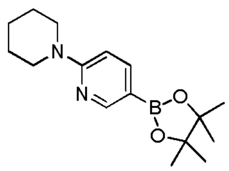 | 4-chloro-N-((1S,2R)-2-(6-fluoro-2-methyl-3-(6-(piperidin-1-yl)pyridin-3-yl) phenyl)-1-(5-oxo-4,5-dihydro-1,3,4-oxadiazol-2-yl)propyl)-2-methoxybenzenesulfonamide |
| 321 | 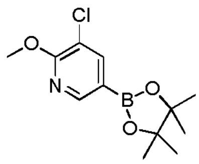 | 4-chloro-N-((1S,2R)-2-(3-(5-chloro-6-methoxypyridin-3-yl)-6-fluoro-2-methylphenyl)-1-(5-oxo-4,5-dihydro-1,3,4-oxadiazol-2-yl)propyl)-2-methoxybenzenesulfonamide |
| 322 | 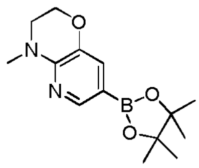 | 4-chloro-N-((1S,2R)-2-(6-fluoro-2-methyl-3-(4-methyl-3,4-dihydro-2H-pyrido [3,2-b][1,4]oxazine-7-yl)phenyl)-1-(5-oxo-4,5-dihydro-1,3,4-oxadiazol-2-yl) propyl) -2-methoxybenzenesulfonamide |
| 323 | 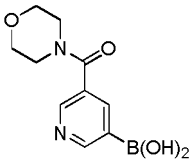 | 4-chloro-N-((1S,2R)-2-(6-fluoro-2-methyl-3-(5-(morpholine-4-carbonyl) pyridin-3-yl)phenyl)-1-(5-oxo-4,5-dihydro-1,3,4-oxadiazol-2-yl)propyl)-2-methoxybenzenesulfonamide |
| 324 | 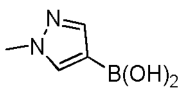 | 5-chloro-2-(N-((1S,2R)-2-(6-fluoro-2-methyl-3-(1-methyl-1H-pyrazol-4-yl) phenyl)-1-(5-oxo-4,5-dihydro-1,3,4-oxadiazol-2-yl)propyl) sulfamoyl) benzamide |

[Example 325]

Synthesis of 4-chloro-N-((1S,2R)-2-(6-fluoro-2,3-dimethylphenyl)-1-(5-oxo-4,5-dihydro-1,3,4-oxadiazol-2-yl)propyl)-3-(1-methyl-1H-pyrazol-4-yl)benzenesulfonamide

(Step 1)

From (2S,3R)-2-amino-3-(6-fluoro-2,3-dimethylphenyl)butanoic acid (200 mg) obtained from Reference Example D1 and 3-bromo-4-chlorobenzenesulfonyl chloride (306 mg), according to the method of Steps 1 and 2 of Example 1,3-bromo-4-chloro-N-((1S,2R)-2-(6-fluoro-2,3-dimethylphenyl)-1-(5-oxo-4,5-dihydro-1,3,4-oxadiazol-2-yl)propyl)benzenesulfonamide (274 mg) was synthesized.

(Step 2)

To a 1,4-dioxane (0.7 ml) solution of 3-bromo-4-chloro-N-((1S,2R)-2-(6-fluoro-2,3-dimethylphenyl)-1-(5-oxo-4,5-dihydro-1,3,4-oxadiazol-2-yl)propyl)benzenesulfonamide (5.6 mg) obtained from the above Step 1, (1-methyl-1H-pyrazol-4-yl)boronic acid (6.2 mg), [1,1'-bis(diphenylphosphino)ferrocene] palladium (II) dichloride dichloromethane adduct (5.0 mg), a sodium carbonate aqueous solution (2 M, 100 μL) were added sequentially at room temperature, and the reaction solution was stirred for 4 hours at 100°C. The reaction solution was allowed to cool to room temperature, insoluble matter was removed by CELITE filtration, and the residue was washed with hexane / ethyl acetate = 1/1 (10 mL). The combined filtrate was concentrated under reduced pressure, and the obtained residue was purified by reverse phase HPLC (water / acetonitrile) to give the title compound.

[Example 326]

Synthesis of 6-chloro-2'-fluoro-N-((1S,2R)-2-(6-fluoro-2,3-dimethylphenyl)-1-(5-oxo-4,5-dihydro-1,3,4-oxadiazol-2-yl)propyl)-[1,1'-biphenyl]-3-sulfonamide

Using 3-bromo-4-chloro-N-((1S,2R)-2-(6-fluoro-2,3-dimethylphenyl)-1-(5-oxo-4,5-dihydro-1,3,4-oxadiazol-2-yl)propyl)benzenesulfonamide obtained from Example 325 Step 1 and (2-fluorophenyl)boronic acid, the title compound was synthesized according to the method of Example 325 Step 2.

[Example 327]

Synthesis of 4-chloro-N-((1S,2R)-2-(6-fluoro-2-methyl-3-(phenylethynyl)phenyl)-1-(5-oxo-4,5-dihydro-1,3,4-oxadiazol-2-yl)propyl)-2-methoxybenzenesulfonamide

To a DMF (1.0 mL) solution of N-((1S,2R)-2-(3-bromo-6-fluoro-2-methylphenyl)-1-(5-oxo-4,5-dihydro-1,3,4-oxadiazol-2-yl)propyl)-4-chloro-2-methoxybenzenesulfonamide (10.9 mg) obtained from Example 298 Step 1, dichlorobis (triphenylphosphine) palladium (II) (1.5 mg), copper (I) iodide (1.5 mg), triethylamine (30 μL) and ethynylbenzene (20 μL) were sequentially added at room temperature, the reaction solution was added at 100°C, and the mixture was stirred for 4 hours. The reaction solution was allowed to cool to room temperature, and insoluble matter was removed by CELITE filtration, and the residue was washed with hexane / ethyl acetate = 1/1 (10 mL). The combined filtrate was concentrated under reduced pressure, and the obtained residue was purified by reverse phase HPLC (water / acetonitrile) to give the title compound.

[Example 328]

Synthesis of 4-amino-5-chloro-N-((1S,2R)-2-(3-chloro-6-fluoro-2-methylphenyl)-1-(5-oxo-4,5-dihydro-1,3,4-oxadiazol-2-yl)propyl)-4-methylchroman-8-sulfonamide

To a benzene (1.5ml) solution of 5-chloro-N-((1S,2R)-2-(3-chloro-6-fluoro-2-methylphenyl)-1-(5-oxo-4,5-dihydro-1,3,4-oxadiazol-2-yl)propyl)-4-hydroxy-4-methylchroman-8-sulfonamide (17 mg) obtained from Example 209A, trimethylsilylazide (50 μL), boron trifluoride-dimethylethercomplex (100 μL) were sequentially added, and the reaction solution was stirred for 1 hour at room temperature. To the reaction solution, a saturated sodium bicarbonate aqueous solution (10 mL) was added, and the mixture was extracted with ethyl acetate/hexane=1/1 (10 mL). The organic layer was washed with saturated saline (10 mL), dried with anhydride sodium sulfate, and concentrated under reduced pressure. The obtained residue was dissolved in THF (1.5 ml) and water (50 μL). Triphenylphosphine (15 mg) was added to the residue, and the reaction solution was stirred for 2 hours at room temperature. Insoluble matter was removed by CELITE filtration, and the residue was washed with ethyl acetate / hexane = 1/1 (10 mL). The combined filtrate was concentrated under reduced pressure, and the obtained residue was purified by reverse phase HPLC (water / acetonitrile) to give the title compound as a 1:1 diastereomeric mixture.

[Example 329]

Synthesis of 4-amino -N-((1S,2R)-2-(3-bromo-6-fluoro-2-methylphenyl)-1-(5-oxo-4,5-dihydro-1,3,4-oxadiazol-2-yl)propyl)-5-chloro-4-methyl-chroman-8-sulfonamide

The title compound was prepared using N-((1S,2R)-2-(3-bromo-6-fluoro-2-methylphenyl)-1-(5-oxo-4,5-dihydro-1,3,4-oxadiazol-2-yl)propyl)-5-chloro-4-hydroxy-4-methylchroman-8-sulfonamide obtained in Example 222A according to the method of example 328.

[Example 330]

Synthesis of 4-amino-5-chloro-N-((1S,2R)-2-(3-chloro-6-fluoro-2-methylphenyl)-1-(5-oxo-4,5-dihydro-1,3,4-oxadiazol-2-yl)propyl)-4-methyl chroman-8-sulfonamide isomer A and isomer B

To a 1,4-dioxane solution (1.0 mL) of a diastereomeric mixture of 4-amino-5-chloro-N-((1S,2R)-2-(3-chloro-6-fluoro-2-methylphenyl)-1-(5-oxo-4,5-dihydro-1,3,4-oxadiazol-2-yl)propyl)-4-methylchroman-8-sulfonamide (6.4 mg) obtained in Example 328, triethylamine (100 μL) and di-tert-butyl dicarbonate (54 mg) were added at room temperature, and the reaction solution was stirred for 4 hours. The reaction solution was concentrated under reduced pressure, the obtained residue was purified by reverse phase HPLC (water / acetonitrile), and the fractions were concentrated to give each of two diastereomeric products. The substance eluted first was designated Compound A, and the substance eluted later was designated as Compound B. The obtained Compounds A and B were each dissolved in hydrochloric acid-dioxane (4 M, 2.0 mL), and the reaction solution was stirred at 70°C for 4 hours. The reaction solution was allowed to cool to room temperature and concentrated under reduced pressure. The substance obtained from compound A was designated as compound 330A, and the substance obtained from compound B as compound 330B.

[Example 331]

Synthesis of 2-(N-((1S,2R)-2-(6-fluoro-2,3-dimethylphenyl)-1-(5-oxo-4,5-dihydro-1,3,4-oxadiazol-2-yl)propyl)sulfamoyl)-4-(1,3,4-oxadiazol-2-yl)benzamide

(Step 1)

Using (2S,3R)-2-amino-3-(6-fluoro-2,3-dimethylphenyl)butanoic acid (80 mg) obtained in Reference Example D1 and ethyl 3-(chlorosulfonyl)-4-cyanobenzoate (146 mg) obtained in Reference Example E13, according to the method of steps 1 and 2 of Example 1, ethyl 4-cyano-3-(N-((1S,2R)-2-(6-fluoro-2,3-dimethylphenyl)-1-(5-oxo-4,5-dihydro-1,3,4-oxadiazol-2-yl)propyl)sulfamoyl)benzoate (40 mg) was obtained.

(Step 2)

To a DMSO (1 mL) solution of ethyl 4-cyano-3-(N-((1S,2R)-2-(6-fluoro-2,3-dimethylphenyl)-1-(5-oxo-4,5-dihydro-1,3,4-oxadiazol-2-yl)propyl)sulfamoyl)benzoate (40 mg) obtained from the above Step 1, 30% hydrogen peroxide water (0.5 mL) and potassium carbonate (20 mg) were added, and the reaction solution was stirred at 70°C for 1 hour. 1M hydrochloric acid was added to the reaction solution, and the mixture was extracted with ethyl acetate. The organic layer was washed with saturated saline, dried over anhydrous magnesium sulfate, concentrated under reduced pressure, and the obtained residue was purified by reversed phase HPLC (water / acetonitrile) to give 4-carbamoyl-3-(N-((1S,2R)-2-(6-fluoro-2,3-dimethylphenyl)-1-(5-oxo-4,5-dihydro-1,3,4-oxadiazol-2-yl)propyl)sulfamoyl)benzoic acid (8.9 mg).

(Step 3)

To a dichloromethane (1.5 mL) solution of 4-carbamoyl-3-(N-((1S,2R)-2-(6-fluoro-2,3-dimethylphenyl)-1-(5-oxo-4,5-dihydro-1,3,4-oxadiazol-2-yl)propyl)sulfamoyl)benzoic acid (16 mg) obtained from the above Step 2, (isocyanoimino) triphenylphosphorane (36 mg) was added thereto, and the reaction solution was stirred at room temperature for 72 hours. The reaction solution was concentrated under reduced pressure, and the obtained residue was purified by reversed phase HPLC (water / acetonitrile) to obtain the title compound (1.1 mg).

[Example 332]

Synthesis of 5-bromo -2-(N-((1S,2R)-2-(6-fluoro-2,3-dimethylphenyl)-1-(5-thioxo-4,5-dihydro-1,3,4-oxadiazol-2-yl)propyl)sulfamoyl)benzamide

(Step 1)

(2S,3R)-2-amino-3-(6-fluoro-2,3-dimethylphenyl)butanoic acid (300 mg) obtained from Reference Example D1 was dissolved in water (5.0 mL) and 1,4-dioxane (5.0 mL), triethylamine (570 μL) was added, and it was cooled to 0°C. 4-Bromo-2-cyanobenzene-1-sulfonyl chloride (362 mg) was added to the reaction solution, and the mixture was stirred at the same temperature for 45 minutes. The reaction solution was added to hydrochloric acid (1 M, 15 mL) and extracted with ethyl acetate (15 mL). The organic layer was washed with saturated saline (20 mL), dried over anhydrous magnesium sulfate, and concentrated under reduced pressure. The residue was purified by silica gel column chromatography (eluent: hexane / ethyl acetate / 2% acetic acid) to give (2S,3R)-2-(4-bromo-2-cyanophenylsulfonamido)-3-(6-fluoro-2,3-dimethylphenyl)butanoic acid (465 mg).

(Step 2)

To a THF (1.5 mL) solution of (2S,3R)-2-(4-bromo-2-cyanophenylsulfonamido)-3-(6-fluoro-2,3-dimethylphenyl)butanoic acid (22 mg) obtained from the above Step 1, CDI (13 mg) was added, the reaction solution was stirred at room temperature for 30 minutes, then hydrazine · monohydrate (12 μL) was added and the mixture was stirred for 20 minutes. The reaction solution was concentrated under reduced pressure, and the obtained residue was dissolved in ethanol (1.2 mL), carbon disulfide (10 μL) and potassium hydroxide (10 mg) were sequentially added, and the reaction solution was stirred at 90°C for 12 hours. The reaction solution was added to hydrochloric acid (1 M, 10 mL) and extracted with ethyl acetate (10 mL). The organic layer was washed with saturated saline(10 mL), dried over anhydrous magnesium sulfate, and concentrated under reduced pressure. The residue was purified by silica gel column chromatography (eluent: hexane / ethyl acetate) to give the title compound.

[Examples 333 to 335]

According to the method of Example 332 steps 1 and 2, the compounds of Examples 333 to 335 shown below were synthesized. The raw materials are listed in the following table.

[Table 20]

| Example | Starting Material | ArSO2Cl | Name of the Synthesized Compound |
|---|---|---|---|
| 333 | Reference Example D10 | 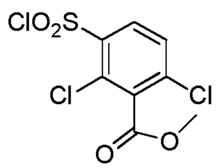 | methyl 2,6-dichloro-3-(N-((1S,2R)-2-(2,3-dihydro-1H-inden-4-yl)-1-(5-thioxo-4,5-dihydro-1,3,4-oxadiazol-2-yl)propyl) sulfamoyl)benzoate |
| 334 | Reference Example D10 | 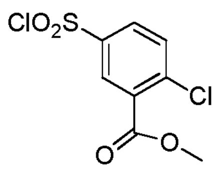 | methyl 2-chloro-5-(N-((1S,2R)-2-(2,3-dihydro-1H-inden-4-yl)-1-(5-thioxo-4,5-dihydro-1,3,4-oxadiazol-2-yl)propyl) sulfamoyl) benzoate |
| 335 | Reference Example D26 | 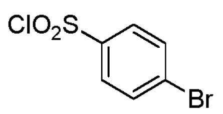 | 4-bromo-N-((1S,2R)-2-(5,5-dimethyl-5,6,7,8-tetrahydronaphthalen-1-yl)-1-(5-thioxo-4,5-dihydro-1,3,4-oxadiazol-2-yl)propyl)benzenesulfonamide |

[Example 336]

Synthesis of 4-chloro-N-((1S,2R)-2-(2,3-dihydro-1H-inden-4-yl)-1-(5-thioxo-4,5-dihydro-1,3,4-oxadiazol-2-yl)propyl)-3-(2-hydroxypropan-2-yl)benzenesulfonamide

(Step 1)

Using (2S,3R)-2-amino-3-(2,3-dihydro-1H-inden-4-yl)butanoic acid (20 mg) and 3-acetyl-4-chlorobenzene-1-sulfonyl chloride (20 mg) obtained in Reference Example D10, according to the method of Example 332 steps 1 and 2, 3-acetyl-4-chloro-N-((1S,2R)-2-(2,3-dihydro-1H-inden-4-yl)-1-(5-thioxo-4,5-dihydro-1,3,4-oxadiazol-2-yl)propyl)benzenesulfonamide (12 mg) was obtained.

(Step 2)

From 3-acetyl-4-chloro-N-((1S,2R)-2-(2,3-dihydro-1H-inden-4-yl)-1-(5-thioxo-4,5-dihydro-1,3,4-oxadiazol-2-yl)propyl)benzenesulfonamide (12 mg) obtained from the above Step 1, the title compound was obtained according to the method of Example 186 Step 2.

[Example 337]

Synthesis of 5-chloro-2-(N-((1S,2R)-2-(6-fluoro-2,3-dimethylphenyl)-1-(5-oxo-4,5-dihydro-1,3,4 oxadiazol-2-yl)propyl)-N-methylsulfamoyl)benzamide

(Step 1)

Using (2S,3R)-2-amino-3-(6-fluoro-2,3-dimethylphenyl)butanoic acid (530 mg) obtained from Reference Example D1 and 4-chloro-2-cyanobenzene-1-sulfonyl chloride (660 mg), according to the method of Example 1 Step 1, (2S,3R)-2-(4-chloro-2-cyanophenylsulfonamido)-3-(6-fluoro-2,3-dimethylphenyl)butanoic acid (777 mg) was obtained.

(Step 2)

To a THF (500 μL) solution of (2S,3R)-2-(4-chloro-2-cyanophenylsulfonamido)-3-(6-fluoro-2,3-dimethylphenyl)butanoic acid (11 mg) obtained from the above Step 1, CDI (15 mg) was added, and the reaction solution was stirred for 1 hour at room temperature. Methanol (1.0 mL) was added to the reaction solution, and the mixture was further stirred for 16 hours. The reaction solution was added to water (10 mL) and extracted with diethyl ether (15 mL). The organic layer was washed with saturated saline (10 mL), dried over anhydrous magnesium sulfate, and concentrated under reduced pressure to give methyl (2S,3R)-2-(4-chloro-2-cyanophenyl)-3-(6-fluoro-2,3-dimethylphenyl)butanoate (12 mg).

(Step 3)

To methyl (2S,3R)-2-(4-chloro-2-cyanophenylsulfonamido)-3-(6-fluoro-2,3-dimethylphenyl)butanoate (100 mg) obtained from the above Step 2, Methanol (2 mL), dichloromethane (2 mL), and a hexane solution of trimethylsilyl diazomethane (0.6 M, 800 μL) were sequentially added, and the reaction solution was stirred at room temperature for 1 hour. By concentrating the reaction solution under reduced pressure, methyl (2S,3R)-2-(4-chloro-2-cyano-N-methylphenylsulfonamido)-3-(6-fluoro-2,3-dimethylphenyl)butanoate (101 mg) was obtained.

(Step 4)

Methyl (2S,3R)-2-(4-chloro-2-cyano-N-methylphenylsulfonamido)-3-(6-fluoro-2,3-dimethylphenyl)butanoate (101 mg) obtained from the Step 3 above, according to the method of Example 245 Step 2, (2S,3R)-2-(4-chloro-2-cyano-N-methylphenylsulfonamido)-3-(6-fluoro-2,3-dimethylphenyl)butanoic acid (8.5 mg) was obtained.

(Step 5)

To (2S,3R)-2-(4-chloro-2-cyano-N-methylphenylsulfonamide) -3-(6-fluoro-2,3-dimethylphenyl)butanoic acid (8.5 mg) obtained from the above Step 4, according to the method of Example 1 Step 2, 4-chloro-2-cyano-N-((1S,2R)-2-(6-fluoro-2,3-dimethylphenyl)-1-(5-oxo-4,5-dihydro-1,3,4-oxadiazol-2-yl)propyl)-N-methylbenzenesulfonamide (6.0 mg) was obtained.

(Step 6)

From 4-chloro-2-cyano-N-((1S,2R)-2-(6-fluoro-2,3-dimethylphenyl)-1-(5-oxo-4,5-dihydro-1,3,4-oxadiazol-2-yl)propyl)-N-methylbenzenesulfonamide (6.0 mg) obtained from the above Step 5, according to the method of Example 1 Step 3, the title compound was obtained.

[Example 344]

Synthesis of 6-chloro-N-((1S,2R)-2-(6-fluoro-2,3-dimethylphenyl)-1-(5-oxo-4,5-dihydro-1,3,4-oxadiazol-2-yl)propyl)-4'-methoxy-[1,1'-biphenyl]-3-sulfonamide

Using 3-bromo-4-chloro-N-((1S,2R)-2-(6-fluoro-2,3-dimethylphenyl)-1-(5-oxo-4,5-dihydro-1,3,4-oxadiazol-2-yl)propyl)benzenesulfonamide and (4-methoxyphenyl)boronic acid obtained from Example 325 step 1, the title compound was synthesized according to the method of Example 325 Step 2.

[Example 345]

Synthesis of 3-N-((1S,2R)-2-(6-fluoro-2,3-dimethylphenyl)-1-(5-oxo-4,5-dihydro-1,3,4-oxadiazol-2-yl)propyl)sulfamoyl)-2'-methoxy-[1,1'-biphenyl]-4-carboxamide

(Step 1)

From (2S,3R)-2-amino-3-(6-fluoro-2,3-dimethylphenyl)butanoic acid obtained from Reference Example D1 and 5-bromo-2-cyanobenzenesulfonyl chloride, according to the method of steps 1 and 2 of Example 1, 5-bromo-2-cyano-N-((1S,2R)-2-(6-fluoro-2,3-dimethylphenyl)-1-(5-oxo-4,5-dihydro-1,3,4-oxadiazol-2-yl)propyl)benzenesulfonamide was synthesized.

(Step 2)

Using 5-bromo-2-cyano-N-((1S,2R)-2-(6-fluoro-2,3-dimethylphenyl)-1-(5-oxo-4,5-dihydro-1,3,4-oxadiazol-2-yl)propyl)benzenesulfonamide obtained from the above Step 1 and 2-methoxyphenylboronic acid, according to the method of Example 325 Step 2 and Example Step 3, the title compound was obtained.

[Example 346]

Synthesis of 4-(N-((1S,2R)-(6-fluoro-2,3-dimethylphenyl)-1-(5-oxo-4,5-dihydro-1,3,4-oxadiazol-2 yl)propyl)sulfamoyl)-3-methoxybenzamide

From 4-cyano-N-((1S,2R)-(6-fluoro-2,3-dimethylphenyl)-1-(5-oxo-4,5-dihydro-1,3,4-oxadiazol-2-yl)propyl)-2-methoxybenzenesulfonamide obtained in Example 342, the title compound was obtained according to the method of Example 1 Step 3.

[Example 347]

Synthesis of 4-(N-((1S,2R)-(6-fluoro-2,3-dimethylphenyl)-1-(5-oxo-4,5-dihydro-1,3,4-oxadiazol-2 yl)propyl)sulfamoyl)benzamide

From 4-cyano-N-((1S,2R)-(6-fluoro-2,3-dimethylphenyl)-1-(5-oxo-4,5-dihydro-1,3,4-oxadiazol-2-yl)propyl)benzenesulfonamide obtained in Example 341, according to the method of Example 1 Step 3, the title compound was obtained.

[Example 348]

Synthesis of 4-bromo -N-((1S,2R)-2-(naphthalen-1-yl)-1-(5-oxo-4,5-dihydro-1,2,4-oxadiazol-3-yl)propyl)benzenesulfonamide

(Step 1)

Using (2S,3R)-2-amino-3-(naphthalen-1-yl)butanoic acid obtained in Reference Example D11 and 4-bromobenzenesulfonyl chloride, according to the method of Step 1 of Example 1, (2S,3R)-2-((4-bromophenyl)sulfonamido)-3-(naphthalen-1-yl)butanoic acid was obtained.

(Step 2)

To a DMF (2.5 mL) solution of (2S,3R)-2-((4-bromophenyl)sulfonamido)-3-(naphthalen-1-yl)butanoic acid (283 mg) obtained from the above Step 1, ammonium chloride (41 mg), HOBt (103 mg), triethylamine (0.264 mL) and WSC (146 mg) were added, and the reaction solution was stirred at room temperature for 3 hours. The reaction solution was added to water and extracted with ethyl acetate. The organic layer was washed with saturated saline, dried over anhydrous magnesium sulfate and concentrated under reduced pressure to obtain (2S,3R)-2-((4-bromophenyl)sulfonamido)-3-(naphthalen-1-yl)butanamide as a crude product.

(Step 3)

To a DMF (2 mL) solution of (2S,3R)-2-((4-bromophenyl)sulfonamido)-3-(naphthalen-1-yl)butanamide obtained from the above Step 2, cyanuric chloride (59 mg) was added at 0°C, and the reaction solution was stirred at room temperature for 1 hour. The reaction solution was added to water and extracted with a mixed solvent of ethyl acetate / toluene. The organic layer was washed with saturated saline, dried over anhydrous sodium sulfate, and concentrated under reduced pressure. The residue was purified by silica gel column chromatography (eluent: hexane / ethyl acetate) to give 4-bromo-N-((1S,2R)-1-cyano-2-(naphthalen-1-yl)propyl)benzene sulfonamide (137 mg).

(Step 4)

To an ethanol (2 mL) solution of 4-bromo-N-((1S,2R)-1-cyano-2-(naphthalen-1-yl)propyl)benzenesulfonamide (137 mg) obtained from the above Step 3, water (0.66 mL) and a 50% aqueous solution of hydroxylamine (0.060 mL) were added, and the reaction solution was stirred at room temperature overnight. The reaction solution was concentrated under reduced pressure, added to water and extracted with ethyl acetate. The organic layer was washed with saturated saline, dried over anhydrous sodium sulfate and concentrated under reduced pressure to give (2S,3R)-2-((4-bromophenyl)sulfonamido)-N-hydroxy-(3-naphthalen-1-yl)butanamide (130 mg) as a crude product.

(Step 5)

To a DMF (1.0 mL) solution of (2S,3R)-2-((4-bromophenyl)sulfonamido)-N-hydroxy-3-(naphthalen-1-yl)butanamide (20 mg) obtained from the above Step 4, Pyridine (0.004 mL) and 2-ethylhexyl chloroformate (0.009 mL) were added, and the reaction solution was stirred at room temperature for 1 hour. Further, xylene was added, and the reaction solution was stirred overnight at 100°C. Water was added to the reaction solution, and the mixture was extracted with a mixed solvent of ethyl acetate / hexane. The organic layer was washed with saturated saline, dried over anhydrous sodium sulfate, and concentrated under reduced pressure. The obtained residue was subjected to silica gel column chromatography (eluent: hexane / ethyl acetate) to give the title compound (37 mg).

[Example 349]

Synthesis of 4-chloro-N-((1S,2R)-2-(6-fluoro-2,3-dimethylphenyl)-1-(5-oxo-4,5-dihydro-1,2,4-oxadiazol-3-yl)propyl)-2-methoxybenzenesulfonamide

(Step 1)

Using (2S,3R)-2-amino-3-(6-fluoro-2,3-dimethylphenyl)butanoic acid obtained in Reference Example D1 and 4-chloro-2-methoxybenzenesulfonyl chloride, according to the method of Example 1 Step 1, (2S,3R)-2-((4-chloro-2-methoxyphenyl)sulfonamido)-3-(6-fluoro-2,3-dimethylphenyl)butanoic acid was obtained.

(Step 2)

From (2S,3R)-2-((4-chloro-2-methoxyphenyl)sulfonamido)-3-(6-fluoro-2,3-dimethylphenyl)butanoic acid obtained from the above Step 1, the titled compound was obtained according to the method of Example 348 Step 2 to Step 5.

[Example 350]

(2S,3R)-2-((4-chloro-2-methoxyphenyl)sulfonamido)-3-(6-fluoro-2,3-dimethylphenyl)butanoic acid (142 mg) obtained from Example 348 Step 1 was dissolved in a DMF (3.3 mL), and WSC (130 mg), HOBt (100 mg), N, N-diisopropylethylamine (200 μL) and thiosemicarbazide (70 mg) were sequentially added, and the reaction solution was stirred at 80°C for 4 hours. The reaction solution was added to a saturated aqueous solution of ammonium chloride (15 mL) and extracted with ethyl acetate (20 mL). The organic layer was washed with saturated saline (15 mL), dried over anhydrous magnesium sulfate, and concentrated under reduced pressure. The residue was purified by silica gel column chromatography (eluent: hexane / ethyl acetate). The obtained residue was dissolved in ethanol (1.5 mL), a 20% aqueous sodium hydroxide solution (2.0 mL) was added, and the reaction solution was stirred at 80°C for 12 hours. The reaction solution was added to hydrochloric acid (1 M, 5.0 mL) and extracted with ethyl acetate (10 mL). The organic layer was washed with saturated saline (5.0 mL), dried over anhydrous sodium sulfate, and concentrated under reduced pressure. The residue was purified by silica gel column chromatography (eluent: hexane / ethyl acetate) to give the title compound.

Hereinafter, the structural formulas and physical properties of Example Compounds 1 to 350 are shown.

[Table 21]

| Example | Structural Formula | Physical Property Value |
|---|---|---|
| 1 | 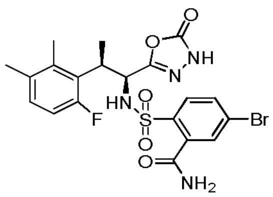 | 1H NMR (CD3OD) δ: 7.74-7.78 (m, 3H), 6.97 (dd, J=8.2, 5.7 Hz, 1H), 6.71 (dd, J=11.7, 8.4 Hz, 1H), 4.78-4.81 (m, 1H), 3.51-3.61 (m, 1H), 2.20 (s, 3H), 2.17 (s, 3H), 1.44 (d, J=7.0 Hz, 3H); LC/MS RT 1.67min, m/z [M-H]$^-$ 525,527 |
| 2 | 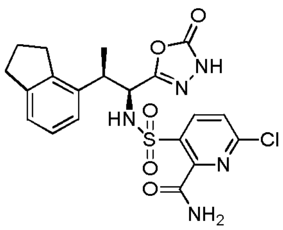 | 1H NMR (CD3OD) δ: 8.20 (1H, d, J = 8.1 Hz), 7.65 (1H, d, J = 8.4 Hz), 7.03-7.00 (1H, m), 6.98-6.96 (2H, m), 4.63-4.61 (1H, m), 3.40-3.36 (1H, m), 2.91-2.82 (4H, m), 2.03-1.99 (2H, m), 1.41 (3H, d, J = 7.0 Hz); LC/MS RT 1.66min, m/z [M-H]$^-$ 476,478 |
| 3 | 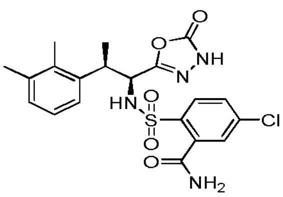 | 1H NMR (CD3OD) δ: 7.77 (d, J=8.4 Hz, 1H), 7.59 (d, J=2.2 Hz, 1H), 7.53 (dd, J=8.4, 2.2 Hz, 1H), 7.03 (dd, J=7.0, 2.2 Hz, 1H), 6.90-6.99 (m, 2H), 4.53 (d, J=9.5 Hz, 1H), 3.52-3.61 (m, 1H), 2.20 (s, 3H), 2.18 (s, 3H), 1.35 (d, J=7.0 Hz, 3H); LC/MS RT 1.63min, m/z [M-H]$^-$ 463,465 |
| 4 | 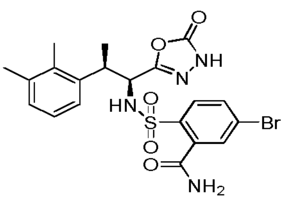 | 1H NMR (CD3OD) δ: 7.74 (s, 1H), 7.67-7.69 (m, 2H), 7.01-7.07 (m, J=6.2 Hz, 1H), 6.91-6.99 (m, 2H), 4.54 (d, J=9.5 Hz, 1H), 3.51-3.65 (m, 1H), 2.20 (s, 3H), 2.17 (s, 3H), 1.35 (d, J=7.0 Hz, 3H); LC/MS RT 1.65min, m/z [M-H]$^-$ 507,509 |

(continued)

| Example | Structural Formula | Physical Property Value |
|---|---|---|
| 5 | 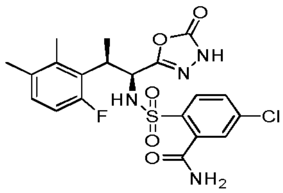 | 1H NMR (CD3OD) δ: 7.84 (d, J=8.4 Hz, 1H), 7.62 (d, J=2.2 Hz, 1H), 7.58 (dd, J=8.4, 2.2 Hz, 1H), 6.98 (dd, J=8.2, 5.7 Hz, 1H), 6.72 (dd, J=11.7, 8.4 Hz, 1H), 4.82 (d, J=11.4 Hz, 1H), 3.50-3.60 (m, 1H), 2.20 (s, 3H), 2.17 (s, 3H), 1.45 (d, J=7.0 Hz, 3H); LC/MS RT 1.65min, m/z $[M-H]^-$ 481,483 |
| 6 | 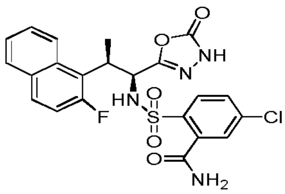 | 1H NMR (CD3OD) δ: 8.11 (d, J=8.8 Hz, 1H), 7.76-7.89 (m, 3H), 7.62 (d, J=2.2 Hz, 1H), 7.51-7.59 (m, 2H), 7.40-7.47 (m, 1H), 7.22 (dd, J=11.5, 9.0 Hz, 1H), 4.90-4.98 (m, 1H), 4.09-4.18 (m, 1H), 1.60 (d, J=7.0 Hz, 3H); LC/MS RT 1.66min, m/z $[M-H]^-$ 503,505 |
| 7 | 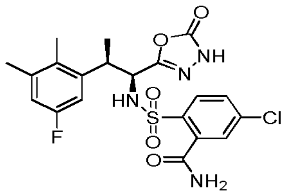 | 1H NMR (CD3OD) δ: 7.75 (d, J=8.4 Hz, 1H), 7.59 (d, J=2.2 Hz, 1H), 7.53 (dd, J=8.4, 2.2 Hz, 1H), 6.80 (dd, J=10.4, 2.7 Hz, 1H), 6.69 (dd, J=9.2, 2.7 Hz, 1H), 4.55 (d, J=8.8 Hz, 1H), 3.53-3.65 (m, 1H), 2.21 (s, 3H), 2.15 (s, 3H), 1.33 (d, J=7.0 Hz, 3H); LC/MS RT 1.65min, m/z $[M-H]^-$ 481,483 |
| 8 | 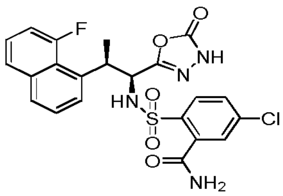 | 1H NMR (CD3OD) δ: 7.62-7.79 (m, 2H), 7.46-7.52 (m, 2H), 7.38-7.45 (m, 2H), 7.29-7.35 (m, 1H), 7.20-7.28 (m, 2H), 4.82-4.86 (m, 1H), 4.44-4.62 (m, 1H), 1.50 (d, J=7.0 Hz, 3H); LC/MS RT 1.64min, m/z $[M-H]^-$ 503,505 |
| 9 | 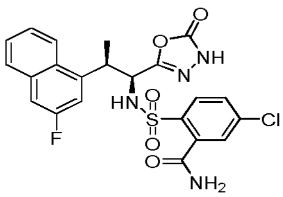 | 1H NMR (CD3OD) δ: 8.01-8.11 (m, 1H), 7.75-7.85 (m, 1H), 7.43-7.59 (m, 4H), 7.22-7.38 (m, 3H), 4.78 (d, J=7.3 Hz, 1H), 4.20 (t, J=7.0 Hz, 1H), 1.52 (d, J=7.0 Hz, 3H); LC/MS RT 1.67min, m/z $[M-H]^-$ 503,505 |
| 10 | 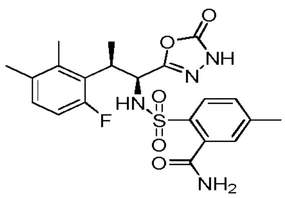 | 1H-NMR (CDCl3) δ: 7.85 (1H, d, J = 7.7 Hz), 7.67 (1H, s), 7.36-7.33 (1H, m), 6.92-6.88 (2H, m), 6.69 (1H, dd, J = 11.7, 8.4 Hz), 6.00 (1H, s), 5.87 (1H, s), 4.89 (1H, t, J = 10.1 Hz), 3.45 (1H, s), 2.42 (3H, s), 2.17-2.15 (6H, m), 1.44 (3H, d, J = 6.6 Hz); LC/MS RT 1.59min, m/z $[M-H]^-$ 461 |
| 11 | 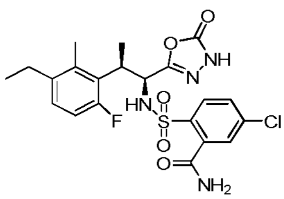 | 1H NMR (CD3OD) δ: 7.84 (d, J=8.4 Hz, 1H), 7.62 (d, J=2.2 Hz, 1H), 7.58 (dd, J=8.4, 2.2 Hz, 1H), 6.98 (dd, J=8.4, 5.9 Hz, 1H), 6.75 (dd, J=11.7, 8.8 Hz, 1H), 4.78 (d, J=11.0 Hz, 1H), 3.50-3.60 (m, 1H), 2.52-2.59 (m, 2H), 2.24 (s, 3H), 1.46 (d, J=7.0 Hz, 3H), 1.06 (t, J=7.5 Hz, 3H),; LC/MS RT 1.73min, m/z $[M-H]^-$ 495,497 |

(continued)

| Example | Structural Formula | Physical Property Value |
|---|---|---|
| 12 | 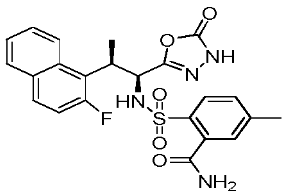 | 1H NMR (CD3OD) δ: 8.10 (br d, J=8.8 Hz, 1H), 7.76-7.84 (m, 2H), 7.72 (d, J=8.1 Hz, 1H), 7.54 (t, J=7.5 Hz, 1H), 7.39-7.45 (m, 2H), 7.33 (d, J=8.1 Hz, 1H), 7.21 (dd, J=11.4, 9.2 Hz, 1H), 4.91 (d, J=11.7 Hz, 1H), 4.07-4.21 (m, 1H), 2.39 (s, 3H), 1.60 (d, J=6.6 Hz, 3H); LC/MS RT 1.61min, m/z [M-H]- 483 |
| 13 | 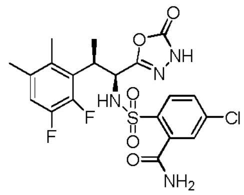 | 1H NMR (CD3OD) δ: 7.86 (d, J=8.4 Hz, 1H), 7.63 (d, J=1.8 Hz, 1H), 7.57-7.61 (m, 1H), 6.91 (dd, J=11.0, 8.4 Hz, 1H), 4.78 (d, J=11.0 Hz, 1H), 3.55-3.66 (m, 1H), 2.18 (s, 3H), 2.16 (s, 3H), 1.47 (d, J=7.0 Hz, 3H); LC/MS RT 1.68min, m/z [M-H]- 499,501 |
| 14 | 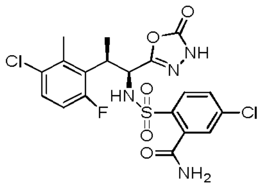 | 1H NMR (CD3OD) δ: 7.86 (d, J=8.4 Hz, 1H), 7.63 (d, J=1.8 Hz, 1H), 7.59 (dd, J=8.4, 2.2 Hz, 1H), 7.25 (dd, J=8.8, 5.1 Hz, 1H), 6.88 (t, J=10.0 Hz, 1H), 4.80 (d, J=11.4 Hz, 1H), 3.55-3.65 (m, 1H), 2.37 (s, 3H), 1.47 (d, J=7.0 Hz, 3H); LC/MS RT 1.68min, m/z [M-H]- 501,503 |
| 15 | 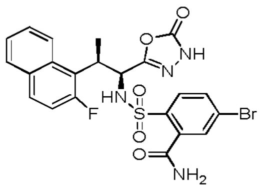 | 1H NMR (CD3OD) δ: 8.11 (d, J=8.8 Hz, 1H), 7.69-7.92 (m, 5H), 7.54 (br t, J=7.7 Hz, 1H), 7.42 (t, J=7.2 Hz, 1H), 7.21 (dd, J=11.4, 9.2 Hz, 1H), 4.89-5.01 (m, 1H), 4.10-4.24 (m, 1H), 1.60 (br d, J=6.6 Hz, 3H); LC/MS RT 1.68min, m/z [M-H]- 547,549 |
| 16 | 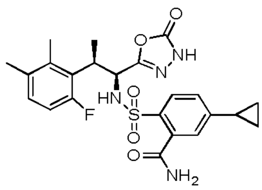 | 1H NMR (CD3OD) δ: 7.69 (d, J=8.1 Hz, 1H), 7.29 (d, J=1.8 Hz, 1H), 7.21 (dd, J=8.2, 2.0 Hz, 1H), 6.97 (dd, J=8.4, 5.9 Hz, 1H), 6.71 (dd, J=12.1, 8.4 Hz, 1H), 4.76 (d, J=11.4 Hz, 1H), 3.46-3.60 (m, 1H), 2.18 (s, 3H), 2.16 (s, 3H), 1.95-2.04 (m, 1H), 1.45 (d, J=7.0 Hz, 3H), 1.06 (dd, J=8.4, 1.8 Hz, 2H), 0.72-0.90 (m, 2H); LC/MS RT 1.67min, m/z [M-H]- 487 |
| 17 | 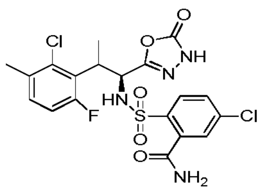 | 1H NMR (CD3OD) δ: 7.84 (d, J=8.4 Hz, 1H), 7.53-7.66 (m, 2H), 7.12-7.22 (m, 1H), 6.89-7.03 (m, 1H), 4.71-4.82 (m, 1H), 3.86-4.04 (m, 1H), 2.28 (s, 3H), 1.47 (br d, J=6.2 Hz, 3H); LC/MS RT 1.66min, m/z [M-H]- 501,503 |
| 18 | 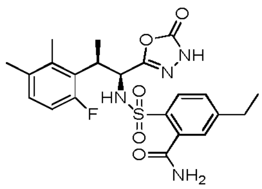 | 1H NMR (CD3OD) δ: 7.74 (d, J=8.4 Hz, 1H), 7.45 (d, J=1.8 Hz, 1H), 7.37 (dd, J=8.2, 1.6 Hz, 1H), 6.97 (dd, J=8.4, 5.9 Hz, 1H), 6.71 (dd, J=11.7, 8.4 Hz, 1H), 4.78 (d, J=11.0 Hz, 1H), 3.47-3.63 (m, 1H), 2.71 (q, J=7.7 Hz, 2H), 2.18 (s, 3H), 2.16 (s, 3H), 1.45 (d, J=7.0 Hz, 3H), 1.21-1.28 (m, 3H); LC/MS RT 1.66min, m/z [M-H]- 475 |
| 19 | 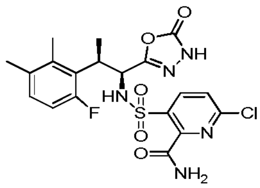 | 1H NMR (CD3OD) δ: 8.32 (d, J=8.4 Hz, 1H), 7.70 (d, J=8.4 Hz, 1H), 6.98 (dd, J=8.4, 5.9 Hz, 1H), 6.72 (dd, J=12.1, 8.4 Hz, 1H), 4.84 (d, J=11.4 Hz, 1H), 3.55-3.68 (m, 1H), 2.22 (s, 3H), 2.18 (s, 3H), 1.48 (d, J=7.0 Hz, 3H); LC/MS RT 1.65min, m/z [M-H]- 482,484 |

(continued)

| Example | Structural Formula | Physical Property Value |
|---|---|---|
| 20 | 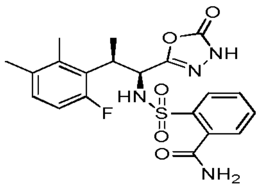 | 1H NMR (CD3OD) δ: 7.87 (d, J=7.6 Hz, 1H), 7.53-7.70 (m, 3H), 6.97 (dd, J=8.2, 5.7 Hz, 1H), 6.71 (dd, J=11.7, 8.4 Hz, 1H), 4.80 (d, J=11.0 Hz, 1H), 3.50-3.52 (m, 1H), 2.19 (s, 3H), 2.17 (s, 3H), 1.44 (d, J=6.6 Hz, 3H); LC/MS RT 1.54min, m/z [M-H]- 447 |
| 21 | 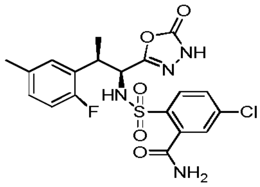 | 1H NMR (CD3OD) δ: 7.76 (d, J=8.4 Hz, 1H), 7.61 (d, J=2.2 Hz, 1H), 7.52-7.55 (m, 1H), 6.96-7.05 (m, 2H), 6.83 (s, 1H), 4.59 (d, J=9.5 Hz, 1H), 3.37-3.44 (m, 1H), 2.22 (s, 3H), 1.41 (d, J=7.0 Hz, 3H); LC/MS RT 1.60min, m/z [M-H]- 467,469 |
| 22 | 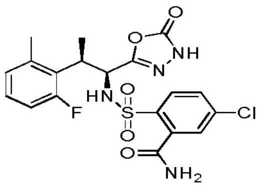 | LC/MS RT 1.6min, m/z [M-H]- 467,469 |
| 23 | 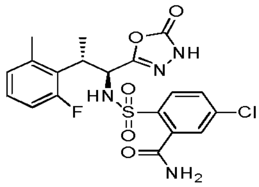 | 1H NMR (CD3OD) δ: 7.73 (d, J=8.4 Hz, 1H), 7.52-7.59 (m, 2H), 7.07 (td, J=7.9, 5.5 Hz, 1H), 6.90-6.97 (m, 1H), 6.66-6.77 (m, 1H), 4.77 (d, J=11.4 Hz, 1H), 3.43-3.59 (m, 1H), 2.38 (s, 3H), 1.46 (d, J=7.0 Hz, 3H); LC/MS RT 1.58min, m/z [M-H]- 467,469 |
| 24 | 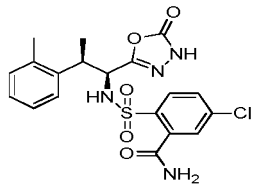 | 1H NMR (cdcl3) δ: 7.73 (d, J=8.4 Hz, 1H), 7.55 (d, J=1.8 Hz, 1H), 7.43 (d, J=8.4 Hz, 1H), 7.00-7.16 (m, 4H), 4.56 (dd, J=15.8, 7.7 Hz, 1H), 3.37-3.62 (m, 1H), 2.29 (s, 3H), 1.38 (d, J=7.0 Hz, 3H); LC/MS RT 1.56min, m/z [M-H]- 449,451 |
| 25 | 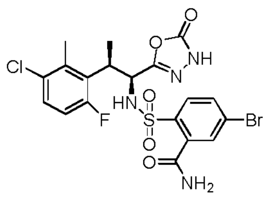 | 1H NMR (CD3OD) δ: 7.74-7.79 (m, 3H), 7.25 (dd, J=8.9, 5.0 Hz, 1H), 6.85-6.94 (m, 1H), 4.77-4.83 (m, 1H), 3.55-3.65 (m, 1H), 2.37 (s, 3H), 1.47 (d, J=7.0 Hz, 3H); LC/MS RT 1.70min, m/z [M-H]- 545,547 |
| 26 | 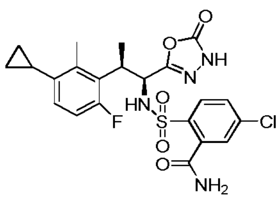 | 1H NMR (CD3OD) δ: 7.84 (d, J=8.4 Hz, 1H), 7.62 (d, J=2.2 Hz, 1H), 7.58 (dd, J=8.4, 2.2 Hz, 1H), 6.94 (dd, J=8.5, 5.9 Hz, 1H), 6.73 (dd, J=11.9, 8.5 Hz, 1H), 4.77 (d, J=11.4 Hz, 1H), 3.50-3.65 (m, 1H), 2.39 (s, 3H), 1.73-1.83 (m, 1H), 1.46 (d, J=7.0 Hz, 3H), 0.80-0.98 (m, 2H), 0.37-0.55 (m, 2H); LC/MS RT 1.76min, m/z [M-H]- 507,509 |

(continued)

| Example | Structural Formula | Physical Property Value |
|---|---|---|
| 27 | 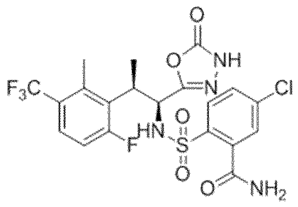 | 1H-NMR (CDCl3) δ: 8.26 (1H, br s), 7.94 (1H, d, J = 8.1 Hz), 7.55-7.47 (3H, m), 6.90 (2H, t, J = 9.7 Hz), 6.19-6.14 (1H, m), 4.90 (1H, t, J = 10.1 Hz), 3.56 (1H, br s), 2.39 (3H, s), 1.48 (3H, d, J = 7.0 Hz), 1.24 (1H, s).; LC/MS RT 1.73min, m/z $[M-H]^-$ 535,537 |
| 28 | 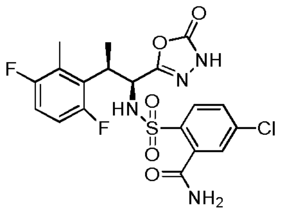 | 1H NMR (CD3OD) δ: 7.86 (d, J=8.4 Hz, 1H), 7.63 (d, J=2.2 Hz, 1H), 7.59 (dd, J=8.4, 2.2 Hz, 1H), 6.83-6.95 (m, 2H), 4.78 (d, J=11.0 Hz, 1H), 3.45-3.56 (m, 1H), 2.20 (d, J=2.2 Hz, 3H), 1.46 (d, J=7.0 Hz, 3H); LC/MS RT 1.60min, m/z $[M-H]^-$ 485,487 |
| 29 | 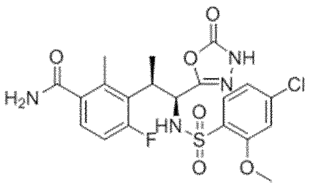 | 1H-NMR (CDCl3) δ: 8.88 (1H, s), 7.80 (1H, d, J = 8.4 Hz), 7.24-7.21 (1H, m), 7.03-7.01 (1H, m), 6.94-6.93 (1H, m), 6.84 (1H, dd, J = 11.4, 8.4 Hz), 6.02-5.95 (2H, m), 5.89 (1H, s), 4.80 (1H, t, J = 10.8 Hz), 3.93 (3H, s), 3.46 (1H, s), 2.35 (3H, s), 1.49 (3H, d, J = 5.9 Hz).; LC/MS RT 1.38min, m/z $[M-H]^-$ 497,499 |
| 30 | 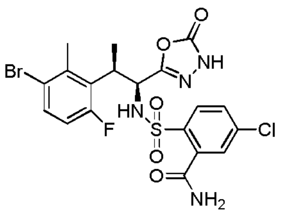 | 1H NMR (CD3OD) δ: 7.85 (d, J=8.4 Hz, 1H), 7.63 (d, J=2.2 Hz, 1H), 7.57-7.61 (m, 1H), 7.44 (dd, J=8.8, 5.1 Hz, 1H), 6.80-6.86 (m, 1H), 4.79 (d, J=11.0 Hz, 1H), 3.55-3.65 (m, 1H), 2.43 (s, 3H), 1.47 (d, J=7.0 Hz, 3H); LC/MS RT 1.70min, m/z $[M-H]^-$ 545,547 |
| 31 | 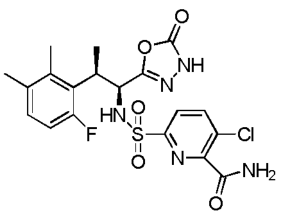 | 1H NMR (CD3OD) δ: 8.16 (d, J=8.4 Hz, 1H), 8.02 (d, J=8.1 Hz, 1H), 6.98 (dd, J=8.6, 5.3 Hz, 1H), 6.65-6.80 (m, 1H), 4.83-4.91 (m, 1H), 3.55-3.65 (m, 1H), 2.22 (s, 3H), 2.18 (s, 3H), 1.47 (d, J=7.3 Hz, 3H); LC/MS RT 1.52min, m/z $[M-H]^-$ 482,484 |
| 32 | 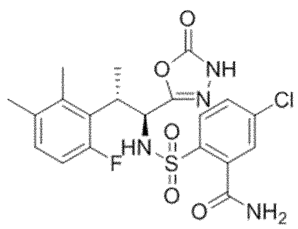 | 1H NMR (CD3OD) δ: 7.67 (1H, d, J = 8.8 Hz), 7.54-7.52 (2H, m), 6.95-6.92 (1H, m), 6.59-6.53 (1H, m), 4.79 (1H, d, J = 11.0 Hz), 3.56-3.54 (1H, m), 2.24 (3H, s), 2.19 (3H, s), 1.15 (3H, d, J = 7.0 Hz); LC/MS RT 1.66min, m/z $[M-H]^-$ 481,483 |
| 33 | 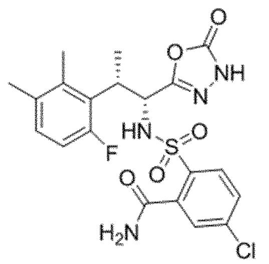 | 1H NMR (CD3OD) δ: 7.84 (d, J=8.4 Hz, 1H), 7.53-7.70 (m, 2H), 6.98 (dd, J=8.2, 5.7 Hz, 1H), 6.72 (dd, J=12.1, 8.4 Hz, 1H), 4.79 (d, J=11.4 Hz, 1H), 3.48-3.61 (m, 1H), 2.20 (s, 3H), 2.17 (s, 3H), 1.45 (d, J=7.0 Hz, 3H); LC/MS RT 1.66min, m/z $[M-H]^-$ 481,483 |

(continued)

| Example | Structural Formula | Physical Property Value |
|---|---|---|
| 34 | 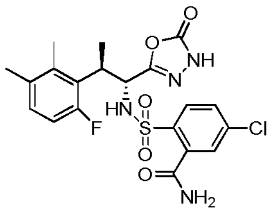 | 1H NMR (CD3OD) δ: 7.68 (dd, J=7.9, 0.9 Hz, 1H), 7.51-7.58 (m, 2H), 6.94 (dd, J=8.2, 6.0 Hz, 1H), 6.52-6.66 (m, 1H), 4.80 (d, J=11.0 Hz, 1H), 3.51-3.68 (m, 1H), 2.25 (s, 3H), 2.20 (s, 3H), 1.16 (d, J=6.6 Hz, 3H); LC/MS RT 1.66min, m/z [M-H]- 481,483 |
| 35 | 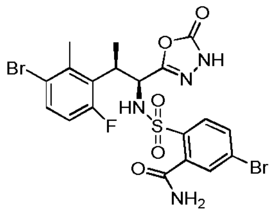 | 1H NMR (CD3OD) δ: 7.77 (s, 3H), 7.41-7.51 (m, 1H), 6.72-6.91 (m, 1H), 4.79 (d, J=11.0 Hz, 1H), 3.52-3.73 (m, 1H), 2.43 (s, 3H), 1.47 (d, J=7.0 Hz, 3H); LC/MS RT 1.73min, m/z [M-H]- 589,591 |
| 36 | 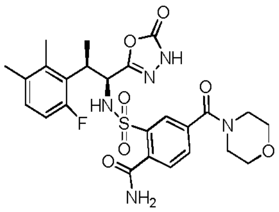 | 1H-NMR (CDCl3) δ: 9.73 (1H, br s), 7.87 (1H, s), 7.64-7.56 (2H, m), 7.06-7.04 (1H, m), 6.91 (1H, dd, J = 8.2, 5.7 Hz), 6.70-6.65 (2H, m), 6.47 (1H, s), 4.87 (1H, t, J = 10.6 Hz), 3.78-3.76 (4H, m), 3.64-3.62 (2H, m), 3.48-3.46 (1H, m), 3.41-3.39 (2H, m), 2.16-2.14 (6H, m), 1.46 (3H, d, J = 6.6 Hz).; LC/MS RT 1.50min, m/z [M-H]- 560 |
| 37 | 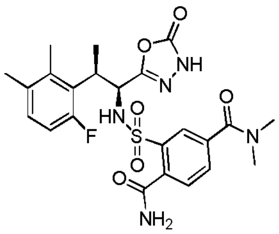 | 1H-NMR (CDCl3) δ: 7.86 (1H, s), 7.65-7.55 (2H, m), 7.06-7.03 (1H, m), 6.93-6.89 (1H, m), 6.71-6.66 (1H, m), 6.14 (1H, s), 6.03 (1H, s), 4.96 (1H, t, J = 10.3 Hz), 3.43-3.41 (1H, m), 3.11 (3H, s), 2.94 (3H, s), 2.15 (6H, s), 1.51 (3H, d, J = 7.0 Hz); LC/MS RT 1.51min, m/z [M-H]- 518 |
| 38 | 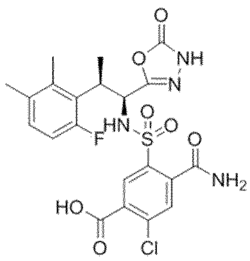 | 1H NMR (CD3OD) δ: 8.18 (1H, s), 7.67 (1H, s), 6.96 (1H, dd, J = 8.4, 5.9 Hz), 6.71 (1H, dd, J = 11.7, 8.4 Hz), 4.81 (1H, d, J = 11.0 Hz), 3.56 (1H, s), 3.33 (1H, s), 2.20 (3H, s), 2.16 (3H, s), 1.45 (3H, d, J = 6.6 Hz).;LC/MS RT 1.46min, m/z [M-H]- 525,527 |
| 39 | 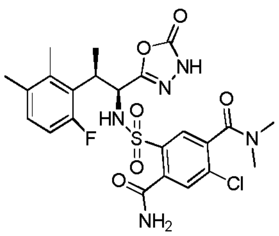 | 1H NMR (CD3OD) δ: 7.88 (1H, s), 7.72 (1H, s), 6.99-6.94 (1H, m), 6.70 (1H, dd, J = 11.7, 8.4 Hz), 4.77 (1H, d, J = 11.0 Hz), 3.58 (1H, s), 3.13 (3H, s), 2.90 (3H, s), 2.22 (3H, s), 2.17 (3H, s), 1.44-1.42 (3H, m); LC/MS RT 1.58min, m/z [M-H]- 552,554 |

(continued)

| Example | Structural Formula | Physical Property Value |
|---|---|---|
| 40 | 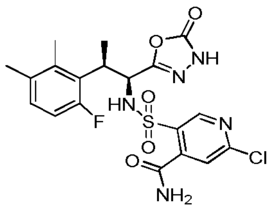 | 1H NMR (CD3OD) δ: 8.76 (s, 1H), 7.62 (s, 1H), 6.98 (dd, J=8.2, 6.0 Hz, 1H), 6.73 (dd, J=11.9, 8.6 Hz, 1H), 4.83-4.86 (m, 1H), 3.51-3.73 (m, 1H), 2.22 (s, 3H), 2.18 (s, 3H), 1.45 (d, J=6.6 Hz, 3H); LC/MS RT 1.61min, m/z $[M-H]^-$ 482,484 |
| 41 | 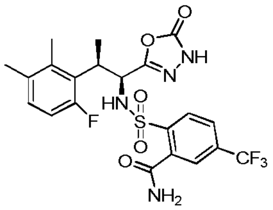 | 1H NMR (CD3OD) δ: 8.06 (d, J=9.2 Hz, 1H), 7.87-7.91 (m, 2H), 6.97 (dd, J=8.3, 5.7 Hz, 1H), 6.72 (dd, J=11.7, 8.3 Hz, 1H), 4.83 (d, J=11.0 Hz, 1H), 3.49-3.66 (m, 1H), 2.20 (s, 3H), 2.17 (s, 3H), 1.45 (d, J=7.0 Hz, 3H); LC/MS RT 1.72min, m/z $[M-H]^-$ 515 |
| 42 | 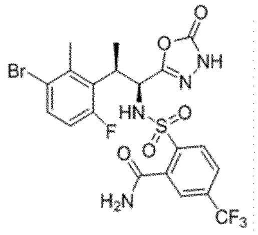 | 1H NMR (CD3OD) δ: 8.08 (d, J=8.1 Hz, 1H), 7.88-7.94 (m, 2H), 7.44 (dd, J=8.8, 5.1 Hz, 1H), 6.82 (dd, J=11.4, 8.8 Hz, 1H), 4.79-4.85 (m, 1H), 3.56-3.71 (m, 1H), 2.18 (s, 3H), 1.47 (d, J=7.0 Hz, 3H); LC/MS RT 1.78min, m/z $[M-H]^-$ 579,581 |
| 43 | 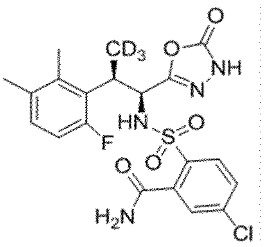 | 1H NMR (CD3OD) δ: 7.84 (d, J=8.4 Hz, 1H), 7.55-7.65 (m, 2H), 6.98 (dd, J=8.4, 5.9 Hz, 1H), 6.72 (dd, J=11.7, 8.4 Hz, 1H), 4.79 (d, J=11.0 Hz, 1H), 3.54 (br d, J=11.4 Hz, 1H), 2.20 (s, 3H), 2.17 (s, 3H); LC/MS RT 1.64min, m/z $[M-H]^-$ 484,486 |
| 44 | 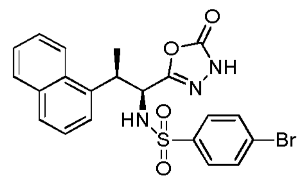 | 1H NMR (CD3OD) δ: 8.01 (d, J=8.4 Hz, 1H), 7.78-7.86 (m, 1H), 7.71 (dd, J=8.6, 2.0 Hz, 1H), 7.50 (s, 4H), 7.37-7.47 (m, 3H), 7.09-7.25 (m, 1H), 4.57 (d, J=9.2 Hz, 1H), 4.05-4.23 (m, 1H), 1.54 (d, J=6.6 Hz, 3H); LC/MS RT 1.82min, m/z $[M-H]^-$ 486,488 |
| 45 | 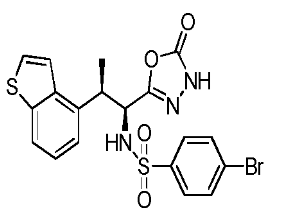 | 1H NMR (CD3OD) δ: 7.76-7.85 (m, 1H), 7.65-7.74 (m, 2H), 7.54 (s, 2H), 7.51 (d, J=5.5 Hz, 1H), 7.34 (d, J=5.5 Hz, 1H), 7.29 (t, J=7.7 Hz, 1H), 7.14-7.23 (m, 1H), 4.67 (d, J=9.5 Hz, 1H), 3.44-3.58 (m, 1H), 1.55 (d, J=7.0 Hz, 3H); LC/MS RT 1.78min, m/z $[M-H]^-$ 492,494 |
| 46 | 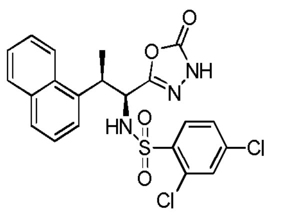 | LC/MS RT 1.89min, m/z $[M-H]^-$ 476,478 |

(continued)

| Example | Structural Formula | Physical Property Value |
|---|---|---|
| 47 | 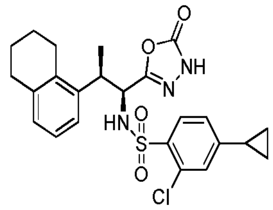 | 1H NMR (CD3OD) δ: 7.83 (d, J=8.4 Hz, 1H), 7.15 (d, J=1.8 Hz, 1H), 7.08 (dd, J=8.1, 1.8 Hz, 1H), 6.90-7.00 (m, 2H), 6.83 (d, J=7.0 Hz, 1H), 4.29 (d, J=10.6 Hz, 1H), 3.54-3.63 (m, 1H), 2.59-2.83 (m, 4H), 1.90-1.98 (m, 1H), 1.61-1.82 (m, 4H), 1.37 (d, J=7.0 Hz, 3H), 1.03-1.12 (m, 2H), 0.76-0.87 (m, 2H); LC/MS RT 1.97min, m/z [M-H]$^-$ 486 |
| 48 | 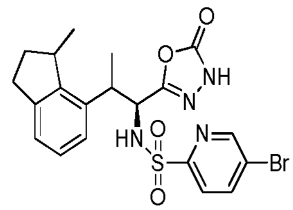 | 1H NMR (CD3OD) δ: 8.58-8.67 (m, 1H), 8.08-8.34 (m, 1H), 7.78-7.96 (m, 1H), 6.89-7.18 (m, 3H), 4.72 (d, J=10.3 Hz, 0.5H), 4.41 (d, J=11.0 Hz, 0.5H), 3.36-3.50 (m, 2H), 2.93-3.10 (m, 1H), 2.70 (dt, J=15.9, 8.2 Hz, 1H), 1.98-2.27 (m, 1H), 1.67-1.85 (m, 1H), 1.46 (d, J=6.6 Hz, 1.5H), 1.35 (d, J=6.6 Hz, 1.5H), 1.15 (d, J=7.0 Hz, 1.5H), 1.10 (d, J=7.0 Hz, 1.5H); LC/MS RT 1.79min, m/z [M-H]$^-$ 491,493 |
| 49 | 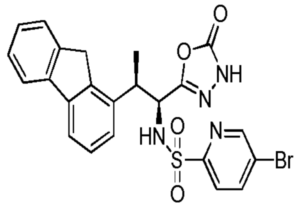 | 1H NMR (CD3OD) δ: 8.53 (dd, J=2.2, 0.7 Hz, 1H), 8.11 (dd, J=8.2, 2.4 Hz, 1H), 7.76 (d, J=7.3 Hz, 1H), 7.64 (d, J=7.3 Hz, 1H), 7.55 (d, J=7.3 Hz, 1H), 7.26-7.41 (m, 4H), 7.16 (d, J=7.7 Hz, 1H), 4.68 (d, J=9.9 Hz, 1H), 3.87 (s, 2H), 3.49-3.61 (m, 1H), 1.50 (d, J=7.0 Hz, 3H); LC/MS RT 1.83min, m/z [M-H]$^-$ 525,527 |
| 50 | 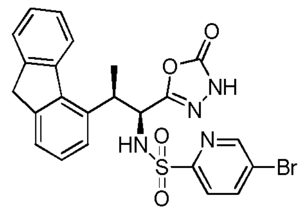 | 1H NMR (CD3OD) δ: 8.44 (d, J=2.2 Hz, 1H), 8.02-8.09 (m, 2H), 7.68 (d, J=8.4 Hz, 1H), 7.57 (d, J=7.3 Hz, 1H), 7.09-7.46 (m, 5H), 4.83-4.85 (m, 1H), 4.30-4.442 (m, 1H), 3.65 (s, 2H), 1.51 (d, J=7.0 Hz, 3H); LC/MS RT 1.82min, m/z [M-H]$^-$ 525,527 |
| 51 | 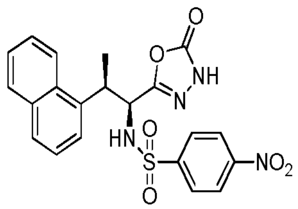 | 1H NMR (CD3OD) δ: 8.07-8.19 (m, 2H), 8.00 (d, J=8.8 Hz, 1H), 7.76 (d, J=8.4 Hz, 3H), 7.63-7.70 (m, 1H), 7.47-7.54 (m, 1H), 7.34-7.45 (m, 3H), 4.63 (d, J=8.1 Hz, 1H), 4.06-4.21 (m, 1H), 1.54 (d, J=7.0 Hz, 3H); LC/MS RT 1.73min, m/z [M-H]$^-$ 453 |
| 52 | 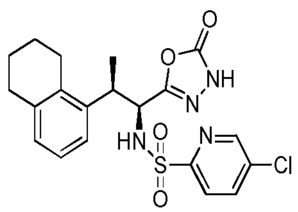 | 1H NMR (CD3OD) δ: 8.52 (d, J=2.6 Hz, 1H), 8.01 (dd, J=8.4, 2.6 Hz, 1H), 7.90 (d, J=8.4 Hz, 1H), 6.97 (d, J=4.8 Hz, 2H), 6.83-6.91 (m, 1H), 4.50 (d, J=10.3 Hz, 1H), 3.51-3.61 (m, 1H), 2.55-2.96 (m, 4H), 1.54-1.91 (m, 4H), 1.36 (d, J=6.6 Hz, 3H); LC/MS RT 1.77min, m/z [M-H]$^-$ 447,449 |
| 53 | 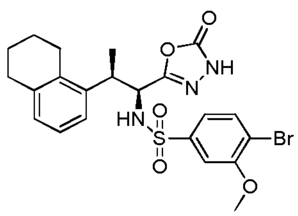 | 1H NMR (CD3OD) δ: 7.94 (d, J=8.4 Hz, 1H), 7.91 (d, J=2.2 Hz, 1H), 7.72 (dd, J=8.4, 2.2 Hz, 1H), 6.99-7.02 (m, 1H), 6.91-6.97 (m, 1H), 6.84 (d, J=7.3 Hz, 1H), 4.54 (d, J=9.5 Hz, 1H), 3.58-3.69 (m, 4H), 2.63-2.85 (m, 4H), 1.64-1.88 (m, 4H), 1.32 (d, J=6.6 Hz, 3H); LC/MS RT 1.92min, m/z [M-H]$^-$ 520,522 |

(continued)

| Example | Structural Formula | Physical Property Value |
|---|---|---|
| 54 | 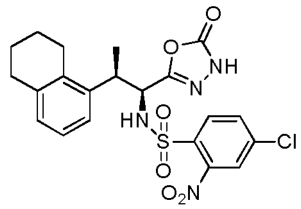 | LC/MS RT 1.94min, m/z [M-H]- 491,493 |
| 55 | 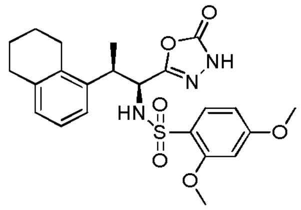 | 1H NMR (CD3OD) δ: 7.66 (d, J=8.7 Hz, 1H), 6.93-7.00 (m, 2H), 6.83 (d, J=7.0 Hz, 1H), 6.51-6.56 (m, 2H), 4.25 (d, J=11.0 Hz, 1H), 3.91 (s, 3H), 3.82 (s, 3H), 3.47-3.61 (m, 1H), 2.60-2.79 (m, 4H), 1.59-1.85 (m, 4H), 1.40 (d, J=7.0 Hz, 3H); LC/MS RT 1.77min, m/z [M-H]- 472 |
| 56 | 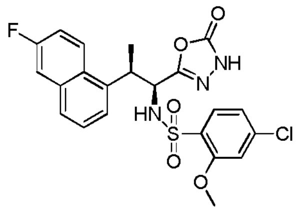 | 1H NMR (CD3OD) δ: 8.13 (dd, J=9.5, 5.5 Hz, 1H), 7.67 (d, J=8.4 Hz, 2H), 7.37-7.53 (m, 3H), 7.30 (td, J=8.8, 2.6 Hz, 1H), 7.08 (d, J=1.8 Hz, 1H), 6.97 (dd, J=8.4, 1.8 Hz, 1H), 4.48 (d, J=10.3 Hz, 1H), 4.11-4.21 (m, 1H), 3.94 (s, 3H), 1.60 (d, J=7.0 Hz, 3H); LC/MS RT 1.80min, m/z [M-H]- 490,492 |
| 57 | 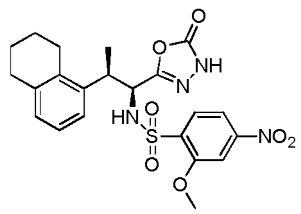 | 1H NMR (CD3OD) δ: 8.00 (d, J=8.4 Hz, 1H), 7.83-7.91 (m, 2H), 6.91-7.02 (m, 2H), 6.78-6.86 (m, 1H), 4.36 (d, J=10.6 Hz, 1H), 4.05 (s, 3H), 3.53-3.63 (m, 1H), 2.59-2.87 (m, 4H), 1.62-1.87 (m, 4H), 1.40 (d, J=7.0 Hz, 3H); LC/MS RT 1.83min, m/z [M-H]- 487,489 |
| 58 | 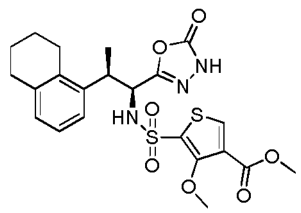 | 1H NMR (CD3OD) δ: 8.28-8.34 (m, 1H), 6.92-7.00 (m, 2H), 6.80-6.89 (m, 1H), 4.43 (d, J=10.6 Hz, 1H), 3.95 (s, 3H), 3.84 (s, 3H), 3.47-3.60 (m, 1H), 2.60-2.85 (m, 4H), 1.60-1.87 (m, 4H), 1.33 (d, J=6.6 Hz, 3H); LC/MS RT 1.80min, m/z [M-H]- 506 |
| 59 | 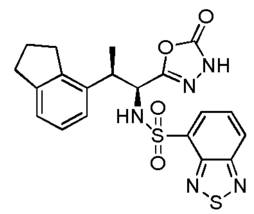 | 1H NMR (CD3OD) δ: 8.12-8.30 (m, 2H), 7.74 (dd, J=8.8, 7.3 Hz, 1H), 6.72-7.02 (m, 3H), 4.52 (d, J=10.3 Hz, 1H), 3.30-3.40 (m, 1H), 2.68-2.92 (m, 4H), 1.88-2.01 (m, 2H), 1.38 (d, J=7.0 Hz, 3H); LC/MS RT 1.69min, m/z [M-H]- 456 |
| 60 | 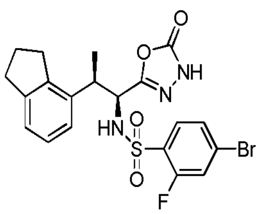 | 1H NMR (cdcl3) δ: 7.60 (t, J=8.1 Hz, 1H), 7.31-7.37 (m, 1H), 7.20-7.26 (m, 1H), 7.06-7.10 (m, 1H), 7.01-7.06 (m, 1H), 6.88 (d, J=7.3 Hz, 1H), 5.46 (br s, 1H), 4.52 (br t, J=7.9 Hz, 1H), 3.29-3.41 (m, 1H), 2.70-2.90 (m, 4H), 1.95-2.07 (m, 2H), 1.38 (d, J=7.0 Hz, 3H); LC/MS RT 1.87min, m/z [M-H]- 494,496 |

(continued)

| Example | Structural Formula | Physical Property Value |
|---|---|---|
| 61 | 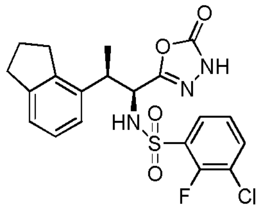 | 1H NMR (cdcl3) δ: 7.62-7.69 (m, 1H), 7.52-7.59 (m, 1H), 7.15 (t, J=8.1 Hz, 1H), 7.03 (s, 2H), 6.86-6.92 (m, 1H), 5.46 (br s, 1H), 4.54 (brt, J=8.1 Hz, 1H), 3.30-3.46 (m, 1H), 2.72-2.91 (m, 4H), 1.96-2.09 (m, 2H), 1.37-1.42 (m, 1H), 1.40 (d, J=7.0 Hz, 2H); LC/MS RT 1.82min, m/z $[M-H]^-$ 450,452 |
| 62 | 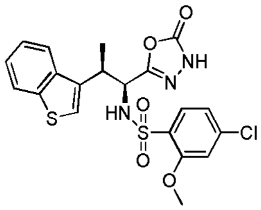 | 1H NMR (CD3OD) δ: 7.79-7.87 (m, 1H), 7.73 (d, J=7.7 Hz, 1H), 7.65 (d, J=8.4 Hz, 1H), 7.40 (s, 1H), 7.27-7.36 (m, 2H), 7.07 (d, J=1.8 Hz, 1H), 6.95 (dd, J=8.4, 2.2 Hz, 1H), 4.46 (d, J=9.9 Hz, 1H), 3.93 (s, 3H), 3.70-3.78 (m, 1H), 1.59 (d, J=7.0 Hz, 3H); LC/MS RT 1.77min, m/z $[M-H]^-$ 478,480 |
| 63 | 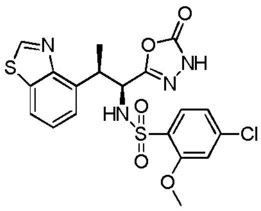 | 1H NMR (CD3OD) δ: 9.16 (1H, s), 7.91-7.88 (1H, m), 7.66 (1H, d, J = 8.4 Hz), 7.39-7.31 (2H, m), 7.07-7.07 (1H, m), 6.99-6.97 (1H, m), 3.95 (3H, s), 3.49-3.48 (1H, m), 3.15-3.13 (1H, m), 1.66 (3H, d, J = 7.0 Hz); LC/MS RT 1.62min, m/z $[M-H]^-$ 479,481 |
| 64 | 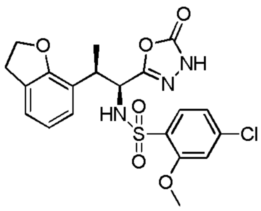 | 1H NMR (CD3OD) δ: 7.70 (d, J=8.4 Hz, 1H), 7.24 (d, J=1.8 Hz, 1H), 7.08-7.11 (m, 1H), 6.99-7.04 (m, 1H), 6.80 (d, J=7.0 Hz, 1H), 6.61-6.73 (m, 1H), 4.55 (d, J=10.6 Hz, 1H), 4.44-4.51 (m, 2H), 3.94 (s, 3H), 3.19-3.28 (m, 1H), 3.09 (t, J=8.6 Hz, 2H), 1.45 (d, J=7.0 Hz, 3H); LC/MS RT 1.68min, m/z $[M-H]^-$ 464,466 |
| 65 | 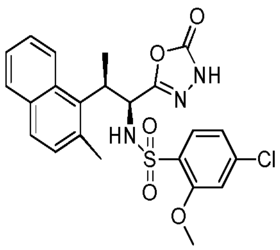 | 1H NMR (CD3OD) δ: 8.07-8.21 (m, 1H), 7.74 (d, J=8.4 Hz, 2H), 7.58 (d, J=8.4 Hz, 1H), 7.44-7.53 (m, 1H), 7.31-7.42 (m, 1H), 7.20 (d, J=8.4 Hz, 1H), 7.10 (d, J=1.8 Hz, 1H), 6.96-7.06 (m, 1H), 5.16 (d, J=11.7 Hz, 1H), 4.07-4.15 (m, 1H), 3.95 (s, 3H), 2.49 (s, 3H), 1.77 (d, J=7.3 Hz, 2.3H), 1.66 (d, J=7.3 Hz, 0.7H); LC/MS RT 1.82min, m/z $[M-H]^-$ 486,488 |
| 66 | 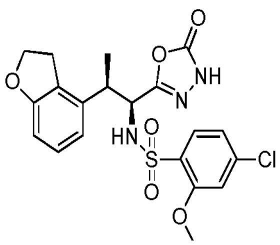 | 1H NMR (CD3OD) δ: 7.67-7.77 (m, 1H), 7.10-7.15 (m, 1H), 7.01-7.05 (m, 1H), 6.97-7.01 (m, 1H), 6.63 (d, J=7.7 Hz, 1H), 6.52 (d, J=7.7 Hz, 1H), 4.36-4.56 (m, 2H), 4.30 (d, J=11.4 Hz, 1H), 3.94 (s, 3H), 3.21-3.29 (m, 1H), 3.13 (t, J=8.6 Hz, 2H), 1.46 (d, J=7.0 Hz, 3H); LC/MS RT 1.62min, m/z $[M-H]^-$ 464,466 |

(continued)

| Example | Structural Formula | Physical Property Value |
|---|---|---|
| 67 | 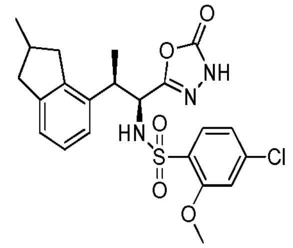 | 1H NMR (CD3OD) δ: 7.71 (dd, J=8.4, 0.7 Hz, 1H), 7.10 (d, J=1.8 Hz, 1H), 7.02 (dd, J=8.2, 1.6 Hz, 2H), 6.91-6.99 (m, 2H), 4.29 (dd, J=11.2, 1.6 Hz, 1H), 3.94 (s, 3H), 3.30-3.36 (m, 1H), 2.91-3.16 (m, 2H), 2.28-2.55 (m, 3H), 1.44 (d, J=7.0 Hz, 3H), 1.11 (d, J=6.4 Hz, 1.5H), 1.0 (d, J=6.4 Hz, 1.5H); LC/MS RT 1.89min, m/z [M-H]$^-$ 476,478 |
| 68 | 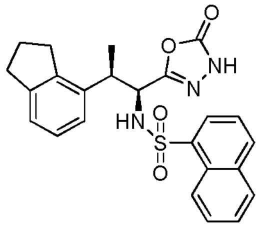 | 1H NMR (CD3OD) δ: 8.62 (d, J=8.8 Hz, 1H), 8.19 (dd, J=7.3, 1.1 Hz, 1H), 8.09 (d, J=8.4 Hz, 1H), 7.95 (d, J=8.4 Hz, 1H), 7.64-7.71 (m, 1H), 7.56-7.62 (m, 1H), 7.48-7.56 (m, 1H), 6.92 (d, J=4.8 Hz, 2H), 6.83 (d, J=4.4 Hz, 1H), 4.20 (d, J=10.6 Hz, 1H), 3.20-3.30 (m, 1H), 2.68-2.83 (m, 4H), 1.83-1.89 (m, 2H), 1.33 (d, J=7.0 Hz, 3H); LC/MS RT 1.82min, m/z [M-H]$^-$ 448 |
| 69 | 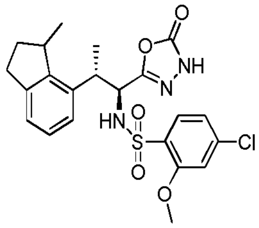 | LC/MS RT 1.86min, m/z [M-H]$^-$ 476,478 |
| 70 | 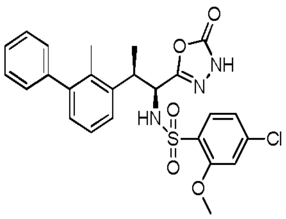 | 1H NMR (CD3OD) δ: 7.75 (d, J=8.4 Hz, 1H), 7.26-7.43 (m, 3H), 7.09-7.21 (m, 5H), 7.04 (dd, J=8.4, 1.8 Hz, 1H), 6.96 (dd, J=7.1, 1.6 Hz, 1H), 4.37 (d, J=10.6 Hz, 1H), 3.95 (s, 3H), 3.61-3.68 (m, 1H), 2.13 (s, 3H), 1.49 (d, J=7.0 Hz, 3H); LC/MS RT 1.93min, m/z [M-H]$^-$ 512,514 |
| 71 | 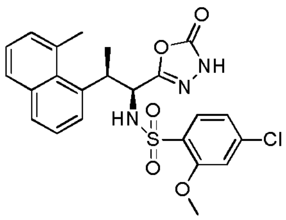 | 1H NMR (CD3OD) δ: 7.72 (d, J=8.4 Hz, 1H), 7.64 (d, J=7.3 Hz, 1H), 7.59-7.62 (m, 1H), 7.46 (d, J=6.2 Hz, 1H), 7.31-7.37 (m, 1H), 7.21-7.29 (m, 2H), 7.06 (d, J=1.8 Hz, 1H), 7.00 (dd, J=8.4, 1.8 Hz, 1H), 4.61-4.74 (m, 1H), 4.37 (d, J=10.6 Hz, 1H), 3.90 (s, 3H), 2.96 (s, 3H), 1.67 (d, J=7.0 Hz, 3H); LC/MS RT 1.81min, m/z [M-H]$^-$ 486,488 |
| 72 | 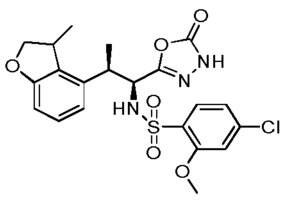 | 1H NMR (CD3OD) δ: 7.72 (d, J=8.4 Hz, 1H), 7.11 (d, J=1.8 Hz, 1H), 7.01-7.09 (m, 2H), 6.71 (d, J=7.7 Hz, 1H), 6.52 (d, J=8.1 Hz, 1H), 4.28-4.33 (m, 1H), 4.23 (d, J=11.0 Hz, 1H), 4.07-4.15 (m, 1H), 3.94 (s, 3H), 3.41-3.50 (m, 1H), 3.33-3.39 (m, 1H), 1.51 (d, J=7.0 Hz, 3H), 1.21 (d, J=7.0 Hz, 3H); LC/MS RT 1.69min, m/z [M-H]$^-$ 478,480 |
| 73 | 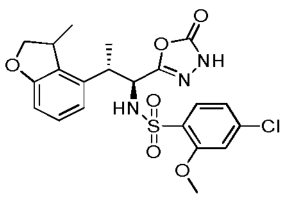 | 1H NMR (CD3OD) δ: 7.76 (d, J=8.4 Hz, 1H), 7.10-7.14 (m, 1H), 6.97-7.09 (m, 2H), 6.62-6.67 (m, 1H), 6.51-6.62 (m, 1H), 4.50-4.57 (m, 1H), 4.38-4.48 (m, 1H), 4.11-4.16 (m, 1H), 3.94 (s, 3H), 3.33-3.46 (m, 2H), 1.41 (d, J=7.0 Hz, 3H), 1.20-1.24 (m, 3H); LC/MS RT 1.66min, m/z [M-H]$^-$ 478,480 |

(continued)

| Example | Structural Formula | Physical Property Value |
|---|---|---|
| 74 | 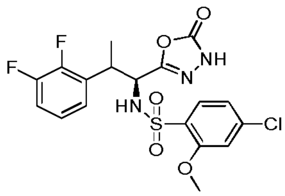 | 1H NMR (CD3OD) δ: 7.71 (d, J=8.4 Hz, 1H), 6.98-7.17 (m, 5H), 4.54 (d, J=11.0 Hz, 0.34H), (d, J=11.0 Hz, 0.68H), 3.95 (s, 1H), 3.85 (s, 2H), 3.48-3.59 (m, 1H), 1.51 (d, J=7.0 Hz, 2H), 1.17 (d, J=7.0 Hz, 1H), ; LC/MS RT 1.67min, m/z $[M-H]^-$ 458,460 |
| 75 | 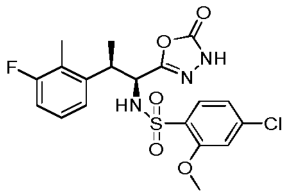 | 1H NMR (CD3OD) δ: 7.72 (d, J=8.4 Hz, 1H), 7.08-7014 (m, 2H), 6.95-7.06 (m, 1H), 6.95-7.06 (m, 1H), 6.84 (t, J=9.0 Hz, 1H), 4.34 (d, J=11.0 Hz, 1H), 3.94 (s, 3H), 3.52-3.63 (m, 1H), 2.17 (d, J=2.2 Hz, 3H), 1.44 (d, J=7.0 Hz, 3H); LC/MS RT 1.70min, m/z $[M-H]^-$ 454,456 |
| 76 | 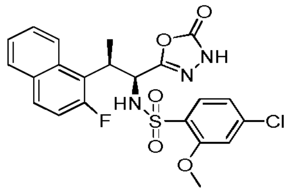 | LC/MS RT 1.78min, m/z $[M-H]^-$ 490,492 |
| 77 | 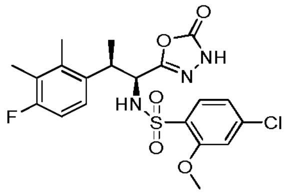 | 1H NMR (CD3OD) δ: 7.71 (d, J=8.4 Hz, 1H), 7.11 (d, J=1.8 Hz, 1H), 6.94-7.06 (m, 2H), 6.73-6.86 (m, 1H), 4.30 (d, J=10.6 Hz, 1H), 3.94 (s, 3H), 3.54-3.64 (m, 1H), 2.20 (s, 3H), 2.10 (s, 3H), 1.41 (d, J=7.0 Hz, 3H); LC/MS RT 1.78min, m/z $[M-H]^-$ 468,470 |
| 78 | 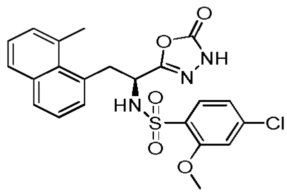 | 1H NMR (CD3OD) δ: 7.68 (d, J=7.3 Hz, 2H), 7.51 (d, J=8.4 Hz, 1H), 7.28-7.37 (m, 2H), 7.20 (t, J=7.6 Hz, 1H), 7.09-7.15 (m, 1H), 7.02 (d, J=1.8 Hz, 1H), 6.88 (dd, J=8.4, 1.8 Hz, 1H), 4.39 (dd, J=9.0, 6.4 Hz, 1H), 3.89-3.99 (m, 1H), 3.88 (s, 3H), 3.62-3.69 (m, 1H), 2.89 (s, 3H); LC/MS RT 1.80min, m/z $[M-H]^-$ 472,474 |
| 79 | 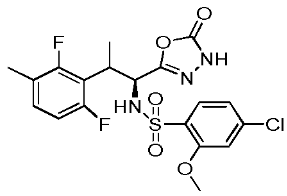 | 1H NMR (CD3OD) δ: 7.73 (d, J=8.4 Hz, 0.5H), 7.66 (d, J=8.4 Hz, 0.5H), 6.98-7.17 (m, 3H), 6.75 (t, J=9.3 Hz, 1H), 4.66 (d, J=11.0 Hz, 0.5H), 4.57 (d, J=11.4 Hz, 0.5H), 3.95 (s, 1.5H), 3.82 (s, 1.5H), 3.61-3.78 (m, 1H), 2.15 (s, 3H), 1.52 (d, J=7.0 Hz, 1.5H), 1.15 (d, J=7.0 Hz, 1.5H); LC/MS RT 1.76min, m/z $[M-H]^-$ 472,474 |
| 80 | 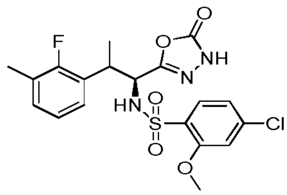 | 1H NMR (CD3OD) δ: 7.61-7.80 (m, 1H), 6.76-7.19 (m, 5H), 4.55 (d, J=11.0 Hz, 0.33H), 4.40 (d, J=11.0 Hz, 0.67H), 3.94 (s, 2H), 3.80 (s, 1H), 3.39-3.49 (m, 1H), 2.15-2.20 (m, 3H), 1.48 (d, J=7.0 Hz, 2H), 1.14 (d, J=7.0 Hz, 1H); LC/MS RT 1.73, 1.76min, m/z $[M-H]^-$ 454,456 |

(continued)

| Example | Structural Formula | Physical Property Value |
|---|---|---|
| 81 | 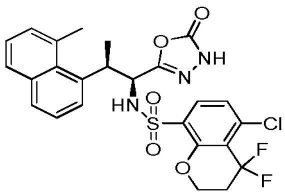 | 1H NMR (CD3OD) δ: 7.80 (d, J=8.4 Hz, 1H), 7.64 (d, J=8.5 Hz, 1H), 7.61 (d, J=7.4 Hz, 1H), 7.43-7.50 (m, 1H), 7.34 (t, J=7.3 Hz, 1H), 7.22-7.29 (m, 2H), 7.12 (d, J=8.4 Hz, 1H), 4.64-4.76 (m, 1H), 4.36-4.49 (m, 3H), 2.96 (s, 3H), 2.47-2.58 (m, 2H), 1.68 (d, J=7.0 Hz, 3H); LC/MS RT 1.88min, m/z [M-H]$^-$ 548,550 |
| 82 | 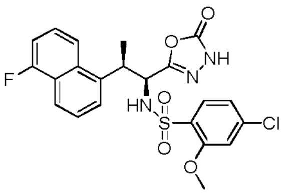 | 1H NMR (DMSO-d6) δ: 11.63 (br s, 1H), 7.31 (br dd, J=8.1 Hz, 1H), 7.88 (d, J=8.1 Hz, 1H), 7.80 (d, J=8.8 Hz, 1H), 7.46-7.62 (m, J=8.4 Hz, 4H), 7.30 (dd, J=10.4, 7.9 Hz, 1H), 7.10-7.26 (m, 2H), 7.01 (dd, J=8.6, 1.6 Hz, 1H), 4.33-4.50 (m, 1H), 4.06-4.22 (m, 1H), 3.86 (s, 3H), 1.45 (d, J=6.6 Hz, 3H); LC/MS RT 1.82min, m/z [M-H]$^-$ 490,492 |
| 83 | 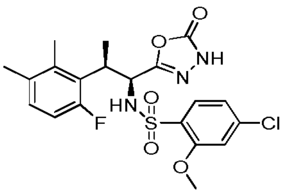 | 1H NMR (CD3OD) δ: 7.74 (d, J=8.4 Hz, 1H), 7.11 (d, J=1.8 Hz, 1H), 7.03-7.07 (m, 1H), 6.96 (dd, J=8.2, 5.7 Hz, 1H), 6.69 (dd, J=11.7, 8.4 Hz, 1H), 4.68 (br d, J=11.0 Hz, 1H), 3.95 (s, 3H), 3.61-3.68 (m, 1H), 2.21 (s, 3H), 2.17 (s, 3H), 1.47 (d, J=6.6 Hz, 3H); LC/MS RT 1.77min, m/z [M-H]$^-$ 468,470 |
| 84 | 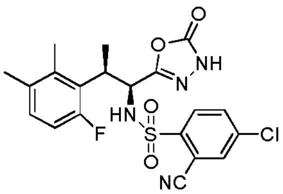 | 1H NMR (CD3OD) δ: 7.87 (d, J=8.4 Hz, 1H), 7.62 (dd, J=8.4, 1.8 Hz, 1H), 7.54 (d, J=2.2 Hz, 1H), 6.98 (dd, J=8.4, 5.9 Hz, 1H), 6.72 (dd, J=11.7, 8.4 Hz, 1H), 4.80 (d, J=11.4 Hz, 1H), 3.52-3.63 (m, 1H), 2.22 (s, 3H), 2.17 (s, 3H), 1.45 (d, J=7.0 Hz, 3H); LC/MS RT 1.77min, m/z [M-H]$^-$ 463,465 |
| 85 | 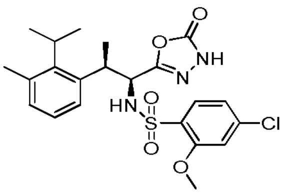 | 1H NMR (CD3OD) δ: 7.73 (d, J=8.1 Hz, 1H), 7.08-7.16 (m, 1H), 6.89-7.07 (m, 4H), 4.40 (d, J-10.3 Hz, 1H), 3.95 (s, 3H), 3.71-3.83 (m, 1H), 3.45-3.58 (m, 1H), 2.33 (s, 3H), 1.43 (d, J=6.6 Hz, 3H), 1.24 (d, J=7.3 Hz, 6H); LC/MS RT 1.88min, m/z [M-H]$^-$ 478,480 |
| 86 | 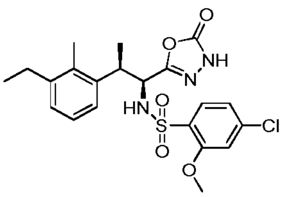 | 1H NMR (CD3OD) δ: 7.73 (d, J=8.4 Hz, 1H), 7.11 (d, J=1.8 Hz, 1H), 6.92-7.05 (m, 4H), 4.35 (d, J=11.0 Hz, 1H), 3.95 (s, 3H), 3.52-3.62 (m, 1H), 2.55-2.78 (m, 2H), 2.24 (s, 3H), 1.44 (d, J=6.6 Hz, 3H), 1.08 (t, J=7.3 Hz, 3H); LC/MS RT 1.82min, m/z [M-H]$^-$ 464,466 |
| 87 | 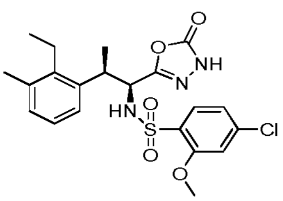 | 1H NMR (CD3OD) δ: 7.72 (d, J=8.4 Hz, 1H), 7.10 (d, J=1.8 Hz, 1H), 6.98-7.06 (m, 3H), 6.94 (d, J=2.2 Hz, 1H), 4.29 (d, J=10.6 Hz, 1H), 3.94 (s, 3H), 3.58-3.63 (m, 1H), 2.51-2.66 (m, 2H), 2.21 (s, 3H), 1.43 (d, J=6.6 Hz, 3H), 1.08 (t, J=7.5 Hz, 3H); LC/MS RT 1.84min, m/z [M-H]$^-$ 464,466 |

(continued)

| Example | Structural Formula | Physical Property Value |
|---|---|---|
| 88 | 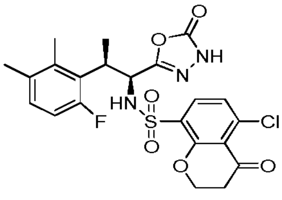 | 1H NMR (CD3OD) δ: 7.88 (d, J=8.4 Hz, 1H), 7.14 (d, J=8.4 Hz, 1H), 6.87-7.06 (m, 1H), 6.67-6.77 (m, 1H), 4.70-4.78 (m, 3H), 3.63-3.71 (m, 1H), 2.84-2.95 (m, 2H), 2.22 (s, 3H), 2.17 (s, 3H), 1.51 (d, J=7.0 Hz, 3H); LC/MS RT 1.71min, m/z [M-H]- 508,510 |
| 89 | 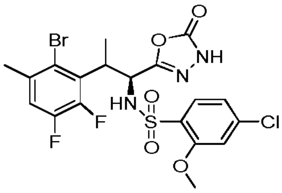 | 1H NMR (CD3OD) δ: 7.74 (d, J=8.4 Hz, 1H), 7.10-7.27 (m, 2H), 7.04 (dd, J=8.4, 1.8 Hz, 1H), 4.64-4.75 (m, 1H), 4.06-4.22 (m, 1H), 3.96 (s, 3H), 2.32 (s, 3H), 1.51 (d, J=7.0 Hz, 3H); LC/MS RT 1.84min, m/z [M-H]- 550,552 |
| 90 | 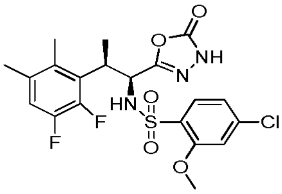 | 1H NMR (CD3OD) δ: 7.75 (d, J=8.4 Hz, 1H), 7.12 (d, J=2.2 Hz, 1H), 7.05 (dd, J=8.4, 1.8 Hz, 1H), 6.89 (dd, J=9.8 Hz, 1H), 4.66 (d, J=11.4 Hz, 1H), 3.95 (s, 3H), 3.63-3.72 (m, 1H), 2.18 (s, 3H), 2.17 (s, 3H), 1.50 (d, J=6.6 Hz, 3H); LC/MS RT 1.80min, m/z [M-H]- 486,488 |
| 91 | 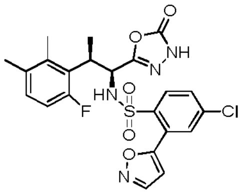 | 1H NMR (CD3OD) δ: 8.51 (d, J=1.8 Hz, 1H), 8.09-8.21 (m, 1H), 7.768-7.73 (m, 2H), 6.92-7.01 (m, 2H), 6.63-6.78 (m, 1H), 4.61-4.71 (m, 1H), 3.53-3.75 (m, 1H), 2.19 (s, 3H), 2.17 (s, 3H), 1.38 (d, J=7.0 Hz, 3H); LC/MS RT 1.82min, m/z [M-H]- 505,507 |
| 92 | 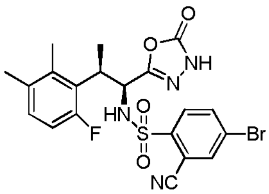 | LC/MS RT 1.79min, m/z [M-H]- 507,509 |
| 93 | 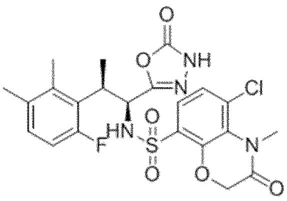 | 1H NMR (CD3OD) δ: 7.45 (1H, d, J = 8.4 Hz), 7.21 (1H, d, J = 8.4 Hz), 6.96 (1H, dd, J = 8.6, 5.9 Hz), 6.70 (1H, dd, J = 12.1, 8.6 Hz), 4.66-4.80 (4H, m), 3.46 (3H, s), 2.19 (3H, s), 2.16 (3H, s), 1.49 (3H, d, J = 6.6 Hz); LC/MS RT 1.71min, m/z [M-H]- 523,525 |
| 94 | 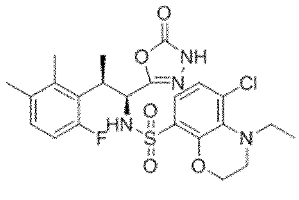 | 1H-NMR (CDCl3) δ: 7.77 (1H, s), 7.42 (1H, d, J = 8.4 Hz), 7.00 (1H, d, J = 8.4 Hz), 6.93 (1H, dd, J = 8.3, 5.9 Hz), 6.69 (1H, dd, J = 11.5, 8.3 Hz), 5.43 (1H, d, J = 10.6 Hz), 4.86 (1H, t, J = 10.6 Hz), 4.34-4.29 (1H, m), 4.23-4.19 (1H, m), 3.41 (1H, br s), 3.10-3.07 (2H, m), 2.98-2.83 (2H, m), 2.18-2.17 (6H, m), 1.56-1.53 (3H, m), 1.27-1.23 (3H, m).; LC/MS RT 1.85min, m/z [M-H]- 523,525 |

(continued)

| Example | Structural Formula | Physical Property Value |
|---|---|---|
| 95 | 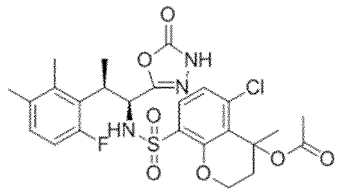 | 1H-NMR (CDCl3) δ: 8.88 (1H, s), 7.68-7.64 (1H, m), 6.98-6.92 (2H, m), 6.73-6.68 (1H, m), 5.48-5.43 (1H, m), 4.95-4.81 (1H, m), 4.55-4.49 (1H, m), 4.34-4.20 (1H, m), 3.45 (1H, s), 2.92-2.82 (1H, m), 2.20-2.18 (6H, m), 2.08 (2H, s), 2.02 (1H, s), 1.98-1.93 (1H, m), 1.86 (1H, s), 1.76 (2H, s), 1.57-1.51 (3H, m).; LC/MS RT 1.82, 1.87min, m/z $[M-H]^-$ 566,568 |
| 96 | 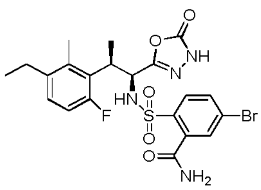 | 1H NMR (CD3OD) δ: 7.74-7.78 (m, 3H), 6.98 (dd, J=8.4, 5.9 Hz, 1H), 6.75 (dd, J=11.7, 8.4 Hz, 1H), 4.79 (d, J=11.0 Hz, 1H), 3.51-3.63 (m, 1H), 2.47-2.68 (m, 2H), 2.24 (s, 3H), 1.45 (d, J=7.0 Hz, 3H), 1.06 (t, J=7.5 Hz, 3H); LC/MS RT 1.75min, m/z $[M-H]^-$ 539,541 |
| 97 | 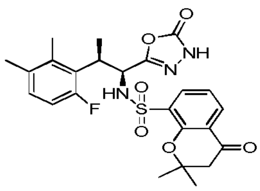 | 1H-NMR (CDCl3) δ: 8.06-7.99 (2H, m), 7.80 (1H, s), 7.07 (1H, t, J = 7.7 Hz), 6.93 (1H, dd, J = 8.5, 5.9 Hz), 6.69 (1H, dd, J = 11.5, 8.5 Hz), 5.28 (1H, d, J = 10.6 Hz), 4.86 (1H, t, J = 10.6 Hz), 3.46 (1H, br s), 2.92 (1H, d, J = 16.9 Hz), 2.69 (1H, d, J = 16.9 Hz), 2.20 (3H, s), 2.18 (3H, s), 1.64 (3H, s), 1.58-1.56 (3H, m), 1.46 (3H, s).; LC/MS RT 1.77min, m/z $[M-H]^-$ 502 |
| 98 | 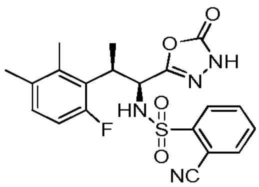 | LC/MS RT 1.66min, m/z $[M-H]^-$ 429 |
| 99 | 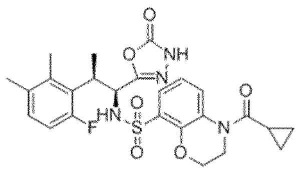 | 1H-NMR (CDCl3) δ: 8.11 (1H, br s), 7.66-7.63 (2H, m), 6.98-6.90 (2H, m), 6.71-6.66 (1H, m), 5.44 (1H, d, J = 10.5 Hz), 4.82 (1H, t, J = 10.5 Hz), 4.56-4.52 (1H, m), 4.47-4.42 (1H, m), 4.01-3.95 (1H, m), 3.44-3.37 (1H, m), 2.17-2.16 (6H, m), 1.89 (1H, br s), 1.55 (3H, d, J = 7.0 Hz), 1.21-1.10 (2H, m), 0.92-0.89 (2H, m); LC/MS RT 1.69min, m/z $[M-H]^-$ 529 |
| 100 | 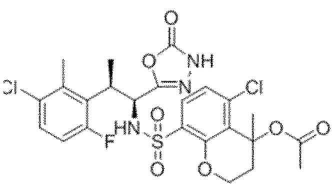 | LC/MS RT 1.83, 1.90min, m/z $[M-H]^-$ 586,588 |
| 101 | 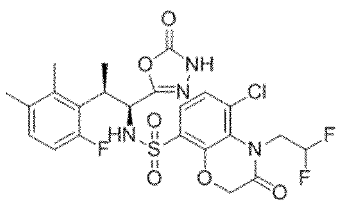 | 1H NMR (CD3OD) δ: 7.51 (1H, d, J = 8.6 Hz), 7.24 (1H, d, J = 8.6 Hz), 6.97 (1H, dd, J = 8.2, 5.7 Hz), 6.71 (1H, dd, J = 11.9, 8.2 Hz), 6.04 (1H, tt, J = 55.5, 3.9 Hz), 4.82-4.58 (7H, m), 3.67-3.62 (1H, m), 2.19 (3H, s), 2.16 (3H, s), 1.47 (3H, d, J = 7.0 Hz); LC/MS RT 1.76min, m/z $[M-H]^-$ 573,575 |
| 102 | 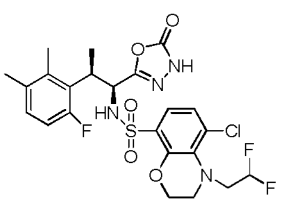 | 1H-NMR (CDCl3) δ: 7.67 (1H, br s), 7.46 (1H, d, J = 8.4 Hz), 7.01 (1H, d, J = 8.4 Hz), 6.94 (1H, dd, J = 8.4, 5.5 Hz), 6.70 (1H, dd, J = 11.4, 8.4 Hz), 6.24 (1H, tt, J = 56.3, 4.5 Hz), 5.41 (1H, d, J = 10.5 Hz), 4.88 (1H, t, J = 10.5 Hz), 4.42-4.37 (1H, m), 4.29-4.25 (1H, m), 3.42 (1H, br s), 3.32-3.17 (4H, m), 2.19-2.17 (6H, m), 1.55-1.54 (3H, m).; LC/MS RT 1.87min, m/z $[M-H]^-$ 559,561 |

(continued)

| Example | Structural Formula | Physical Property Value |
|---|---|---|
| 103 | 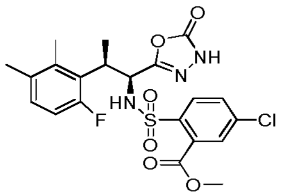 | LC/MS RT 1.87min, m/z [M-H]- 496,498 |
| 104 | 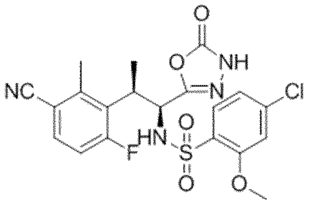 | 1H-NMR (CDCl3) δ: 8.21 (1H, br s), 7.79 (1H, d, J = 8.4 Hz), 7.49 (1H, dd, J = 8.6, 5.3 Hz), 7.04-7.01 (1H, m), 6.96-6.92 (2H, m), 5.60-5.56 (1H, m), 4.79 (1H, t, J = 10.6 Hz), 3.95 (3H, s), 3.43 (1H, br s), 2.53 (3H, s), 1.55 (3H, d, J = 7.0 Hz).; LC/MS RT 1.64min, m/z [M-H]- 479,481 |
| 105 | 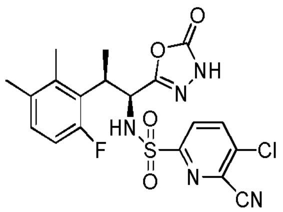 | LC/MS RT 1.71min, m/z [M-H]- 464,466 |
| 106 | 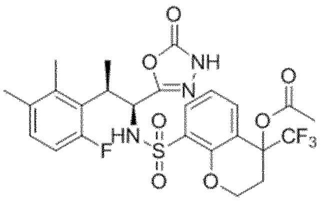 | 1H-NMR (CDCl3) δ: 7.92-7.86 (1H, m), 7.56-7.46 (1H, m), 7.08-7.00 (1H, m), 6.97-6.92 (1H, m), 6.73-6.67 (1H, m), 5.52-5.45 (1H, m), 4.96-4.83 (1H, m), 4.61-4.37 (3H, m), 3.49 (1H, br s), 2.90-2.82 (1H, m), 2.54-2.48 (1H, m), 2.19-2.16 (6H, m), 2.09-2.09 (3H, m), 1.57-1.50 (2H, m); LC/MS RT 1.81 1.85min, m/z [M-H]- 586 |
| 107 | 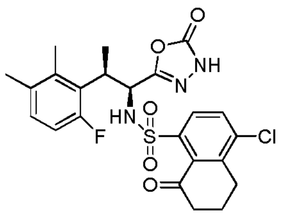 | LC/MS RT 1.90min, m/z [M-H]- 506,508 |
| 108 | 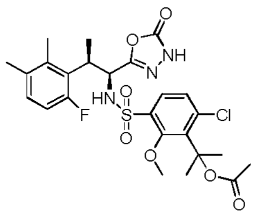 | LC/MS RT 1.98min, m/z [M-H]- 568,570 |
| 109 | 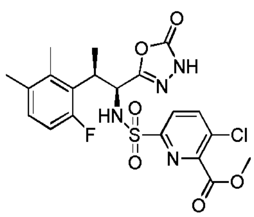 | LC/MS RT 1.71min, m/z [M-H]- 497,499 |

(continued)

| Example | Structural Formula | Physical Property Value |
|---|---|---|
| 110 | 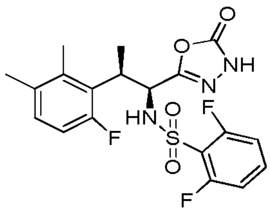 | 1H NMR (CD3OD) δ: 7.53-7.63 (m, 1H), 7.02-7.19 (m, 2H), 6.95-7.01 (m, 1H), 6.72 (dd, J=11.9, 8.6 Hz, 1H), 4.82-4.98 (m, 1H), 3.65-3.74 (m, 1H), 2.24 (s, 3H), 2.18 (s, 3H), 1.46 (d, J=7.0 Hz, 3H); LC/MS RT 1.69min, m/z [M-H]- 440 |
| 111 | 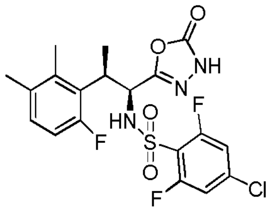 | LC/MS RT 1.79min, m/z [M-H]- 474,476 |
| 112 | 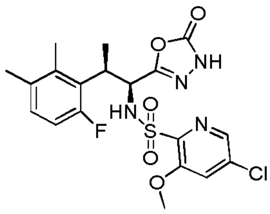 | 1H NMR (CD3OD) δ: 8.05 (d, J=1.8 Hz, 1H), 7.76 (d, J=1.8 Hz, 1H), 6.97 (dd, J=8.2, 5.7 Hz, 1H), 6.70 (dd, J=11.7, 8.4 Hz, 1H), 4.82 (d, J=11.4 Hz, 1H), 3.99 (s, 3H), 3.66-3.76 (m, 1H), 2.25 (s, 3H), 2.18 (s, 3H), 1.51 (d, J=7.0 Hz, 3H); LC/MS RT 1.62min, m/z [M-H]- 469,471 |
| 113 | 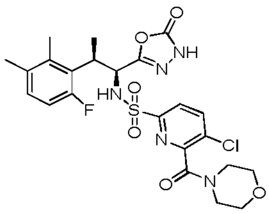 | 1H NMR (CD3OD) δ: 8.16 (d, J=8.4 Hz, 1H), 7.97 (d, J=8.4 Hz, 1H), 6.99 (dd, J=8.6, 6.0 Hz, 1H), 6.73 (dd, J=11.9, 8.6 Hz, 1H), 4.94 (d, J=11.4 Hz, 1H), 3.75-3.83 (m, 4H), 3.63-3.72 (m, 2H), 3.55-3.62 (m, 1H), 3.22-3.27 (m, 2H), 2.21 (s, 3H), 2.19 (s, 3H), 1.47 (d, J=7.3 Hz, 3H); LC/MS RT 1.57min, m/z [M-H]- 552,554 |
| 114 | 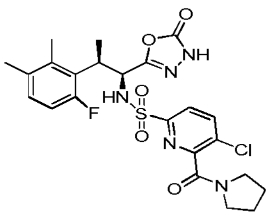 | 1H NMR (CD3OD) δ: 8.17 (d, J=8.4 Hz, 1H), 7.98 (d, J=8.4 Hz, 1H), 6.99 (dd, J=8.4, 5.9 Hz, 1H), 6.73 (dd, J=11.7, 8.4 Hz, 1H), 4.94 (d, J=11.4 Hz, 1H), 3.58-3.66 (m, 3H), 3.21-3.29 (m, 2H), 2.21 (s, 3H), 2.19 (s, 3H), 1.94-2.01 (m, 4H), 1.46 (d, J=7.0 Hz, 3H); LC/MS RT 1.65min, m/z [M-H]- 536,538 |
| 115 | 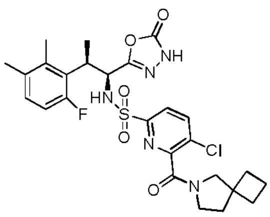 | 1H NMR (CD3OD) δ: 8.16 (d, J=8.4 Hz, 1H), 7.93-8.02 (m, 1H), 6.99 (dd, J=8.2, 6.0 Hz, 1H), 6.72 (dd, J=11.7, 8.4 Hz, 1H), 4.94 (dd, J=11.2, 3.1 Hz, 1H), 3.53-3.64 (m, 3H), 3.29-3.36 (m, 2H), 3.23-3.29 (m, 2H), 2.21 (s, 3H), 2.18 (s, 3H), 1.92-2.08 (m, 6H), 1.46 (br d, J=7.0 Hz, 3H); LC/MS RT 1.83min, m/z [M-H]- 576,578 |
| 116 | 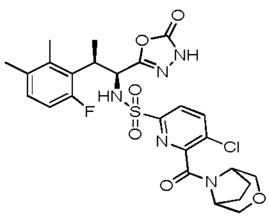 | 1H NMR (CD3OD) δ: 8.16 (dd, J=8.4, 5.1 Hz, 1H), 7.97 (dd, J=11.7, 8.4 Hz, 1H), 6.96-7.02 (m, 1H), 6.73 (dd, J=11.5, 8.4 Hz, 1H), 4.88-4.99 (m, 1H), 4.43-4.50 (m, 1H), 4.21-4.28 (m, 2H), 3.56-3.64 (m, 1H), 3.37-3.49 (m, 1H), 3.16 (d, J=12.8 Hz, 1H), 2.96 (d, J=12.8 Hz, 1H), 2.21 (s, 3H), 2.18 (s, 3H), 1.88-2.07 (m, 4H), 1.47 (d, J=7.0 Hz, 3H); LC/MS RT 1.61min, m/z [M-H]- 578,580 |

(continued)

| Example | Structural Formula | Physical Property Value |
|---|---|---|
| 117 | 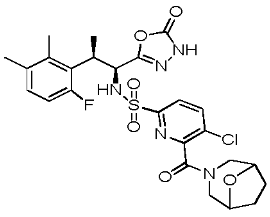 | 1H NMR (CD3OD) δ: 8.17 (dd, J=8.4, 2.6 Hz, 1H), 7.97 (dd, J=8.4, 2.2 Hz, 1H), 6.99 (dd, J=8.4, 5.7 Hz, 1H), 6.73 (dd, J=11.5, 8.4 Hz, 1H), 4.91-4.97 (m, 1H), 4.66-4.73 (m, 1H), 3.80-3.90 (m, 2H), 3.54-3.77 (m, 4H), 2.19-2.22 (m, 3H), 2.18 (s, 3H), 2.03-2.12 (m, 4H), 1.46 (d, J=7.0 Hz, 3H); LC/MS RT 1.62min, m/z $[M-H]^-$ 578,580 |
| 118 | 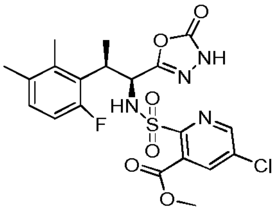 | LC/MS RT 1.72min, m/z$[M-H]^-$ 497,499 |
| 119 | 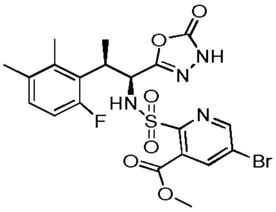 | LC/MS RT 1.73min, m/z $[M-H]^-$ 541,543 |
| 120 | 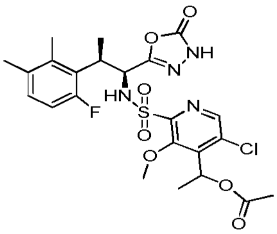 | LC/MS RT 1.80min, m/z $[M-H]^-$ 555,557 |
| 121 | 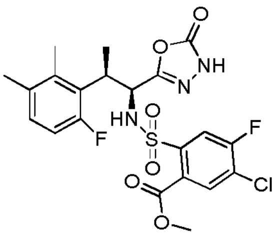 | LC/MS RT 1.93min, m/z $[M-H]^-$ 514,516 |
| 122 | 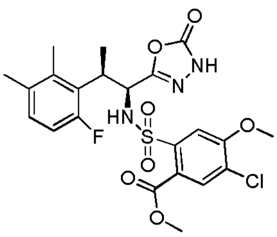 | LC/MS RT 1.91min, m/z $[M-H]^-$ 526,528 |
| 123 | 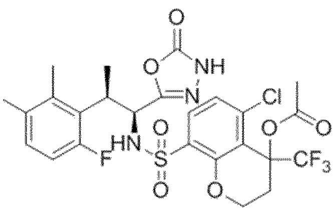 | 1H-NMR (CDCl3) δ: 8.56 (0.5H, s), 8.04 (0.5H, s), 7.83-7.78 (1H, m), 7.07-7.04 (1H, m), 6.96-6.93 (1H, m), 6.74-6.68 (1H, m), 5.43-5.40 (1H, m), 4.97-4.91 (0.5H, m), 4.84-4.79 (0.5H, m), 4.60-4.42 (2H, m), 3.51 (1H, s), 2.94 (1H, s), 2.53-2.44 (1H, m), 2.22-2.17 (9H, m), 1.57-1.50 (3H, m).; LC/MS RT 1.89, 1.94min, m/z $[M-H]^-$ 620,622 |

(continued)

| Example | Structural Formula | Physical Property Value |
|---|---|---|
| 124 | 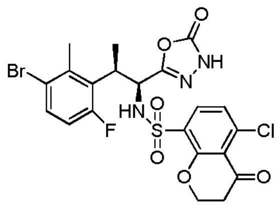 | 1H NMR (CD3OD) δ: 7.88 (d, J=8.4 Hz, 1H), 7.43 (dd, J=8.8, 5.5 Hz, 1H), 7.15 (d, J=8.4 Hz, 1H), 6.82 (dd, J=11.2, 9.0 Hz, 1H), 4.69-4.80 (m, 3H), 3.63-3.79 (m, 1H), 2.85-2.92 (m, 2H), 2.44 (s, 3H), 1.54 (d, J=7.0 Hz, 3H); LC/MS RT 1.77min, m/z $[M-H]^-$ 572,574 |
| 125 | 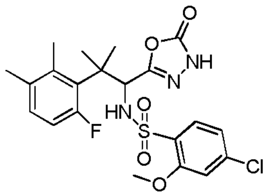 | 1H-NMR (CDCl3) δ: 8.47 (1H, br s), 7.67 (1H, d, J = 8.4 Hz), 6.96-6.93 (2H, m), 6.82-6.82 (1H, m), 6.65 (1H, dd, J = 14.5, 8.2 Hz), 5.67 (1H, d, J = 9.6 Hz), 5.01 (1H, dd, J = 9.6, 1.6 Hz), 3.88 (3H, s), 2.29 (3H, s), 2.15 (3H, s), 1.66 (3H, d, J = 4.0 Hz), 1.58 (3H, d, J = 3.7 Hz).; LC/MS RT 1.87min, m/z $[M-H]^-$ 482,484 |
| 126 | 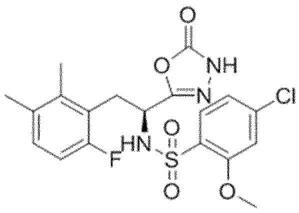 | 1H NMR (CD3OD) δ: 7.63 (1H, d, J = 8.4 Hz), 7.07 (1H, s), 6.98-6.94 (2H, m), 6.70-6.66 (1H, m), 4.40 (1H, t, J = 7.7 Hz), 3.94 (3H, s), 3.26-3.23 (1H, m), 3.16-3.11 (1H, m), 2.17 (3H, s), 2.14 (3H, s); LC/MS RT 1.76min, m/z $[M-H]^-$ 454,456 |
| 127 | 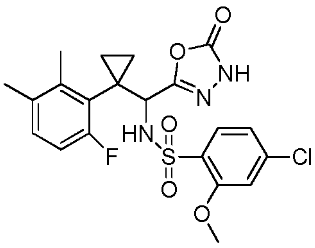 | 1H NMR (CD3OD) δ: 7.72 (1H, dd, J = 14.3, 8.4 Hz), 7.12-7.06 (1H, m), 7.05-6.99 (2H, m), 6.72-6.63 (1H, m), 4.34-4.16 (1H, m), 3.86-3.81 (3H, m), 2.37-2.30 (3H, m), 2.19 (3H, s), 1.57-1.54 (1H, m), 1.35-1.28 (1H, m), 0.93-0.87 (1H, m), 0.72-0.67 (1H, m); LC/MS RT 1.85min, m/z $[M-H]^-$ 480,482 |
| 128 | 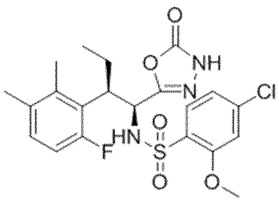 | 1H-NMR (CDCl3) δ: 7.84 (1H, br s), 7.78 (1H, d, J = 8.1 Hz), 7.00 (1H, dd, J = 8.4, 1.8 Hz), 6.95-6.91 (2H, m), 6.68 (1H, dd, J = 11.7, 8.4 Hz), 5.46 (1H, d, J = 10.3 Hz), 4.82 (1H, t, J = 10.6 Hz), 3.94 (3H, s), 3.28-3.22 (1H, m), 2.27-2.25 (1H, m), 2.19-2.16 (6H, m), 1.96-1.87 (1H, m), 0.79 (3H, t, J = 7.3 Hz).; LC/MS RT 1.85min, m/z $[M-H]^-$ 482,484 |
| 129 | 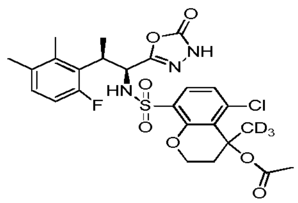 | 1H NMR (CD3OD) δ: 7.58-7.73 (m, 1H), 6.91-7.11 (m, 2H), 6.62-6.81 (m, 1H), 4.71-4.82 (m, 1H), 4.49-4.61 (m, 2H), 3.57-3.79 (m, 1H), 2.70-2.95 (m, 1H), 2.28-2.38 (m, 0.5H), 2.20-2.25 (m, 3H), 2.16-2.24 (m, 3H), 2.08-2.14 (m, 0.5H), 1.98-2.02 (m, 3H), 1.42-1.56 (m, 3H); LC/MS RT 1.81, 1.87min, m/z $[M-H]^-$ 569,571 |
| 130 | 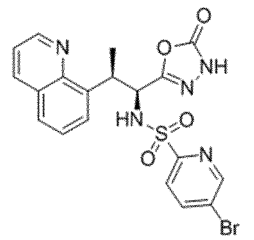 | 1H NMR (CD3OD) δ: 8.87 (dd, J=4.2, 1.6 Hz, 1H), 8.39 (d, J=2.6 Hz, 1H), 8.12-8.36 (m, 1H), 7.98 (dd, J=8.2, 2.4 Hz, 1H), 7.69-7.86 (m, 1H), 7.55-7.68 (m, 2H), 7.42-7.51 (m, 2H), 5.21 (d, J=7.7 Hz, 1H), 4.40-4.53 (m, 1H), 1.55 (d, J=7.3 Hz, 3H); LC/MS RT 1.82min, m/z $[M-H]^-$ 488,490 |

(continued)

| Example | Structural Formula | Physical Property Value |
|---|---|---|
| 131 | 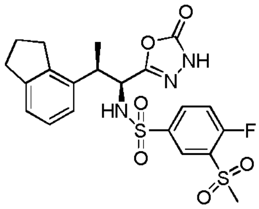 | 1H NMR (CD3OD) δ: 8.28 (dd, J=6.2, 2.6 Hz, 1H), 8.08 (ddd, J=8.7, 4.5, 2.6 Hz, 1H), 7.43-7.54 (m, 1H), 7.00-7.09 (m, 2H), 6.98 (d, J=2.2 Hz, 1H), 4.42 (d, J=10.6 Hz, 1H), 3.31-3.41 (m, 1H), 3.28 (s, 3H), 2.79-2.87 (m, 4H), 1.92-2.07 (m, 2H), 1.42 (d, J=7.0 Hz, 3H); LC/MS RT 1.67min, m/z $[M-H]^-$ 494 |
| 132 | 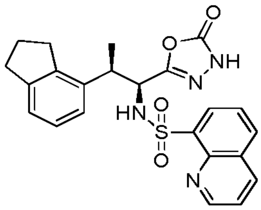 | 1H NMR (CD3OD) δ: 9.01 (dd, J=4.2, 1.6 Hz, 1H), 8.28-8.45 (m, 2H), 8.15 (d, J=7.0 Hz, 1H), 7.64-7.71 (m, 1H), 7.57-7.63 (m, 1H), 6.91-7.00 (m, 2H), 6.85-6.91 (m, 1H), 4.45 (d, J=10.6 Hz, 1H), 3.28-3.42 (m, 1H), 2.68-2.94 (m, 4H), 1.78-2.08 (m, 2H), 1.45 (d, J=7.0 Hz, 3H); LC/MS RT 1.72min, m/z $[M-H]^-$ 449 |
| 133 | 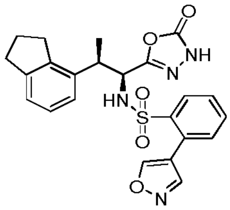 | 1H NMR (CD3OD) δ: 8.88 (s, 1H), 8.59 (s, 1H), 8.01-8.08 (m, 1H), 7.56-7.65 (m, 1H), 7.45-7.54 (m, 1H), 7.36-7.42 (m, 1H), 6.98-7.06 (m, 2H), 6.85-6.93 (m, 1H), 4.23 (d, J=11.0 Hz, 1H), 3.19-3.31 (m, 1H), 2.75-2.82 (m, 4H), 1.82-2.08 (m, 2H), 1.35 (d, J=7.0 Hz, 3H); LC/MS RT 1.76min, m/z $[M-H]^-$ 465 |
| 134 | 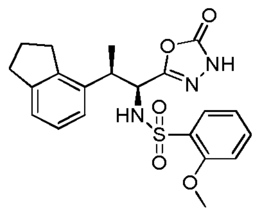 | 1H NMR (cdcl3) δ: 7.81 (dd, J=7.7, 1.8 Hz, 1H), 7.73 (br s, 1H), 7.43-7.50 (m, 1H), 7.03-7.07 (m, 2H), 7.00 (t, J=7.7 Hz, 1H), 6.90-6.95 (m, 2H), 5.48 (d, J=10.6 Hz, 1H), 4.47 (t, J=10.3 Hz, 1H), 3.95 (s, 3H), 3.14-3.33 (m, 1H), 2.77-2.88 (m, 4H), 1.95-2.10 (m, 2H), 1.49 (d, J=7.0 Hz, 3H); LC/MS RT 1.69min, m/z $[M-H]^-$ 428 |
| 135 | 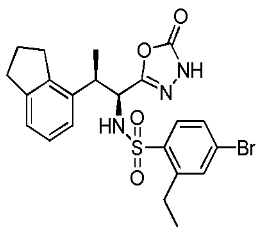 | 1H NMR (CD3OD) δ: 7.72 (d, J=8.4 Hz, 1H), 7.47 (d, J=1.8 Hz, 1H), 7.42 (dd, J=8.4, 2.2 Hz, 1H), 6.98-7.03 (m, 2H), 6.89-6.95 (m, 1H), 4.28 (d, J=10.3 Hz, 1H), 3.25-3.33 (m, 1H), 2.88-3.11 (m, 2H), 2.75-2.85 (m, 4H), 1.84-2.10 (m, 2H), 1.39 (d, J=7.0 Hz, 3H), 1.25 (t, J=7.5 Hz, 3H); LC/MS RT 2.02min, m/z $[M-H]^-$ 506,508 |
| 136 | 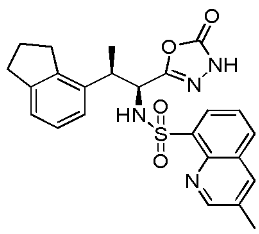 | 1H NMR (CD3OD) δ: 8.86 (d, J=2.2 Hz, 1H), 8.24 (dd, J=7.3, 1.5 Hz, 1H), 8.12 (s, 1H), 8.06 (dd, J=8.1, 1.5 Hz, 1H), 7.61 (t, J=7.6 Hz, 1H), 6.82-7.03 (m, 3H), 4.40 (d, J=11.0 Hz, 1H), 3.32-3.40 (m, 1H), 2.70-2.89 (m, 4H), 2.54 (s, 3H), 1.79-2.13 (m, 2H), 1.45 (d, J=7.0 Hz, 3H); LC/MS RT 1.79min, m/z $[M-H]^-$ 463 |
| 137 | 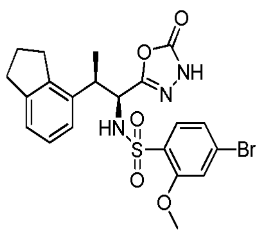 | 1H NMR (CD3OD) δ: 7.64 (d, J=8.4 Hz, 1H), 7.24 (d, J=1.5 Hz, 1H), 7.18 (dd, J=8.4, 1.8 Hz, 1H), 6.97-7.07 (m, 2H), 6.90-6.96 (m, 1H), 4.30 (d, J=11.0 Hz, 1H), 3.94 (s, 3H), 3.32-3.38 (m, 1H), 2.78-2.89 (m, 4H), 1.90-2.11 (m, 2H), 1.44 (d, J=7.0 Hz, 3H); LC/MS RT 1.82min, m/z $[M-H]^-$ 506,508 |

(continued)

| Example | Structural Formula | Physical Property Value |
|---|---|---|
| 138 | 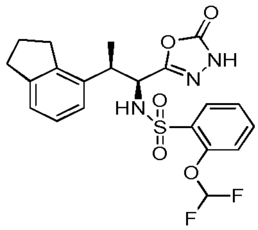 | 1H NMR (CD3OD) δ: 7.89 (dd, J=7.9, 1.6 Hz, 1H), 7.59 (td, J=8.0, 1.6 Hz, 1H), 7.26-7.33 (m, 3H), 6.99-7.05 (m, 2H), 6.92-6.96 (m, 1H), 4.41 (d, J=10.6 Hz, 1H), 3.36-3.42 (m, 1H), 2.78-2.91 (m, 4H), 1.91-2.04 (m, 2H), 1.40 (d, J=7.0 Hz, 3H); LC/MS RT 1.78min, m/z $[M-H]^-$ 464 |
| 139 | 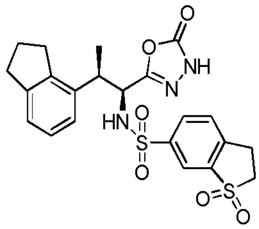 | 1H NMR (CD3OD) δ: 8.00 (d, J=1.8 Hz, 1H), 7.94 (dd, J=8.1, 1.8 Hz, 1H), 7.58 (d, J=8.1 Hz, 1H), 6.99-7.07 (m, 2H), 6.94-6.99 (m, 1H), 4.41 (d, J=10.3 Hz, 1H), 3.56-3.60 (m, 2H), 3.39-3.46 (m, 2H), 3.25-3.29 (m, 1H), 2.78-2.87 (m, 4H), 1.93-2.06 (m, 2H), 1.40 (d, J=7.0 Hz, 3H); LC/MS RT 1.60min, m/z $[M-H]^-$ 488 |
| 140 | 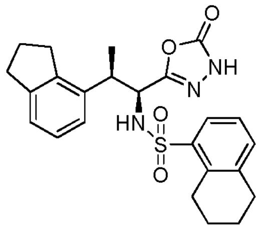 | 1H NMR (CD3OD) δ: 7.39-7.43 (m, 1H), 7.36-3.38 (m, 1H), 7.10 (d, J=8.1 Hz, 1H), 7.00-7.06 (m, 2H), 6.93-6.98 (m, 1H), 4.30 (d, J=11.0 Hz, 1H), 3.30-3.35 (m, 1H), 2.74-2.87 (m, 8H), 1.91-2.10 (m, 2H), 1.77-1.82 (m, 4H), 1.41 (d, J=6.6 Hz, 3H); LC/MS RT 1.91min, m/z $[M-H]^-$ 452 |
| 141 | 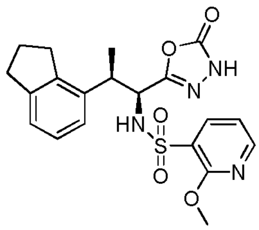 | 1H NMR (CD3OD) δ: 8.53-8.55 (m, 1H), 8.23-8.31 (m, 1H), 8.10 (dd, J=7.5, 2.0 Hz, 1H), 7.39-7.50 (m, 1H), 7.00-7.05 (m, 2H), 4.32 (d, J=10.6 Hz, 1H), 4.03 (s, 3H), 3.45-3.52 (m, 1H), 2.76-2.93 (m, 4H), 1.93-2.12 (m, 2H), 1.44 (d, J=7.0 Hz, 3H); LC/MS RT 1.65min, m/z $[M-H]^-$ 429 |
| 142 | 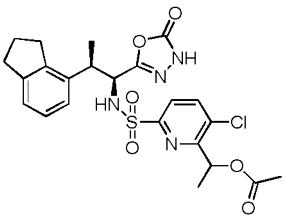 | LC/MS RT 1.80min, m/z $[M-H]^-$ 519,521 |
| 143 | 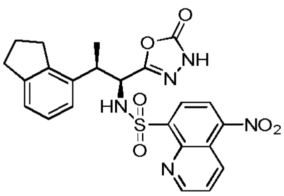 | 1H NMR (CD3OD) δ: 8.86-9.01 (m, 1H), 8.29-8.55 (m, 1H), 7.95-8.24 (m, 1H), 7.37-7.52 (m, 1H), 6.63-7.24 (m, 4H), 4.26-4.46 (m, 1H), 3.14-3.39 (m, 1H), 2.71-2.88 (m, 4H), 1.81-2.10 (m, 2H), 1.43 (d, J=7.0 Hz, 3H); LC/MS RT 1.80min, m/z $[M-H]^-$ 494 |
| 144 | 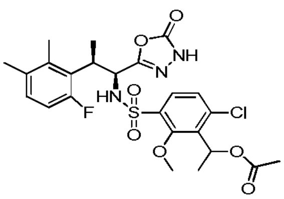 | LC/MS RT 1.92min, m/z $[M-H]^-$ 554,556 |

(continued)

| Example | Structural Formula | Physical Property Value |
|---|---|---|
| 145 | 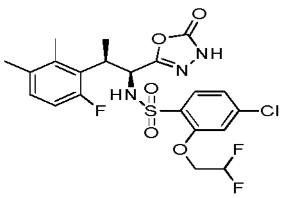 | LC/MS RT 1.86min, m/z [M-H]- 518,520 |
| 146 | 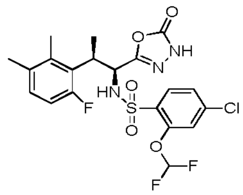 | 1H NMR (CD3OD) δ: 7.90 (d, J=8.4 Hz, 1H), 7.37 (dd, J=8.4, 1.8 Hz, 1H), 7.33-7.35 (m, 1H), 6.88-7.05 (m, 1H), 6.70 (dd, J=11.7, 8.4 Hz, 1H), 5.29-5.43 (m, 1H), 4.74 (d, J=11.4 Hz, 1H), 3.59-3.75 (m, 1H), 2.22 (s, 3H), 2.18 (s, 3H), 1.45 (d, J=7.0Hz, 3H); LC/MS RT 1.86min, m/z [M-H]- 504,506 |
| 147 | 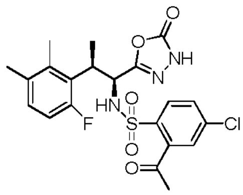 | LC/MS RT 1.83min, m/z [M-H]- 480,482 |
| 148 | 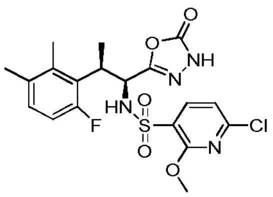 | 1H NMR (CD3OD) δ: 8.10 (d, J=8.1 Hz, 1H), 7.10 (d, J=8.1 Hz, 1H), 6.97 (dd, J=8.4, 5.9 Hz, 1H), 6.70 (dd, J=11.7, 8.4 Hz, 1H), 4.69 (d, J=11.4 Hz, 1H), 4.03 (s, 3H), 3.60-3.74 (m, 1H), 2.22 (s, 3H), 2.18 (s, 3H), 1.47 (d, J=7.0 Hz, 3H); LC/MS RT 1.77min, m/z [M-H]- 469,471 |
| 149 | 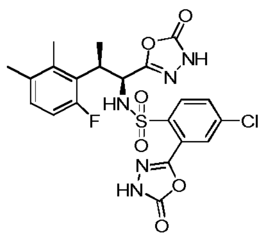 | 1H NMR (CD3OD) δ: 8.14-8.23 (m, 2H), 8.05-8.10 (m, 1H), 6.99 (dd, J=8.4, 6.0 Hz, 1H), 6.75 (dd, J=11.9, 8.4 Hz, 1H), 5.40 (d, J=11.4 Hz, 1H), 3.65-3.77 (m, 1H), 2.23 (s, 3H), 2.20 (s, 3H), 1.40 (d, J=7.0 Hz, 3H); LC/MS RT 1.66min, m/z [M-H]- 522,524 |
| 150 | 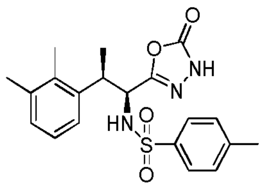 | 1H NMR (cdcl3) δ: 8.28-8.53 (m, 1H), 7.54 (br d, J=8.1 Hz, 2H), 7.18 (br d, J=8.1 Hz, 2H), 6.90-7.08 (m, 3H), 5.17 (br d, J=9.2 Hz, 1H), 4.40 (t, J=9.9 Hz, 1H), 3.49 (s, 2H), 3.44-3.61 (m, 1H), 2.38 (s, 3H), 2.20 (s, 3H), 2.11 (s, 3H), 1.34 (d, J=7.0 Hz, 3H); LC/MS RT 1.72min, m/z [M-H]- 400 |
| 151 | 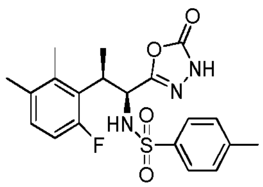 | 1H NMR (cdcl3) δ: 5.27 (d, J=9.9 Hz, 1H), 7.69 (d, J=8.4 Hz, 2H), 7.26 (d, J=7.7 Hz, 4H), 6.94 (dd, J=8.1, 5.9 Hz, 1H), 6.71 (dd, J=11.4, 8.4 Hz, 1H), 5.27 (br d, J=9.9 Hz, 1H), 4.79 (t, J=10.3 Hz, 1H), 3.31-3.48 (m, 1H), 2.38 (s, 3H), 2.17 (s, 3H), 2.15 (s, 3H), 1.42 (br d, J=7.0 Hz, 3H); LC/MS RT 1.72min, m/z [M-H]- 418 |

(continued)

| Example | Structural Formula | Physical Property Value |
|---|---|---|
| 152 | 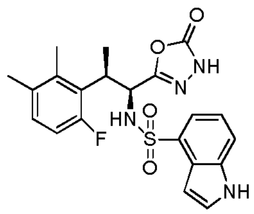 | 1H-NMR (CDCl3) δ: 8.75 (1H, s), 7.72 (1H, d, J = 7.7 Hz), 7.55 (1H, d, J = 8.1 Hz), 7.38-7.37 (1H, m), 7.22 (1H, t, J = 7.9 Hz), 6.91-6.86 (2H, m), 6.66-6.61 (1H, m), 5.48 (1H, d, J = 10.4 Hz), 4.74 (1H, t, J = 10.4 Hz), 3.34 (1H, br s), 2.12 (3H, s), 2.08 (3H, s), 1.36 (3H, d, J = 6.6 Hz); LC/MS RT 1.60min, m/z [M-H]$^-$ 443 |
| 153 | 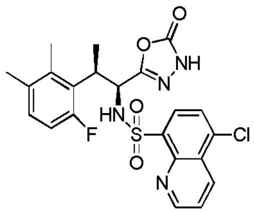 | 1H NMR (CD3OD) δ: 9.09 (dd, J=4.2, 1.6 Hz, 1H), 8.69 (d, J=8.9 Hz, 1H), 8.34 (d, J=8.1 Hz, 1H), 7.82 (d, J=8.1 Hz, 1H), 7.70-7.76 (m, 1H), 6.91 (dd, J=8.4, 5.9 Hz, 1H), 6.55-6.68 (m, 1H), 4.80 (d, J=11.4 Hz, 1H), 3.62-3.70 (m, 1H), 2.18 (s, 3H), 2.13 (s, 3H), 1.49 (d, J=6.6 Hz, 3H); LC/MS RT 1.80min, m/z [M-H]$^-$ 489,491 |
| 154 | 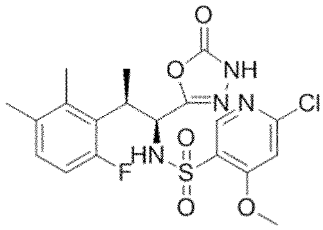 | 1H-NMR (CDCl3) δ: 8.63 (1H, s), 6.98-6.93 (1H, m), 6.92 (1H, s), 6.71 (1H, dd, J = 11.7, 8.4 Hz), 5.57 (1H, br s), 4.94-4.90 (1H, m), 4.03 (3H, s), 3.45 (1H, s), 2.18 (6H, s), 1.53 (3H, d, J = 7.0 Hz); LC/MS RT 1.65min, m/z [M-H]$^-$ 469,471 |
| 155 | 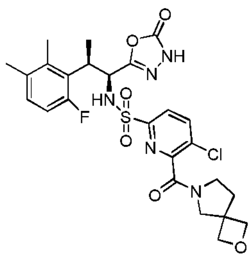 | 1H-NMR (CDCl3) δ: 7.95-7.91 (2H, m), 6.96-6.92 (1H, m), 6.70 (1H, dd, J = 11.5, 8.4 Hz), 4.98 (1H, t, J = 11.5 Hz), 4.76 (1H, d, J = 6.2 Hz), 4.62 (2H, t, J = 6.2 Hz), 4.58-4.55 (1H, m), 4.05-3.77 (1H, m), 3.70-3.66 (2H, m), 3.52-3.25 (4H, m), 2.31-2.21 (2H, m), 2.18 (3H, s), 2.14 (3H, d, J = 9.2 Hz), 1.53 (3H, d, J = 6.6 Hz).; LC/MS RT 1.54min, m/z [M-H]$^-$ 578,580 |
| 156 | 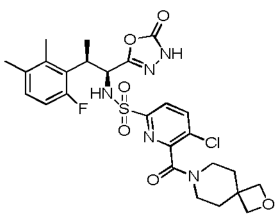 | 1H-NMR (CDCl3) δ: 7.95-7.92 (2H, m), 6.94 (1H, dd, J = 8.3, 5.9 Hz), 6.68 (1H, dd, J = 11.4, 8.3 Hz), 4.95 (1H, d, J = 10.6 Hz), 4.51-4.45 (4H, m), 3.82-3.80 (1H, m), 3.58-3.51 (1H, m), 3.44 (1H, br s), 3.16-3.03 (2H, m), 2.18 (3H, s), 2.15 (3H, s), 2.02-1.98 (2H, m), 1.92-1.88 (2H, m), 1.53 (3H, d, J = 7.0 Hz); LC/MS RT 1.56min, m/z [M-H]$^-$ 592,594 |
| 157 | 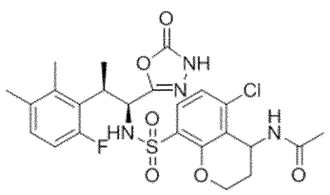 | 1H-NMR (CDCl3) δ: 10.23 (1H, s), 7.64 (1H, d, J = 8.4 Hz), 7.02 (1H, d, J = 8.4 Hz), 6.92 (1H, dd, J = 8.4, 5.9 Hz), 6.68 (1H, dd, J = 11.7, 8.4 Hz), 6.30 (1H, d, J = 6.2 Hz), 5.58 (1H, d, J = 10.3 Hz), 5.23 (1H, s), 4.79 (1H, t, J = 10.4 Hz), 4.54-4.51 (1H, m), 4.21-4.17 (1H, m), 3.54 (1H, s), 3.48 (1H, s), 2.19 (3H, s), 2.18 551,553 1.96 (3H, s), 1.53 (3H, d, J = 7.0 Hz); LC/MS RT 1.61min, m/z [M-H]$^-$ |
| 158 | 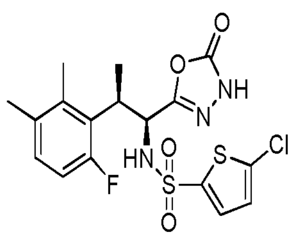 | 1H-NMR (CDCl3) δ: 8.12 (1H, br s), 7.37 (1H, d, J = 4.0 Hz), 6.97 (1H, dd, J = 8.3, 5.7 Hz), 6.86 (1H, d, J = 4.0 Hz), 6.72 (1H, dd, J = 11.7, 8.3 Hz), 5.24 (1H, br s), 4.84 (1H, t, J = 10.1 Hz), 3.48-3.43 (1H, m), 2.19 (3H, s), 2.17 (3H, s), 1.45 (3H, d, J = 7.0 Hz).; LC/MS RT 1.79min, m/z [M-H]$^-$ 444,446 |

(continued)

| Example | Structural Formula | Physical Property Value |
|---|---|---|
| 159 | 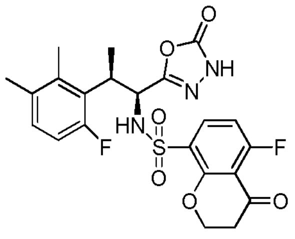 | ; LC/MS RT 1.65min, m/z [M-H]- 492 |
| 160 | 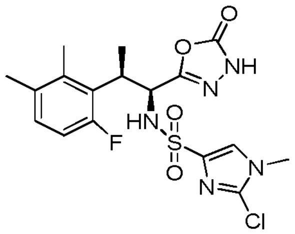 | 1H-NMR (CDCl3) δ: 8.52 (1H, br s), 7.48 (1H, s), 6.96-6.92 (1H, m), 6.73-6.68 (1H, m), 5.53 (1H, d, J = 9.5 Hz), 4.80 (1H, t, J = 9.5 Hz), 3.66 (3H, s), 3.48 (1H, br s), 2.21 (3H, s), 2.19 (3H, s), 1.56-1.55 (3H, m).; LC/MS RT 1.53min, m/z [M-H]- 442,444 |
| 161 | 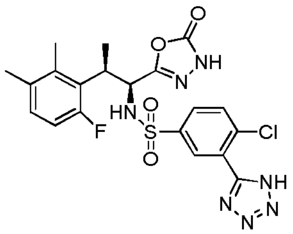 | 1H-NMR (CDCl3) δ: 8.34-8.54 (1H, m), 7.98 (1H, dd, J = 8.5, 2.4 Hz), 7.65 (1H, d, J = 8.5 Hz), 6.93-6.89 (1H, m), 6.67 (1H, dd, J = 11.4, 8.4 Hz), 6.16-6.14 (1H, m), 4.91 (1H, t, J = 10.3 Hz), 3.51 (2H, br s), 2.16 (1H, s), 2.14 (3H, s), 2.12 (3H, s), 1.53 (3H, d, J = 7.0 Hz).; LC/MS RT 1.58min, m/z [M-H]- 506,508 |
| 162 | 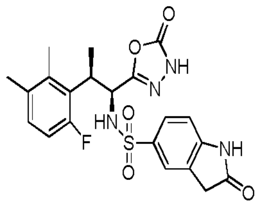 | 1H NMR (CD3OD) δ: 7.64 (dd, J=8.1, 1.8 Hz, 1H), 7.60 (s, 1H), 6.97 (dd, J=8.4, 5.5 Hz, 1H), 6.92 (d, J=8.1 Hz, 1H), 6.71 (dd, J=11.9, 8.1 Hz, 1H), 4.67 (d, J=11.0 Hz, 1H), 3.53-3.56 (m, 3H), 2.17 (s, 3H), 2.16 (s, 3H), 1.46 (d, J=6.6 Hz, 3H); LC/MS RT 1.49min, m/z [M-H]- 459 |
| 163 | 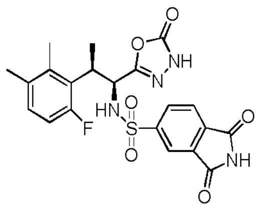 | 1H NMR (CD3OD) δ: 8.11-8.21 (m, 2H), 7.90-7.98 (m, 1H), 6.97 (dd, J=8.4, 5.9 Hz, 1H), 6.72 (dd, J=11.7, 8.4 Hz, 1H), 4.77 (d, J=11.0 Hz, 1H), 3.53-3.72 (m, 1H), 2.18 (s, 3H), 2.16 (s, 3H), 1.47 (d, J=7.0 Hz, 3H); LC/MS RT 1.58min, m/z [M-H]- 473 |
| 164 | 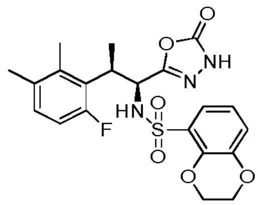 | 1H NMR (CD3OD) δ: 7.30 (dd, J=8.1, 1.5 Hz, 1H), 7.01 (dd, J=8.1, 1.5 Hz, 1H), 6.96 (dd, J=8.4, 5.9 Hz, 1H), 6.86 (t, J=8.0 Hz, 1H), 6.70 (dd, J=11.9, 8.6 Hz, 1H), 4.74 (d, J=11.0 Hz, 1H), 4.37-4.45 (m, 2H), 4.19-4.34 (m, 2H), 3.61-3.77 (m, 1H), 2.21 (s, 3H), 2.17 (s, 3H), 1.49 (d, J=6.6 Hz, 3H); LC/MS RT 1.67min, m/z [M-H]- 462 |
| 165 | 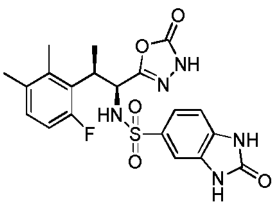 | 1H NMR (CD3OD) δ: 7.47 (dd, J=8.4, 1.8 Hz, 1H), 7.43 (d, J=1.8 Hz, 1H), 7.07 (d, J=8.3 Hz, 1H), 6.96 (dd, J=8.3, 5.7 Hz, 1H), 6.70 (dd, J=11.7, 8.4 Hz, 1H), 4.68 (d, J=11.4 Hz, 1H), 3.53 (br dd, J=10.8, 7.1 Hz, 1H), 2.17 (s, 3H), 2.15 (s, 3H), 1.43 (d, J=6.6 Hz, 3H); LC/MS RT 1.42min, m/z [M-H]- 460 |

(continued)

| Example | Structural Formula | Physical Property Value |
| --- | --- | --- |
| 166 | 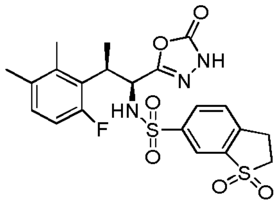 | LC/MS RT 1.59min, m/z [M-H]- 494 |
| 167 | 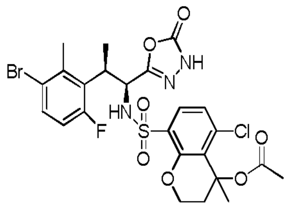 | LC/MS RT 1.87, 1.92min, m/z [M-H]- 630,632 |
| 168 | 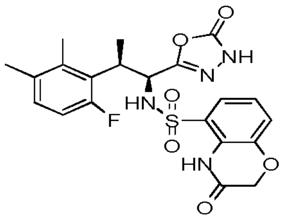 | 1H NMR (CD3OD) δ: 7.40 (dd, J=8.1, 1.5 Hz, 1H), 7.16 (dd, J=8.1, 1.1 Hz, 1H), 7.04 (t, J=8.2 Hz, 1H), 6.98 (dd, J=8.4, 5.7 Hz, 1H), 6.72 (dd, J=11.5, 8.4 Hz, 1H), 4.68 (d, J=11.4 Hz, 1H), 4.63-4.66 (m, 2H), 3.50-3.59 (m, 1H), 2.17 (s, 6H), 1.45 (d, J=7.0 Hz, 3H); LC/MS RT 1.65min, m/z [M-H]- 475 |
| 169 | 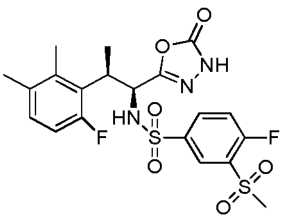 | 1H NMR (CD3OD) δ: 8.30 (dd, J=6.2, 2.2 Hz, 1H), 8.12 (ddd, J=8.7, 4.5, 2.6 Hz, 1H), 7.52 (t, J=9.1 Hz, 1H), 6.98 (dd, J=8.4, 5.9 Hz, 1H), 6.73 (dd, J=11.7, 8.4 Hz, 1H), 4.75 (d, J=11.4 Hz, 1H), 3.52-3.64 (m, 1H), 3.28(s, 3H), 2.20 (s, 3H), 2.17 (s, 3H), 1.47 (d, J=6.6 Hz, 3H); LC/MS RT 1.65min, m/z [M-H]- 500 |
| 170 | 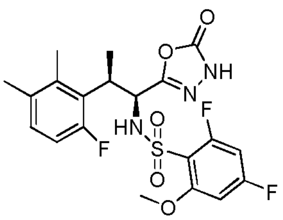 | 1H NMR (CD3OD) δ: 6.97 (dd, J=8.4, 5.9 Hz, 1H), 6.68-6.78 (m, 2H), 6.59-6.67 (m, 1H), 4.80 (d, J=11.4 Hz, 1H), 3.94 (s, 3H), 3.63-3.75 (m, 1H), 2.23 (s, 3H), 2.18 (s, 3H), 1.50 (d, J=6.6 Hz, 3H); LC/MS RT 1.71min, m/z [M-H]- 470 |
| 171 | 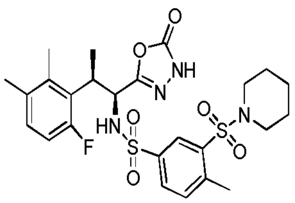 | 1H NMR (CD3OD) δ: 8.22 (d, J=1.8 Hz, 1H), 7.83 (dd, J=8.1, 2.2 Hz, 1H), 7.51 (d, J=8.1 Hz, 1H), 6.93-7.03 (m, 1H), 6.66-6.80 (m, 1H), 4.71 (d, J=11.3 Hz, 1H), 3.51-3.64 (m, 1H), 3.07-3.26 (m, 4H), 2.64 (s, 3H), 2.18 (s, 3H), 2.17 (s, 3H), 1.55-1.71 (m, 6H), 1.43 (d, J=6.6 Hz, 3H); LC/MS RT 1.89min, m/z [M-H]- 565 |
| 172 | 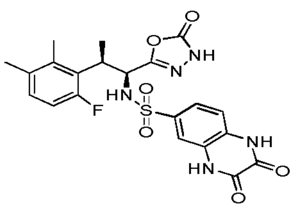 | 1H NMR (CD3OD) δ: 7.59 (d, J=1.9 Hz, 1H), 7.50 (dd, J=8.6, 1.9Hz, 1H), 7.21 (d, J=8.3 Hz, 1H), 6.97 (dd, J=8.3, 5.7 Hz, 1H), 6.71 (dd, J=11.7, 8.4 Hz, 1H), 4.70 (d, J=11.0 Hz, 1H), 3.51-3.58 (m, 1H), 2.17 (s, 3H), 2.16 (s, 3H), 1.45 (d, J=7.0 Hz, 3H); LC/MS RT 1.39min, m/z [M-H]- 488 |

(continued)

| Example | Structural Formula | Physical Property Value |
|---|---|---|
| 173 | 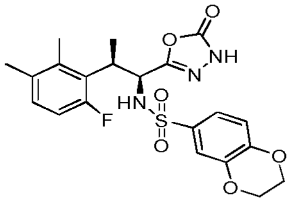 | 1H NMR (CD3OD) δ: 7.19-7.25 (m, 2H), 6.97 (dd, J=8.4, 5.9 Hz, 1H), 6.87 (d, J=8.4 Hz, 1H), 6.72 (dd, J=11.7, 8.4 Hz, 1H), 4.66 (d, J=11.4 Hz, 1H), 4.22-4.31 (m, 4H), 3.40-3.65 (m, 1H), 2.16 (s, 6H), 1.44 (d, J=7.3 Hz, 3H); LC/MS RT 1.67min, m/z $[M-H]^-$ 462 |
| 174 | 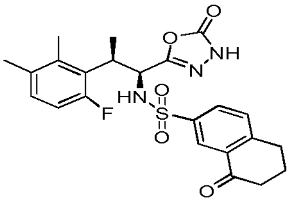 | LC/MS RT 1.68min, m/z $[M-H]^-$ 472 |
| 175 | 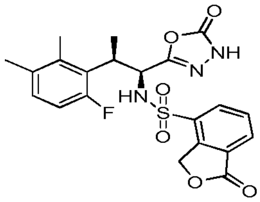 | 1H NMR (CD3OD) δ: 8.05 (dd, J=7.5, 2.0 Hz, 2H), 7.73 (t, J=7.7 Hz, 1H), 6.97 (dd, J=8.6, 5.9 Hz, 1H), 6.72 (dd, J=11.7, 8.6 Hz, 1H), 5.52-5.71 (m, 2H), 4.72-4.79 (m, 1H), 3.54-3.61 (m, 1H), 2.17 (s, 3H), 2.16 (s, 3H), 1.46 (d, J=7.0 Hz, 3H); LC/MS RT 1.65min, m/z $[M-H]^-$ 460 |
| 176 | 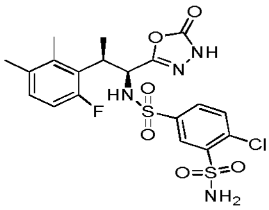 | 1H NMR (CD3OD) δ: 8.43 (d, J=2.2 Hz, 1H), 7.88-7.95 (m, 1H), 7.72 (d, J=8.1 Hz, 1H), 6.98 (dd, J=8.4, 5.9 Hz, 1H), 6.73 (dd, J=11.4, 8.4 Hz, 1H), 4.23 (d, J=10.6 Hz, 1H), 3.43-3.55 (m, 1H), 2.25 (s, 3H), 2.20 (s, 3H), 1.46 (d, J=7.0 Hz, 3H); LC/MS RT 1.61min, m/z $[M-H]^-$ 517,519 |
| 177 | 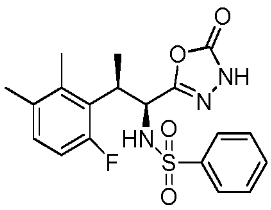 | 1H NMR (cdcl3) δ: 8.27 (brs, 1H), 7.77-7.89 (m, 2H), 7.40-7.59 (m, 3H), 6.94 (dd, J=8.3, 5.7 Hz, 1H), 6.71 (dd, J=11.7, 8.3 Hz, 1H), 5.31 (br d, J=10.3 Hz, 1H), 4.82 (t, J=10.3 Hz, 1H), 3.36-3.47 (m, 1H), 2.17 (s, 3H), 2.15 (s, 3H), 1.43 (d, J=5.9 Hz, 3H); LC/MS RT 1.66min, m/z $[M-H]^-$ 404 |
| 178 | 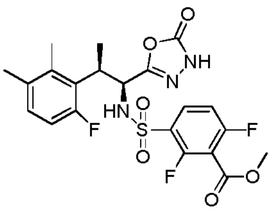 | LC/MS RT 1.75min, m/z $[M-H]^-$ 498 |
| 179 | 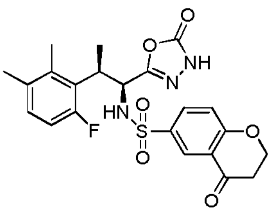 | LC/MS RT 1.64min, m/z $[M-H]^-$ 474 |

(continued)

| Example | Structural Formula | Physical Property Value |
|---|---|---|
| 180 | 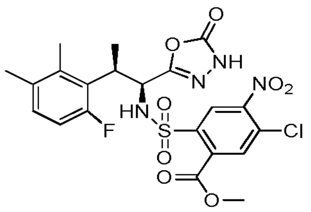 | LC/MS RT 1.90min, m/z [M-H]- 541,543 |
| 181 | 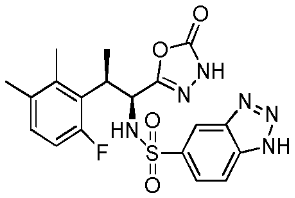 | 1H NMR (CD3OD) δ: 8.38 (s, 1H), 7.93 (d, J=9.0 Hz, 1H), 7.83 (dd, J=8.8, 1.8 Hz, 1H), 6.95 (dd, J=8.3, 5.7 Hz, 1H), 6.70 (dd, J=11.7, 8.3Hz, 1H), 4.75 (d, J=11.0 Hz, 1H), 3.50-3.65 (m, 1H), 2.16 (s, 3H), 2.14 (s, 3H), 1.45 (d, J=7.0 Hz, 3H); LC/MS RT 1.50min, m/z [M-H]- 445 |
| 182 | 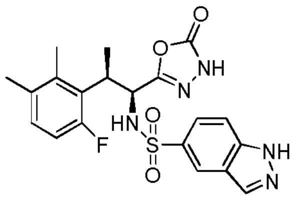 | 1H NMR (CD3OD) δ: 8.28 (s, 1H), 8.19 (s, 1H), 7.73 (dd, J=8.8, 1.5 Hz, 1H), 7.61 (d, J=8.8 Hz, 1H), 6.94 (dd, J=8.4, 5.9 Hz, 1H), 6.69 (dd, J=11.7, 8.4 Hz, 1H), 4.72 (d, J=11.4 Hz, 1H), 3.47-3.65 (m, 1H), 2.16 (s, 3H), 2.14 (s, 3H), 1.43 (d, J=7.0 Hz, 3H); LC/MS RT 1.53min, m/z [M-H]- 444 |
| 183 | 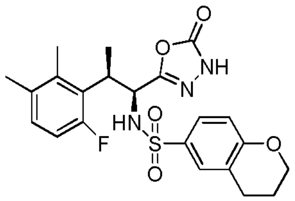 | 1H NMR (CD3OD) δ: 7.40-7.48 (m, 2H), 6.97 (dd, J=8.4, 5.9 Hz, 1H), 6.69-6.79 (m, 2H), 4.65 (d, J=11.4 Hz, 1H), 4.16-4.22 (m, 2H), 3.48-3.60 (m, 1H), 2.78 (t, J=6.2 Hz, 2H), 2.17 (s, 6H), 1.94-2.04 (m, 2H), 1.45 (d, J=7.0 Hz, 3H); LC/MS RT 1.72min, m/z [M-H]- 460 |
| 184 | 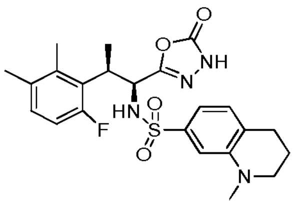 | 1H NMR (CD3OD) δ: 6.84-7.00 (m, 4H), 6.71 (dd, J=11.7, 8.4 Hz, 1H), 4.66 (d, J=11.4 Hz, 1H), 3.47-3.56 (m, 1H), 3.20-3.28 (m, 2H), 2.89 (s, 3H), 2.72 (t, J=6.4 Hz, 2H), 2.15-2.18 (m, 6H), 1.85-1.99 (m, 2H), 1.44 (d, J=7.0 Hz, 3H); LC/MS RT 1.73min, m/z [M-H]- 473 |
| 185 | 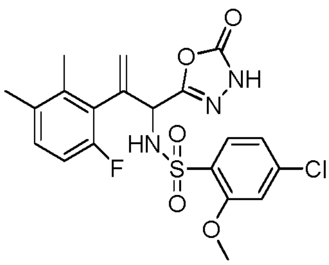 | 1H-NMR (CDCl3) δ: 8.47 (1H, br s), 7.70 (1H, d, J = 8.8 Hz), 7.02 (1H, dd, J = 8.2, 6.0 Hz), 6.97 (1H, dd, J = 8.4, 1.8 Hz), 6.90-6.89 (1H, m), 6.72 (1H, t, J = 8.8 Hz), 5.74-5.73 (1H, m), 5.67 (1H, br s), 5.30-5.30 (1H, m), 5.14 (1H, d, J = 9.2 Hz), 3.92 (3H, s), 2.19 (3H, s), 2.11 (3H, s).; LC/MS RT 1.80min, m/z [M-H]- 466,468 |
| 186 | 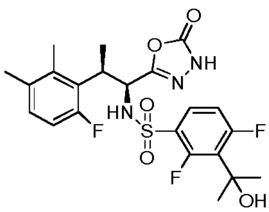 | 1H NMR (CD3OD) δ: 7.69-7.82 (m, 1H), 6.91-7.10 (m, 2H), 6.71 (dd, J=11.7, 8.4 Hz, 1H), 4.74 (d, J=11.0 Hz, 1H), 3.59-3.71 (m, 1H), 2.21 (s, 3H), 2.18 (s, 3H), 1.66 (s, 6H), 1.46 (d, J=6.6 Hz, 3H); LC/MS RT 1.68min, m/z [M-H]- 498 |

(continued)

| Example | Structural Formula | Physical Property Value |
|---|---|---|
| 187 | 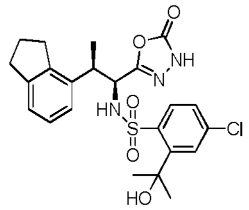 | 1H NMR (CD3OD) δ: 7.94 (d, J=8.8 Hz, 1H), 7.49 (d, J=2.2 Hz, 1H), 7.32 (d, J=8.8 Hz, 1H), 6.98-7.07 (m, 2H), 6.90-6.98 (m, 1H), 4.43 (d, J=10.3 Hz, 1H), 3.19-3.27 (m, 1H), 2.74-2.91 (m, 4H), 1.90-2.08 (m, 2H), 1.71 (s, 3H), 1.65 (s, 3H), 1.42 (d, J=7.0 Hz, 3H); LC/MS RT 1.93min, m/z [M-H]- 490,492 |
| 188 | 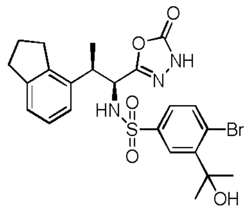 | 1H NMR (CD3OD) δ: 8.29 (d, J=2.6 Hz, 1H), 7.67 (d, J=8.1 Hz, 1H), 7.45 (dd, J=8.2, 2.4 Hz, 1H), 6.90-7.10 (m, 3H), 4.34 (d, J=10.6 Hz, 1H), 3.24-3.31 (m, 1H), 2.79-2.84 (m, 4H), 1.90-2.09 (m, 2H), 1.70 (s, 6H), 1.39 (d, J=7.0 Hz, 3H); LC/MS RT 1.8min, m/z [M-H]- 534,536 |
| 189 | 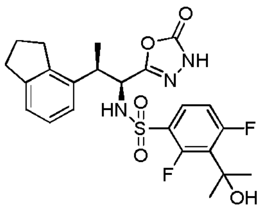 | 1H NMR (CD3OD) δ: 7.69-7.76 (m, 1H), 6.89-7.14 (m, 4H), 4.39 (d, J=11.0 Hz, 1H), 3.24-3.31 (m, 1H), 2.77-2.97 (m, 4H), 1.91-2.07 (m, 2H), 1.67 (s, 6H), 1.44 (d, J=7.0 Hz, 3H); LC/MS RT 1.7min, m/z [M-H]- 492 |
| 190 | 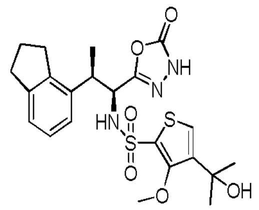 | 1H NMR (CD3OD) δ: 7.37-7.50 (m, 1H), 7.03 (s, 2H), 6.91-6.99 (m, 1H), 4.41 (d, J=10.6 Hz, 1H), 4.06 (s, 3H), 3.32-3.36 (m, 1H), 2.77-2.96 (m, 4H), 1.93-2.06 (m, 2H), 1.54 (s, 3H), 1.50 (s, 3H), 1.35 (d, J=7.0 Hz, 3H); LC/MS RT 1.67min, m/z [M-H]- 492 |
| 191 | 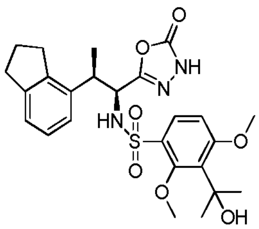 | 1H NMR (CD3OD) δ: 7.76 (d, J=9.2 Hz, 1H), 6.97-7.08 (m, 2H), 6.81-6.95 (m, 2H), 4.32 (d, J=11.0 Hz, 1H), 3.91 (s, 6H), 3.33-3.40 (m, 1H), 2.70-2.98 (m, 4H), 1.91-2.13 (m, 2H), 1.66 (s, 3H), 1.65 (s, 3H), 1.41 (d, J=7.0 Hz, 3H); LC/MS RT 1.78min, m/z [M-H]- 516 |
| 192 | 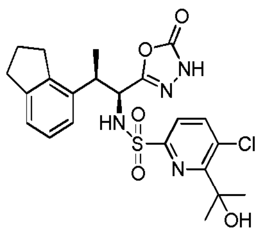 | 1H NMR (CD3OD) δ: 8.45-8.43 (2H, m), 8.02 (1H, d, J = 8.1 Hz), 7.82 (1H, d, J = 8.1 Hz), 7.04-7.02 (2H, m), 6.98-6.95 (1H, m), 4.63 (1H, s), 4.51 (1H, d, J = 11.0 Hz), 2.92-2.82 (4H, m), 2.05-1.97 (2H, m), 1.69 (3H, s), 1.68 (3H, s), 1.42 (3H, d, J = 7.0 Hz); LC/MS RT 1.74min, m/z [M-H]- 491,493 |
| 193 | 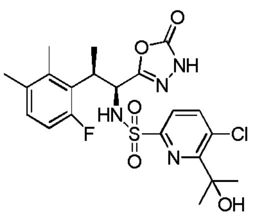 | 1H NMR (CD3OD) δ: 8.04 (d, J=8.3 Hz, 1H), 8.02 (d, J=8.3 Hz, 1H), 6.98 (dd, J=8.4, 5.9 Hz, 1H), 6.71 (dd, J=11.7, 8.4 Hz, 1H), 4.83 (d, J=11.4 Hz, 1H), 3.55-3.68 (m, 1H), 2.23 (s, 3H), 2.18 (s, 3H), 1.67 (d, J=6.6 Hz, 6H), 1.46 (d, J=7.0 Hz, 3H); LC/MS RT 1.71min, m/z [M-H]- 497,499 |

(continued)

| Example | Structural Formula | Physical Property Value |
|---|---|---|
| 194 | 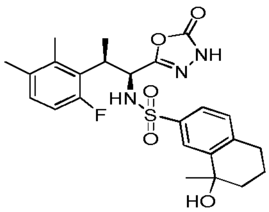 | 1H NMR (CD3OD) δ: 8.01-8.15 (m, 1H), 7.46-7.69 (m, 1H), 7.14 (d, J=8.1 Hz, 1H), 6.93-7.01 (m, 1H), 6.66-6.76 (m, 1H), 4.69 (d, J=11.4 Hz, 1H), 3.47-3.61 (m, 1H), 2.72-2.88 (m, 2H), 2.07-2.25 (m, 6H), 1.76-1.94 (m, 4H), 1.39-1.52 (m, 6H); LC/MS RT 1.66min, m/z [M-H]- 488 |
| 195 | 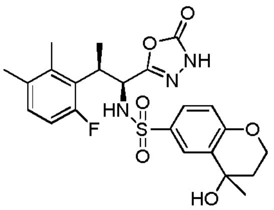 | 1H NMR (CD3OD) δ: 7.96 (dd, J=6.8, 2.4 Hz, 1H), 7.50 (dt, J=8.7, 2.6 Hz, 1H), 6.97 (dd, J=8.4, 5.9 Hz, 1H), 6.77-6.83 (m, 1H), 6.68-6.76 (m, 1H), 4.67 (dd, J=11.2, 3.1 Hz, 1H), 4.29-4.38 (m, 1H), 4.21-4.28 (m, 1H), 3.47-3.62 (m, 1H), 2.13-2.24 (m, 6H), 2.02-2.11 (m, 2H), 1.58 (d, J=11.0 Hz, 3H), 1.39-1.49 (m, 3H); LC/MS RT 1.60min, m/z [M-H]- 490 |
| 196 | 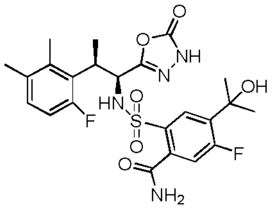 | 1H NMR (CD3OD) δ: 8.25 (d, J=7.3 Hz, 1H), 7.31 (d, J=11.0 Hz, 1H), 6.97 (dd, J=8.4, 5.9 Hz, 1H), 6.71 (dd, J=11.7, 8.4 Hz, 1H), 4.79 (d, J=11.0 Hz, 1H), 3.51-3.62 (m, 1H), 2.21 (s, 3H), 2.17 (s, 3H), 1.57 (s, 6H), 1.46 (d, J=7.0 Hz, 3H); LC/MS RT 1.56min, m/z [M-H]- 523 |
| 197 | 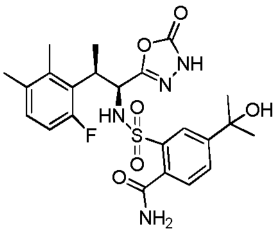 | 1H NMR (CD3OD) δ: 8.02 (d, J=1.8 Hz, 1H), 7.74 (dd, J=8.1, 1.8 Hz, 1H), 7.58 (d, J=8.1 Hz, 1H), 6.97 (dd, J=8.3, 5.7 Hz, 1H), 6.71 (dd, J=11.7, 8.3 Hz, 1H), 4.80 (d, J=11.0 Hz, 1H), 3.49-3.62 (m, 1H), 2.20 (s, 3H), 2.17 (s, 3H), 1.53 (s, 6H), 1.44 (d, J=6.6 Hz, 3H); LC/MS RT 1.52min, m/z [M-H]- 505 |
| 198 | 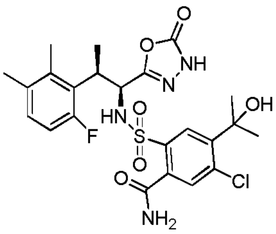 | 1H NMR (CD3OD) δ: 8.41 (s, 1H), 7.60 (s, 1H), 6.97 (dd, J=8.3, 6.0 Hz, 1H), 6.71 (dd, J=11.7, 8.3 Hz, 1H), 4.80 (d, J=11.0 Hz, 1H), 3.52-3.64 (m, 1H), 2.20 (s, 3H), 2.17 (s, 3H), 1.68 (s, 6H), 1.45 (d, J=7.0 Hz, 3H); LC/MS RT 1.61min, m/z [M-H]- 539,541 |
| 199 | 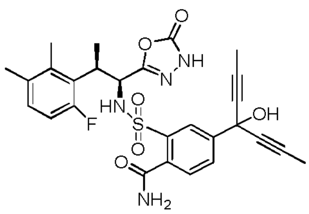 | 1H NMR (CD3OD) δ: 8.25 (d, J=1.8 Hz, 1H), 7.95 (dd, J=7.9, 2.0 Hz, 1H), 7.61 (d, J=8.1 Hz, 1H), 6.93-7.02 (m, 1H), 6.66-6.75 (m, 1H), 4.75-4.85 (m, 1H), 3.52-3.62 (m, 1H), 2.20 (s, 3H), 2.17 (s, 3H), 1.88 (s, 6H), 1.40 (d, J=6.2 Hz, 3H); LC/MS RT 1.63min, m/z [M-H]- 553 |
| 200A | 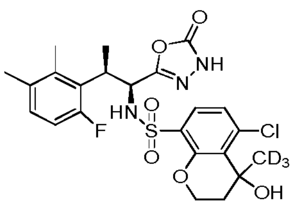 | 1H NMR (CD3OD) δ: 7.61 (d, J=8.4 Hz, 1H), 7.02 (d, J=8.5 Hz, 1H), 6.96 (dd, J=8.2, 5.7 Hz, 1H), 6.70 (dd, J=11.7, 8.4 Hz, 1H), 4.71 (d, J=11.4 Hz, 1H), 4.40-4.46 (m, 1H), 4.26 (td, J=10.8, 2.6 Hz, 1H), 3.62-3.71 (m, 1H), 2.23 (s, 3H), 2.17 (s, 3H), 2.05-2.13 (m, 2H), 1.50 (d, J=7.0 Hz, 3H); LC/MS RT 1.64min, m/z [M-H]- 527,529 |

(continued)

| Example | Structural Formula | Physical Property Value |
|---|---|---|
| 200B | 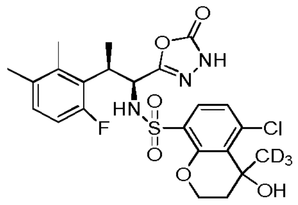 | 1H NMR (CD3OD) δ: 7.59 (d, J=8.8 Hz, 1H), 7.02 (d, J=8.4 Hz, 1H), 6.97 (dd, J=8.4, 5.9 Hz, 1H), 6.71 (dd, J=11.7, 8.4 Hz, 1H), 4.75 (d, J=11.4 Hz, 1H), 4.41-4.48 (m, 1H), 4.32 (td, J=10.7, 2.7 Hz, 1H), 3.63-3.73 (m, 1H), 2.22 (s, 3H), 2.18 (s, 3H), 2.05-2.13 (m, 2H), 1.48 (d, J=7.0 Hz, 3H); LC/MS RT 1.71min, m/z $[M-H]^-$ 527,529 |
| 201 | 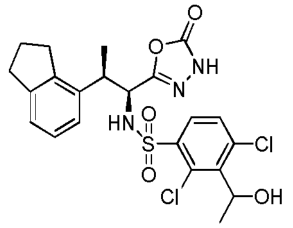 | 1H NMR (CD3OD) δ: 7.90 (dd, J=8.6, 2.7 Hz, 1H), 7.47 (dd, J=8.4, 2.2 Hz, 1H), 6.98-7.05 (m, 2H), 6.91-6.95 (m, 1H), 5.67 (dd, J=6.8, 5.3 Hz, 1H), 4.34 (d, J=11.0 Hz, 1H), 3.39-3.48 (m, 1H), 2.75-2.95 (m, 4H), 1.92-2.05 (m, 2H), 1.57 (d, J=7.0 Hz, 3H), 1.44 (dd, J=6.6, 1.5 Hz, 3H); LC/MS RT 1.74min, m/z $[M-H]^-$ 510,512 |
| 202 | 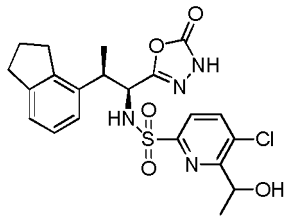 | 1H NMR (CD3OD) δ: 7.98 (d, J=8.4 Hz, 1H), 7.79 (d, J=8.8 Hz, 1H), 7.00-7.04 (m, 2H), 6.93-6.98 (m 1H), 5.19-5.29 (m, 1H), 4.48-4.54 (m, 1H), 3.33-3.42 (m, 1H), 2.70-3.01 (m, 4H), 1.97-2.10 (m, 2H), 1.38-1.52 (m, 6H); LC/MS RT 1.66min, m/z $[M-H]^-$ 477,479 |
| 203 | 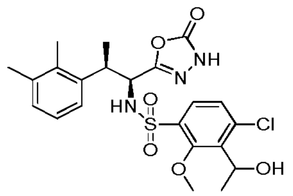 | 1H NMR (CD3OD) δ: 7.64-7.72 (m, 1H), 7.27 (dd, J=8.4, 1.5 Hz, 1H), 6.95-7.02 (m, 2H), 6.90-6.94 (m, 1H), 5.34-5.48 (m, 1H), 4.38 (d, J=11.0 Hz, 1H), 4.00 (s, 3H), 3.60-3.76 (m, 1H), 2.20 (s, 3H), 2.19 (s, 3H), 1.57-1.65 (m, 3H), 1.37 (d, J=7.0 Hz, 3H); LC/MS RT 1.68, 1.74min, m/z $[M-H]^-$ 494,496 |
| 204 | 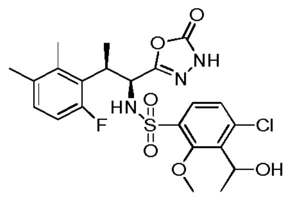 | 1H NMR (CD3OD) δ: 7.69 (dd, J=8.4, 7.0 Hz, 1H), 7.28 (dd, J=8.8, 2.6 Hz, 1H), 6.96 (dd, J=8.5, 5.7 Hz, 1H), 6.69 (dd, J=11.7, 8.5 Hz, 1H), 5.30-5.52 (m, 1H), 4.70-4.77 (m, 1H), 4.01 (d, J=1.5 Hz, 3H), 3.61-3.74 (m, 1H), 2.21 (s, 3H), 2.17 (s, 3H), 1.57-1.64 (m, 3H), 1.41-1.48 (m, 3H); LC/MS RT 1.70, 1.75min, m/z $[M-H]^-$ 512,514 |
| 205 | 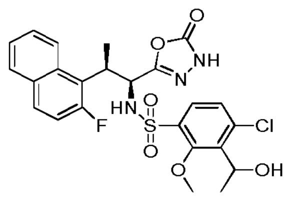 | 1H NMR (CD3OD) δ: 8.09-8.21 (m, 1H), 7.74-7.92 (m, 2H), 7.68-7.73 (m, 1H), 7.39-7.64 (m, 2H), 7.24-7.30 (m, 1H), 7.15-7.23 (m, 1H), 5.30-5.55 (m, 1H), 4.16-4.35 (m, 1H), 4.03 (d, J=2.6 Hz, 3H), 3.80 (d, J=9.5 Hz, 1H), 1.55-1.67 (m, 3H), 1.27-1.41 (m, 3H); LC/MS RT 1.72 1.77min, m/z $[M-H]^-$ 534,536 |
| 206A | 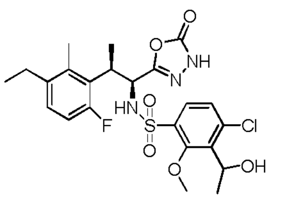 | 1H NMR (CD3OD) δ: 7.69 (d, J=8.5 Hz, 1H), 7.28 (d, J=8.5 Hz, 1H), 6.97 (dd, J=8.4, 5.7 Hz, 1H), 6.73 (dd, J=11.7, 8.3 Hz, 1H), 5.40-5.48 (m, 1H), 4.72 (d, J=11.5 Hz, 1H), 4.01 (s, 3H), 3.64-3.75 (m, 1H), 2.51-2.60 (m, 2H), 2.25 (s, 3H), 1.62 (d, J=6.8 Hz, 3H), 1.46 (d, J=6.1 Hz, 3H), 1.06 (t, J=7.4 Hz, 3H); LC/MS RT 1.78min, m/z $[M-H]^-$ 526,528 |

(continued)

| Example | Structural Formula | Physical Property Value |
|---|---|---|
| 207A | 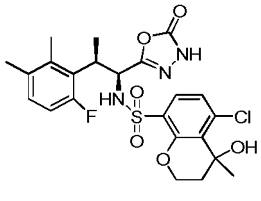 | 1H-NMR (CDCl3) δ: 7.78 (1H, br s), 7.67 (1H, d, J = 8.4 Hz), 6.99 (1H, d, J = 8.4 Hz), 6.93 (1H, dd, J = 8.3, 5.9 Hz), 6.70 (1H, dd, J = 11.5, 8.3 Hz), 5.41 (1H, d, J = 10.4 Hz), 4.89 (1H, t, J = 10.4 Hz), 4.49-4.44 (1H, m), 4.32-4.25 (1H, m), 3.46 (1H, br s), 3.25 (1H, s), 2.36-2.29 (1H, m), 2.19 (3H, s), 2.18 (3H, s), 2.10-2.05 (1H, m), 1.79 (3H, s), 1.55-1.53 (3H, m); LC/MS RT 1.64min, m/z $[M-H]^-$ 524,526 |
| 207B | 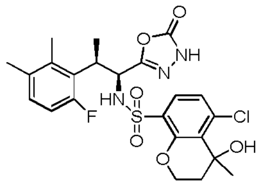 | 1H-NMR (CDCl3) δ: 8.41 (1H, s), 7.64 (1H, d, J = 8.4 Hz), 7.00 (1H, d, J = 8.4 Hz), 6.93 (1H, dd, J = 8.2, 5.9 Hz), 6.70 (1H, dd, J = 11.5, 8.2 Hz), 5.40 (1H, d, J = 11.0 Hz), 4.85 (1H, t, J = 11.0 Hz), 4.45-4.44 (1H, m), 4.33-4.30 (1H, m), 3.48 (1H, s), 3.40 (1H, s), 2.32-2.30 (1H, m), 2.19-2.16 (6H, m), 2.14-2.12 (1H, m), 1.78 (3H, s), 1.57-1.55 (3H, m); LC/MS RT 1.71min, m/z $[M-H]^-$ 524,526 |
| 208A | 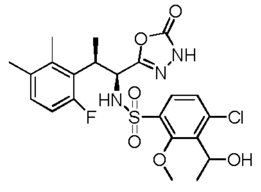 | 1H NMR (cd3od) δ: 7.75 (d, J=8.4 Hz, 1H), 7.33 (d, J=8.4 Hz, 1H), 6.96 (dd, J=8.3, 6.0 Hz, 1H), 6.69 (dd, J=11.7, 8.3 Hz, 1H), 5.49 (q, J=6.6 Hz, 1H), 4.73 (d, J=11.4 Hz, 1H), 4.00 (s, 3H), 3.60-3.74 (m, 1H), 2.21 (s, 3H), 2.17 (s, 3H), 1.66 (d, J=7.0 Hz, 3H), 1.44 (d, J=7.0 Hz, 3H); LC/MS RT 1.70min, m/z $[M-H]^-$ 512,514 |
| 208B | 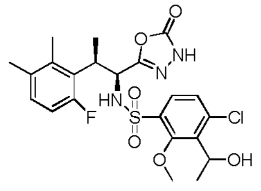 | 1H NMR (CD3OD) δ: 7.70 (d, J=8.8 Hz, 1H), 7.30 (d, J=8.4 Hz, 1H), 6.96 (dd, J=8.3, 6.0 Hz, 1H), 6.69 (dd, J=11.7, 8.3 Hz, 1H), 5.38 (q, J=6.6 Hz, 1H), 4.73 (d, J=11.4 Hz, 1H), 4.00 (s, 3H), 3.60-3.74 (m, 1H), 2.21 (s, 3H), 2.17 (s, 3H), 1.58 (d, J=7.0 Hz, 3H), 1.44 (d, J=7.0 Hz, 3H); LC/MS RT 1.76min, m/z $[M-H]^-$ 512,514 |
| 209A | 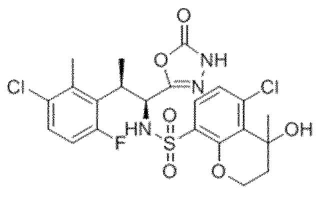 | 1H-NMR (CDCl3) δ: 8.15 (1H, s), 7.66 (1H, d, J = 8.4 Hz), 7.19 (1H, dd, J = 8.6, 5.1 Hz), 6.99 (1H, d, J = 8.6 Hz), 6.78 (1H, dd, J = 10.8, 9.0 Hz), 5.52 (1H, d, J = 11.0 Hz), 4.87 (1H, t, J = 10.4 Hz), 4.47-4.44 (1H, m), 4.28-4.25 (1H, m), 3.48 (1H, s), 3.29 (1H, s), 2.37 (3H, s), 2.32-2.28 (1H, m), 2.09-2.06 (1H, m), 1.78 (3H, s), 1.54 (3H, d, J = 7.0 Hz).; LC/MS RT 1.68min, m/z $[M-H]^-$ 544,546 |
| 209B | 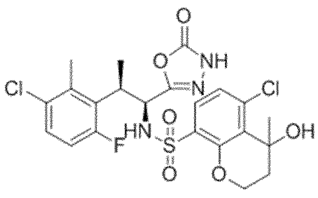 | 1H-NMR (CDCl3) δ: 8.68 (1H, br s), 7.64 (1H, d, J = 8.8 Hz), 7.19 (1H, dd, J = 8.8, 4.9 Hz), 7.01 (1H, d, J = 8.8 Hz), 6.78 (1H, dd, J = 10.8, 8.8 Hz), 5.47-5.42 (1H, m), 4.81 (1H, t, J = 10.9 Hz), 4.45-4.42 (1H, m), 4.32 (1H, t, J = 10.9 Hz), 3.53 (1H, br s), 3.40 (1H, br s), 2.35 (3H, s), 2.33-2.27 (1H, m), 2.15-2.10 (1H, m), 1.78 (3H, s), 1.59-1.58 (3H, m).; LC/MS RT 1.74min, m/z $[M-H]^-$ 544,546 |
| 210 | 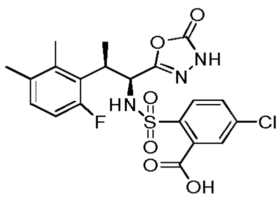 | LC/MS RT 1.66min, m/z $[M-H]^-$ 482,484 |

(continued)

| Example | Structural Formula | Physical Property Value |
|---|---|---|
| 211A | 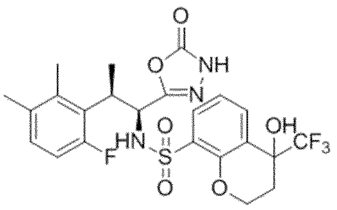 | 1H-NMR (CDCl3) δ: 8.04 (1H, s), 7.86 (1H, dd, J = 8.2, 1.5 Hz), 7.77 (1H, d, J = 8.2 Hz), 7.03 (1H, t, J = 8.2 Hz), 6.92 (1H, dd, J = 8.3, 5.9 Hz), 6.68 (1H, dd, J = 11.5, 8.3 Hz), 5.58 (1H, d, J = 10.2 Hz), 4.86 (1H, t, J = 10.2 Hz), 4.48-4.43 (2H, m), 3.60 (1H, s), 3.25 (1H, s), 2.40-2.33 (1H, m), 2.24-2.20 (1H, m), 2.20 (3H, s), 2.18 (3H, s), 1.53 (3H, d, J = 7.0 Hz).; LC/MS RT 1.67min, m/z [M-H]- |
| 212 | 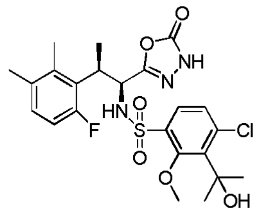 | 1H NMR (CD3OD) δ: 7.68 (d, J=8.4 Hz, 1H), 7.31 (d, J=8.4 Hz, 1H), 6.94-7.06 (m, 1H), 6.67-6.76 (m, 1H), 4.73-4.80 (m, 1H), 3.92 (s, 3H), 3.67-3.77 (m, 1H), 2.22 (s, 3H), 2.17 (s, 3H), 1.83 (s, 3H), 1.78 (s, 3H), 1.47 (d, J=7.0 Hz, 3H); LC/MS RT 1.84min, m/z [M-H]- 526,528 |
| 213 | 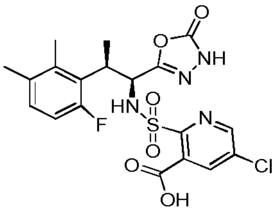 | LC/MS RT 1.47min, m/z [M-H]- 483,485 |
| 214 | 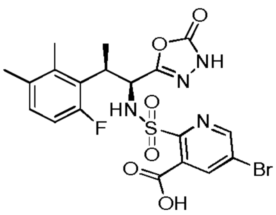 | LC/MS RT 1.49min, m/z [M-H]- 527,529 |
| 215 | 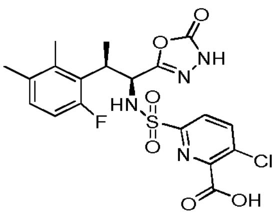 | LC/MS RT 1.49min, m/z [M-H]- 483,485 |
| 216 | 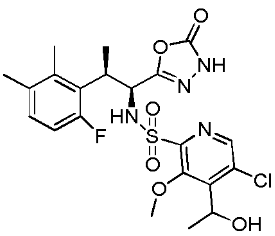 | 1H NMR (CD3OD) δ: 8.19 (d, J=5.9 Hz, 1H), 6.98 (dd, J=8.1, 5.9 Hz, 1H), 6.71 (dd, J=11.7, 8.4 Hz, 1H), 5.35-5.44 (m, 1H), 4.86-4.91 (m, 1H), 4.03-4.06 (m, 3H), 3.66-3.77 (m, 1H), 2.27 (s, 3H), 2.20 (s, 3H), 1.56-1.63 (m, 3H), 1.51 (d, J=6.6 Hz, 3H); LC/MS RT 1.62min, m/z [M-H]- 513,515 |
| 217 | 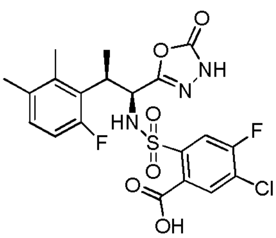 | LC/MS RT 1.73min, m/z [M-H]- 500,502 |

(continued)

| Example | Structural Formula | Physical Property Value |
|---|---|---|
| 218 | 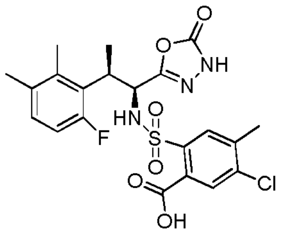 | LC/MS RT 1.74min, m/z $[M-H]^-$ 496,498 |
| 219 | 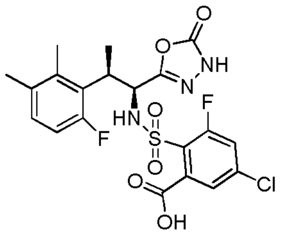 | LC/MS RT 1.59min, m/z $[M-H]^-$ 500,502 |
| 220A | 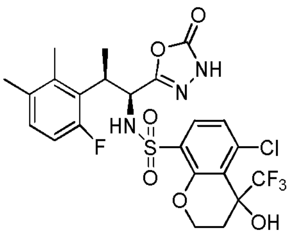 | 1H-NMR (CDCl3) δ: 8.07 (1H, s), 7.86 (1H, d, J = 8.8 Hz), 7.07 (1H, d, J = 8.8 Hz), 6.94 (1H, dd, J = 8.3, 5.7 Hz), 6.70 (1H, dd, J = 11.7, 8.3 Hz), 5.49 (1H, d, J = 9.9 Hz), 4.86 (1H, t, J = 10.1 Hz), 4.74 (1H, s), 4.61-4.51 (1H, m), 4.39 (1H, t, J = 12.5 Hz), 3.49 (1H, s), 2.57-2.53 (1H, m), 2.36-2.34 (1H, m), 2.20-2.17 (6H, m), 1.52 (3H, d, J = 7.0 Hz).; LC/MS RT 1.74min, m/z $[M-H]^-$ 578,580 |
| 220B | 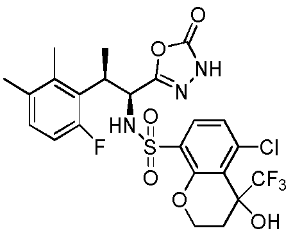 | 1H-NMR (CDCl3) δ: 8.26 (1H, s), 7.79 (1H, d, J = 8.6 Hz), 7.06 (1H, d, J = 8.6 Hz), 6.96-6.93 (1H, m), 6.73-6.68 (1H, m), 5.40 (1H, d, J = 10.6 Hz), 4.86 (1H, t, J = 10.6 Hz), 4.53-4.46 (3H, m), 3.41 (1H, s), 2.61-2.57 (1H, m), 2.37-2.34 (1H, m), 2.19-2.16 (6H, m), 1.52 (3H, d, J = 7.0 Hz).; LC/MS RT 1.80min, m/z $[M-H]^-$ 578,580 |
| 221 | 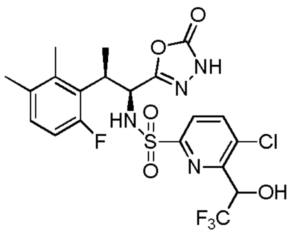 | 1H-NMR (CDCl3) δ: 8.51-8.51 (1H, m), 8.28-8.27 (1H, m), 6.93 (1H, dd, J = 8.4, 5.9 Hz), 6.71-6.64 (1H, m), 6.05 (1H, br s), 5.60-5.55 (1H, m), 5.03-4.98 (1H, m), 3.54 (1H, s), 2.17 (6H, d, J = 3.7 Hz), 1.49 (3H, d, J = 7.0 Hz); LC/MS RT 1.72min, m/z $[M-H]^-$ 537,539 |
| 222A | 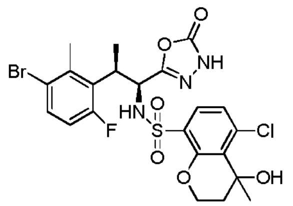 | 1H NMR (CD3OD) δ: 7.61 (d, J=8.4 Hz, 1H), 7.42 (dd, J=9.0, 5.3 Hz, 1H), 7.03 (d, J=8.4 Hz, 1H), 6.81 (dd, J=11.2, 9.0 Hz, 1H), 4.69 (d, J=11.4 Hz, 1H), 4.39-4.47 (m, 1H), 4.22-4.33 (m, 1H), 3.63-3.78 (m, 1H), 2.44 (s, 3H), 2.17-2.24 (m, 1H), 2.05-2.15 (m, 1H), 1.75 (s, 3H), 1.52 (d, J=6.6 Hz, 3H); LC/MS RT 1.70min, m/z $[M-H]^-$ 588,590 |

(continued)

| Example | Structural Formula | Physical Property Value |
|---|---|---|
| 222B | 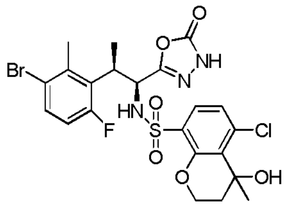 | 1H NMR (CD3OD) δ: 7.60 (d, J=8.6 Hz, 1H), 7.43 (dd, J=8.9, 5.1 Hz, 1H), 7.02 (d, J=8.6 Hz, 1H), 6.82 (dd, J=11.2, 8.9 Hz, 1H), 4.74 (d, J=11.4 Hz, 1H), 4.39-4.47 (m, 1H), 4.33 (td, J=10.8, 2.6 Hz, 1H), 3.65-3.77 (m, 1H), 2.44 (s, 3H), 2.22-2.31 (m, 1H), 2.05-2.12 (m, 1H), 1.75 (s, 3H), 1.50 (d, J=7.0 Hz, 3H); LC/MS RT 1.76min, m/z [M-H]$^-$ 588,590 |
| 223 | 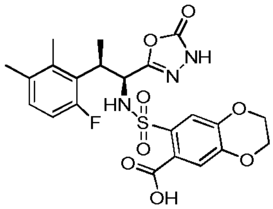 | LC/MS RT 1.61min, m/z [M-H]$^-$ 506 |
| 224A | 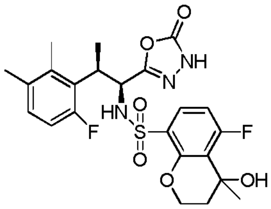 | 1H NMR (CD3OD) δ: 7.70 (dd, J=8.6, 5.9 Hz, 1H), 6.96 (dd, J=8.6, 5.9 Hz, 1H), 6.61-6.82 (m, 2H), 4.70 (d, J=11.4 Hz, 1H), 4.29-4.49 (m, 2H), 3.60-3.79 (m, 1H), 2.22 (s, 3H), 2.17 (s, 3H), 2.04-2.12 (m, 2H), 1.67 (d, J=1.8 Hz, 3H), 1.50 (d, J=7.0 Hz, 3H); LC/MS RT 1.6min, m/z [M-H]$^-$ 508 |
| 224B | 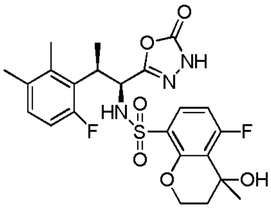 | 1H NMR (CD3OD) δ: 7.70 (dd, J=8.8, 5.9 Hz, 1H), 6.97 (dd, J=8.4, 5.9 Hz, 1H), 6.68-6.75 (m, 2H), 4.74 (d, J=11.4 Hz, 1H), 4.38 (t, J=5.5 Hz, 2H), 3.63-3.71 (m, 1H), 2.21 (s, 3H), 2.18 (s, 3H), 2.03-2.12 (m, 2H), 1.66 (d, J=1.8 Hz, 3H), 1.48 (d, J=7.0 Hz, 3H); LC/MS RT 1.65min, m/z [M-H]$^-$ 508 |
| 225A | 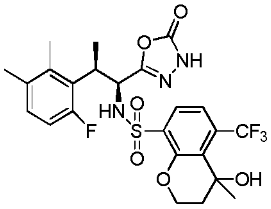 | 1H NMR (CD3OD) δ: 7.79 (d, J=8.4 Hz, 1H), 7.36 (d, J=8.8 Hz, 1H), 6.97 (dd, J=8.4, 5.9 Hz, 1H), 6.71 (dd, J=11.7, 8.4 Hz, 1H), 4.79 (d, J=11.4 Hz, 1H), 4.64-4.69 (m, 1H), 4.42-4.49 (m, 1H), 3.61-3.76 (m, 1H), 2.25-2.41 (m, 1H), 2.21 (s, 3H), 2.17 (s, 3H), 2.01-2.11 (m, 1H), 1.62 (s, 3H), 1.48 (d, J=7.0 Hz, 3H); LC/MS RT 1.72min, m/z [M-H]$^-$ 558 |
| 226A | 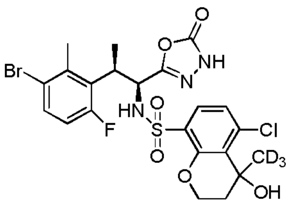 | 1H NMR (CD3OD) δ: 7.61 (d, J=8.6 Hz, 1H), 7.42 (dd, J=8.6, 5.1 Hz, 1H), 7.03 (d, J=8.6 Hz, 1H), 6.81 (dd, J=11.4, 8.6 Hz, 1H), 4.69 (d, J=11.0 Hz, 1H), 4.35-4.46 (m, 1H), 4.26 (td, J=10.9, 2.7 Hz, 1H), 3.66-3.75 (m, 1H), 2.44 (s, 3H), 2.15-2.26 (m, 1H), 2.05-2.13 (m, 1H), 1.52 (d, J=6.6 Hz, 3H); LC/MS RT 1.70min, m/z [M-H]$^-$ 591,593 |
| 226B | 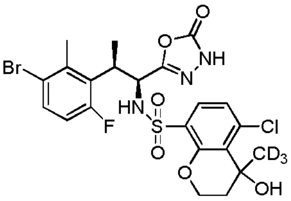 | LC/MS RT 1.76min, m/z [M-H]$^-$ 591,593 |

(continued)

| Example | Structural Formula | Physical Property Value |
|---|---|---|
| 227A | 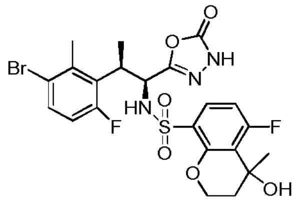 | 1H NMR (CD3OD) δ: 7.70 (dd, J=8.8, 5.9 Hz, 1H), 7.42 (dd, J=8.8, 5.1 Hz, 1H), 6.80 (dd, J=11.0, 9.0 Hz, 1H), 6.72 (dd, J=11.0, 9.0 Hz, 1H), 4.68 (d, J=11.4 Hz, 1H), 4.31-4.42 (m, 2H), 3.66-3.74 (m, 1H), 2.42 (s, 3H), 2.04-2.15 (m, 2H), 1.67 (d, J=1.8 Hz, 3H), 1.52 (d, J=7.0 Hz, 3H); LC/MS RT 1.66min, m/z [M-H]$^-$ 572,574 |
| 227B | 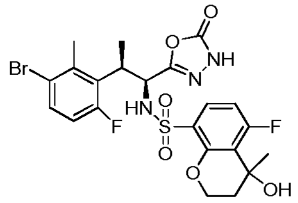 | 1H NMR (CD3OD) δ: 7.71 (dd, J=8.8, 5.9 Hz, 1H), 7.43 (dd, J=9.0, 5.3 Hz, 1H), 6.82 (dd, J=11.4, 8.8 Hz, 1H), 6.73 (dd, J=10.6, 8.8 Hz, 1H), 4.73 (d, J=11.4 Hz, 1H), 4.38 (t, J=5.5 Hz, 2H), 3.65-3.72 (m, 1H), 2.44 (s, 3H), 2.05-2.13 (m, 2H), 1.66 (d, J=1.8 Hz, 3H), 1.50 (d, J=6.6 Hz, 3H); LC/MS RT 1.71min, m/z [M-H]$^-$ 572,574 |
| 228A | 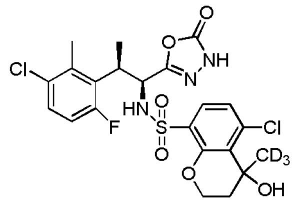 | 1H NMR (CD3OD) δ: 7.61 (d, J=8.4 Hz, 1H), 7.24 (dd, J=8.8, 5.1 Hz, 1H), 7.03 (d, J=8.8 Hz, 1H), 6.87 (dd, J=11.0, 8.8 Hz, 1H), 4.69 (d, J=11.4 Hz, 1H), 4.41-4.47 (m, 1H), 4.26 (td, J=10.9, 2.4 Hz, 1H), 3.65-3.72 (m, 1H), 2.39 (s, 3H), 2.15-2.25 (m, 1H), 2.05-2.13 (m, 1H), 1.52 (d, J=6.6 Hz, 3H); LC/MS RT 1.68min, m/z [M-H]$^-$ 547,549 |
| 228B | 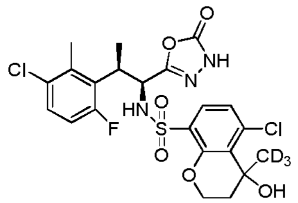 | 1H NMR (CD3OD) δ: 7.60 (d, J=8.4 Hz, 1H), 7.24 (dd, J=9.0, 4.9 Hz, 1H), 7.02 (d, J=8.4 Hz, 1H), 6.87 (dd, J=11.2, 9.0 Hz, 1H), 4.74 (d, J=11.4 Hz, 1H), 4.41-4.46 (m, 1H), 4.29-4.36 (m, 1H), 3.64-3.74 (m, 1H), 2.38 (s, 3H), 2.22-2.29 (m, 1H), 2.05-2.13 (m, 1H), 1.50 (d, J=7.0 Hz, 3H); LC/MS RT 1.74min, m/z [M-H]$^-$ 547,549 |
| 229A | 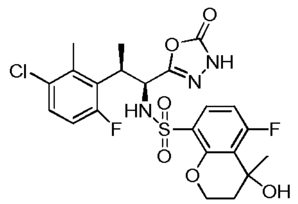 | 1H NMR (CD3OD) δ: 7.70 (dd, J=8.8, 5.9 Hz, 1H), 7.42 (dd, J=8.8, 5.1 Hz, 1H), 6.80 (dd, J=11.2, 9.0 Hz, 1H), 6.72 (dd, J=10.8, 9.0 Hz, 1H), 4.68 (d, J=11.4 Hz, 1H), 4.31-4.42 (m, 2H), 3.66-3.74 (m, 1H), 2.44 (s, 3H), 2.05-2.11 (m, 2H), 1.67 (d, J=1.8 Hz, 3H), 1.52 (d, J=7.0 Hz, 3H); LC/MS RT 1.64min, m/z [M-H]$^-$ 528,530 |
| 229B | 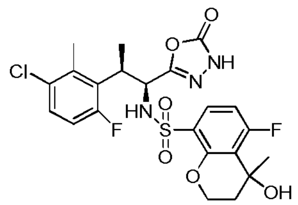 | 1H NMR (CD3OD) δ: 7.71 (dd, J=8.9, 5.9 Hz, 1H), 7.43 (dd, J=8.9, 5.3 Hz, 1H), 6.82 (dd, J=11.4, 8.8 Hz, 1H), 6.73 (dd, J=10.6, 8.8 Hz, 1H), 4.73 (d, J=11.4 Hz, 1H), 4.38 (t, J=5.5 Hz, 2H), 3.63-3.78 (m, 1H), 2.44 (s, 3H), 2.04-2.16 (m, 2H), 1.66 (d, J=1.8 Hz, 3H), 1.50 (d, J=6.6 Hz, 3H); LC/MS RT 1.69min, m/z [M-H]$^-$ 528,530 |
| 230A | 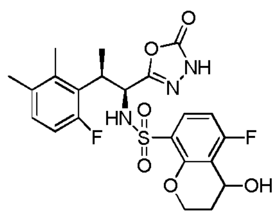 | 1H NMR (CD3OD) δ: 7.73 (dd, J=8.8, 6.2 Hz, 1H), 6.96 (dd, J=8.4, 5.9 Hz, 1H), 6.66-6.77 (m, 2H), 4.69 (d, J=11.4 Hz, 1H), 4.84-4.90 (m, 1H), 4.53-4.60 (m, 1H), 4.35 (ddd, J=13.1, 10.9, 2.4 Hz, 1H), 3.62-3.71 (m, 1H), 2.21 (s, 3H), 2.17 (s, 3H), 1.95-2.12 (m, 2H), 1.49 (d, J=7.0 Hz, 3H); LC/MS RT 1.57min, m/z [M-H]$^-$ 494 |

(continued)

| Example | Structural Formula | Physical Property Value |
|---|---|---|
| 230B | 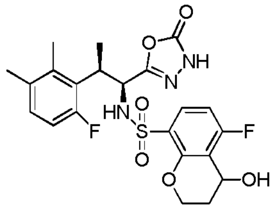 | 1H NMR (CD3OD) δ: 7.77 (dd, J=8.8, 6.2 Hz, 1H), 6.97 (dd, J=8.2, 5.7 Hz, 1H), 6.68-6.78 (m, 2H), 4.85-4.93 (m, 1H), 4.74 (d, J=11.4 Hz, 1H), 4.51-4.60 (m, 1H), 4.33 (td, J=11.5, 3.3 Hz, 1H), 3.62-3.71 (m, 1H), 2.22 (s, 3H), 2.18 (s, 3H), 1.96-2.09 (m, 2H), 1.47 (d, J=6.6 Hz, 3H); LC/MS RT 1.61min, m/z $[M-H]^-$ 494 |
| 231 | 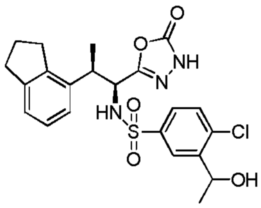 | 1H NMR (CD3OD) δ: 8.04-8.10 (m, 1H), 7.54-7.61 (m, 1H), 7.38-7.46 (m, 1H), 6.94-7.09 (m, 3H), 5.17 (q, J=6.5 Hz, 1H), 4.36 (dd, J=10.6, 5.1 Hz, 1H), 3.24-3.33 (m, 1H), 2.77-2.91 (m, 4H), 1.90-2.07 (m, 2H), 1.38-1.42 (m, 6H); LC/MS RT 1.72min, m/z $[M-H]^-$ 476,478 |
| 232 | 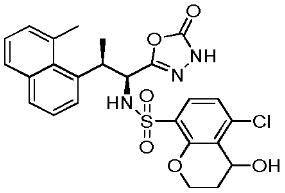 | 1H NMR (CD3OD) δ: 7.59-7.69 (m, 3H), 7.46 (dd, J=7.3, 1.1 Hz, 1H), 7.30-7.38 (m, 1H), 7.22-7.29 (m, 2H), 7.02 (d, J=8.4 Hz, 1H), 4.85-4.90 (m, 1H), 4.63-4.72 (m, 1H), 4.50-4.59 (m, 1H), 4.39 (d, J=10.6 Hz, 1H), 4.26-4.35 (m, 1H), 2.96 (s, 3H), 1.97-2.05 (m, 2H), 1.68 (d, J=6.6 Hz, 3H); LC/MS RT 1.65min, m/z $[M-H]^-$ 528,530 |
| 233 | 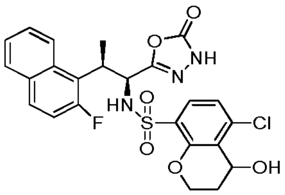 | 1H NMR (CD3OD) δ: 8.10 (d, J=8.4 Hz, 1H), 7.75-7.93 (m, 2H), 7.63-7.75 (m, 1H), 7.51-7.60 (m, 1H), 7.39-7.48 (m, 1H), 7.16-7.29 (m, 1H), 7.05 (t, J=9.0 Hz, 1H), 4.91-4.93 (m, 1H), 4.56-4.64 (m, 2H), 4.28-4.45 (m, 1H), 4.17-4.27 (m, 1H), 1.98-2.13 (m, 2H), 1.64 (d, J=6.6 Hz, 3H); LC/MS RT 1.63, 1.68min, m/z $[M-H]^-$ 532,534 |
| 234A | 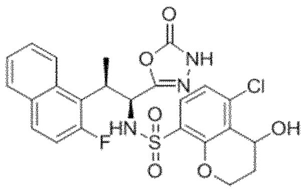 | 1H-NMR (CDCl3) δ: 7.99 (1H, d, J = 8.8 Hz), 7.78 (1H, d, J = 8.1 Hz), 7.72-7.68 (2H, m), 7.65 (1H, s), 7.56 (1H, t, J = 7.9 Hz), 7.43 (1H, t, J = 7.3 Hz), 7.18-7.12 (1H, m), 6.99 (1H, d, J = 8.4 Hz), 5.57 (1H, d, J = 10.6 Hz), 5.05-5.00 (1H, m), 4.93 (1H, s), 4.51-4.48 (1H, m), 4.40-4.33 (1H, m), 4.00 (1H, s), 2.47 532,534 2.16-2.07 (2H, m), 1.69-1.68 (3H, m).; LC/MS RT 1.63min, m/z $[M-H]^-$ |
| 235A | 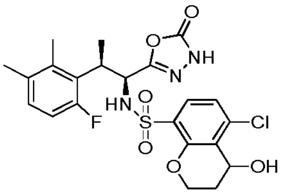 | 1H NMR (CD3OD) δ: 7.66 (d, J=8.4 Hz, 1H), 7.05 (d, J=8.4 Hz, 1H), 6.96 (dd, J=8.1, 5.5 Hz, 1H), 6.69 (dd, J=11.7, 8.4 Hz, 1H), 4.86-4.93 (m, 1H), 4.71 (d, J=11.4 Hz, 1H), 4.53-4.61 (m, 1H), 4.29-4.39 (m, 1H), 3.63-3.71 (m, 1H), 2.21 (s, 3H), 2.17 (s, 3H), 2.01-2.06 (m, 2H), 1.49 (d, J=7.0 Hz, 3H); LC/MS RT 1.61min, m/z $[M-H]^-$ 510,512 |
| 235B | 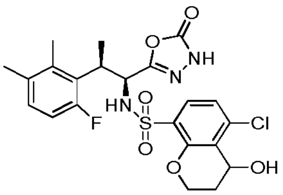 | 1H NMR (CD3OD) δ: 7.70 (d, J=8.4 Hz, 1H), 7.07 (d, J=8.4 Hz, 1H), 6.97 (dd, J=8.3, 5.7 Hz, 1H), 6.71 (dd, J=11.7, 8.3 Hz, 1H), 4.92-4.95 (m, 1H), 4.75 (d, J=11.4 Hz, 1H), 4.53-4.60 (m, 1H), 4.26-4.39 (m, 1H), 3.58-3.75 (m, 1H), 2.22 (s, 3H), 2.18 (s, 3H), 1.95-2.14 (m, 2H), 1.47 (d, J=7.0 Hz, 3H); LC/MS RT 1.67min, m/z $[M-H]^-$ 510,512 |

(continued)

| Example | Structural Formula | Physical Property Value |
|---|---|---|
| 236 | 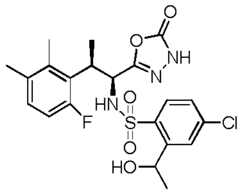 | 1H NMR (CD3OD) δ: 7.71-7.88 (m, 2H), 7.31-7.42 (m, 1H), 6.93-7.04 (m, 1H), 6.65-6.77 (m, 1H), 5.53-5.80 (m, 1H), 4.72-4.89 (m, 1H), 3.54-3.66 (m, 1H), 2.20 (d, J=2.2 Hz, 3H), 2.17 (s, 3H), 1.48 (d, J=7.0 Hz, 3H), 1.42-1.47 (m, 3H); LC/MS RT 1.70, 1.75min, m/z [M-H]- 482,484 |
| 237A | 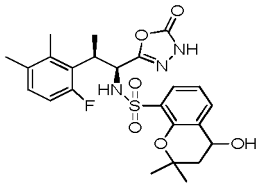 | 1H-NMR (CDCl3) δ: 7.72-7.69 (1H, m), 7.61-7.59 (1H, m), 7.45 (1H, s), 6.99-6.90 (2H, m), 6.70-6.65 (1H, m), 5.34-5.32 (1H, m), 4.89-4.81 (2H, m), 3.49 (1H, br s), 2.35 (1H, s), 2.25 (1H, dd, J = 14.4, 6.0 Hz), 2.19 (3H, s), 2.17 (3H, s), 1.97 (1H, dd, J = 14.4, 7.3 Hz), 1.55-1.54 (6H, m), 1.48 (3H, s).; LC/MS RT 1.66min, m/z [M-H]- 504,506 |
| 238A | 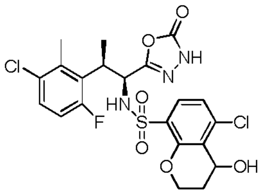 | 1H NMR (CD3OD) δ: 7.67 (d, J=8.5 Hz, 1H), 7.08-7.17 (m, 1H), 7.04-7.07 (m, 1H), 6.86 (dd, J=11.1, 8.9 Hz, 1H), 4.70 (d, J=11.5 Hz, 1H), 4.89-4.94 (m, 1H), 4.53-4.61 (m, 1H), 4.29-4.40 (m, 1H), 3.64-3.77 (m, 1H), 2.38 (s, 3H), 2.00-2.12 (m, 2H), 1.51 (d, J=7.6 Hz, 3H); LC/MS RT 1.65min, m/z [M-H]- 530,532 |
| 239A | 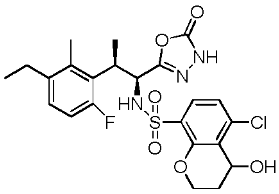 | 1H NMR (CD3OD) δ: 7.67 (d, J=8.4 Hz, 1H), 7.05 (d, J=8.4 Hz, 1H), 6.96 (dd, J=8.4, 5.9 Hz, 1H), 6.73 (dd, J=11.7, 8.4 Hz, 1H), 4.89-4.94 (m, 1H), 4.69 (d, J=11.4 Hz, 1H), 4.51-4.61 (m, 1H), 4.27-4.43 (m, 1H), 3.59-3.77 (m, 1H), 2.47-2.68 (m, 2H), 2.25 (s, 3H), 2.03-2.13 (m, 2H), 1.50 (d, J=7.0 Hz, 3H), 1.05 (t, J=7.5 Hz, 3H); LC/MS RT 1.7min, m/z [M-H]- 524,526 |
| 240 | 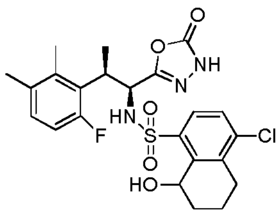 | 1H NMR (CD3OD) δ: 7.76 (d, J=8.4 Hz, 1H), 7.46 (d, J=8.8 Hz, 1H), 6.96 (dd, J=8.3, 6.0 Hz, 1H), 6.70 (dd, J=11.7, 8.3 Hz, 1H), 5.60 (t, J=3.1 Hz, 1H), 4.67 (d, J=11.0 Hz, 1H), 3.44-3.51 (m, 1H), 2.96-3.06 (m, 1H), 2.52-2.65 (m, 1H), 2.18 (s, 3H), 2.16 (s, 3H), 1.97-2.10 (m, 2H), 1.78-1.84 (m, 1H), 1.66-1.77 (m, 1H), 1.41 (d, J=7.0 Hz, 3H); LC/MS RT 1.89min, m/z [M-H]- 508,510 |
| 241 | 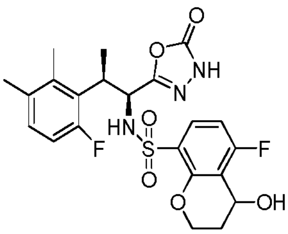 | 1H NMR (CD3OD) δ: 7.69-7.88 (m, 1H), 6.93-7.11 (m, 1H), 6.66-6.84 (m, 2H), 4.88-4.93 (m, 1H), 4.64-4.80 (m, 1H), 4.48-4.64 (m, 1H), 4.22-4.43 (m, 1H), 3.62-3.69 (m, 1H), 2.22 (s, 3H), 2.17 (s, 3H), 1.93-2.09 (2H, m), 1.44-1.52 (m, 3H); LC/MS RT 1.57, 1.61min, m/z [M-H]- 494 |
| 242 | 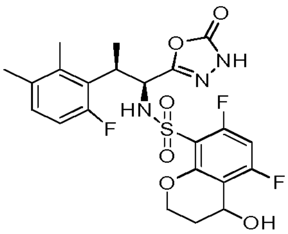 | 1H NMR (CD3OD) δ: 6.94-7.02 (m, 1H), 6.67-6.78 (m, 1H), 6.53-6.65 (m, 1H), 4.79-4.87 (m, 1H), 4.48-4.71 (m, 2H), 4.25-4.42 (m, 1H), 3.57-3.82 (m, 1H), 2.23 (s, 3H), 2.18 (s, 3H), 1.95-2.11 (m, 2H), 1.50 (d, J=6.2 Hz, 3H); LC/MS RT 1.59, 1.62min, m/z [M-H]- 512 |

(continued)

| Example | Structural Formula | Physical Property Value |
|---|---|---|
| 243A | 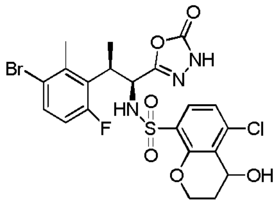 | 1H NMR (CD3OD) δ: 7.67 (d, J=8.4 Hz, 1H), 7.42 (dd, J=8.8, 5.1 Hz, 1H), 7.06 (d, J=8.4 Hz, 1H), 6.80 (dd, J=11.0, 8.8 Hz, 1H), 4.90-4.95 (m, 1H), 4.69 (d, J=11.4 Hz, 1H), 4.54-4.59 (m, 1H), 4.30-4.37 (m, 1H), 3.63-3.78 (m, 1H), 2.43 (s, 3H), 2.03-2.19 (m, 2H), 1.51 (d, J=7.0 Hz, 3H); LC/MS RT 1.67min, m/z [M-H]$^-$ 574,576 |
| 243B | 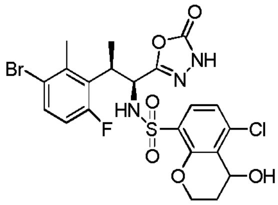 | 1H NMR (CD3OD) δ: 7.70 (d, J=8.7 Hz, 1H), 7.43 (dd, J=8.8, 5.1 Hz, 1H), 7.07 (d, J=8.7 Hz, 1H), 6.82 (dd, J=11.2, 8.8 Hz, 1H), 4.92-4.95 (m, 1H), 4.75 (d, J=11.4 Hz, 1H), 4.53-4.58 (m, 1H), 4.28-4.37 (m, 1H), 3.65-3.75 (m, 1H), 2.44 (s, 3H), 1.95-2.13 (m, 2H), 1.49 (d, J=7.0 Hz, 3H); LC/MS RT 1.72min, m/z [M-H]- 574,576 |
| 244A | 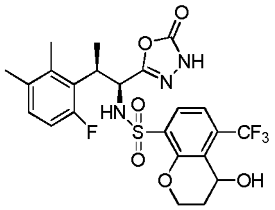 | 1H NMR (CD3OD) δ: 7.85 (d, J=8.3 Hz, 1H), 7.30 (d, J=8.3 Hz, 1H), 6.96 (dd, J=8.4, 5.9 Hz, 1H), 6.69 (dd, J=11.7, 8.4 Hz, 1H), 5.00-5.04 (m, 1H), 4.74 (d, J=11.4 Hz, 1H), 4.59-4.68 (m, 1H), 4.49-4.56 (m, 1H), 3.64-3.72 (m, 1H), 2.21 (s, 3H), 2.17 (s, 3H), 2.00-2.13 (m, 2H), 1.51 (d, J=6.6 Hz, 3H); LC/MS RT 1.7min, m/z [M-H]$^-$544 |
| 244B | 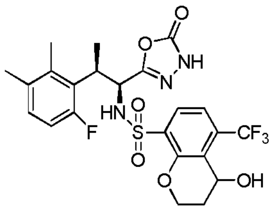 | 1H NMR (CD3OD) δ: 7.89 (d, J=8.4 Hz, 1H), 7.33 (d, J=8.4 Hz, 1H), 6.97 (dd, J=8.3, 5.7 Hz, 1H), 6.71 (dd, J=11.7, 8.3 Hz, 1H), 5.04-5.07 (m, 1H), 4.79 (d, J=11.0 Hz, 1H), 4.46-4.65 (m, 2H), 3.63-3.77 (m, 1H), 2.22 (s, 3H), 2.18 (s, 3H), 1.95-2.14 (m, 2H), 1.47 (d, J=6.6 Hz, 3H); LC/MS RT 1.76min, m/z [M-H]$^-$ 544 |
| 245 | 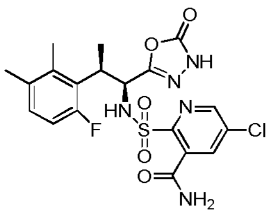 | 1H NMR (CD3OD) δ: 8.51 (d, J=2.6 Hz, 1H), 8.06 (d, J=2.6 Hz, 1H), 6.98 (dd, J=8.3, 6.0 Hz, 1H), 6.71 (dd, J=11.7, 8.3 Hz, 1H), 4.89-4.94 (m, 1H), 3.63-3.70 (m, 1H), 2.26 (s, 3H), 2.20 (s, 3H), 1.49 (d, J=6.6 Hz, 3H); LC/MS RT 1.47min, m/z [M-H]$^-$ 482,484 |
| 246 | 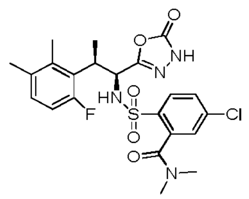 | 1H NMR (CD3OD) δ: 7.73-7.97 (m, 1H), 7.51-7.68 (m, 1H), 7.46 (s, 1H), 6.99 (dd, J=8.4, 5.7 Hz, 1H), 6.73 (dd, J=11.8, 8.4 Hz, 1H), 4.80 (d, J=11.2 Hz, 1H), 3.54-3.66 (m, 1H), 3.11 (s, 3H), 2.88 (s, 3H), 2.23 (s, 3H), 2.19 (s, 3H), 1.44-1.54 (m, 3H); LC/MS RT 1.79min, m/z [M-H]$^-$ 509,511 |
| 247 | 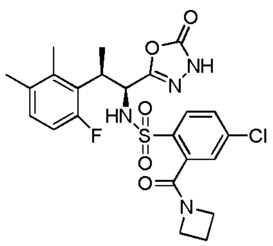 | 1H NMR (CD3OD) δ: 7.84 (d, J=8.1 Hz, 1H), 7.58 (dd, J=8.4, 2.2 Hz, 1H), 7.52 (d, J=2.2 Hz, 1H), 6.96-7.01 (m, 1H), 6.71-6.75 (m, 1H), 4.74-4.85 (m, 1H), 3.93-4.32 (m, 4H), 3.53-3.67 (m, 1H), 2.32-2.46 (m, 2H), 2.21 (s, 3H), 2.18 (s, 3H), 1.43 (d, J=7.3 Hz, 3H); LC/MS RT 1.81min, m/z [M-H]$^-$ 521,523 |

(continued)

| Example | Structural Formula | Physical Property Value |
|---|---|---|
| 248 | 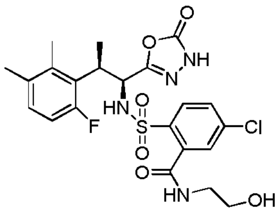 | 1H NMR (CD3OD) δ: 7.52-7.89 (m, 3H), 6.94-7.03 (m, 1H), 6.66-6.78 (m, 1H), 4.79 (d, J=11.4 Hz, 1H), 3.43-3.88 (m, 5H), 2.21 (s, 3H), 2.15 (s, 3H), 1.49 (d, J=7.7 Hz, 3H),; LC/MS RT 1.61, 1.66min, m/z $[M-H]^-$ 525,527 |
| 249 | 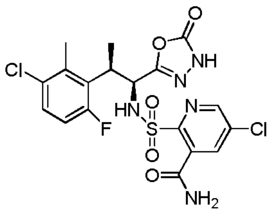 | 1H NMR (CD3OD) δ: 8.51 (d, J=2.2 Hz, 1H), 8.06 (d, J=2.2 Hz, 1H), 7.26 (dd, J=8.9, 5.1 Hz, 1H), 6.88 (dd, J=11.2, 8.9 Hz, 1H), 4.92 (d, J=11.4 Hz, 1H), 3.61-3.74 (m, 1H), 2.42 (s, 3H), 1.50 (d, J=7.0 Hz, 3H); LC/MS RT 1.51min, m/z $[M-H]^-$ 502,504 |
| 250 | 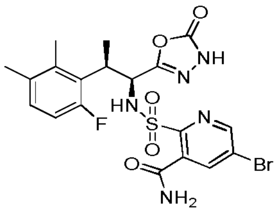 | 1H NMR (CD3OD) δ: 8.61 (d, J=1.8 Hz, 1H), 8.20 (d, J=2.2 Hz, 1H), 6.98 (dd, J=8.4, 5.9 Hz, 1H), 6.71 (dd, J=11.7, 8.4 Hz, 1H), 4.92 (d, J=11.0 Hz, 1H), 3.56-3.83 (m, 1H), 2.25 (s, 3H), 2.20 (s, 3H), 1.48 (d, J=6.6 Hz, 3H); LC/MS RT 1.49min, m/z $[M-H]^-$ 526,528 |
| 251 | 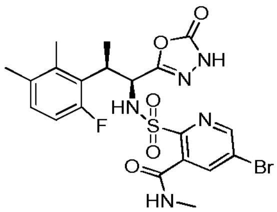 | 1H NMR (CD3OD) δ: 8.60 (d, J=2.2 Hz, 1H), 8.16 (d, J=2.2 Hz, 1H), 6.99 (dd, J=8.2, 5.7 Hz, 1H), 6.71 (dd, J=11.7, 8.4 Hz, 1H), 4.88-4.93 (m, 1H), 3.55-3.75 (m, 1H), 2.93 (s, 3H), 2.26 (s, 3H), 2.23 (s, 3H), 1.48 (d, J=7.0 Hz, 3H); LC/MS RT 1.56min, m/z $[M-H]^-$ 542,544 |
| 252 | 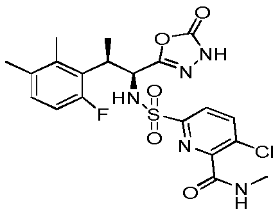 | 1H NMR (CD3OD) δ: 8.17 (d, J=8.4 Hz, 1H), 8.02 (d, J=8.4 Hz, 1H), 6.98 (dd, J=8.6, 5.7 Hz, 1H), 6.66-6.79 (m, 1H), 4.83-4.90 (m, 1H), 3.56-3.71 (m, 1H), 2.94 (s, 3H), 2.23 (s, 3H), 2.18 (s, 3H), 1.48 (d, J=7.0 Hz, 3H); LC/MS RT 1.57min, m/z $[M-H]^-$ 496,498 |
| 253 | 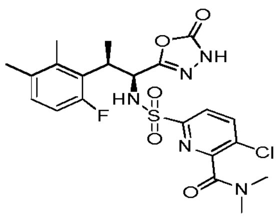 | 1H NMR (CD3OD) δ: 8.16 (d, J=8.4 Hz, 1H), 7.98 (d, J=8.4 Hz, 1H), 6.96-7.18 (m, 1H), 6.73 (dd, J=11.7, 8.8 Hz, 1H), 4.92-4.98 (m, 1H), 3.57-3.67 (m, 1H), 3.12 (s, 3H), 2.85 (s, 3H), 2.21 (s, 3H), 2.19 (s, 3H), 1.46 (d, J=7.0 Hz, 3H); LC/MS RT 1.56min, m/z $[M-H]^-$ 510,512 |
| 254 | 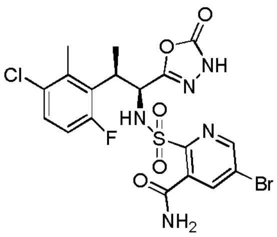 | 1H NMR (CD3OD) δ: 8.61 (d, J=2.2 Hz, 1H), 8.20 (d, J=2.2 Hz, 1H), 7.26 (dd, J=9.0, 4.9 Hz, 1H), 6.88 (dd, J=11.2, 9.0 Hz, 1H), 4.91 (d, J=11.0 Hz, 1H), 3.61-3.76 (m, 1H), 2.42 (s, 3H), 1.50 (d, J=7.0 Hz, 3H); LC/MS RT 1.53min, m/z $[M-H]^-$ 546,548 |

(continued)

| Example | Structural Formula | Physical Property Value |
|---|---|---|
| 255 | 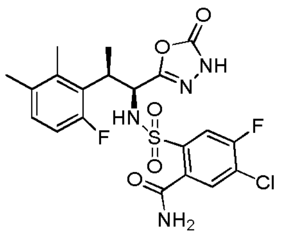 | 1H NMR (CD3OD) $\delta$: 7.76-7.80 (m, 1H), 7.69-7.73 (m, 1H), 6.98 (dd, J=8.3, 5.9 Hz, 1H), 6.73 (dd, J=11.7, 8.3 Hz, 1H), 4.82 (d, J=11.0 Hz, 1H), 3.51-3.65 (m, 1H), 2.22 (s, 3H), 2.18 (s, 3H), 1.46 (d, J=7.0 Hz, 3H); LC/MS RT 1.71min, m/z $[M-H]^-$ 499,501 |
| 256 | 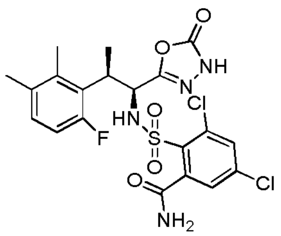 | 1H NMR (CD3OD) $\delta$: 7.66 (d, J=2.2 Hz, 1H), 7.41 (d, J=2.2 Hz, 1H), 6.98 (dd, J=8.3, 5.9 Hz, 1H), 6.70 (dd, J=11.5, 8.3 Hz, 1H), 4.92-5.00 (m, 1H), 3.67-3.79 (m, 1H), 2.25 (s, 3H), 2.18 (s, 3H), 1.47 (d, J=7.0 Hz, 3H); LC/MS RT 1.55min, m/z $[M-H]^-$ 515,517 |
| 257 | 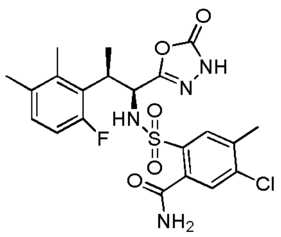 | 1H NMR (CD3OD) $\delta$: 7.73 (s, 1H), 7.61 (s, 1H), 6.98 (dd, J=8.1, 5.9 Hz, 1H), 6.68-6.77 (m, 1H), 4.79-4.85 (m, 1H), 3.52-3.65 (m, 1H), 2.42 (s, 3H), 2.19 (s, 3H), 2.17 (s, 3H), 1.46 (d, J=7.0 Hz, 3H); LC/MS RT 1.7min, m/z $[M-H]^-$ 495,497 |
| 258 | 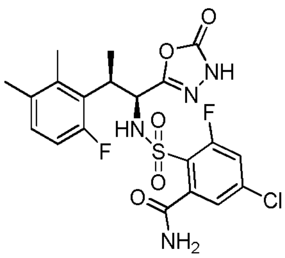 | 1H NMR (CD3OD) $\delta$: 7.43 (dd, J=10.3, 2.2 Hz, 1H), 7.30 (d, J=1.1 Hz, 1H), 6.98 (dd, J=8.3, 5.7 Hz, 1H), 6.71 (dd, J=11.7, 8.3 Hz, 1H), 4.94 (d, J=11.4 Hz, 1H), 3.60-3.75 (m, 1H), 2.23 (s, 3H), 2.18 (s, 3H), 1.45 (d, J=7.0 Hz, 3H); LC/MS RT 1.55min, m/z $[M-H]^-$ 499,501 |
| 259 | 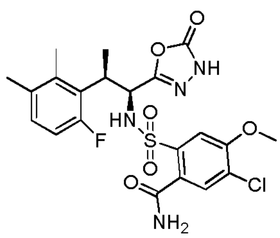 | 1H NMR (CD3OD) $\delta$: 7.66 (s, 1H), 7.48 (s, 1H), 6.98 (dd, J=8.3, 5.9 Hz, 1H), 6.72 (dd, J=11.9, 8.3 Hz, 1H), 4.80-4.85 (m, 1H), 3.97 (s, 3H), 3.52-3.60 (m, 1H), 2.20 (s, 3H), 2.17 (s, 3H), 1.46-1.50 (m, 3H); LC/MS RT 1.67min, m/z $[M-H]^-$ 511,513 |
| 260 | 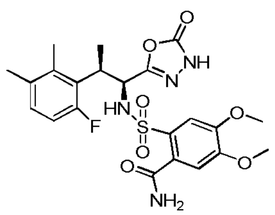 | 1H NMR (CD3OD) $\delta$: 7.35 (s, 1H), 7.16 (s, 1H), 6.97 (dd, J=8.5, 5.7 Hz, 1H), 6.72 (dd, J=11.7, 8.5 Hz, 1H), 4.79 (d, J=11.0 Hz, 1H), 3.90 (s, 3H), 3.88 (s, 3H), 3.47-3.56 (m, 1H), 2.20 (s, 3H), 2.17 (s, 3H), 1.48 (d, J=7.0 Hz, 3H); LC/MS RT 1.55min, m/z $[M-H]^-$ 507 |

(continued)

| Example | Structural Formula | Physical Property Value |
|---|---|---|
| 261 | 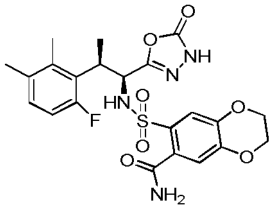 | 1H NMR (CD3OD) δ: 7.26 (s, 1H), 7.10 (s, 1H), 6.97 (dd, J=8.4, 5.9 Hz, 1H), 6.72 (dd, J=12.1, 8.4 Hz, 1H), 4.75 (d, J=11.4 Hz, 1H), 4.29-4.32 (m, 4H), 3.44-3.60 (m, 1H), 2.19 (s, 3H), 2.17 (s, 3H), 1.47 (d, J=7.0 Hz, 3H); LC/MS RT 1.61min, m/z $[M-H]^-$ 505 |
| 262 | 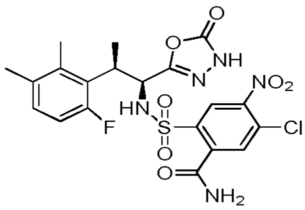 | 1H NMR (CD3OD) δ: 8.36 (s, 1H), 7.90 (s, 1H), 6.98 (dd, J=8.3, 5.9 Hz, 1H), 6.73 (dd, J=11.9, 8.3 Hz, 1H), 4.82-4.86 (m, 1H), 3.55-3.65 (m, 1H), 2.22 (s, 3H), 2.18 (s, 3H), 1.47 (d, J=7.0 Hz, 3H); LC/MS RT 1.72min, m/z $[M-H]^-$ 526,528 |
| 263 | 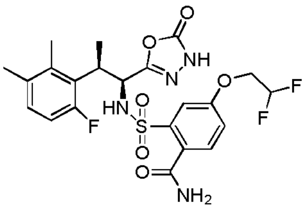 | 1H NMR (CD3OD) δ: 7.60 (d, J=8.4 Hz, 1H), 7.42 (d, J=2.6 Hz, 1H), 7.21 (dd, J=8.6, 2.7 Hz, 1H), 6.98 (dd, J=8.4, 5.9 Hz, 1H), 6.72 (dd, J=11.7, 8.4 Hz, 1H), 6.20 (tt, J=55.0, 3.7 Hz, 1H), 4.80 (d, J=11.0 Hz, 1H), 4.31 (tdd, J=13.6, 3.7, 2.6 Hz, 2H), 3.51-3.58 (m, 1H), 2.20 (s, 3H), 2.17 (s, 3H), 1.46 (d, J=6.6 Hz, 3H); LC/MS RT 1.67min, m/z $[M-H]^-$ 527 |
| 264 | 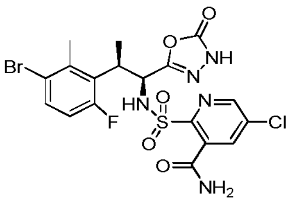 | 1H NMR (CD3OD) δ: 8.51 (d, J=2.2 Hz, 1H), 8.07 (d, J=2.2 Hz, 1H), 7.45 (dd, J=8.8, 5.5 Hz, 1H), 6.77-6.91 (m, 1H), 4.89-4.95 (m, 1H), 3.65-3.75 (m, 1H), 2.48 (s, 3H), 1.50 (d, J=7.0 Hz, 3H); LC/MS RT 1.54min, m/z $[M-H]^-$ 546,548 |
| 265 | 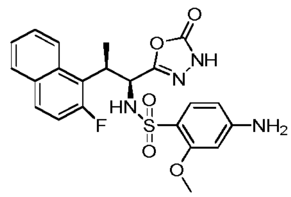 | 1H NMR (CD3OD) δ: 8.10 (d, J=8.8 Hz, 1H), 7.81 (d, J=8.6 Hz, 1H), 7.75-7.79 (m, 1H), 7.54 (t, J=7.7 Hz, 1H), 7.39-7.45 (m, 2H), 7.20 (dd, J=11.7, 9.2 Hz, 1H), 6.11-6.24 (m, 2H), 4.73 (d, J=11.4 Hz, 1H), 4.12-4.30 (m, 1H), 3.85 (s, 3H), 1.62 (d, J=6.6 Hz, 3H); LC/MS RT 1.56min, m/z $[M-H]^-$ 471 |
| 266 | 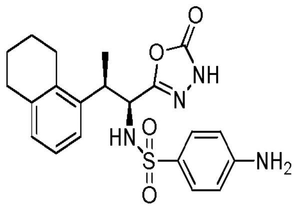 | 1H NMR (CD3OD) δ: 7.37-7.45 (m, 2H), 6.94-7.08 (m, 2H), 6.85 (d, J=6.6 Hz, 1H), 6.51-6.62 (m, 2H), 4.24 (d, J=10.6 Hz, 1H), 3.36-3.48 (m, 1H), 2.59-2.75 (m, 4H), 1.59-1.85 (m, 4H), 1.29-1.44 (d, J=6.6 Hz, 3H); LC/MS RT 1.61min, m/z $[M-H]^-$ 427 |
| 267 | 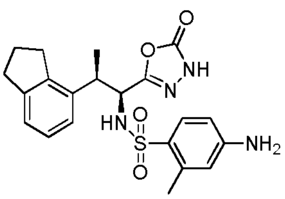 | 1H NMR (CD3OD) δ: 7.52 (d, J=9.2 Hz, 1H), 7.01 (s, 2H), 6.87-6.96 (m, 1H), 6.35-6.48 (m, 2H), 4.17 (d, J=10.6 Hz, 1H), 3.20-3.35 (m, 1H), 2.75-2.84 (m, 4H), 2.42 (s, 3H), 1.85-2.09 (m, 2H), 1.38 (d, J=7.0 Hz, 3H); LC/MS RT 1.58min, m/z $[M-H]^-$427 |

(continued)

| Example | Structural Formula | Physical Property Value |
|---|---|---|
| 268 | 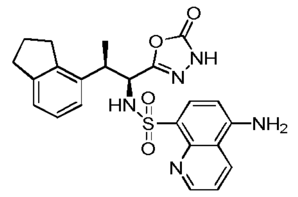 | 1H NMR (CD3OD) δ: 8.82-8.91 (m, 1H), 8.48 (d, J=8.4 Hz, 1H), 8.00 (d, J=8.4 Hz, 1H), 7.36-7.45 (m, 1H), 6.83-7.06 (m, 3H), 6.68 (d, J=8.4 Hz, 1H), 4.31 (d, J=10.3 Hz, 1H), 3.34-3.46 (m, 1H), 2.72-2.82 (m, 4H), 1.81-2.10 (m, 2H), 1.43 (d, J=6.6 Hz, 3H); LC/MS RT 1.57min, m/z [M-H]- 464 |
| 269 | 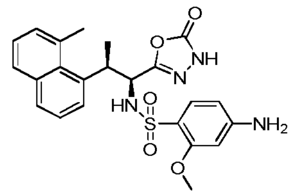 | 1H NMR (CD3OD) δ: 7.64 (d, J=8.0 Hz, 1H), 7.60 (dd, J=7.6, 1.9 Hz, 1H), 7.43-7.47 (m, 1H), 7.38 (d, J=8.5 Hz, 1H), 7.34 (t, J=7.8 Hz, 1H), 7.21-7.27 (m, 2H), 6.12-6.16 (m, 2H), 4.59-4.66 (m, 1H), 4.26 (d, J=10.9 Hz, 1H), 3.80 (s, 3H), 2.95 (s, 3H), 1.66 (d, J=6.6 Hz, 3H); LC/MS RT 1.58min, m/z [M-H]- 467 |
| 270 | 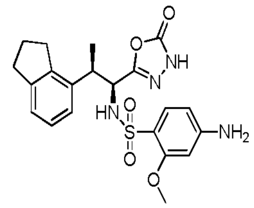 | 1H NMR (CD3OD) 7.38 (d, J=8.4 Hz, 1H), 6.98-7.07 (m, 2H), 6.91-6.95 (m, 1H), 6.20 (d, J=1.8 Hz, 1H), 6.16 (dd, J=8.6, 2.0 Hz, 1H), 4.57-4.63 (m, 1H), 4.21 (d, J=11.0 Hz, 1H), 3.83 (s, 3H), 2.73-2.91 (m, 4H), 1.89-2.04 (m, 2H), 1.43 (d, J=6.6 Hz, 3H); LC/MS RT 1.55min, m/z [M-H]- 443 |
| 271 | 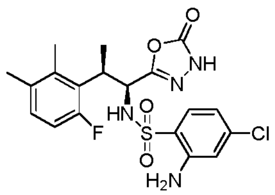 | LC/MS RT 1.75min, m/z [M-H]- 453,455 |
| 272 | 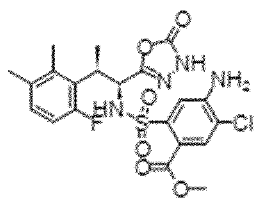 | LC/MS RT 1.8min, m/z [M-H]- 511,513 |
| 273 | 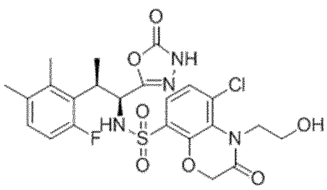 | 1H NMR (CD3OD) δ: 7.46 (1H, d, J = 8.6 Hz), 7.21 (1H, d, J = 8.6 Hz), 6.98-6.95 (1H, m), 6.71 (1H, dd, J = 11.9, 8.2 Hz), 4.79-4.66 (3H, m), 4.41-4.34 (1H, m), 4.24-4.18 (1H, m), 3.75-3.62 (3H, m), 2.19 (3H, s), 2.16 (3H, s), 1.49 (3H, d, J = 6.6 Hz); LC/MS RT 1.61min, m/z [M-H]- 553,555 |
| 274 | 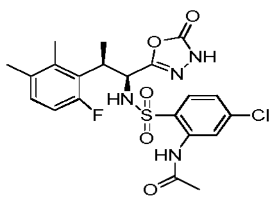 | 1H NMR (CD3OD) δ: 7.53 (d, J=8.8 Hz, 1H), 6.96-7.04 (m, 1H), 6.71-6.81 (m, 2H), 6.55-6.60 (m, 1H), 4.66 (d, J=11.4 Hz, 1H), 3.50-3.66 (m, 1H), 2.23 (s, 3H), 2.21 (s, 3H), 2.17 (s, 3H), 1.45 (d, J=6.6 Hz, 3H); LC/MS RT 1.92min, m/z [M-H]- 495,497 |

(continued)

| Example | Structural Formula | Physical Property Value |
|---|---|---|
| 275 | 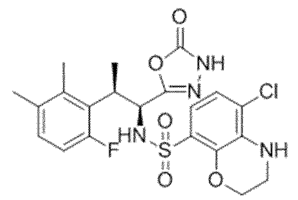 | 1H-NMR (CDCl3) δ: 7.63 (1H, s), 7.10 (1H, d, J = 8.4 Hz), 6.94-6.88 (2H, m), 6.69 (1H, dd, J = 11.5, 8.6 Hz), 5.38 (1H, d, J = 10.6 Hz), 4.86 (1H, t, J = 10.8 Hz), 4.41-4.40 (4H, m), 3.53-3.52 (2H, m), 2.18-2.17 (6H, m), 1.57-1.54 (3H, m).; LC/MS RT 1.71min, m/z [M-H]- 495,497 |
| 276 | 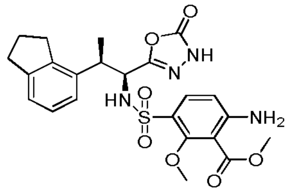 | 1H-NMR (CDCl3) δ: 8.07 (1H, br s), 7.64 (1H, d, J = 8.8 Hz), 7.07-7.06 (2H, m), 6.95-6.93 (1H, m), 6.38 (1H, d, J = 8.8 Hz), 5.68 (2H, s), 5.45 (1H, d, J = 10.4 Hz), 4.37 (1H, t, J = 10.4 Hz), 3.95 (3H, s), 3.88 (3H, s), 3.28-3.21 (1H, m), 2.88-2.83 (4H, m), 2.04-1.99 (2H, m), 1.49 (3H, d, J = 7.0 Hz).; LC/MS RT 1.67min, m/z [M-H]- 501 |
| 277 | 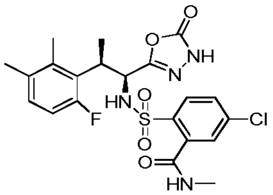 | 1H NMR (CD3OD) δ: 7.83 (d, J=8.4 Hz, 1H), 7.58-7.60 (m, 1H), 7.55-7.57 (m, 1H), 6.94-7.02 (m, 1H), 6.67-6.77 (m, 1H), 4.76-4.82 (m, 1H), 3.57-3.76 (m, 1H), 2.92 (s, 3H), 2.21 (s, 3H), 2.17 (s, 3H), 1.46 (d, J=7.0 Hz, 3H); LC/MS RT 1.74min, m/z [M-H]- 495,497 |
| 278 | 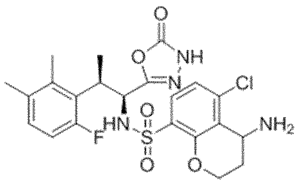 | LC/MS RT 1.34min, m/z [M-H]- 509,511 |
| 279 | 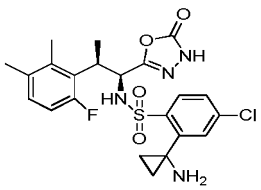 | LC/MS RT 1.4min, m/z [M-H]- 493,495 |
| 280 | 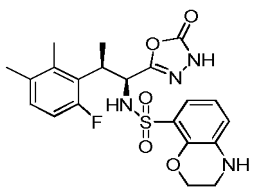 | 1H-NMR (CDCl3) δ: 8.12 (1H, br s), 7.15-7.13 (1H, m), 6.93-6.90 (1H, m), 6.75-6.64 (3H, m), 5.51 (1H, d, J = 10.6 Hz), 4.87 (1H, t, J = 10.6 Hz), 4.37-4.35 (2H, m), 3.93 (1H, br s), 3.43-3.40 (3H, m), 2.19 (3H, s), 2.17 (3H, s), 1.54 (3H, d, J = 7.0 Hz).; LC/MS RT 1.6min, m/z [M-H]- 461 |
| 281 | | 1H NMR (CD3OD) δ: 7.90 (d, J=1.0 Hz, 1H), 7.35-7.49 (m, 2H), 6.97 (t, J=1.0 Hz, 1H), 6.73 (dd, J=1.0 Hz, 1H), 4.70 (br d, J=11.4 Hz, 1H), 4.28-4.52 (m, 2H), 3.38-3.83 (m, 5H), 2.17 (s, 5H), 1.49 (d, J=1.0 Hz, 3H); LC/MS RT 1.29min, m/z [M-H]- 459 |
| 282 | 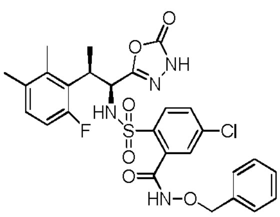 | LC/MS RT 1.94min, m/z [M-H]- 587,589 |

(continued)

| Example | Structural Formula | Physical Property Value |
|---|---|---|
| 283 | 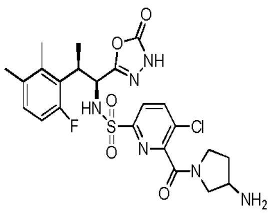 | 1H NMR (CD3OD) δ: 7.94-7.99 (m, 1H), 7.84-7.91 (m, 1H), 6.81-6.93 (m, 1H), 6.61 (dd, J=11.5, 8.6 Hz, 1H), 4.75 (d, J=10.3 Hz, 1H), 3.37-3.89 (m, 5H), 2.88-3.03 (m, 1H), 2.24 (s, 3H), 2.14 (s, 3H), 1.75-1.85 (m, 1H), 1.36-1.53 (m, 4H); LC/MS RT 1.25min, m/z $[M-H]^-$ 551,553 |
| 284 | 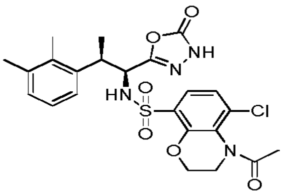 | 1H-NMR (CDCl3) δ: 7.08 (1H, d, J = 8.8 Hz), 7.02-6.97 (3H, m), 6.88 (1H, d, J = 8.4 Hz), 5.42 (1H, d, J = 10.8 Hz), 4.56 (1H, t, J = 10.8 Hz), 4.47-4.36 (3H, m), 3.55-3.44 (3H, m), 2.24 (3H, s), 2.22 (3H, s), 2.17 (3H, s), 1.47 (3H, d, J = 7.0 Hz).; LC/MS RT 1.88min, m/z $[M-H]^-$ 519,521 |
| 285 | 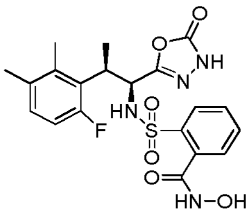 | 1H NMR (CD3OD) δ: 7.84-7.95 (m, 1H), 7.50-7.66 (m, 3H), 6.97 (dd, J=8.4, 5.9 Hz, 1H), 6.71 (dd, J=11.7, 8.4 Hz, 1H), 4.80 (d, J=11.0 Hz, 1H), 3.50-3.63 (m, 1H), 2.20 (s, 3H), 2.17 (s, 3H), 1.44 (d, J=6.6 Hz, 3H); LC/MS RT 1.48min, m/z $[[M-H]^-$ 463 |
| 286 | 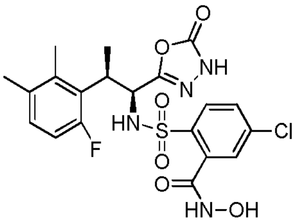 | 1H NMR (CD3OD) δ: 7.87 (d, J=8.4 Hz, 1H), 7.62 (dd, J=8.8, 2.2 Hz, 1H), 7.54 (d, J=2.2 Hz, 1H), 6.98 (dd, J=8.2, 5.7 Hz, 1H), 6.72 (dd, J=11.9, 8.2 Hz, 1H), 4.80 (d, J=11.0 Hz, 1H), 3.53-3.63 (m, 1H), 2.21 (s, 3H), 2.17 (s, 3H), 1.45 (d, J=7.0 Hz, 3H); LC/MS RT 1.59min, m/z $[M-H]^-$ 497,499 |
| 287 | 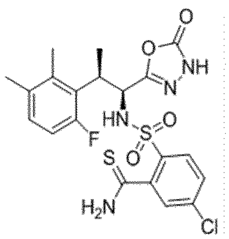 | 1H-NMR (CDCl3) δ: 8.70 (1H, s), 8.51-8.48 (1H, m), 7.87-7.84 (2H, m), 7.44 (1H, dd, J = 8.6, 2.1 Hz), 7.39 (1H, d, J = 2.1 Hz), 6.93 (1H, dd, J = 8.4, 5.9 Hz), 6.71-6.65 (2H, m), 4.87 (1H, t, J = 10.1 Hz), 3.53-3.48 (1H, m), 2.18-2.17 (6H, m), 1.43 (3H, d, J = 7.0 Hz).; LC/MS RT 1.72min, m/z $[M-H]^-$ 497,499 |
| 288 | 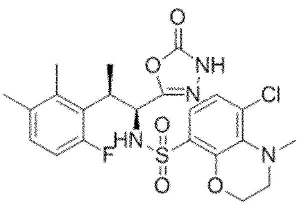 | LC/MS RT 1.76min, m/z $[M-H]^-$ 509,511 |
| 289 | 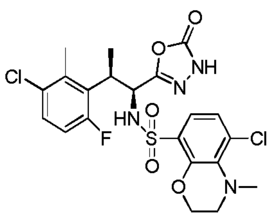 | LC/MS RT 1.8min, m/z $[M-H]^-$ 529,531 |

(continued)

| Example | Structural Formula | Physical Property Value |
|---|---|---|
| 290 | 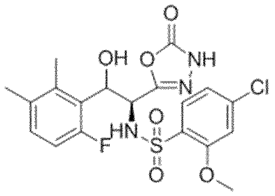 | 1H NMR (CD3OD) δ: 7.73 (1H, d, J = 8.1 Hz), 7.16-7.15 (1H, m), 7.06-7.01 (2H, m), 6.71 (1H, dd, J = 10.6, 8.4 Hz), 5.38 (1H, d, J = 9.5 Hz), 4.68 (1H, d, J = 9.5 Hz), 3.96 (3H, s), 3.33 (1H, s), 2.25 (3H, s), 2.16 (3H, s); LC/MS RT 1.53min, m/z [M-H]- 470,472 |
| 291 | 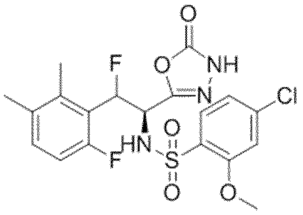 | LC/MS RT 1.75, 1.76min, m/z [M-H]- 472,474 |
| 292 | 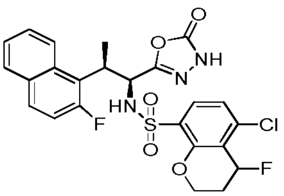 | 1H-NMR (CDCl3) δ: 7.98 (1H, d, J = 8.8 Hz), 7.94 (1H, s), 7.86-7.84 (1H, m), 7.78 (1H, d, J = 8.1 Hz), 7.72-7.70 (1H, m), 7.55 (1H, t, J = 7.7 Hz), 7.42 (1H, t, J = 7.3 Hz), 7.17-7.12 (1H, m), 7.08 (1H, d, J = 8.4 Hz), 5.79-5.67 (1H, m), 5.62 (1H, d, J = 10.4 Hz), 4.94 (1H, t, J = 10.4 Hz), 4.60-4.56 (1H, m), 4.23-4.20 (1H, m), 3.99 (1H, s), 3.67-3.51 (1H, m), 2.38-2.35 (1H, m), 1.69 (3H, d, J = 5.9 Hz).; LC/MS RT 1.8min, m/z [M-H]- 534,536 |
| 293 | 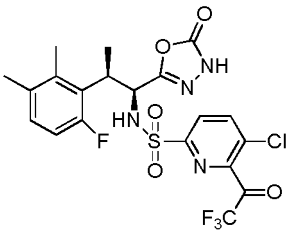 | 1H-NMR (CDCl3) δ: 8.56-8.54 (1H, m), 8.37-8.35 (1H, m), 8.10 (1H, s), 6.97-6.90 (1H, m), 6.71-6.64 (1H, m), 5.82 (1H, br s), 5.03-4.97 (1H, m), 3.54-3.52 (1H, m), 2.19-2.17 (6H, m), 1.53-1.44 (3H, m).; LC/MS RT 1.64min, m/z [M-H]- 535,537 |
| 294 | 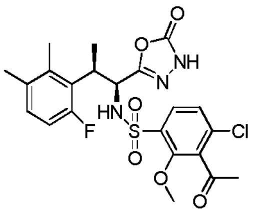 | LC/MS RT 1.83min, m/z [M-H]- 510,512 |
| 295 | 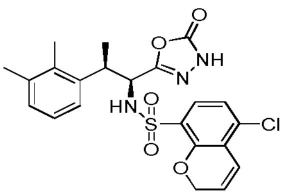 | 1H NMR (CD3OD) δ: 7.48 (d, J=8.8 Hz, 1H), 6.91-7.03 (m, 4H), 6.70 (dt, J-10.3, 2.0 Hz, 1H), 6.01 (dt, J=10.3, 3.7 Hz, 1H), 4.98-5.04 (m, 2H), 4.33 (d, J=10.6 Hz, 1H), 3.60-3.68 (m, 1H), 2.20 (s, 3H), 2.19 (s, 3H), 1.42 (d, J=6.6 Hz, 3H); LC/MS RT 1.82min, m/z [M-H]- 474,476 |
| 296 | 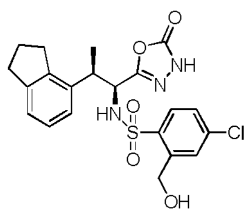 | LC/MS RT 1.73min, m/z [M-H]- 462,464 |

(continued)

| Example | Structural Formula | Physical Property Value |
|---|---|---|
| 297 | 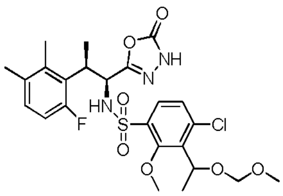 | 1H NMR (CD3OD) δ: 7.69-7.80 (m, 1H), 7.30-7.40 (m, 1H), 6.93-7.06 (m, 1H), 6.69-6.82 (m, 1H), 5.38-5.49 (m, 1H), 4.77 (d, J=11.2 Hz, 1H), 4.66 (d, J=11.7 Hz, 1H), 4.50 (d, J=11.7 Hz, 1H), 4.02 (s, 3H), 3.68-3.75 (m, 1H), 2.86 (s, 3H), 2.24 (s, 3H), 2.18 (s, 3H), 1.64 (d, J=6.6 Hz, 3H), 1.48 (d, J=7.1 Hz, 3H); LC/MS RT 1.92min, m/z [M-H]$^{-}$ 556,558 |
| 298 | 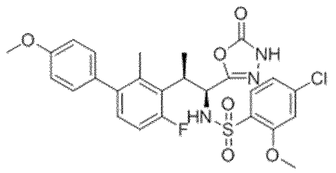 | 1H-NMR (CDCl3) δ: 7.81 (1H, d, J = 8.4 Hz), 7.06-6.88 (7H, m), 6.82 (1H, dd, J = 11.4, 8.4 Hz), 5.40 (1H, d, J = 10.4 Hz), 4.86 (1H, t, J = 10.4 Hz), 3.96 (3H, s), 3.83 (3H, s), 3.42 (1H, br s), 2.16 (3H, s), 1.59 (3H, d, J = 7.0 Hz); LC/MS RT 1.93min, m/z [M-H]$^{-}$ 560,562 |
| 299 | 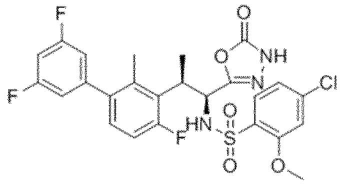 | 1H-NMR (CDCl3) δ: 7.89 (1H, br s), 7.81 (1H, d, J = 8.4 Hz), 7.04-6.98 (2H, m), 6.93 (1H, s), 6.88-6.75 (2H, m), 6.68-6.66 (2H, m), 5.44 (1H, d, J = 10.7 Hz), 4.85 (1H, t, J = 10.7 Hz), 3.96 (3H, s), 3.41 (1H, br s), 2.16 (3H, s), 1.59 - 1.57 (3H, m).; LC/MS RT 1.96min, m/z [M-H]$^{-}$ 566,568 |
| 300 | 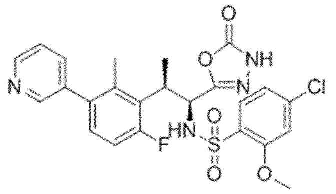 | 1H-NMR (CDCl3) δ: 8.57-8.55 (1H, m), 8.45-8.43 (1H, m), 7.82 (1H, d, J = 8.4 Hz), 7.64-7.61 (1H, m), 7.44-7.41 (1H, m), 7.05-7.00 (2H, m), 6.94-6.88 (2H, m), 5.60-5.57 (1H, m), 4.82 (1H, t, J = 10.4 Hz), 3.96 (3H, s), 3.92 (1H, s), 3.46 (1H, s), 2.15 (3H, s), 1.61 (3H, d, J = 6.6 Hz).; LC/MS RT 1.4min, m/z [M-H]$^{-}$ 531,533 |
| 301 | 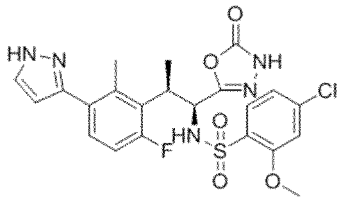 | 1H-NMR (CDCl3) δ: 7.82 (1H, d, J = 8.1 Hz), 7.59 (1H, s), 7.19-7.16 (1H, m), 7.02 (1H, d, J = 8.8 Hz), 6.92 (1H, s), 6.87-6.82 (1H, m), 6.28 (1H, s), 5.85 (1H, br s), 4.84 (1H, t, J = 10.1 Hz), 3.90 (3H, s), 3.45 (1H, br s), 2.24 (3H, s), 1.56 (3H, d, J = 6.6 Hz); LC/MS RT 1.56min, m/z [M-H]$^{-}$ 520,522 |
| 302 | 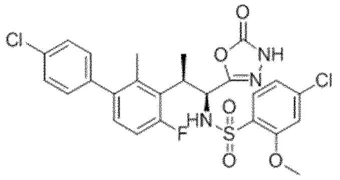 | 1H-NMR (CDCl3) δ: 7.91 (1H, br s), 7.81 (1H, d, J = 8.4 Hz), 7.34 (2H, d, J = 8.4 Hz), 7.07-6.97 (4H, m), 6.86-6.81 (1H, m), 6.84 (1H, t, J = 9.9 Hz), 5.44 (1H, d, J = 10.6 Hz), 4.86 (1H, t, J = 10.6 Hz), 3.96 (3H, s), 3.41 (1H, br s), 2.14 (3H, s), 1.58 (3H, d, J = 7.0 Hz).; LC/MS RT 2.05min, m/z [M-H]$^{-}$ 564,566 |
| 303 | 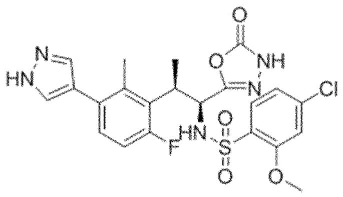 | 1H-NMR (CDCl3) δ: 7.81 (1H, d, J = 8.4 Hz), 7.52-7.51 (2H, m), 7.09-6.99 (4H, m), 6.93-6.93 (1H, m), 6.85-6.80 (1H, m), 5.49-5.46 (1H, m), 4.88-4.83 (1H, m), 3.96 (3H, s), 3.43 (1H, br s), 2.27 (3H, s), 1.58 (3H, d, J = 7.0 Hz).; LC/MS RT 1.54min, m/z [M-H]$^{-}$ 520,522 |
| 304 | 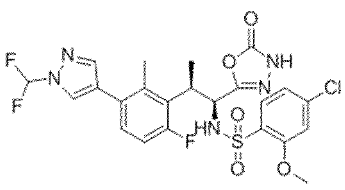 | 1H-NMR (CDCl3) δ: 8.06 (1H, br s), 7.81 (1H, d, J = 8.4 Hz), 7.73 (1H, s), 7.59 (1H, s), 7.57-7.54 (1H, m), 7.41-7.36 (1H, m), 7.03-7.00 (1H, m), 6.93-6.92 (1H, m), 6.84 (1H, dd, J = 11.0, 8.4 Hz), 5.45 (1H, d, J = 10.4 Hz), 4.86 (1H, t, J = 10.4 Hz), 3.96 (3H, s), 3.44 (1H, br s), 2.27 (3H, s), 1.58 (3H, d, J = 9.5 Hz).; LC/MS RT 1.75min, m/z [M-H]$^{-}$ 570,572 |

(continued)

| Example | Structural Formula | Physical Property Value |
|---|---|---|
| 305 | 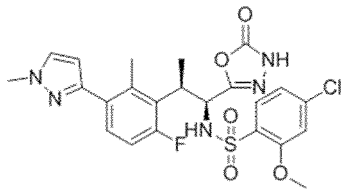 | 1H-NMR (CDCl3) δ: 8.81 (1H, br s), 7.80 (1H, d, J = 8.8 Hz), 7.36 (1H, s), 7.23-7.20 (1H, m), 7.01 (1H, d, J = 8.4 Hz), 6.93 (1H, s), 6.82 (1H, t, J = 9.7 Hz), 6.19 (1H, s), 5.50 (1H, d, J = 10.4 Hz), 4.86 (1H, t, J = 10.4 Hz), 3.95 (3H, s), 3.90 (3H, s), 3.45 (1H, br s), 2.30 (3H, s), 1.56 (3H, d, J = 6.6 Hz).; LC/MS RT 1.64min, m/z $[M-H]^-$ 534,536 |
| 306 | 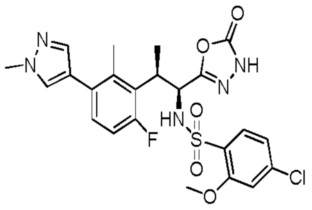 | 1H-NMR (CDCl3) δ: 8.48 (1H, s), 7.80 (1H, d, J = 8.4 Hz), 7.39 (1H, s), 7.28 (1H, s), 7.06-6.99 (2H, m), 6.92-6.92 (1H, m), 6.80 (1H, dd, J = 11.2, 8.6 Hz), 5.56 (1H, d, J = 10.6 Hz), 4.84 (1H, t, J = 10.6 Hz), 3.94 (3H, s), 3.91 (3H, s), 3.44 (1H, s), 2.27 (3H, s), 1.57 (3H, d, J = 7.0 Hz).; LC/MS RT 1.62min, m/z $[M-H]^-$ 534,536 |
| 307 | 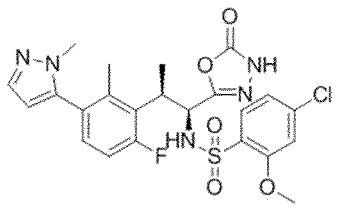 | LC/MS RT 1.65min, m/z $[M-H]^-$ 534,536 |
| 308 | 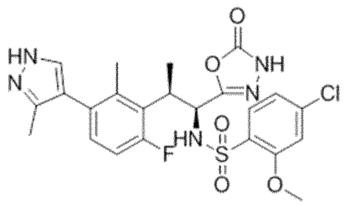 | LC/MS RT 1.82min, m/z $[M-H]^-$ 534,536 |
| 309 | 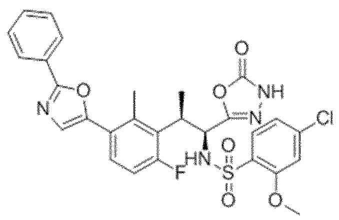 | LC/MS RT 1.94min, m/z $[M-H]^-$ 597,599 |
| 310 | 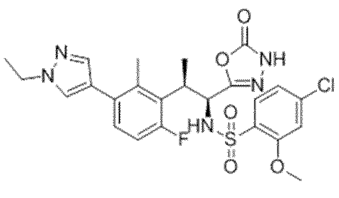 | 1H-NMR (CDCl3) δ: 8.50 (1H, s), 7.80 (1H, d, J = 8.4 Hz), 7.40 (1H, s), 7.32 (1H, s), 7.05 (1H, dd, J = 8.5, 5.9 Hz), 7.01 (1H, dd, J = 8.4, 1.5 Hz), 6.92 (1H, d, J = 1.8 Hz), 6.80 (1H, dd, J = 11.2, 8.5 Hz), 5.56 (1H, d, J = 10.3 Hz), 4.85 (1H, t, J = 10.8 Hz), 4.18 (2H, q, J = 7.3 Hz), 3.94 (3H, s), 3.44 (1H, br s), 2.28 (3H, s), 1.57 (3H, d, J = 7.0 Hz), 1.50 (3H, t, J = 7.3 Hz).; LC/MS RT 1.68min, m/z $[M-H]^-$ 548,550 |
| 311 | 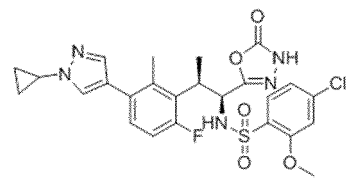 | LC/MS RT 1.71min, m/z $[M-H]^-$ 560,562 |

(continued)

| Example | Structural Formula | Physical Property Value |
|---|---|---|
| 312 | 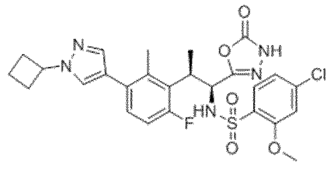 | 1H-NMR (CDCl3) δ: 7.80 (1H, d, J = 8.4 Hz), 7.42 (1H, s), 7.36 (1H, s), 7.06-6.99 (2H, m), 6.92 (1H, s), 6.82-6.77 (1H, m), 5.53 (1H, d, J = 10.7 Hz), 4.85 (1H, t, J = 10.7 Hz), 4.75 (1H, t, J = 8.4 Hz), 3.94 (3H, s), 3.44 (1H, br s), 2.55-2.46 (4H, m), 2.28 (3H, s), 1.89-1.82 (2H, m), 1.56 (3H, d, J = 7.0 Hz); LC/MS RT 1.8min, m/z $[M-H]^-$ 574,576 |
| 313 | 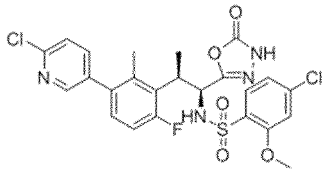 | 1H-NMR (CDCl3) δ: 8.20 (1H, d, J = 2.0 Hz), 7.81 (1H, d, J = 8.4 Hz), 7.47-7.44 (1H, m), 7.35 (1H, d, J = 8.4 Hz), 7.05-7.02 (1H, m), 7.01-6.99 (1H, m), 6.94 (1H, d, J = 2.0 Hz), 6.92-6.87 (1H, m), 5.43 (1H, d, J = 10.6 Hz), 4.86 (1H, t, J = 10.6 Hz), 3.96 (3H, s), 3.42 (1H, br s), 2.16 (3H, s), 1.59 (3H, d, J = 7.0 Hz); LC/MS RT 1.82min, m/z $[M-H]^-$ 565,567 |
| 314 | 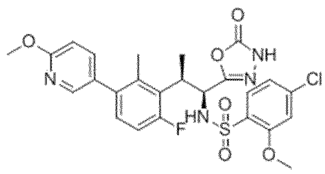 | 1H-NMR (CDCl3) δ: 8.94 (1H, br s), 7.91 (1H, d, J = 2.0 Hz), 7.80 (1H, d, J = 8.4 Hz), 7.39-7.36 (1H, m), 7.01-6.97 (2H, m), 6.93 (1H, d, J = 1.0 Hz), 6.85 (1H, dd, J = 11.4, 8.4 Hz), 6.76 (1H, d, J = 8.4 Hz), 5.60 (1H, d, J = 10.4 Hz), 4.84 (1H, t, J = 10.4 Hz), 3.94 (3H, s), 3.93 (3H, s), 3.44 (1H, br s), 2.16 (3H, s), 1.58 (3H, d, J = 7.0 Hz).; LC/MS RT 1.82min, m/z $[M-H]^-$ 561,563 |
| 315 | 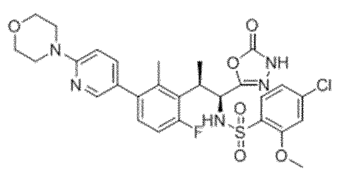 | 1H-NMR (CDCl3) δ: 7.95 (1H, d, J = 2.0 Hz), 7.79 (1H, d, J = 8.5 Hz), 7.32 (1H, dd, J = 8.5, 2.4 Hz), 7.00-6.95 (2H, m), 6.92 (1H, d, J = 2.0 Hz), 6.83 (1H, dd, J = 11.2, 8.7 Hz), 6.65 (1H, d, J = 8.7 Hz), 5.61 (1H, d, J = 10.5 Hz), 4.84 (1H, t, J = 10.5 Hz), 3.93 (3H, s), 3.84-3.80 (4H, m), 3.51-3.44 (5H, m), 2.17 (3H, s), 1.57 (3H, d, J = 7.0 Hz); LC/MS RT 1.48min, m/z $[M-H]^-$ 616,618 |
| 316 | 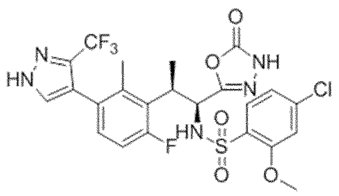 | 1H NMR (CD3OD) δ: 8.53 (1H, s), 7.74 (1H, d, J = 8.4 Hz), 7.61 (1H, s), 7.12-7.09 (1H, m), 7.05-6.98 (2H, m), 6.88-6.83 (1H, m), 4.70-4.60 (2H, m), 3.94 (3H, s), 2.08 (3H, s), 1.52 (3H, d, J = 7.0 Hz); LC/MS RT 1.68min, m/z $[M-H]^-$ 588,590 |
| 317 | 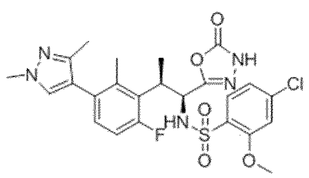 | 1H NMR (CD3OD) δ: 8.27 (1H, br s), 7.75 (1H, d, J = 8.4 Hz), 7.34 (1H, s), 7.12 (1H, d, J = 1.8 Hz), 7.04 (1H, dd, J = 8.4, 1.8 Hz), 6.96 (1H, dd, J = 8.4, 5.9 Hz), 6.84 (1H, dd, J = 11.4, 8.4 Hz), 4.68 (1H, d, J = 11.4 Hz), 3.94 (3H, s), 3.82 (3H, s), 3.67-3.62 (1H, m), 2.13 (3H, s), 1.88 (3H, s), 1.52 (3H, d, J = 7.0 Hz); LC/MS RT 1.63min, m/z $[M-H]^-$ 548,550 |
| 318 | 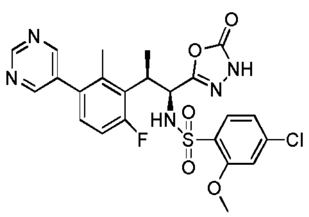 | 1H-NMR (CDCl3) δ: 9.20 (1H, s), 8.60 (2H, s), 8.02 (1H, br s), 7.82 (1H, d, J = 8.1 Hz), 7.05-7.03 (2H, m), 6.97-6.92 (2H, m), 5.53 (1H, d, J = 11.1 Hz), 4.87 (1H, t, J = 11.1 Hz), 3.96 (3H, s), 3.45 (1H, br s), 2.20 (3H, s), 1.61 (3H, d, J = 7.0 Hz).; LC/MS RT 1.55min, m/z $[M-H]^-$ 532,534 |
| 319 | 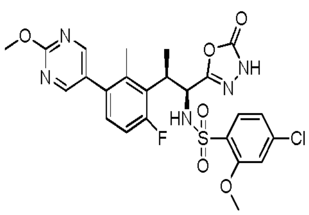 | 1H-NMR (CDCl3) δ: 8.34 (2H, s), 7.81 (1H, d, J = 8.4 Hz), 7.03-6.88 (4H, m), 5.57 (1H, d, J = 10.6 Hz), 4.85 (1H, t, J = 10.6 Hz), 4.04 (3H, s), 3.95 (3H, s), 3.44 (1H, br s), 2.18 (3H, s), 1.59 (3H, d, J = 7.0 Hz); LC/MS RT 1.66min, m/z $[M-H]^-$ 562,564 |

(continued)

| Example | Structural Formula | Physical Property Value |
|---|---|---|
| 320 | 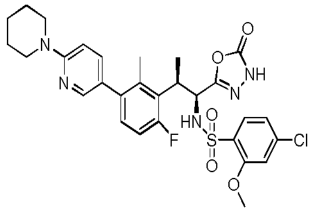 | 1H-NMR (CDCl3) δ: 8.12 (1H, br s), 7.99 (1H, s), 7.80 (1H, d, J = 7.7 Hz), 7.35 (1H, d, J = 8.8 Hz), 7.03-6.93 (3H, m), 6.87-6.81 (1H, m), 6.72 (1H, d, J = 8.4 Hz), 5.52 (1H, d, J = 10.6 Hz), 4.79 (1H, t, J = 10.6 Hz), 3.94 (3H, s), 3.57-3.55 (4H, m), 3.45 (1H, br s), 2.17 (3H, s), 1.69-1.66 (6H, m), 1.59 (3H, d, J = 6.6 Hz).; LC/MS RT 1.49min, m/z [M-H]- 614,616 |
| 321 | 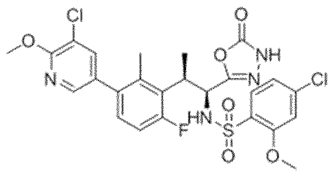 | 1H-NMR (CDCl3) δ: 8.19-8.08 (2H, m), 7.80 (1H, d, J = 8.4 Hz), 7.37-7.27 (1H, m), 7.02 (1H, d, J = 8.4 Hz), 6.95-6.92 (2H, m), 6.88-6.83 (1H, m), 5.50 (1H, d, J = 10.3 Hz), 4.83 (1H, t, J = 11.0 Hz), 3.95-3.94 (3H, m), 3.77 (3H, s), 3.42 (1H, br s), 2.04-2.03 (3H, m), 1.58 (3H, d, J = 6.6 Hz).; LC/MS RT 1.96min, m/z [M-H]- 595,597 |
| 322 | 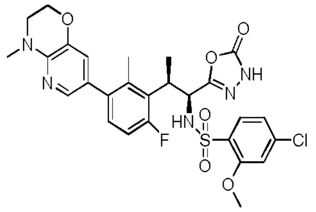 | 1H-NMR (CDCl3) δ: 8.08-8.05 (2H, m), 7.80 (1H, d, J = 8.1 Hz), 7.53 (1H, s), 7.02-6.93 (2H, m), 6.85-6.81 (2H, m), 5.55 (1H, d, J = 10.9 Hz), 4.80 (1H, t, J = 10.9 Hz), 4.28-4.25 (2H, m), 3.94 (3H, s), 3.51 (2H, s), 3.44 (1H, br s), 3.14 (3H, s), 2.17 (3H, s), 1.59 (3H, d, J = 6.2 Hz).; LC/MS RT 1.44min, m/z [M-H]- 602,604 |
| 323 | 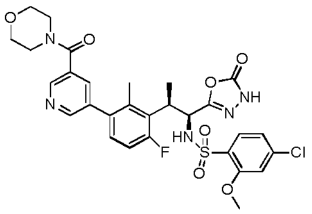 | 1H NMR (CD3OD) δ: 8.60 (1H, s), 8.45 (1H, s), 7.76 (1H, d, J = 8.4 Hz), 7.68 (1H, s), 7.14-7.10 (2H, m), 7.05 (1H, dd, J = 8.4, 1.8 Hz), 6.97 (1H, dd, J = 11.2, 8.4 Hz), 4.70 (1H, d, J = 11.2 Hz), 3.94 (3H, s), 3.77-3.74 (4H, m), 3.69-3.63 (4H, m), 3.48-3.46 (1H, m), 2.20 (3H, s), 1.55 (3H, d, J = 7.0 Hz); LC/MS RT 1.52min, m/z [M-H]- 644,646 |
| 324 | 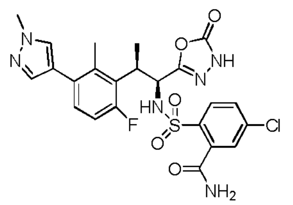 | 1H-NMR (CDCl3) δ: 7.96 (1H, d, J = 8.1 Hz), 7.55-7.51 (2H, m), 7.40 (1H, s), 7.28 (1H, s), 7.04 (1H, dd, J = 8.4, 5.9 Hz), 6.84-6.79 (2H, m), 6.05-6.03 (1H, m), 5.98-5.96 (1H, m), 4.95-4.90 (1H, m), 3.92 (3H, s), 3.50 (1H, br s), 2.28 (3H, s), 1.49 (3H, d, J = 7.3 Hz); LC/MS RT 1.46min, m/z [M-H]- 547,549 |
| 325 | 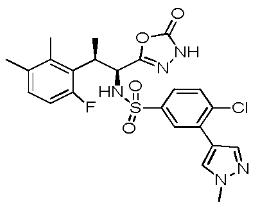 | 1H-NMR (CDCl3) δ: 9.65 (1H, br s), 7.81-7.79 (2H, m), 7.74 (1H, s), 7.60-7.59 (1H, m), 7.50-7.48 (1H, m), 6.95-6.91 (1H, m), 6.73-6.68 (1H, m), 6.20 (1H, d, J = 10.0 Hz), 4.90 (1H, t, J = 10.0 Hz), 3.92 (3H, s), 3.47 (1H, br s), 2.15 (3H, s), 2.13 (3H, s), 1.50 (3H, d, J = 6.6 Hz).; LC/MS RT 1.69min, m/z [M-H]- 518,520 |
| 326 | 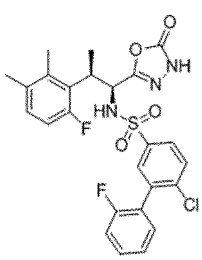 | 1H-NMR (CDCl3) δ: 7.81-7.79 (2H, m), 7.76 (1H, dd, J = 8.5, 2.2 Hz), 7.58 (1H, d, J = 8.3 Hz), 7.46-7.42 (1H, m), 7.31-7.28 (2H, m), 7.17 (1H, t, J = 8.8 Hz), 6.97-6.93 (1H, m), 6.71 (1H, dd, J = 11.7, 8.3 Hz), 5.05 (1H, d, J = 10.0 Hz), 4.85 (1H, t, J = 10.0 Hz), 3.43 (1H, br s), 2.18 (3H, s), 2.16 (3H, s), 1.47 (3H, d, J = 5.9 Hz).; LC/MS RT 1.96min, m/z [M-H]- 532,534 |

(continued)

| Example | Structural Formula | Physical Property Value |
|---|---|---|
| 327 | 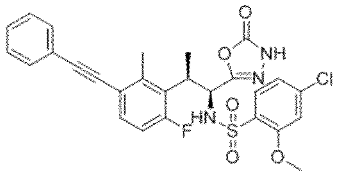 | 1H-NMR (CDCl3) δ: 7.80 (1H, d, J = 8.1 Hz), 7.50-7.47 (2H, m), 7.36-7.33 (5H, m), 7.02 (1H, dd, J = 8.4, 1.8 Hz), 6.93 (1H, d, J = 1.5 Hz), 6.81 (1H, dd, J = 11.4, 8.4 Hz), 5.41 (1H, d, J = 10.5 Hz), 4.82 (1H, t, J = 10.5 Hz), 3.97 (3H, s), 3.42 (1H, br s), 2.51 (3H, s), 1.57-1.54 (3H, m).; LC/MS RT 2.06min, m/z [M-H]- 554,556 |
| 328 | 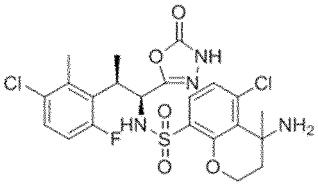 | 1H NMR (CD3OD) δ: 8.34 (1H, br s), 7.76-7.65 (1H, m), 7.25-7.22 (1H, m), 7.17-7.09 (1H, m), 6.90-6.85 (1H, m), 4.79-4.70 (2H, m), 4.59-4.51 (2H, m), 4.41-4.26 (2H, m), 3.72-3.66 (1H, m), 2.38-2.37 (6H, m), 2.33-2.23 (2H, m), 1.86 (3H, d, J = 28.2 Hz).; LC/MS RT 1.39min, m/z [M-H]- 543,545 |
| 329 | 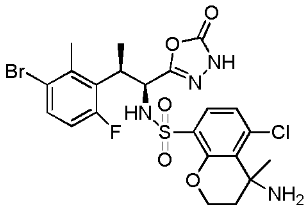 | 1H NMR (CD3OD) δ: 7.68-7.79 (m, 1H), 7.42-7.46 (m, 1H), 7.12-7.20 (m, 1H), 6.79-6.88 (m, 1H), 4.71-4.82 (m, 1H), 4.54-4.65 (m, 1H), 4.25-4.46 (m, 1H), 3.63-3.77 (m, 1H), 2.44 (s, 3H), 2.32-2.38 (m, 2H), 1.89-1.94 (m, 3H), 1.51-1.56 (m, 3H); LC/MS RT 1.39min, m/z [M-H]- 587,589 |
| 330A | 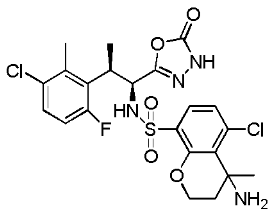 | LC/MS RT 1.37min, m/z [M-H]- 543,545 |
| 330B | 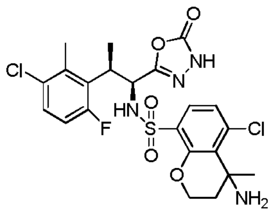 | LC/MS RT 1.37min, m/z [M-H]- 543,545 |
| 331 | 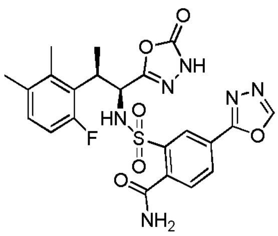 | 1H-NMR (CDCl3) δ: 8.54 (2H, s), 8.42 (1H, s), 8.32-8.30 (1H, m), 7.75 (1H, d, J = 8.1 Hz), 6.96-6.88 (2H, m), 6.67 (1H, dd, J = 11.4, 8.4 Hz), 6.30 (1H, s), 6.10 (1H, s), 4.97 (1H, t, J = 10.3 Hz), 3.48 (1H, s), 2.16 (3H, s), 2.15 (3H, s), 1.48 (3H, d, J = 7.0 Hz).; LC/MS RT 1.5min, m/z [M-H]- 515 |
| 332 | 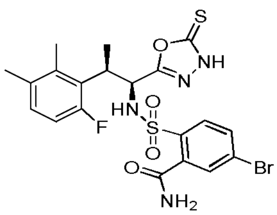 | 1H NMR (CD3OD) δ: 7.68-7.79 (m, 3H), 6.97 (dd, J=8.5, 5.7 Hz, 1H), 6.71 (dd, J=11.7, 8.5 Hz, 1H), 4.89-5.02 (m, 1H), 3.58-3.65 (m, 1H), 2.20 (s, 3H), 2.15 (s, 3H), 1.47 (d, J=7.3 Hz, 3H); LC/MS RT 1.75min, m/z [M-H]- 541,543 |

(continued)

| Example | Structural Formula | Physical Property Value |
|---|---|---|
| 333 | 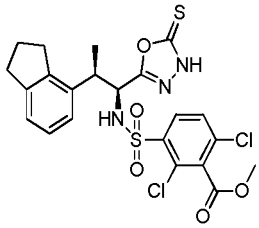 | 1H NMR (CD3OD) δ: 7.73 (d, J=8.8 Hz, 1H), 7.39 (d, J=8.6 Hz, 1H), 6.81-6.99 (m, 3H), 4.15 (d, J=6.2 Hz, 1H), 3.98 (s, 3H), 3.38-3.43 (m, 1H), 2.75-2.92 (m, 4H), 1.92-2.13 (m, 2H), 1.27 (d, J=7.0 Hz, 3H); LC/MS RT 1.95min, m/z [M-H]- 540,542 |
| 334 | 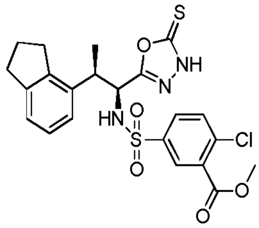 | LC/MS RT 1.89min, m/z [M-H]- 506,508 |
| 335 | 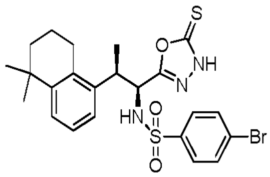 | 1H NMR (CD3OD) δ: 7.65-7.74 (m, 2H), 7.57-7.64 (m, 1H), 7.50 (d, J=3.3 Hz, 1H), 6.98-7.18 (m, 2H), 6.79-6.84 (m, 1H), 3.99 (d, J=9.9 Hz, 1H), 3.40-3.50 (m 1H), 2.70-2.86 (m, 1H), 2.57-2.66 (m, 1H), 2.32-2.51 (m, 2H), 1.82 (s, 3H), 1.45-1.54 (m, 2H), 1.44 (s, 3H), 1.26 (d, J=7.0 Hz, 3H); LC/MS RT 2.11min, m/z [M-H]- 534,536 |
| 336 | 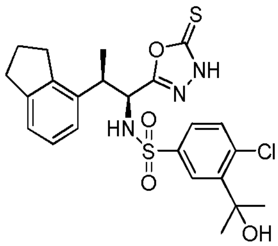 | 1H NMR (CD3OD) δ: 8.02 (d, J=8.4 Hz, 1H), 7.56 (d, J=8.8 Hz, 1H), 6.97-6.99 (m, 3H), 6.88-6.90 (m, 1H), 4.31 (d, J=10.3 Hz, 1H), 3.34-3.46 (m, 1H), 2.71-2.97 (m, 4H), 2.14 (s, 6H), 1.93-2.02 (m, 2H), 1.40 (d, J=7.0 Hz, 3H); LC/MS RT 1.87min, m/z [M-H]- 506,508 |
| 337 | 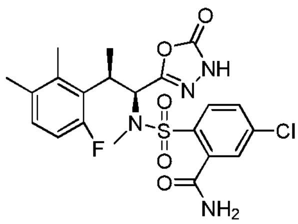 | 1H NMR (CD3OD) : 7.93 (d, J=8.8 Hz, 1H), 7.62 (dd, J=8.8, 2.2 Hz, 1H), 7.51 (d, J=2.2 Hz, 1H), 7.01 (dd, J=8.1, 5.9 Hz, 1H), 6.76 (dd, J=11.9, 8.6 Hz, 1H), 5.58 (dd, J=11.7, 1.8 Hz, 1H), 3.76-3.90 (m, 1H), 3.06 (s, 3H), 2.27 (s, 3H), 2.20 (s, 3H), 1.33 (d, J=7.0 Hz, 3H); LC/MS RT 1.64min, m/z [M-H]- 495,497 |
| 338 | 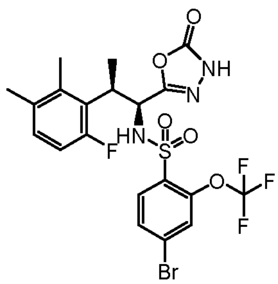 | 1H-NMR (CDCl3) δ: 8.15 (1H, s), 7.86 (1H, d, J = 8.4 Hz), 7.52 (1H, dd, J = 8.4, 1.5 Hz), 7.48-7.47 (1H, m), 6.94 (1H, dd, J = 8.2, 5.7 Hz), 6.69 (1H, dd, J = 11.7, 8.4 Hz), 5.33 (1H, d, J= 9.9 Hz), 4.85 (1H, t, J = 10.3 Hz), 3.50-3.45 (1H, m), 2.18 (6H, s), 1.47 (3H, d, J = 7.0 Hz).; LC/MS RT 1.95min, m/ [M-H]-566,568 |

(continued)

| Example | Structural Formula | Physical Property Value |
|---|---|---|
| 339 | 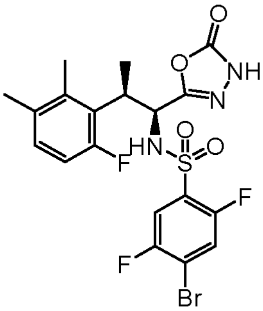 | 1H-NMR (CDCl3) δ: 7.97 (1H, s), 7.64-7.61 (1H, m), 7.40 (1H, dd, J = 8.8, 5.1 Hz), 6.96 (1H, dd, J = 8.1, 5.9 Hz), 6.70 (1H, dd, J = 11.4, 8.4 Hz), 5.40 (1H, d, J = 9.2 Hz), 4.89 (1H, t, J = 9.3 Hz), 3.52-3.47 (1H, m), 2.19 (6H, s), 1.48 (3H, d, J = 7.3 Hz).; LC/MS RT 1.86min, m/ [M-H]-519,521 |
| 340 | 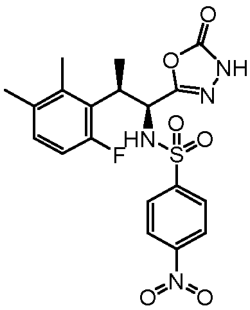 | 1H-NMR (CDCl3) δ: 8.31-8.28 (2H, m), 8.13 (1H, s), 8.02-7.98 (2H, m), 6.95 (1H, dd, J = 8.4, 5.9 Hz), 6.70 (1H, dd, J = 11.7, 8.4 Hz), 5.32-5.29 (1H, m), 4.87 (1H, t, J = 9.9 Hz), 3.48-3.44 (1H, m), 2.17 (3H, s), 2.16 (3H, s), 1.43 (3H, d, J = 7.0 Hz).; LC/MS RT 1.73min, m/ [M-H]-449 |
| 341 | 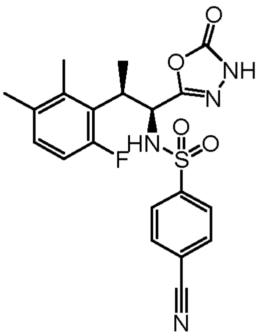 | 1H-NMR (CDCl3) δ: 8.42 (1H, s), 7.94-7.92 (2H, m), 7.77-7.75 (2H, m), 6.95 (1H, dd, J = 8.4, 5.9 Hz), 6.70 (1H, dd, J = 11.7, 8.4 Hz), 5.45 (1H, d, J = 9.5 Hz), 4.84 (1H, t, J = 9.7 Hz), 3.48-3.42 (1H, m), 2.18 (3H, s), 2.15 (3H, s), 1.42 (3H, d, J = 7.0 Hz).; LC/MS RT 1.67min, m/ [M-H]-429 |
| 342 | 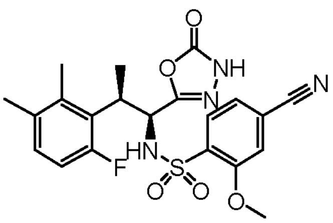 | 1H-NMR (CDCl3) δ: 8.62 (1H, br s), 7.98 (1H, d, J = 8.1 Hz), 7.33 (1H, dd, J = 8.1, 1.1 Hz), 7.20 (1H, d, J = 1.1 Hz), 6.93 (1H, dd, J = 8.4, 5.9 Hz), 6.68 (1H, dd, J = 11.7, 8.4 Hz), 5.55 (1H, d, J = 10.3 Hz), 4.84 (1H, t, J = 10.6 Hz), 4.01 (3H, s), 3.43 (1H, br s), 2.17 (6H, s), 1.52 (3H, d, J = 7.0 Hz).; LC/MS RT 1.71min, m/ [M-H]-459,461 |
| 343 | 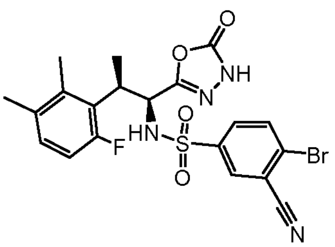 | 1H-NMR (CD3OD) δ: 8.25 (1H, s), 8.09 (1H, d, J = 2.2 Hz), 7.95-7.86 (2H, m), 7.00-6.96 (1H, m), 6.72 (1H, dd, J = 11.5, 8.2 Hz), 4.74 (1H, d, J = 11.4 Hz), 3.57-3.54 (1H, m), 2.18 (3H, s), 2.17 (3H, s), 1.45 (3H, d, J = 7.0 Hz).; LC/MS RT 1.78min, m/ [M-H]-507,509 |

(continued)

| Example | Structural Formula | Physical Property Value |
|---|---|---|
| 344 | 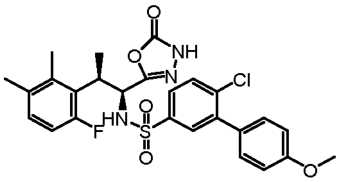 | 1H-NMR (CDCl3) δ: 7.98 (1H, s), 7.77 (1H, d, J = 2.6 Hz), 7.65 (1H, dd, J = 8.4, 2.6 Hz), 7.52 (1H, d, J = 8.4 Hz), 7.36-7.34 (2H, m), 6.99-6.92 (4H, m), 6.70 (1H, dd, J = 11.7, 8.4 Hz), 5.11 (1H, d, J = 10.0 Hz), 4.85 (1H, t, J = 10.1 Hz), 3.85 (3H, s), 3.43-3.41 (1H, m), 2.17 (3H, s), 2.15 (3H, s), 1.45 (3H, d, J = 6.2 Hz).; LC/MS RT 1.97min, m/ [M-H]-544,546 |
| 345 | 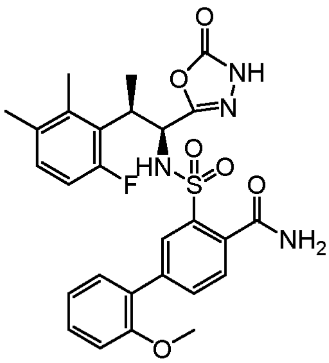 | 1H-NMR (CDCl3) δ: 8.08 (1H, d, J = 1.8 Hz), 7.71 (2H, dd, J = 7.9, 1.6 Hz), 7.60 (1H, d, J = 8.1 Hz), 7.39-7.35 (1H, m), 7.28-7.26 (2H, m), 7.19 (1H, d, J = 10.3 Hz), 7.07-7.03 (1H, m), 7.00-6.98 (1H, m), 6.89 (1H, dd, J = 8.4, 5.9 Hz), 6.67 (1H, dd, J = 11.4, 8.4 Hz), 6.06 (1H, s), 5.90 (1H, s), 4.95 (1H, t, J = 10.6 Hz), 3.85 (3H, s), 3.46-3.44 (1H, m), 2.16 (3H, s), 2.15 (3H, s), 1.50 (3H, d, J = 7.0 Hz).; LC/MS RT 1.8min, m/ [M-H]-553,555 |
| 346 | 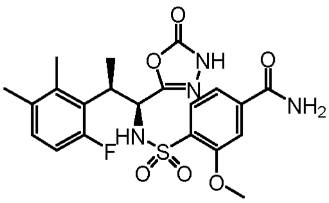 | 1H-NMR (CDCl3) δ: 10.58 (1H, s), 7.93 (1H, d, J = 8.4 Hz), 7.36-7.34 (2H, m), 6.91 (1H, dd, J = 8.2, 5.7 Hz), 6.68 (1H, dd, J = 11.7, 8.4 Hz), 6.44 (2H, s), 5.56 (1H, d, J = 10.3 Hz), 4.84 (1H, t, J = 11.0 Hz), 4.02 (3H, s), 3.42 (1H, br s), 2.17-2.16 (6H, m), 1.56 (3H, d, J = 7.0 Hz).; LC/MS RT 1.51min, m/ [M-H]-477 |
| 347 | 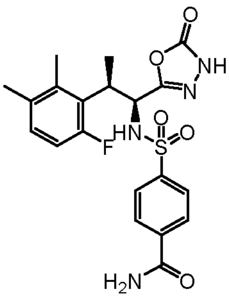 | 1H-NMR (CD3OD) δ: 7.96-7.93 (2H, m), 7.86-7.83 (2H, m), 6.96 (1H, dd, J = 8.4, 5.9 Hz), 6.71 (1H, dd, J = 11.7, 8.4 Hz), 4.74 (1H, d, J = 11.0 Hz), 3.57-3.53 (1H, m), 2.17 (3H, s), 2.15 (3H, s), 1.42 (3H, d, J = 7.0 Hz).; LC/MS RT 1.46min, m/ [M-H]-447 |
| 348 | 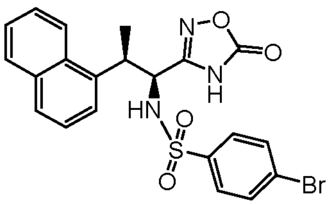 | LC/MS RT min, m/ [M-H]-486,488 |
| 349 | 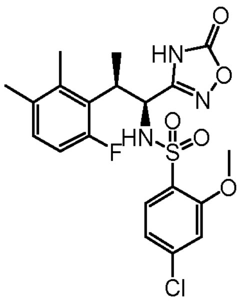 | 1H-NMR (CDCl3) δ: 7.80 (1H, d, J = 8.4 Hz), 7.05 (1H, dd, J = 8.4, 1.8 Hz), 6.96-6.93 (2H, m), 6.68 (1H, dd, J = 11.7, 8.4 Hz), 5.96 (1H, d, J = 9.9 Hz), 4.78 (1H, t, J = 9.7 Hz), 3.91 (3H, s), 3.54-3.49 (1H, m), 2.17 (3H, s), 2.13 (3H, s), 2.49 (3H, d, J = 7.0 Hz).; LC/MS RT 1.77min, m/ [M-H]-468,470 |

(continued)

| Example | Structural Formula | Physical Property Value |
|---|---|---|
| 350 | 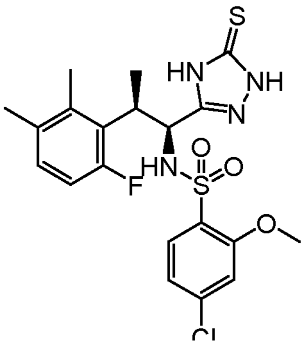 | 1H-NMR (CDCl3) δ: 11.70 (1H, s), 11.48 (1H, s), 7.75 (1H, d, J = 8.4 Hz), 6.92-6.89 (2H, m), 6.73-6.68 (1H, m), 6.62 (1H, d, J = 8.4 Hz), 6.16 (1H, d, J = 9.2 Hz), 4.89 (1H, t, J = 10.3 Hz), 3.85 (3H, s), 3.60-3.58 (1H, m), 2.16 (3H, s), 2.13 (3H, s), 1.47 (3H, d, J = 6.6 Hz).; LC/MS RT 1.6min, m/ [M-H]-483,485 |

Test Example

The compound according to the present invention was evaluated using the following test method.

[Test Example 1 Human RNR inhibition effect]

The inhibitory activity against the ribonucleotide reduction reaction (RNR inhibitory activity) of the Example compound was determined by measuring the formation of deoxycytidine diphosphate (hereinafter referred to as dCDP) from cytidine diphosphate (hereinafter referred to as CDP) by the following method.

Human M1 subunit (isoform 1, GenBank accession No: NM_001033), to which a histidine tag is fused at the amino terminus, and human M2 subunit (mutant lacking amino terminal 59 amino acids of isoform 2, GenBank accession No: NM_001034), to which a histidine tag is fused, were overexpressed in Escherichia coli and were solubilized after collection, and histidine tagged human M1 and histidine tagged human M2 proteins were purified on a nickel chelate column. A mixture of the histidine tagged human M1 and histidine tagged human M2 proteins was used as RNR in the ribonucleotide reduction reaction. [to]

For measuring the inhibitory activity of the Example compound against the ribonucleotide reduction reaction, the method described in the document [CANCER RESEARCH 64, 1 - 6, 2004] was referred to.

First, Example compounds were serially diluted with DMSO. Next, human M1 protein and human M2 protein were added to a 0.02% aqueous albumin solution derived from fetal bovine serum, a DMSO solution of the Example compound or the control DMSO solution (final concentration of DMSO was 1%) was added, and the mixture was allowed to stand for 20 minutes. Thereafter, the reaction buffer [50 mM HEPES buffer (pH 7.2) at the final concentration, 4 mM magnesium acetate at the final concentration, 100 mM potassium chloride at the final concentration, 6 mM dithiothreitol at the final concentration, 2 mM adenosine triphosphate at the final concentration, 0.24 mM nicotinamide adenine dinucleotide phosphate at final concentration] and 10 μM CDP at the final concentration were added and incubated at 37°C for 30 minutes to perform ribonucleotide reduction reaction. Immediately after the reaction, the reaction was stopped by heating at 100°C for 15 minutes, followed by centrifugation at 10,000 rpm for 10 minutes. After the centrifugation, a portion (5 μL) of the resulting supernatant was analyzed with a high performance liquid chromatography (Shimadzu Corporation, Prominence) using Shim-pack XR-ODS (manufactured by Shimadzu GLC Co., 3.0 × 100 mm). Elution was carried out at a measurement wavelength of 265 nm at a flow rate of 0.5 mL / min by a 9-minute concentration gradient from the 12:13 mixture of mobile phase A (10 mM potassium dihydrogen phosphate (pH 6.7), 10 mM tetrabutylammonium, 0.25% methanol) and mobile phase B (50 mM potassium dihydrogen phosphate (pH 6.7), 5.6 mM tetrabutylammonium, 30% methanol) to the same 2: 3 mixture to measure the substrate CDP (RT 5.9 min) and the reaction product dCDP (RT 6.2 min).

The inhibitory activity of the Example compound was determined by the following equation, and the concentrations of Example compounds inhibiting the ribonucleotide reduction reaction by 50% are shown as $IC_{50}$ (μM) in Table 22.

[Mathematical Formula 1]

$$\text{Inhibition rate (\%)} = \left[ 1 - \frac{\text{Amount of produced dCDP where test compound added (pmol)}}{\text{Amount of produced dCDP of control (pmol)}} \right] \times 100$$

As a result, it is apparent from the following table that the sulfonamide compound represented by formula (I) has an excellent RNR inhibitory action.

[Table 22]

| Example Number | RNR inhibitory activity $IC_{50}$ (μM) | Example Number | RNR inhibitory activity $IC_{50}$ (μM) | Example Number | RNR inhibitory activity $IC_{50}$ (μM) | Example Number | RNR inhibitory activity $IC_{50}$ (μM) |
|---|---|---|---|---|---|---|---|
| 1 | 0.06 | 39 | 0.04 | 100 | 0.30 | 152 | 0.71 |
| 3 | 0.30 | 40 | 0.13 | 101 | 0.13 | 153 | 0.11 |
| 4 | 0.38 | 41 | 0.10 | 102 | 0.14 | 155 | 0.13 |
| 5 | 0.14 | 42 | 0.20 | 103 | 0.41 | 156 | 0.08 |
| 6 | 0.11 | 43 | 0.08 | 104 | 0.84 | 157 | 0.10 |
| 7 | 0.45 | 46 | 0.84 | 105 | 0.16 | 158 | 0.45 |
| 9 | 0.60 | 48 | 0.60 | 106 | 0.27 | 159 | 0.16 |
| 10 | 0.14 | 49 | 0.80 | 107 | 0.24 | 161 | 0.28 |
| 11 | 0.18 | 50 | 0.85 | 108 | 0.43 | 162 | 0.74 |
| 12 | 0.17 | 52 | 0.77 | 109 | 0.06 | 164 | 0.33 |
| 13 | 0.14 | 60 | 0.99 | 110 | 0.96 | 165 | 0.83 |
| 14 | 0.25 | 67 | 0.70 | 111 | 0.27 | 167 | 0.08 |
| 15 | 0.10 | 71 | 0.24 | 112 | 0.15 | 169 | 0.19 |
| 16 | 0.13 | 76 | 0.20 | 113 | 0.06 | 171 | 0.47 |
| 17 | 0.50 | 81 | 0.28 | 114 | 0.06 | 172 | 0.82 |
| 18 | 0.13 | 83 | 0.14 | 115 | 0.18 | 173 | 0.13 |
| 19 | 0.19 | 84 | 0.36 | 116 | 0.07 | 174 | 0.35 |
| 20 | 0.26 | 85 | 0.84 | 117 | 0.03 | 176 | 0.81 |
| 21 | 0.24 | 86 | 0.40 | 118 | 0.34 | 178 | 0.17 |
| 22 | 0.34 | 87 | 0.84 | 119 | 0.45 | 179 | 0.28 |
| 23 | 0.74 | 88 | 0.15 | 120 | 0.43 | 181 | 0.66 |
| 25 | 0.15 | 89 | 0.42 | 123 | 0.11 | 182 | 0.41 |
| 26 | 0.16 | 90 | 0.16 | 124 | 0.09 | 183 | 0.32 |
| 27 | 0.55 | 91 | 0.23 | 129 | 0.10 | 184 | 0.22 |

(continued)

| Example Number | RNR inhibitory activity $IC_{50}$ ($\mu$M) | Example Number | RNR inhibitory activity $IC_{50}$ ($\mu$M) | Example Number | RNR inhibitory activity $IC_{50}$ ($\mu$M) | Example Number | RNR inhibitory activity $IC_{50}$ ($\mu$M) |
|---|---|---|---|---|---|---|---|
| 28 | 0.50 | 92 | 0.20 | 137 | 0.59 | 185 | 0.60 |
| 30 | 0.15 | 93 | 0.1 | 142 | 0.21 | 186 | 0.09 |
| 31 | 0.1 | 94 | 0.11 | 144 | 0.17 | 188 | 0.64 |
| 32 | 0.79 | 95 | 0.14 | 145 | 0.44 | 189 | 0.55 |
| 35 | 0.13 | 96 | 0.10 | 146 | 0.26 | 192 | 0.44 |
| 36 | 0.11 | 97 | 0.24 | 147 | 0.27 | 193 | 0.09 |
| 37 | 0.14 | 98 | 0.64 | 148 | 0.10 | 194 | 0.36 |
| 38 | 0.19 | 99 | 0.29 | 151 | 0.41 | 195 | 0.18 |
| | | | | | | | |
| Example Number | RNR inhibitory activity $IC_{50}$ ($\mu$M) | Example Number | RNR inhibitory activity $IC_{50}$ ($\mu$M) | Example Number | RNR inhibitory activity $IC_{50}$ ($\mu$M) | Example Number | RNR inhibitory activity $IC_{50}$ ($\mu$M) |
| 196 | 0.08 | 224A | 0.09 | 247 | 0.41 | 283 | 0.15 |
| 197 | 0.06 | 224B | 0.10 | 248 | 0.27 | 284 | 0.56 |
| 198 | 0.06 | 225A | 0.12 | 249 | 0.10 | 285 | 0.31 |
| 199 | 0.35 | 226A | 0.05 | 250 | 0.02 | 286 | 0.07 |
| 200A | 0.03 | 226B | 0.07 | 251 | 0.25 | 287 | 0.05 |
| 200B | 0.08 | 227A | 0.05 | 252 | 0.06 | 288 | 0.06 |
| 201 | 0.17 | 227B | 0.09 | 253 | 0.08 | 289 | 0.13 |
| 202 | 0.40 | 228A | 0.08 | 254 | 0.07 | 290 | 0.84 |
| 203 | 0.18 | 228B | 0.14 | 255 | 0.12 | 292 | 0.16 |
| 204 | 0.15 | 229A | 0.06 | 256 | 0.42 | 294 | 0.11 |
| 205 | 0.08 | 229B | 0.11 | 257 | 0.10 | 295 | 0.79 |
| 206A | 0.15 | 230A | 0.12 | 258 | 0.14 | 298 | 0.30 |
| 207A | 0.13 | 230B | 0.05 | 259 | 0.10 | 299 | 0.94 |
| 207B | 0.09 | 231 | 0.65 | 260 | 0.36 | 300 | 0.34 |
| 208A | 0.10 | 232 | 0.23 | 261 | 0.09 | 301 | 0.29 |
| 208B | 0.06 | 233 | 0.13 | 262 | 0.13 | 302 | 0.49 |
| 209A | 0.10 | 234A | 0.31 | 263 | 0.07 | 303 | 0.16 |
| 209B | 0.18 | 235A | 0.08 | 264 | 0.06 | 304 | 0.16 |
| 210 | 0.18 | 235B | 0.07 | 265 | 0.26 | 305 | 0.24 |
| 211A | 0.12 | 236 | 0.38 | 266 | 0.85 | 306 | 0.09 |
| 212 | 0.11 | 237A | 0.29 | 269 | 0.51 | 308 | 0.18 |
| 213 | 0.50 | 238A | 0.11 | 270 | 0.73 | 310 | 0.17 |
| 214 | 0.99 | 239A | 0.20 | 271 | 0.23 | 311 | 0.22 |

(continued)

| Example Number | RNR inhibitory activity $IC_{50}$ (μM) | Example Number | RNR inhibitory activity $IC_{50}$ (μM) | Example Number | RNR inhibitory activity $IC_{50}$ (μM) | Example Number | RNR inhibitory activity $IC_{50}$ (μM) |
|---|---|---|---|---|---|---|---|
| 215 | 0.19 | 240 | 0.91 | 272 | 0.66 | 312 | 0.22 |
| 216 | 0.20 | 241 | 0.14 | 273 | 0.13 | 313 | 0.34 |
| 217 | 0.96 | 242 | 0.23 | 274 | 0.44 | 314 | 0.26 |
| 219 | 0.27 | 243A | 0.07 | 275 | 0.10 | 315 | 0.19 |
| 220A | 0.06 | 243B | 0.10 | 277 | 0.37 | 317 | 0.28 |
| 220B | 0.08 | 244A | 0.09 | 278 | 0.13 | 318 | 0.54 |
| 222A | 0.08 | 244B | 0.22 | 280 | 0.42 | 319 | 0.28 |
| 222B | 0.06 | 245 | 0.04 | 281 | 0.76 | 320 | 0.60 |
| 223 | 0.79 | 246 | 0.50 | 282 | 0.91 | 322 | 0.22 |

| Example Number | RNR inhibitory activity $IC_{50}$ (μM) | Example Number | RNR inhibitory activity $IC_{50}$ (μM) |
|---|---|---|---|
| 323 | 0.42 | 338 | 0.10 |
| 324 | 0.17 | 339 | 0.51 |
| 325 | 0.22 | 340 | 0.27 |
| 328 | 0.11 | 341 | 0.41 |
| 329 | 0.06 | 342 | 0.09 |
| 330A | 0.12 | 343 | 0.64 |
| 330B | 0.46 | 344 | 0.72 |
| 331 | 0.09 | 345 | 0.25 |
| 332 | 0.13 | 346 | 0.42 |
| 333 | 0.31 | 349 | 0.25 |
| 334 | 0.83 | 350 | 0.46 |
| 337 | 0.08 | | |

[Test Example 2 Cell proliferation inhibitory effect on human breast cancer cell line]

Human derived breast cancer cell line HCC 1806 cells (American Type Culture Collection, ATCC) were daily passaged at a cell density not exceeding 80% in ATCC recommended Roswell Park Memorial Institute medium (RPMI-1640) containing 10% fetal bovine serum (FBS). In order to start the test of cell proliferation inhibitory activity, HCC 1806 cells were suspended in the above medium, after seeing at 180 μL in each well of a 96-well flat bottom plate so that the number of cells per well was 2,000, the cells were cultured at 37°C for 1 day in an incubator containing 5% carbon dioxide gas.

On the next day, the Example compound was dissolved in DMSO, and 20 μL of a drug additive solution diluted serially with distilled water to 10 times of the final concentration was added to each well of the culture plate of the cells, and the cells were cultured at 37°C for 72 hours in an incubator containing 5% carbon dioxide gas. After culturing for 72 hours, 20 μL of glutaraldehyde was added to each well and allowed to stand for 30 minutes, then the plate was washed 10 times with water and was dried. 100 μL of a stain solution (0.05% crystal violet in a 20% methanol solution) was added to each well and allowed to stand for 30 minutes, then the plate was washed 10 times with water and was

dried. 100 μL of an extract solution (0.1 N $NaH_2PO_4$: 100% ethanol = 1:1) was added to each well and mixed, and the mixture was measured at a wavelength of 540 nm using a plate reader (MTP-450 manufactured by Corona Electric Co., Ltd.).

The growth inhibition rate was calculated from the following formula, and the concentration ($IC_{50}$ (μM)) of a compound inhibiting 50% was determined. The results are shown in Table 23.

$$\text{Growth inhibition rate (\%)} = \{(C-B) - (T-B)\} / (C-B) \times 100$$

T: Absorbance of well to which Example compound was added

C: Absorbance of wells to which no Example compound was added

B: Absorbance of wells to which no cell was added

As a result, as is clear from the following Table 23, it was revealed that all the sulfonamide compounds represented by formula (I) have growth inhibitory activity against human-derived breast cancer cells.

[Table 23]

| Example Number | Cell growth suppression $IC_{50}$ (μM) | Example Number | Cell growth suppression $IC_{50}$ (μM) | Example Number | Cell growth suppression $IC_{50}$ (μM) | Example Number | Cell growth suppression $IC_{50}$ (μM) |
|---|---|---|---|---|---|---|---|
| 1 | 0.16 | 113 | 0.60 | 216 | 0.67 | 257 | 0.20 |
| 5 | 0.20 | 114 | 0.37 | 220A | 0.37 | 258 | 0.58 |
| 6 | 0.29 | 116 | 0.32 | 220B | 0.50 | 259 | 0.35 |
| 10 | 0.56 | 117 | 0.31 | 222A | 0.06 | 261 | 0.72 |
| 11 | 0.64 | 123 | 0.17 | 222B | 0.67 | 262 | 0.17 |
| 12 | 0.50 | 129 | 0.08 | 224A | 0.10 | 264 | 0.76 |
| 13 | 0.31 | 144 | 0.96 | 224B | 0.77 | 273 | 0.81 |
| 14 | 0.56 | 146 | 0.83 | 225A | 0.60 | 275 | 0.37 |
| 15 | 0.40 | 147 | 0.65 | 226A | 0.08 | 278 | 0.59 |
| 18 | 0.58 | 148 | 0.40 | 226B | 0.30 | 288 | 0.15 |
| 19 | 0.94 | 153 | 0.91 | 227A | 0.19 | 289 | 0.60 |
| 25 | 0.59 | 156 | 0.87 | 228A | 0.14 | 292 | 0.75 |
| 26 | 0.98 | 157 | 0.37 | 229A | 0.31 | 294 | 0.39 |
| 30 | 0.80 | 167 | 0.14 | 230A | 0.29 | 303 | 0.99 |
| 35 | 0.67 | 186 | 0.64 | 230B | 0.78 | 304 | 0.94 |
| 37 | 0.82 | 193 | 0.11 | 232 | 0.33 | 308 | 0.87 |
| 39 | 0.23 | 196 | 0.32 | 233 | 0.28 | 310 | 0.35 |
| 40 | 0.59 | 197 | 0.41 | 234A | 0.57 | 311 | 0.52 |
| 41 | 0.40 | 198 | 0.05 | 235A | 0.13 | 312 | 0.87 |
| 43 | 0.28 | 200A | 0.05 | 235B | 0.40 | 315 | 0.93 |
| 71 | 0.79 | 200B | 0.46 | 238A | 0.44 | 328 | 0.41 |
| 76 | 0.44 | 203 | 0.81 | 239A | 0.72 | 329 | 0.24 |
| 83 | 0.50 | 204 | 0.15 | 241 | 0.49 | 330A | 0.24 |
| 91 | 0.98 | 205 | 0.25 | 243A | 0.29 | 337 | 0.30 |

(continued)

| Example Number | Cell growth suppression $IC_{50}$ (μM) | Example Number | Cell growth suppression $IC_{50}$ (μM) | Example Number | Cell growth suppression $IC_{50}$ (μM) | Example Number | Cell growth suppression $IC_{50}$ (μM) |
|---|---|---|---|---|---|---|---|
| 93 | 0.28 | 206A | 0.57 | 243B | 0.70 | | |
| 94 | 0.48 | 207A | 0.07 | 244A | 0.72 | | |
| 95 | 0.14 | 207B | 0.25 | 245 | 0.15 | | |
| 96 | 0.95 | 208A | 0.33 | 249 | 0.14 | | |
| 100 | 0.39 | 208B | 0.05 | 250 | 0.12 | | |
| 101 | 0.81 | 209A | 0.06 | 252 | 0.74 | | |
| 102 | 0.66 | 209B | 0.82 | 253 | 0.23 | | |
| 106 | 0.38 | 211A | 0.85 | 254 | 0.25 | | |
| 109 | 0.40 | 212 | 0.26 | 255 | 0.48 | | |

[Test Example 3 Cell proliferation inhibitory effect on human cancer-derived cancer cell lines]

According to the method of Test Example 2, the cell proliferation inhibitory effect on various cancer cell lines as described in Table 24 was evaluated. NCI-H460, CFPAC-1, MSTO-211H, DU145, ACHN, HCT116, NCI-H2228 and NCI-H2170 cells were purchased from ATCC, A2780 and RPMI7932 cells were purchased from European Collection of Cell Cultures, GB-1 and HLE cells were purchased from JCRB Cell Bank, A673 cells were purchased from DS Pharma Biomedical Co., Ltd., and NUGC-3 cells were purchased from Health Science Research Resources Bank.

As a result, as is clear from the following table, it was revealed that the sulfonamide compounds represented by formula (I) have growth inhibitory activity against various types of cancer cells derived from humans.

[Table 24]

| cell line | | NUGC-3 | NCI-H460 | CFPAC-1 | A673 | GB-1 | HLE | MSTO-211H |
|---|---|---|---|---|---|---|---|---|
| Carcinoma type | | Stomach Cancer | Lung Cancer | Pancreatic Cancer | Ewing's sarcoma | Glioblastoma | Liver Cancer | Mesothelioma |
| Culture medium | | RPMI-1640 + 10 % FBS | ATCC recommended RPMI-1640 + 10 % FBS | IMDM + 10 % FBS | DMEM + 10 % FBS | DMEM + 10 % FBS | DMEM + 10 % FBS | ATCC recommended RPMI-1640 + 10 % FBS |
| cell number (cell/well) | | 2000 | 1000 | 2000 | 2000 | 3000 | 3000 | 6000 |
| IC50 (μM) | Example 5 | 1.22 | 0.73 | 0.94 | 1.09 | 1.57 | 0.79 | 0.70 |
| | Example 235A | 0.71 | 0.35 | 0.35 | 0.61 | 1.12 | 0.42 | 0.39 |
| | Example 11 | 3.11 | 1.50 | 1.71 | 2.56 | 5.22 | 1.74 | 1.54 |
| | Example 1 | 1.12 | 0.57 | 0.54 | 0.92 | 1.56 | 0.56 | 0.65 |
| | Example 14 | 2.83 | 1.35 | 1.42 | 1.85 | 4.60 | 1.30 | 1.58 |
| | Example 209A | 0.40 | 0.25 | 0.33 | 0.32 | 0.64 | 0.26 | 0.32 |
| | Example 222A | 0.36 | 0.18 | 0.23 | 0.25 | 0.46 | 0.20 | 0.27 |
| | Example 200A | 0.27 | 0.13 | 0.17 | 0.18 | 0.37 | 0.14 | 0.17 |
| | Example 228A | 0.51 | 0.31 | 0.36 | 0.40 | 0.85 | 0.29 | 0.37 |

| cell line | | DU145 | A2780 | ACHN | HCT116 | RPMI7932 | NCI-H2228 | NCI-H2170 |
|---|---|---|---|---|---|---|---|---|
| Carcinoma type | | Prostate Cancer | Ovarian Cancer | Kidney Cancer | Colorectal Cancer | Melanoma | Lung Cancer | Lung Cancer |
| Culture medium | | EMEM + 0.1 mM non-essential amino acid + 1 mM sodium pyruvate + 10 % FBS | RPMI-1640 + 10% FBS | EMEM + 10 % FBS | McCoy's 5A + 10 % FBS | RPMI-1640 + 10 % FBS | ATCC recommended RPMI-1640 + 10 % FBS | ATCC recommended RPMI-1640 + 10 % FBS |
| cell number (cell/well) | | 5000 | 2000 | 2000 | 1000 | 4000 | 5000 | 5000 |
| IC50 (μM) | Example 5 | 1.04 | 0.83 | 0.75 | 0.91 | 2.67 | 1.27 | 1.89 |
| | Example 235A | 0.53 | 0.40 | 0.38 | 0.48 | 1.23 | 0.88 | 1.10 |
| | Example 11 | 1.84 | 2.08 | 1.50 | 2.30 | 4.74 | 3.21 | 3.90 |
| | Example 1 | 0.73 | 0.63 | 0.68 | 0.75 | 1.74 | 1.35 | 1.41 |
| | Example 14 | 2.22 | 1.71 | 0.98 | 2.20 | 3.21 | 3.53 | 4.18 |
| | Example 209A | 0.31 | 0.30 | 0.22 | 0.28 | 0.72 | 0.73 | 0.57 |
| | Example 222A | 0.26 | 0.19 | 0.17 | 0.27 | 0.51 | 0.48 | 0.52 |
| | Example 200A | 0.17 | 0.13 | 0.13 | 0.22 | 0.43 | 0.50 | 0.49 |
| | Example 228A | 0.34 | 0.38 | 0.32 | 0.38 | 0.65 | 0.74 | 0.88 |

[Test Example 4 Evaluation of Antitumor Effect Using Human-Derived Blood Cancer Cell Line (MV-4-11) Subcutaneous Transplantation Model (In vivo)]

A human-derived blood cancer cell line MV-4-11 was transplanted subcutaneously into a nude mouse (BALB/cA Jcl-nu/nu, CLEA Japan, Inc.), and at the time when the tumor volume of the nude mouse on which the engrafted tumor reached 100 to 300 $mm^3$, four mice were assigned to each group by random stratification so that the average of the tumor volumes of each group was uniform (day 0), and the Example compound was orally administered daily at 100 mg / kg / day once per day for 14 days. Example Compound dosing solutions were prepared using 0.5% HPMC.

An electric balance for animals was used in body weight measurement. Rate of change in body weight on day n (BWCn) from body weight on day n (BWn) was calculatedaccording to the following equation.

$$\text{Rate of change in body weight BWCn (\%)} = (\text{BWn} - \text{BW0}) / \text{BW0} \times 100$$

Tumor volume (TV) was calculated according to the following equation by sandwiching and measuring the major axis and the minor axis with a digital caliper.

$$\text{Tumor volume (mm}^3\text{)} = \text{Major axis (mm)} \times \text{Minor axis (mm)} \times \text{Minor axis (mm)} / 2$$

In order to compare the chronological transition of proliferation of tumor for the administration of each compound, relative tumor volume (RTV) setting the tumor volume at the time of grouping as 1 as the tumor proliferation rate was calculated according to the following formula, and the transition of the average value of RTV of each individual are shown in Figs. 1 to 4.

$$\text{RTV} = \text{(tumor volume at the day of tumor volume measurement)} / \text{(tumor volume at the time of the grouping)}$$

When the average RTV value of the Example compound-administered group on the final evaluation day is smaller than the average RTV value of the control group, and a statistically significant difference (Student-t test) is shown, the Example compound was determined to be significantly effective, and the statically significant difference is marked with * in the figure (*: $p < 0.05$).

As a result, it was revealed that all the sulfonamide compounds represented by formula (I) shows a significant antitumor effect.

[Test Example 5 Demonstration of Effect of Combination of Sulfonamide Compound and Other antitumor agent]

Distributors of reagents, distributors of tumor cell lines, media, and the numbers of cells to be seeded, used in this Test Example are shown in the following Tables 25 and 26.

[Table 25]

| Reagent | Supplier |
|---|---|
| CELLect® Fetal Bovine Serum (FBS) | MP Biomedicals, LLC. |
| Fetal Bovine Serum, dialyzed, US origin (D-FBS) | Thermo Fisher Scientific, Inc. |
| DMEM (High Glucose) with L-Glutamine and Phenol Red (DMEM) | Wako Pure Chemical Industries, Ltd. |
| IMDM | Thermo Fisher Scientific, Inc. |
| McCoy's 5A (Modified) Medium (McCoy's 5A) | Thermo Fisher Scientific, Inc. |
| 2-Fluoroadenine-9-β-D-arabinofuranoside (Fludarabine nucleoside) | Sigma-Aldrich Japan |
| Cytosine β-D-arabinofuranoside hydrochloride (Cytarabine) | Sigma-Aldrich Japan |
| 5-Aza-2'-deoxycytidine (Decitabine) | Sigma-Aldrich Japan |
| SGI-110 (Guadecitabine) (Guadecitabine) | Adooq Bioscience, LLC. |

(continued)

| Reagent | Supplier |
|---|---|
| Gemcitabine (Gemcitabine) | Taiho Pharmaceutical Co., Ltd. |
| FTD (Trifluridine) | Yuki Gosei Kogyo Co., Ltd. |
| 5-Fluorouracil (5-Fluorouracil) | Wako Pure Chemical Industries, Ltd. |
| 5-Azacytidine (Azacytidine) | Sigma-Aldrich Japan |
| AZD6738 | Adooq Bioscience, LLC. |
| LY2606368 (Prexasertib) | Medchemexpress Co., Ltd. |
| SCH900776 | Adooq Bioscience, LLC. |
| Briplatin® Injection 10 mg (Cisplatin) | Bristol-Myers Squibb Company |
| Oxaliplatin (Oxaliplatin) | Tokyo Chemical Industry Co., Ltd. |
| Paraplatin®Injection 450 mg (Carboplatin) | Bristol-Myers Squibb Company |
| Etoposide (Etoposide) | Sigma-Aldrich Japan |
| Sunitinib, Free Base (Sunitinib) | LC Laboratories, Inc. |
| Cabozantinib (XL-184, salt form) (Cabozantinib) | Taiho Pharmaceutical Co., Ltd. |
| PKC-412 (Midostaurin) | Santa Cruz Biotechnology, Inc. |
| Lapatinib, Di-p-Toluenesulfonate Salt (Lapatinib) | LC Laboratories, Inc. |
| Luminespib (AUY-922, NVP-AUY922) (Luminespib) | Selleck Chemicals, LLC. |
| Olaparib (AZD2281, Ku-0059436) (Olaparib) | Selleck Chemicals, LLC. |
| BMN-673 (Talazoparib) | Chemscene, LLC. |

[Table 26]

| Tumor cell line (origin) | Distributor of cell line | Medium | The number of cells to be seeded per well (number) |
|---|---|---|---|
| A549 (Human lung cancer) | Dainippon Pharmaceutical Co., Ltd. (present DS PHARMA BIOMEDICAL CO., LTD. ) | DMEM containing 10% FBS (DMEM containing 10% D-FBS for trifluridine evaluation) | 1500 |
| MV-4-11 (Human blood cancer) | ATCC | IMDM containing 10% FBS | 1500 |
| CFPAC-1 (Human pancreatic cancer) | ATCC | IMDM containing 10% FBS | 2000 |
| HCT116 (Human large intestine cancer) | ATCC | McCoy's 5A containing 10% FBS | 1000 |

Each cell line was seeded at 90 μL/well to a 96-well culture plate (Thermo Fisher Scientific, Inc.) according to the above tables. The cell-seeded plate was cultured in an incubator set to 37°C and 5% $CO_2$. On the day following seeding, varying concentrations of the sulfonamide compound and the other antitumor agent were added in combination to the cells. Specifically, ten serial dilutions (including 0 nM) was prepared as to the sulfonamide compound (Example Compounds 1, 5, 14, 209A, and 235A) and the the other antitumor agent using Otsuka distilled water (Otsuka Pharmaceutical Factory; hereinafter referred to as DW). The serial dilutions of each compound, or DW was added at 5 μL/well to the plate such that each compound and DW, or the sulfonamide compound and the other antitumor agent were combined. The common ratio of concentrations was 1.5 for all the drugs, and the highest concentration (indicated by final concentration) of each compound added to each cell line is shown in the following Tables 27-30. The plate after

the addition was cultured at 37°C for 3 days under 5% $CO_2$ conditions.

Three days later, CellTiter-Glo(R) 2.0 Reagent (Promega Corporation) was added at 100 μL/well, and the amount of luminescence was measured using a plate reader EnSpire(R) Multimode Plate Reader (PerkinElmer Co., Ltd.).

[Table 27]

| Tumor cell line | Sulfonamide compound | | Other antitumor agent | |
|---|---|---|---|---|
| | Compound name | Maximum concentration (nM) | Compound name | Maximum concentration (nM) |
| A549 | Example Compound 1 | 7210 | Fludarabine nucleoside | 152000 |
| | | | Cytarabine | 1850 |
| | | | Decitabine | 15200 |
| | | | Guadecitabine | 5570 |
| | | | Gemcitabine | 42.5 |
| | | | Trifluridine | 3270 |
| | | | 5-Fluorouracil | 13700 |
| | | | Azacytidine | 20800 |
| | | | AZD6738 | 17700 |
| | | | Prexasertib | 50.6 |
| | | | SCH900776 | 23300 |
| | | | Cisplatin | 32400 |
| | | | Oxaliplatin | 5370 |
| | | | Carboplatin | 268000 |
| | | | Etoposide | 19200 |
| | | | Sunitinib | 14200 |
| | | | Cabozantinib | 30400 |
| | | | Midostaurin | 1270 |
| | | | Lapatinib | 30400 |
| | | | Luminespib | 68.3 |
| | | | Olaparib | 75000 |
| | | | Talazoparib | 40500 |
| | Example Compound 5 | 8830 | Fludarabine nucleoside | 101000 |
| | | | Cytarabine | 841 |
| | | | Decitabine | 15300 |
| | | | Guadecitabine | 5340 |
| | | | Gemcitabine | 42.5 |
| | | | Trifluridine | 3270 |
| | | | 5-Fluorouracil | 21800 |
| | | | Azacytidine | 20700 |
| | | | AZD6738 | 17700 |

(continued)

| Tumor cell line | Sulfonamide compound | | Other antitumor agent | |
|---|---|---|---|---|
| | Compound name | Maximum concentration (nM) | Compound name | Maximum concentration (nM) |
| | | | Prexasertib | 50.6 |
| | | | SCH900776 | 23300 |
| | | | Cisplatin | 32400 |
| | | | Oxaliplatin | 5370 |
| | | | Carboplatin | 268000 |
| | | | Etoposide | 19200 |
| | | | Sunitinib | 14200 |
| | | | Cabozantinib | 30400 |
| | | | Midostaurin | 1270 |
| | | | Lapatinib | 30400 |
| | | | Luminespib | 68.3 |
| | | | Olaparib | 75000 |
| | | | Talazoparib | 40500 |

[Table 28]

| Tumor cell line | Sulfonamide compound | | Other antitumor agent | |
|---|---|---|---|---|
| | Compound name | Maximum concentration (nM) | Compound name | Maximum concentration (nM) |
| A549 | Example Compound 14 | 22400 | Fludarabine nucleoside | 152000 |
| | | | Cytarabine | 1850 |
| | | | Decitabine | 15200 |
| | | | Guadecitabine | 5570 |
| | | | Gemcitabine | 42.5 |
| | | | Trifluridine | 3270 |
| | | | 5-Fluorouracil | 13700 |
| | | | Azacytidine | 20700 |
| | | | AZD6738 | 17700 |
| | | | Prexasertib | 50.6 |
| | | | SCH900776 | 23300 |
| | | | Cisplatin | 32400 |
| | | | Oxaliplatin | 5370 |
| | | | Carboplatin | 268000 |
| | | | Etoposide | 19200 |
| | | | Sunitinib | 14200 |

(continued)

| Tumor cell line | Sulfonamide compound | | Other antitumor agent | |
|---|---|---|---|---|
| | Compound name | Maximum concentration (nM) | Compound name | Maximum concentration (nM) |
| | | | Cabozantinib | 30400 |
| | | | Midostaurin | 1270 |
| | | | Lapatinib | 30400 |
| | | | Luminespib | 68.3 |
| | | | Olaparib | 75000 |
| | | | Talazoparib | 40500 |
| | Example Compound 209A | 4820 | Fludarabine nucleoside | 152000 |
| | | | Cytarabine | 1850 |
| | | | Decitabine | 15200 |
| | | | Guadecitabine | 5570 |
| | | | Gemcitabine | 42.5 |
| | | | Trifluridine | 3270 |
| | | | 5-Fluorouracil | 21800 |
| | | | Azacytidine | 20800 |
| | | | AZD6738 | 17700 |
| | | | Prexasertib | 50.6 |
| | | | SCH900776 | 23300 |
| | | | Cisplatin | 32400 |
| | | | Oxaliplatin | 5370 |
| | | | Carboplatin | 268000 |
| | | | Etoposide | 19200 |
| | | | Sunitinib | 14200 |
| | | | Cabozantinib | 30400 |
| | | | Midostaurin | 1270 |
| | | | Lapatinib | 30400 |
| | | | Luminespib | 68.3 |
| | | | Olaparib | 75000 |
| | | | Talazoparib | 40500 |

[Table 29]

| Tumor cell line | Sulfonamide compound | | Other antitumor agent | |
|---|---|---|---|---|
| | Compound name | Maximum concentration (nM) | Compound name | Maximum concentration (nM) |
| A549 | Example Compound 235A | 5920 | Fludarabine nucleoside | 101000 |
| | | | Cytarabine | 841 |
| | | | Decitabine | 15300 |
| | | | Guadecitabine | 5340 |
| | | | Gemcitabine | 42.5 |
| | | | Trifluridine | 3270 |
| | | | 5-Fluorouracil | 21800 |
| | | | Azacytidine | 20800 |
| | | | AZD6738 | 17700 |
| | | | Prexasertib | 50.6 |
| | | | SCH900776 | 23300 |
| | | | Cisplatin | 32400 |
| | | | Oxaliplatin | 5370 |
| | | | Carboplatin | 268000 |
| | | | Etoposide | 19200 |
| | | | Sunitinib | 14200 |
| | | | Cabozantinib | 30400 |
| | | | Midostaurin | 1270 |
| | | | Lapatinib | 30400 |
| | | | Luminespib | 27.5 |
| | | | Olaparib | 75000 |
| | | | Talazoparib | 40500 |

[Table 30]

| Tumor cell line | Sulfonamide compound | | Other antitumor agent | |
|---|---|---|---|---|
| | Compound name | Maximum concentration (nM) | Compound name | Maximum concentration (nM) |
| | Example Compound 1 | 2070 | Fludarabine nucleoside | 46700 |
| | | | Cytarabine | 1990 |
| | | | Decitabine | 571 |
| | | | Guadecitabine | 203 |

(continued)

| Tumor cell line | Sulfonamide compound | | Other antitumor agent | |
|---|---|---|---|---|
| | Compound name | Maximum concentration (nM) | Compound name | Maximum concentration (nM) |
| MV-4-11 | Example Compound 5 | 2810 | Fludarabine nucleoside | 46700 |
| | | | Cytarabine | 1990 |
| | | | Decitabine | 571 |
| | | | Guadecitabine | 203 |
| | Example Compound 14 | 5910 | Fludarabine nucleoside | 46700 |
| | | | Cytarabine | 1990 |
| | | | Decitabine | 571 |
| | | | Guadecitabine | 203 |
| | Example Compound 209A | 1100 | Fludarabine nucleoside | 46700 |
| | | | Cytarabine | 1990 |
| | | | Decitabine | 571 |
| | | | Guadecitabine | 203 |
| | Example Compound 235A | 1540 | Fludarabine nucleoside | 46700 |
| | | | Cytarabine | 1990 |
| | | | Decitabine | 571 |
| | | | Guadecitabine | 203 |

(continued)

| Tumor cell line | Sulfonamide compound | | Other antitumor agent | |
|---|---|---|---|---|
| | Compound name | Maximum concentration (nM) | Compound name | Maximum concentration (nM) |
| CFPAC-1 | Example Compound 1 | 5770 | Gemcitabine | 40.1 |
| | | | AZD6738 | 15800 |
| | | | Prexasertib | 48.5 |
| | | | SCH900776 | 4320 |
| | Example Compound 5 | 7970 | Gemcitabine | 40.1 |
| | | | AZD6738 | 15800 |
| | | | Prexasertib | 48.5 |
| | | | SCH900776 | 4320 |
| | Example Compound 14 | 15800 | Gemcitabine | 40.1 |
| | | | AZD6738 | 15800 |
| | | | Prexasertib | 48.5 |
| | | | SCH900776 | 4320 |
| | Example Compound 209A | 3860 | Gemcitabine | 40.1 |
| | | | AZD6738 | 15800 |
| | | | Prexasertib | 48.5 |
| | | | SCH900776 | 4320 |
| | Example Compound 235A | 5480 | Gemcitabine | 40.1 |
| | | | AZD6738 | 15800 |
| | | | Prexasertib | 48.5 |
| | | | SCH900776 | 4320 |
| HCT116 | Example Compound 1 | 5830 | AZD6738 | 3860 |
| | | | Prexasertib | 122 |
| | Example Compound 5 | 6460 | AZD6738 | 3860 |
| | | | Prexasertib | 122 |
| | Example Compound 14 | 17100 | AZD6738 | 3860 |
| | | | Prexasertib | 122 |
| | Example Compound 209A | 3200 | AZD6738 | 3860 |
| | | | Prexasertib | 122 |
| | Example Compound 235A | 5410 | AZD6738 | 3860 |
| | | | Prexasertib | 122 |

Enhancement in effect by combined use of drugs was evaluated according to the method described in the known documents (Trends Pharmacol. Sci. 4, 450-454, 1983; and Pharmacol. Rev. 58 (3), 621-681, 2006).

An average value from 3 wells was calculated as to each drug condition from the obtained data, and cell survival rate normalized against control wells supplemented with the vehicle (DW) was calculated. A Fa (fractional inhibition) value was calculated by subtracting the cell survival rate from 1. The Fa value of the concentration at which 0.01 < Fa < 0.999 held and linear correlation coefficient r of the median-effect plot was larger than 0.92 was input to drug administration effect analysis software CalcuSyn Version 2.0 (Biosoft) based on the Median Effect method to calculate a

combination index (CI) value (Synergy 1,3-21,2014).

The combinatorial effect was determined according to the following Table 31 (Pharmacol. Rev. 58 (3), 621-681, 2006).

[Table 31]

| Range of CI (upper limit) | Description |
|---|---|
| 0.1 | Very strong synergistic activity |
| 0.3 | Strong synergistic activity |
| 0.7 | Synergistic activity |
| 0.85 | Moderate synergistic activity |
| 0.9 | Slight synergistic activity |
| 1.0 | Almost additive |
| 1.2 | Slight antagonistic activity |
| 1.45 | Moderate antagonistic activity |
| 3.3 | Antagonistic activity |
| 10 | Strong antagonistic activity |
| >10 | Very strong antagonistic activity |

The results are shown in the following Tables 32-51.

[Table 32]

| Cell line: A549 (Human lung cancer cell line) | | | | | |
|---|---|---|---|---|---|
| Other antitumor agent in combined use with Example Compound 1 | Example Compound 1 (nM) | Other antitumor agent (nM) | Molar ratio of drugs in combined use (Example Compound 1: Other antitumor agent) | Fa value | CI value |
| Fludarabine nucleoside | 281 | 5920 | 1 : 21.08 | 0.2653 | 0.550 |
| | 422 | 8900 | | 0.6824 | 0.388 |
| | 633 | 13300 | | 0.8650 | 0.367 |
| | 949 | 20000 | | 0.8719 | 0.537 |
| Cytarabine | 422 | 108 | 1 : 0.2566 | 0.4482 | 0.770 |
| | 633 | 162 | | 0.6164 | 0.614 |
| | 949 | 244 | | 0.7886 | 0.461 |
| | 1420 | 364 | | 0.8557 | 0.491 |
| Decitabine | 633 | 1330 | 1 : 2.108 | 0.3872 | 0.532 |
| | 949 | 2000 | | 0.5507 | 0.501 |
| | 1420 | 2990 | | 0.6575 | 0.583 |
| Guadecitabine | 633 | 489 | 1 : 0.7725 | 0.4374 | 0.471 |
| | 949 | 733 | | 0.5583 | 0.502 |
| | 1420 | 1100 | | 0.6756 | 0.563 |
| Gemcitabine | 1420 | 8.37 | 1 : 0.005895 | 0.8410 | 0.858 |

(continued)

| Cell line: A549 (Human lung cancer cell line) | | | | | |
|---|---|---|---|---|---|
| Other antitumor agent in combined use with Example Compound 1 | Example Compound 1 (nM) | Other antitumor agent (nM) | Molar ratio of drugs in combined use (Example Compound 1: Other antitumor agent) | Fa value | CI value |
| Trifluridine | 949 | 430 | 1 : 0.4535 | 0.6005 | 0.890 |
| | 1420 | 644 | | 0.8230 | 0.717 |
| | 2140 | 970 | | 0.8988 | 0.761 |
| 5-Fluorouracil | 1420 | 2700 | 1 : 1.900 | 0.6426 | 0.881 |
| Azacytidine | 949 | 2740 | 1 : 2.885 | 0.5060 | 0.895 |
| | 1420 | 4100 | | 0.7364 | 0.676 |
| AZD6738 | 422 | 1040 | 1 : 2.455 | 0.5145 | 0.449 |
| | 633 | 1550 | | 0.7602 | 0.350 |
| | 949 | 2330 | | 0.8849 | 0.315 |
| | 1420 | 3490 | | 0.8809 | 0.482 |
| Prexasertib | 281 | 1.97 | 1 : 0.007018 | 0.3570 | 0.349 |
| | 422 | 2.96 | | 0.7264 | 0.226 |
| | 633 | 4.44 | | 0.8863 | 0.193 |
| | 949 | 6.66 | | 0.8866 | 0.288 |
| SCH900776 | 281 | 908 | 1 : 3.232 | 0.6117 | 0.196 |
| | 422 | 1360 | | 0.7479 | 0.195 |
| | 633 | 2050 | | 0.8581 | 0.190 |
| | 949 | 3070 | | 0.8783 | 0.257 |
| Cisplatin | 2140 | 9620 | 1 : 4.494 | 0.9125 | 0.848 |
| | 3200 | 14400 | | 0.9675 | 0.694 |
| Oxaliplatin | 1420 | 1060 | 1 : 0.7448 | 0.7172 | 0.831 |
| Carboplatin | 2140 | 79500 | 1 : 37.17 | 0.9198 | 0.632 |
| | 3200 | 119000 | | 0.9734 | 0.502 |
| | 4810 | 179000 | | 0.9841 | 0.569 |
| Etoposide | 1420 | 3780 | 1 : 2.663 | 0.6999 | 0.772 |
| Sunitinib | 949 | 1870 | 1 : 1.969 | 0.6537 | 0.721 |
| | 1420 | 2800 | | 0.8162 | 0.688 |
| Cabozantinib | 2140 | 9020 | 1 : 4.216 | 0.9173 | 0.790 |
| Midostaurin | 1420 | 250 | 1 : 0.1761 | 0.7361 | 0.871 |
| Lapatinib | 1420 | 5990 | 1 : 4.216 | 0.8412 | 0.480 |
| Luminespib | 1420 | 13.5 | 1 : 0.009473 | 0.7741 | 0.894 |
| Olaparib | 1420 | 14800 | 1 : 10.40 | 0.6398 | 0.761 |
| Talazoparib | 1420 | 7980 | 1 : 5.617 | 0.6630 | 0.661 |

[Table 33]

| Cell line: MV-4-11 (Human blood cancer cell line) | | | | | |
|---|---|---|---|---|---|
| Other antitumor agent in combined use with Example Compound 1 | Example Compound 1 (nM) | Other antitumor agent (nM) | Molar ratio of drugs in combined use (Example Compound 1: Other antitumor agent) | Fa value | CI value |
| Fludarabine nucleoside | 182 | 4110 | 1 : 22.56 | 0.1868 | 0.783 |
| | 273 | 6160 | | 0.8795 | 0.592 |
| Cytarabine | 121 | 116 | 1 : 0.9614 | 0.1611 | 0.683 |
| | 182 | 175 | | 0.4508 | 0.603 |
| | 273 | 262 | | 0.7678 | 0.574 |
| | 409 | 393 | | 0.9443 | 0.538 |
| | 613 | 589 | | 0.9960 | 0.419 |
| Decitabine | 182 | 50.2 | 1 : 0.2758 | 0.3873 | 0.643 |
| | 273 | 75.3 | | 0.5342 | 0.680 |
| | 409 | 113 | | 0.7570 | 0.667 |
| | 613 | 169 | | 0.9292 | 0.672 |
| | 920 | 254 | | 0.9900 | 0.666 |
| Guadecitabine | 121 | 11.9 | 1 : 0.09807 | 0.2186 | 0.706 |
| | 182 | 17.8 | | 0.3225 | 0.741 |
| | 273 | 26.8 | | 0.5192 | 0.705 |
| | 409 | 40.1 | | 0.7788 | 0.658 |
| | 613 | 60.1 | | 0.9347 | 0.671 |
| | 920 | 90.2 | | 0.9921 | 0.639 |

[Table 34]

| Cell line: CFPAC-1 (Human pancreatic cancer cell line) | | | | | |
|---|---|---|---|---|---|
| Other antitumor agent in combined use with Example Compound 1 | Example Compound 1 (nM) | Other antitumor agent (nM) | Molar ratio of drugs in combined use (Example Compound 1: Other antitumor agent) | Fa value | CI value |
| Gemcitabine | 507 | 3.52 | 1 : 0.006950 | 0.5009 | 0.821 |
| | 760 | 5.28 | | 0.6416 | 0.790 |
| AZD6738 | 338 | 925 | 1 : 2.738 | 0.4791 | 0.484 |
| | 507 | 1390 | | 0.7266 | 0.356 |
| | 760 | 2080 | | 0.7884 | 0.427 |
| Prexasertib | 225 | 1.89 | 1 : 0.008406 | 0.2319 | 0.825 |
| | 338 | 2.84 | | 0.6056 | 0.580 |
| | 507 | 4.26 | | 0.7542 | 0.633 |

(continued)

| Cell line: CFPAC-1 (Human pancreatic cancer cell line) | | | | | |
|---|---|---|---|---|---|
| Other antitumor agent in combined use with Example Compound 1 | Example Compound 1 (nM) | Other antitumor agent (nM) | Molar ratio of drugs in combined use (Example Compound 1: Other antitumor agent) | Fa value | CI value |
| SCH900776 | 338 | 253 | 1 : 0.7487 | 0.4162 | 0.496 |
| | 507 | 380 | | 0.6944 | 0.369 |
| | 760 | 569 | | 0.7479 | 0.472 |

[Table 35]

| Cell line: HCT 116 (Human large intestine cancer cell line) | | | | | |
|---|---|---|---|---|---|
| Other antitumor agent in combined use with Example Compound 1 | Example Compound 1 (nM) | Other antitumor agent (nM) | Molar ratio of drugs in combined use (Example Compound 1 : Other antitumor agent) | Fa value | CI value |
| AZD6738 | 768 | 508 | 1 : 0.6621 | 0.8782 | 0.264 |
| | 1150 | 761 | | 0.9579 | 0.218 |
| Prexasertib | 341 | 7.14 | 1 : 0.02093 | 0.6993 | 0.451 |
| | 512 | 10.7 | | 0.9038 | 0.243 |
| | 768 | 16.1 | | 0.9579 | 0.197 |

[Table 36]

| Cell line: A549 (Human lung cancer cell line) | | | | | |
|---|---|---|---|---|---|
| Other antitumor agent in combined use with Example Compound 5 | Example Compound 5 (nM) | Other antitumor agent (nM) | Molar ratio of drugs in combined use (Example Compound 5 : Other antitumor agent) | Fa value | CI value |
| Fludarabine nucleoside | 517 | 5910 | 1 : 11.44 | 0.1823 | 0.818 |
| | 775 | 8870 | | 0.5070 | 0.559 |
| | 1160 | 13300 | | 0.8089 | 0.405 |
| | 1740 | 19900 | | 0.8431 | 0.537 |
| Cytarabine | 1160 | 110 | 1 : 0.09524 | 0.4712 | 0.842 |
| | 1740 | 166 | | 0.7121 | 0.619 |
| Decitabine | 775 | 1340 | 1 : 1.733 | 0.3618 | 0.528 |
| | 1160 | 2010 | | 0.4748 | 0.573 |
| | 1740 | 3020 | | 0.5994 | 0.627 |
| Guadecitabine | 775 | 469 | 1 : 0.6048 | 0.3478 | 0.558 |
| | 1160 | 702 | | 0.4476 | 0.627 |
| | 1740 | 1050 | | 0.5997 | 0.640 |
| Gemcitabine | 1160 | 5.58 | 1 : 0.004813 | 0.6357 | 0.865 |
| | 1740 | 8.37 | | 0.8623 | 0.757 |

(continued)

| Cell line: A549 (Human lung cancer cell line) | | | | | |
|---|---|---|---|---|---|
| Other antitumor agent in combined use with Example Compound 5 | Example Compound 5 (nM) | Other antitumor agent (nM) | Molar ratio of drugs in combined use (Example Compound 5 : Other antitumor agent) | Fa value | CI value |
| Trifluridine | 1160 | 430 | 1 : 0.3703 | 0.6401 | 0.842 |
| | 1740 | 644 | | 0.8343 | 0.696 |
| | 2620 | 970 | | 0.9017 | 0.744 |
| 5-Fluorouracil | 1740 | 4300 | 1 : 2.469 | 0.7276 | 0.835 |
| Azacytidine | 1740 | 4080 | 1 : 2.344 | 0.6539 | 0.820 |
| AZD6738 | 517 | 1037 | 1 : 2.005 | 0.5213 | 0.486 |
| | 775 | 1554 | | 0.7989 | 0.318 |
| | 1160 | 2326 | | 0.8880 | 0.309 |
| Prexasertib | 345 | 1.98 | 1 : 0.005730 | 0.4283 | 0.363 |
| | 517 | 2.96 | | 0.7673 | 0.207 |
| | 775 | 4.44 | | 0.8934 | 0.174 |
| SCH900776 | 345 | 910 | 1 : 2.639 | 0.5875 | 0.287 |
| | 517 | 1360 | | 0.7191 | 0.250 |
| | 775 | 2050 | | 0.8457 | 0.210 |
| | 1160 | 3060 | | 0.8825 | 0.255 |
| | 1740 | 4590 | | 0.8708 | 0.411 |
| Cisplatin | 2620 | 3610 | 1 : 3.669 | 0.9341 | 0.701 |
| | 3920 | 14400 | | 0.9720 | 0.608 |
| Oxaliplatin | 1740 | 1060 | 1 : 0.6082 | 0.7638 | 0.787 |
| Carboplatin | 2620 | 79500 | 1 : 30.35 | 0.8844 | 0.519 |
| | 3920 | 119000 | | 0.9458 | 0.474 |
| | 5890 | 179000 | | 0.9560 | 0.627 |
| | 8830 | 268000 | | 0.9773 | 0.635 |
| Etoposide | 1740 | 3780 | 1 : 2.174 | 0.7574 | 0.631 |
| Sunitinib | 1160 | 1870 | 1 : 1.608 | 0.7514 | 0.740 |
| | 1740 | 2800 | | 0.8380 | 0.811 |
| Cabozantinib | 1740 | 5990 | 1 : 3.443 | 0.8307 | 0.688 |
| Midostaurin | 1740 | 250 | 1 : 0.1438 | 0.8366 | 0.624 |
| Lapatinib | 1740 | 5990 | 1 : 3.443 | 0.8672 | 0.400 |
| Luminespib | 1740 | 13.5 | 1 : 0.007735 | 0.8341 | 0.828 |
| Olaparib | 1740 | 14800 | 1 : 8.494 | 0.7460 | 0.575 |
| Talazoparib | 1740 | 7980 | 1 : 4.587 | 0.7568 | 0.508 |

[Table 37]

| Cell line: MV-4-11 (Human blood cancer cell line) | | | | | |
|---|---|---|---|---|---|
| Other antitumor agent in combined use with Example Compound 5 | Example Compound 5 (nM) | Other antitumor agent (nM) | Molar ratio of drugs in combined use (Example Compound 5 : Other antitumor agent) | Fa value | CI value |
| Fludarabine nucleoside | 370 | 6150 | 1 : 16.62 | 0.6600 | 0.739 |
| | 555 | 9220 | | 0.9981 | 0.293 |
| Cytarabine | 247 | 175 | 1 : 0.7082 | 0.4093 | 0.638 |
| | 370 | 262 | | 0.6725 | 0.651 |
| | 555 | 393 | | 0.9212 | 0.553 |
| | 833 | 590 | | 0.9948 | 0.366 |
| | 1250 | 885 | | 0.9986 | 0.386 |
| Decitabine | 247 | 50.2 | 1 : 0.2032 | 0.3382 | 0.685 |
| | 370 | 75.2 | | 0.4753 | 0.725 |
| | 555 | 113 | | 0.6924 | 0.709 |
| | 833 | 169 | | 0.8888 | 0.693 |
| | 1250 | 254 | | 0.9844 | 0.608 |
| Guadecitabine | 164 | 11.8 | 1 : 0.07224 | 0.2498 | 0.640 |
| | 247 | 17.8 | | 0.3527 | 0.683 |
| | 370 | 26.7 | | 0.4905 | 0.727 |
| | 555 | 40.1 | | 0.7205 | 0.690 |
| | 833 | 60.2 | | 0.9023 | 0.673 |
| | 1250 | 90.3 | | 0.9892 | 0.557 |

[Table 38]

| Cell line: CFPAC-1 (Human pancreatic cancer cell line) | | | | | |
|---|---|---|---|---|---|
| Other antitumor agent in combined use with Example Compound 5 | Example Compound 5 (nM) | Other antitumor agent (nM) | Molar ratio of drugs in combined use (Example Compound 5 : Other antitumor agent) | Fa value | CI value |
| Gemcitabine | 700 | 3.52 | 1 : 0.005031 | 0.5292 | 0.814 |
| | 1050 | 5.28 | | 0.6974 | 0.788 |
| AZD6738 | 311 | 616 | 1 : 1.982 | 0.2221 | 0.640 |
| | 466 | 924 | | 0.5986 | 0.365 |
| | 700 | 1390 | | 0.7586 | 0.358 |
| | 1050 | 2080 | | 0.7838 | 0.495 |
| Prexasertib | 311 | 1.89 | 1 : 0.006085 | 0.3644 | 0.579 |
| | 466 | 2.84 | | 0.6946 | 0.413 |
| | 700 | 4.26 | | 0.7564 | 0.525 |
| SCH900776 | 466 | 253 | 1 : 0.5420 | 0.6428 | 0.283 |
| | 700 | 379 | | 0.7803 | 0.283 |

[Table 39]

| Cell line: HCT 116 (Human large intestine cancer cell line) | | | | | |
|---|---|---|---|---|---|
| Other antitumor agent in combined use with Example Compound 5 | Example Compound 5 (nM) | Other antitumor agent (nM) | Molar ratio of drugs in combined use (Example Compound 5 : Other antitumor agent) | Fa value | CI value |
| AZD6738 | 567 | 339 | 1 : 0.5975 | 0.3831 | 0.649 |
| | 851 | 508 | | 0.9186 | 0.231 |
| | 1280 | 765 | | 0.9567 | 0.250 |
| Prexasertib | 567 | 10.7 | 1 : 0.01889 | 0.7563 | 0.289 |
| | 851 | 16.1 | | 0.9499 | 0.180 |

[Table 40]

| Cell line: A549 (Human lung cancer cell line) | | | | | |
|---|---|---|---|---|---|
| Other antitumor agent in combined use with Example Compound 14 | Example Compound 14 (nM) | Other antitumor agent (nM) | Molar ratio of drugs in combined use (Example Compound 14: Other antitumor agent) | Fa value | CI value |
| Fludarabine nucleoside | 874 | 5930 | 1 : 6.786 | 0.3063 | 0.549 |
| | 1310 | 8890 | | 0.7007 | 0.387 |
| | 1970 | 13400 | | 0.8610 | 0.377 |
| | 2950 | 20000 | | 0.8736 | 0.537 |
| Cytarabine | 874 | 72.2 | 1 : 0.08259 | 0.3745 | 0.670 |
| | 1310 | 108 | | 0.4356 | 0.790 |
| | 1970 | 163 | | 0.6170 | 0.616 |
| | 2950 | 244 | | 0.7754 | 0.498 |
| | 4420 | 365 | | 0.8591 | 0.490 |
| Decitabine | 1970 | 1340 | 1 : 0.6786 | 0.3867 | 0.542 |
| | 2950 | 2000 | | 0.5183 | 0.577 |
| | 4420 | 3000 | | 0.6737 | 0.602 |
| Guadecitabine | 1310 | 326 | 1 : 0.2487 | 0.2913 | 0.561 |
| | 1970 | 490 | | 0.4560 | 0.503 |
| | 2950 | 734 | | 0.5553 | 0.582 |
| | 4420 | 1100 | | 0.6875 | 0.620 |
| Gemcitabine | 4420 | 8.38 | 1 : 0.001897 | 0.8565 | 0.752 |
| Trifluridine | 1970 | 288 | 1 : 0.1460 | 0.4363 | 0.892 |
| | 2950 | 431 | | 0.6485 | 0.844 |
| | 4420 | 645 | | 0.8577 | 0.678 |
| | 6640 | 969 | | 0.9068 | 0.791 |
| 5-Fluorouracil | 4420 | 2700 | 1 : 0.6116 | 0.6619 | 0.880 |
| Azacytidine | 4420 | 4080 | 1 : 0.9241 | 0.7570 | 0.744 |

(continued)

| Cell line: A549 (Human lung cancer cell line) | | | | | |
|---|---|---|---|---|---|
| Other antitumor agent in combined use with Example Compound 14 | Example Compound 14 (nM) | Other antitumor agent (nM) | Molar ratio of drugs in combined use (Example Compound 14: Other antitumor agent) | Fa value | CI value |
| AZD6738 | 1310 | 1040 | 1 : 0.7902 | 0.5577 | 0.432 |
| | 1970 | 1560 | | 0.7900 | 0.331 |
| | 2950 | 2330 | | 0.8857 | 0.322 |
| | 4420 | 3490 | | 0.8807 | 0.497 |
| Prexasertib | 874 | 1.97 | 1 : 0.002259 | 0.4973 | 0.277 |
| | 1310 | 2.96 | | 0.7864 | 0.193 |
| | 1970 | 4.45 | | 0.8986 | 0.177 |
| SCH900776 | 874 | 909 | 1 : 1.040 | 0.6512 | 0.194 |
| | 1310 | 1360 | | 0.7883 | 0.184 |
| | 1970 | 2050 | | 0.8714 | 0.191 |
| | 2950 | 3070 | | 0.8814 | 0.271 |
| Cisplatin | 6640 | 9600 | 1 : 1.446 | 0.9244 | 0.791 |
| | 9960 | 14400 | | 0.9690 | 0.692 |
| Oxaliplatin | 4420 | 1060 | 1 : 0.2397 | 0.7498 | 0.829 |
| Carboplatin | 6640 | 79400 | 1 : 11.96 | 0.9157 | 0.620 |
| | 9960 | 119000 | | 0.9630 | 0.605 |
| | 14900 | 178000 | | 0.9756 | 0.734 |
| | 22400 | 268000 | | 0.9882 | 0.773 |
| Etoposide | 4420 | 3790 | 1 : 0.8571 | 0.7245 | 0.707 |
| Sunitinib | 2950 | 1870 | 1 : 0.6339 | 0.7017 | 0.693 |
| | 4420 | 2800 | | 0.8248 | 0.728 |
| Cabozantinib | 4420 | 6000 | 1 : 1.357 | 0.7829 | 0.868 |
| Midostaurin | 4420 | 251 | 1 : 0.05670 | 0.8117 | 0.719 |
| Lapatinib | 4420 | 6000 | 1 : 1.357 | 0.8641 | 0.512 |
| Luminespib | 4420 | 13.5 | 1 : 0.003049 | 0.8373 | 0.786 |
| Olaparib | 4420 | 14800 | 1 : 3.348 | 0.7031 | 0.668 |
| Talazoparib | 4420 | 7990 | 1 : 1.808 | 0.7406 | 0.540 |

[Table 41]

| Cell line: MV-4-11 (Human blood cancer cell line) | | | | | |
|---|---|---|---|---|---|
| Other antitumor agent in combined use with Example Compound 14 | Example Compound 14 (nM) | Other antitumor agent (nM) | Molar ratio of drugs in combined use (Example Compound 14: Other antitumor agent) | Fa value | CI value |
| Fludarabine nucleoside | 778 | 6150 | 1 : 7.902 | 0.5954 | 0.786 |
| | 1170 | 9250 | | 0.9984 | 0.296 |
| Cytarabine | 519 | 175 | 1 : 0.3367 | 0.2908 | 0.766 |
| | 778 | 262 | | 0.6118 | 0.691 |
| | 1170 | 394 | | 0.8743 | 0.599 |
| | 1750 | 589 | | 0.9860 | 0.387 |
| | 2630 | 886 | | 0.9984 | 0.269 |
| Decitabine | 519 | 50.1 | 1 : 0.09662 | 0.3361 | 0.707 |
| | 778 | 75.2 | | 0.4089 | 0.872 |
| | 1170 | 113 | | 0.5994 | 0.849 |
| | 1750 | 169 | | 0.8350 | 0.717 |
| | 2630 | 254 | | 0.9588 | 0.599 |
| Guadecitabine | 519 | 17.8 | 1 : 0.03435 | 0.3318 | 0.767 |
| | 778 | 26.7 | | 0.4449 | 0.844 |
| | 1170 | 40.2 | | 0.6448 | 0.797 |
| | 1750 | 60.1 | | 0.8620 | 0.668 |
| | 2630 | 90.3 | | 0.9686 | 0.545 |

[Table 42]

| Cell line: CFPAC-1 (Human pancreatic cancer cell line) | | | | | |
|---|---|---|---|---|---|
| Other antitumor agent in combined use with Example Compound 14 | Example Compound 14 (nM) | Other antitumor agent (nM) | Molar ratio of drugs in combined use (Example Compound 14: Other antitumor agent) | Fa value | CI value |
| Gemcitabine | 2080 | 5.28 | 1 : 0.002538 | 0.6749 | 0.756 |
| | 3120 | 7.92 | | 0.7790 | 0.752 |
| AZD6738 | 925 | 925 | 1 : 1.000 | 0.5930 | 0.296 |
| | 1390 | 1390 | | 0.7353 | 0.259 |
| Prexasertib | 616 | 1.89 | 1 : 0.003070 | 0.2455 | 0.886 |
| | 925 | 2.84 | | 0.6771 | 0.452 |
| | 1390 | 4.27 | | 0.7725 | 0.514 |
| SCH900776 | 925 | 253 | 1 : 0.2734 | 0.4714 | 0.465 |
| | 1390 | 380 | | 0.7231 | 0.322 |
| | 2080 | 569 | | 0.7671 | 0.409 |

[Table 43]

| Cell line: HCT116 (Human large intestine cancer cell line) | | | | | |
|---|---|---|---|---|---|
| Other antitumor agent in combined use with Example Compound 14 | Example Compound 14 (nM) | Other antitumor agent (nM) | Molar ratio of drugs in combined use (Example Compound 14: Other antitumor agent) | Fa value | CI value |
| AZD6738 | 1000 | 226 | 1 : 0.2257 | 0.4422 | 0.804 |
| | 1500 | 339 | | 0.9071 | 0.235 |
| | 2250 | 508 | | 0.9646 | 0.192 |
| | 3380 | 763 | | 0.9574 | 0.322 |
| Prexasertib | 1000 | 7.14 | 1 : 0.007135 | 0.3054 | 0.535 |
| | 1500 | 10.7 | | 0.8469 | 0.208 |
| | 2250 | 16.1 | | 0.9453 | 0.174 |

[Table 44]

| Cell line: A549 (Human lung cancer cell line) | | | | | |
|---|---|---|---|---|---|
| Other antitumor agent in combined use with Example Compound 209A | Example Compound 209A (nM) | Other antitumor agent (nM) | Molar ratio of drugs in combined use (Example Compound 209A: Other antitumor agent) | Fa value | CI value |
| Fludarabine nucleoside | 188 | 5930 | 1 : 31.54 | 0.3666 | 0.558 |
| | 282 | 8890 | | 0.7059 | 0.386 |
| | 423 | 13300 | | 0.8660 | 0.339 |
| | 635 | 20000 | | 0.8634 | 0.515 |
| Cytarabine | 188 | 72.2 | 1 : 0.3838 | 0.3588 | 0.777 |
| | 282 | 108 | | 0.4675 | 0.759 |
| | 423 | 162 | | 0.6516 | 0.581 |
| | 635 | 244 | | 0.7994 | 0.474 |
| | 952 | 365 | | 0.8410 | 0.574 |
| Decitabine | 423 | 1330 | 1 : 3.154 | 0.4274 | 0.542 |
| | 635 | 2000 | | 0.5615 | 0.569 |
| Guadecitabine | 423 | 489 | 1 : 1.156 | 0.4525 | 0.577 |
| | 635 | 734 | | 0.6179 | 0.522 |
| Gemcitabine | 635 | 5.60 | 1 : 0.008817 | 0.7031 | 0.792 |
| | 952 | 8.39 | | 0.8749 | 0.791 |
| Trifluridine | 423 | 287 | 1 : 0.6784 | 0.4046 | 0.883 |
| | 635 | 431 | | 0.5907 | 0.897 |
| | 952 | 646 | | 0.8235 | 0.733 |
| | 1430 | 970 | | 0.9021 | 0.775 |
| 5-Fluorouracil | 952 | 4310 | 1 : 4.523 | 0.7187 | 0.853 |
| Azacytidine | 635 | 2740 | 1 : 4.315 | 0.6005 | 0.850 |
| | 952 | 4110 | | 0.8264 | 0.520 |

(continued)

| Cell line: A549 (Human lung cancer cell line) | | | | | |
|---|---|---|---|---|---|
| Other antitumor agent in combined use with Example Compound 209A | Example Compound 209A (nM) | Other antitumor agent (nM) | Molar ratio of drugs in combined use (Example Compound 209A: Other antitumor agent) | Fa value | CI value |
| AZD6738 | 282 | 1040 | 1 : 3.672 | 0.5590 | 0.402 |
| | 423 | 1550 | | 0.7925 | 0.315 |
| | 635 | 2330 | | 0.8851 | 0.317 |
| Prexasertib | 188 | 1.97 | 1 : 0.01050 | 0.5015 | 0.258 |
| | 282 | 2.96 | | 0.7685 | 0.189 |
| | 423 | 4.44 | | 0.8944 | 0.169 |
| SCH900776 | 188 | 909 | 1 : 4.834 | 0.6553 | 0.180 |
| | 282 | 1360 | | 0.7965 | 0.170 |
| | 423 | 2040 | | 0.8795 | 0.177 |
| | 635 | 3070 | | 0.8744 | 0.273 |
| Cisplatin | 1430 | 9610 | 1 : 6.722 | 0.9358 | 0.709 |
| | 2140 | 14400 | | 0.9740 | 0.632 |
| Oxaliplatin | 952 | 1060 | 1 : 1.114 | 0.7832 | 0.762 |
| Carboplatin | 1430 | 79500 | 1 : 55.60 | 0.9282 | 0.618 |
| | 2140 | 119000 | | 0.9721 | 0.528 |
| | 3210 | 178000 | | 0.9813 | 0.630 |
| Etoposide | 952 | 3790 | 1 : 3.983 | 0.7460 | 0.635 |
| Sunitinib | 635 | 1870 | 1 : 2.946 | 0.6919 | 0.688 |
| | 952 | 2800 | | 0.8234 | 0.723 |
| Cabozantinib | 952 | 6000 | 1 : 6.307 | 0.8982 | 0.486 |
| Midostaurin | 952 | 251 | 1 : 0.2635 | 0.8520 | 0.583 |
| Lapatinib | 952 | 6000 | 1 : 6.307 | 0.8816 | 0.443 |
| Luminespib | 952 | 13.5 | 1 : 0.01417 | 0.8172 | 0.832 |
| Olaparib | 952 | 14800 | 1 : 15.56 | 0.7947 | 0.478 |
| Talazoparib | 952 | 8000 | 1 : 8.402 | 0.7998 | 0.422 |

[Table 45]

| Cell line: MV-4-11 (Human blood cancer cell line) | | | | | |
|---|---|---|---|---|---|
| Other antitumor agent in combined use with Example Compound 209A | Example Compound 209A (nM) | Other antitumor agent (nM) | Molar ratio of drugs in combined use (Example Compound 209A: Other antitumor agent) | Fa value | CI value |
| Fludarabine nucleoside | 145 | 6160 | 1 : 42.45 | 0.9458 | 0.598 |

(continued)

| Cell line: MV-4-11 (Human blood cancer cell line) | | | | | |
|---|---|---|---|---|---|
| Other antitumor agent in combined use with Example Compound 209A | Example Compound 209A (nM) | Other antitumor agent (nM) | Molar ratio of drugs in combined use (Example Compound 209A: Other antitumor agent) | Fa value | CI value |
| Cytarabine | 96.6 | 175 | 1 : 1.809 | 0.5055 | 0.739 |
| | 145 | 262 | | 0.8261 | 0.664 |
| | 217 | 393 | | 0.9736 | 0.510 |
| | 326 | 590 | | 0.9981 | 0.330 |
| Decitabine | 64.4 | 33.4 | 1 : 0.5191 | 0.2551 | 0.793 |
| | 96.6 | 50.1 | | 0.3812 | 0.839 |
| | 145 | 75.2 | | 0.6143 | 0.790 |
| | 217 | 113 | | 0.8536 | 0.715 |
| | 326 | 169 | | 0.9689 | 0.627 |
| Guadecitabine | 96.6 | 17.8 | 1 : 0.1845 | 0.4376 | 0.766 |
| | 145 | 26.8 | | 0.6306 | 0.767 |
| | 217 | 40.0 | | 0.8301 | 0.753 |
| | 326 | 60.1 | | 0.9675 | 0.631 |

[Table 46]

| Cell line: CFPAC-1 (Human pancreatic cancer cell line) | | | | | |
|---|---|---|---|---|---|
| Other antitumor agent in combined use with Example Compound 209A | Example Compound 209A (nM) | Other antitumor agent (nM) | Molar ratio of drugs in combined use (Example Compound 209A: Other antitumor agent) | Fa value | CI value |
| Gemcitabine | 339 | 3.52 | 1 : 0.01039 | 0.5348 | 0.855 |
| | 508 | 5.28 | | 0.6998 | 0.736 |
| AZD6738 | 226 | 925 | 1 : 4.093 | 0.5736 | 0.478 |
| | 339 | 1390 | | 0.7322 | 0.429 |
| | 508 | 2080 | | 0.7827 | 0.531 |
| Prexasertib | 151 | 1.90 | 1 : 0.01256 | 0.3163 | 0.735 |
| | 226 | 2.84 | | 0.6872 | 0.558 |
| | 339 | 4.26 | | 0.7662 | 0.704 |
| SCH900776 | 226 | 253 | 1 : 1.119 | 0.5579 | 0.385 |
| | 339 | 379 | | 0.7356 | 0.348 |
| | 508 | 568 | | 0.7649 | 0.474 |

[Table 47]

| Cell line: HCT116 (Human large intestine cancer cell line) | | | | | |
|---|---|---|---|---|---|
| Other antitumor agent in combined use with Example Compound 209A | Example Compound 209A (nM) | Other antitumor agent (nM) | Molar ratio of drugs in combined use (Example Compound 209A: Other antitumor agent) | Fa value | CI value |
| AZD6738 | 281 | 339 | 1 : 1.206 | 0.2770 | 0.877 |
| | 421 | 508 | | 0.8773 | 0.289 |
| | 632 | 762 | | 0.9571 | 0.242 |
| Prexasertib | 187 | 7.13 | 1 : 0.03813 | 0.1703 | 0.792 |
| | 281 | 10.7 | | 0.8191 | 0.230 |
| | 421 | 16.1 | | 0.9441 | 0.173 |

[Table 48]

| Cell line: A549 (Human lung cancer cell line) | | | | | |
|---|---|---|---|---|---|
| Other antitumor agent in combined use with Example Compound 235A | Example Compound 235A (nM) | Other antitumor agent (nM) | Molar ratio of drugs in combined use (Example Compound 235A: Other antitumor agent) | Fa value | CI value |
| Fludarabine nucleoside | 520 | 8870 | 1 : 17.06 | 0.4460 | 0.568 |
| | 780 | 13300 | | 0.7798 | 0.423 |
| | 1170 | 20000 | | 0.8525 | 0.504 |
| | 1750 | 29900 | | 0.8595 | 0.734 |
| Cytarabine | 780 | 111 | 1 : 0.1421 | 0.4015 | 0.878 |
| | 1170 | 166 | | 0.6327 | 0.693 |
| | 1750 | 249 | | 0.8184 | 0.581 |
| Decitabine | 520 | 1340 | 1 : 2.584 | 0.2897 | 0.570 |
| | 780 | 2020 | | 0.4383 | 0.567 |
| | 1170 | 3020 | | 0.5537 | 0.650 |
| Guadecitabine | 520 | 469 | 1 : 0.9020 | 0.2878 | 0.568 |
| | 780 | 704 | | 0.4237 | 0.583 |
| | 1170 | 1060 | | 0.5377 | 0.670 |
| Gemcitabine | 780 | 5.60 | 1 : 0.007179 | 0.6511 | 0.844 |
| | 1170 | 8.40 | | 0.8624 | 0.714 |
| Trifluridine | 780 | 431 | 1 : 0.5524 | 0.5850 | 0.869 |
| | 1170 | 646 | | 0.8250 | 0.680 |
| | 1750 | 967 | | 0.8950 | 0.739 |
| 5-Fluorouracil | 1170 | 4310 | 1 : 3.682 | 0.6738 | 0.880 |
| Azacytidine | 780 | 2740 | 1 : 3.514 | 0.5392 | 0.822 |
| | 1170 | 4110 | | 0.6909 | 0.767 |

(continued)

| Cell line: A549 (Human lung cancer cell line) | | | | | |
|---|---|---|---|---|---|
| Other antitumor agent in combined use with Example Compound 235A | Example Compound 235A (nM) | Other antitumor agent (nM) | Molar ratio of drugs in combined use (Example Compound 235A: Other antitumor agent) | Fa value | CI value |
| AZD6738 | 346 | 1030 | 1 : 2.990 | 0.5478 | 0.435 |
| | 520 | 1550 | | 0.7920 | 0.323 |
| | 780 | 2330 | | 0.8869 | 0.314 |
| | 1170 | 3500 | | 0.8817 | 0.486 |
| Prexasertib | 231 | 1.97 | 1 : 0.008547 | 0.4315 | 0.331 |
| | 346 | 2.96 | | 0.7784 | 0.191 |
| | 520 | 4.44 | | 0.8915 | 0.174 |
| SCH900776 | 231 | 909 | 1 : 3.936 | 0.6135 | 0.262 |
| | 346 | 1360 | | 0.7038 | 0.280 |
| | 520 | 2050 | | 0.8035 | 0.275 |
| | 780 | 3070 | | 0.8771 | 0.275 |
| | 1170 | 4610 | | 0.8705 | 0.430 |
| Cisplatin | 1750 | 9580 | 1 : 5.473 | 0.9113 | 0.832 |
| | 2630 | 14400 | | 0.9711 | 0.635 |
| Oxaliplatin | 1170 | 1060 | 1 : 0.9071 | 0.7355 | 0.793 |
| Carboplatin | 1750 | 79200 | 1 : 45.27 | 0.8325 | 0.609 |
| | 2630 | 119000 | | 0.9438 | 0.461 |
| | 3950 | 179000 | | 0.9600 | 0.575 |
| | 5920 | 268000 | | 0.9739 | 0.688 |
| Etoposide | 1170 | 3790 | 1 : 3.243 | 0.6978 | 0.763 |
| Sunitinib | 780 | 1870 | 1 : 2.399 | 0.6775 | 0.709 |
| | 1170 | 2810 | | 0.8225 | 0.675 |
| Cabozantinib | 1170 | 6010 | 1 : 5.135 | 0.7659 | 0.871 |
| Midostaurin | 1170 | 251 | 1 : 0.2145 | 0.7639 | 0.775 |
| Lapatinib | 1170 | 6010 | 1 : 5.135 | 0.8726 | 0.417 |
| Luminespib | 1750 | 8.13 | 1 : 0.004645 | 0.8795 | 0.819 |
| Olaparib | 1170 | 14800 | 1 : 12.67 | 0.6832 | 0.683 |
| Talazoparib | 1170 | 8000 | 1 : 6.841 | 0.6990 | 0.585 |

[Table 49]

| Cell line: MV-4-11 (Human blood cancer cell line) | | | | | |
|---|---|---|---|---|---|
| Other antitumor agent in combined use with Example Compound 235A | Example Compound 235A (nM) | Other antitumor agent (nM) | Molar ratio of drugs in combined use (Example Compound 235A: Other antitumor agent) | Fa value | CI value |
| Fludarabine nucleoside | 203 | 6150 | 1 : 30.32 | 0.7607 | 0.746 |
| | 304 | 9220 | | 0.9988 | 0.320 |
| Cytarabine | 90.1 | 116 | 1 : 1.292 | 0.1704 | 0.740 |
| | 135 | 174 | | 0.4683 | 0.645 |
| | 203 | 262 | | 0.7568 | 0.624 |
| | 304 | 393 | | 0.9522 | 0.510 |
| | 456 | 589 | | 0.9958 | 0.365 |
| | 684 | 884 | | 0.9989 | 0.374 |
| Decitabine | 90.1 | 33.4 | 1 : 0.3708 | 0.2126 | 0.725 |
| | 135 | 50.1 | | 0.3356 | 0.757 |
| | 203 | 75.3 | | 0.5302 | 0.764 |
| | 304 | 113 | | 0.7532 | 0.760 |
| | 456 | 169 | | 0.9339 | 0.687 |
| | 684 | 254 | | 0.9935 | 0.535 |
| Guadecitabine | 90.1 | 11.9 | 1 : 0.1318 | 0.2567 | 0.633 |
| | 135 | 17.8 | | 0.4131 | 0.638 |
| | 203 | 26.8 | | 0.5802 | 0.696 |
| | 304 | 40.1 | | 0.7974 | 0.689 |
| | 456 | 60.1 | | 0.9524 | 0.617 |
| | 684 | 90.2 | | 0.9930 | 0.544 |

[Table 50]

| Cell line: CFPAC-1 (Human pancreatic cancer cell line) | | | | | |
|---|---|---|---|---|---|
| Other antitumor agent in combined use with Example Compound 235A | Example Compound 235A (nM) | Other antitumor agent (nM) | Molar ratio of drugs in combined use (Example Compound 235A: Other antitumor agent) | Fa value | CI value |
| Gemcitabine | 722 | 5.28 | 1 : 0.007318 | 0.6762 | 0.722 |
| AZD6738 | 214 | 617 | 1 : 2.883 | 0.2450 | 0.777 |
| | 321 | 925 | | 0.5873 | 0.338 |
| | 481 | 1390 | | 0.7446 | 0.295 |
| | 722 | 2080 | | 0.7899 | 0.368 |
| Prexasertib | 214 | 1.89 | 1 : 0.008850 | 0.3469 | 0.645 |
| | 321 | 2.84 | | 0.6687 | 0.344 |
| | 481 | 4.26 | | 0.7578 | 0.372 |

(continued)

| Cell line: CFPAC-1 (Human pancreatic cancer cell line) | | | | | |
|---|---|---|---|---|---|
| Other antitumor agent in combined use with Example Compound 235A | Example Compound 235A (nM) | Other antitumor agent (nM) | Molar ratio of drugs in combined use (Example Compound 235A: Other antitumor agent) | Fa value | CI value |
| SCH900776 | 321 | 253 | 1 : 0.7883 | 0.4978 | 0.474 |
| | 481 | 379 | | 0.7223 | 0.382 |
| | 722 | 569 | | 0.7722 | 0.484 |

[Table 51]

| Cell line: HCT116 (Human large intestine cancer cell line) | | | | | |
|---|---|---|---|---|---|
| Other antitumor agent in combined use with Example Compound 235A | Example Compound 235A (nM) | Other antitumor agent (nM) | Molar ratio of drugs in combined use (Example Compound 235A: Other antitumor agent) | Fa value | CI value |
| AZD6738 | 475 | 339 | 1 : 0.7135 | 0.6249 | 0.429 |
| | 712 | 508 | | 0.9468 | 0.182 |
| Prexasertib | 317 | 7.15 | 1 : 0.02255 | 0.3131 | 0.557 |
| | 475 | 10.7 | | 0.8595 | 0.224 |
| | 712 | 16.1 | | 0.9459 | 0.197 |

It is apparent from these results that all the sulfonamide compounds represented by formula (I) synergistically inhibit the growth of a human lung cancer cell line, a human blood cancer cell line, a human pancreatic cancer cell line, and a human large intestine cancer cell line when used in combination with any of various antitumor agents including an antimetabolite, a platinum drug, a plant alkaloid drug, and a molecular targeting drug.

Furthermore, the combined administration groups of the sulfonamide compound represented by formula (I) and the other antitumor agent in the in vivo test using nude mice (BALB/cA Jcl-nu/nu) in which human tumor cells were transplanted showed a statistically significant antitumor effect as compared with the single administration groups of each drug on the final evaluation day. In addition, the rate of change in body weight in the combined administration groups was less than 20% of the body weight before administration (on day 0) and was change within an acceptable range.

All publications, patents and patent applications cited herein are incorporated herein by reference in their entirety.

## Claims

1. A combination formulation for treating and/or preventing tumor, comprising a sulfonamide compound represented by the following formula (I):

[Formula 1]

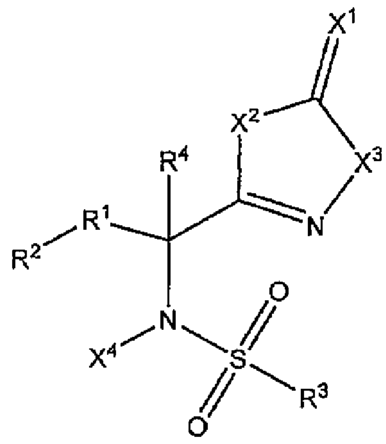

(I)

wherein $X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)
or a salt thereof, and other antitumor agent(s).

**2.** The combination formulation according to claim 1, wherein in formula (I):

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
$R^{11}$ and $R^{12}$ are the same or different and represent a hydrogen atom or a C1-C6 alkyl group;
$R^2$ represents a C6-C14 aromatic hydrocarbon group, wherein $R^2$ may have $R^{21}$ as a substituent;
$R^{21}$ represents a halogen atom or a C1-C6 alkyl group (when two or more of $R^{21}$ are present, $R^{21}$ are the same as or different from each other);
$R^3$ represents a C6-C14 aromatic hydrocarbon group which may have $R^{31}$ as a substituent or may be fused with a 4-8 membered saturated heterocyclic ring (wherein the saturated heterocyclic ring may have Rc as a substituent);
$R^{31}$ represents a halogen atom or an aminocarbonyl group (when two or more of $R^{31}$ are present, $R^{31}$ are the same as or different from each other);

Rc represents a halogen atom, a hydroxy group, or a C1-C6 alkyl group (when two or more of Rc are present, Rc are the same as or different from each other); and
$R^4$ represents a hydrogen atom.

**3.** The combination formulation according to claim 1 or 2, wherein in formula (I),

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
one of $R^{11}$ and $R^{12}$ represents a hydrogen atom, and the other represents a C1-C6 alkyl group;
$R^2$ represents a phenyl group, wherein $R^2$ may have $R^{21}$ as a substituent;
$R^{21}$ represents a halogen atom or a C1-C6 alkyl group (when two or more of $R^{21}$ are present, $R^{21}$ are the same as or different from each other);
$R^3$ represents a phenyl group which may have $R^{31}$ as a substituent or may be fused with a monocyclic 6 membered saturated heterocyclic ring having one oxygen atom (wherein the saturated heterocyclic ring may have Rc as a substituent);
$R^{31}$ represents a halogen atom or an aminocarbonyl group (when two or more of $R^{31}$ are present, $R^{31}$ are the same as or different from each other);
Rc represents a halogen atom, a hydroxy group, or a C1-C6 alkyl group (when two or more of Rc are present, Rc are the same as or different from each other); and
$R^4$ represents a hydrogen atom.

**4.** The combination formulation according to any one of claims 1-3, wherein in formula (I),

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
one of $R^{11}$ and $R^{12}$ represents a hydrogen atom, and the other represents a methyl group;
$R^2$ represents a phenyl group having $R^{21}$ as a substituent;
$R^{21}$ represents a halogen atom or a C1-C6 alkyl group (when two or more of $R^{21}$ are present, $R^{21}$ are the same as or different from each other);
$R^3$ represents a phenyl group having $R^{31}$ as a substituent or a chromanyl group having Rc as a substituent;
$R^{31}$ represents a halogen atom or an aminocarbonyl group (when two or more of $R^{31}$ are present, $R^{31}$ are the same as or different from each other);
Rc represents a halogen atom, a hydroxy group, or a C1-C6 alkyl group (when two or more of Rc are present, Rc are the same as or different from each other); and
$R^4$ represents a hydrogen atom.

**5.** The combination formulation according to any one of claims 1-4, wherein the sulfonamide compound is selected from the following compounds (1)-(5):

(1) 5-bromo-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide;
(2) 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide;
(3) 5-chloro-2-(N-((1S,2R)-2-(3-chloro-6-fluoro-2-methylphenyl)-1-(5-oxo-4,5-dihydro-1,3,4-oxadiazol-2-yl)propyl)sulfamoyl)benzamide;
(4) 5-chloro-N-((1S,2R)-2-(3-chloro-6-fluoro-2-methylphenyl)-1-(5-oxo-4,5-dihydro-1,3,4-oxadiazol-2-yl)propyl)-4-hydroxy-4-methylchromane-8-sulfonamide; and
(5) 5-chloro-N-((1S,2R)-2-(6-fluoro-2,3-dimethylphenyl)-1-(5-oxo-4,5-dihydro-1,3,4-oxadiazol-2-yl)propyl)-4-hydroxychromane-8-sulfonamide.

**6.** The combination formulation according to any one of claims 1-5, wherein the sulfonamide compound represented by formula (I) or a salt thereof and the other antitumor agent(s) are administered concurrently, sequentially, or in a

staggered manner.

**7.** The combination formulation according to any one of claims 1-6, wherein the other antitumor agent(s) is at least one or more selected from an antimetabolite, a platinum drug, a plant alkaloid drug, and a molecular targeting drug.

**8.** The combination formulation according to any one of claims 1-7, wherein the other antitumor agent(s) is at least one or more selected from 5-fluorouracil (5-FU), trifluridine, fludarabine (or an active metabolite fludarabine nucleoside), cytarabine, gemcitabine, decitabine, guadecitabine, azacitidine, cisplatin, oxaliplatin, carboplatin, etoposide, AZD6738, prexasertib, SCH900776, luminespib, olaparib, talazoparib, lapatinib, sunitinib, cabozantinib, and midostaurin.

**9.** An agent for enhancing an antitumor effect of other antitumor agent(s), comprising a sulfonamide compound represented by the following formula (I):

[Formula 2]

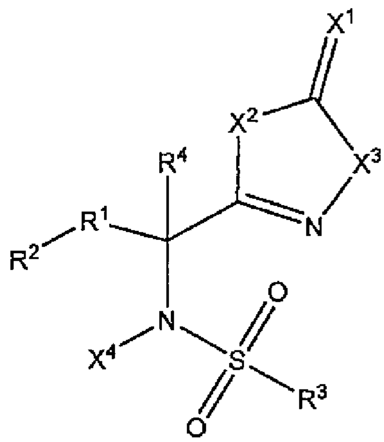

(I)

wherein $X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)
or a salt thereof as an active ingredient.

**10.** An antitumor agent for treating a cancer patient dosed with other antitumor agent(s), comprising a sulfonamide compound or a salt thereof, wherein the sulfonamide compound is a compound represented by the following formula (I):

[Formula 3]

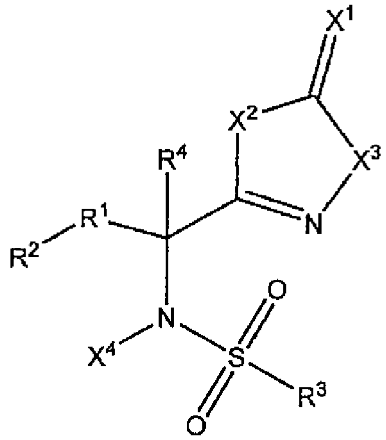

(I)

wherein $X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom).

**11.** A pharmaceutical composition comprising a sulfonamide compound or a salt thereof and other antitumor agent(s), wherein the sulfonamide compound is a compound represented by the following formula (I):

[Formula 4]

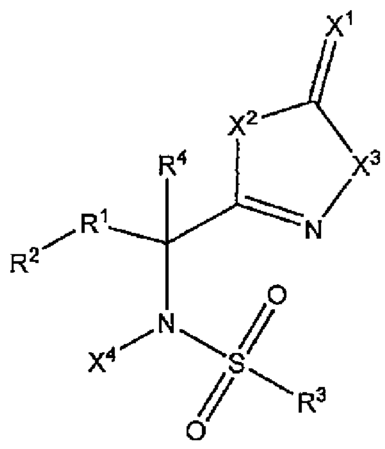

(I)

wherein $X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom).

**12.** A sulfonamide compound represented by the following formula (I):

[Formula 5]

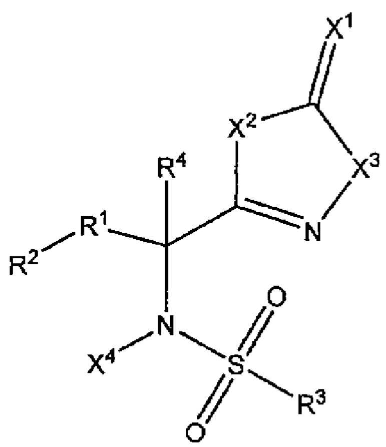

(I)

wherein $X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents 4-methylphenyl group, and $R^4$ represents a hydrogen atom)
or a salt thereof for use in treating and/or preventing tumor by administering the sulfonamide compound or a salt thereof and other antitumor agent(s) concurrently, sequentially, or in a staggered manner.

**13.** A product comprising a sulfonamide compound represented by the following formula (I):

[Formula 6]

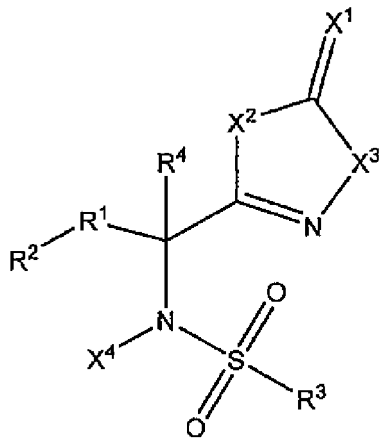

(I)

wherein $X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms

to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)
or a salt thereof for use in treating and/or preventing tumor by administering the sulfonamide compound or a salt thereof and other antitumor agent(s) concurrently, sequentially, or in a staggered manner,
and the other antitumor agent(s), as a combination formulation.

**14.** A method of treating and/or preventing tumor comprising administering a sulfonamide compound represented by the following formula (I):

[Formula 7]

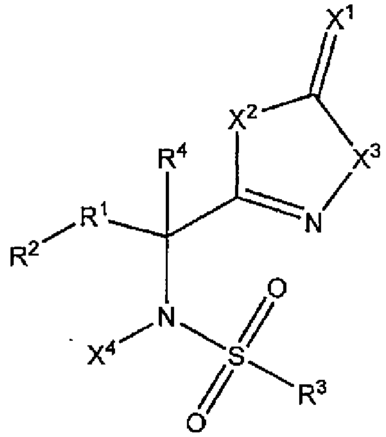

(I)

wherein $X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)

or a salt thereof and other antitumor agent(s) concurrently, sequentially, or in a staggered manner.

**15.** A method of treating and/or preventing tumor comprising administering a sulfonamide compound represented by the following formula (I):

[Formula 8]

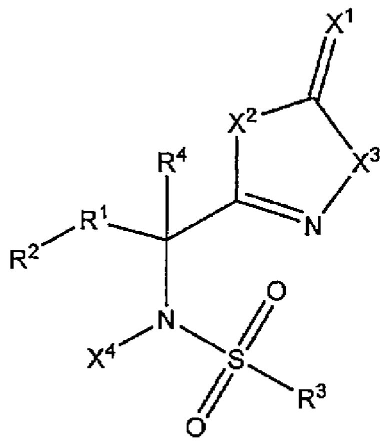

(I)

wherein $X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ are the same or different and represent a hydrogen atom, a halogen atom, a hydroxy group, or a C1-C6 alkyl group, alternatively may be taken together with the carbon atoms to which $R^{11}$ and $R^{12}$ are attached to form a saturated hydrocarbon ring having 3 to 8 carbon atoms;
$R^2$ represents a C6-C14 aromatic hydrocarbon group or a 9 or 10 membered fully unsaturated heterocyclic group, wherein $R^2$ may have substituents, and when $R^2$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents;
$R^3$ represents a C6-C14 aromatic hydrocarbon group or a 5-10 membered fully unsaturated heterocyclic group, wherein $R^3$ may have substituents, and when $R^3$ has two substituents on the carbon atoms which are adjacent to each other on the aromatic hydrocarbon ring, the substituents may be fused together with the carbon atoms to which the substituents are attached to form a saturated or partially unsaturated 4-8 membered hydrocarbon ring or heterocyclic ring, either of which may have substituents; and
$R^4$ represents a hydrogen atom or a C1-C6 alkyl group;
(with the proviso that $X^1$ is an oxygen atom when $X^2$ represents an oxygen atom, $X^3$ represents -NH-, $X^4$ represents a hydrogen atom, $R^1$ represents $-CH_2-$, $R_2$ represents a phenyl group, $R^3$ represents 4-methylphenyl group, and $R^4$ represents a hydrogen atom)
or a salt thereof to a cancer patient dosed with other antitumor agent(s).

[Figure 1]

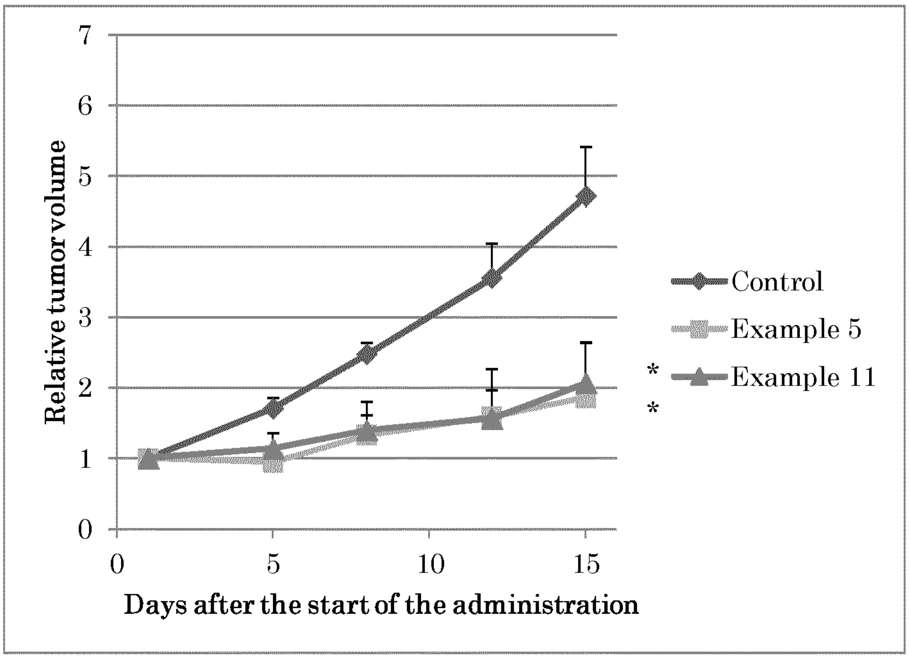


[Figure 2]

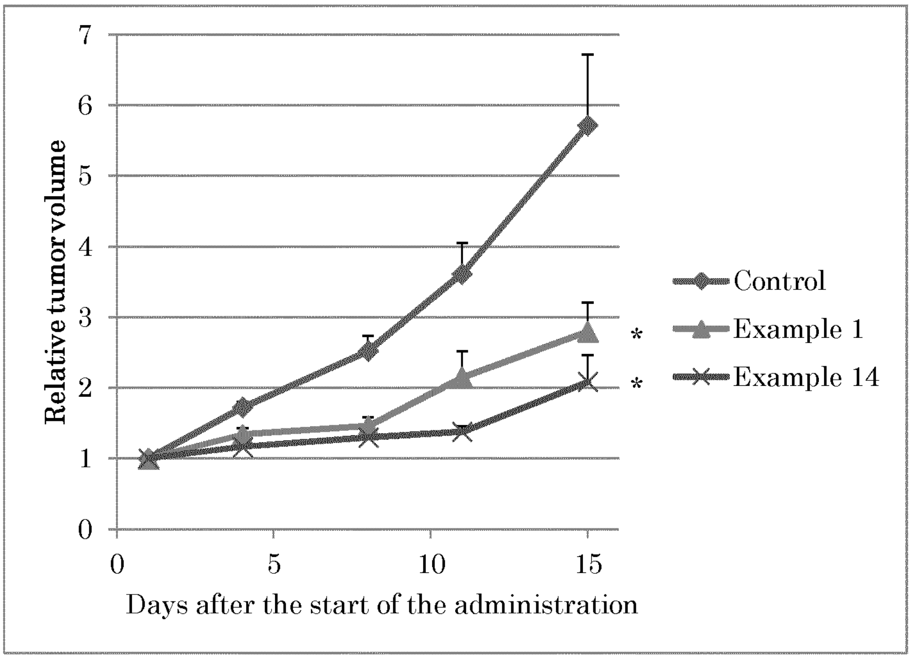

[Figure 3]

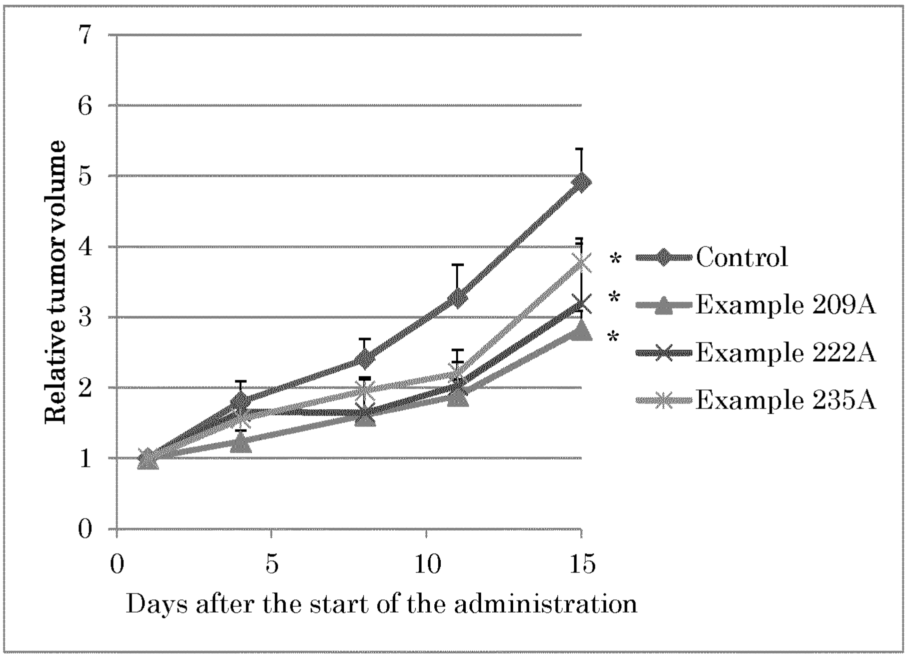


[Figure 4]

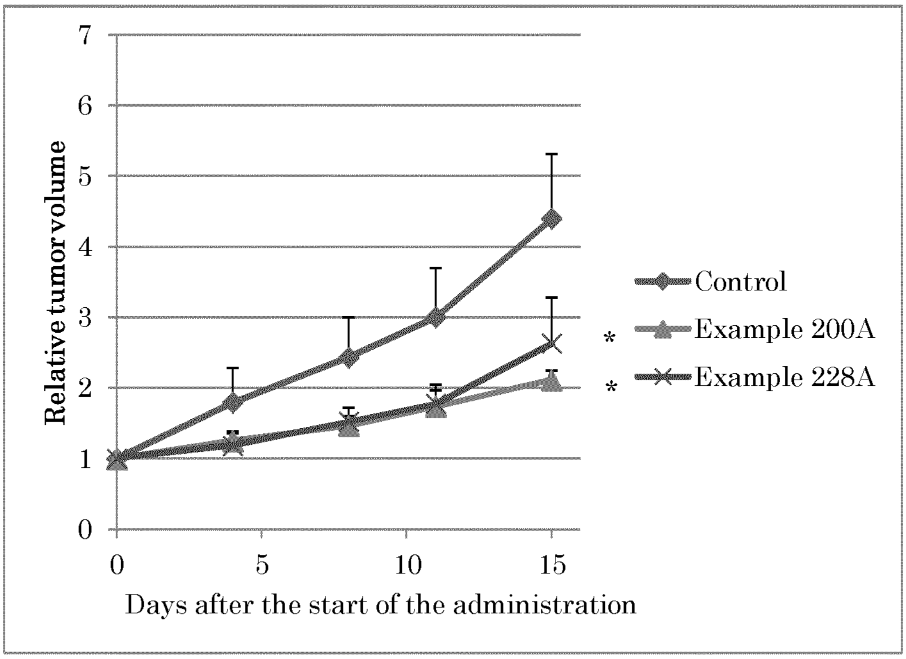

**INTERNATIONAL SEARCH REPORT**

International application No.
PCT/JP2018/043697

A. CLASSIFICATION OF SUBJECT MATTER
Int.Cl. A61K31/4245(2006.01)i, A61K45/00(2006.01)i, A61P35/00(2006.01)i, A61P43/00(2006.01)i, C07D249/04(2006.01)i
According to International Patent Classification (IPC) or to both national classification and IPC

B. FIELDS SEARCHED

Minimum documentation searched (classification system followed by classification symbols)
Int.Cl. A61K31/4245, A61K45/00, A61P35/00, A61P43/00, C07D249/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN), JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| P, X<br>P, A | WO 2017/209155 A1 (TAIHO PHARMACEUTICAL CO., LTD.) 07 December 2017, claims 1-33 & CA 3025887 A1, claims 1-33 & TW 201800395 A | 12<br>1-11, 13-15 |
| A | WO 2017/150725 A1 (TAIHO PHARMACEUTICAL CO., LTD.) 08 September 2017 & AU 2017226389 A & KR 10-2018-0118719 A & TW 201733590 A | 1-15 |
| A | WO 2017/111074 A1 (TAIHO PHARMACEUTICAL CO., LTD.) 29 June 2017 & EP 3395345 A1 & AU 2016379290 A & KR 10-2018-0095054 A & TW 201729811 A | 1-15 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 January 2019 (28.01.2019) | 05 February 2019 (05.02.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017229681 A
- WO 2011071565 A


### Non-patent literature cited in the description


- *Annu. Rev. Biochem.,* 1998, vol. 67, 71-98
- *J. Biol. Chem.,* 1970, vol. 245, 5228-5233
- *Nat. Commun.,* 2014, vol. 5
- *Clin. Sci.,* 2013, vol. 124, 567-578
- *Expert. Opin. Ther. Targets,* 2013, vol. 17, 1423-1437
- *Biochem. Pharmacol.,* 2000, vol. 59, 983-991
- *Biochem. Pharmacol.,* 2009, vol. 78, 1178-11 85
- *Cancer Res.,* 1994, vol. 54, 3686-3691
- *Pharmacol. Rev.,* 2005, vol. 57, 547-583
- *Future Oncol.,* 2012, vol. 8, 145-150
- *Biochem. Pharmacol.,* 2007, vol. 73, 1548-1557
- *Tetrahedoron Lett.,* 2010, vol. 51, 418-421
- Development of Pharmaceuticals. Molecular Design. Hirokawa Shoten, 1990, vol. 7, 163-198
- *CANCER RESEARCH,* 2004, vol. 64 (1 - 6
- *Trends Pharmacol. Sci.,* 1983, vol. 4, 450-454
- *Pharmacol. Rev.,* 2006, vol. 58 (3), 621-681
- *Synergy,* 2014, vol. 1, 3-21